(19) Europäisches Patentamt
European Patent Office
Office européen des brevets

(11) **EP 4 043 581 A1**

(12) **EUROPEAN PATENT APPLICATION**

(43) Date of publication:
**17.08.2022 Bulletin 2022/33**

(21) Application number: **21215237.5**

(22) Date of filing: **26.05.2017**

(51) International Patent Classification (IPC):
***C12Q 1/68*** *(2018.01)* ***G16B 25/00*** *(2019.01)*
***G16B 30/00*** *(2019.01)* ***G16B 35/00*** *(2019.01)*
***G16C 20/60*** *(2019.01)* ***G16H 15/00*** *(2018.01)*
***C12Q 1/6844*** *(2018.01)* ***G16B 30/10*** *(2019.01)*
***G16H 50/30*** *(2018.01)*

(52) Cooperative Patent Classification (CPC):
(C-Sets available)
**C12Q 1/6844; G16B 25/00; G16B 30/00; G16B 30/10; G16B 35/00; G16C 20/60; G16H 15/00; G16H 50/30;** Y02A 90/10 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2016 US 201662342839 P**

(62) Document number(s) of the earlier application(s) in accordance with Art. 76 EPC:
**17729636.5 / 3 464 626**

(71) Applicant: **Sequenom, Inc.**
**San Diego, CA 92121 (US)**

(72) Inventors:
- **JENSEN, Taylor Jacob**
  **San Diego, CA 92131 (US)**
- **EHRICH, Mathias**
  **San Diego, CA 92109 (US)**
- **VAN DEN BOOM, Dirk**
  **Encinitas, CA 92024 (US)**
- **TYNAN, John Allen**
  **San Diego, CA 92128 (US)**
- **KIM, Sung Kyun**
  **San Diego, CA 92129 (US)**
- **BURCHAM, Timothy S.**
  **Encinitas, CA 92024 (US)**
- **ELLISON, Christopher K.**
  **San Diego, CA 92122 (US)**
- **SUN, Youting**
  **San Diego, CA 92129 (US)**

(74) Representative: **Vossius & Partner**
**Patentanwälte Rechtsanwälte mbB**
**Siebertstraße 3**
**81675 München (DE)**

Remarks:
- This application was filed on 16.12.2021 as a divisional application to the application mentioned under INID code 62.
- Claims filed after the date of filing of the application / after the date of receipt of the divisional application (Rule 68(4) EPC).

(54) **METHOD FOR GENERATING A PARALOG ASSAY SYSTEM**

(57) Technology provided herein relates in part to methods, processes, machines and apparatuses for detecting genetic variations. In some embodiments, the technology is related to non-invasive assessment of aneuploidis.

FIG. 1

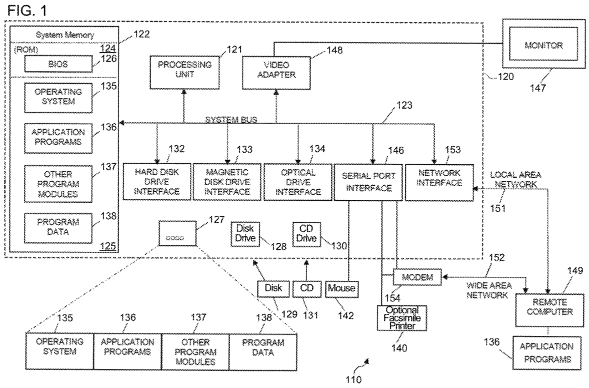


EP 4 043 581 A1

(52) Cooperative Patent Classification (CPC): (Cont.)

C-Sets
**C12Q 1/6844, C12Q 2537/143, C12Q 2537/16,**
**C12Q 2537/165, C12Q 2545/114**

SEQUENCE LISTING

<110> Sequenom, Inc.

<120> Methods for Detecting Genetic Variations

<130> AB3154 EP/1

<150> US 62/342,839
<151> 2016-05-27

<160> 1800

<170> PatentIn version 3.5

<210> 1
<211> 22
<212> DNA
<213> Artificial Sequence

<220>
<223> Synthetic Construct

<400> 1
ggataacagg tagatgagaa ag 22

<210> 2
<211> 23
<212> DNA
<213> Artificial Sequence

<220>
<223> Synthetic Construct

<400> 2
caaattataa atgttctagc ctc 23

<210> 3
<211> 20
<212> DNA
<213> Artificial Sequence

<220>
<223> Synthetic Construct

<400> 3
gatcagatca atttggatgg 20

<210> 4
<211> 18
<212> DNA
<213> Artificial Sequence

<220>
<223> Synthetic Construct

<400> 4
acccagtctc cagttcct 18

<210> 5
<211> 20
<212> DNA
<213> Artificial Sequence

<220>
<223> Synthetic Construct

<400> 5
ctagtgaggg ttttgttttg 20

<210> 6
<211> 22
<212> DNA
<213> Artificial Sequence

<220>
<223> Synthetic Construct

<400> 6
tcaatctctt cctctctact gg 22

<210> 7
<211> 18
<212> DNA
<213> Artificial Sequence

<220>
<223> Synthetic Construct

<400> 7
gcgtatgatc aggcaccc 18

<210> 8
<211> 18
<212> DNA
<213> Artificial Sequence

<220>
<223> Synthetic Construct

<400> 8
accaggggta cagtggtg 18

<210> 9
<211> 19
<212> DNA
<213> Artificial Sequence

<220>
<223> Synthetic Construct

<400> 9
agcaagagcc cctgcatgt 19

<210> 10
<211> 21
<212> DNA

<213> Artificial Sequence

<220>
<223> Synthetic Construct

<400> 10
ctgtggattg taggtgtaat g 21

<210> 11
<211> 19
<212> DNA
<213> Artificial Sequence

<220>
<223> Synthetic Construct

<400> 11
taggcatgaa aggggcctg 19

<210> 12
<211> 18
<212> DNA
<213> Artificial Sequence

<220>
<223> Synthetic Construct

<400> 12
aaagccccga cctcagga 18

<210> 13
<211> 18
<212> DNA
<213> Artificial Sequence

<220>
<223> Synthetic Construct

<400> 13
ggtaggccag aacatgcc 18

<210> 14
<211> 20
<212> DNA
<213> Artificial Sequence

<220>
<223> Synthetic Construct

<400> 14
tatacccgaa aggaccaaga 20

<210> 15
<211> 18
<212> DNA
<213> Artificial Sequence

<220>

<223>  Synthetic Construct

<400>  15
gtgcgtgggt gtagggtg                                                    18

<210>  16
<211>  18
<212>  DNA
<213>  Artificial Sequence

<220>
<223>  Synthetic Construct

<400>  16
gtggactcgc acccaagg                                                    18

<210>  17
<211>  18
<212>  DNA
<213>  Artificial Sequence

<220>
<223>  Synthetic Construct

<400>  17
tgtcctatga ggatgtgg                                                    18

<210>  18
<211>  18
<212>  DNA
<213>  Artificial Sequence

<220>
<223>  Synthetic Construct

<400>  18
ccggtagcgt gccaagtc                                                    18

<210>  19
<211>  18
<212>  DNA
<213>  Artificial Sequence

<220>
<223>  Synthetic Construct

<400>  19
gcaaactgag tcgccctc                                                    18

<210>  20
<211>  19
<212>  DNA
<213>  Artificial Sequence

<220>
<223>  Synthetic Construct

<400>  20

catccccatc aatgctcag 19

<210> 21
<211> 18
<212> DNA
<213> Artificial Sequence

<220>
<223> Synthetic Construct

<400> 21
cccttggtgc tccttcag 18

<210> 22
<211> 18
<212> DNA
<213> Artificial Sequence

<220>
<223> Synthetic Construct

<400> 22
tcaggccatc aatcaccc 18

<210> 23
<211> 18
<212> DNA
<213> Artificial Sequence

<220>
<223> Synthetic Construct

<400> 23
tgccaggcta gacgattc 18

<210> 24
<211> 19
<212> DNA
<213> Artificial Sequence

<220>
<223> Synthetic Construct

<400> 24
gactgtgcct tcacctctc 19

<210> 25
<211> 18
<212> DNA
<213> Artificial Sequence

<220>
<223> Synthetic Construct

<400> 25
gcaagcatgt cagcaacc 18

<210> 26
<211> 20
<212> DNA
<213> Artificial Sequence

<220>
<223> Synthetic Construct

<400> 26
tggagaagtt aggaaaatca 20

<210> 27
<211> 19
<212> DNA
<213> Artificial Sequence

<220>
<223> Synthetic Construct

<400> 27
ccccaactcc aacaggatt 19

<210> 28
<211> 20
<212> DNA
<213> Artificial Sequence

<220>
<223> Synthetic Construct

<400> 28
ttacaaagca tctccgatca 20

<210> 29
<211> 20
<212> DNA
<213> Artificial Sequence

<220>
<223> Synthetic Construct

<400> 29
ggagtagctg tagttgcaga 20

<210> 30
<211> 18
<212> DNA
<213> Artificial Sequence

<220>
<223> Synthetic Construct

<400> 30
caccttagtg gccatttt 18

<210> 31
<211> 20
<212> DNA

<213> Artificial Sequence

<220>
<223> Synthetic Construct

<400> 31
aggggagggg aatgacttat 20

<210> 32
<211> 21
<212> DNA
<213> Artificial Sequence

<220>
<223> Synthetic Construct

<400> 32
tgtctgtggt ggtttctgtt t 21

<210> 33
<211> 18
<212> DNA
<213> Artificial Sequence

<220>
<223> Synthetic Construct

<400> 33
cccaggatgt gggactca 18

<210> 34
<211> 18
<212> DNA
<213> Artificial Sequence

<220>
<223> Synthetic Construct

<400> 34
ccccatacac tactcccc 18

<210> 35
<211> 18
<212> DNA
<213> Artificial Sequence

<220>
<223> Synthetic Construct

<400> 35
agtgctgggg ttcaggct 18

<210> 36
<211> 18
<212> DNA
<213> Artificial Sequence

<220>

<223> Synthetic Construct

<400> 36
agtccccgaa ggtcccac 18

<210> 37
<211> 18
<212> DNA
<213> Artificial Sequence

<220>
<223> Synthetic Construct

<400> 37
tctggcttgc cataggtg 18

<210> 38
<211> 18
<212> DNA
<213> Artificial Sequence

<220>
<223> Synthetic Construct

<400> 38
gctgagattg aggccctg 18

<210> 39
<211> 19
<212> DNA
<213> Artificial Sequence

<220>
<223> Synthetic Construct

<400> 39
gctggcaaac agttcaaca 19

<210> 40
<211> 18
<212> DNA
<213> Artificial Sequence

<220>
<223> Synthetic Construct

<400> 40
cgccatcgaa gaatacat 18

<210> 41
<211> 20
<212> DNA
<213> Artificial Sequence

<220>
<223> Synthetic Construct

<400> 41

tggatgagtc agggatcttg 20

<210> 42
<211> 20
<212> DNA
<213> Artificial Sequence

<220>
<223> Synthetic Construct

<400> 42
aagcccacgc ttaggttagg 20

<210> 43
<211> 22
<212> DNA
<213> Artificial Sequence

<220>
<223> Synthetic Construct

<400> 43
cattctgtaa actgtgcttg tc 22

<210> 44
<211> 20
<212> DNA
<213> Artificial Sequence

<220>
<223> Synthetic Construct

<400> 44
cttaacggga ccacattgaa 20

<210> 45
<211> 21
<212> DNA
<213> Artificial Sequence

<220>
<223> Synthetic Construct

<400> 45
gggtgactaa aactcccgtt a 21

<210> 46
<211> 20
<212> DNA
<213> Artificial Sequence

<220>
<223> Synthetic Construct

<400> 46
ttagctcagc ctgctggatt 20

<210> 47
<211> 20
<212> DNA
<213> Artificial Sequence

<220>
<223> Synthetic Construct

<400> 47
ttgcatactg acagcgagaa 20

<210> 48
<211> 20
<212> DNA
<213> Artificial Sequence

<220>
<223> Synthetic Construct

<400> 48
ttggcgcaga tgtgatagag 20

<210> 49
<211> 21
<212> DNA
<213> Artificial Sequence

<220>
<223> Synthetic Construct

<400> 49
cctcgtatta ttcccacgtt t 21

<210> 50
<211> 20
<212> DNA
<213> Artificial Sequence

<220>
<223> Synthetic Construct

<400> 50
tcaggctgga tctactggaa 20

<210> 51
<211> 19
<212> DNA
<213> Artificial Sequence

<220>
<223> Synthetic Construct

<400> 51
ttcacgcctg tgactggtt 19

<210> 52
<211> 19
<212> DNA

<213> Artificial Sequence

<220>
<223> Synthetic Construct

<400> 52
gatgcatcgc tgggtaaag 19

<210> 53
<211> 20
<212> DNA
<213> Artificial Sequence

<220>
<223> Synthetic Construct

<400> 53
cagattggtg agtcctcgaa 20

<210> 54
<211> 20
<212> DNA
<213> Artificial Sequence

<220>
<223> Synthetic Construct

<400> 54
ctgacaaaca atcgaatagg 20

<210> 55
<211> 18
<212> DNA
<213> Artificial Sequence

<220>
<223> Synthetic Construct

<400> 55
gtgcggttgg gcagagta 18

<210> 56
<211> 18
<212> DNA
<213> Artificial Sequence

<220>
<223> Synthetic Construct

<400> 56
cgagggcaag gtggtgtg 18

<210> 57
<211> 19
<212> DNA
<213> Artificial Sequence

<220>

<223>  Synthetic Construct

<400>  57
agttgggaag gaggggtcc                                                    19

<210>  58
<211>  19
<212>  DNA
<213>  Artificial Sequence

<220>
<223>  Synthetic Construct

<400>  58
atttaacacc ccacaccca                                                    19

<210>  59
<211>  18
<212>  DNA
<213>  Artificial Sequence

<220>
<223>  Synthetic Construct

<400>  59
gtgctgggca gcttgtct                                                     18

<210>  60
<211>  18
<212>  DNA
<213>  Artificial Sequence

<220>
<223>  Synthetic Construct

<400>  60
ggcctagagg agggagtg                                                     18

<210>  61
<211>  20
<212>  DNA
<213>  Artificial Sequence

<220>
<223>  Synthetic Construct

<400>  61
ggcaaaagct cgtatcatgt                                                   20

<210>  62
<211>  19
<212>  DNA
<213>  Artificial Sequence

<220>
<223>  Synthetic Construct

<400>  62

ggttgcctcc cagcttctc 19

<210> 63
<211> 20
<212> DNA
<213> Artificial Sequence

<220>
<223> Synthetic Construct

<400> 63
aggggtgtac tcagggtgac 20

<210> 64
<211> 19
<212> DNA
<213> Artificial Sequence

<220>
<223> Synthetic Construct

<400> 64
ggttccacct cgtggtctg 19

<210> 65
<211> 18
<212> DNA
<213> Artificial Sequence

<220>
<223> Synthetic Construct

<400> 65
atcaccaaag ctcacacc 18

<210> 66
<211> 18
<212> DNA
<213> Artificial Sequence

<220>
<223> Synthetic Construct

<400> 66
tttgttcacg gcggaagc 18

<210> 67
<211> 20
<212> DNA
<213> Artificial Sequence

<220>
<223> Synthetic Construct

<400> 67
cctcaactct tgagaccacc 20

<210> 68
<211> 18
<212> DNA
<213> Artificial Sequence

<220>
<223> Synthetic Construct

<400> 68
tgttggttct gcgctctg 18

<210> 69
<211> 18
<212> DNA
<213> Artificial Sequence

<220>
<223> Synthetic Construct

<400> 69
cttcgtcagc gttcctgg 18

<210> 70
<211> 18
<212> DNA
<213> Artificial Sequence

<220>
<223> Synthetic Construct

<400> 70
ggcggctgga caggtttt 18

<210> 71
<211> 20
<212> DNA
<213> Artificial Sequence

<220>
<223> Synthetic Construct

<400> 71
tgggatgaga gtgagtattg 20

<210> 72
<211> 18
<212> DNA
<213> Artificial Sequence

<220>
<223> Synthetic Construct

<400> 72
cacaggggct tgccatta 18

<210> 73
<211> 21
<212> DNA

<213> Artificial Sequence

<220>
<223> Synthetic Construct

<400> 73
ctcggtgcta gtcttatgga a 21

<210> 74
<211> 20
<212> DNA
<213> Artificial Sequence

<220>
<223> Synthetic Construct

<400> 74
ggaccaagat aggaggtggg 20

<210> 75
<211> 21
<212> DNA
<213> Artificial Sequence

<220>
<223> Synthetic Construct

<400> 75
atgggcttac atctctgtat c 21

<210> 76
<211> 20
<212> DNA
<213> Artificial Sequence

<220>
<223> Synthetic Construct

<400> 76
tcatagagtg gccaagtttc 20

<210> 77
<211> 20
<212> DNA
<213> Artificial Sequence

<220>
<223> Synthetic Construct

<400> 77
caaatccaca acctcatact 20

<210> 78
<211> 18
<212> DNA
<213> Artificial Sequence

<220>

<223> Synthetic Construct

<400> 78
gcccagagag ggacatca 18

<210> 79
<211> 22
<212> DNA
<213> Artificial Sequence

<220>
<223> Synthetic Construct

<400> 79
aaatacattt accaggccat ca 22

<210> 80
<211> 20
<212> DNA
<213> Artificial Sequence

<220>
<223> Synthetic Construct

<400> 80
tcaagtacgt ttccatccca 20

<210> 81
<211> 21
<212> DNA
<213> Artificial Sequence

<220>
<223> Synthetic Construct

<400> 81
ggaacattta acaatgtgca g 21

<210> 82
<211> 19
<212> DNA
<213> Artificial Sequence

<220>
<223> Synthetic Construct

<400> 82
tttactaccc ttggatgct 19

<210> 83
<211> 20
<212> DNA
<213> Artificial Sequence

<220>
<223> Synthetic Construct

<400> 83

cactaatgac ccaatgaatc 20

<210> 84
<211> 18
<212> DNA
<213> Artificial Sequence

<220>
<223> Synthetic Construct

<400> 84
acgtgggtgc ttgtgttt 18

<210> 85
<211> 18
<212> DNA
<213> Artificial Sequence

<220>
<223> Synthetic Construct

<400> 85
agaggagggc agcatctc 18

<210> 86
<211> 19
<212> DNA
<213> Artificial Sequence

<220>
<223> Synthetic Construct

<400> 86
tggataggca tgtagacct 19

<210> 87
<211> 20
<212> DNA
<213> Artificial Sequence

<220>
<223> Synthetic Construct

<400> 87
ggcaagggag aagacagcag 20

<210> 88
<211> 18
<212> DNA
<213> Artificial Sequence

<220>
<223> Synthetic Construct

<400> 88
agaagcaggc tgtcgatt 18

<210> 89
<211> 19
<212> DNA
<213> Artificial Sequence

<220>
<223> Synthetic Construct

<400> 89
caattgcatc taccatcca 19

<210> 90
<211> 23
<212> DNA
<213> Artificial Sequence

<220>
<223> Synthetic Construct

<400> 90
tgaagttgtt ggtttaattt tcc 23

<210> 91
<211> 18
<212> DNA
<213> Artificial Sequence

<220>
<223> Synthetic Construct

<400> 91
ctgagcgctg tcccatta 18

<210> 92
<211> 18
<212> DNA
<213> Artificial Sequence

<220>
<223> Synthetic Construct

<400> 92
ccgtccctac cttaccct 18

<210> 93
<211> 20
<212> DNA
<213> Artificial Sequence

<220>
<223> Synthetic Construct

<400> 93
ggagccagag agccagtttt 20

<210> 94
<211> 22
<212> DNA

<213> Artificial Sequence

<220>
<223> Synthetic Construct

<400> 94
gagacacctg tcaccaagac ac 22

<210> 95
<211> 20
<212> DNA
<213> Artificial Sequence

<220>
<223> Synthetic Construct

<400> 95
aggatgcagt tctggaaaca 20

<210> 96
<211> 19
<212> DNA
<213> Artificial Sequence

<220>
<223> Synthetic Construct

<400> 96
aaaattgtca ctgccggtc 19

<210> 97
<211> 20
<212> DNA
<213> Artificial Sequence

<220>
<223> Synthetic Construct

<400> 97
acctggccct agcatctacc 20

<210> 98
<211> 18
<212> DNA
<213> Artificial Sequence

<220>
<223> Synthetic Construct

<400> 98
ggtcaggggt tgggagtt 18

<210> 99
<211> 18
<212> DNA
<213> Artificial Sequence

<220>

<223>  Synthetic Construct

<400>  99
ctgtaagggg tcacgggg                                                    18

<210>  100
<211>  18
<212>  DNA
<213>  Artificial Sequence

<220>
<223>  Synthetic Construct

<400>  100
ccagctctta gctccctt                                                    18

<210>  101
<211>  18
<212>  DNA
<213>  Artificial Sequence

<220>
<223>  Synthetic Construct

<400>  101
agggcaggac cagaacca                                                    18

<210>  102
<211>  18
<212>  DNA
<213>  Artificial Sequence

<220>
<223>  Synthetic Construct

<400>  102
tgtcatgtcc ctggctgt                                                    18

<210>  103
<211>  18
<212>  DNA
<213>  Artificial Sequence

<220>
<223>  Synthetic Construct

<400>  103
tgctcaccac ccttttcg                                                    18

<210>  104
<211>  18
<212>  DNA
<213>  Artificial Sequence

<220>
<223>  Synthetic Construct

<400>  104

gagagcagaa gggagaaa 18

<210> 105
<211> 19
<212> DNA
<213> Artificial Sequence

<220>
<223> Synthetic Construct

<400> 105
taaagcaggg gtaagattg 19

<210> 106
<211> 20
<212> DNA
<213> Artificial Sequence

<220>
<223> Synthetic Construct

<400> 106
tggagtctca gtcagtcatt 20

<210> 107
<211> 18
<212> DNA
<213> Artificial Sequence

<220>
<223> Synthetic Construct

<400> 107
agaggctttg acttggtt 18

<210> 108
<211> 18
<212> DNA
<213> Artificial Sequence

<220>
<223> Synthetic Construct

<400> 108
cccagccaag tatgtttt 18

<210> 109
<211> 20
<212> DNA
<213> Artificial Sequence

<220>
<223> Synthetic Construct

<400> 109
tgaaatatgc agatgagtga 20

<210> 110
<211> 21
<212> DNA
<213> Artificial Sequence

<220>
<223> Synthetic Construct

<400> 110
tgactcggtt actgagcatg a 21

<210> 111
<211> 20
<212> DNA
<213> Artificial Sequence

<220>
<223> Synthetic Construct

<400> 111
ggaattatct gagcatgcag 20

<210> 112
<211> 18
<212> DNA
<213> Artificial Sequence

<220>
<223> Synthetic Construct

<400> 112
atgcacacct atgcattt 18

<210> 113
<211> 22
<212> DNA
<213> Artificial Sequence

<220>
<223> Synthetic Construct

<400> 113
caaatgaata aaatgtccct gc 22

<210> 114
<211> 20
<212> DNA
<213> Artificial Sequence

<220>
<223> Synthetic Construct

<400> 114
caagaaagtt ggtgctagtg 20

<210> 115
<211> 22
<212> DNA

<213> Artificial Sequence

<220>
<223> Synthetic Construct

<400> 115
cccatttgaa gttacacgtt tt 22

<210> 116
<211> 20
<212> DNA
<213> Artificial Sequence

<220>
<223> Synthetic Construct

<400> 116
tgaagactta aggggcaaag 20

<210> 117
<211> 19
<212> DNA
<213> Artificial Sequence

<220>
<223> Synthetic Construct

<400> 117
tggccatgtg accaagttt 19

<210> 118
<211> 20
<212> DNA
<213> Artificial Sequence

<220>
<223> Synthetic Construct

<400> 118
ggaagacttt tggaagttgc 20

<210> 119
<211> 21
<212> DNA
<213> Artificial Sequence

<220>
<223> Synthetic Construct

<400> 119
tggaatttgg attctacctt g 21

<210> 120
<211> 20
<212> DNA
<213> Artificial Sequence

<220>

<223> Synthetic Construct

<400> 120
tccatttagc tcaccgttcc 20

<210> 121
<211> 20
<212> DNA
<213> Artificial Sequence

<220>
<223> Synthetic Construct

<400> 121
ccctactagg taccttgggt 20

<210> 122
<211> 24
<212> DNA
<213> Artificial Sequence

<220>
<223> Synthetic Construct

<400> 122
gcttctagta tccccattac ttat 24

<210> 123
<211> 18
<212> DNA
<213> Artificial Sequence

<220>
<223> Synthetic Construct

<400> 123
gagccccaac atggtttc 18

<210> 124
<211> 22
<212> DNA
<213> Artificial Sequence

<220>
<223> Synthetic Construct

<400> 124
ggaagagaac tgatcaccga ta 22

<210> 125
<211> 18
<212> DNA
<213> Artificial Sequence

<220>
<223> Synthetic Construct

<400> 125

gaggcttgat gggaatga 18

<210> 126
<211> 18
<212> DNA
<213> Artificial Sequence

<220>
<223> Synthetic Construct

<400> 126
gaggttagct caggcccc 18

<210> 127
<211> 20
<212> DNA
<213> Artificial Sequence

<220>
<223> Synthetic Construct

<400> 127
cactttacca ggcacacagg 20

<210> 128
<211> 21
<212> DNA
<213> Artificial Sequence

<220>
<223> Synthetic Construct

<400> 128
agatcaatgg agtgagagag g 21

<210> 129
<211> 20
<212> DNA
<213> Artificial Sequence

<220>
<223> Synthetic Construct

<400> 129
agtgaggtgg ttggattaca 20

<210> 130
<211> 21
<212> DNA
<213> Artificial Sequence

<220>
<223> Synthetic Construct

<400> 130
cctctgacac atgtattgat t 21

<210> 131
<211> 20
<212> DNA
<213> Artificial Sequence

<220>
<223> Synthetic Construct

<400> 131
tggcaaagac ctagaaaggg 20

<210> 132
<211> 19
<212> DNA
<213> Artificial Sequence

<220>
<223> Synthetic Construct

<400> 132
aagccttttg tggggtaaa 19

<210> 133
<211> 18
<212> DNA
<213> Artificial Sequence

<220>
<223> Synthetic Construct

<400> 133
atggacacgg cctcctct 18

<210> 134
<211> 19
<212> DNA
<213> Artificial Sequence

<220>
<223> Synthetic Construct

<400> 134
catcaatcag acagcctcg 19

<210> 135
<211> 18
<212> DNA
<213> Artificial Sequence

<220>
<223> Synthetic Construct

<400> 135
tggaacctga gcgcttga 18

<210> 136
<211> 19
<212> DNA

<213> Artificial Sequence

<220>
<223> Synthetic Construct

<400> 136
aaggttcagt ggtgttttg 19

<210> 137
<211> 20
<212> DNA
<213> Artificial Sequence

<220>
<223> Synthetic Construct

<400> 137
ggaaggcatt cagaggacat 20

<210> 138
<211> 18
<212> DNA
<213> Artificial Sequence

<220>
<223> Synthetic Construct

<400> 138
cagcggggtc ctagttct 18

<210> 139
<211> 20
<212> DNA
<213> Artificial Sequence

<220>
<223> Synthetic Construct

<400> 139
acactcaaca aactgtggca 20

<210> 140
<211> 19
<212> DNA
<213> Artificial Sequence

<220>
<223> Synthetic Construct

<400> 140
ggcacctgct gttcttttg 19

<210> 141
<211> 18
<212> DNA
<213> Artificial Sequence

<220>

<223> Synthetic Construct

<400> 141
tctctgggtg actgcccc 18

<210> 142
<211> 18
<212> DNA
<213> Artificial Sequence

<220>
<223> Synthetic Construct

<400> 142
tgtttgatgg gaggatgt 18

<210> 143
<211> 19
<212> DNA
<213> Artificial Sequence

<220>
<223> Synthetic Construct

<400> 143
cattccggaa gggtctgag 19

<210> 144
<211> 20
<212> DNA
<213> Artificial Sequence

<220>
<223> Synthetic Construct

<400> 144
attagtactg ccatgtcctg 20

<210> 145
<211> 18
<212> DNA
<213> Artificial Sequence

<220>
<223> Synthetic Construct

<400> 145
cctggctcta ggcactca 18

<210> 146
<211> 18
<212> DNA
<213> Artificial Sequence

<220>
<223> Synthetic Construct

<400> 146

aattagggct aggggtgt 18

<210> 147
<211> 22
<212> DNA
<213> Artificial Sequence

<220>
<223> Synthetic Construct

<400> 147
attaaccttt cagttactct gg 22

<210> 148
<211> 20
<212> DNA
<213> Artificial Sequence

<220>
<223> Synthetic Construct

<400> 148
atgtcggctt tctatattgc 20

<210> 149
<211> 23
<212> DNA
<213> Artificial Sequence

<220>
<223> Synthetic Construct

<400> 149
ggtacgtatg tctcatctca ccc 23

<210> 150
<211> 18
<212> DNA
<213> Artificial Sequence

<220>
<223> Synthetic Construct

<400> 150
tgggatttgg gacccact 18

<210> 151
<211> 18
<212> DNA
<213> Artificial Sequence

<220>
<223> Synthetic Construct

<400> 151
cagcatctct ccgaaaac 18

<210>  152
<211>  18
<212>  DNA
<213>  Artificial Sequence

<220>
<223>  Synthetic Construct

<400>  152
ggctccacag cactggct    18

<210>  153
<211>  21
<212>  DNA
<213>  Artificial Sequence

<220>
<223>  Synthetic Construct

<400>  153
aaagtcaaag atgtggcagg a    21

<210>  154
<211>  20
<212>  DNA
<213>  Artificial Sequence

<220>
<223>  Synthetic Construct

<400>  154
gaagcaccgg atttagctct    20

<210>  155
<211>  20
<212>  DNA
<213>  Artificial Sequence

<220>
<223>  Synthetic Construct

<400>  155
tccatcccca ggataacact    20

<210>  156
<211>  22
<212>  DNA
<213>  Artificial Sequence

<220>
<223>  Synthetic Construct

<400>  156
gagtatggtt tgcatggtag ga    22

<210>  157
<211>  18
<212>  DNA

<213> Artificial Sequence

<220>
<223> Synthetic Construct

<400> 157
caatcttggg gacccctt 18

<210> 158
<211> 20
<212> DNA
<213> Artificial Sequence

<220>
<223> Synthetic Construct

<400> 158
tctctggaat ggagacctgc 20

<210> 159
<211> 18
<212> DNA
<213> Artificial Sequence

<220>
<223> Synthetic Construct

<400> 159
tggggatcat cagtctgt 18

<210> 160
<211> 18
<212> DNA
<213> Artificial Sequence

<220>
<223> Synthetic Construct

<400> 160
gagacgtgca gtcagggc 18

<210> 161
<211> 18
<212> DNA
<213> Artificial Sequence

<220>
<223> Synthetic Construct

<400> 161
aatgtcgcgt tcagggag 18

<210> 162
<211> 20
<212> DNA
<213> Artificial Sequence

<220>

<223> Synthetic Construct

<400> 162
tcccttaggt gccacaactt 20

<210> 163
<211> 18
<212> DNA
<213> Artificial Sequence

<220>
<223> Synthetic Construct

<400> 163
cctgctgtta gggtgcaa 18

<210> 164
<211> 18
<212> DNA
<213> Artificial Sequence

<220>
<223> Synthetic Construct

<400> 164
aggggcccac tgtgacta 18

<210> 165
<211> 18
<212> DNA
<213> Artificial Sequence

<220>
<223> Synthetic Construct

<400> 165
ggctgggcta gacttggg 18

<210> 166
<211> 18
<212> DNA
<213> Artificial Sequence

<220>
<223> Synthetic Construct

<400> 166
ccacggcaag tgagggag 18

<210> 167
<211> 18
<212> DNA
<213> Artificial Sequence

<220>
<223> Synthetic Construct

<400> 167

agggtccatg atgttggg 18

<210> 168
<211> 18
<212> DNA
<213> Artificial Sequence

<220>
<223> Synthetic Construct

<400> 168
ctggaggttc ctggtcct 18

<210> 169
<211> 20
<212> DNA
<213> Artificial Sequence

<220>
<223> Synthetic Construct

<400> 169
tgcccagcct gtgatatttt 20

<210> 170
<211> 18
<212> DNA
<213> Artificial Sequence

<220>
<223> Synthetic Construct

<400> 170
cttggcctaa gggattga 18

<210> 171
<211> 18
<212> DNA
<213> Artificial Sequence

<220>
<223> Synthetic Construct

<400> 171
acctcctggg tgagctgg 18

<210> 172
<211> 18
<212> DNA
<213> Artificial Sequence

<220>
<223> Synthetic Construct

<400> 172
ccaggccatc tggcatct 18

<210> 173
<211> 18
<212> DNA
<213> Artificial Sequence

<220>
<223> Synthetic Construct

<400> 173
cttggctaag tcggcccc 18

<210> 174
<211> 19
<212> DNA
<213> Artificial Sequence

<220>
<223> Synthetic Construct

<400> 174
tcctggcctc tagaaagga 19

<210> 175
<211> 19
<212> DNA
<213> Artificial Sequence

<220>
<223> Synthetic Construct

<400> 175
cctttaccgt ggaagcagg 19

<210> 176
<211> 20
<212> DNA
<213> Artificial Sequence

<220>
<223> Synthetic Construct

<400> 176
ccacccaggc tagaacttca 20

<210> 177
<211> 21
<212> DNA
<213> Artificial Sequence

<220>
<223> Synthetic Construct

<400> 177
gtgactagcc cagatcacca g 21

<210> 178
<211> 21
<212> DNA

<213> Artificial Sequence

<220>
<223> Synthetic Construct

<400> 178
tggaacacag taaatccgag a 21

<210> 179
<211> 18
<212> DNA
<213> Artificial Sequence

<220>
<223> Synthetic Construct

<400> 179
aggggcggca agagtcag 18

<210> 180
<211> 18
<212> DNA
<213> Artificial Sequence

<220>
<223> Synthetic Construct

<400> 180
taacttggcc tccccatc 18

<210> 181
<211> 18
<212> DNA
<213> Artificial Sequence

<220>
<223> Synthetic Construct

<400> 181
gccaggtagg ctcctttc 18

<210> 182
<211> 19
<212> DNA
<213> Artificial Sequence

<220>
<223> Synthetic Construct

<400> 182
gtacagcagc atgcgggat 19

<210> 183
<211> 18
<212> DNA
<213> Artificial Sequence

<220>

<223> Synthetic Construct

<400> 183
gggatgggca ccaaactc 18

<210> 184
<211> 18
<212> DNA
<213> Artificial Sequence

<220>
<223> Synthetic Construct

<400> 184
atgacacctg ccacttgc 18

<210> 185
<211> 19
<212> DNA
<213> Artificial Sequence

<220>
<223> Synthetic Construct

<400> 185
tctgatttgg gctttggaa 19

<210> 186
<211> 21
<212> DNA
<213> Artificial Sequence

<220>
<223> Synthetic Construct

<400> 186
tcagctacaa ctacatgagg a 21

<210> 187
<211> 20
<212> DNA
<213> Artificial Sequence

<220>
<223> Synthetic Construct

<400> 187
tagccaattt ggagctcact 20

<210> 188
<211> 21
<212> DNA
<213> Artificial Sequence

<220>
<223> Synthetic Construct

<400> 188

aggagagacg ttcgtatgca a 21

<210> 189
<211> 18
<212> DNA
<213> Artificial Sequence

<220>
<223> Synthetic Construct

<400> 189
cagacaaggg catcagtc 18

<210> 190
<211> 18
<212> DNA
<213> Artificial Sequence

<220>
<223> Synthetic Construct

<400> 190
ccctgaggga ttggcaag 18

<210> 191
<211> 18
<212> DNA
<213> Artificial Sequence

<220>
<223> Synthetic Construct

<400> 191
tgcaaatcac cttgttca 18

<210> 192
<211> 19
<212> DNA
<213> Artificial Sequence

<220>
<223> Synthetic Construct

<400> 192
tcaatcttca aatatgcca 19

<210> 193
<211> 24
<212> DNA
<213> Artificial Sequence

<220>
<223> Synthetic Construct

<400> 193
gttaaacaga agtagtgaga acag 24

<210> 194
<211> 18
<212> DNA
<213> Artificial Sequence

<220>
<223> Synthetic Construct

<400> 194
ccacagccag cctcctac 18

<210> 195
<211> 23
<212> DNA
<213> Artificial Sequence

<220>
<223> Synthetic Construct

<400> 195
gtttgagtta ctgtctaatg tcc 23

<210> 196
<211> 19
<212> DNA
<213> Artificial Sequence

<220>
<223> Synthetic Construct

<400> 196
cactagagga cctgccctg 19

<210> 197
<211> 18
<212> DNA
<213> Artificial Sequence

<220>
<223> Synthetic Construct

<400> 197
agcccagttt tctaggga 18

<210> 198
<211> 21
<212> DNA
<213> Artificial Sequence

<220>
<223> Synthetic Construct

<400> 198
agttagatcc taattcccaa a 21

<210> 199
<211> 18
<212> DNA

<213> Artificial Sequence

<220>
<223> Synthetic Construct

<400> 199
agagggagtg ggtgtcca 18

<210> 200
<211> 19
<212> DNA
<213> Artificial Sequence

<220>
<223> Synthetic Construct

<400> 200
cacagttcca ttcatggtc 19

<210> 201
<211> 20
<212> DNA
<213> Artificial Sequence

<220>
<223> Synthetic Construct

<400> 201
ggccttatcg taaatgtttt 20

<210> 202
<211> 20
<212> DNA
<213> Artificial Sequence

<220>
<223> Synthetic Construct

<400> 202
cttcatgttc tactgttccc 20

<210> 203
<211> 19
<212> DNA
<213> Artificial Sequence

<220>
<223> Synthetic Construct

<400> 203
cccttaagaa aggaggcta 19

<210> 204
<211> 18
<212> DNA
<213> Artificial Sequence

<220>

<223> Synthetic Construct

<400> 204
gatgccgacc aaaccctg 18

<210> 205
<211> 20
<212> DNA
<213> Artificial Sequence

<220>
<223> Synthetic Construct

<400> 205
gtgggaaaag ttagtgtcaa 20

<210> 206
<211> 20
<212> DNA
<213> Artificial Sequence

<220>
<223> Synthetic Construct

<400> 206
ttttcaactc tgtcacttcc 20

<210> 207
<211> 19
<212> DNA
<213> Artificial Sequence

<220>
<223> Synthetic Construct

<400> 207
cgttgaaccc gatttcctt 19

<210> 208
<211> 18
<212> DNA
<213> Artificial Sequence

<220>
<223> Synthetic Construct

<400> 208
acaaacgctg ctgaaccc 18

<210> 209
<211> 18
<212> DNA
<213> Artificial Sequence

<220>
<223> Synthetic Construct

<400> 209

cagcagagcc ctgacctc 18

<210> 210
<211> 18
<212> DNA
<213> Artificial Sequence

<220>
<223> Synthetic Construct

<400> 210
acctctgcga tgactgcc 18

<210> 211
<211> 18
<212> DNA
<213> Artificial Sequence

<220>
<223> Synthetic Construct

<400> 211
agtgggcaga ctctggaa 18

<210> 212
<211> 18
<212> DNA
<213> Artificial Sequence

<220>
<223> Synthetic Construct

<400> 212
tgcctctcag ccttccca 18

<210> 213
<211> 19
<212> DNA
<213> Artificial Sequence

<220>
<223> Synthetic Construct

<400> 213
cttggggtag gtggtgtcc 19

<210> 214
<211> 20
<212> DNA
<213> Artificial Sequence

<220>
<223> Synthetic Construct

<400> 214
ttatgccctg aagtggtttc 20

<210> 215
<211> 18
<212> DNA
<213> Artificial Sequence

<220>
<223> Synthetic Construct

<400> 215
acacccggga aggcttag 18

<210> 216
<211> 18
<212> DNA
<213> Artificial Sequence

<220>
<223> Synthetic Construct

<400> 216
ggatgtcagc ttggagcc 18

<210> 217
<211> 18
<212> DNA
<213> Artificial Sequence

<220>
<223> Synthetic Construct

<400> 217
ctgcacctcc cgctctct 18

<210> 218
<211> 18
<212> DNA
<213> Artificial Sequence

<220>
<223> Synthetic Construct

<400> 218
gagtccttcc tcccgcct 18

<210> 219
<211> 20
<212> DNA
<213> Artificial Sequence

<220>
<223> Synthetic Construct

<400> 219
tctgcccatc aatgacttct 20

<210> 220
<211> 20
<212> DNA

<213>  Artificial Sequence

<220>
<223>  Synthetic Construct

<400>  220
tgggcgggaa agtaaataga                                                   20

<210>  221
<211>  18
<212>  DNA
<213>  Artificial Sequence

<220>
<223>  Synthetic Construct

<400>  221
cagggtctca ggcagcgt                                                     18

<210>  222
<211>  20
<212>  DNA
<213>  Artificial Sequence

<220>
<223>  Synthetic Construct

<400>  222
gcatgaagat aaatcgcttg                                                   20

<210>  223
<211>  18
<212>  DNA
<213>  Artificial Sequence

<220>
<223>  Synthetic Construct

<400>  223
acatggagcc acggtcag                                                     18

<210>  224
<211>  19
<212>  DNA
<213>  Artificial Sequence

<220>
<223>  Synthetic Construct

<400>  224
tgtagccatg tgacccatc                                                    19

<210>  225
<211>  18
<212>  DNA
<213>  Artificial Sequence

<220>

<223> Synthetic Construct

<400> 225
ggggaattag ggcctgtg 18

<210> 226
<211> 18
<212> DNA
<213> Artificial Sequence

<220>
<223> Synthetic Construct

<400> 226
acccacagag tgcggcag 18

<210> 227
<211> 18
<212> DNA
<213> Artificial Sequence

<220>
<223> Synthetic Construct

<400> 227
gtgaacgctc actcactc 18

<210> 228
<211> 18
<212> DNA
<213> Artificial Sequence

<220>
<223> Synthetic Construct

<400> 228
ctccttggac ccaggcac 18

<210> 229
<211> 18
<212> DNA
<213> Artificial Sequence

<220>
<223> Synthetic Construct

<400> 229
gtggtatcag gggaacct 18

<210> 230
<211> 20
<212> DNA
<213> Artificial Sequence

<220>
<223> Synthetic Construct

<400> 230

cctgtggcat tttgtattta 20

<210> 231
<211> 19
<212> DNA
<213> Artificial Sequence

<220>
<223> Synthetic Construct

<400> 231
agagggtggg tctggtctg 19

<210> 232
<211> 19
<212> DNA
<213> Artificial Sequence

<220>
<223> Synthetic Construct

<400> 232
gtgatgtcat tcccgctgt 19

<210> 233
<211> 18
<212> DNA
<213> Artificial Sequence

<220>
<223> Synthetic Construct

<400> 233
cctgcaagtt tgtgtcct 18

<210> 234
<211> 22
<212> DNA
<213> Artificial Sequence

<220>
<223> Synthetic Construct

<400> 234
aatagagtag aatgaaagcc ac 22

<210> 235
<211> 20
<212> DNA
<213> Artificial Sequence

<220>
<223> Synthetic Construct

<400> 235
cttcttgtta aggctgaaag 20

<210> 236
<211> 18
<212> DNA
<213> Artificial Sequence

<220>
<223> Synthetic Construct

<400> 236
tccataggtt ggcagatg 18

<210> 237
<211> 18
<212> DNA
<213> Artificial Sequence

<220>
<223> Synthetic Construct

<400> 237
tgaatggcat cagcttag 18

<210> 238
<211> 20
<212> DNA
<213> Artificial Sequence

<220>
<223> Synthetic Construct

<400> 238
tattgaatcg gagtctgcac 20

<210> 239
<211> 18
<212> DNA
<213> Artificial Sequence

<220>
<223> Synthetic Construct

<400> 239
ggctgcactt cagattgg 18

<210> 240
<211> 18
<212> DNA
<213> Artificial Sequence

<220>
<223> Synthetic Construct

<400> 240
tggctagggt agggattc 18

<210> 241
<211> 19
<212> DNA

<213> Artificial Sequence

<220>
<223> Synthetic Construct

<400> 241
acctccaccc tcacctgaa 19

<210> 242
<211> 21
<212> DNA
<213> Artificial Sequence

<220>
<223> Synthetic Construct

<400> 242
ttagagcgct tctttacgga g 21

<210> 243
<211> 23
<212> DNA
<213> Artificial Sequence

<220>
<223> Synthetic Construct

<400> 243
gcctgatagg aacctattga tga 23

<210> 244
<211> 22
<212> DNA
<213> Artificial Sequence

<220>
<223> Synthetic Construct

<400> 244
ggactgaaga tctagacgga ta 22

<210> 245
<211> 18
<212> DNA
<213> Artificial Sequence

<220>
<223> Synthetic Construct

<400> 245
ttggctattg acaccaac 18

<210> 246
<211> 18
<212> DNA
<213> Artificial Sequence

<220>

<223> Synthetic Construct

<400> 246
ggatcagagc acgcagcc 18

<210> 247
<211> 18
<212> DNA
<213> Artificial Sequence

<220>
<223> Synthetic Construct

<400> 247
ctttgcgact gccctgat 18

<210> 248
<211> 20
<212> DNA
<213> Artificial Sequence

<220>
<223> Synthetic Construct

<400> 248
agccatatca cctttcgcat 20

<210> 249
<211> 21
<212> DNA
<213> Artificial Sequence

<220>
<223> Synthetic Construct

<400> 249
gtttaatgga attctgtttg g 21

<210> 250
<211> 20
<212> DNA
<213> Artificial Sequence

<220>
<223> Synthetic Construct

<400> 250
tgcataggct aaactggcaa 20

<210> 251
<211> 18
<212> DNA
<213> Artificial Sequence

<220>
<223> Synthetic Construct

<400> 251

cagcagtgca aggtcctg 18

<210> 252
<211> 18
<212> DNA
<213> Artificial Sequence

<220>
<223> Synthetic Construct

<400> 252
ccgtctctgt caggctgg 18

<210> 253
<211> 20
<212> DNA
<213> Artificial Sequence

<220>
<223> Synthetic Construct

<400> 253
agcagaacca cacgttcttt 20

<210> 254
<211> 20
<212> DNA
<213> Artificial Sequence

<220>
<223> Synthetic Construct

<400> 254
cagcagattt gcagggttta 20

<210> 255
<211> 18
<212> DNA
<213> Artificial Sequence

<220>
<223> Synthetic Construct

<400> 255
cctgttcctc ctgctccc 18

<210> 256
<211> 20
<212> DNA
<213> Artificial Sequence

<220>
<223> Synthetic Construct

<400> 256
agcaaatatt caccatgcag 20

<210> 257
<211> 18
<212> DNA
<213> Artificial Sequence

<220>
<223> Synthetic Construct

<400> 257
cctcagtagg tccctgcc 18

<210> 258
<211> 19
<212> DNA
<213> Artificial Sequence

<220>
<223> Synthetic Construct

<400> 258
cctcacaatt agaacagca 19

<210> 259
<211> 18
<212> DNA
<213> Artificial Sequence

<220>
<223> Synthetic Construct

<400> 259
ttgccacgag gatgaggt 18

<210> 260
<211> 20
<212> DNA
<213> Artificial Sequence

<220>
<223> Synthetic Construct

<400> 260
tttgaagacc agaggttggg 20

<210> 261
<211> 21
<212> DNA
<213> Artificial Sequence

<220>
<223> Synthetic Construct

<400> 261
tggattatca ttgttctggg c 21

<210> 262
<211> 18
<212> DNA

<213> Artificial Sequence

<220>
<223> Synthetic Construct

<400> 262
catttcagca gacagcat 18

<210> 263
<211> 18
<212> DNA
<213> Artificial Sequence

<220>
<223> Synthetic Construct

<400> 263
attctcagag gccaggga 18

<210> 264
<211> 18
<212> DNA
<213> Artificial Sequence

<220>
<223> Synthetic Construct

<400> 264
agcatgtgag cctctcgc 18

<210> 265
<211> 18
<212> DNA
<213> Artificial Sequence

<220>
<223> Synthetic Construct

<400> 265
tgctgggaca tcggggta 18

<210> 266
<211> 24
<212> DNA
<213> Artificial Sequence

<220>
<223> Synthetic Construct

<400> 266
caactgagaa aatagtgtta cttc 24

<210> 267
<211> 19
<212> DNA
<213> Artificial Sequence

<220>

<223> Synthetic Construct

<400> 267
tgttattgga gaaggcaga 19

<210> 268
<211> 21
<212> DNA
<213> Artificial Sequence

<220>
<223> Synthetic Construct

<400> 268
tgcctgtctt tctcttattt a 21

<210> 269
<211> 20
<212> DNA
<213> Artificial Sequence

<220>
<223> Synthetic Construct

<400> 269
aaataccacc tccgctcaca 20

<210> 270
<211> 21
<212> DNA
<213> Artificial Sequence

<220>
<223> Synthetic Construct

<400> 270
gcaggtgaac gttattatcc c 21

<210> 271
<211> 18
<212> DNA
<213> Artificial Sequence

<220>
<223> Synthetic Construct

<400> 271
agcttccctt tcgtgtct 18

<210> 272
<211> 18
<212> DNA
<213> Artificial Sequence

<220>
<223> Synthetic Construct

<400> 272

ctttcccaac aggccgct 18

<210> 273
<211> 18
<212> DNA
<213> Artificial Sequence

<220>
<223> Synthetic Construct

<400> 273
aaggagcccc tcggtgta 18

<210> 274
<211> 22
<212> DNA
<213> Artificial Sequence

<220>
<223> Synthetic Construct

<400> 274
gtcttggagt taggaaccac ca 22

<210> 275
<211> 20
<212> DNA
<213> Artificial Sequence

<220>
<223> Synthetic Construct

<400> 275
tatacagcgg ctcatcttcc 20

<210> 276
<211> 21
<212> DNA
<213> Artificial Sequence

<220>
<223> Synthetic Construct

<400> 276
acaatcataa ttgaggccac c 21

<210> 277
<211> 20
<212> DNA
<213> Artificial Sequence

<220>
<223> Synthetic Construct

<400> 277
agtgcttcat cttccctttc 20

<210> 278
<211> 19
<212> DNA
<213> Artificial Sequence

<220>
<223> Synthetic Construct

<400> 278
agccccacac acctgtctt 19

<210> 279
<211> 24
<212> DNA
<213> Artificial Sequence

<220>
<223> Synthetic Construct

<400> 279
ggatctgata gttgaggagt tctg 24

<210> 280
<211> 19
<212> DNA
<213> Artificial Sequence

<220>
<223> Synthetic Construct

<400> 280
aaggggatga ggatgggtc 19

<210> 281
<211> 20
<212> DNA
<213> Artificial Sequence

<220>
<223> Synthetic Construct

<400> 281
accctacccc ttattccctg 20

<210> 282
<211> 20
<212> DNA
<213> Artificial Sequence

<220>
<223> Synthetic Construct

<400> 282
caggactgct gtcccattac 20

<210> 283
<211> 19
<212> DNA

<213> Artificial Sequence

<220>
<223> Synthetic Construct

<400> 283
cttcccatgc ttgtcacat 19

<210> 284
<211> 19
<212> DNA
<213> Artificial Sequence

<220>
<223> Synthetic Construct

<400> 284
aggctcaata agatgatgg 19

<210> 285
<211> 19
<212> DNA
<213> Artificial Sequence

<220>
<223> Synthetic Construct

<400> 285
ccatcactcg gaacattgc 19

<210> 286
<211> 20
<212> DNA
<213> Artificial Sequence

<220>
<223> Synthetic Construct

<400> 286
tgtgagtccc tgcttgtgat 20

<210> 287
<211> 20
<212> DNA
<213> Artificial Sequence

<220>
<223> Synthetic Construct

<400> 287
ggccatgaat gggtagtcac 20

<210> 288
<211> 18
<212> DNA
<213> Artificial Sequence

<220>

<223> Synthetic Construct

<400> 288
ggtgggaaga cgcagttg 18

<210> 289
<211> 20
<212> DNA
<213> Artificial Sequence

<220>
<223> Synthetic Construct

<400> 289
attcaaccca ctatcatgga 20

<210> 290
<211> 20
<212> DNA
<213> Artificial Sequence

<220>
<223> Synthetic Construct

<400> 290
ttcatctttc ttccactccc 20

<210> 291
<211> 20
<212> DNA
<213> Artificial Sequence

<220>
<223> Synthetic Construct

<400> 291
tgcattagtt tgctgaattt 20

<210> 292
<211> 22
<212> DNA
<213> Artificial Sequence

<220>
<223> Synthetic Construct

<400> 292
tgtatagttt tgttcatctt gg 22

<210> 293
<211> 19
<212> DNA
<213> Artificial Sequence

<220>
<223> Synthetic Construct

<400> 293

tgcctcatac tttgtggat 19

<210> 294
<211> 22
<212> DNA
<213> Artificial Sequence

<220>
<223> Synthetic Construct

<400> 294
tgtgagaaaa ttgaggctta ga 22

<210> 295
<211> 19
<212> DNA
<213> Artificial Sequence

<220>
<223> Synthetic Construct

<400> 295
aaggttggag ggatattgg 19

<210> 296
<211> 18
<212> DNA
<213> Artificial Sequence

<220>
<223> Synthetic Construct

<400> 296
aggtgatgca gacagaaa 18

<210> 297
<211> 20
<212> DNA
<213> Artificial Sequence

<220>
<223> Synthetic Construct

<400> 297
tccagtaacc accagtctat 20

<210> 298
<211> 18
<212> DNA
<213> Artificial Sequence

<220>
<223> Synthetic Construct

<400> 298
agccaagcat ggataagc 18

<210> 299
<211> 18
<212> DNA
<213> Artificial Sequence

<220>
<223> Synthetic Construct

<400> 299
ccatggcgac tttcttcc 18

<210> 300
<211> 18
<212> DNA
<213> Artificial Sequence

<220>
<223> Synthetic Construct

<400> 300
aggttggcat caacaaag 18

<210> 301
<211> 20
<212> DNA
<213> Artificial Sequence

<220>
<223> Synthetic Construct

<400> 301
catacatgtg gatgtgaagc 20

<210> 302
<211> 21
<212> DNA
<213> Artificial Sequence

<220>
<223> Synthetic Construct

<400> 302
atggtggtca cataggtata a 21

<210> 303
<211> 21
<212> DNA
<213> Artificial Sequence

<220>
<223> Synthetic Construct

<400> 303
gaaacgctta gacttgcatc c 21

<210> 304
<211> 19
<212> DNA

<213>  Artificial Sequence

<220>
<223>  Synthetic Construct

<400>  304
gctagccaat ctgccgttc                                                    19

<210>  305
<211>  18
<212>  DNA
<213>  Artificial Sequence

<220>
<223>  Synthetic Construct

<400>  305
tgtgtagtgt ctgtgccc                                                     18

<210>  306
<211>  18
<212>  DNA
<213>  Artificial Sequence

<220>
<223>  Synthetic Construct

<400>  306
gtttccccac cctcaccc                                                     18

<210>  307
<211>  18
<212>  DNA
<213>  Artificial Sequence

<220>
<223>  Synthetic Construct

<400>  307
gtgccacttg aaatggtg                                                     18

<210>  308
<211>  20
<212>  DNA
<213>  Artificial Sequence

<220>
<223>  Synthetic Construct

<400>  308
gggctttaca ggatactgac                                                   20

<210>  309
<211>  18
<212>  DNA
<213>  Artificial Sequence

<220>

<223>  Synthetic Construct

<400>  309
ccatgcatcc tccctaga                                                      18

<210>  310
<211>  18
<212>  DNA
<213>  Artificial Sequence

<220>
<223>  Synthetic Construct

<400>  310
aatagcccaa aagaccaa                                                      18

<210>  311
<211>  18
<212>  DNA
<213>  Artificial Sequence

<220>
<223>  Synthetic Construct

<400>  311
aggaaattga agcaatgg                                                      18

<210>  312
<211>  20
<212>  DNA
<213>  Artificial Sequence

<220>
<223>  Synthetic Construct

<400>  312
gcacagtcat caatgacaca                                                    20

<210>  313
<211>  20
<212>  DNA
<213>  Artificial Sequence

<220>
<223>  Synthetic Construct

<400>  313
ttctgaaatg taggcattgt                                                    20

<210>  314
<211>  20
<212>  DNA
<213>  Artificial Sequence

<220>
<223>  Synthetic Construct

<400>  314

attcagcaga tttctattgc 20

<210> 315
<211> 21
<212> DNA
<213> Artificial Sequence

<220>
<223> Synthetic Construct

<400> 315
tgaagtattg gtgtgtgtgc c 21

<210> 316
<211> 23
<212> DNA
<213> Artificial Sequence

<220>
<223> Synthetic Construct

<400> 316
tggcattata gagttgatat cct 23

<210> 317
<211> 20
<212> DNA
<213> Artificial Sequence

<220>
<223> Synthetic Construct

<400> 317
gaccacaata tgctacccca 20

<210> 318
<211> 18
<212> DNA
<213> Artificial Sequence

<220>
<223> Synthetic Construct

<400> 318
gagccatggc acccaact 18

<210> 319
<211> 19
<212> DNA
<213> Artificial Sequence

<220>
<223> Synthetic Construct

<400> 319
ctcatgaaca gcaacaatc 19

<210> 320
<211> 20
<212> DNA
<213> Artificial Sequence

<220>
<223> Synthetic Construct

<400> 320
aaactgctca aagctgaata 20

<210> 321
<211> 20
<212> DNA
<213> Artificial Sequence

<220>
<223> Synthetic Construct

<400> 321
cattgttaat tcagttggct 20

<210> 322
<211> 20
<212> DNA
<213> Artificial Sequence

<220>
<223> Synthetic Construct

<400> 322
gcatatgcaa agcacattta 20

<210> 323
<211> 21
<212> DNA
<213> Artificial Sequence

<220>
<223> Synthetic Construct

<400> 323
tcttccgtta gagttgatac a 21

<210> 324
<211> 20
<212> DNA
<213> Artificial Sequence

<220>
<223> Synthetic Construct

<400> 324
tgcttgtagg ttctgtgcca 20

<210> 325
<211> 22
<212> DNA

<213>  Artificial Sequence

<220>
<223>  Synthetic Construct

<400>  325
ctctctcata cacacacaga ga                                              22

<210>  326
<211>  23
<212>  DNA
<213>  Artificial Sequence

<220>
<223>  Synthetic Construct

<400>  326
ctcaacatac tgtaatcaaa agg                                             23

<210>  327
<211>  18
<212>  DNA
<213>  Artificial Sequence

<220>
<223>  Synthetic Construct

<400>  327
ccctgggcat aggcaact                                                   18

<210>  328
<211>  19
<212>  DNA
<213>  Artificial Sequence

<220>
<223>  Synthetic Construct

<400>  328
ctgaaaacat ctagcccaa                                                  19

<210>  329
<211>  19
<212>  DNA
<213>  Artificial Sequence

<220>
<223>  Synthetic Construct

<400>  329
gagaggggat gtgcatttg                                                  19

<210>  330
<211>  20
<212>  DNA
<213>  Artificial Sequence

<220>

<223> Synthetic Construct

<400> 330
aagttcctcc aatggtccaa 20

<210> 331
<211> 21
<212> DNA
<213> Artificial Sequence

<220>
<223> Synthetic Construct

<400> 331
ggaacatctc tttagcattt g 21

<210> 332
<211> 18
<212> DNA
<213> Artificial Sequence

<220>
<223> Synthetic Construct

<400> 332
ggctccttag tgaacctg 18

<210> 333
<211> 20
<212> DNA
<213> Artificial Sequence

<220>
<223> Synthetic Construct

<400> 333
ccaacagtcc tcacttaggg 20

<210> 334
<211> 19
<212> DNA
<213> Artificial Sequence

<220>
<223> Synthetic Construct

<400> 334
aagctggagg cagattcgt 19

<210> 335
<211> 19
<212> DNA
<213> Artificial Sequence

<220>
<223> Synthetic Construct

<400> 335

tcctttctcc acatctgac 19

<210> 336
<211> 18
<212> DNA
<213> Artificial Sequence

<220>
<223> Synthetic Construct

<400> 336
aaaccgcttc tccacaca 18

<210> 337
<211> 19
<212> DNA
<213> Artificial Sequence

<220>
<223> Synthetic Construct

<400> 337
ccatggagtg gaatggaga 19

<210> 338
<211> 20
<212> DNA
<213> Artificial Sequence

<220>
<223> Synthetic Construct

<400> 338
tgaccttgac cttgtgacca 20

<210> 339
<211> 23
<212> DNA
<213> Artificial Sequence

<220>
<223> Synthetic Construct

<400> 339
gtgtaccttg tcattatgta agc 23

<210> 340
<211> 21
<212> DNA
<213> Artificial Sequence

<220>
<223> Synthetic Construct

<400> 340
tctcgacatg tacccttctc c 21

<210> 341
<211> 20
<212> DNA
<213> Artificial Sequence

<220>
<223> Synthetic Construct

<400> 341
gggtgtgggc attcactatc 20

<210> 342
<211> 22
<212> DNA
<213> Artificial Sequence

<220>
<223> Synthetic Construct

<400> 342
tcagtcctgc tatctacctc ca 22

<210> 343
<211> 18
<212> DNA
<213> Artificial Sequence

<220>
<223> Synthetic Construct

<400> 343
tgcccttcca ccttcagt 18

<210> 344
<211> 20
<212> DNA
<213> Artificial Sequence

<220>
<223> Synthetic Construct

<400> 344
ttgctagcta taccaccaac 20

<210> 345
<211> 21
<212> DNA
<213> Artificial Sequence

<220>
<223> Synthetic Construct

<400> 345
tgggaggtaa tgagatggag a 21

<210> 346
<211> 18
<212> DNA

<213> Artificial Sequence

<220>
<223> Synthetic Construct

<400> 346
acctgcgagc caggattc 18

<210> 347
<211> 22
<212> DNA
<213> Artificial Sequence

<220>
<223> Synthetic Construct

<400> 347
tggtgttttc actattagga tt 22

<210> 348
<211> 21
<212> DNA
<213> Artificial Sequence

<220>
<223> Synthetic Construct

<400> 348
cagacaacta aatacacgga a 21

<210> 349
<211> 21
<212> DNA
<213> Artificial Sequence

<220>
<223> Synthetic Construct

<400> 349
gggatgagtc cataagatga c 21

<210> 350
<211> 18
<212> DNA
<213> Artificial Sequence

<220>
<223> Synthetic Construct

<400> 350
ccagggatga cccatgtc 18

<210> 351
<211> 18
<212> DNA
<213> Artificial Sequence

<220>

<223> Synthetic Construct

<400> 351
gtctgctcta caggcggg 18

<210> 352
<211> 19
<212> DNA
<213> Artificial Sequence

<220>
<223> Synthetic Construct

<400> 352
ccaaccaact acccacacc 19

<210> 353
<211> 19
<212> DNA
<213> Artificial Sequence

<220>
<223> Synthetic Construct

<400> 353
gcaaccaatg aggggacct 19

<210> 354
<211> 18
<212> DNA
<213> Artificial Sequence

<220>
<223> Synthetic Construct

<400> 354
ccagtctaca gcccaaaa 18

<210> 355
<211> 19
<212> DNA
<213> Artificial Sequence

<220>
<223> Synthetic Construct

<400> 355
agataaggtg aagctgggg 19

<210> 356
<211> 18
<212> DNA
<213> Artificial Sequence

<220>
<223> Synthetic Construct

<400> 356

aggcattggc atgttggg 18

<210> 357
<211> 19
<212> DNA
<213> Artificial Sequence

<220>
<223> Synthetic Construct

<400> 357
gagcaggcag ctcacactc 19

<210> 358
<211> 20
<212> DNA
<213> Artificial Sequence

<220>
<223> Synthetic Construct

<400> 358
gaatccccat ggcatgatct 20

<210> 359
<211> 18
<212> DNA
<213> Artificial Sequence

<220>
<223> Synthetic Construct

<400> 359
atgggtactg gaggcagc 18

<210> 360
<211> 20
<212> DNA
<213> Artificial Sequence

<220>
<223> Synthetic Construct

<400> 360
caaaaggtca ggatcagtgc 20

<210> 361
<211> 18
<212> DNA
<213> Artificial Sequence

<220>
<223> Synthetic Construct

<400> 361
gtgcagttgg cagagggg 18

<210> 362
<211> 20
<212> DNA
<213> Artificial Sequence

<220>
<223> Synthetic Construct

<400> 362
agacttgggc tatgcctaca 20

<210> 363
<211> 20
<212> DNA
<213> Artificial Sequence

<220>
<223> Synthetic Construct

<400> 363
acgtcttatt gcaccttgcc 20

<210> 364
<211> 20
<212> DNA
<213> Artificial Sequence

<220>
<223> Synthetic Construct

<400> 364
tggacagtcg ttatcccttg 20

<210> 365
<211> 19
<212> DNA
<213> Artificial Sequence

<220>
<223> Synthetic Construct

<400> 365
gagtatggac tgagggctt 19

<210> 366
<211> 19
<212> DNA
<213> Artificial Sequence

<220>
<223> Synthetic Construct

<400> 366
tcaaccacct gtcagagcc 19

<210> 367
<211> 20
<212> DNA

<213> Artificial Sequence

<220>
<223> Synthetic Construct

<400> 367
ttgtaccgag ttaccttgag 20

<210> 368
<211> 18
<212> DNA
<213> Artificial Sequence

<220>
<223> Synthetic Construct

<400> 368
tgaagcaacc aaaggtgg 18

<210> 369
<211> 20
<212> DNA
<213> Artificial Sequence

<220>
<223> Synthetic Construct

<400> 369
aagtactagg ctcttgctcc 20

<210> 370
<211> 20
<212> DNA
<213> Artificial Sequence

<220>
<223> Synthetic Construct

<400> 370
tgtggtttat gctacctgag 20

<210> 371
<211> 18
<212> DNA
<213> Artificial Sequence

<220>
<223> Synthetic Construct

<400> 371
caaggaacct ggtcccca 18

<210> 372
<211> 18
<212> DNA
<213> Artificial Sequence

<220>

<223> Synthetic Construct

<400> 372
accatggaga tggcatta 18

<210> 373
<211> 20
<212> DNA
<213> Artificial Sequence

<220>
<223> Synthetic Construct

<400> 373
aaccaagtca acctcaatca 20

<210> 374
<211> 20
<212> DNA
<213> Artificial Sequence

<220>
<223> Synthetic Construct

<400> 374
gattgattca gtgaccccaa 20

<210> 375
<211> 21
<212> DNA
<213> Artificial Sequence

<220>
<223> Synthetic Construct

<400> 375
gacaagtctc tgttctcaag g 21

<210> 376
<211> 18
<212> DNA
<213> Artificial Sequence

<220>
<223> Synthetic Construct

<400> 376
acttggcacc acctaaat 18

<210> 377
<211> 18
<212> DNA
<213> Artificial Sequence

<220>
<223> Synthetic Construct

<400> 377

tcacacgtga atgggcct 18

<210> 378
<211> 18
<212> DNA
<213> Artificial Sequence

<220>
<223> Synthetic Construct

<400> 378
ttcgcactca cagggcaa 18

<210> 379
<211> 19
<212> DNA
<213> Artificial Sequence

<220>
<223> Synthetic Construct

<400> 379
tgggtattga gatccaagg 19

<210> 380
<211> 20
<212> DNA
<213> Artificial Sequence

<220>
<223> Synthetic Construct

<400> 380
tctatcacct accacctgcc 20

<210> 381
<211> 18
<212> DNA
<213> Artificial Sequence

<220>
<223> Synthetic Construct

<400> 381
tgagccgttg ggtctatt 18

<210> 382
<211> 19
<212> DNA
<213> Artificial Sequence

<220>
<223> Synthetic Construct

<400> 382
ctagggctct gtgaagttt 19

<210> 383
<211> 19
<212> DNA
<213> Artificial Sequence

<220>
<223> Synthetic Construct

<400> 383
agaggtgaca tgagggaca 19

<210> 384
<211> 20
<212> DNA
<213> Artificial Sequence

<220>
<223> Synthetic Construct

<400> 384
atcgccatct gaccttcctt 20

<210> 385
<211> 19
<212> DNA
<213> Artificial Sequence

<220>
<223> Synthetic Construct

<400> 385
tgcattccca attacaagc 19

<210> 386
<211> 18
<212> DNA
<213> Artificial Sequence

<220>
<223> Synthetic Construct

<400> 386
gccaggggac actcacat 18

<210> 387
<211> 18
<212> DNA
<213> Artificial Sequence

<220>
<223> Synthetic Construct

<400> 387
tggcctgtag catgggtt 18

<210> 388
<211> 19
<212> DNA

<213> Artificial Sequence

<220>
<223> Synthetic Construct

<400> 388
agttgcccag tgttgctca 19

<210> 389
<211> 19
<212> DNA
<213> Artificial Sequence

<220>
<223> Synthetic Construct

<400> 389
aggacaagaa agggtgtca 19

<210> 390
<211> 18
<212> DNA
<213> Artificial Sequence

<220>
<223> Synthetic Construct

<400> 390
atggtagtca gggttcca 18

<210> 391
<211> 19
<212> DNA
<213> Artificial Sequence

<220>
<223> Synthetic Construct

<400> 391
ggaaaatcca atcttgaca 19

<210> 392
<211> 21
<212> DNA
<213> Artificial Sequence

<220>
<223> Synthetic Construct

<400> 392
ttttatagtg tcccaccttt t 21

<210> 393
<211> 19
<212> DNA
<213> Artificial Sequence

<220>

<223> Synthetic Construct

<400> 393
ttgaaacaag gcatgaata 19

<210> 394
<211> 20
<212> DNA
<213> Artificial Sequence

<220>
<223> Synthetic Construct

<400> 394
gagagagatt ccaagccagt 20

<210> 395
<211> 19
<212> DNA
<213> Artificial Sequence

<220>
<223> Synthetic Construct

<400> 395
gcatgcattt cataccatt 19

<210> 396
<211> 23
<212> DNA
<213> Artificial Sequence

<220>
<223> Synthetic Construct

<400> 396
aagtcataga agaaaatagc tca 23

<210> 397
<211> 18
<212> DNA
<213> Artificial Sequence

<220>
<223> Synthetic Construct

<400> 397
aaaggttcct gttcttgc 18

<210> 398
<211> 23
<212> DNA
<213> Artificial Sequence

<220>
<223> Synthetic Construct

<400> 398

cacaataaga ccgtatacat ttg 23

<210> 399
<211> 20
<212> DNA
<213> Artificial Sequence

<220>
<223> Synthetic Construct

<400> 399
gggtgatgag ctagagagtg 20

<210> 400
<211> 20
<212> DNA
<213> Artificial Sequence

<220>
<223> Synthetic Construct

<400> 400
aggctacgct caaatgtcac 20

<210> 401
<211> 20
<212> DNA
<213> Artificial Sequence

<220>
<223> Synthetic Construct

<400> 401
atgacccatt ggtaggtcca 20

<210> 402
<211> 19
<212> DNA
<213> Artificial Sequence

<220>
<223> Synthetic Construct

<400> 402
catggtaatg gccaaggaa 19

<210> 403
<211> 19
<212> DNA
<213> Artificial Sequence

<220>
<223> Synthetic Construct

<400> 403
tggagcttat tccagtctc 19

<210> 404
<211> 22
<212> DNA
<213> Artificial Sequence

<220>
<223> Synthetic Construct

<400> 404
ccatttactt acctatcgtg tc 22

<210> 405
<211> 20
<212> DNA
<213> Artificial Sequence

<220>
<223> Synthetic Construct

<400> 405
ggtacaagga gccgtgtcag 20

<210> 406
<211> 19
<212> DNA
<213> Artificial Sequence

<220>
<223> Synthetic Construct

<400> 406
ccagagtcca ttcccaaca 19

<210> 407
<211> 18
<212> DNA
<213> Artificial Sequence

<220>
<223> Synthetic Construct

<400> 407
aagacggccg agcttcac 18

<210> 408
<211> 21
<212> DNA
<213> Artificial Sequence

<220>
<223> Synthetic Construct

<400> 408
tgtaatcaag tgaccaaaag g 21

<210> 409
<211> 20
<212> DNA

<213> Artificial Sequence

<220>
<223> Synthetic Construct

<400> 409
cacaacaagt gtgtgcatga 20

<210> 410
<211> 18
<212> DNA
<213> Artificial Sequence

<220>
<223> Synthetic Construct

<400> 410
tctacgcact gttccgca 18

<210> 411
<211> 20
<212> DNA
<213> Artificial Sequence

<220>
<223> Synthetic Construct

<400> 411
accaggggct atgtgctatt 20

<210> 412
<211> 21
<212> DNA
<213> Artificial Sequence

<220>
<223> Synthetic Construct

<400> 412
gcagtcagta tagtttggtg c 21

<210> 413
<211> 18
<212> DNA
<213> Artificial Sequence

<220>
<223> Synthetic Construct

<400> 413
ccaccatctg ggccatac 18

<210> 414
<211> 20
<212> DNA
<213> Artificial Sequence

<220>

<223> Synthetic Construct

<400> 414
agattgtgtg aggctgccat 20

<210> 415
<211> 21
<212> DNA
<213> Artificial Sequence

<220>
<223> Synthetic Construct

<400> 415
aagatccaat gcaacttgaa a 21

<210> 416
<211> 20
<212> DNA
<213> Artificial Sequence

<220>
<223> Synthetic Construct

<400> 416
ctgtgtccac tgtccactcc 20

<210> 417
<211> 18
<212> DNA
<213> Artificial Sequence

<220>
<223> Synthetic Construct

<400> 417
tggtgcctgg cagctttc 18

<210> 418
<211> 18
<212> DNA
<213> Artificial Sequence

<220>
<223> Synthetic Construct

<400> 418
ttgttgggag gaccggat 18

<210> 419
<211> 18
<212> DNA
<213> Artificial Sequence

<220>
<223> Synthetic Construct

<400> 419

ggcaaaggag ttggcctt 18

<210> 420
<211> 19
<212> DNA
<213> Artificial Sequence

<220>
<223> Synthetic Construct

<400> 420
gggacacctc ctgctgact 19

<210> 421
<211> 18
<212> DNA
<213> Artificial Sequence

<220>
<223> Synthetic Construct

<400> 421
tgaagcccac tcagacat 18

<210> 422
<211> 20
<212> DNA
<213> Artificial Sequence

<220>
<223> Synthetic Construct

<400> 422
cttcccatcc acggtagaga 20

<210> 423
<211> 21
<212> DNA
<213> Artificial Sequence

<220>
<223> Synthetic Construct

<400> 423
gcaagtccta aagcaatagc c 21

<210> 424
<211> 20
<212> DNA
<213> Artificial Sequence

<220>
<223> Synthetic Construct

<400> 424
ctttaagctc aaggcttggt 20

<210> 425
<211> 19
<212> DNA
<213> Artificial Sequence

<220>
<223> Synthetic Construct

<400> 425
tgcagtgatg agttttgaa 19

<210> 426
<211> 23
<212> DNA
<213> Artificial Sequence

<220>
<223> Synthetic Construct

<400> 426
ttaaattgct atctgtatag ccc 23

<210> 427
<211> 18
<212> DNA
<213> Artificial Sequence

<220>
<223> Synthetic Construct

<400> 427
tgctaaatga gtcccctt 18

<210> 428
<211> 20
<212> DNA
<213> Artificial Sequence

<220>
<223> Synthetic Construct

<400> 428
gcagatgtct ttgagaatca 20

<210> 429
<211> 20
<212> DNA
<213> Artificial Sequence

<220>
<223> Synthetic Construct

<400> 429
gtatgtcggg gcacagaagg 20

<210> 430
<211> 21
<212> DNA

<213> Artificial Sequence

<220>
<223> Synthetic Construct

<400> 430
aaaggcaata gcacagggta a 21

<210> 431
<211> 18
<212> DNA
<213> Artificial Sequence

<220>
<223> Synthetic Construct

<400> 431
tgccctctag accattga 18

<210> 432
<211> 20
<212> DNA
<213> Artificial Sequence

<220>
<223> Synthetic Construct

<400> 432
tatggaaacc agcaattgag 20

<210> 433
<211> 23
<212> DNA
<213> Artificial Sequence

<220>
<223> Synthetic Construct

<400> 433
catgtctagg atccatcaat gtg 23

<210> 434
<211> 20
<212> DNA
<213> Artificial Sequence

<220>
<223> Synthetic Construct

<400> 434
tgatagatgc ggctctgttc 20

<210> 435
<211> 21
<212> DNA
<213> Artificial Sequence

<220>

<223> Synthetic Construct

<400> 435
tgccactaat cattatacgt t 21

<210> 436
<211> 23
<212> DNA
<213> Artificial Sequence

<220>
<223> Synthetic Construct

<400> 436
acttaactaa atgacctgaa aga 23

<210> 437
<211> 21
<212> DNA
<213> Artificial Sequence

<220>
<223> Synthetic Construct

<400> 437
tgttttcaga acaagacaag a 21

<210> 438
<211> 18
<212> DNA
<213> Artificial Sequence

<220>
<223> Synthetic Construct

<400> 438
acagctaagg cagcttct 18

<210> 439
<211> 20
<212> DNA
<213> Artificial Sequence

<220>
<223> Synthetic Construct

<400> 439
tgggtgaaga ctgtgaagca 20

<210> 440
<211> 18
<212> DNA
<213> Artificial Sequence

<220>
<223> Synthetic Construct

<400> 440

aaaagggccc ctcacagc 18

<210> 441
<211> 20
<212> DNA
<213> Artificial Sequence

<220>
<223> Synthetic Construct

<400> 441
gagtgcacag agtgctagat 20

<210> 442
<211> 20
<212> DNA
<213> Artificial Sequence

<220>
<223> Synthetic Construct

<400> 442
gaactggacc agagaaactg 20

<210> 443
<211> 20
<212> DNA
<213> Artificial Sequence

<220>
<223> Synthetic Construct

<400> 443
acctgactgt tgaacacact 20

<210> 444
<211> 19
<212> DNA
<213> Artificial Sequence

<220>
<223> Synthetic Construct

<400> 444
caccattgga tttagcaac 19

<210> 445
<211> 21
<212> DNA
<213> Artificial Sequence

<220>
<223> Synthetic Construct

<400> 445
ttctttaaat ctgcctttgg a 21

<210> 446
<211> 20
<212> DNA
<213> Artificial Sequence

<220>
<223> Synthetic Construct

<400> 446
aaggtaggag gaagggaata 20

<210> 447
<211> 18
<212> DNA
<213> Artificial Sequence

<220>
<223> Synthetic Construct

<400> 447
ttcgggtaca ggagccta 18

<210> 448
<211> 18
<212> DNA
<213> Artificial Sequence

<220>
<223> Synthetic Construct

<400> 448
cagaagacct cgttgcac 18

<210> 449
<211> 23
<212> DNA
<213> Artificial Sequence

<220>
<223> Synthetic Construct

<400> 449
gcttaaattt caaccagtag ttt 23

<210> 450
<211> 18
<212> DNA
<213> Artificial Sequence

<220>
<223> Synthetic Construct

<400> 450
ggtgggaatt aagggtta 18

<210> 451
<211> 18
<212> DNA

<213> Artificial Sequence

<220>
<223> Synthetic Construct

<400> 451
ctgtgcttgc ttggattt 18

<210> 452
<211> 18
<212> DNA
<213> Artificial Sequence

<220>
<223> Synthetic Construct

<400> 452
cacaagcagg tccatgag 18

<210> 453
<211> 19
<212> DNA
<213> Artificial Sequence

<220>
<223> Synthetic Construct

<400> 453
gccaatattc tcaagttcc 19

<210> 454
<211> 19
<212> DNA
<213> Artificial Sequence

<220>
<223> Synthetic Construct

<400> 454
ctgtccctga tctttggca 19

<210> 455
<211> 24
<212> DNA
<213> Artificial Sequence

<220>
<223> Synthetic Construct

<400> 455
cacacagttt atttgtaaag tagc 24

<210> 456
<211> 18
<212> DNA
<213> Artificial Sequence

<220>

<223> Synthetic Construct

<400> 456
caagaggcaa gggcagag 18

<210> 457
<211> 19
<212> DNA
<213> Artificial Sequence

<220>
<223> Synthetic Construct

<400> 457
gtgtcgggca cagtgctaa 19

<210> 458
<211> 20
<212> DNA
<213> Artificial Sequence

<220>
<223> Synthetic Construct

<400> 458
gtccacaagt ttggtaacct 20

<210> 459
<211> 21
<212> DNA
<213> Artificial Sequence

<220>
<223> Synthetic Construct

<400> 459
attactcaga ggcagaaatc a 21

<210> 460
<211> 18
<212> DNA
<213> Artificial Sequence

<220>
<223> Synthetic Construct

<400> 460
gcaatctcgg tcatcaga 18

<210> 461
<211> 18
<212> DNA
<213> Artificial Sequence

<220>
<223> Synthetic Construct

<400> 461

catcaagaca ggctccga 18

<210> 462
<211> 18
<212> DNA
<213> Artificial Sequence

<220>
<223> Synthetic Construct

<400> 462
cttcagggtc tggatggc 18

<210> 463
<211> 18
<212> DNA
<213> Artificial Sequence

<220>
<223> Synthetic Construct

<400> 463
ggcccaggct ctcaaaac 18

<210> 464
<211> 18
<212> DNA
<213> Artificial Sequence

<220>
<223> Synthetic Construct

<400> 464
gatctggttg ctgcccat 18

<210> 465
<211> 18
<212> DNA
<213> Artificial Sequence

<220>
<223> Synthetic Construct

<400> 465
ggagccgttg cggtagat 18

<210> 466
<211> 22
<212> DNA
<213> Artificial Sequence

<220>
<223> Synthetic Construct

<400> 466
gagtacctca agtgcctgac cc 22

<210> 467
<211> 19
<212> DNA
<213> Artificial Sequence

<220>
<223> Synthetic Construct

<400> 467
tggtacttgg tgctgacgg 19

<210> 468
<211> 19
<212> DNA
<213> Artificial Sequence

<220>
<223> Synthetic Construct

<400> 468
ggagtacgac ctgtcgctg 19

<210> 469
<211> 20
<212> DNA
<213> Artificial Sequence

<220>
<223> Synthetic Construct

<400> 469
tcctggcaat cttatcttga 20

<210> 470
<211> 22
<212> DNA
<213> Artificial Sequence

<220>
<223> Synthetic Construct

<400> 470
cttaggtgaa aattgatacc cc 22

<210> 471
<211> 18
<212> DNA
<213> Artificial Sequence

<220>
<223> Synthetic Construct

<400> 471
tcatgttgca ccagagag 18

<210> 472
<211> 18
<212> DNA

<213> Artificial Sequence

<220>
<223> Synthetic Construct

<400> 472
gctcaggatg gacaactg 18

<210> 473
<211> 18
<212> DNA
<213> Artificial Sequence

<220>
<223> Synthetic Construct

<400> 473
gcacatgcac agtgggga 18

<210> 474
<211> 19
<212> DNA
<213> Artificial Sequence

<220>
<223> Synthetic Construct

<400> 474
caccccaacc ttttattgc 19

<210> 475
<211> 19
<212> DNA
<213> Artificial Sequence

<220>
<223> Synthetic Construct

<400> 475
gctgagtgtg agtcccctt 19

<210> 476
<211> 20
<212> DNA
<213> Artificial Sequence

<220>
<223> Synthetic Construct

<400> 476
agcctagcct gaacacacct 20

<210> 477
<211> 18
<212> DNA
<213> Artificial Sequence

<220>

<223> Synthetic Construct

<400> 477
agctgcttct tcacgggc 18

<210> 478
<211> 22
<212> DNA
<213> Artificial Sequence

<220>
<223> Synthetic Construct

<400> 478
cagagaagct agcaccttca ga 22

<210> 479
<211> 20
<212> DNA
<213> Artificial Sequence

<220>
<223> Synthetic Construct

<400> 479
aaattgtgct aactggtgcc 20

<210> 480
<211> 21
<212> DNA
<213> Artificial Sequence

<220>
<223> Synthetic Construct

<400> 480
aaagcagcta ccatgaccat t 21

<210> 481
<211> 20
<212> DNA
<213> Artificial Sequence

<220>
<223> Synthetic Construct

<400> 481
tgctaacatt cgagtgagtt 20

<210> 482
<211> 18
<212> DNA
<213> Artificial Sequence

<220>
<223> Synthetic Construct

<400> 482

gcagattaca atggcaga 18

<210> 483
<211> 19
<212> DNA
<213> Artificial Sequence

<220>
<223> Synthetic Construct

<400> 483
agggcctctt cctgtcctg 19

<210> 484
<211> 23
<212> DNA
<213> Artificial Sequence

<220>
<223> Synthetic Construct

<400> 484
atgagaataa actaaacaca ccc 23

<210> 485
<211> 18
<212> DNA
<213> Artificial Sequence

<220>
<223> Synthetic Construct

<400> 485
gctgatgagt ccaaaagc 18

<210> 486
<211> 19
<212> DNA
<213> Artificial Sequence

<220>
<223> Synthetic Construct

<400> 486
tccatggaga ttgaagcat 19

<210> 487
<211> 18
<212> DNA
<213> Artificial Sequence

<220>
<223> Synthetic Construct

<400> 487
aaggagaagg ggcaacct 18

<210> 488
<211> 19
<212> DNA
<213> Artificial Sequence

<220>
<223> Synthetic Construct

<400> 488
ggtgctcata cagccaatc 19

<210> 489
<211> 18
<212> DNA
<213> Artificial Sequence

<220>
<223> Synthetic Construct

<400> 489
gggccaaacc caacccta 18

<210> 490
<211> 19
<212> DNA
<213> Artificial Sequence

<220>
<223> Synthetic Construct

<400> 490
gatcactgat ggacgagcc 19

<210> 491
<211> 18
<212> DNA
<213> Artificial Sequence

<220>
<223> Synthetic Construct

<400> 491
atgttgcact caaacggg 18

<210> 492
<211> 21
<212> DNA
<213> Artificial Sequence

<220>
<223> Synthetic Construct

<400> 492
aaatgggtta agctgtaagt t 21

<210> 493
<211> 19
<212> DNA

<213>  Artificial Sequence

<220>
<223>  Synthetic Construct

<400>  493
gtctggagtc ttcagcttg                                                    19

<210>  494
<211>  19
<212>  DNA
<213>  Artificial Sequence

<220>
<223>  Synthetic Construct

<400>  494
agaactgttg gatgacacc                                                    19

<210>  495
<211>  22
<212>  DNA
<213>  Artificial Sequence

<220>
<223>  Synthetic Construct

<400>  495
ttaaccctac tctttaaatt gg                                                22

<210>  496
<211>  18
<212>  DNA
<213>  Artificial Sequence

<220>
<223>  Synthetic Construct

<400>  496
acttgtgact ttgctgcc                                                     18

<210>  497
<211>  18
<212>  DNA
<213>  Artificial Sequence

<220>
<223>  Synthetic Construct

<400>  497
aaaccctgat ttgagcca                                                     18

<210>  498
<211>  18
<212>  DNA
<213>  Artificial Sequence

<220>

<223> Synthetic Construct

<400> 498
acctgagggg aatgtcca 18

<210> 499
<211> 20
<212> DNA
<213> Artificial Sequence

<220>
<223> Synthetic Construct

<400> 499
tcagtgcgcg aaatgtagtt 20

<210> 500
<211> 18
<212> DNA
<213> Artificial Sequence

<220>
<223> Synthetic Construct

<400> 500
agctgtccac ctggtgct 18

<210> 501
<211> 20
<212> DNA
<213> Artificial Sequence

<220>
<223> Synthetic Construct

<400> 501
ggtgcccatg gagactagag 20

<210> 502
<211> 18
<212> DNA
<213> Artificial Sequence

<220>
<223> Synthetic Construct

<400> 502
gcaatctcct cgcctctg 18

<210> 503
<211> 19
<212> DNA
<213> Artificial Sequence

<220>
<223> Synthetic Construct

<400> 503

cctgttttcc tgccttcct 19

<210> 504
<211> 18
<212> DNA
<213> Artificial Sequence

<220>
<223> Synthetic Construct

<400> 504
atctgtctcc ctgaccaa 18

<210> 505
<211> 21
<212> DNA
<213> Artificial Sequence

<220>
<223> Synthetic Construct

<400> 505
accctataag aaaaccccat a 21

<210> 506
<211> 18
<212> DNA
<213> Artificial Sequence

<220>
<223> Synthetic Construct

<400> 506
gggtagtggg agacggga 18

<210> 507
<211> 19
<212> DNA
<213> Artificial Sequence

<220>
<223> Synthetic Construct

<400> 507
tgtcccatag gcaccacaa 19

<210> 508
<211> 20
<212> DNA
<213> Artificial Sequence

<220>
<223> Synthetic Construct

<400> 508
tgccaagaag gacaacaacc 20

<210> 509
<211> 18
<212> DNA
<213> Artificial Sequence

<220>
<223> Synthetic Construct

<400> 509
ccccttaggtc ctgtggca 18

<210> 510
<211> 19
<212> DNA
<213> Artificial Sequence

<220>
<223> Synthetic Construct

<400> 510
ttttgtttgg agccaggtt 19

<210> 511
<211> 19
<212> DNA
<213> Artificial Sequence

<220>
<223> Synthetic Construct

<400> 511
caatgcattt aagcaatga 19

<210> 512
<211> 18
<212> DNA
<213> Artificial Sequence

<220>
<223> Synthetic Construct

<400> 512
atctttgggt cacaatcc 18

<210> 513
<211> 23
<212> DNA
<213> Artificial Sequence

<220>
<223> Synthetic Construct

<400> 513
cccacaagaa tgataaagat aga 23

<210> 514
<211> 23
<212> DNA

<213> Artificial Sequence

<220>
<223> Synthetic Construct

<400> 514
aaagtgattt gatttgacca tcc 23

<210> 515
<211> 18
<212> DNA
<213> Artificial Sequence

<220>
<223> Synthetic Construct

<400> 515
gatttggcct ggggatgg 18

<210> 516
<211> 21
<212> DNA
<213> Artificial Sequence

<220>
<223> Synthetic Construct

<400> 516
gtgactcact caccttgctc t 21

<210> 517
<211> 18
<212> DNA
<213> Artificial Sequence

<220>
<223> Synthetic Construct

<400> 517
aggcatttgg caaccctg 18

<210> 518
<211> 18
<212> DNA
<213> Artificial Sequence

<220>
<223> Synthetic Construct

<400> 518
aggtactgcc agtggtcc 18

<210> 519
<211> 20
<212> DNA
<213> Artificial Sequence

<220>

<223> Synthetic Construct

<400> 519
agtatctgca aggccaacaa 20

<210> 520
<211> 20
<212> DNA
<213> Artificial Sequence

<220>
<223> Synthetic Construct

<400> 520
gtggtctgct agtatgtgct 20

<210> 521
<211> 23
<212> DNA
<213> Artificial Sequence

<220>
<223> Synthetic Construct

<400> 521
caaactaaat catgatactc tgc 23

<210> 522
<211> 20
<212> DNA
<213> Artificial Sequence

<220>
<223> Synthetic Construct

<400> 522
tgccataggt tttatgactc 20

<210> 523
<211> 18
<212> DNA
<213> Artificial Sequence

<220>
<223> Synthetic Construct

<400> 523
tgcacctctg ctattgct 18

<210> 524
<211> 19
<212> DNA
<213> Artificial Sequence

<220>
<223> Synthetic Construct

<400> 524

tctccatgga ctaatgagc 19

<210> 525
<211> 20
<212> DNA
<213> Artificial Sequence

<220>
<223> Synthetic Construct

<400> 525
gtggctctgt ggctccatgt 20

<210> 526
<211> 18
<212> DNA
<213> Artificial Sequence

<220>
<223> Synthetic Construct

<400> 526
atccaatgac tggcatct 18

<210> 527
<211> 21
<212> DNA
<213> Artificial Sequence

<220>
<223> Synthetic Construct

<400> 527
gagaatgaag caaaaggtaa t 21

<210> 528
<211> 18
<212> DNA
<213> Artificial Sequence

<220>
<223> Synthetic Construct

<400> 528
cttcttcatc cacccaaa 18

<210> 529
<211> 20
<212> DNA
<213> Artificial Sequence

<220>
<223> Synthetic Construct

<400> 529
caaacttccc agtcattaaa 20

<210> 530
<211> 19
<212> DNA
<213> Artificial Sequence

<220>
<223> Synthetic Construct

<400> 530
ccccaaggca aagtatcca 19

<210> 531
<211> 21
<212> DNA
<213> Artificial Sequence

<220>
<223> Synthetic Construct

<400> 531
ccatcaccaa tacttaagac a 21

<210> 532
<211> 19
<212> DNA
<213> Artificial Sequence

<220>
<223> Synthetic Construct

<400> 532
tcttggagct gatacctct 19

<210> 533
<211> 19
<212> DNA
<213> Artificial Sequence

<220>
<223> Synthetic Construct

<400> 533
gcagtagttc aagagcaca 19

<210> 534
<211> 18
<212> DNA
<213> Artificial Sequence

<220>
<223> Synthetic Construct

<400> 534
tggtcctcca ggtagctc 18

<210> 535
<211> 18
<212> DNA

<213>  Artificial Sequence

<220>
<223>  Synthetic Construct

<400>  535
tctgcttctt cacttggg                                                    18

<210>  536
<211>  20
<212>  DNA
<213>  Artificial Sequence

<220>
<223>  Synthetic Construct

<400>  536
caaatagcag catgtagtgc                                                  20

<210>  537
<211>  20
<212>  DNA
<213>  Artificial Sequence

<220>
<223>  Synthetic Construct

<400>  537
cacacctaca cacacttgct                                                  20

<210>  538
<211>  18
<212>  DNA
<213>  Artificial Sequence

<220>
<223>  Synthetic Construct

<400>  538
ccaaagctct gtccctgc                                                    18

<210>  539
<211>  19
<212>  DNA
<213>  Artificial Sequence

<220>
<223>  Synthetic Construct

<400>  539
ttacctttgc ctaaaacca                                                   19

<210>  540
<211>  19
<212>  DNA
<213>  Artificial Sequence

<220>

<223> Synthetic Construct

<400> 540
gcaataccca actaatccc 19

<210> 541
<211> 20
<212> DNA
<213> Artificial Sequence

<220>
<223> Synthetic Construct

<400> 541
aaaagttcag gagccaccag 20

<210> 542
<211> 18
<212> DNA
<213> Artificial Sequence

<220>
<223> Synthetic Construct

<400> 542
ggtgcattgc cacgtctt 18

<210> 543
<211> 22
<212> DNA
<213> Artificial Sequence

<220>
<223> Synthetic Construct

<400> 543
atgtcctatt cattcatacc tc 22

<210> 544
<211> 23
<212> DNA
<213> Artificial Sequence

<220>
<223> Synthetic Construct

<400> 544
atgggtaaaa tacaatgggt aag 23

<210> 545
<211> 21
<212> DNA
<213> Artificial Sequence

<220>
<223> Synthetic Construct

<400> 545

tctaagtaat ctggcaatgt g 21

<210> 546
<211> 18
<212> DNA
<213> Artificial Sequence

<220>
<223> Synthetic Construct

<400> 546
tggtatatct gcaaccca 18

<210> 547
<211> 19
<212> DNA
<213> Artificial Sequence

<220>
<223> Synthetic Construct

<400> 547
tatgcaaatg gaaagaggg 19

<210> 548
<211> 19
<212> DNA
<213> Artificial Sequence

<220>
<223> Synthetic Construct

<400> 548
aaatgttaaa gggattcca 19

<210> 549
<211> 21
<212> DNA
<213> Artificial Sequence

<220>
<223> Synthetic Construct

<400> 549
tatgcatacc gaaaagttcc c 21

<210> 550
<211> 21
<212> DNA
<213> Artificial Sequence

<220>
<223> Synthetic Construct

<400> 550
ttttgcacca acctatttag t 21

<210> 551
<211> 18
<212> DNA
<213> Artificial Sequence

<220>
<223> Synthetic Construct

<400> 551
aaattcctga agcagctc 18

<210> 552
<211> 18
<212> DNA
<213> Artificial Sequence

<220>
<223> Synthetic Construct

<400> 552
cccaaagtcc cgagcatc 18

<210> 553
<211> 18
<212> DNA
<213> Artificial Sequence

<220>
<223> Synthetic Construct

<400> 553
gcctgatgcc agaggtcc 18

<210> 554
<211> 18
<212> DNA
<213> Artificial Sequence

<220>
<223> Synthetic Construct

<400> 554
cctgtgggtt acggggag 18

<210> 555
<211> 19
<212> DNA
<213> Artificial Sequence

<220>
<223> Synthetic Construct

<400> 555
agcaaccttg gatcatctg 19

<210> 556
<211> 22
<212> DNA

<213> Artificial Sequence

<220>
<223> Synthetic Construct

<400> 556
aattacaatt aatccctcac tg 22

<210> 557
<211> 21
<212> DNA
<213> Artificial Sequence

<220>
<223> Synthetic Construct

<400> 557
ctcacatcag ggaaaatgac c 21

<210> 558
<211> 18
<212> DNA
<213> Artificial Sequence

<220>
<223> Synthetic Construct

<400> 558
gacccaccgc tgagaagg 18

<210> 559
<211> 18
<212> DNA
<213> Artificial Sequence

<220>
<223> Synthetic Construct

<400> 559
tccaaacacc ccatctcc 18

<210> 560
<211> 20
<212> DNA
<213> Artificial Sequence

<220>
<223> Synthetic Construct

<400> 560
tccctaccca gtttcgtatg 20

<210> 561
<211> 20
<212> DNA
<213> Artificial Sequence

<220>

<223> Synthetic Construct

<400> 561
aggaggagga gcacacctta 20

<210> 562
<211> 19
<212> DNA
<213> Artificial Sequence

<220>
<223> Synthetic Construct

<400> 562
aaaaccttca ctggctttc 19

<210> 563
<211> 19
<212> DNA
<213> Artificial Sequence

<220>
<223> Synthetic Construct

<400> 563
gggcaaagat gactgatac 19

<210> 564
<211> 20
<212> DNA
<213> Artificial Sequence

<220>
<223> Synthetic Construct

<400> 564
tgggtcagag acaaaaggat 20

<210> 565
<211> 20
<212> DNA
<213> Artificial Sequence

<220>
<223> Synthetic Construct

<400> 565
ccatgtaccc actagcagtt 20

<210> 566
<211> 21
<212> DNA
<213> Artificial Sequence

<220>
<223> Synthetic Construct

<400> 566

aaactgtaca aaaggcaagg g 21

<210> 567
<211> 20
<212> DNA
<213> Artificial Sequence

<220>
<223> Synthetic Construct

<400> 567
tttcttcctc catgtgtatt 20

<210> 568
<211> 18
<212> DNA
<213> Artificial Sequence

<220>
<223> Synthetic Construct

<400> 568
gaggttggaa taaagcca 18

<210> 569
<211> 20
<212> DNA
<213> Artificial Sequence

<220>
<223> Synthetic Construct

<400> 569
tgacattagg ccagacactt 20

<210> 570
<211> 20
<212> DNA
<213> Artificial Sequence

<220>
<223> Synthetic Construct

<400> 570
tgatttaatt ctcccatcaa 20

<210> 571
<211> 18
<212> DNA
<213> Artificial Sequence

<220>
<223> Synthetic Construct

<400> 571
gaggcgtccc ctggataa 18

<210> 572
<211> 20
<212> DNA
<213> Artificial Sequence

<220>
<223> Synthetic Construct

<400> 572
gagtggcaag aaggaagctg 20

<210> 573
<211> 18
<212> DNA
<213> Artificial Sequence

<220>
<223> Synthetic Construct

<400> 573
tagcgaccac tgaggact 18

<210> 574
<211> 18
<212> DNA
<213> Artificial Sequence

<220>
<223> Synthetic Construct

<400> 574
tgcccatgag agaagccc 18

<210> 575
<211> 20
<212> DNA
<213> Artificial Sequence

<220>
<223> Synthetic Construct

<400> 575
gcctcgaaga tatcctccct 20

<210> 576
<211> 18
<212> DNA
<213> Artificial Sequence

<220>
<223> Synthetic Construct

<400> 576
tggcatgcaa ctgtactc 18

<210> 577
<211> 18
<212> DNA

<213>  Artificial Sequence

<220>
<223>  Synthetic Construct

<400>  577
tgattagtgt tcccctgg                                                     18

<210>  578
<211>  22
<212>  DNA
<213>  Artificial Sequence

<220>
<223>  Synthetic Construct

<400>  578
caaaaggatg taaagaagat cc                                                22

<210>  579
<211>  18
<212>  DNA
<213>  Artificial Sequence

<220>
<223>  Synthetic Construct

<400>  579
cacagtgacc tccgggtt                                                     18

<210>  580
<211>  20
<212>  DNA
<213>  Artificial Sequence

<220>
<223>  Synthetic Construct

<400>  580
gatgtggagt tccattatga                                                   20

<210>  581
<211>  18
<212>  DNA
<213>  Artificial Sequence

<220>
<223>  Synthetic Construct

<400>  581
tgatgctgta ctgtggca                                                     18

<210>  582
<211>  18
<212>  DNA
<213>  Artificial Sequence

<220>

<223> Synthetic Construct

<400> 582
ggcctgaggt gctgagga 18

<210> 583
<211> 23
<212> DNA
<213> Artificial Sequence

<220>
<223> Synthetic Construct

<400> 583
gagacactga ggaactatag gaa 23

<210> 584
<211> 18
<212> DNA
<213> Artificial Sequence

<220>
<223> Synthetic Construct

<400> 584
tacctcccca gcctcttt 18

<210> 585
<211> 19
<212> DNA
<213> Artificial Sequence

<220>
<223> Synthetic Construct

<400> 585
gcagaggata aattgaagg 19

<210> 586
<211> 19
<212> DNA
<213> Artificial Sequence

<220>
<223> Synthetic Construct

<400> 586
tcggagctca gcttcccat 19

<210> 587
<211> 21
<212> DNA
<213> Artificial Sequence

<220>
<223> Synthetic Construct

<400> 587

gttaggtgta gtgcttaagg g 21

<210> 588
<211> 18
<212> DNA
<213> Artificial Sequence

<220>
<223> Synthetic Construct

<400> 588
ttcttgccac aatcactc 18

<210> 589
<211> 23
<212> DNA
<213> Artificial Sequence

<220>
<223> Synthetic Construct

<400> 589
tcaggaatat aggtgaataa caa 23

<210> 590
<211> 18
<212> DNA
<213> Artificial Sequence

<220>
<223> Synthetic Construct

<400> 590
tgttgacctc aatttggc 18

<210> 591
<211> 20
<212> DNA
<213> Artificial Sequence

<220>
<223> Synthetic Construct

<400> 591
tttgaaatga aaacctcact 20

<210> 592
<211> 19
<212> DNA
<213> Artificial Sequence

<220>
<223> Synthetic Construct

<400> 592
ctaggtgagg tcattgctg 19

<210> 593
<211> 19
<212> DNA
<213> Artificial Sequence

<220>
<223> Synthetic Construct

<400> 593
gaggaattct gggccaatc 19

<210> 594
<211> 20
<212> DNA
<213> Artificial Sequence

<220>
<223> Synthetic Construct

<400> 594
tgcttatgat tggatgtgga 20

<210> 595
<211> 19
<212> DNA
<213> Artificial Sequence

<220>
<223> Synthetic Construct

<400> 595
tgaatatgaa gacttgggg 19

<210> 596
<211> 21
<212> DNA
<213> Artificial Sequence

<220>
<223> Synthetic Construct

<400> 596
ttcttgaggt ttagtgcaat c 21

<210> 597
<211> 19
<212> DNA
<213> Artificial Sequence

<220>
<223> Synthetic Construct

<400> 597
gctgccagta gtaccatca 19

<210> 598
<211> 20
<212> DNA

<213> Artificial Sequence

<220>
<223> Synthetic Construct

<400> 598
tgtgttcctc cctgcataag 20

<210> 599
<211> 20
<212> DNA
<213> Artificial Sequence

<220>
<223> Synthetic Construct

<400> 599
tgaaaagtaa tttggaacga 20

<210> 600
<211> 19
<212> DNA
<213> Artificial Sequence

<220>
<223> Synthetic Construct

<400> 600
ctccgaatct tttcttgga 19

<210> 601
<211> 24
<212> DNA
<213> Artificial Sequence

<220>
<223> Synthetic Construct

<400> 601
tcggttagta ttctaagcaa tgtt 24

<210> 602
<211> 20
<212> DNA
<213> Artificial Sequence

<220>
<223> Synthetic Construct

<400> 602
aagccattcc taaactagca 20

<210> 603
<211> 20
<212> DNA
<213> Artificial Sequence

<220>

<223> Synthetic Construct

<400> 603
tgtgggctca tggttaactg 20

<210> 604
<211> 20
<212> DNA
<213> Artificial Sequence

<220>
<223> Synthetic Construct

<400> 604
aaacgaaagg caaaactgag 20

<210> 605
<211> 19
<212> DNA
<213> Artificial Sequence

<220>
<223> Synthetic Construct

<400> 605
tccttatctc ccaggacac 19

<210> 606
<211> 20
<212> DNA
<213> Artificial Sequence

<220>
<223> Synthetic Construct

<400> 606
acaatttccc agttagatga 20

<210> 607
<211> 18
<212> DNA
<213> Artificial Sequence

<220>
<223> Synthetic Construct

<400> 607
acttccctgc agccctct 18

<210> 608
<211> 18
<212> DNA
<213> Artificial Sequence

<220>
<223> Synthetic Construct

<400> 608

cgtcttggtc ttcctcct 18

<210> 609
<211> 18
<212> DNA
<213> Artificial Sequence

<220>
<223> Synthetic Construct

<400> 609
ggagctgcga cacggaga 18

<210> 610
<211> 18
<212> DNA
<213> Artificial Sequence

<220>
<223> Synthetic Construct

<400> 610
cagccagaag gatgtgcg 18

<210> 611
<211> 22
<212> DNA
<213> Artificial Sequence

<220>
<223> Synthetic Construct

<400> 611
tgatacaaac tcaaacccta tg 22

<210> 612
<211> 19
<212> DNA
<213> Artificial Sequence

<220>
<223> Synthetic Construct

<400> 612
gcattagcca ttttcagtt 19

<210> 613
<211> 20
<212> DNA
<213> Artificial Sequence

<220>
<223> Synthetic Construct

<400> 613
tggttggttg ttgaatactt 20

<210> 614
<211> 19
<212> DNA
<213> Artificial Sequence

<220>
<223> Synthetic Construct

<400> 614
gaatgcagca aagcatgag 19

<210> 615
<211> 20
<212> DNA
<213> Artificial Sequence

<220>
<223> Synthetic Construct

<400> 615
ggccatagag acatagtcag 20

<210> 616
<211> 19
<212> DNA
<213> Artificial Sequence

<220>
<223> Synthetic Construct

<400> 616
ggcaaggaga tatgtcagc 19

<210> 617
<211> 19
<212> DNA
<213> Artificial Sequence

<220>
<223> Synthetic Construct

<400> 617
gctgccaacc tccagactc 19

<210> 618
<211> 20
<212> DNA
<213> Artificial Sequence

<220>
<223> Synthetic Construct

<400> 618
gcttatggat gtaagcaatg 20

<210> 619
<211> 22
<212> DNA

<213> Artificial Sequence

<220>
<223> Synthetic Construct

<400> 619
ggaagaccat ctgatactat gt 22

<210> 620
<211> 18
<212> DNA
<213> Artificial Sequence

<220>
<223> Synthetic Construct

<400> 620
gaaatggcct tattgcat 18

<210> 621
<211> 22
<212> DNA
<213> Artificial Sequence

<220>
<223> Synthetic Construct

<400> 621
gtttaaccaa ctttaaccaa ga 22

<210> 622
<211> 19
<212> DNA
<213> Artificial Sequence

<220>
<223> Synthetic Construct

<400> 622
tatgcaaatg gagctattg 19

<210> 623
<211> 18
<212> DNA
<213> Artificial Sequence

<220>
<223> Synthetic Construct

<400> 623
ttgagtgctt tgggtcac 18

<210> 624
<211> 20
<212> DNA
<213> Artificial Sequence

<220>

<223> Synthetic Construct

<400> 624
ttgaacacaa attcaggttt 20

<210> 625
<211> 19
<212> DNA
<213> Artificial Sequence

<220>
<223> Synthetic Construct

<400> 625
ccaaaggcta gtgcacatt 19

<210> 626
<211> 20
<212> DNA
<213> Artificial Sequence

<220>
<223> Synthetic Construct

<400> 626
attggtgcaa attactgttt 20

<210> 627
<211> 19
<212> DNA
<213> Artificial Sequence

<220>
<223> Synthetic Construct

<400> 627
ttaggtttta gacttgccc 19

<210> 628
<211> 20
<212> DNA
<213> Artificial Sequence

<220>
<223> Synthetic Construct

<400> 628
tcctttggct ctattctctt 20

<210> 629
<211> 23
<212> DNA
<213> Artificial Sequence

<220>
<223> Synthetic Construct

<400> 629

agcataatgt tttatctcaa ctc 23

<210> 630
<211> 20
<212> DNA
<213> Artificial Sequence

<220>
<223> Synthetic Construct

<400> 630
caagtaatga tgggctttta 20

<210> 631
<211> 18
<212> DNA
<213> Artificial Sequence

<220>
<223> Synthetic Construct

<400> 631
tgtgcaccac ctattcaa 18

<210> 632
<211> 19
<212> DNA
<213> Artificial Sequence

<220>
<223> Synthetic Construct

<400> 632
gaatcagtgg ttggcatgg 19

<210> 633
<211> 18
<212> DNA
<213> Artificial Sequence

<220>
<223> Synthetic Construct

<400> 633
ggccaggact gggatctg 18

<210> 634
<211> 18
<212> DNA
<213> Artificial Sequence

<220>
<223> Synthetic Construct

<400> 634
gggcttcagt gagaacct 18

<210> 635
<211> 20
<212> DNA
<213> Artificial Sequence

<220>
<223> Synthetic Construct

<400> 635
tgaaaccaat accaaggagt 20

<210> 636
<211> 21
<212> DNA
<213> Artificial Sequence

<220>
<223> Synthetic Construct

<400> 636
tctcgagata aaatgggctg t 21

<210> 637
<211> 21
<212> DNA
<213> Artificial Sequence

<220>
<223> Synthetic Construct

<400> 637
ttgcatcaga aagtaagtgg g 21

<210> 638
<211> 21
<212> DNA
<213> Artificial Sequence

<220>
<223> Synthetic Construct

<400> 638
ttgtaggctt attacggtgt t 21

<210> 639
<211> 18
<212> DNA
<213> Artificial Sequence

<220>
<223> Synthetic Construct

<400> 639
agaccccttt gcaggact 18

<210> 640
<211> 18
<212> DNA

<213> Artificial Sequence

<220>
<223> Synthetic Construct

<400> 640
gaaggtgttt ggttccca 18

<210> 641
<211> 19
<212> DNA
<213> Artificial Sequence

<220>
<223> Synthetic Construct

<400> 641
gcctgtcgtt attggactt 19

<210> 642
<211> 20
<212> DNA
<213> Artificial Sequence

<220>
<223> Synthetic Construct

<400> 642
tcccctcatc taaaggcaca 20

<210> 643
<211> 18
<212> DNA
<213> Artificial Sequence

<220>
<223> Synthetic Construct

<400> 643
gcaccacggg agtcatgt 18

<210> 644
<211> 20
<212> DNA
<213> Artificial Sequence

<220>
<223> Synthetic Construct

<400> 644
gcattggagg tagcaatggt 20

<210> 645
<211> 19
<212> DNA
<213> Artificial Sequence

<220>

<223> Synthetic Construct

<400> 645
ggaggcagaa gggaagcta 19

<210> 646
<211> 23
<212> DNA
<213> Artificial Sequence

<220>
<223> Synthetic Construct

<400> 646
ggagcgaaag ctcaagctta aaa 23

<210> 647
<211> 20
<212> DNA
<213> Artificial Sequence

<220>
<223> Synthetic Construct

<400> 647
gcctggatct gcaacttacc 20

<210> 648
<211> 18
<212> DNA
<213> Artificial Sequence

<220>
<223> Synthetic Construct

<400> 648
cacaggtact gcaacgag 18

<210> 649
<211> 20
<212> DNA
<213> Artificial Sequence

<220>
<223> Synthetic Construct

<400> 649
cttggcatac actttcccac 20

<210> 650
<211> 19
<212> DNA
<213> Artificial Sequence

<220>
<223> Synthetic Construct

<400> 650

tcctcatagc cacataaca 19

<210> 651
<211> 19
<212> DNA
<213> Artificial Sequence

<220>
<223> Synthetic Construct

<400> 651
gtttcactgc tgctgagtt 19

<210> 652
<211> 18
<212> DNA
<213> Artificial Sequence

<220>
<223> Synthetic Construct

<400> 652
caatggccag tgagaacc 18

<210> 653
<211> 21
<212> DNA
<213> Artificial Sequence

<220>
<223> Synthetic Construct

<400> 653
gctctaggga tctgctgtct g 21

<210> 654
<211> 19
<212> DNA
<213> Artificial Sequence

<220>
<223> Synthetic Construct

<400> 654
tggcataaga tctaccctc 19

<210> 655
<211> 18
<212> DNA
<213> Artificial Sequence

<220>
<223> Synthetic Construct

<400> 655
tacttcacgg cctcgtcc 18

<210> 656
<211> 18
<212> DNA
<213> Artificial Sequence

<220>
<223> Synthetic Construct

<400> 656
ctgtgtgtgc tgggcctg 18

<210> 657
<211> 18
<212> DNA
<213> Artificial Sequence

<220>
<223> Synthetic Construct

<400> 657
gcggcctgct acttccag 18

<210> 658
<211> 18
<212> DNA
<213> Artificial Sequence

<220>
<223> Synthetic Construct

<400> 658
tcaagtcgtg ctcctggc 18

<210> 659
<211> 21
<212> DNA
<213> Artificial Sequence

<220>
<223> Synthetic Construct

<400> 659
ttcttgctca agtatactgc t 21

<210> 660
<211> 20
<212> DNA
<213> Artificial Sequence

<220>
<223> Synthetic Construct

<400> 660
ggagtcacat gacatacaca 20

<210> 661
<211> 21
<212> DNA

<213> Artificial Sequence

<220>
<223> Synthetic Construct

<400> 661
catcaattca tggagggatt c 21

<210> 662
<211> 18
<212> DNA
<213> Artificial Sequence

<220>
<223> Synthetic Construct

<400> 662
tggagcgagg atacagga 18

<210> 663
<211> 23
<212> DNA
<213> Artificial Sequence

<220>
<223> Synthetic Construct

<400> 663
tcctacaaga atattagcag ccc 23

<210> 664
<211> 18
<212> DNA
<213> Artificial Sequence

<220>
<223> Synthetic Construct

<400> 664
gattgctgat ggtatggc 18

<210> 665
<211> 18
<212> DNA
<213> Artificial Sequence

<220>
<223> Synthetic Construct

<400> 665
ttgggaaatc gaatggca 18

<210> 666
<211> 21
<212> DNA
<213> Artificial Sequence

<220>

<223> Synthetic Construct

<400> 666
aaaatattcc aagagcttcc a 21

<210> 667
<211> 20
<212> DNA
<213> Artificial Sequence

<220>
<223> Synthetic Construct

<400> 667
caacagcata tagtggaaca 20

<210> 668
<211> 20
<212> DNA
<213> Artificial Sequence

<220>
<223> Synthetic Construct

<400> 668
ccaggtacaa tgatgccaga 20

<210> 669
<211> 20
<212> DNA
<213> Artificial Sequence

<220>
<223> Synthetic Construct

<400> 669
accattgtca agtttcctaa 20

<210> 670
<211> 19
<212> DNA
<213> Artificial Sequence

<220>
<223> Synthetic Construct

<400> 670
tattcgtgat tgggatgag 19

<210> 671
<211> 18
<212> DNA
<213> Artificial Sequence

<220>
<223> Synthetic Construct

<400> 671

tgcagagctg agtagctg 18

<210> 672
<211> 18
<212> DNA
<213> Artificial Sequence

<220>
<223> Synthetic Construct

<400> 672
tggaaacttt ctgcaaac 18

<210> 673
<211> 19
<212> DNA
<213> Artificial Sequence

<220>
<223> Synthetic Construct

<400> 673
cagaatgaaa ggaacctca 19

<210> 674
<211> 20
<212> DNA
<213> Artificial Sequence

<220>
<223> Synthetic Construct

<400> 674
gagcttgatt tgaagctttt 20

<210> 675
<211> 19
<212> DNA
<213> Artificial Sequence

<220>
<223> Synthetic Construct

<400> 675
tgaggttctg gagttgtca 19

<210> 676
<211> 19
<212> DNA
<213> Artificial Sequence

<220>
<223> Synthetic Construct

<400> 676
gggagtctca caagggaca 19

<210> 677
<211> 20
<212> DNA
<213> Artificial Sequence

<220>
<223> Synthetic Construct

<400> 677
gcagaagttt tatgttggac 20

<210> 678
<211> 18
<212> DNA
<213> Artificial Sequence

<220>
<223> Synthetic Construct

<400> 678
atttccactc atgctcaa 18

<210> 679
<211> 19
<212> DNA
<213> Artificial Sequence

<220>
<223> Synthetic Construct

<400> 679
ggcttttctc tagctggtt 19

<210> 680
<211> 19
<212> DNA
<213> Artificial Sequence

<220>
<223> Synthetic Construct

<400> 680
tttaacaata tgccatccc 19

<210> 681
<211> 18
<212> DNA
<213> Artificial Sequence

<220>
<223> Synthetic Construct

<400> 681
gacagctaca caggggca 18

<210> 682
<211> 19
<212> DNA

<213> Artificial Sequence

<220>
<223> Synthetic Construct

<400> 682
gcacagaacc ccagagtca 19

<210> 683
<211> 19
<212> DNA
<213> Artificial Sequence

<220>
<223> Synthetic Construct

<400> 683
gctaaagcag acttggact 19

<210> 684
<211> 18
<212> DNA
<213> Artificial Sequence

<220>
<223> Synthetic Construct

<400> 684
ctcccagcct ttgtagag 18

<210> 685
<211> 24
<212> DNA
<213> Artificial Sequence

<220>
<223> Synthetic Construct

<400> 685
gacaaagaat acagacttca taga 24

<210> 686
<211> 20
<212> DNA
<213> Artificial Sequence

<220>
<223> Synthetic Construct

<400> 686
ctcaagggtt ttgttgttgt 20

<210> 687
<211> 19
<212> DNA
<213> Artificial Sequence

<220>

<223> Synthetic Construct

<400> 687
ggaaccaaac gcttcgact 19

<210> 688
<211> 20
<212> DNA
<213> Artificial Sequence

<220>
<223> Synthetic Construct

<400> 688
cttgcatgag accagcttca 20

<210> 689
<211> 19
<212> DNA
<213> Artificial Sequence

<220>
<223> Synthetic Construct

<400> 689
ggaggaaatg aggagttca 19

<210> 690
<211> 18
<212> DNA
<213> Artificial Sequence

<220>
<223> Synthetic Construct

<400> 690
atgcataccc actgcctg 18

<210> 691
<211> 19
<212> DNA
<213> Artificial Sequence

<220>
<223> Synthetic Construct

<400> 691
caggccttcc acatcaagt 19

<210> 692
<211> 19
<212> DNA
<213> Artificial Sequence

<220>
<223> Synthetic Construct

<400> 692

tcctacctac ctccctggc 19

<210> 693
<211> 19
<212> DNA
<213> Artificial Sequence

<220>
<223> Synthetic Construct

<400> 693
agttacccac acctttggt 19

<210> 694
<211> 19
<212> DNA
<213> Artificial Sequence

<220>
<223> Synthetic Construct

<400> 694
cccagcacca ctgagtttc 19

<210> 695
<211> 19
<212> DNA
<213> Artificial Sequence

<220>
<223> Synthetic Construct

<400> 695
atcgacccaa tcattacat 19

<210> 696
<211> 18
<212> DNA
<213> Artificial Sequence

<220>
<223> Synthetic Construct

<400> 696
atgataggaa gtgggcaa 18

<210> 697
<211> 18
<212> DNA
<213> Artificial Sequence

<220>
<223> Synthetic Construct

<400> 697
ccagagagca gaattcca 18

<210> 698
<211> 18
<212> DNA
<213> Artificial Sequence

<220>
<223> Synthetic Construct

<400> 698
tcccaccact gcagaaag 18

<210> 699
<211> 18
<212> DNA
<213> Artificial Sequence

<220>
<223> Synthetic Construct

<400> 699
ccccaagaaa gaggctca 18

<210> 700
<211> 18
<212> DNA
<213> Artificial Sequence

<220>
<223> Synthetic Construct

<400> 700
aggggctgga tctggatt 18

<210> 701
<211> 19
<212> DNA
<213> Artificial Sequence

<220>
<223> Synthetic Construct

<400> 701
attgtctgaa ctcaacccc 19

<210> 702
<211> 19
<212> DNA
<213> Artificial Sequence

<220>
<223> Synthetic Construct

<400> 702
gtccaaggtc acacaagtt 19

<210> 703
<211> 18
<212> DNA

<213> Artificial Sequence

<220>
<223> Synthetic Construct

<400> 703
gtggggagtg aaggttta 18

<210> 704
<211> 19
<212> DNA
<213> Artificial Sequence

<220>
<223> Synthetic Construct

<400> 704
aactgttgtg cagtgtttg 19

<210> 705
<211> 18
<212> DNA
<213> Artificial Sequence

<220>
<223> Synthetic Construct

<400> 705
ttcctggcaa agttgttc 18

<210> 706
<211> 22
<212> DNA
<213> Artificial Sequence

<220>
<223> Synthetic Construct

<400> 706
ataggacata acaaatgaat cc 22

<210> 707
<211> 18
<212> DNA
<213> Artificial Sequence

<220>
<223> Synthetic Construct

<400> 707
gcaaactgca tggactaa 18

<210> 708
<211> 18
<212> DNA
<213> Artificial Sequence

<220>

<223> Synthetic Construct

<400> 708
ggggaacagg tctgtctt 18

<210> 709
<211> 18
<212> DNA
<213> Artificial Sequence

<220>
<223> Synthetic Construct

<400> 709
atgcaacacc cttcactg 18

<210> 710
<211> 19
<212> DNA
<213> Artificial Sequence

<220>
<223> Synthetic Construct

<400> 710
ggctccctag acatagctc 19

<210> 711
<211> 18
<212> DNA
<213> Artificial Sequence

<220>
<223> Synthetic Construct

<400> 711
ggagaccgac actgatga 18

<210> 712
<211> 18
<212> DNA
<213> Artificial Sequence

<220>
<223> Synthetic Construct

<400> 712
agatgtccac agcacaga 18

<210> 713
<211> 19
<212> DNA
<213> Artificial Sequence

<220>
<223> Synthetic Construct

<400> 713

caaagaccag acaagttcc 19

<210> 714
<211> 21
<212> DNA
<213> Artificial Sequence

<220>
<223> Synthetic Construct

<400> 714
ccctgtgtaa ttcttatctc a 21

<210> 715
<211> 23
<212> DNA
<213> Artificial Sequence

<220>
<223> Synthetic Construct

<400> 715
tgtatcagtt ggagtagtta cca 23

<210> 716
<211> 20
<212> DNA
<213> Artificial Sequence

<220>
<223> Synthetic Construct

<400> 716
ctgtcctgtg aatccatccc 20

<210> 717
<211> 21
<212> DNA
<213> Artificial Sequence

<220>
<223> Synthetic Construct

<400> 717
cgcccggcct gtatatatct t 21

<210> 718
<211> 20
<212> DNA
<213> Artificial Sequence

<220>
<223> Synthetic Construct

<400> 718
tggaaagttg gctattcctc 20

<210> 719
<211> 19
<212> DNA
<213> Artificial Sequence

<220>
<223> Synthetic Construct

<400> 719
cctaatgggt gtgacttct 19

<210> 720
<211> 19
<212> DNA
<213> Artificial Sequence

<220>
<223> Synthetic Construct

<400> 720
tcaccaatga gcatatgaa 19

<210> 721
<211> 19
<212> DNA
<213> Artificial Sequence

<220>
<223> Synthetic Construct

<400> 721
gatggggacc cagactgtt 19

<210> 722
<211> 18
<212> DNA
<213> Artificial Sequence

<220>
<223> Synthetic Construct

<400> 722
ccacgcaggg cttcagtc 18

<210> 723
<211> 20
<212> DNA
<213> Artificial Sequence

<220>
<223> Synthetic Construct

<400> 723
caagtttgaa cgcacatgct 20

<210> 724
<211> 20
<212> DNA

<213>  Artificial Sequence

<220>
<223>  Synthetic Construct

<400>  724
tggtgacgtc ctgttatttg                                                    20

<210>  725
<211>  18
<212>  DNA
<213>  Artificial Sequence

<220>
<223>  Synthetic Construct

<400>  725
gtggcccgga tcctcaac                                                      18

<210>  726
<211>  18
<212>  DNA
<213>  Artificial Sequence

<220>
<223>  Synthetic Construct

<400>  726
agcctgcctc ctctcctc                                                      18

<210>  727
<211>  18
<212>  DNA
<213>  Artificial Sequence

<220>
<223>  Synthetic Construct

<400>  727
ccttagtttt gggcgcag                                                      18

<210>  728
<211>  18
<212>  DNA
<213>  Artificial Sequence

<220>
<223>  Synthetic Construct

<400>  728
gcagcggcct ccctaaga                                                      18

<210>  729
<211>  19
<212>  DNA
<213>  Artificial Sequence

<220>

<223> Synthetic Construct

<400> 729
attccctctc cctgagccc 19

<210> 730
<211> 22
<212> DNA
<213> Artificial Sequence

<220>
<223> Synthetic Construct

<400> 730
tgtgaagtac ttagaatagc ca 22

<210> 731
<211> 20
<212> DNA
<213> Artificial Sequence

<220>
<223> Synthetic Construct

<400> 731
attcctttcc tttgaatgat 20

<210> 732
<211> 21
<212> DNA
<213> Artificial Sequence

<220>
<223> Synthetic Construct

<400> 732
gatggaccag aaacaagaaa a 21

<210> 733
<211> 18
<212> DNA
<213> Artificial Sequence

<220>
<223> Synthetic Construct

<400> 733
gctctgcgct gtctctcc 18

<210> 734
<211> 18
<212> DNA
<213> Artificial Sequence

<220>
<223> Synthetic Construct

<400> 734

atctgctcgt gcttcgcc 18

<210> 735
<211> 18
<212> DNA
<213> Artificial Sequence

<220>
<223> Synthetic Construct

<400> 735
attgcactgc actccacc 18

<210> 736
<211> 22
<212> DNA
<213> Artificial Sequence

<220>
<223> Synthetic Construct

<400> 736
gaaagcactt ttgttttcgt tt 22

<210> 737
<211> 19
<212> DNA
<213> Artificial Sequence

<220>
<223> Synthetic Construct

<400> 737
gaaagaaaag gtgaggcta 19

<210> 738
<211> 20
<212> DNA
<213> Artificial Sequence

<220>
<223> Synthetic Construct

<400> 738
tactgtggag tggtgggctt 20

<210> 739
<211> 20
<212> DNA
<213> Artificial Sequence

<220>
<223> Synthetic Construct

<400> 739
tgtatggtta tatctggcct 20

<210> 740
<211> 19
<212> DNA
<213> Artificial Sequence

<220>
<223> Synthetic Construct

<400> 740
gagcaacaga gggaacaga 19

<210> 741
<211> 22
<212> DNA
<213> Artificial Sequence

<220>
<223> Synthetic Construct

<400> 741
gaccaggacc tataccagac tc 22

<210> 742
<211> 20
<212> DNA
<213> Artificial Sequence

<220>
<223> Synthetic Construct

<400> 742
ttccggtctg tagctactcc 20

<210> 743
<211> 23
<212> DNA
<213> Artificial Sequence

<220>
<223> Synthetic Construct

<400> 743
atcaaggaat ctctctgata ctg 23

<210> 744
<211> 20
<212> DNA
<213> Artificial Sequence

<220>
<223> Synthetic Construct

<400> 744
aaccctatat gctatgtggc 20

<210> 745
<211> 19
<212> DNA

<213> Artificial Sequence

<220>
<223> Synthetic Construct

<400> 745
tgcagtatgt atttgcggc 19

<210> 746
<211> 19
<212> DNA
<213> Artificial Sequence

<220>
<223> Synthetic Construct

<400> 746
caagggacag actccctgc 19

<210> 747
<211> 19
<212> DNA
<213> Artificial Sequence

<220>
<223> Synthetic Construct

<400> 747
aaagacctgc gatcaatag 19

<210> 748
<211> 22
<212> DNA
<213> Artificial Sequence

<220>
<223> Synthetic Construct

<400> 748
gaactttgac ctccataatt cc 22

<210> 749
<211> 18
<212> DNA
<213> Artificial Sequence

<220>
<223> Synthetic Construct

<400> 749
ccaggcaaaa gagggcag 18

<210> 750
<211> 19
<212> DNA
<213> Artificial Sequence

<220>

<223> Synthetic Construct

<400> 750
tggtaccaga ccattccag 19

<210> 751
<211> 18
<212> DNA
<213> Artificial Sequence

<220>
<223> Synthetic Construct

<400> 751
ttagttcagc cactctgc 18

<210> 752
<211> 19
<212> DNA
<213> Artificial Sequence

<220>
<223> Synthetic Construct

<400> 752
gatggacaaa tcccagtaa 19

<210> 753
<211> 18
<212> DNA
<213> Artificial Sequence

<220>
<223> Synthetic Construct

<400> 753
catactgcag tcggtcag 18

<210> 754
<211> 20
<212> DNA
<213> Artificial Sequence

<220>
<223> Synthetic Construct

<400> 754
cacgagcttc atcctagcgg 20

<210> 755
<211> 20
<212> DNA
<213> Artificial Sequence

<220>
<223> Synthetic Construct

<400> 755

cccagttcaa gtggtgttcc 20

<210> 756
<211> 20
<212> DNA
<213> Artificial Sequence

<220>
<223> Synthetic Construct

<400> 756
ggagttcgag agtagccaaa 20

<210> 757
<211> 18
<212> DNA
<213> Artificial Sequence

<220>
<223> Synthetic Construct

<400> 757
cagggttttc tgaagcac 18

<210> 758
<211> 19
<212> DNA
<213> Artificial Sequence

<220>
<223> Synthetic Construct

<400> 758
aggatatccc aattaaccc 19

<210> 759
<211> 20
<212> DNA
<213> Artificial Sequence

<220>
<223> Synthetic Construct

<400> 759
ccttcagcct cccaatataa 20

<210> 760
<211> 19
<212> DNA
<213> Artificial Sequence

<220>
<223> Synthetic Construct

<400> 760
gacaaaggag gagggcttg 19

<210> 761
<211> 20
<212> DNA
<213> Artificial Sequence

<220>
<223> Synthetic Construct

<400> 761
agcatggatc tcattgactc 20

<210> 762
<211> 18
<212> DNA
<213> Artificial Sequence

<220>
<223> Synthetic Construct

<400> 762
ttttcccttc cccacaag 18

<210> 763
<211> 19
<212> DNA
<213> Artificial Sequence

<220>
<223> Synthetic Construct

<400> 763
agggcaccag aggactcac 19

<210> 764
<211> 18
<212> DNA
<213> Artificial Sequence

<220>
<223> Synthetic Construct

<400> 764
tgtggttggg gatggaag 18

<210> 765
<211> 20
<212> DNA
<213> Artificial Sequence

<220>
<223> Synthetic Construct

<400> 765
tacaccctt gttgtattgg 20

<210> 766
<211> 20
<212> DNA

<213> Artificial Sequence

<220>
<223> Synthetic Construct

<400> 766
tggacactca ccatgtgata 20

<210> 767
<211> 19
<212> DNA
<213> Artificial Sequence

<220>
<223> Synthetic Construct

<400> 767
tcagaacaat ataacgggg 19

<210> 768
<211> 20
<212> DNA
<213> Artificial Sequence

<220>
<223> Synthetic Construct

<400> 768
cctaatagcc agcggtggag 20

<210> 769
<211> 22
<212> DNA
<213> Artificial Sequence

<220>
<223> Synthetic Construct

<400> 769
ggtgggagag gatgatatga ca 22

<210> 770
<211> 19
<212> DNA
<213> Artificial Sequence

<220>
<223> Synthetic Construct

<400> 770
tctccccacc cttgatatt 19

<210> 771
<211> 19
<212> DNA
<213> Artificial Sequence

<220>

<223> Synthetic Construct

<400> 771
gactgaagct tgggattag 19

<210> 772
<211> 18
<212> DNA
<213> Artificial Sequence

<220>
<223> Synthetic Construct

<400> 772
tcattaacca gccccatc 18

<210> 773
<211> 18
<212> DNA
<213> Artificial Sequence

<220>
<223> Synthetic Construct

<400> 773
ccattgtttc atggtcct 18

<210> 774
<211> 19
<212> DNA
<213> Artificial Sequence

<220>
<223> Synthetic Construct

<400> 774
tccagtgatt ctggttctt 19

<210> 775
<211> 18
<212> DNA
<213> Artificial Sequence

<220>
<223> Synthetic Construct

<400> 775
gcactcacac ttgcccag 18

<210> 776
<211> 21
<212> DNA
<213> Artificial Sequence

<220>
<223> Synthetic Construct

<400> 776

cttacctggc tctggagtta c 21

<210> 777
<211> 18
<212> DNA
<213> Artificial Sequence

<220>
<223> Synthetic Construct

<400> 777
ttatgctgct aaggcagg 18

<210> 778
<211> 18
<212> DNA
<213> Artificial Sequence

<220>
<223> Synthetic Construct

<400> 778
cccctctcca catcaggg 18

<210> 779
<211> 19
<212> DNA
<213> Artificial Sequence

<220>
<223> Synthetic Construct

<400> 779
agagactcac ctcccctgg 19

<210> 780
<211> 20
<212> DNA
<213> Artificial Sequence

<220>
<223> Synthetic Construct

<400> 780
tttcaaaagg gcttaaactt 20

<210> 781
<211> 19
<212> DNA
<213> Artificial Sequence

<220>
<223> Synthetic Construct

<400> 781
gattgtcttt accccaatg 19

<210> 782
<211> 21
<212> DNA
<213> Artificial Sequence

<220>
<223> Synthetic Construct

<400> 782
tgatacacct acagtgcatt t 21

<210> 783
<211> 18
<212> DNA
<213> Artificial Sequence

<220>
<223> Synthetic Construct

<400> 783
gttgggcgca agtctagg 18

<210> 784
<211> 18
<212> DNA
<213> Artificial Sequence

<220>
<223> Synthetic Construct

<400> 784
cttggcagcc atagtgga 18

<210> 785
<211> 19
<212> DNA
<213> Artificial Sequence

<220>
<223> Synthetic Construct

<400> 785
tatggcttat ggcagattc 19

<210> 786
<211> 21
<212> DNA
<213> Artificial Sequence

<220>
<223> Synthetic Construct

<400> 786
ggaatttaag tccctcttag c 21

<210> 787
<211> 24
<212> DNA

<213> Artificial Sequence

<220>
<223> Synthetic Construct

<400> 787
gatcacaact atgaatcgca tacc 24

<210> 788
<211> 19
<212> DNA
<213> Artificial Sequence

<220>
<223> Synthetic Construct

<400> 788
caaatcaaga ggttccaat 19

<210> 789
<211> 24
<212> DNA
<213> Artificial Sequence

<220>
<223> Synthetic Construct

<400> 789
cagaataagg aggacagggc taag 24

<210> 790
<211> 18
<212> DNA
<213> Artificial Sequence

<220>
<223> Synthetic Construct

<400> 790
gaggggctgg ggcttgta 18

<210> 791
<211> 20
<212> DNA
<213> Artificial Sequence

<220>
<223> Synthetic Construct

<400> 791
cacatggaca caaaggaaca 20

<210> 792
<211> 19
<212> DNA
<213> Artificial Sequence

<220>

<223> Synthetic Construct

<400> 792
tcctcttccc tccctctat 19

<210> 793
<211> 18
<212> DNA
<213> Artificial Sequence

<220>
<223> Synthetic Construct

<400> 793
cagtcccagg gcaaggat 18

<210> 794
<211> 19
<212> DNA
<213> Artificial Sequence

<220>
<223> Synthetic Construct

<400> 794
acaggtcctc cctcaggct 19

<210> 795
<211> 20
<212> DNA
<213> Artificial Sequence

<220>
<223> Synthetic Construct

<400> 795
gcatcacacc agagaatcca 20

<210> 796
<211> 18
<212> DNA
<213> Artificial Sequence

<220>
<223> Synthetic Construct

<400> 796
tggaagtgca ccgacctg 18

<210> 797
<211> 21
<212> DNA
<213> Artificial Sequence

<220>
<223> Synthetic Construct

<400> 797

cagcagaata atgatgatga a 21

<210> 798
<211> 19
<212> DNA
<213> Artificial Sequence

<220>
<223> Synthetic Construct

<400> 798
cccattcaga tgtctctca 19

<210> 799
<211> 20
<212> DNA
<213> Artificial Sequence

<220>
<223> Synthetic Construct

<400> 799
gaaacttgag ccgtgatgaa 20

<210> 800
<211> 20
<212> DNA
<213> Artificial Sequence

<220>
<223> Synthetic Construct

<400> 800
ttgaaagaat caacaggcat 20

<210> 801
<211> 19
<212> DNA
<213> Artificial Sequence

<220>
<223> Synthetic Construct

<400> 801
tacagcccgt ttggtcatc 19

<210> 802
<211> 20
<212> DNA
<213> Artificial Sequence

<220>
<223> Synthetic Construct

<400> 802
ggaaagcgac taccacgact 20

<210> 803
<211> 18
<212> DNA
<213> Artificial Sequence

<220>
<223> Synthetic Construct

<400> 803
gagagagacg ccgagcca 18

<210> 804
<211> 18
<212> DNA
<213> Artificial Sequence

<220>
<223> Synthetic Construct

<400> 804
cccgacgcga gatgtctt 18

<210> 805
<211> 18
<212> DNA
<213> Artificial Sequence

<220>
<223> Synthetic Construct

<400> 805
ggttcaggct tcacccac 18

<210> 806
<211> 20
<212> DNA
<213> Artificial Sequence

<220>
<223> Synthetic Construct

<400> 806
caggccgcaa taagtctaca 20

<210> 807
<211> 18
<212> DNA
<213> Artificial Sequence

<220>
<223> Synthetic Construct

<400> 807
ggaatctgca ggcgctac 18

<210> 808
<211> 19
<212> DNA

<213> Artificial Sequence

<220>
<223> Synthetic Construct

<400> 808
ggtgggaagc aagatcaat 19

<210> 809
<211> 18
<212> DNA
<213> Artificial Sequence

<220>
<223> Synthetic Construct

<400> 809
aagtaaagcc ctgttccc 18

<210> 810
<211> 18
<212> DNA
<213> Artificial Sequence

<220>
<223> Synthetic Construct

<400> 810
tagggaaggc aaccttat 18

<210> 811
<211> 20
<212> DNA
<213> Artificial Sequence

<220>
<223> Synthetic Construct

<400> 811
gaaacctctg cgatggtcta 20

<210> 812
<211> 21
<212> DNA
<213> Artificial Sequence

<220>
<223> Synthetic Construct

<400> 812
tctcctgatc tcctccttga t 21

<210> 813
<211> 20
<212> DNA
<213> Artificial Sequence

<220>

<223> Synthetic Construct

<400> 813
ccagggcagt taagtcctgt 20

<210> 814
<211> 20
<212> DNA
<213> Artificial Sequence

<220>
<223> Synthetic Construct

<400> 814
ttcccctcct ctactcttag 20

<210> 815
<211> 18
<212> DNA
<213> Artificial Sequence

<220>
<223> Synthetic Construct

<400> 815
ggggaccttg ttcaactt 18

<210> 816
<211> 20
<212> DNA
<213> Artificial Sequence

<220>
<223> Synthetic Construct

<400> 816
ccagttgctc accataggta 20

<210> 817
<211> 20
<212> DNA
<213> Artificial Sequence

<220>
<223> Synthetic Construct

<400> 817
agttaatcac caaagccaaa 20

<210> 818
<211> 23
<212> DNA
<213> Artificial Sequence

<220>
<223> Synthetic Construct

<400> 818

tgtgtttact tgcttgttga gtc 23

<210> 819
<211> 20
<212> DNA
<213> Artificial Sequence

<220>
<223> Synthetic Construct

<400> 819
caggtgtgac cggagacttt 20

<210> 820
<211> 18
<212> DNA
<213> Artificial Sequence

<220>
<223> Synthetic Construct

<400> 820
tattttccac ggtccgca 18

<210> 821
<211> 20
<212> DNA
<213> Artificial Sequence

<220>
<223> Synthetic Construct

<400> 821
tggggagact acacctgaca 20

<210> 822
<211> 19
<212> DNA
<213> Artificial Sequence

<220>
<223> Synthetic Construct

<400> 822
gaaccattgg tgcgttcac 19

<210> 823
<211> 19
<212> DNA
<213> Artificial Sequence

<220>
<223> Synthetic Construct

<400> 823
ggcatagcct aggagcagc 19

<210> 824
<211> 20
<212> DNA
<213> Artificial Sequence

<220>
<223> Synthetic Construct

<400> 824
acttgggcca ccaaactcta 20

<210> 825
<211> 19
<212> DNA
<213> Artificial Sequence

<220>
<223> Synthetic Construct

<400> 825
aaaatcatgg cccctgaag 19

<210> 826
<211> 20
<212> DNA
<213> Artificial Sequence

<220>
<223> Synthetic Construct

<400> 826
cctcacttgt cagcgggata 20

<210> 827
<211> 21
<212> DNA
<213> Artificial Sequence

<220>
<223> Synthetic Construct

<400> 827
aatgtggtga tgatctcctg g 21

<210> 828
<211> 18
<212> DNA
<213> Artificial Sequence

<220>
<223> Synthetic Construct

<400> 828
tgttcagcca gcgtcaaa 18

<210> 829
<211> 18
<212> DNA

<213> Artificial Sequence

<220>
<223> Synthetic Construct

<400> 829
tggccatgag cagcagat 18

<210> 830
<211> 18
<212> DNA
<213> Artificial Sequence

<220>
<223> Synthetic Construct

<400> 830
ctgatcagag cacgcctc 18

<210> 831
<211> 18
<212> DNA
<213> Artificial Sequence

<220>
<223> Synthetic Construct

<400> 831
cactccgggc cattcttt 18

<210> 832
<211> 19
<212> DNA
<213> Artificial Sequence

<220>
<223> Synthetic Construct

<400> 832
gactctgcat ctccccgag 19

<210> 833
<211> 18
<212> DNA
<213> Artificial Sequence

<220>
<223> Synthetic Construct

<400> 833
acctggctct ggccttca 18

<210> 834
<211> 20
<212> DNA
<213> Artificial Sequence

<220>

<223> Synthetic Construct

<400> 834
aggacatctc ggaggacagc 20

<210> 835
<211> 19
<212> DNA
<213> Artificial Sequence

<220>
<223> Synthetic Construct

<400> 835
acactactgg gaggctggg 19

<210> 836
<211> 21
<212> DNA
<213> Artificial Sequence

<220>
<223> Synthetic Construct

<400> 836
aacttgtggc ctgtccttat g 21

<210> 837
<211> 22
<212> DNA
<213> Artificial Sequence

<220>
<223> Synthetic Construct

<400> 837
attcatgcct atgtaataaa gc 22

<210> 838
<211> 19
<212> DNA
<213> Artificial Sequence

<220>
<223> Synthetic Construct

<400> 838
tggaaggtct ctgatccca 19

<210> 839
<211> 24
<212> DNA
<213> Artificial Sequence

<220>
<223> Synthetic Construct

<400> 839

ggagataaat agagaagtag aagg 24

<210> 840
<211> 23
<212> DNA
<213> Artificial Sequence

<220>
<223> Synthetic Construct

<400> 840
gacaaacata tggttaaaag act 23

<210> 841
<211> 18
<212> DNA
<213> Artificial Sequence

<220>
<223> Synthetic Construct

<400> 841
cactaccgga gtgcaggg 18

<210> 842
<211> 23
<212> DNA
<213> Artificial Sequence

<220>
<223> Synthetic Construct

<400> 842
tcacaaaaga tctatagaat tgg 23

<210> 843
<211> 20
<212> DNA
<213> Artificial Sequence

<220>
<223> Synthetic Construct

<400> 843
tcagacacct tgggactgag 20

<210> 844
<211> 20
<212> DNA
<213> Artificial Sequence

<220>
<223> Synthetic Construct

<400> 844
ggcacgtagg gtacaagagc 20

<210> 845
<211> 20
<212> DNA
<213> Artificial Sequence

<220>
<223> Synthetic Construct

<400> 845
ttacccatcg aatcaccttg 20

<210> 846
<211> 20
<212> DNA
<213> Artificial Sequence

<220>
<223> Synthetic Construct

<400> 846
gcgagaaaac gaaggatcag 20

<210> 847
<211> 19
<212> DNA
<213> Artificial Sequence

<220>
<223> Synthetic Construct

<400> 847
cttgacttgc cttcccctg 19

<210> 848
<211> 20
<212> DNA
<213> Artificial Sequence

<220>
<223> Synthetic Construct

<400> 848
ataggcatct ctcccggaac 20

<210> 849
<211> 23
<212> DNA
<213> Artificial Sequence

<220>
<223> Synthetic Construct

<400> 849
ctcttttaag ctttctggtt aat 23

<210> 850
<211> 20
<212> DNA

<213> Artificial Sequence

<220>
<223> Synthetic Construct

<400> 850
tgccagatat ggtggataac 20

<210> 851
<211> 20
<212> DNA
<213> Artificial Sequence

<220>
<223> Synthetic Construct

<400> 851
aggtaaggag ggaggaagcc 20

<210> 852
<211> 21
<212> DNA
<213> Artificial Sequence

<220>
<223> Synthetic Construct

<400> 852
ggaagcctga atggactaac a 21

<210> 853
<211> 21
<212> DNA
<213> Artificial Sequence

<220>
<223> Synthetic Construct

<400> 853
gcaatctgca tctctcacct c 21

<210> 854
<211> 20
<212> DNA
<213> Artificial Sequence

<220>
<223> Synthetic Construct

<400> 854
aggcctcgta taactgtggg 20

<210> 855
<211> 19
<212> DNA
<213> Artificial Sequence

<220>

<223> Synthetic Construct

<400> 855
tgtacaaccg agccacacc 19

<210> 856
<211> 20
<212> DNA
<213> Artificial Sequence

<220>
<223> Synthetic Construct

<400> 856
gctactacag ggagtgggca 20

<210> 857
<211> 19
<212> DNA
<213> Artificial Sequence

<220>
<223> Synthetic Construct

<400> 857
cagcatcaac tgccatgtg 19

<210> 858
<211> 18
<212> DNA
<213> Artificial Sequence

<220>
<223> Synthetic Construct

<400> 858
agcttggaag accgggag 18

<210> 859
<211> 20
<212> DNA
<213> Artificial Sequence

<220>
<223> Synthetic Construct

<400> 859
tgcatctcac actggttgta 20

<210> 860
<211> 20
<212> DNA
<213> Artificial Sequence

<220>
<223> Synthetic Construct

<400> 860

ggcacagttt gattagggga 20

<210> 861
<211> 21
<212> DNA
<213> Artificial Sequence

<220>
<223> Synthetic Construct

<400> 861
ttaattcgtg catgtcactg g 21

<210> 862
<211> 20
<212> DNA
<213> Artificial Sequence

<220>
<223> Synthetic Construct

<400> 862
gcacatgggg acctaagaga 20

<210> 863
<211> 20
<212> DNA
<213> Artificial Sequence

<220>
<223> Synthetic Construct

<400> 863
tgtcctaaag cctagggcag 20

<210> 864
<211> 20
<212> DNA
<213> Artificial Sequence

<220>
<223> Synthetic Construct

<400> 864
gccctatgat acagccatgc 20

<210> 865
<211> 18
<212> DNA
<213> Artificial Sequence

<220>
<223> Synthetic Construct

<400> 865
ctagggtttg cacggcag 18

<210> 866
<211> 19
<212> DNA
<213> Artificial Sequence

<220>
<223> Synthetic Construct

<400> 866
agctcagagg agaggggct 19

<210> 867
<211> 20
<212> DNA
<213> Artificial Sequence

<220>
<223> Synthetic Construct

<400> 867
tactgttgac ccatccccac 20

<210> 868
<211> 19
<212> DNA
<213> Artificial Sequence

<220>
<223> Synthetic Construct

<400> 868
cacagcaagc ttaggagcc 19

<210> 869
<211> 18
<212> DNA
<213> Artificial Sequence

<220>
<223> Synthetic Construct

<400> 869
acttccctgc caacccat 18

<210> 870
<211> 19
<212> DNA
<213> Artificial Sequence

<220>
<223> Synthetic Construct

<400> 870
tctccctgca cctctgctc 19

<210> 871
<211> 18
<212> DNA

<213> Artificial Sequence

<220>
<223> Synthetic Construct

<400> 871
agaagtggcc ctggggtg 18

<210> 872
<211> 18
<212> DNA
<213> Artificial Sequence

<220>
<223> Synthetic Construct

<400> 872
tgagatccca caacccct 18

<210> 873
<211> 19
<212> DNA
<213> Artificial Sequence

<220>
<223> Synthetic Construct

<400> 873
ctggctcaat ctcagcagg 19

<210> 874
<211> 18
<212> DNA
<213> Artificial Sequence

<220>
<223> Synthetic Construct

<400> 874
ccaggtggtg cctctgtt 18

<210> 875
<211> 20
<212> DNA
<213> Artificial Sequence

<220>
<223> Synthetic Construct

<400> 875
ctcatgggcc tttagactgg 20

<210> 876
<211> 20
<212> DNA
<213> Artificial Sequence

<220>

<223> Synthetic Construct

<400> 876
ttcctgtacc agcgaacaag 20

<210> 877
<211> 20
<212> DNA
<213> Artificial Sequence

<220>
<223> Synthetic Construct

<400> 877
cccacaacac gtgtgaattt 20

<210> 878
<211> 20
<212> DNA
<213> Artificial Sequence

<220>
<223> Synthetic Construct

<400> 878
cccctgcaat gatatggttt 20

<210> 879
<211> 20
<212> DNA
<213> Artificial Sequence

<220>
<223> Synthetic Construct

<400> 879
gaaacgctga gtattcgagg 20

<210> 880
<211> 22
<212> DNA
<213> Artificial Sequence

<220>
<223> Synthetic Construct

<400> 880
caccttaggt tcaacatcca ca 22

<210> 881
<211> 23
<212> DNA
<213> Artificial Sequence

<220>
<223> Synthetic Construct

<400> 881

aatgactaga tccaacgata taa 23

<210> 882
<211> 20
<212> DNA
<213> Artificial Sequence

<220>
<223> Synthetic Construct

<400> 882
ctggtgggaa tgtaacccag 20

<210> 883
<211> 23
<212> DNA
<213> Artificial Sequence

<220>
<223> Synthetic Construct

<400> 883
accctttgat ctaatgattc taa 23

<210> 884
<211> 21
<212> DNA
<213> Artificial Sequence

<220>
<223> Synthetic Construct

<400> 884
tgtgtcattt cgaacgtgaa t 21

<210> 885
<211> 20
<212> DNA
<213> Artificial Sequence

<220>
<223> Synthetic Construct

<400> 885
acaccttctt ccatgttggg 20

<210> 886
<211> 20
<212> DNA
<213> Artificial Sequence

<220>
<223> Synthetic Construct

<400> 886
tggtaaaacc ctttcgaagt 20

<210> 887
<211> 22
<212> DNA
<213> Artificial Sequence

<220>
<223> Synthetic Construct

<400> 887
gcagagcaag taatagagga ca 22

<210> 888
<211> 20
<212> DNA
<213> Artificial Sequence

<220>
<223> Synthetic Construct

<400> 888
ggagtggagc taacagtggc 20

<210> 889
<211> 20
<212> DNA
<213> Artificial Sequence

<220>
<223> Synthetic Construct

<400> 889
ggagtcaagt gggttgtcat 20

<210> 890
<211> 20
<212> DNA
<213> Artificial Sequence

<220>
<223> Synthetic Construct

<400> 890
tccatttgat gctcgcttag 20

<210> 891
<211> 21
<212> DNA
<213> Artificial Sequence

<220>
<223> Synthetic Construct

<400> 891
ttctacttcc tcgtctcttc a 21

<210> 892
<211> 24
<212> DNA

<213> Artificial Sequence

<220>
<223> Synthetic Construct

<400> 892
catgttttat agtttcttcc cttc 24

<210> 893
<211> 22
<212> DNA
<213> Artificial Sequence

<220>
<223> Synthetic Construct

<400> 893
catagcatta tgagtggtga ct 22

<210> 894
<211> 20
<212> DNA
<213> Artificial Sequence

<220>
<223> Synthetic Construct

<400> 894
ttctgcaaca cgcacttaaa 20

<210> 895
<211> 20
<212> DNA
<213> Artificial Sequence

<220>
<223> Synthetic Construct

<400> 895
tgcttgcttg tttgatcacc 20

<210> 896
<211> 20
<212> DNA
<213> Artificial Sequence

<220>
<223> Synthetic Construct

<400> 896
caatgctaga ttggaggctg 20

<210> 897
<211> 20
<212> DNA
<213> Artificial Sequence

<220>

<223> Synthetic Construct

<400> 897
ggagtcagtg gtggagagga 20

<210> 898
<211> 19
<212> DNA
<213> Artificial Sequence

<220>
<223> Synthetic Construct

<400> 898
ttctttgttc cctcctggc 19

<210> 899
<211> 24
<212> DNA
<213> Artificial Sequence

<220>
<223> Synthetic Construct

<400> 899
aactcgagat acataacatg caca 24

<210> 900
<211> 22
<212> DNA
<213> Artificial Sequence

<220>
<223> Synthetic Construct

<400> 900
tggtagtttc atgagtgtat gc 22

<210> 901
<211> 21
<212> DNA
<213> Artificial Sequence

<220>
<223> Synthetic Construct

<400> 901
caaatgtcca ttaacttgtg a 21

<210> 902
<211> 22
<212> DNA
<213> Artificial Sequence

<220>
<223> Synthetic Construct

<400> 902

tgccgagtca gtagttagtt cc 22

<210> 903
<211> 23
<212> DNA
<213> Artificial Sequence

<220>
<223> Synthetic Construct

<400> 903
cattgaatgg tcttactact ttt 23

<210> 904
<211> 21
<212> DNA
<213> Artificial Sequence

<220>
<223> Synthetic Construct

<400> 904
agcctagaaa gaaacctcac a 21

<210> 905
<211> 24
<212> DNA
<213> Artificial Sequence

<220>
<223> Synthetic Construct

<400> 905
cttctgacta ttatgtggtt cttt 24

<210> 906
<211> 23
<212> DNA
<213> Artificial Sequence

<220>
<223> Synthetic Construct

<400> 906
ccctatcaaa atcccaatgg tct 23

<210> 907
<211> 20
<212> DNA
<213> Artificial Sequence

<220>
<223> Synthetic Construct

<400> 907
ggcgtggctg tgtttaacta 20

<210> 908
<211> 19
<212> DNA
<213> Artificial Sequence

<220>
<223> Synthetic Construct

<400> 908
gaatgaggac agcgggtct 19

<210> 909
<211> 24
<212> DNA
<213> Artificial Sequence

<220>
<223> Synthetic Construct

<400> 909
tgtagccttt taaagacaga ctgg 24

<210> 910
<211> 18
<212> DNA
<213> Artificial Sequence

<220>
<223> Synthetic Construct

<400> 910
accatgcctt gcctcttt 18

<210> 911
<211> 19
<212> DNA
<213> Artificial Sequence

<220>
<223> Synthetic Construct

<400> 911
ggtgctccga agccattag 19

<210> 912
<211> 18
<212> DNA
<213> Artificial Sequence

<220>
<223> Synthetic Construct

<400> 912
gagcaaagct ggcacacg 18

<210> 913
<211> 18
<212> DNA

<213> Artificial Sequence

<220>
<223> Synthetic Construct

<400> 913
tctcccaggg ctcaccag 18

<210> 914
<211> 18
<212> DNA
<213> Artificial Sequence

<220>
<223> Synthetic Construct

<400> 914
gcagccatgg caatccac 18

<210> 915
<211> 20
<212> DNA
<213> Artificial Sequence

<220>
<223> Synthetic Construct

<400> 915
cagtcgcttt tcttgaccct 20

<210> 916
<211> 18
<212> DNA
<213> Artificial Sequence

<220>
<223> Synthetic Construct

<400> 916
tagttcagga tgtgggcg 18

<210> 917
<211> 18
<212> DNA
<213> Artificial Sequence

<220>
<223> Synthetic Construct

<400> 917
accctcacgg ctgacatc 18

<210> 918
<211> 19
<212> DNA
<213> Artificial Sequence

<220>

<223> Synthetic Construct

<400> 918
aggggtaagg agaggggtc 19

<210> 919
<211> 19
<212> DNA
<213> Artificial Sequence

<220>
<223> Synthetic Construct

<400> 919
gataggagga aggcggctc 19

<210> 920
<211> 20
<212> DNA
<213> Artificial Sequence

<220>
<223> Synthetic Construct

<400> 920
gtcctatcca ggaccgcatc 20

<210> 921
<211> 18
<212> DNA
<213> Artificial Sequence

<220>
<223> Synthetic Construct

<400> 921
ggggccgtgt caggaagt 18

<210> 922
<211> 19
<212> DNA
<213> Artificial Sequence

<220>
<223> Synthetic Construct

<400> 922
gacaaggacg aggacaggg 19

<210> 923
<211> 18
<212> DNA
<213> Artificial Sequence

<220>
<223> Synthetic Construct

<400> 923

ttgggatcat ggcacagg 18

<210> 924
<211> 19
<212> DNA
<213> Artificial Sequence

<220>
<223> Synthetic Construct

<400> 924
gacctcaaac ctccgtcca 19

<210> 925
<211> 18
<212> DNA
<213> Artificial Sequence

<220>
<223> Synthetic Construct

<400> 925
tcccctctag actgttgg 18

<210> 926
<211> 19
<212> DNA
<213> Artificial Sequence

<220>
<223> Synthetic Construct

<400> 926
cactacaagg cgacaccat 19

<210> 927
<211> 22
<212> DNA
<213> Artificial Sequence

<220>
<223> Synthetic Construct

<400> 927
tttagcaact gactgtcata ag 22

<210> 928
<211> 21
<212> DNA
<213> Artificial Sequence

<220>
<223> Synthetic Construct

<400> 928
ttccttgagg gctaagatta c 21

<210> 929
<211> 20
<212> DNA
<213> Artificial Sequence

<220>
<223> Synthetic Construct

<400> 929
atagcatgca gggagtcacc 20

<210> 930
<211> 18
<212> DNA
<213> Artificial Sequence

<220>
<223> Synthetic Construct

<400> 930
actgggcaca gcaagcag 18

<210> 931
<211> 18
<212> DNA
<213> Artificial Sequence

<220>
<223> Synthetic Construct

<400> 931
tgagttcgct cctgggtc 18

<210> 932
<211> 20
<212> DNA
<213> Artificial Sequence

<220>
<223> Synthetic Construct

<400> 932
gcttgggtaa gaaggggtct 20

<210> 933
<211> 20
<212> DNA
<213> Artificial Sequence

<220>
<223> Synthetic Construct

<400> 933
tcctgtccct tctcaccttg 20

<210> 934
<211> 22
<212> DNA

<213> Artificial Sequence

<220>
<223> Synthetic Construct

<400> 934
cagaaacagc aatgctagat ca 22

<210> 935
<211> 18
<212> DNA
<213> Artificial Sequence

<220>
<223> Synthetic Construct

<400> 935
tgaggtcttg gatggagg 18

<210> 936
<211> 20
<212> DNA
<213> Artificial Sequence

<220>
<223> Synthetic Construct

<400> 936
tcaacatgga atggggaact 20

<210> 937
<211> 20
<212> DNA
<213> Artificial Sequence

<220>
<223> Synthetic Construct

<400> 937
gagtcctgcc atagcatcaa 20

<210> 938
<211> 20
<212> DNA
<213> Artificial Sequence

<220>
<223> Synthetic Construct

<400> 938
gcctcctgca ttctagtccc 20

<210> 939
<211> 20
<212> DNA
<213> Artificial Sequence

<220>

<223> Synthetic Construct

<400> 939
ggactcactg acctcccttc 20

<210> 940
<211> 19
<212> DNA
<213> Artificial Sequence

<220>
<223> Synthetic Construct

<400> 940
ctggttctgt ccatgtgcc 19

<210> 941
<211> 21
<212> DNA
<213> Artificial Sequence

<220>
<223> Synthetic Construct

<400> 941
ggtgaaaatg gacttggacc t 21

<210> 942
<211> 19
<212> DNA
<213> Artificial Sequence

<220>
<223> Synthetic Construct

<400> 942
aagatccgtc atcggaagg 19

<210> 943
<211> 22
<212> DNA
<213> Artificial Sequence

<220>
<223> Synthetic Construct

<400> 943
ggtccatgag acactctatg cc 22

<210> 944
<211> 24
<212> DNA
<213> Artificial Sequence

<220>
<223> Synthetic Construct

<400> 944

gggtatcagt ttatcagtca atca 24

<210> 945
<211> 21
<212> DNA
<213> Artificial Sequence

<220>
<223> Synthetic Construct

<400> 945
gggagttgag ctgtgtgcta t 21

<210> 946
<211> 20
<212> DNA
<213> Artificial Sequence

<220>
<223> Synthetic Construct

<400> 946
tggagtgaga cctaggcaac 20

<210> 947
<211> 20
<212> DNA
<213> Artificial Sequence

<220>
<223> Synthetic Construct

<400> 947
agcatcttct gacacgcaag 20

<210> 948
<211> 21
<212> DNA
<213> Artificial Sequence

<220>
<223> Synthetic Construct

<400> 948
gccctgattg gatagtagtg c 21

<210> 949
<211> 20
<212> DNA
<213> Artificial Sequence

<220>
<223> Synthetic Construct

<400> 949
tgaagccgag aaatggtgag 20

<210> 950
<211> 19
<212> DNA
<213> Artificial Sequence

<220>
<223> Synthetic Construct

<400> 950
agactaggtc ccgtcaccg 19

<210> 951
<211> 18
<212> DNA
<213> Artificial Sequence

<220>
<223> Synthetic Construct

<400> 951
gcctagacca ctagagcc 18

<210> 952
<211> 18
<212> DNA
<213> Artificial Sequence

<220>
<223> Synthetic Construct

<400> 952
ggcgaaaacc agtgtctt 18

<210> 953
<211> 19
<212> DNA
<213> Artificial Sequence

<220>
<223> Synthetic Construct

<400> 953
ccttagcaca aacgccctt 19

<210> 954
<211> 20
<212> DNA
<213> Artificial Sequence

<220>
<223> Synthetic Construct

<400> 954
ccgaatgtgg ctaaggaaac 20

<210> 955
<211> 20
<212> DNA

<213> Artificial Sequence

<220>
<223> Synthetic Construct

<400> 955
agtcccaagg gataaggtgg 20

<210> 956
<211> 19
<212> DNA
<213> Artificial Sequence

<220>
<223> Synthetic Construct

<400> 956
gggagtggca gtgctttct 19

<210> 957
<211> 18
<212> DNA
<213> Artificial Sequence

<220>
<223> Synthetic Construct

<400> 957
aggtgagaga gggtgggc 18

<210> 958
<211> 20
<212> DNA
<213> Artificial Sequence

<220>
<223> Synthetic Construct

<400> 958
cttcagggtt gaacgctctc 20

<210> 959
<211> 22
<212> DNA
<213> Artificial Sequence

<220>
<223> Synthetic Construct

<400> 959
cagccaattg tatatgagaa aa 22

<210> 960
<211> 24
<212> DNA
<213> Artificial Sequence

<220>

<223> Synthetic Construct

<400> 960
ttgtgacttc aataatactc tctc 24

<210> 961
<211> 18
<212> DNA
<213> Artificial Sequence

<220>
<223> Synthetic Construct

<400> 961
ggaatgtgga agcccttg 18

<210> 962
<211> 18
<212> DNA
<213> Artificial Sequence

<220>
<223> Synthetic Construct

<400> 962
actgccgatg ccagtcac 18

<210> 963
<211> 19
<212> DNA
<213> Artificial Sequence

<220>
<223> Synthetic Construct

<400> 963
atcactgggg cctggtcta 19

<210> 964
<211> 20
<212> DNA
<213> Artificial Sequence

<220>
<223> Synthetic Construct

<400> 964
tacaggacaa cgcacagggt 20

<210> 965
<211> 20
<212> DNA
<213> Artificial Sequence

<220>
<223> Synthetic Construct

<400> 965

ctccacattg caacactacc 20

<210> 966
<211> 20
<212> DNA
<213> Artificial Sequence

<220>
<223> Synthetic Construct

<400> 966
ttgggctgtc atctgggttt 20

<210> 967
<211> 21
<212> DNA
<213> Artificial Sequence

<220>
<223> Synthetic Construct

<400> 967
tccacactgt ccagcattac t 21

<210> 968
<211> 22
<212> DNA
<213> Artificial Sequence

<220>
<223> Synthetic Construct

<400> 968
tggaatcctg agaaagaaag tt 22

<210> 969
<211> 18
<212> DNA
<213> Artificial Sequence

<220>
<223> Synthetic Construct

<400> 969
cccaattagg atcacaca 18

<210> 970
<211> 18
<212> DNA
<213> Artificial Sequence

<220>
<223> Synthetic Construct

<400> 970
atttgggcaa ggggaggt 18

<210> 971
<211> 20
<212> DNA
<213> Artificial Sequence

<220>
<223> Synthetic Construct

<400> 971
cagctagatt cgggatctgt 20

<210> 972
<211> 18
<212> DNA
<213> Artificial Sequence

<220>
<223> Synthetic Construct

<400> 972
gctttgtggg gtgcaaat 18

<210> 973
<211> 23
<212> DNA
<213> Artificial Sequence

<220>
<223> Synthetic Construct

<400> 973
tcaactgcag acttattcag aca 23

<210> 974
<211> 20
<212> DNA
<213> Artificial Sequence

<220>
<223> Synthetic Construct

<400> 974
caccaatcat cagcctgaaa 20

<210> 975
<211> 18
<212> DNA
<213> Artificial Sequence

<220>
<223> Synthetic Construct

<400> 975
cccaaatact gcaccaga 18

<210> 976
<211> 18
<212> DNA

<213> Artificial Sequence

<220>
<223> Synthetic Construct

<400> 976
ctagcctcac aaacacca 18

<210> 977
<211> 22
<212> DNA
<213> Artificial Sequence

<220>
<223> Synthetic Construct

<400> 977
gcaatttaat tcttagtggc at 22

<210> 978
<211> 23
<212> DNA
<213> Artificial Sequence

<220>
<223> Synthetic Construct

<400> 978
gttttaccaa atattcaagt gag 23

<210> 979
<211> 18
<212> DNA
<213> Artificial Sequence

<220>
<223> Synthetic Construct

<400> 979
acagattcct cagccttt 18

<210> 980
<211> 20
<212> DNA
<213> Artificial Sequence

<220>
<223> Synthetic Construct

<400> 980
ttctcctgga ataagacccc 20

<210> 981
<211> 19
<212> DNA
<213> Artificial Sequence

<220>

<223> Synthetic Construct

<400> 981
tttaccaggt tcttggcat 19

<210> 982
<211> 18
<212> DNA
<213> Artificial Sequence

<220>
<223> Synthetic Construct

<400> 982
tgtgaccacc tgccagtc 18

<210> 983
<211> 20
<212> DNA
<213> Artificial Sequence

<220>
<223> Synthetic Construct

<400> 983
ttattgaatc tggttggatt 20

<210> 984
<211> 20
<212> DNA
<213> Artificial Sequence

<220>
<223> Synthetic Construct

<400> 984
gtctgaagta ttgcaaagca 20

<210> 985
<211> 18
<212> DNA
<213> Artificial Sequence

<220>
<223> Synthetic Construct

<400> 985
tgtagggcat ctctaggc 18

<210> 986
<211> 18
<212> DNA
<213> Artificial Sequence

<220>
<223> Synthetic Construct

<400> 986

attggtggag gaccctta 18

<210> 987
<211> 23
<212> DNA
<213> Artificial Sequence

<220>
<223> Synthetic Construct

<400> 987
cagtatgcaa ttatgacaca tag 23

<210> 988
<211> 22
<212> DNA
<213> Artificial Sequence

<220>
<223> Synthetic Construct

<400> 988
acttgttaaa gaagcactgt cc 22

<210> 989
<211> 20
<212> DNA
<213> Artificial Sequence

<220>
<223> Synthetic Construct

<400> 989
tcctggaact taagctcatc 20

<210> 990
<211> 19
<212> DNA
<213> Artificial Sequence

<220>
<223> Synthetic Construct

<400> 990
acagaccagt caagcaatg 19

<210> 991
<211> 18
<212> DNA
<213> Artificial Sequence

<220>
<223> Synthetic Construct

<400> 991
ctcctgctga tgtgcccc 18

<210> 992
<211> 18
<212> DNA
<213> Artificial Sequence

<220>
<223> Synthetic Construct

<400> 992
agggctgcct gtttgggt 18

<210> 993
<211> 24
<212> DNA
<213> Artificial Sequence

<220>
<223> Synthetic Construct

<400> 993
tcagatagca ttttatctcc taga 24

<210> 994
<211> 20
<212> DNA
<213> Artificial Sequence

<220>
<223> Synthetic Construct

<400> 994
tggatcctac tgtccaagtt 20

<210> 995
<211> 20
<212> DNA
<213> Artificial Sequence

<220>
<223> Synthetic Construct

<400> 995
ccagcaatga catgattacc 20

<210> 996
<211> 22
<212> DNA
<213> Artificial Sequence

<220>
<223> Synthetic Construct

<400> 996
cactctagag gagtcataag cc 22

<210> 997
<211> 22
<212> DNA

<213> Artificial Sequence

<220>
<223> Synthetic Construct

<400> 997
gaagtcattc ttgaagtgaa aa 22

<210> 998
<211> 21
<212> DNA
<213> Artificial Sequence

<220>
<223> Synthetic Construct

<400> 998
cattaacata aagagaggct g 21

<210> 999
<211> 18
<212> DNA
<213> Artificial Sequence

<220>
<223> Synthetic Construct

<400> 999
atcatccagg tggcttac 18

<210> 1000
<211> 18
<212> DNA
<213> Artificial Sequence

<220>
<223> Synthetic Construct

<400> 1000
tgtccatgag gtcctctc 18

<210> 1001
<211> 19
<212> DNA
<213> Artificial Sequence

<220>
<223> Synthetic Construct

<400> 1001
ggattcctag gaggccaaa 19

<210> 1002
<211> 18
<212> DNA
<213> Artificial Sequence

<220>

<223> Synthetic Construct

<400> 1002
ttgatgggac tctctcca 18

<210> 1003
<211> 23
<212> DNA
<213> Artificial Sequence

<220>
<223> Synthetic Construct

<400> 1003
ctcataattc tcgaggcatt gaa 23

<210> 1004
<211> 22
<212> DNA
<213> Artificial Sequence

<220>
<223> Synthetic Construct

<400> 1004
ttaagctttg ttttgctgta ac 22

<210> 1005
<211> 20
<212> DNA
<213> Artificial Sequence

<220>
<223> Synthetic Construct

<400> 1005
aaaggcacag tgggtataaa 20

<210> 1006
<211> 22
<212> DNA
<213> Artificial Sequence

<220>
<223> Synthetic Construct

<400> 1006
tggtttgctt tgtttctgac aa 22

<210> 1007
<211> 23
<212> DNA
<213> Artificial Sequence

<220>
<223> Synthetic Construct

<400> 1007

atagtaaagg aagttctcca ggc 23

<210> 1008
<211> 21
<212> DNA
<213> Artificial Sequence

<220>
<223> Synthetic Construct

<400> 1008
tttagcattt ctagtgctgt t 21

<210> 1009
<211> 20
<212> DNA
<213> Artificial Sequence

<220>
<223> Synthetic Construct

<400> 1009
aacccaagca aaaggtaagg 20

<210> 1010
<211> 23
<212> DNA
<213> Artificial Sequence

<220>
<223> Synthetic Construct

<400> 1010
actggtaaca tgtatttggg tct 23

<210> 1011
<211> 19
<212> DNA
<213> Artificial Sequence

<220>
<223> Synthetic Construct

<400> 1011
attcacaaat ccaatcctg 19

<210> 1012
<211> 19
<212> DNA
<213> Artificial Sequence

<220>
<223> Synthetic Construct

<400> 1012
tttcagggat aaagcccat 19

<210> 1013
<211> 19
<212> DNA
<213> Artificial Sequence

<220>
<223> Synthetic Construct

<400> 1013
accagaaatc tggaggtga 19

<210> 1014
<211> 19
<212> DNA
<213> Artificial Sequence

<220>
<223> Synthetic Construct

<400> 1014
ccagtagctt ctgttccat 19

<210> 1015
<211> 20
<212> DNA
<213> Artificial Sequence

<220>
<223> Synthetic Construct

<400> 1015
ttttgtggat tttacttgga 20

<210> 1016
<211> 18
<212> DNA
<213> Artificial Sequence

<220>
<223> Synthetic Construct

<400> 1016
tttcctcccc tgccagaa 18

<210> 1017
<211> 19
<212> DNA
<213> Artificial Sequence

<220>
<223> Synthetic Construct

<400> 1017
ttagggaaac ccaaagaca 19

<210> 1018
<211> 22
<212> DNA

<213> Artificial Sequence

<220>
<223> Synthetic Construct

<400> 1018
ctatagctgc agatgccaga gc 22

<210> 1019
<211> 20
<212> DNA
<213> Artificial Sequence

<220>
<223> Synthetic Construct

<400> 1019
caaattaagc atcacatcca 20

<210> 1020
<211> 22
<212> DNA
<213> Artificial Sequence

<220>
<223> Synthetic Construct

<400> 1020
gaaattctgt ctgataattc ca 22

<210> 1021
<211> 20
<212> DNA
<213> Artificial Sequence

<220>
<223> Synthetic Construct

<400> 1021
aggtgatgtc actcagcaac 20

<210> 1022
<211> 20
<212> DNA
<213> Artificial Sequence

<220>
<223> Synthetic Construct

<400> 1022
gacaaaagca agcctgtggt 20

<210> 1023
<211> 23
<212> DNA
<213> Artificial Sequence

<220>

<223> Synthetic Construct

<400> 1023
tgctagcact aatcagaaga agg 23

<210> 1024
<211> 20
<212> DNA
<213> Artificial Sequence

<220>
<223> Synthetic Construct

<400> 1024
ttctgaagtc tgctttgtct 20

<210> 1025
<211> 20
<212> DNA
<213> Artificial Sequence

<220>
<223> Synthetic Construct

<400> 1025
gtgccaaata acaatgaatc 20

<210> 1026
<211> 21
<212> DNA
<213> Artificial Sequence

<220>
<223> Synthetic Construct

<400> 1026
gggttgtaaa agtctgcaag t 21

<210> 1027
<211> 23
<212> DNA
<213> Artificial Sequence

<220>
<223> Synthetic Construct

<400> 1027
tcatgtctaa cttttacttg agg 23

<210> 1028
<211> 19
<212> DNA
<213> Artificial Sequence

<220>
<223> Synthetic Construct

<400> 1028

tcttctgctt cttttaggc 19

<210> 1029
<211> 20
<212> DNA
<213> Artificial Sequence

<220>
<223> Synthetic Construct

<400> 1029
ttatttacca ggaccaagtg 20

<210> 1030
<211> 18
<212> DNA
<213> Artificial Sequence

<220>
<223> Synthetic Construct

<400> 1030
ggggttgcct taattgat 18

<210> 1031
<211> 23
<212> DNA
<213> Artificial Sequence

<220>
<223> Synthetic Construct

<400> 1031
gatcaatgga atgtgataga gtg 23

<210> 1032
<211> 20
<212> DNA
<213> Artificial Sequence

<220>
<223> Synthetic Construct

<400> 1032
tgaactgcct ttgttccttt 20

<210> 1033
<211> 18
<212> DNA
<213> Artificial Sequence

<220>
<223> Synthetic Construct

<400> 1033
tggccaaatg ctagtgat 18

<210> 1034
<211> 22
<212> DNA
<213> Artificial Sequence

<220>
<223> Synthetic Construct

<400> 1034
aagcttctta aggagataaa ca 22

<210> 1035
<211> 19
<212> DNA
<213> Artificial Sequence

<220>
<223> Synthetic Construct

<400> 1035
ctccacccta ccccagcct 19

<210> 1036
<211> 20
<212> DNA
<213> Artificial Sequence

<220>
<223> Synthetic Construct

<400> 1036
gagagagcta gagagccaga 20

<210> 1037
<211> 19
<212> DNA
<213> Artificial Sequence

<220>
<223> Synthetic Construct

<400> 1037
gagggtatcc caggaccgt 19

<210> 1038
<211> 20
<212> DNA
<213> Artificial Sequence

<220>
<223> Synthetic Construct

<400> 1038
tcaggacagt ttgtgctccc 20

<210> 1039
<211> 18
<212> DNA

<213> Artificial Sequence

<220>
<223> Synthetic Construct

<400> 1039
ccctcgttgt gcctgaag 18

<210> 1040
<211> 20
<212> DNA
<213> Artificial Sequence

<220>
<223> Synthetic Construct

<400> 1040
acaggctttt ggtcgtaagg 20

<210> 1041
<211> 18
<212> DNA
<213> Artificial Sequence

<220>
<223> Synthetic Construct

<400> 1041
cacaggagca gcagaggg 18

<210> 1042
<211> 19
<212> DNA
<213> Artificial Sequence

<220>
<223> Synthetic Construct

<400> 1042
gagtctcgct ctggagaaa 19

<210> 1043
<211> 20
<212> DNA
<213> Artificial Sequence

<220>
<223> Synthetic Construct

<400> 1043
gggaggtgca gatctcttag 20

<210> 1044
<211> 18
<212> DNA
<213> Artificial Sequence

<220>

<223> Synthetic Construct

<400> 1044
acctaggtct ggctcatc 18

<210> 1045
<211> 20
<212> DNA
<213> Artificial Sequence

<220>
<223> Synthetic Construct

<400> 1045
aggctagaaa actaatgcca 20

<210> 1046
<211> 24
<212> DNA
<213> Artificial Sequence

<220>
<223> Synthetic Construct

<400> 1046
tgaaccttgt aacaaattcc agta 24

<210> 1047
<211> 18
<212> DNA
<213> Artificial Sequence

<220>
<223> Synthetic Construct

<400> 1047
aggcccatgg ccaatatc 18

<210> 1048
<211> 18
<212> DNA
<213> Artificial Sequence

<220>
<223> Synthetic Construct

<400> 1048
gctgtgcatg acaatgct 18

<210> 1049
<211> 19
<212> DNA
<213> Artificial Sequence

<220>
<223> Synthetic Construct

<400> 1049

ggaagcattt tgggagtta 19

<210> 1050
<211> 18
<212> DNA
<213> Artificial Sequence

<220>
<223> Synthetic Construct

<400> 1050
ccagacaagg gagaagtc 18

<210> 1051
<211> 18
<212> DNA
<213> Artificial Sequence

<220>
<223> Synthetic Construct

<400> 1051
agttcaccct cgatgtgc 18

<210> 1052
<211> 18
<212> DNA
<213> Artificial Sequence

<220>
<223> Synthetic Construct

<400> 1052
ggggctgttg gagatgag 18

<210> 1053
<211> 18
<212> DNA
<213> Artificial Sequence

<220>
<223> Synthetic Construct

<400> 1053
gaaagggcca ggagctga 18

<210> 1054
<211> 18
<212> DNA
<213> Artificial Sequence

<220>
<223> Synthetic Construct

<400> 1054
aaagccaccc ctgcagta 18

<210> 1055
<211> 20
<212> DNA
<213> Artificial Sequence

<220>
<223> Synthetic Construct

<400> 1055
gtggcctatc aggtctgtct 20

<210> 1056
<211> 18
<212> DNA
<213> Artificial Sequence

<220>
<223> Synthetic Construct

<400> 1056
ccatggtttg ggtttaca 18

<210> 1057
<211> 21
<212> DNA
<213> Artificial Sequence

<220>
<223> Synthetic Construct

<400> 1057
cagtttggtg ccttagatgt c 21

<210> 1058
<211> 22
<212> DNA
<213> Artificial Sequence

<220>
<223> Synthetic Construct

<400> 1058
caggatagag tcctagaagt gg 22

<210> 1059
<211> 18
<212> DNA
<213> Artificial Sequence

<220>
<223> Synthetic Construct

<400> 1059
ggacctggcc agcacttt 18

<210> 1060
<211> 19
<212> DNA

<213> Artificial Sequence

<220>
<223> Synthetic Construct

<400> 1060
ttagggcccc aagcttaaa 19

<210> 1061
<211> 19
<212> DNA
<213> Artificial Sequence

<220>
<223> Synthetic Construct

<400> 1061
ctgagtttta agtgccaca 19

<210> 1062
<211> 22
<212> DNA
<213> Artificial Sequence

<220>
<223> Synthetic Construct

<400> 1062
aagtacaagt ctgagagcct aa 22

<210> 1063
<211> 24
<212> DNA
<213> Artificial Sequence

<220>
<223> Synthetic Construct

<400> 1063
gtgtgtgaca ttttagagtt agat 24

<210> 1064
<211> 20
<212> DNA
<213> Artificial Sequence

<220>
<223> Synthetic Construct

<400> 1064
tcctgttgat tcctacattc 20

<210> 1065
<211> 20
<212> DNA
<213> Artificial Sequence

<220>

<223>  Synthetic Construct

<400>  1065
gcagaattga tgcaactaca                                                   20

<210>  1066
<211>  20
<212>  DNA
<213>  Artificial Sequence

<220>
<223>  Synthetic Construct

<400>  1066
ctgaaagact tccatttctg                                                   20

<210>  1067
<211>  21
<212>  DNA
<213>  Artificial Sequence

<220>
<223>  Synthetic Construct

<400>  1067
gaaactaagt gacctgcttc t                                                 21

<210>  1068
<211>  19
<212>  DNA
<213>  Artificial Sequence

<220>
<223>  Synthetic Construct

<400>  1068
ggtttggatg atgtgttgc                                                    19

<210>  1069
<211>  19
<212>  DNA
<213>  Artificial Sequence

<220>
<223>  Synthetic Construct

<400>  1069
aaagatcagt aagcggtgc                                                    19

<210>  1070
<211>  21
<212>  DNA
<213>  Artificial Sequence

<220>
<223>  Synthetic Construct

<400>  1070

gttaggcagg tgctttctac a 21

<210> 1071
<211> 20
<212> DNA
<213> Artificial Sequence

<220>
<223> Synthetic Construct

<400> 1071
gtgttttacc agtgctcccc 20

<210> 1072
<211> 20
<212> DNA
<213> Artificial Sequence

<220>
<223> Synthetic Construct

<400> 1072
gaacctacct ctgggttgga 20

<210> 1073
<211> 18
<212> DNA
<213> Artificial Sequence

<220>
<223> Synthetic Construct

<400> 1073
aggcacatgg ggcttcct 18

<210> 1074
<211> 18
<212> DNA
<213> Artificial Sequence

<220>
<223> Synthetic Construct

<400> 1074
cctaaggctc ttccattg 18

<210> 1075
<211> 22
<212> DNA
<213> Artificial Sequence

<220>
<223> Synthetic Construct

<400> 1075
gacatctaga tatgggaaaa ca 22

<210> 1076
<211> 19
<212> DNA
<213> Artificial Sequence

<220>
<223> Synthetic Construct

<400> 1076
tccaaggatt ggaggacac 19

<210> 1077
<211> 18
<212> DNA
<213> Artificial Sequence

<220>
<223> Synthetic Construct

<400> 1077
aggtgggaat gggaatgg 18

<210> 1078
<211> 20
<212> DNA
<213> Artificial Sequence

<220>
<223> Synthetic Construct

<400> 1078
ttagcaccat aatccatgtg 20

<210> 1079
<211> 19
<212> DNA
<213> Artificial Sequence

<220>
<223> Synthetic Construct

<400> 1079
aggactgctg ccctttcta 19

<210> 1080
<211> 21
<212> DNA
<213> Artificial Sequence

<220>
<223> Synthetic Construct

<400> 1080
ctgtaaacag tggtccatta g 21

<210> 1081
<211> 21
<212> DNA

<213>  Artificial Sequence

<220>
<223>  Synthetic Construct

<400>  1081
tcagaccaca agatagtgaa t                                                  21

<210>  1082
<211>  20
<212>  DNA
<213>  Artificial Sequence

<220>
<223>  Synthetic Construct

<400>  1082
ctctccaggc tgggcacata                                                    20

<210>  1083
<211>  20
<212>  DNA
<213>  Artificial Sequence

<220>
<223>  Synthetic Construct

<400>  1083
ttcagtcacc ttcaactgat                                                    20

<210>  1084
<211>  18
<212>  DNA
<213>  Artificial Sequence

<220>
<223>  Synthetic Construct

<400>  1084
tttgagtaaa gcattggg                                                      18

<210>  1085
<211>  18
<212>  DNA
<213>  Artificial Sequence

<220>
<223>  Synthetic Construct

<400>  1085
gctgtgcatt caccatgt                                                      18

<210>  1086
<211>  19
<212>  DNA
<213>  Artificial Sequence

<220>

<223> Synthetic Construct

<400> 1086
ttctatgggg aaatcaggc 19

<210> 1087
<211> 20
<212> DNA
<213> Artificial Sequence

<220>
<223> Synthetic Construct

<400> 1087
cctttatggg aggaggagtt 20

<210> 1088
<211> 20
<212> DNA
<213> Artificial Sequence

<220>
<223> Synthetic Construct

<400> 1088
atgctgatca aggcagtttt 20

<210> 1089
<211> 19
<212> DNA
<213> Artificial Sequence

<220>
<223> Synthetic Construct

<400> 1089
gaagaggcac aaccaagat 19

<210> 1090
<211> 20
<212> DNA
<213> Artificial Sequence

<220>
<223> Synthetic Construct

<400> 1090
tgtgaccttg agagctaaga 20

<210> 1091
<211> 20
<212> DNA
<213> Artificial Sequence

<220>
<223> Synthetic Construct

<400> 1091

cttgttgaca gctttctctc 20

<210> 1092
<211> 18
<212> DNA
<213> Artificial Sequence

<220>
<223> Synthetic Construct

<400> 1092
agaaatccac tggagctg 18

<210> 1093
<211> 20
<212> DNA
<213> Artificial Sequence

<220>
<223> Synthetic Construct

<400> 1093
cacaaggaca tcctgaagcc 20

<210> 1094
<211> 18
<212> DNA
<213> Artificial Sequence

<220>
<223> Synthetic Construct

<400> 1094
tctgcggagg ttttccct 18

<210> 1095
<211> 20
<212> DNA
<213> Artificial Sequence

<220>
<223> Synthetic Construct

<400> 1095
ctacctgttc cagtccttcc 20

<210> 1096
<211> 19
<212> DNA
<213> Artificial Sequence

<220>
<223> Synthetic Construct

<400> 1096
gtgttccagc tgtggttgc 19

<210> 1097
<211> 20
<212> DNA
<213> Artificial Sequence

<220>
<223> Synthetic Construct

<400> 1097
tggttgatct ttacccatcc 20

<210> 1098
<211> 18
<212> DNA
<213> Artificial Sequence

<220>
<223> Synthetic Construct

<400> 1098
atgctacaca cgaaggcg 18

<210> 1099
<211> 18
<212> DNA
<213> Artificial Sequence

<220>
<223> Synthetic Construct

<400> 1099
ctggagcctg gccctttc 18

<210> 1100
<211> 18
<212> DNA
<213> Artificial Sequence

<220>
<223> Synthetic Construct

<400> 1100
cagaggcgga tgacgatg 18

<210> 1101
<211> 18
<212> DNA
<213> Artificial Sequence

<220>
<223> Synthetic Construct

<400> 1101
agcaacagtc accctcag 18

<210> 1102
<211> 18
<212> DNA

<213> Artificial Sequence

<220>
<223> Synthetic Construct

<400> 1102
gcacagaagt cgtggcct 18

<210> 1103
<211> 20
<212> DNA
<213> Artificial Sequence

<220>
<223> Synthetic Construct

<400> 1103
acttttgaat gccgcaattt 20

<210> 1104
<211> 24
<212> DNA
<213> Artificial Sequence

<220>
<223> Synthetic Construct

<400> 1104
cactacttgt actgctgaca tcca 24

<210> 1105
<211> 22
<212> DNA
<213> Artificial Sequence

<220>
<223> Synthetic Construct

<400> 1105
agggttacat attcccctgt tt 22

<210> 1106
<211> 22
<212> DNA
<213> Artificial Sequence

<220>
<223> Synthetic Construct

<400> 1106
agaactaata attgctaacc ca 22

<210> 1107
<211> 22
<212> DNA
<213> Artificial Sequence

<220>

<223> Synthetic Construct

<400> 1107
gcaaacatta tacacacaat gg 22

<210> 1108
<211> 21
<212> DNA
<213> Artificial Sequence

<220>
<223> Synthetic Construct

<400> 1108
ttgtctgtgt acttgtgctc t 21

<210> 1109
<211> 20
<212> DNA
<213> Artificial Sequence

<220>
<223> Synthetic Construct

<400> 1109
aaggtcttcc atccttctta 20

<210> 1110
<211> 20
<212> DNA
<213> Artificial Sequence

<220>
<223> Synthetic Construct

<400> 1110
ttattcccaa ttaacttcca 20

<210> 1111
<211> 18
<212> DNA
<213> Artificial Sequence

<220>
<223> Synthetic Construct

<400> 1111
acacaaggcc cagcaatc 18

<210> 1112
<211> 22
<212> DNA
<213> Artificial Sequence

<220>
<223> Synthetic Construct

<400> 1112

gataaggttg acgtggtcaa ag 22

<210> 1113
<211> 19
<212> DNA
<213> Artificial Sequence

<220>
<223> Synthetic Construct

<400> 1113
tacaatttc cacagcagc 19

<210> 1114
<211> 19
<212> DNA
<213> Artificial Sequence

<220>
<223> Synthetic Construct

<400> 1114
caaaaggtaa gagttggca 19

<210> 1115
<211> 18
<212> DNA
<213> Artificial Sequence

<220>
<223> Synthetic Construct

<400> 1115
aagtgctgag ggatgggg 18

<210> 1116
<211> 18
<212> DNA
<213> Artificial Sequence

<220>
<223> Synthetic Construct

<400> 1116
tgctatggtg aacagagg 18

<210> 1117
<211> 19
<212> DNA
<213> Artificial Sequence

<220>
<223> Synthetic Construct

<400> 1117
tttctgacag gtcgtaggg 19

<210> 1118
<211> 22
<212> DNA
<213> Artificial Sequence

<220>
<223> Synthetic Construct

<400> 1118
acatcatgat aggatgagca ac 22

<210> 1119
<211> 18
<212> DNA
<213> Artificial Sequence

<220>
<223> Synthetic Construct

<400> 1119
tcgggtgaaa gaacatgg 18

<210> 1120
<211> 18
<212> DNA
<213> Artificial Sequence

<220>
<223> Synthetic Construct

<400> 1120
agggctgagc atcccatc 18

<210> 1121
<211> 23
<212> DNA
<213> Artificial Sequence

<220>
<223> Synthetic Construct

<400> 1121
ttggctatga agaatgtatt gag 23

<210> 1122
<211> 20
<212> DNA
<213> Artificial Sequence

<220>
<223> Synthetic Construct

<400> 1122
gaaagggttt ccaggtcaac 20

<210> 1123
<211> 21
<212> DNA

<213> Artificial Sequence

<220>
<223> Synthetic Construct

<400> 1123
gcaaggcaag aacaatatcc a 21

<210> 1124
<211> 19
<212> DNA
<213> Artificial Sequence

<220>
<223> Synthetic Construct

<400> 1124
ttttcttgat ctggcattt 19

<210> 1125
<211> 18
<212> DNA
<213> Artificial Sequence

<220>
<223> Synthetic Construct

<400> 1125
aggatgcatg tggggatg 18

<210> 1126
<211> 21
<212> DNA
<213> Artificial Sequence

<220>
<223> Synthetic Construct

<400> 1126
tattttcctt gaatgctaca c 21

<210> 1127
<211> 20
<212> DNA
<213> Artificial Sequence

<220>
<223> Synthetic Construct

<400> 1127
caatttatac cacactgcaa 20

<210> 1128
<211> 22
<212> DNA
<213> Artificial Sequence

<220>

<223> Synthetic Construct

<400> 1128
gagacttgtt ttatggttca gc 22

<210> 1129
<211> 19
<212> DNA
<213> Artificial Sequence

<220>
<223> Synthetic Construct

<400> 1129
gtcaattgca aatggtttt 19

<210> 1130
<211> 20
<212> DNA
<213> Artificial Sequence

<220>
<223> Synthetic Construct

<400> 1130
catgtagaaa cacagaccca 20

<210> 1131
<211> 22
<212> DNA
<213> Artificial Sequence

<220>
<223> Synthetic Construct

<400> 1131
ccagagatca gggagttgta gg 22

<210> 1132
<211> 19
<212> DNA
<213> Artificial Sequence

<220>
<223> Synthetic Construct

<400> 1132
ctgaactggg caccaagag 19

<210> 1133
<211> 18
<212> DNA
<213> Artificial Sequence

<220>
<223> Synthetic Construct

<400> 1133

cctcctaatg gcaagtca 18

<210> 1134
<211> 22
<212> DNA
<213> Artificial Sequence

<220>
<223> Synthetic Construct

<400> 1134
tgctgtactt taccagaagc at 22

<210> 1135
<211> 20
<212> DNA
<213> Artificial Sequence

<220>
<223> Synthetic Construct

<400> 1135
ctgccccaca gagttgcagt 20

<210> 1136
<211> 22
<212> DNA
<213> Artificial Sequence

<220>
<223> Synthetic Construct

<400> 1136
tgctctaact gccttacata tt 22

<210> 1137
<211> 19
<212> DNA
<213> Artificial Sequence

<220>
<223> Synthetic Construct

<400> 1137
agcccatgaa ggcttccaa 19

<210> 1138
<211> 20
<212> DNA
<213> Artificial Sequence

<220>
<223> Synthetic Construct

<400> 1138
gtctctgacc tagctccctt 20

<210> 1139
<211> 19
<212> DNA
<213> Artificial Sequence

<220>
<223> Synthetic Construct

<400> 1139
ggagggccct atcttgtga 19

<210> 1140
<211> 22
<212> DNA
<213> Artificial Sequence

<220>
<223> Synthetic Construct

<400> 1140
gcagagtata tgaccaggtg ga 22

<210> 1141
<211> 20
<212> DNA
<213> Artificial Sequence

<220>
<223> Synthetic Construct

<400> 1141
ggttgaagat tgtgaactgc 20

<210> 1142
<211> 18
<212> DNA
<213> Artificial Sequence

<220>
<223> Synthetic Construct

<400> 1142
attgatgtga gtgagggc 18

<210> 1143
<211> 19
<212> DNA
<213> Artificial Sequence

<220>
<223> Synthetic Construct

<400> 1143
acactcaaca tttcgggga 19

<210> 1144
<211> 19
<212> DNA

<213> Artificial Sequence

<220>
<223> Synthetic Construct

<400> 1144
gaagttccag atgactcca 19

<210> 1145
<211> 21
<212> DNA
<213> Artificial Sequence

<220>
<223> Synthetic Construct

<400> 1145
gcatcagtgc aatctgctac a 21

<210> 1146
<211> 18
<212> DNA
<213> Artificial Sequence

<220>
<223> Synthetic Construct

<400> 1146
gaaatcctcg gcgctctt 18

<210> 1147
<211> 18
<212> DNA
<213> Artificial Sequence

<220>
<223> Synthetic Construct

<400> 1147
taaggacgct cgagactg 18

<210> 1148
<211> 18
<212> DNA
<213> Artificial Sequence

<220>
<223> Synthetic Construct

<400> 1148
tttacgctgt ccccattt 18

<210> 1149
<211> 22
<212> DNA
<213> Artificial Sequence

<220>

<223> Synthetic Construct

<400> 1149
gatggggaca tgatttgtaa ag 22

<210> 1150
<211> 20
<212> DNA
<213> Artificial Sequence

<220>
<223> Synthetic Construct

<400> 1150
aactccgctg cactgtatcc 20

<210> 1151
<211> 20
<212> DNA
<213> Artificial Sequence

<220>
<223> Synthetic Construct

<400> 1151
gcaagcaaag gacagtaaga 20

<210> 1152
<211> 19
<212> DNA
<213> Artificial Sequence

<220>
<223> Synthetic Construct

<400> 1152
tggatctcct ccttcagca 19

<210> 1153
<211> 18
<212> DNA
<213> Artificial Sequence

<220>
<223> Synthetic Construct

<400> 1153
caatttgggc actgtggt 18

<210> 1154
<211> 22
<212> DNA
<213> Artificial Sequence

<220>
<223> Synthetic Construct

<400> 1154

cttgctgtct atttgttacc tg 22

<210> 1155
<211> 21
<212> DNA
<213> Artificial Sequence

<220>
<223> Synthetic Construct

<400> 1155
gttgacaagt tgcttgtagt t 21

<210> 1156
<211> 23
<212> DNA
<213> Artificial Sequence

<220>
<223> Synthetic Construct

<400> 1156
agattcttaa gctcttgatg ata 23

<210> 1157
<211> 20
<212> DNA
<213> Artificial Sequence

<220>
<223> Synthetic Construct

<400> 1157
tttgttttcc atgtgaagtt 20

<210> 1158
<211> 20
<212> DNA
<213> Artificial Sequence

<220>
<223> Synthetic Construct

<400> 1158
ttctgatgcc aacacaaata 20

<210> 1159
<211> 21
<212> DNA
<213> Artificial Sequence

<220>
<223> Synthetic Construct

<400> 1159
tgctactgcc tttaagaaag a 21

<210> 1160
<211> 24
<212> DNA
<213> Artificial Sequence

<220>
<223> Synthetic Construct

<400> 1160
aagttagtca cagatttgtt ttgc 24

<210> 1161
<211> 22
<212> DNA
<213> Artificial Sequence

<220>
<223> Synthetic Construct

<400> 1161
tgatgtacaa aactttggat ga 22

<210> 1162
<211> 18
<212> DNA
<213> Artificial Sequence

<220>
<223> Synthetic Construct

<400> 1162
gagttggtgg attttcct 18

<210> 1163
<211> 20
<212> DNA
<213> Artificial Sequence

<220>
<223> Synthetic Construct

<400> 1163
gacatcatcc attcaacacc 20

<210> 1164
<211> 19
<212> DNA
<213> Artificial Sequence

<220>
<223> Synthetic Construct

<400> 1164
tgcttagtgc ttggctaat 19

<210> 1165
<211> 20
<212> DNA

<213> Artificial Sequence

<220>
<223> Synthetic Construct

<400> 1165
gcaggtaata caccatcaat 20

<210> 1166
<211> 24
<212> DNA
<213> Artificial Sequence

<220>
<223> Synthetic Construct

<400> 1166
caaattttaa actgagtgag agtc 24

<210> 1167
<211> 22
<212> DNA
<213> Artificial Sequence

<220>
<223> Synthetic Construct

<400> 1167
aaaacttcag attaagaacc ac 22

<210> 1168
<211> 23
<212> DNA
<213> Artificial Sequence

<220>
<223> Synthetic Construct

<400> 1168
tgagacttct aggtcttagg tta 23

<210> 1169
<211> 23
<212> DNA
<213> Artificial Sequence

<220>
<223> Synthetic Construct

<400> 1169
ttccatctct aagtcaaatt ggt 23

<210> 1170
<211> 21
<212> DNA
<213> Artificial Sequence

<220>

<223>  Synthetic Construct

<400>  1170
gcaatggttt tgtgtagcat c                                                  21

<210>  1171
<211>  23
<212>  DNA
<213>  Artificial Sequence

<220>
<223>  Synthetic Construct

<400>  1171
gttctactta atcactgact ggt                                                23

<210>  1172
<211>  24
<212>  DNA
<213>  Artificial Sequence

<220>
<223>  Synthetic Construct

<400>  1172
gatcatagtc ttaggagttc attt                                               24

<210>  1173
<211>  23
<212>  DNA
<213>  Artificial Sequence

<220>
<223>  Synthetic Construct

<400>  1173
aagggtttga aatatagctg ttc                                                23

<210>  1174
<211>  18
<212>  DNA
<213>  Artificial Sequence

<220>
<223>  Synthetic Construct

<400>  1174
atggccattc aatacgat                                                      18

<210>  1175
<211>  19
<212>  DNA
<213>  Artificial Sequence

<220>
<223>  Synthetic Construct

<400>  1175

atcaaattgg cttacttgc 19

<210> 1176
<211> 18
<212> DNA
<213> Artificial Sequence

<220>
<223> Synthetic Construct

<400> 1176
aggacttaag gggccaac 18

<210> 1177
<211> 20
<212> DNA
<213> Artificial Sequence

<220>
<223> Synthetic Construct

<400> 1177
ttccactagc aagaatggtt 20

<210> 1178
<211> 20
<212> DNA
<213> Artificial Sequence

<220>
<223> Synthetic Construct

<400> 1178
actgttggac tgcggctaag 20

<210> 1179
<211> 22
<212> DNA
<213> Artificial Sequence

<220>
<223> Synthetic Construct

<400> 1179
aatcttagga gaaaagtgtt ca 22

<210> 1180
<211> 22
<212> DNA
<213> Artificial Sequence

<220>
<223> Synthetic Construct

<400> 1180
aaggggaatt ttctcacctc aa 22

<210> 1181
<211> 21
<212> DNA
<213> Artificial Sequence

<220>
<223> Synthetic Construct

<400> 1181
aattagggat cagaatctca a 21

<210> 1182
<211> 20
<212> DNA
<213> Artificial Sequence

<220>
<223> Synthetic Construct

<400> 1182
ctggaaatta atacaagggg 20

<210> 1183
<211> 19
<212> DNA
<213> Artificial Sequence

<220>
<223> Synthetic Construct

<400> 1183
ctctatatgc atgttgcca 19

<210> 1184
<211> 18
<212> DNA
<213> Artificial Sequence

<220>
<223> Synthetic Construct

<400> 1184
gtccttaggc acaaatgg 18

<210> 1185
<211> 20
<212> DNA
<213> Artificial Sequence

<220>
<223> Synthetic Construct

<400> 1185
agtgttcatt ccatcttgag 20

<210> 1186
<211> 20
<212> DNA

<213>  Artificial Sequence

<220>
<223>  Synthetic Construct

<400>  1186
gcaacactaa ggtaactggc                                                    20

<210>  1187
<211>  18
<212>  DNA
<213>  Artificial Sequence

<220>
<223>  Synthetic Construct

<400>  1187
tgtccatact ggcccttt                                                      18

<210>  1188
<211>  20
<212>  DNA
<213>  Artificial Sequence

<220>
<223>  Synthetic Construct

<400>  1188
ttaacagcag caaacagatg                                                    20

<210>  1189
<211>  19
<212>  DNA
<213>  Artificial Sequence

<220>
<223>  Synthetic Construct

<400>  1189
tagaaggcca cacaatgcc                                                     19

<210>  1190
<211>  20
<212>  DNA
<213>  Artificial Sequence

<220>
<223>  Synthetic Construct

<400>  1190
agtcccagat gaaggggttt                                                    20

<210>  1191
<211>  19
<212>  DNA
<213>  Artificial Sequence

<220>

<223> Synthetic Construct

<400> 1191
cattcctggc ctgagaaca 19

<210> 1192
<211> 22
<212> DNA
<213> Artificial Sequence

<220>
<223> Synthetic Construct

<400> 1192
ttcacattta ccaactactg aa 22

<210> 1193
<211> 21
<212> DNA
<213> Artificial Sequence

<220>
<223> Synthetic Construct

<400> 1193
acctgaccca ctttaactta g 21

<210> 1194
<211> 18
<212> DNA
<213> Artificial Sequence

<220>
<223> Synthetic Construct

<400> 1194
tgtcaggttg ttgactgc 18

<210> 1195
<211> 18
<212> DNA
<213> Artificial Sequence

<220>
<223> Synthetic Construct

<400> 1195
ggaacacagc tcccttat 18

<210> 1196
<211> 24
<212> DNA
<213> Artificial Sequence

<220>
<223> Synthetic Construct

<400> 1196

agtagaacag attagattcc atgt 24

<210> 1197
<211> 21
<212> DNA
<213> Artificial Sequence

<220>
<223> Synthetic Construct

<400> 1197
gttattgcca tgattccatg t 21

<210> 1198
<211> 18
<212> DNA
<213> Artificial Sequence

<220>
<223> Synthetic Construct

<400> 1198
gtgggtgaac tgcttgcc 18

<210> 1199
<211> 20
<212> DNA
<213> Artificial Sequence

<220>
<223> Synthetic Construct

<400> 1199
gatgtgtcaa ggcattggat 20

<210> 1200
<211> 20
<212> DNA
<213> Artificial Sequence

<220>
<223> Synthetic Construct

<400> 1200
tgtgacttgg gcacctagaa 20

<210> 1201
<211> 18
<212> DNA
<213> Artificial Sequence

<220>
<223> Synthetic Construct

<400> 1201
caactgtgct tgccggat 18

<210> 1202
<211> 22
<212> DNA
<213> Artificial Sequence

<220>
<223> Synthetic Construct

<400> 1202
tgatcactaa tagaccactt ga 22

<210> 1203
<211> 20
<212> DNA
<213> Artificial Sequence

<220>
<223> Synthetic Construct

<400> 1203
ttgatcatcc ttcctaccac 20

<210> 1204
<211> 18
<212> DNA
<213> Artificial Sequence

<220>
<223> Synthetic Construct

<400> 1204
agatgccctc cttggaca 18

<210> 1205
<211> 20
<212> DNA
<213> Artificial Sequence

<220>
<223> Synthetic Construct

<400> 1205
tctcaggcct atgacttcaa 20

<210> 1206
<211> 22
<212> DNA
<213> Artificial Sequence

<220>
<223> Synthetic Construct

<400> 1206
tcaagtaaga caagtaccag ga 22

<210> 1207
<211> 19
<212> DNA

<213> Artificial Sequence

<220>
<223> Synthetic Construct

<400> 1207
aatgcccatt gccctactg 19

<210> 1208
<211> 20
<212> DNA
<213> Artificial Sequence

<220>
<223> Synthetic Construct

<400> 1208
attgggaaat catccatgtg 20

<210> 1209
<211> 19
<212> DNA
<213> Artificial Sequence

<220>
<223> Synthetic Construct

<400> 1209
ttggccagac cagatgtaa 19

<210> 1210
<211> 22
<212> DNA
<213> Artificial Sequence

<220>
<223> Synthetic Construct

<400> 1210
catctaatgt ctgaatagtg gg 22

<210> 1211
<211> 18
<212> DNA
<213> Artificial Sequence

<220>
<223> Synthetic Construct

<400> 1211
gtgactcatg gcccaagt 18

<210> 1212
<211> 20
<212> DNA
<213> Artificial Sequence

<220>

<223> Synthetic Construct

<400> 1212
ggtgcaacat aaagtcaaaa 20

<210> 1213
<211> 24
<212> DNA
<213> Artificial Sequence

<220>
<223> Synthetic Construct

<400> 1213
aaaggtagtt ctctaagtta ccaa 24

<210> 1214
<211> 20
<212> DNA
<213> Artificial Sequence

<220>
<223> Synthetic Construct

<400> 1214
aacataattt ggatgggtct 20

<210> 1215
<211> 21
<212> DNA
<213> Artificial Sequence

<220>
<223> Synthetic Construct

<400> 1215
tgccacaatg ttaataaaag g 21

<210> 1216
<211> 20
<212> DNA
<213> Artificial Sequence

<220>
<223> Synthetic Construct

<400> 1216
tttacagcaa atcggcctta 20

<210> 1217
<211> 20
<212> DNA
<213> Artificial Sequence

<220>
<223> Synthetic Construct

<400> 1217

ttccacccta tgtaagacct 20

<210> 1218
<211> 18
<212> DNA
<213> Artificial Sequence

<220>
<223> Synthetic Construct

<400> 1218
accattcgct attagccc 18

<210> 1219
<211> 22
<212> DNA
<213> Artificial Sequence

<220>
<223> Synthetic Construct

<400> 1219
cttgtaatgc tgtgtggaat ac 22

<210> 1220
<211> 23
<212> DNA
<213> Artificial Sequence

<220>
<223> Synthetic Construct

<400> 1220
aactaccatc cgtggactta cag 23

<210> 1221
<211> 18
<212> DNA
<213> Artificial Sequence

<220>
<223> Synthetic Construct

<400> 1221
tgctgtgcat atccaact 18

<210> 1222
<211> 22
<212> DNA
<213> Artificial Sequence

<220>
<223> Synthetic Construct

<400> 1222
cttaatgagt caccaagtta cc 22

<210> 1223
<211> 21
<212> DNA
<213> Artificial Sequence

<220>
<223> Synthetic Construct

<400> 1223
agttatttgt tggtaatggc a 21

<210> 1224
<211> 22
<212> DNA
<213> Artificial Sequence

<220>
<223> Synthetic Construct

<400> 1224
aggtccctat aggtgaatct tg 22

<210> 1225
<211> 19
<212> DNA
<213> Artificial Sequence

<220>
<223> Synthetic Construct

<400> 1225
aatgttgcct ccaaaaccc 19

<210> 1226
<211> 18
<212> DNA
<213> Artificial Sequence

<220>
<223> Synthetic Construct

<400> 1226
tgcatcttgc tgctctta 18

<210> 1227
<211> 18
<212> DNA
<213> Artificial Sequence

<220>
<223> Synthetic Construct

<400> 1227
acatgcccca tgtcactg 18

<210> 1228
<211> 18
<212> DNA

<213> Artificial Sequence

<220>
<223> Synthetic Construct

<400> 1228
gacatttgca tcagaggg 18

<210> 1229
<211> 18
<212> DNA
<213> Artificial Sequence

<220>
<223> Synthetic Construct

<400> 1229
aaatgcattc agtttcca 18

<210> 1230
<211> 19
<212> DNA
<213> Artificial Sequence

<220>
<223> Synthetic Construct

<400> 1230
tcagtcctca agttcacca 19

<210> 1231
<211> 20
<212> DNA
<213> Artificial Sequence

<220>
<223> Synthetic Construct

<400> 1231
ggtgaaagga gatctggaac 20

<210> 1232
<211> 18
<212> DNA
<213> Artificial Sequence

<220>
<223> Synthetic Construct

<400> 1232
atagaggcag cttgggct 18

<210> 1233
<211> 20
<212> DNA
<213> Artificial Sequence

<220>

<223> Synthetic Construct

<400> 1233
ggggcaccag gagtgtagat 20

<210> 1234
<211> 18
<212> DNA
<213> Artificial Sequence

<220>
<223> Synthetic Construct

<400> 1234
tattgctagg agcctgcc 18

<210> 1235
<211> 19
<212> DNA
<213> Artificial Sequence

<220>
<223> Synthetic Construct

<400> 1235
aatgctacag catgacaaa 19

<210> 1236
<211> 18
<212> DNA
<213> Artificial Sequence

<220>
<223> Synthetic Construct

<400> 1236
gcagcttctc tatccagg 18

<210> 1237
<211> 18
<212> DNA
<213> Artificial Sequence

<220>
<223> Synthetic Construct

<400> 1237
tctttgtgtc ccttgttg 18

<210> 1238
<211> 19
<212> DNA
<213> Artificial Sequence

<220>
<223> Synthetic Construct

<400> 1238

aaaaggttgg tgatgaaga 19

<210> 1239
<211> 21
<212> DNA
<213> Artificial Sequence

<220>
<223> Synthetic Construct

<400> 1239
ttctaaccta catgatccac a 21

<210> 1240
<211> 18
<212> DNA
<213> Artificial Sequence

<220>
<223> Synthetic Construct

<400> 1240
ttacatgcca cagctcag 18

<210> 1241
<211> 18
<212> DNA
<213> Artificial Sequence

<220>
<223> Synthetic Construct

<400> 1241
gctttaaacc agggttcc 18

<210> 1242
<211> 18
<212> DNA
<213> Artificial Sequence

<220>
<223> Synthetic Construct

<400> 1242
catctcaggc acatgcaa 18

<210> 1243
<211> 20
<212> DNA
<213> Artificial Sequence

<220>
<223> Synthetic Construct

<400> 1243
tcaccaactt ctttcttcaa 20

<210> 1244
<211> 18
<212> DNA
<213> Artificial Sequence

<220>
<223> Synthetic Construct

<400> 1244
caaggatcag cagccctc 18

<210> 1245
<211> 21
<212> DNA
<213> Artificial Sequence

<220>
<223> Synthetic Construct

<400> 1245
ttgcataatg aagagccatg t 21

<210> 1246
<211> 19
<212> DNA
<213> Artificial Sequence

<220>
<223> Synthetic Construct

<400> 1246
ttgctgatac tggtgcaaa 19

<210> 1247
<211> 20
<212> DNA
<213> Artificial Sequence

<220>
<223> Synthetic Construct

<400> 1247
caataagctt ggccagaaat 20

<210> 1248
<211> 22
<212> DNA
<213> Artificial Sequence

<220>
<223> Synthetic Construct

<400> 1248
tgaagtctca tctctacttc gt 22

<210> 1249
<211> 19
<212> DNA

<213> Artificial Sequence

<220>
<223> Synthetic Construct

<400> 1249
gagctccaac tccaaacca 19

<210> 1250
<211> 19
<212> DNA
<213> Artificial Sequence

<220>
<223> Synthetic Construct

<400> 1250
ccctgactca gacgtggtg 19

<210> 1251
<211> 20
<212> DNA
<213> Artificial Sequence

<220>
<223> Synthetic Construct

<400> 1251
cagctaatgc cacatggtaa 20

<210> 1252
<211> 19
<212> DNA
<213> Artificial Sequence

<220>
<223> Synthetic Construct

<400> 1252
gtaacgtggc attgtcccc 19

<210> 1253
<211> 19
<212> DNA
<213> Artificial Sequence

<220>
<223> Synthetic Construct

<400> 1253
aacccttatc taggtgcca 19

<210> 1254
<211> 18
<212> DNA
<213> Artificial Sequence

<220>

<223> Synthetic Construct

<400> 1254
atgctgcctg gagggctt 18

<210> 1255
<211> 20
<212> DNA
<213> Artificial Sequence

<220>
<223> Synthetic Construct

<400> 1255
cttaaacccc tttaccccaa 20

<210> 1256
<211> 19
<212> DNA
<213> Artificial Sequence

<220>
<223> Synthetic Construct

<400> 1256
tggcctcaag ctcctcatc 19

<210> 1257
<211> 21
<212> DNA
<213> Artificial Sequence

<220>
<223> Synthetic Construct

<400> 1257
gaatctggtc tgcattgtat t 21

<210> 1258
<211> 18
<212> DNA
<213> Artificial Sequence

<220>
<223> Synthetic Construct

<400> 1258
gcaagctacc ccttgcag 18

<210> 1259
<211> 20
<212> DNA
<213> Artificial Sequence

<220>
<223> Synthetic Construct

<400> 1259

cattccacgt taggtgacaa 20

<210> 1260
<211> 21
<212> DNA
<213> Artificial Sequence

<220>
<223> Synthetic Construct

<400> 1260
gggtctacac cagattgctc t 21

<210> 1261
<211> 21
<212> DNA
<213> Artificial Sequence

<220>
<223> Synthetic Construct

<400> 1261
cacacagatt ctggtaaaga c 21

<210> 1262
<211> 20
<212> DNA
<213> Artificial Sequence

<220>
<223> Synthetic Construct

<400> 1262
aaaatggcag tctacatcat 20

<210> 1263
<211> 20
<212> DNA
<213> Artificial Sequence

<220>
<223> Synthetic Construct

<400> 1263
cacacaaaga atcagcatta 20

<210> 1264
<211> 19
<212> DNA
<213> Artificial Sequence

<220>
<223> Synthetic Construct

<400> 1264
tgagttaatg aatctgcca 19

<210> 1265
<211> 22
<212> DNA
<213> Artificial Sequence

<220>
<223> Synthetic Construct

<400> 1265
aatggattat gaagttatag cc 22

<210> 1266
<211> 20
<212> DNA
<213> Artificial Sequence

<220>
<223> Synthetic Construct

<400> 1266
ttgggtgtag ctctagtttg 20

<210> 1267
<211> 19
<212> DNA
<213> Artificial Sequence

<220>
<223> Synthetic Construct

<400> 1267
gtgaggctat ttctccctg 19

<210> 1268
<211> 18
<212> DNA
<213> Artificial Sequence

<220>
<223> Synthetic Construct

<400> 1268
gtcccttgcc ctgcagtt 18

<210> 1269
<211> 19
<212> DNA
<213> Artificial Sequence

<220>
<223> Synthetic Construct

<400> 1269
gttccctcca caaagtttc 19

<210> 1270
<211> 18
<212> DNA

<213>  Artificial Sequence

<220>
<223>  Synthetic Construct

<400>  1270
ggagttgtga cagttgcc                                                    18

<210>  1271
<211>  21
<212>  DNA
<213>  Artificial Sequence

<220>
<223>  Synthetic Construct

<400>  1271
ctaaatcact ttcacaacca c                                                21

<210>  1272
<211>  21
<212>  DNA
<213>  Artificial Sequence

<220>
<223>  Synthetic Construct

<400>  1272
aataaacctc cattcataag g                                                21

<210>  1273
<211>  20
<212>  DNA
<213>  Artificial Sequence

<220>
<223>  Synthetic Construct

<400>  1273
ttgacattcc ttgatctttg                                                  20

<210>  1274
<211>  22
<212>  DNA
<213>  Artificial Sequence

<220>
<223>  Synthetic Construct

<400>  1274
cccttattca atattaggtt tg                                               22

<210>  1275
<211>  19
<212>  DNA
<213>  Artificial Sequence

<220>

<223> Synthetic Construct

<400> 1275
accagagatg actggggtg 19

<210> 1276
<211> 18
<212> DNA
<213> Artificial Sequence

<220>
<223> Synthetic Construct

<400> 1276
caggtcaggc tggttcag 18

<210> 1277
<211> 19
<212> DNA
<213> Artificial Sequence

<220>
<223> Synthetic Construct

<400> 1277
gaatactgca ggaagggtt 19

<210> 1278
<211> 24
<212> DNA
<213> Artificial Sequence

<220>
<223> Synthetic Construct

<400> 1278
gacactaata cagagtgtgt tcgc 24

<210> 1279
<211> 21
<212> DNA
<213> Artificial Sequence

<220>
<223> Synthetic Construct

<400> 1279
gtccaacatg ttcctaatac a 21

<210> 1280
<211> 19
<212> DNA
<213> Artificial Sequence

<220>
<223> Synthetic Construct

<400> 1280

tccttttgac cgtccaagt 19

<210> 1281
<211> 22
<212> DNA
<213> Artificial Sequence

<220>
<223> Synthetic Construct

<400> 1281
gaagctaagg tctccttctc aa 22

<210> 1282
<211> 20
<212> DNA
<213> Artificial Sequence

<220>
<223> Synthetic Construct

<400> 1282
tagacgctgg gtagatgcaa 20

<210> 1283
<211> 18
<212> DNA
<213> Artificial Sequence

<220>
<223> Synthetic Construct

<400> 1283
tcgcagaagc catgtccc 18

<210> 1284
<211> 18
<212> DNA
<213> Artificial Sequence

<220>
<223> Synthetic Construct

<400> 1284
tgcacttacg cttcagca 18

<210> 1285
<211> 20
<212> DNA
<213> Artificial Sequence

<220>
<223> Synthetic Construct

<400> 1285
atgatcactt ggaagatttg 20

<210> 1286
<211> 20
<212> DNA
<213> Artificial Sequence

<220>
<223> Synthetic Construct

<400> 1286
acaggtcatt gaaacagaca 20

<210> 1287
<211> 19
<212> DNA
<213> Artificial Sequence

<220>
<223> Synthetic Construct

<400> 1287
acagacatca cattagcca 19

<210> 1288
<211> 19
<212> DNA
<213> Artificial Sequence

<220>
<223> Synthetic Construct

<400> 1288
aggcccttct cattgtatc 19

<210> 1289
<211> 22
<212> DNA
<213> Artificial Sequence

<220>
<223> Synthetic Construct

<400> 1289
gacaggtgga taagtagcaa ca 22

<210> 1290
<211> 20
<212> DNA
<213> Artificial Sequence

<220>
<223> Synthetic Construct

<400> 1290
tgggttacca tttgtggttt 20

<210> 1291
<211> 19
<212> DNA

<213> Artificial Sequence

<220>
<223> Synthetic Construct

<400> 1291
tgtgtaccag ggacaaatg 19

<210> 1292
<211> 20
<212> DNA
<213> Artificial Sequence

<220>
<223> Synthetic Construct

<400> 1292
aaggcttgac aataatttgg 20

<210> 1293
<211> 18
<212> DNA
<213> Artificial Sequence

<220>
<223> Synthetic Construct

<400> 1293
catttcctca tcacaagc 18

<210> 1294
<211> 18
<212> DNA
<213> Artificial Sequence

<220>
<223> Synthetic Construct

<400> 1294
cagctgcttg taccctga 18

<210> 1295
<211> 21
<212> DNA
<213> Artificial Sequence

<220>
<223> Synthetic Construct

<400> 1295
catcattccc tatttgactg a 21

<210> 1296
<211> 22
<212> DNA
<213> Artificial Sequence

<220>

<223> Synthetic Construct

<400> 1296
cttgatagt atttgccact cc 22

<210> 1297
<211> 22
<212> DNA
<213> Artificial Sequence

<220>
<223> Synthetic Construct

<400> 1297
cagaatatct aaaaccccta ga 22

<210> 1298
<211> 22
<212> DNA
<213> Artificial Sequence

<220>
<223> Synthetic Construct

<400> 1298
gtggatttgg aaaactcaaa ca 22

<210> 1299
<211> 18
<212> DNA
<213> Artificial Sequence

<220>
<223> Synthetic Construct

<400> 1299
atcccagacc cctcacct 18

<210> 1300
<211> 19
<212> DNA
<213> Artificial Sequence

<220>
<223> Synthetic Construct

<400> 1300
gagggagaat ggacagggc 19

<210> 1301
<211> 19
<212> DNA
<213> Artificial Sequence

<220>
<223> Synthetic Construct

<400> 1301

aggacctgac cctggctcc 19

<210> 1302
<211> 18
<212> DNA
<213> Artificial Sequence

<220>
<223> Synthetic Construct

<400> 1302
ggctaaaggg gaaggaag 18

<210> 1303
<211> 18
<212> DNA
<213> Artificial Sequence

<220>
<223> Synthetic Construct

<400> 1303
caatggcagt ctcccgtg 18

<210> 1304
<211> 21
<212> DNA
<213> Artificial Sequence

<220>
<223> Synthetic Construct

<400> 1304
tgggaattca tggataagca a 21

<210> 1305
<211> 19
<212> DNA
<213> Artificial Sequence

<220>
<223> Synthetic Construct

<400> 1305
acatcagcca gcacccatt 19

<210> 1306
<211> 20
<212> DNA
<213> Artificial Sequence

<220>
<223> Synthetic Construct

<400> 1306
aagggacttg atgggaaaca 20

<210> 1307
<211> 18
<212> DNA
<213> Artificial Sequence

<220>
<223> Synthetic Construct

<400> 1307
atttgaactc gcagcccc 18

<210> 1308
<211> 22
<212> DNA
<213> Artificial Sequence

<220>
<223> Synthetic Construct

<400> 1308
gaaaccatgg gaagttattg ac 22

<210> 1309
<211> 22
<212> DNA
<213> Artificial Sequence

<220>
<223> Synthetic Construct

<400> 1309
tgctttaagg tgtcaaaatt gc 22

<210> 1310
<211> 20
<212> DNA
<213> Artificial Sequence

<220>
<223> Synthetic Construct

<400> 1310
tgtgtagcag cagggtataa 20

<210> 1311
<211> 21
<212> DNA
<213> Artificial Sequence

<220>
<223> Synthetic Construct

<400> 1311
taagcacaaa ggttacagct a 21

<210> 1312
<211> 21
<212> DNA

<213> Artificial Sequence

<220>
<223> Synthetic Construct

<400> 1312
agacgaggtc aaatctgctc c 21

<210> 1313
<211> 20
<212> DNA
<213> Artificial Sequence

<220>
<223> Synthetic Construct

<400> 1313
cagtgcttag gaagtggata 20

<210> 1314
<211> 18
<212> DNA
<213> Artificial Sequence

<220>
<223> Synthetic Construct

<400> 1314
atcactgggg aaaagtgc 18

<210> 1315
<211> 18
<212> DNA
<213> Artificial Sequence

<220>
<223> Synthetic Construct

<400> 1315
tgccactgca ccaggaga 18

<210> 1316
<211> 22
<212> DNA
<213> Artificial Sequence

<220>
<223> Synthetic Construct

<400> 1316
agaggctttt ctcttcccca tc 22

<210> 1317
<211> 18
<212> DNA
<213> Artificial Sequence

<220>

<223> Synthetic Construct

<400> 1317
tttgcatccc tcggttct 18

<210> 1318
<211> 20
<212> DNA
<213> Artificial Sequence

<220>
<223> Synthetic Construct

<400> 1318
tgagatggct ctggtaattt 20

<210> 1319
<211> 23
<212> DNA
<213> Artificial Sequence

<220>
<223> Synthetic Construct

<400> 1319
gtgttaaata acccattcaa ggt 23

<210> 1320
<211> 20
<212> DNA
<213> Artificial Sequence

<220>
<223> Synthetic Construct

<400> 1320
ggctactggt gtgtaggggc 20

<210> 1321
<211> 19
<212> DNA
<213> Artificial Sequence

<220>
<223> Synthetic Construct

<400> 1321
ccagtgactc atctgtgct 19

<210> 1322
<211> 18
<212> DNA
<213> Artificial Sequence

<220>
<223> Synthetic Construct

<400> 1322

gggacaggat ctcggctt 18

<210> 1323
<211> 20
<212> DNA
<213> Artificial Sequence

<220>
<223> Synthetic Construct

<400> 1323
tcaaggaacc aagactacac 20

<210> 1324
<211> 22
<212> DNA
<213> Artificial Sequence

<220>
<223> Synthetic Construct

<400> 1324
ttggtgttta tggatgagtg gt 22

<210> 1325
<211> 20
<212> DNA
<213> Artificial Sequence

<220>
<223> Synthetic Construct

<400> 1325
cctcttgacc ccaggtattc 20

<210> 1326
<211> 19
<212> DNA
<213> Artificial Sequence

<220>
<223> Synthetic Construct

<400> 1326
gggtgctgct agatgctga 19

<210> 1327
<211> 20
<212> DNA
<213> Artificial Sequence

<220>
<223> Synthetic Construct

<400> 1327
ttaccaactc ctagaagcca 20

<210> 1328
<211> 21
<212> DNA
<213> Artificial Sequence

<220>
<223> Synthetic Construct

<400> 1328
cagatgaagc tcaggtattt t 21

<210> 1329
<211> 20
<212> DNA
<213> Artificial Sequence

<220>
<223> Synthetic Construct

<400> 1329
gttcacagca tgtataagcc 20

<210> 1330
<211> 20
<212> DNA
<213> Artificial Sequence

<220>
<223> Synthetic Construct

<400> 1330
gccaatattt caggtaaaga 20

<210> 1331
<211> 22
<212> DNA
<213> Artificial Sequence

<220>
<223> Synthetic Construct

<400> 1331
tcaagtttaa atccagagtt tc 22

<210> 1332
<211> 19
<212> DNA
<213> Artificial Sequence

<220>
<223> Synthetic Construct

<400> 1332
ggaggcttga acatcctac 19

<210> 1333
<211> 20
<212> DNA

<213> Artificial Sequence

<220>
<223> Synthetic Construct

<400> 1333
ctatgtttag cactccctca 20

<210> 1334
<211> 18
<212> DNA
<213> Artificial Sequence

<220>
<223> Synthetic Construct

<400> 1334
tccccagaag tggacctg 18

<210> 1335
<211> 22
<212> DNA
<213> Artificial Sequence

<220>
<223> Synthetic Construct

<400> 1335
tagtgcttga gagcaatgga tg 22

<210> 1336
<211> 18
<212> DNA
<213> Artificial Sequence

<220>
<223> Synthetic Construct

<400> 1336
cacaaaggag ccatgctg 18

<210> 1337
<211> 18
<212> DNA
<213> Artificial Sequence

<220>
<223> Synthetic Construct

<400> 1337
gtggcagctc tgtctctg 18

<210> 1338
<211> 19
<212> DNA
<213> Artificial Sequence

<220>

<223> Synthetic Construct

<400> 1338
taagtgaagg gacttggag 19

<210> 1339
<211> 19
<212> DNA
<213> Artificial Sequence

<220>
<223> Synthetic Construct

<400> 1339
gaaccgccta gaaggcaac 19

<210> 1340
<211> 18
<212> DNA
<213> Artificial Sequence

<220>
<223> Synthetic Construct

<400> 1340
cgcagcccac agctaagt 18

<210> 1341
<211> 18
<212> DNA
<213> Artificial Sequence

<220>
<223> Synthetic Construct

<400> 1341
aggctgagtg gctgcaca 18

<210> 1342
<211> 18
<212> DNA
<213> Artificial Sequence

<220>
<223> Synthetic Construct

<400> 1342
gcattagggc acctggtc 18

<210> 1343
<211> 18
<212> DNA
<213> Artificial Sequence

<220>
<223> Synthetic Construct

<400> 1343

ggtggacaaa acgacccc 18

<210> 1344
<211> 20
<212> DNA
<213> Artificial Sequence

<220>
<223> Synthetic Construct

<400> 1344
aaattgcatc tggctacaca 20

<210> 1345
<211> 20
<212> DNA
<213> Artificial Sequence

<220>
<223> Synthetic Construct

<400> 1345
ggataccctt tgagcccttg 20

<210> 1346
<211> 20
<212> DNA
<213> Artificial Sequence

<220>
<223> Synthetic Construct

<400> 1346
catgaactcc atgaactctt 20

<210> 1347
<211> 19
<212> DNA
<213> Artificial Sequence

<220>
<223> Synthetic Construct

<400> 1347
gactaggatt agccagcgg 19

<210> 1348
<211> 18
<212> DNA
<213> Artificial Sequence

<220>
<223> Synthetic Construct

<400> 1348
tccagggctt ctcctggg 18

<210> 1349
<211> 21
<212> DNA
<213> Artificial Sequence

<220>
<223> Synthetic Construct

<400> 1349
agacaagtag ctgacctggg g 21

<210> 1350
<211> 18
<212> DNA
<213> Artificial Sequence

<220>
<223> Synthetic Construct

<400> 1350
aggacatggg gctggttt 18

<210> 1351
<211> 18
<212> DNA
<213> Artificial Sequence

<220>
<223> Synthetic Construct

<400> 1351
cctgcaggca cctgtttc 18

<210> 1352
<211> 21
<212> DNA
<213> Artificial Sequence

<220>
<223> Synthetic Construct

<400> 1352
acatcagatg ggttcacact c 21

<210> 1353
<211> 18
<212> DNA
<213> Artificial Sequence

<220>
<223> Synthetic Construct

<400> 1353
aacttggtgg gaagggaa 18

<210> 1354
<211> 18
<212> DNA

<213> Artificial Sequence

<220>
<223> Synthetic Construct

<400> 1354
ggcctgagca caggtttc 18

<210> 1355
<211> 20
<212> DNA
<213> Artificial Sequence

<220>
<223> Synthetic Construct

<400> 1355
aggaataacc tgcagcacca 20

<210> 1356
<211> 22
<212> DNA
<213> Artificial Sequence

<220>
<223> Synthetic Construct

<400> 1356
acctgtcagt tcaatgtgta aa 22

<210> 1357
<211> 18
<212> DNA
<213> Artificial Sequence

<220>
<223> Synthetic Construct

<400> 1357
ttgagcacga ataaaggc 18

<210> 1358
<211> 18
<212> DNA
<213> Artificial Sequence

<220>
<223> Synthetic Construct

<400> 1358
ggagcagtgt ttagagca 18

<210> 1359
<211> 20
<212> DNA
<213> Artificial Sequence

<220>

<223> Synthetic Construct

<400> 1359
cttgcctagg gtgactgaca 20

<210> 1360
<211> 23
<212> DNA
<213> Artificial Sequence

<220>
<223> Synthetic Construct

<400> 1360
ggagtaagaa ttggggttag gtc 23

<210> 1361
<211> 18
<212> DNA
<213> Artificial Sequence

<220>
<223> Synthetic Construct

<400> 1361
cagtgggaga tggggcag 18

<210> 1362
<211> 20
<212> DNA
<213> Artificial Sequence

<220>
<223> Synthetic Construct

<400> 1362
ctgagccctg ggtagtaaca 20

<210> 1363
<211> 20
<212> DNA
<213> Artificial Sequence

<220>
<223> Synthetic Construct

<400> 1363
ttgcttgcta ttgaattgtg 20

<210> 1364
<211> 20
<212> DNA
<213> Artificial Sequence

<220>
<223> Synthetic Construct

<400> 1364

tccactgggg ttatcttttg 20

<210> 1365
<211> 18
<212> DNA
<213> Artificial Sequence

<220>
<223> Synthetic Construct

<400> 1365
gggtaacata tgcaccaa 18

<210> 1366
<211> 18
<212> DNA
<213> Artificial Sequence

<220>
<223> Synthetic Construct

<400> 1366
ttcatgttga tgtttggg 18

<210> 1367
<211> 18
<212> DNA
<213> Artificial Sequence

<220>
<223> Synthetic Construct

<400> 1367
agcaatgagt gaacgggc 18

<210> 1368
<211> 21
<212> DNA
<213> Artificial Sequence

<220>
<223> Synthetic Construct

<400> 1368
tttcctgagc tctatttaac a 21

<210> 1369
<211> 20
<212> DNA
<213> Artificial Sequence

<220>
<223> Synthetic Construct

<400> 1369
aacatcctgt gtctgctttg 20

<210> 1370
<211> 20
<212> DNA
<213> Artificial Sequence

<220>
<223> Synthetic Construct

<400> 1370
ggccactatc atggaccaat 20

<210> 1371
<211> 18
<212> DNA
<213> Artificial Sequence

<220>
<223> Synthetic Construct

<400> 1371
agcaatgggc cttgtacc 18

<210> 1372
<211> 18
<212> DNA
<213> Artificial Sequence

<220>
<223> Synthetic Construct

<400> 1372
gtgaggcact cctgaagc 18

<210> 1373
<211> 20
<212> DNA
<213> Artificial Sequence

<220>
<223> Synthetic Construct

<400> 1373
atgatgtaac tcccctttcct 20

<210> 1374
<211> 20
<212> DNA
<213> Artificial Sequence

<220>
<223> Synthetic Construct

<400> 1374
agtacaaggt gcacagccct 20

<210> 1375
<211> 20
<212> DNA

<213> Artificial Sequence

<220>
<223> Synthetic Construct

<400> 1375
gttggctcgt gtggatacag 20

<210> 1376
<211> 18
<212> DNA
<213> Artificial Sequence

<220>
<223> Synthetic Construct

<400> 1376
ttcactgacc atgctgct 18

<210> 1377
<211> 23
<212> DNA
<213> Artificial Sequence

<220>
<223> Synthetic Construct

<400> 1377
aatttattgc catgtacact tac 23

<210> 1378
<211> 20
<212> DNA
<213> Artificial Sequence

<220>
<223> Synthetic Construct

<400> 1378
ccttgctgaa aggttaaatc 20

<210> 1379
<211> 18
<212> DNA
<213> Artificial Sequence

<220>
<223> Synthetic Construct

<400> 1379
gagcagctca ctctcgcc 18

<210> 1380
<211> 18
<212> DNA
<213> Artificial Sequence

<220>

<223>  Synthetic Construct

<400>  1380
caccatccac ctgggttc                                                     18

<210>  1381
<211>  20
<212>  DNA
<213>  Artificial Sequence

<220>
<223>  Synthetic Construct

<400>  1381
acaagggcca gatcatcaac                                                   20

<210>  1382
<211>  19
<212>  DNA
<213>  Artificial Sequence

<220>
<223>  Synthetic Construct

<400>  1382
agagccccac ttgtccatt                                                    19

<210>  1383
<211>  18
<212>  DNA
<213>  Artificial Sequence

<220>
<223>  Synthetic Construct

<400>  1383
catatgttgt ccatcccc                                                     18

<210>  1384
<211>  22
<212>  DNA
<213>  Artificial Sequence

<220>
<223>  Synthetic Construct

<400>  1384
cagtgatatg ggatagtggg tc                                                22

<210>  1385
<211>  20
<212>  DNA
<213>  Artificial Sequence

<220>
<223>  Synthetic Construct

<400>  1385

agcttctgaa tcttggtctt 20

<210> 1386
<211> 18
<212> DNA
<213> Artificial Sequence

<220>
<223> Synthetic Construct

<400> 1386
gggtggaatg attgtgcg 18

<210> 1387
<211> 20
<212> DNA
<213> Artificial Sequence

<220>
<223> Synthetic Construct

<400> 1387
gctgtgatgt catttaggct 20

<210> 1388
<211> 20
<212> DNA
<213> Artificial Sequence

<220>
<223> Synthetic Construct

<400> 1388
gaacccaaag tggctgcttc 20

<210> 1389
<211> 18
<212> DNA
<213> Artificial Sequence

<220>
<223> Synthetic Construct

<400> 1389
tgtgaaggga ttgtccca 18

<210> 1390
<211> 20
<212> DNA
<213> Artificial Sequence

<220>
<223> Synthetic Construct

<400> 1390
tcacttcttc ctcttctttg 20

<210> 1391
<211> 20
<212> DNA
<213> Artificial Sequence

<220>
<223> Synthetic Construct

<400> 1391
aaacattaat tctctgcctg 20

<210> 1392
<211> 18
<212> DNA
<213> Artificial Sequence

<220>
<223> Synthetic Construct

<400> 1392
cctttcagcc tccagttt 18

<210> 1393
<211> 19
<212> DNA
<213> Artificial Sequence

<220>
<223> Synthetic Construct

<400> 1393
aaagctgcac attttacct 19

<210> 1394
<211> 19
<212> DNA
<213> Artificial Sequence

<220>
<223> Synthetic Construct

<400> 1394
taagggtggg gcttctagc 19

<210> 1395
<211> 20
<212> DNA
<213> Artificial Sequence

<220>
<223> Synthetic Construct

<400> 1395
attgtgccta aaagagggaa 20

<210> 1396
<211> 20
<212> DNA

<213> Artificial Sequence

<220>
<223> Synthetic Construct

<400> 1396
tcaagcttct atccgctatc 20

<210> 1397
<211> 22
<212> DNA
<213> Artificial Sequence

<220>
<223> Synthetic Construct

<400> 1397
gaataaagag cacaagtgga ga 22

<210> 1398
<211> 22
<212> DNA
<213> Artificial Sequence

<220>
<223> Synthetic Construct

<400> 1398
caagtcttgg tctttactca tt 22

<210> 1399
<211> 20
<212> DNA
<213> Artificial Sequence

<220>
<223> Synthetic Construct

<400> 1399
attctgtcat tggtcctaaa 20

<210> 1400
<211> 19
<212> DNA
<213> Artificial Sequence

<220>
<223> Synthetic Construct

<400> 1400
acaagttgga aggcagcag 19

<210> 1401
<211> 20
<212> DNA
<213> Artificial Sequence

<220>

<223> Synthetic Construct

<400> 1401
tgtgggtctt gcttctcaca 20

<210> 1402
<211> 23
<212> DNA
<213> Artificial Sequence

<220>
<223> Synthetic Construct

<400> 1402
gataagcaat aatgattgtg gtg 23

<210> 1403
<211> 20
<212> DNA
<213> Artificial Sequence

<220>
<223> Synthetic Construct

<400> 1403
tgaaacccag ggctgtaaac 20

<210> 1404
<211> 19
<212> DNA
<213> Artificial Sequence

<220>
<223> Synthetic Construct

<400> 1404
cttggtagca ccaaagctg 19

<210> 1405
<211> 21
<212> DNA
<213> Artificial Sequence

<220>
<223> Synthetic Construct

<400> 1405
ctgatgatgg gaaagaacaa a 21

<210> 1406
<211> 18
<212> DNA
<213> Artificial Sequence

<220>
<223> Synthetic Construct

<400> 1406

ttaggggatt ctccttcc 18

<210> 1407
<211> 20
<212> DNA
<213> Artificial Sequence

<220>
<223> Synthetic Construct

<400> 1407
tctaatcagc caccatctcc 20

<210> 1408
<211> 18
<212> DNA
<213> Artificial Sequence

<220>
<223> Synthetic Construct

<400> 1408
actgctcagc ctcaacca 18

<210> 1409
<211> 18
<212> DNA
<213> Artificial Sequence

<220>
<223> Synthetic Construct

<400> 1409
tgacccattc ccaaaatg 18

<210> 1410
<211> 23
<212> DNA
<213> Artificial Sequence

<220>
<223> Synthetic Construct

<400> 1410
agctgggaca tgcttctggt tag 23

<210> 1411
<211> 19
<212> DNA
<213> Artificial Sequence

<220>
<223> Synthetic Construct

<400> 1411
catagcccct attcaaatc 19

<210> 1412
<211> 20
<212> DNA
<213> Artificial Sequence

<220>
<223> Synthetic Construct

<400> 1412
gcagagaaga ccagtaggct 20

<210> 1413
<211> 20
<212> DNA
<213> Artificial Sequence

<220>
<223> Synthetic Construct

<400> 1413
tcagggaaga ctatcctcaa 20

<210> 1414
<211> 20
<212> DNA
<213> Artificial Sequence

<220>
<223> Synthetic Construct

<400> 1414
cagccctaaa gtccagttcc 20

<210> 1415
<211> 21
<212> DNA
<213> Artificial Sequence

<220>
<223> Synthetic Construct

<400> 1415
tttgcgccat ctagagaaga t 21

<210> 1416
<211> 19
<212> DNA
<213> Artificial Sequence

<220>
<223> Synthetic Construct

<400> 1416
taacgcagtt caggatggc 19

<210> 1417
<211> 24
<212> DNA

<213> Artificial Sequence

<220>
<223> Synthetic Construct

<400> 1417
gcttgtaatc tagatgtagc tggt 24

<210> 1418
<211> 18
<212> DNA
<213> Artificial Sequence

<220>
<223> Synthetic Construct

<400> 1418
gagagcaggg acatacgc 18

<210> 1419
<211> 21
<212> DNA
<213> Artificial Sequence

<220>
<223> Synthetic Construct

<400> 1419
gcaagttcca gctctgttga c 21

<210> 1420
<211> 18
<212> DNA
<213> Artificial Sequence

<220>
<223> Synthetic Construct

<400> 1420
tggctagcag ctggttca 18

<210> 1421
<211> 18
<212> DNA
<213> Artificial Sequence

<220>
<223> Synthetic Construct

<400> 1421
gccggagttt gtggtgat 18

<210> 1422
<211> 18
<212> DNA
<213> Artificial Sequence

<220>

<223> Synthetic Construct

<400> 1422
ggaggaggag atggcagc 18

<210> 1423
<211> 20
<212> DNA
<213> Artificial Sequence

<220>
<223> Synthetic Construct

<400> 1423
tgcacagcta gaaggttggc 20

<210> 1424
<211> 22
<212> DNA
<213> Artificial Sequence

<220>
<223> Synthetic Construct

<400> 1424
agtacgtata tcctgcatgg gg 22

<210> 1425
<211> 22
<212> DNA
<213> Artificial Sequence

<220>
<223> Synthetic Construct

<400> 1425
tgtctctgca gacagatgaa ga 22

<210> 1426
<211> 20
<212> DNA
<213> Artificial Sequence

<220>
<223> Synthetic Construct

<400> 1426
ggctttagct gtataaggca 20

<210> 1427
<211> 20
<212> DNA
<213> Artificial Sequence

<220>
<223> Synthetic Construct

<400> 1427

tctttgtggt ttcttctgat 20

<210> 1428
<211> 21
<212> DNA
<213> Artificial Sequence

<220>
<223> Synthetic Construct

<400> 1428
tgggattcat catagtaact g 21

<210> 1429
<211> 20
<212> DNA
<213> Artificial Sequence

<220>
<223> Synthetic Construct

<400> 1429
agactcagga ggatgaaagt 20

<210> 1430
<211> 18
<212> DNA
<213> Artificial Sequence

<220>
<223> Synthetic Construct

<400> 1430
gctggaagtc caggctgt 18

<210> 1431
<211> 20
<212> DNA
<213> Artificial Sequence

<220>
<223> Synthetic Construct

<400> 1431
aaaaccaaga gtcagacaca 20

<210> 1432
<211> 18
<212> DNA
<213> Artificial Sequence

<220>
<223> Synthetic Construct

<400> 1432
tggggtcttg ggttctgc 18

<210> 1433
<211> 19
<212> DNA
<213> Artificial Sequence

<220>
<223> Synthetic Construct

<400> 1433
cttcggagga gaagaccct 19

<210> 1434
<211> 18
<212> DNA
<213> Artificial Sequence

<220>
<223> Synthetic Construct

<400> 1434
agagtgaccc tccgggat 18

<210> 1435
<211> 20
<212> DNA
<213> Artificial Sequence

<220>
<223> Synthetic Construct

<400> 1435
gtcctctatc ccaccatccc 20

<210> 1436
<211> 24
<212> DNA
<213> Artificial Sequence

<220>
<223> Synthetic Construct

<400> 1436
aggtaagtac cagaagacag ctca 24

<210> 1437
<211> 18
<212> DNA
<213> Artificial Sequence

<220>
<223> Synthetic Construct

<400> 1437
ttaccaccgc ataacctg 18

<210> 1438
<211> 23
<212> DNA

<213> Artificial Sequence

<220>
<223> Synthetic Construct

<400> 1438
tcacagatgg gagcagtttc ata 23

<210> 1439
<211> 20
<212> DNA
<213> Artificial Sequence

<220>
<223> Synthetic Construct

<400> 1439
acttgtccat ccagtccttg 20

<210> 1440
<211> 18
<212> DNA
<213> Artificial Sequence

<220>
<223> Synthetic Construct

<400> 1440
aattccgtgt cagcccac 18

<210> 1441
<211> 19
<212> DNA
<213> Artificial Sequence

<220>
<223> Synthetic Construct

<400> 1441
tggcttggat acctctagt 19

<210> 1442
<211> 19
<212> DNA
<213> Artificial Sequence

<220>
<223> Synthetic Construct

<400> 1442
aggtacttct gaggagcaa 19

<210> 1443
<211> 18
<212> DNA
<213> Artificial Sequence

<220>

<223> Synthetic Construct

<400> 1443
tggaggcagg cagaggtc 18

<210> 1444
<211> 18
<212> DNA
<213> Artificial Sequence

<220>
<223> Synthetic Construct

<400> 1444
gccgcttcta gtggcttc 18

<210> 1445
<211> 24
<212> DNA
<213> Artificial Sequence

<220>
<223> Synthetic Construct

<400> 1445
atacgcacat gtgtatacct gctt 24

<210> 1446
<211> 18
<212> DNA
<213> Artificial Sequence

<220>
<223> Synthetic Construct

<400> 1446
gcacactgca cgctgaca 18

<210> 1447
<211> 19
<212> DNA
<213> Artificial Sequence

<220>
<223> Synthetic Construct

<400> 1447
tttcccacct atctcagtt 19

<210> 1448
<211> 20
<212> DNA
<213> Artificial Sequence

<220>
<223> Synthetic Construct

<400> 1448

ctttctactc tgatgcatgg 20

<210> 1449
<211> 18
<212> DNA
<213> Artificial Sequence

<220>
<223> Synthetic Construct

<400> 1449
ccatttgaat caagtcca 18

<210> 1450
<211> 22
<212> DNA
<213> Artificial Sequence

<220>
<223> Synthetic Construct

<400> 1450
acttgatagg ttatgctact cc 22

<210> 1451
<211> 22
<212> DNA
<213> Artificial Sequence

<220>
<223> Synthetic Construct

<400> 1451
cttttagccc tgtacactct at 22

<210> 1452
<211> 23
<212> DNA
<213> Artificial Sequence

<220>
<223> Synthetic Construct

<400> 1452
ttccataatc ttactctgtg aaa 23

<210> 1453
<211> 18
<212> DNA
<213> Artificial Sequence

<220>
<223> Synthetic Construct

<400> 1453
agggcagcca ctatgccc 18

<210> 1454
<211> 19
<212> DNA
<213> Artificial Sequence

<220>
<223> Synthetic Construct

<400> 1454
gagatgggag ctgtggagc 19

<210> 1455
<211> 20
<212> DNA
<213> Artificial Sequence

<220>
<223> Synthetic Construct

<400> 1455
cataggtttg aagcagtcac 20

<210> 1456
<211> 22
<212> DNA
<213> Artificial Sequence

<220>
<223> Synthetic Construct

<400> 1456
ggctcagtag aggtttagta tg 22

<210> 1457
<211> 23
<212> DNA
<213> Artificial Sequence

<220>
<223> Synthetic Construct

<400> 1457
tgggtggaat ttctttatcc aac 23

<210> 1458
<211> 18
<212> DNA
<213> Artificial Sequence

<220>
<223> Synthetic Construct

<400> 1458
tgtggctcct gatcatct 18

<210> 1459
<211> 18
<212> DNA

<213> Artificial Sequence

<220>
<223> Synthetic Construct

<400> 1459
ctgctgcctc cgaagctc 18

<210> 1460
<211> 18
<212> DNA
<213> Artificial Sequence

<220>
<223> Synthetic Construct

<400> 1460
gagggacctg tgaccttt 18

<210> 1461
<211> 22
<212> DNA
<213> Artificial Sequence

<220>
<223> Synthetic Construct

<400> 1461
aaaccaatta ctgtgctaga ga 22

<210> 1462
<211> 18
<212> DNA
<213> Artificial Sequence

<220>
<223> Synthetic Construct

<400> 1462
aattgcagtt gcaaacat 18

<210> 1463
<211> 18
<212> DNA
<213> Artificial Sequence

<220>
<223> Synthetic Construct

<400> 1463
gctgacctgg agacctgc 18

<210> 1464
<211> 20
<212> DNA
<213> Artificial Sequence

<220>

<223> Synthetic Construct

<400> 1464
tatagctagc aaggctgggc 20

<210> 1465
<211> 22
<212> DNA
<213> Artificial Sequence

<220>
<223> Synthetic Construct

<400> 1465
gaggacagaa gggactctag ga 22

<210> 1466
<211> 20
<212> DNA
<213> Artificial Sequence

<220>
<223> Synthetic Construct

<400> 1466
accaatggtt agtcagcaaa 20

<210> 1467
<211> 18
<212> DNA
<213> Artificial Sequence

<220>
<223> Synthetic Construct

<400> 1467
tacaaagccg tttcctca 18

<210> 1468
<211> 18
<212> DNA
<213> Artificial Sequence

<220>
<223> Synthetic Construct

<400> 1468
ggctcatctg tcaccctg 18

<210> 1469
<211> 20
<212> DNA
<213> Artificial Sequence

<220>
<223> Synthetic Construct

<400> 1469

tggtttggtg ataaatgaga 20

<210> 1470
<211> 18
<212> DNA
<213> Artificial Sequence

<220>
<223> Synthetic Construct

<400> 1470
ctctgctccc cgtcacac 18

<210> 1471
<211> 18
<212> DNA
<213> Artificial Sequence

<220>
<223> Synthetic Construct

<400> 1471
cccacgcatg gctaggat 18

<210> 1472
<211> 21
<212> DNA
<213> Artificial Sequence

<220>
<223> Synthetic Construct

<400> 1472
gtctgaaccc ttagttagga c 21

<210> 1473
<211> 18
<212> DNA
<213> Artificial Sequence

<220>
<223> Synthetic Construct

<400> 1473
cctccgtgac ctccaagc 18

<210> 1474
<211> 18
<212> DNA
<213> Artificial Sequence

<220>
<223> Synthetic Construct

<400> 1474
ccagatgctg cccaccac 18

<210> 1475
<211> 18
<212> DNA
<213> Artificial Sequence

<220>
<223> Synthetic Construct

<400> 1475
tgccaagtgt ggtccctg 18

<210> 1476
<211> 18
<212> DNA
<213> Artificial Sequence

<220>
<223> Synthetic Construct

<400> 1476
ctccctgagc gtggatgg 18

<210> 1477
<211> 19
<212> DNA
<213> Artificial Sequence

<220>
<223> Synthetic Construct

<400> 1477
gctgcaggat aggggctac 19

<210> 1478
<211> 19
<212> DNA
<213> Artificial Sequence

<220>
<223> Synthetic Construct

<400> 1478
atcaagggca ggtggctaa 19

<210> 1479
<211> 18
<212> DNA
<213> Artificial Sequence

<220>
<223> Synthetic Construct

<400> 1479
ctgatcatcc tcgtcagg 18

<210> 1480
<211> 18
<212> DNA

<213> Artificial Sequence

<220>
<223> Synthetic Construct

<400> 1480
ggttctggct gaagggag 18

<210> 1481
<211> 18
<212> DNA
<213> Artificial Sequence

<220>
<223> Synthetic Construct

<400> 1481
accgcactgg tcctgagt 18

<210> 1482
<211> 20
<212> DNA
<213> Artificial Sequence

<220>
<223> Synthetic Construct

<400> 1482
taagggtgtg cctgacatga 20

<210> 1483
<211> 20
<212> DNA
<213> Artificial Sequence

<220>
<223> Synthetic Construct

<400> 1483
gtggaaggtg agattgggaa 20

<210> 1484
<211> 21
<212> DNA
<213> Artificial Sequence

<220>
<223> Synthetic Construct

<400> 1484
ttcgaacatg acctgaaaag c 21

<210> 1485
<211> 18
<212> DNA
<213> Artificial Sequence

<220>

<223> Synthetic Construct

<400> 1485
tcagggtctt ggcaggaa 18

<210> 1486
<211> 18
<212> DNA
<213> Artificial Sequence

<220>
<223> Synthetic Construct

<400> 1486
tggaggtgct ccaggact 18

<210> 1487
<211> 18
<212> DNA
<213> Artificial Sequence

<220>
<223> Synthetic Construct

<400> 1487
accccacgcc tacaccag 18

<210> 1488
<211> 18
<212> DNA
<213> Artificial Sequence

<220>
<223> Synthetic Construct

<400> 1488
aaaggtcctg cacacccg 18

<210> 1489
<211> 18
<212> DNA
<213> Artificial Sequence

<220>
<223> Synthetic Construct

<400> 1489
cctagagcgg tgatccca 18

<210> 1490
<211> 19
<212> DNA
<213> Artificial Sequence

<220>
<223> Synthetic Construct

<400> 1490

gaggtcatgg aatgtgggc 19

<210> 1491
<211> 20
<212> DNA
<213> Artificial Sequence

<220>
<223> Synthetic Construct

<400> 1491
ggttctatgc aggagccgac 20

<210> 1492
<211> 18
<212> DNA
<213> Artificial Sequence

<220>
<223> Synthetic Construct

<400> 1492
taagagggac catcggca 18

<210> 1493
<211> 20
<212> DNA
<213> Artificial Sequence

<220>
<223> Synthetic Construct

<400> 1493
gctcgtagtt cgccttcaac 20

<210> 1494
<211> 19
<212> DNA
<213> Artificial Sequence

<220>
<223> Synthetic Construct

<400> 1494
cgccttagtc acggctttc 19

<210> 1495
<211> 23
<212> DNA
<213> Artificial Sequence

<220>
<223> Synthetic Construct

<400> 1495
tgtagagaat ctgaatagac cat 23

<210> 1496
<211> 19
<212> DNA
<213> Artificial Sequence

<220>
<223> Synthetic Construct

<400> 1496
tcctaatgtg aaatcggag 19

<210> 1497
<211> 19
<212> DNA
<213> Artificial Sequence

<220>
<223> Synthetic Construct

<400> 1497
cttgcagggg tcatgctaa 19

<210> 1498
<211> 20
<212> DNA
<213> Artificial Sequence

<220>
<223> Synthetic Construct

<400> 1498
gaaaccgtgg caatatccta 20

<210> 1499
<211> 19
<212> DNA
<213> Artificial Sequence

<220>
<223> Synthetic Construct

<400> 1499
ttgccaacgt tctgctttt 19

<210> 1500
<211> 19
<212> DNA
<213> Artificial Sequence

<220>
<223> Synthetic Construct

<400> 1500
tgctgcagaa agtgagggt 19

<210> 1501
<211> 19
<212> DNA

<213> Artificial Sequence

<220>
<223> Synthetic Construct

<400> 1501
aggcagctcc cttgcagat 19

<210> 1502
<211> 21
<212> DNA
<213> Artificial Sequence

<220>
<223> Synthetic Construct

<400> 1502
cccctctgac ttctgtgagt c 21

<210> 1503
<211> 20
<212> DNA
<213> Artificial Sequence

<220>
<223> Synthetic Construct

<400> 1503
agttgcaaag aaagcactcc 20

<210> 1504
<211> 20
<212> DNA
<213> Artificial Sequence

<220>
<223> Synthetic Construct

<400> 1504
tgttaacacc tgttgcattt 20

<210> 1505
<211> 18
<212> DNA
<213> Artificial Sequence

<220>
<223> Synthetic Construct

<400> 1505
cgctggcata tgctgtca 18

<210> 1506
<211> 18
<212> DNA
<213> Artificial Sequence

<220>

<223> Synthetic Construct

<400> 1506
gctgaaggaa gcccgaat 18

<210> 1507
<211> 20
<212> DNA
<213> Artificial Sequence

<220>
<223> Synthetic Construct

<400> 1507
ctggagtaat actgtccagc 20

<210> 1508
<211> 19
<212> DNA
<213> Artificial Sequence

<220>
<223> Synthetic Construct

<400> 1508
cagtatcatg agctggtgg 19

<210> 1509
<211> 19
<212> DNA
<213> Artificial Sequence

<220>
<223> Synthetic Construct

<400> 1509
ggagtgtgca ttgacagcc 19

<210> 1510
<211> 20
<212> DNA
<213> Artificial Sequence

<220>
<223> Synthetic Construct

<400> 1510
gaagatgctc tgaggcaaac 20

<210> 1511
<211> 20
<212> DNA
<213> Artificial Sequence

<220>
<223> Synthetic Construct

<400> 1511

ctttggcaat ggaacattat 20

<210> 1512
<211> 24
<212> DNA
<213> Artificial Sequence

<220>
<223> Synthetic Construct

<400> 1512
ccttactcag acaaactctt cgag 24

<210> 1513
<211> 19
<212> DNA
<213> Artificial Sequence

<220>
<223> Synthetic Construct

<400> 1513
tgacctccaa ggagaggaa 19

<210> 1514
<211> 21
<212> DNA
<213> Artificial Sequence

<220>
<223> Synthetic Construct

<400> 1514
gtcaagggtc agattctagt g 21

<210> 1515
<211> 22
<212> DNA
<213> Artificial Sequence

<220>
<223> Synthetic Construct

<400> 1515
ctgtttgtcc aaaattcaac cc 22

<210> 1516
<211> 18
<212> DNA
<213> Artificial Sequence

<220>
<223> Synthetic Construct

<400> 1516
catttgccaa acctcaga 18

<210> 1517
<211> 22
<212> DNA
<213> Artificial Sequence

<220>
<223> Synthetic Construct

<400> 1517
aatgttttat acagctctca gc 22

<210> 1518
<211> 20
<212> DNA
<213> Artificial Sequence

<220>
<223> Synthetic Construct

<400> 1518
tgttctagaa acagtgcctt 20

<210> 1519
<211> 24
<212> DNA
<213> Artificial Sequence

<220>
<223> Synthetic Construct

<400> 1519
ggatagaata tttcaagggg acta 24

<210> 1520
<211> 21
<212> DNA
<213> Artificial Sequence

<220>
<223> Synthetic Construct

<400> 1520
aaagctcttt gtctctgaaa g 21

<210> 1521
<211> 23
<212> DNA
<213> Artificial Sequence

<220>
<223> Synthetic Construct

<400> 1521
atgatgataa ttcttctgaa cac 23

<210> 1522
<211> 20
<212> DNA

<213>  Artificial Sequence

<220>
<223>  Synthetic Construct

<400>  1522
ttatccatca ttcaaaggaa                                                   20

<210>  1523
<211>  19
<212>  DNA
<213>  Artificial Sequence

<220>
<223>  Synthetic Construct

<400>  1523
ggaggtcaag aggggaaaa                                                    19

<210>  1524
<211>  19
<212>  DNA
<213>  Artificial Sequence

<220>
<223>  Synthetic Construct

<400>  1524
tttaacccag tcctcctct                                                    19

<210>  1525
<211>  20
<212>  DNA
<213>  Artificial Sequence

<220>
<223>  Synthetic Construct

<400>  1525
tcctaccaat gaccctataa                                                   20

<210>  1526
<211>  21
<212>  DNA
<213>  Artificial Sequence

<220>
<223>  Synthetic Construct

<400>  1526
ttctgccatt tggcattaca t                                                 21

<210>  1527
<211>  19
<212>  DNA
<213>  Artificial Sequence

<220>

<223> Synthetic Construct

<400> 1527
tgtaagttgc agtttgcag 19

<210> 1528
<211> 20
<212> DNA
<213> Artificial Sequence

<220>
<223> Synthetic Construct

<400> 1528
tctaccgtta tgccacttga 20

<210> 1529
<211> 18
<212> DNA
<213> Artificial Sequence

<220>
<223> Synthetic Construct

<400> 1529
accagtggtt ctggctcc 18

<210> 1530
<211> 21
<212> DNA
<213> Artificial Sequence

<220>
<223> Synthetic Construct

<400> 1530
aagcattcaa gatgagttac a 21

<210> 1531
<211> 18
<212> DNA
<213> Artificial Sequence

<220>
<223> Synthetic Construct

<400> 1531
tctgctgccg tagagcct 18

<210> 1532
<211> 18
<212> DNA
<213> Artificial Sequence

<220>
<223> Synthetic Construct

<400> 1532

gagtccctgc ggtgtcct 18

<210> 1533
<211> 18
<212> DNA
<213> Artificial Sequence

<220>
<223> Synthetic Construct

<400> 1533
agaacccttg cctgaccc 18

<210> 1534
<211> 18
<212> DNA
<213> Artificial Sequence

<220>
<223> Synthetic Construct

<400> 1534
gccattagca agggcctg 18

<210> 1535
<211> 20
<212> DNA
<213> Artificial Sequence

<220>
<223> Synthetic Construct

<400> 1535
tctgcaggtc tctgttcaaa 20

<210> 1536
<211> 24
<212> DNA
<213> Artificial Sequence

<220>
<223> Synthetic Construct

<400> 1536
gctgctgttt taggtagagt agtt 24

<210> 1537
<211> 21
<212> DNA
<213> Artificial Sequence

<220>
<223> Synthetic Construct

<400> 1537
gaatctttgt gaatgtatgg a 21

<210> 1538
<211> 21
<212> DNA
<213> Artificial Sequence

<220>
<223> Synthetic Construct

<400> 1538
aggtataagt gagctgaacc a 21

<210> 1539
<211> 19
<212> DNA
<213> Artificial Sequence

<220>
<223> Synthetic Construct

<400> 1539
cttgctgcat catccaaga 19

<210> 1540
<211> 18
<212> DNA
<213> Artificial Sequence

<220>
<223> Synthetic Construct

<400> 1540
gcaagcttgg ccctcttt 18

<210> 1541
<211> 20
<212> DNA
<213> Artificial Sequence

<220>
<223> Synthetic Construct

<400> 1541
gcctatttcc agggcatatt 20

<210> 1542
<211> 20
<212> DNA
<213> Artificial Sequence

<220>
<223> Synthetic Construct

<400> 1542
tgcagggtta tctttccttt 20

<210> 1543
<211> 18
<212> DNA

<213> Artificial Sequence

<220>
<223> Synthetic Construct

<400> 1543
ggtcacagct tcatcccc 18

<210> 1544
<211> 20
<212> DNA
<213> Artificial Sequence

<220>
<223> Synthetic Construct

<400> 1544
taccttatta gtggggcaaa 20

<210> 1545
<211> 22
<212> DNA
<213> Artificial Sequence

<220>
<223> Synthetic Construct

<400> 1545
agtgtccttg atagacacaa gt 22

<210> 1546
<211> 18
<212> DNA
<213> Artificial Sequence

<220>
<223> Synthetic Construct

<400> 1546
tgccaaaaga gcacagac 18

<210> 1547
<211> 18
<212> DNA
<213> Artificial Sequence

<220>
<223> Synthetic Construct

<400> 1547
gcagccgtct ctgtcctc 18

<210> 1548
<211> 19
<212> DNA
<213> Artificial Sequence

<220>

<223> Synthetic Construct

<400> 1548
gcccaacaga actgacaca 19

<210> 1549
<211> 19
<212> DNA
<213> Artificial Sequence

<220>
<223> Synthetic Construct

<400> 1549
gctctatgac cggcgtcag 19

<210> 1550
<211> 19
<212> DNA
<213> Artificial Sequence

<220>
<223> Synthetic Construct

<400> 1550
ccgtcatctg ctggatctg 19

<210> 1551
<211> 18
<212> DNA
<213> Artificial Sequence

<220>
<223> Synthetic Construct

<400> 1551
cccctagtca ggccgaga 18

<210> 1552
<211> 19
<212> DNA
<213> Artificial Sequence

<220>
<223> Synthetic Construct

<400> 1552
cttcaagcac cggggacac 19

<210> 1553
<211> 21
<212> DNA
<213> Artificial Sequence

<220>
<223> Synthetic Construct

<400> 1553

cttttacctg agctcaattt t 21

<210> 1554
<211> 22
<212> DNA
<213> Artificial Sequence

<220>
<223> Synthetic Construct

<400> 1554
catggtaact acaaggtgtc tt 22

<210> 1555
<211> 22
<212> DNA
<213> Artificial Sequence

<220>
<223> Synthetic Construct

<400> 1555
atgtatatcc aaacaaggat ct 22

<210> 1556
<211> 22
<212> DNA
<213> Artificial Sequence

<220>
<223> Synthetic Construct

<400> 1556
ttgtgttctt atgagctgta aa 22

<210> 1557
<211> 22
<212> DNA
<213> Artificial Sequence

<220>
<223> Synthetic Construct

<400> 1557
atgcgtctgt agtcacagct cc 22

<210> 1558
<211> 18
<212> DNA
<213> Artificial Sequence

<220>
<223> Synthetic Construct

<400> 1558
cagtgcattg gcctgacc 18

<210> 1559
<211> 20
<212> DNA
<213> Artificial Sequence

<220>
<223> Synthetic Construct

<400> 1559
cgagctggga agttgcaaaa 20

<210> 1560
<211> 20
<212> DNA
<213> Artificial Sequence

<220>
<223> Synthetic Construct

<400> 1560
cttgatgatt tcatgagggg 20

<210> 1561
<211> 22
<212> DNA
<213> Artificial Sequence

<220>
<223> Synthetic Construct

<400> 1561
gcatgcattt agaagcttac ct 22

<210> 1562
<211> 21
<212> DNA
<213> Artificial Sequence

<220>
<223> Synthetic Construct

<400> 1562
gctttaatgg caatcaagtt t 21

<210> 1563
<211> 18
<212> DNA
<213> Artificial Sequence

<220>
<223> Synthetic Construct

<400> 1563
ccccaactgt tgagagag 18

<210> 1564
<211> 18
<212> DNA

<213> Artificial Sequence

<220>
<223> Synthetic Construct

<400> 1564
acaggggaaa tggctccc 18

<210> 1565
<211> 22
<212> DNA
<213> Artificial Sequence

<220>
<223> Synthetic Construct

<400> 1565
cattgctcat aacttcaatg ac 22

<210> 1566
<211> 18
<212> DNA
<213> Artificial Sequence

<220>
<223> Synthetic Construct

<400> 1566
cccttatgat ttggggta 18

<210> 1567
<211> 22
<212> DNA
<213> Artificial Sequence

<220>
<223> Synthetic Construct

<400> 1567
actcattgat aacttctttt gc 22

<210> 1568
<211> 24
<212> DNA
<213> Artificial Sequence

<220>
<223> Synthetic Construct

<400> 1568
actgcaacag aaacaaaact tgac 24

<210> 1569
<211> 19
<212> DNA
<213> Artificial Sequence

<220>

<223> Synthetic Construct

<400> 1569
tgatggcaag agaaacagg 19

<210> 1570
<211> 19
<212> DNA
<213> Artificial Sequence

<220>
<223> Synthetic Construct

<400> 1570
aaagatgatc ctgaatggg 19

<210> 1571
<211> 22
<212> DNA
<213> Artificial Sequence

<220>
<223> Synthetic Construct

<400> 1571
gcaaaatctt attaccaagt gt 22

<210> 1572
<211> 19
<212> DNA
<213> Artificial Sequence

<220>
<223> Synthetic Construct

<400> 1572
atttcaatag ctggcattt 19

<210> 1573
<211> 20
<212> DNA
<213> Artificial Sequence

<220>
<223> Synthetic Construct

<400> 1573
ctgtccttca aggtagccca 20

<210> 1574
<211> 22
<212> DNA
<213> Artificial Sequence

<220>
<223> Synthetic Construct

<400> 1574

gtatgatatg caggtaccac cc 22

<210> 1575
<211> 18
<212> DNA
<213> Artificial Sequence

<220>
<223> Synthetic Construct

<400> 1575
cagccaccac accaacca 18

<210> 1576
<211> 19
<212> DNA
<213> Artificial Sequence

<220>
<223> Synthetic Construct

<400> 1576
cctctggctc caagaaggt 19

<210> 1577
<211> 20
<212> DNA
<213> Artificial Sequence

<220>
<223> Synthetic Construct

<400> 1577
caaagactat caagacctgg 20

<210> 1578
<211> 19
<212> DNA
<213> Artificial Sequence

<220>
<223> Synthetic Construct

<400> 1578
gcatcttcca ctttgtttc 19

<210> 1579
<211> 18
<212> DNA
<213> Artificial Sequence

<220>
<223> Synthetic Construct

<400> 1579
gctgcaacat gggcttca 18

<210> 1580
<211> 21
<212> DNA
<213> Artificial Sequence

<220>
<223> Synthetic Construct

<400> 1580
tttcacgaat gtgtcattat c 21

<210> 1581
<211> 21
<212> DNA
<213> Artificial Sequence

<220>
<223> Synthetic Construct

<400> 1581
gaaggtgcaa ctaaaagaaa c 21

<210> 1582
<211> 21
<212> DNA
<213> Artificial Sequence

<220>
<223> Synthetic Construct

<400> 1582
tgtctttatt agcaaccaca a 21

<210> 1583
<211> 22
<212> DNA
<213> Artificial Sequence

<220>
<223> Synthetic Construct

<400> 1583
ccccttagat aggaatttga gc 22

<210> 1584
<211> 19
<212> DNA
<213> Artificial Sequence

<220>
<223> Synthetic Construct

<400> 1584
caaggtaccc actggaccc 19

<210> 1585
<211> 19
<212> DNA

<213> Artificial Sequence

<220>
<223> Synthetic Construct

<400> 1585
actttggaaaa cctgggtga 19

<210> 1586
<211> 18
<212> DNA
<213> Artificial Sequence

<220>
<223> Synthetic Construct

<400> 1586
tacccgcctc tgttgtgc 18

<210> 1587
<211> 18
<212> DNA
<213> Artificial Sequence

<220>
<223> Synthetic Construct

<400> 1587
tttggattta ccgaatga 18

<210> 1588
<211> 18
<212> DNA
<213> Artificial Sequence

<220>
<223> Synthetic Construct

<400> 1588
attttgtggt ggatgcaa 18

<210> 1589
<211> 18
<212> DNA
<213> Artificial Sequence

<220>
<223> Synthetic Construct

<400> 1589
aggcccatcg ctcatctt 18

<210> 1590
<211> 19
<212> DNA
<213> Artificial Sequence

<220>

<223> Synthetic Construct

<400> 1590
accactggcc tcagttcaa 19

<210> 1591
<211> 18
<212> DNA
<213> Artificial Sequence

<220>
<223> Synthetic Construct

<400> 1591
gcttcagagc cagatggg 18

<210> 1592
<211> 18
<212> DNA
<213> Artificial Sequence

<220>
<223> Synthetic Construct

<400> 1592
gtccaaccca agggcaag 18

<210> 1593
<211> 21
<212> DNA
<213> Artificial Sequence

<220>
<223> Synthetic Construct

<400> 1593
agtcccattt aatgccaagt g 21

<210> 1594
<211> 20
<212> DNA
<213> Artificial Sequence

<220>
<223> Synthetic Construct

<400> 1594
tgaaaagttg gatcagttgt 20

<210> 1595
<211> 20
<212> DNA
<213> Artificial Sequence

<220>
<223> Synthetic Construct

<400> 1595

tgttcctctt gttattgctt 20

<210> 1596
<211> 22
<212> DNA
<213> Artificial Sequence

<220>
<223> Synthetic Construct

<400> 1596
cacaaaacat gtctctacaa tg 22

<210> 1597
<211> 18
<212> DNA
<213> Artificial Sequence

<220>
<223> Synthetic Construct

<400> 1597
ctacccttcg ggccagtt 18

<210> 1598
<211> 20
<212> DNA
<213> Artificial Sequence

<220>
<223> Synthetic Construct

<400> 1598
agctatccct ccagagtccc 20

<210> 1599
<211> 23
<212> DNA
<213> Artificial Sequence

<220>
<223> Synthetic Construct

<400> 1599
cagaacattt atttctattg ctg 23

<210> 1600
<211> 21
<212> DNA
<213> Artificial Sequence

<220>
<223> Synthetic Construct

<400> 1600
gcaacccaag taactatcca c 21

<210> 1601
<211> 22
<212> DNA
<213> Artificial Sequence

<220>
<223> Synthetic Construct

<400> 1601
gcagttcgag aaatgaaata ga 22

<210> 1602
<211> 20
<212> DNA
<213> Artificial Sequence

<220>
<223> Synthetic Construct

<400> 1602
ttagaccctc attttgccca 20

<210> 1603
<211> 22
<212> DNA
<213> Artificial Sequence

<220>
<223> Synthetic Construct

<400> 1603
ggagtgttaa gtttgcagaa gc 22

<210> 1604
<211> 22
<212> DNA
<213> Artificial Sequence

<220>
<223> Synthetic Construct

<400> 1604
ggtgggtaca ccagatatac ag 22

<210> 1605
<211> 21
<212> DNA
<213> Artificial Sequence

<220>
<223> Synthetic Construct

<400> 1605
tctggaagct atggactctt c 21

<210> 1606
<211> 22
<212> DNA

<213>  Artificial Sequence

<220>
<223>  Synthetic Construct

<400>  1606
tgcagactaa gacaaaggtt tt                                                22

<210>  1607
<211>  20
<212>  DNA
<213>  Artificial Sequence

<220>
<223>  Synthetic Construct

<400>  1607
gagtccctca ccccattctt                                                   20

<210>  1608
<211>  18
<212>  DNA
<213>  Artificial Sequence

<220>
<223>  Synthetic Construct

<400>  1608
ttaccctgcc atcaaggg                                                     18

<210>  1609
<211>  20
<212>  DNA
<213>  Artificial Sequence

<220>
<223>  Synthetic Construct

<400>  1609
cctggcacca accattctat                                                   20

<210>  1610
<211>  20
<212>  DNA
<213>  Artificial Sequence

<220>
<223>  Synthetic Construct

<400>  1610
aggcaaatac cctgtgattc                                                   20

<210>  1611
<211>  20
<212>  DNA
<213>  Artificial Sequence

<220>

<223> Synthetic Construct

<400> 1611
aaggcaatcc acaggagaaa 20

<210> 1612
<211> 21
<212> DNA
<213> Artificial Sequence

<220>
<223> Synthetic Construct

<400> 1612
tgctgagttt taggaggtct g 21

<210> 1613
<211> 21
<212> DNA
<213> Artificial Sequence

<220>
<223> Synthetic Construct

<400> 1613
ggggacaata gcagtcctac a 21

<210> 1614
<211> 21
<212> DNA
<213> Artificial Sequence

<220>
<223> Synthetic Construct

<400> 1614
tgcaacaaca agtacaacca a 21

<210> 1615
<211> 21
<212> DNA
<213> Artificial Sequence

<220>
<223> Synthetic Construct

<400> 1615
tggccatgtt caatacactt c 21

<210> 1616
<211> 20
<212> DNA
<213> Artificial Sequence

<220>
<223> Synthetic Construct

<400> 1616

tcagggattt ggaagactga 20

<210> 1617
<211> 22
<212> DNA
<213> Artificial Sequence

<220>
<223> Synthetic Construct

<400> 1617
acctacacac agaaacacac cc 22

<210> 1618
<211> 22
<212> DNA
<213> Artificial Sequence

<220>
<223> Synthetic Construct

<400> 1618
cctgatgtat gatgtctttg cc 22

<210> 1619
<211> 21
<212> DNA
<213> Artificial Sequence

<220>
<223> Synthetic Construct

<400> 1619
ttgtttcttt tctctctccc c 21

<210> 1620
<211> 20
<212> DNA
<213> Artificial Sequence

<220>
<223> Synthetic Construct

<400> 1620
agaccaaagt ccagaaggca 20

<210> 1621
<211> 22
<212> DNA
<213> Artificial Sequence

<220>
<223> Synthetic Construct

<400> 1621
tgtcaggatc tacttatgct tt 22

<210> 1622
<211> 23
<212> DNA
<213> Artificial Sequence

<220>
<223> Synthetic Construct

<400> 1622
tgttcttgca ataaagtaag cta 23

<210> 1623
<211> 21
<212> DNA
<213> Artificial Sequence

<220>
<223> Synthetic Construct

<400> 1623
agttgtgatt agcccaggag a 21

<210> 1624
<211> 20
<212> DNA
<213> Artificial Sequence

<220>
<223> Synthetic Construct

<400> 1624
ttccaccgag tagtgggaac 20

<210> 1625
<211> 18
<212> DNA
<213> Artificial Sequence

<220>
<223> Synthetic Construct

<400> 1625
tgatagcgcc cctaaccc 18

<210> 1626
<211> 21
<212> DNA
<213> Artificial Sequence

<220>
<223> Synthetic Construct

<400> 1626
cgctattggg tttctacact g 21

<210> 1627
<211> 18
<212> DNA

<213> Artificial Sequence

<220>
<223> Synthetic Construct

<400> 1627
ttcctcggcc ctgtgcta 18

<210> 1628
<211> 19
<212> DNA
<213> Artificial Sequence

<220>
<223> Synthetic Construct

<400> 1628
atgctaaggc catgcccag 19

<210> 1629
<211> 21
<212> DNA
<213> Artificial Sequence

<220>
<223> Synthetic Construct

<400> 1629
tcagtaatca ctccctgtcc c 21

<210> 1630
<211> 21
<212> DNA
<213> Artificial Sequence

<220>
<223> Synthetic Construct

<400> 1630
aaaactagaa tagtggttgc c 21

<210> 1631
<211> 23
<212> DNA
<213> Artificial Sequence

<220>
<223> Synthetic Construct

<400> 1631
ccctctgagc taattagtgc tat 23

<210> 1632
<211> 23
<212> DNA
<213> Artificial Sequence

<220>

<223> Synthetic Construct

<400> 1632
acgagtaagt aaatgtgagt gga 23

<210> 1633
<211> 21
<212> DNA
<213> Artificial Sequence

<220>
<223> Synthetic Construct

<400> 1633
ttttgacacc atagctaagt c 21

<210> 1634
<211> 23
<212> DNA
<213> Artificial Sequence

<220>
<223> Synthetic Construct

<400> 1634
agaattaaga gcatcagtta aga 23

<210> 1635
<211> 21
<212> DNA
<213> Artificial Sequence

<220>
<223> Synthetic Construct

<400> 1635
tgtggagttt cttcttcatt c 21

<210> 1636
<211> 22
<212> DNA
<213> Artificial Sequence

<220>
<223> Synthetic Construct

<400> 1636
caagatgctt caacaacaac aa 22

<210> 1637
<211> 20
<212> DNA
<213> Artificial Sequence

<220>
<223> Synthetic Construct

<400> 1637

ttttggaagt gtttgagaaa 20

<210> 1638
<211> 21
<212> DNA
<213> Artificial Sequence

<220>
<223> Synthetic Construct

<400> 1638
gaaaggtcag gaccagatag c 21

<210> 1639
<211> 23
<212> DNA
<213> Artificial Sequence

<220>
<223> Synthetic Construct

<400> 1639
tgttctctgt ttgttctcac tct 23

<210> 1640
<211> 21
<212> DNA
<213> Artificial Sequence

<220>
<223> Synthetic Construct

<400> 1640
caccaaagct agcagaagga a 21

<210> 1641
<211> 20
<212> DNA
<213> Artificial Sequence

<220>
<223> Synthetic Construct

<400> 1641
cagggcctga aaggaaccta 20

<210> 1642
<211> 19
<212> DNA
<213> Artificial Sequence

<220>
<223> Synthetic Construct

<400> 1642
ttcagttgcc agaagcagc 19

<210> 1643
<211> 24
<212> DNA
<213> Artificial Sequence

<220>
<223> Synthetic Construct

<400> 1643
gaatactctt agccctttca cagc 24

<210> 1644
<211> 21
<212> DNA
<213> Artificial Sequence

<220>
<223> Synthetic Construct

<400> 1644
acttaaatgg taaggtggca a 21

<210> 1645
<211> 23
<212> DNA
<213> Artificial Sequence

<220>
<223> Synthetic Construct

<400> 1645
tggttatgcc agttagagta aga 23

<210> 1646
<211> 24
<212> DNA
<213> Artificial Sequence

<220>
<223> Synthetic Construct

<400> 1646
cctctaggga ataatataga caga 24

<210> 1647
<211> 20
<212> DNA
<213> Artificial Sequence

<220>
<223> Synthetic Construct

<400> 1647
gcaatgctct ccaatagtaa 20

<210> 1648
<211> 23
<212> DNA

<213>  Artificial Sequence

<220>
<223>  Synthetic Construct

<400>  1648
caattaactt tacctgcttc ttt                                               23

<210>  1649
<211>  19
<212>  DNA
<213>  Artificial Sequence

<220>
<223>  Synthetic Construct

<400>  1649
agtgtcttgg aggcagggc                                                    19

<210>  1650
<211>  20
<212>  DNA
<213>  Artificial Sequence

<220>
<223>  Synthetic Construct

<400>  1650
aggttcacct ccagagcaag                                                   20

<210>  1651
<211>  19
<212>  DNA
<213>  Artificial Sequence

<220>
<223>  Synthetic Construct

<400>  1651
caggaccata gggtgaggg                                                    19

<210>  1652
<211>  18
<212>  DNA
<213>  Artificial Sequence

<220>
<223>  Synthetic Construct

<400>  1652
cctgaccttg ctaggggc                                                     18

<210>  1653
<211>  20
<212>  DNA
<213>  Artificial Sequence

<220>

<223> Synthetic Construct

<400> 1653
acagacagga cacacaccca 20

<210> 1654
<211> 19
<212> DNA
<213> Artificial Sequence

<220>
<223> Synthetic Construct

<400> 1654
gcaagcttcc ttcaccctg 19

<210> 1655
<211> 20
<212> DNA
<213> Artificial Sequence

<220>
<223> Synthetic Construct

<400> 1655
ccatatgcct gagaccaacc 20

<210> 1656
<211> 19
<212> DNA
<213> Artificial Sequence

<220>
<223> Synthetic Construct

<400> 1656
gctgtgtaag gaggtgccc 19

<210> 1657
<211> 20
<212> DNA
<213> Artificial Sequence

<220>
<223> Synthetic Construct

<400> 1657
atgggacgta tctcggatgt 20

<210> 1658
<211> 20
<212> DNA
<213> Artificial Sequence

<220>
<223> Synthetic Construct

<400> 1658

gactgttcag ggctcacgat 20

<210> 1659
<211> 19
<212> DNA
<213> Artificial Sequence

<220>
<223> Synthetic Construct

<400> 1659
gtttccatgc ttgtgaagg 19

<210> 1660
<211> 19
<212> DNA
<213> Artificial Sequence

<220>
<223> Synthetic Construct

<400> 1660
gtaggacagg tgggacgct 19

<210> 1661
<211> 20
<212> DNA
<213> Artificial Sequence

<220>
<223> Synthetic Construct

<400> 1661
ggctacctgg gtagtcatgg 20

<210> 1662
<211> 22
<212> DNA
<213> Artificial Sequence

<220>
<223> Synthetic Construct

<400> 1662
tctcacctga tgtggaagta gc 22

<210> 1663
<211> 20
<212> DNA
<213> Artificial Sequence

<220>
<223> Synthetic Construct

<400> 1663
ctgctctatc tcaccctccc 20

<210> 1664
<211> 21
<212> DNA
<213> Artificial Sequence

<220>
<223> Synthetic Construct

<400> 1664
tcagattgtg aatcctgagc a 21

<210> 1665
<211> 18
<212> DNA
<213> Artificial Sequence

<220>
<223> Synthetic Construct

<400> 1665
aagtggggat ggggaatg 18

<210> 1666
<211> 20
<212> DNA
<213> Artificial Sequence

<220>
<223> Synthetic Construct

<400> 1666
gactgatgct cagcctaaac 20

<210> 1667
<211> 21
<212> DNA
<213> Artificial Sequence

<220>
<223> Synthetic Construct

<400> 1667
aggctagttc tatcctatgc c 21

<210> 1668
<211> 23
<212> DNA
<213> Artificial Sequence

<220>
<223> Synthetic Construct

<400> 1668
tgcatacaga aatatatgtc caa 23

<210> 1669
<211> 24
<212> DNA

<213> Artificial Sequence

<220>
<223> Synthetic Construct

<400> 1669
ggtttcttgg actacctgat cata 24

<210> 1670
<211> 18
<212> DNA
<213> Artificial Sequence

<220>
<223> Synthetic Construct

<400> 1670
gctcaggcag cgctatgt 18

<210> 1671
<211> 18
<212> DNA
<213> Artificial Sequence

<220>
<223> Synthetic Construct

<400> 1671
atctgtccca accctgcg 18

<210> 1672
<211> 20
<212> DNA
<213> Artificial Sequence

<220>
<223> Synthetic Construct

<400> 1672
gtgtgggatc agacctggag 20

<210> 1673
<211> 21
<212> DNA
<213> Artificial Sequence

<220>
<223> Synthetic Construct

<400> 1673
caataccatc aaccaattag a 21

<210> 1674
<211> 20
<212> DNA
<213> Artificial Sequence

<220>

<223> Synthetic Construct

<400> 1674
tggctgtctt ttgtggaatg 20

<210> 1675
<211> 20
<212> DNA
<213> Artificial Sequence

<220>
<223> Synthetic Construct

<400> 1675
atggtttcac tggcgaattt 20

<210> 1676
<211> 20
<212> DNA
<213> Artificial Sequence

<220>
<223> Synthetic Construct

<400> 1676
gagatgctcc ttctgcttct 20

<210> 1677
<211> 21
<212> DNA
<213> Artificial Sequence

<220>
<223> Synthetic Construct

<400> 1677
tgaactccat atgctacaac a 21

<210> 1678
<211> 20
<212> DNA
<213> Artificial Sequence

<220>
<223> Synthetic Construct

<400> 1678
tctggcatct ttcagtcagc 20

<210> 1679
<211> 20
<212> DNA
<213> Artificial Sequence

<220>
<223> Synthetic Construct

<400> 1679

cagctaccag gaaaacgtcc 20

<210> 1680
<211> 20
<212> DNA
<213> Artificial Sequence

<220>
<223> Synthetic Construct

<400> 1680
ggacgggagt ttacacgaag 20

<210> 1681
<211> 20
<212> DNA
<213> Artificial Sequence

<220>
<223> Synthetic Construct

<400> 1681
tgcatctaga ggcccaatct 20

<210> 1682
<211> 19
<212> DNA
<213> Artificial Sequence

<220>
<223> Synthetic Construct

<400> 1682
tcactccgag ttggcattt 19

<210> 1683
<211> 20
<212> DNA
<213> Artificial Sequence

<220>
<223> Synthetic Construct

<400> 1683
gaaggaggcc ctggtgtact 20

<210> 1684
<211> 19
<212> DNA
<213> Artificial Sequence

<220>
<223> Synthetic Construct

<400> 1684
cacagattcc tcccatagc 19

<210> 1685
<211> 21
<212> DNA
<213> Artificial Sequence

<220>
<223> Synthetic Construct

<400> 1685
tcagcaatca aacaaagcaa t 21

<210> 1686
<211> 20
<212> DNA
<213> Artificial Sequence

<220>
<223> Synthetic Construct

<400> 1686
aagctgtcat ggtttcttgt 20

<210> 1687
<211> 20
<212> DNA
<213> Artificial Sequence

<220>
<223> Synthetic Construct

<400> 1687
ttcccaattt caaccttgct 20

<210> 1688
<211> 22
<212> DNA
<213> Artificial Sequence

<220>
<223> Synthetic Construct

<400> 1688
gccttatcct gtatcctagc tg 22

<210> 1689
<211> 18
<212> DNA
<213> Artificial Sequence

<220>
<223> Synthetic Construct

<400> 1689
aaaagctgac caggccat 18

<210> 1690
<211> 23
<212> DNA

<213> Artificial Sequence

<220>
<223> Synthetic Construct

<400> 1690
tactagcttg cttcctaaat gcc 23

<210> 1691
<211> 23
<212> DNA
<213> Artificial Sequence

<220>
<223> Synthetic Construct

<400> 1691
actcactttc cattaattct gtg 23

<210> 1692
<211> 20
<212> DNA
<213> Artificial Sequence

<220>
<223> Synthetic Construct

<400> 1692
atttatcctc ctccctcccc 20

<210> 1693
<211> 20
<212> DNA
<213> Artificial Sequence

<220>
<223> Synthetic Construct

<400> 1693
tccaagagtg gtttgggaac 20

<210> 1694
<211> 22
<212> DNA
<213> Artificial Sequence

<220>
<223> Synthetic Construct

<400> 1694
ttgcacaatg tactaagtca ac 22

<210> 1695
<211> 24
<212> DNA
<213> Artificial Sequence

<220>

<223> Synthetic Construct

<400> 1695
ttactttgta ctctacagtg cttg 24

<210> 1696
<211> 24
<212> DNA
<213> Artificial Sequence

<220>
<223> Synthetic Construct

<400> 1696
gtgtaattta atagaacaaa cccc 24

<210> 1697
<211> 19
<212> DNA
<213> Artificial Sequence

<220>
<223> Synthetic Construct

<400> 1697
gctggtgggt ttgtgtgta 19

<210> 1698
<211> 20
<212> DNA
<213> Artificial Sequence

<220>
<223> Synthetic Construct

<400> 1698
caagatgcaa gagctctaaa 20

<210> 1699
<211> 21
<212> DNA
<213> Artificial Sequence

<220>
<223> Synthetic Construct

<400> 1699
aaagtgaaca actgaatggc a 21

<210> 1700
<211> 20
<212> DNA
<213> Artificial Sequence

<220>
<223> Synthetic Construct

<400> 1700

tagaggtggt tgttgcacag 20

<210> 1701
<211> 21
<212> DNA
<213> Artificial Sequence

<220>
<223> Synthetic Construct

<400> 1701
agctctgtga aaggaagaaa a 21

<210> 1702
<211> 19
<212> DNA
<213> Artificial Sequence

<220>
<223> Synthetic Construct

<400> 1702
ttcccagggt tttctgagc 19

<210> 1703
<211> 21
<212> DNA
<213> Artificial Sequence

<220>
<223> Synthetic Construct

<400> 1703
gtcatataat ccaacaatcc c 21

<210> 1704
<211> 21
<212> DNA
<213> Artificial Sequence

<220>
<223> Synthetic Construct

<400> 1704
aaatgcagac atctcttgga g 21

<210> 1705
<211> 20
<212> DNA
<213> Artificial Sequence

<220>
<223> Synthetic Construct

<400> 1705
gatgcccaga actgaatgct 20

<210> 1706
<211> 20
<212> DNA
<213> Artificial Sequence

<220>
<223> Synthetic Construct

<400> 1706
gctatgaagg tggctcttcc 20

<210> 1707
<211> 22
<212> DNA
<213> Artificial Sequence

<220>
<223> Synthetic Construct

<400> 1707
gcagcacata tctaaatggt cc 22

<210> 1708
<211> 24
<212> DNA
<213> Artificial Sequence

<220>
<223> Synthetic Construct

<400> 1708
tgatatgctg tgtttccatt aaca 24

<210> 1709
<211> 18
<212> DNA
<213> Artificial Sequence

<220>
<223> Synthetic Construct

<400> 1709
ttgagcccca ggacattc 18

<210> 1710
<211> 23
<212> DNA
<213> Artificial Sequence

<220>
<223> Synthetic Construct

<400> 1710
aagagctata cttgctctaa aca 23

<210> 1711
<211> 21
<212> DNA

<213> Artificial Sequence

<220>
<223> Synthetic Construct

<400> 1711
ctctaggaaa cagtctggct t 21

<210> 1712
<211> 19
<212> DNA
<213> Artificial Sequence

<220>
<223> Synthetic Construct

<400> 1712
agcagcagag aggggaaac 19

<210> 1713
<211> 20
<212> DNA
<213> Artificial Sequence

<220>
<223> Synthetic Construct

<400> 1713
tgcaaagcag cttatattcc 20

<210> 1714
<211> 21
<212> DNA
<213> Artificial Sequence

<220>
<223> Synthetic Construct

<400> 1714
actaatgcct tgaaaggtaa a 21

<210> 1715
<211> 24
<212> DNA
<213> Artificial Sequence

<220>
<223> Synthetic Construct

<400> 1715
gcttcttaag agctcagtta atgc 24

<210> 1716
<211> 20
<212> DNA
<213> Artificial Sequence

<220>

<223> Synthetic Construct

<400> 1716
tgctaacaat gccatcttgc 20

<210> 1717
<211> 18
<212> DNA
<213> Artificial Sequence

<220>
<223> Synthetic Construct

<400> 1717
cacttgctgc caggaaaa 18

<210> 1718
<211> 21
<212> DNA
<213> Artificial Sequence

<220>
<223> Synthetic Construct

<400> 1718
gtatgcaaaa gtcctcctcc c 21

<210> 1719
<211> 20
<212> DNA
<213> Artificial Sequence

<220>
<223> Synthetic Construct

<400> 1719
ggaattggat caaatgatta 20

<210> 1720
<211> 19
<212> DNA
<213> Artificial Sequence

<220>
<223> Synthetic Construct

<400> 1720
taatggcatt tgctgtggt 19

<210> 1721
<211> 22
<212> DNA
<213> Artificial Sequence

<220>
<223> Synthetic Construct

<400> 1721

gattagggcc tcgtccttat ga 22

<210> 1722
<211> 24
<212> DNA
<213> Artificial Sequence

<220>
<223> Synthetic Construct

<400> 1722
gtaagacaaa tgaactaagg aggt 24

<210> 1723
<211> 23
<212> DNA
<213> Artificial Sequence

<220>
<223> Synthetic Construct

<400> 1723
tcctaaactt aaagagaaag ttg 23

<210> 1724
<211> 19
<212> DNA
<213> Artificial Sequence

<220>
<223> Synthetic Construct

<400> 1724
gaaaatgctg cagacaaaa 19

<210> 1725
<211> 18
<212> DNA
<213> Artificial Sequence

<220>
<223> Synthetic Construct

<400> 1725
gtcacccatc ccacaaac 18

<210> 1726
<211> 24
<212> DNA
<213> Artificial Sequence

<220>
<223> Synthetic Construct

<400> 1726
atagatatgc ttaacatacc agtg 24

<210> 1727
<211> 20
<212> DNA
<213> Artificial Sequence

<220>
<223> Synthetic Construct

<400> 1727
gtagattttg cccacgtttt 20

<210> 1728
<211> 23
<212> DNA
<213> Artificial Sequence

<220>
<223> Synthetic Construct

<400> 1728
gacctaaatg taaaatgaca aac 23

<210> 1729
<211> 20
<212> DNA
<213> Artificial Sequence

<220>
<223> Synthetic Construct

<400> 1729
agagcctttg cttttccata 20

<210> 1730
<211> 20
<212> DNA
<213> Artificial Sequence

<220>
<223> Synthetic Construct

<400> 1730
gcaaactcag tcaaaaccca 20

<210> 1731
<211> 24
<212> DNA
<213> Artificial Sequence

<220>
<223> Synthetic Construct

<400> 1731
actttgttac gatatatggt tgtc 24

<210> 1732
<211> 21
<212> DNA

<213> Artificial Sequence

<220>
<223> Synthetic Construct

<400> 1732
tatctacaag atattggggc a 21

<210> 1733
<211> 19
<212> DNA
<213> Artificial Sequence

<220>
<223> Synthetic Construct

<400> 1733
ctctttccac cctccaccc 19

<210> 1734
<211> 21
<212> DNA
<213> Artificial Sequence

<220>
<223> Synthetic Construct

<400> 1734
tgaaaacaca agaagggaac a 21

<210> 1735
<211> 22
<212> DNA
<213> Artificial Sequence

<220>
<223> Synthetic Construct

<400> 1735
tggtgcatat gtgcttatgt gt 22

<210> 1736
<211> 23
<212> DNA
<213> Artificial Sequence

<220>
<223> Synthetic Construct

<400> 1736
attcactaca gcaaagacat gga 23

<210> 1737
<211> 21
<212> DNA
<213> Artificial Sequence

<220>

<223> Synthetic Construct

<400> 1737
tgggattcct gagtctaatg g 21

<210> 1738
<211> 21
<212> DNA
<213> Artificial Sequence

<220>
<223> Synthetic Construct

<400> 1738
tcattgcaga aagcagtatg a 21

<210> 1739
<211> 20
<212> DNA
<213> Artificial Sequence

<220>
<223> Synthetic Construct

<400> 1739
aagacccaga gcaatccctt 20

<210> 1740
<211> 23
<212> DNA
<213> Artificial Sequence

<220>
<223> Synthetic Construct

<400> 1740
tgcactatca gacactagca ttc 23

<210> 1741
<211> 20
<212> DNA
<213> Artificial Sequence

<220>
<223> Synthetic Construct

<400> 1741
acccaggctt ttgaacttgc 20

<210> 1742
<211> 20
<212> DNA
<213> Artificial Sequence

<220>
<223> Synthetic Construct

<400> 1742

gaggaaatga gccatggaaa 20

<210> 1743
<211> 21
<212> DNA
<213> Artificial Sequence

<220>
<223> Synthetic Construct

<400> 1743
gggttgacac tttccagatc a 21

<210> 1744
<211> 24
<212> DNA
<213> Artificial Sequence

<220>
<223> Synthetic Construct

<400> 1744
tgttttctta tttcctggct acat 24

<210> 1745
<211> 22
<212> DNA
<213> Artificial Sequence

<220>
<223> Synthetic Construct

<400> 1745
agtgtgagca ctaagttgaa ga 22

<210> 1746
<211> 21
<212> DNA
<213> Artificial Sequence

<220>
<223> Synthetic Construct

<400> 1746
tcagccctag tctgacagtc c 21

<210> 1747
<211> 20
<212> DNA
<213> Artificial Sequence

<220>
<223> Synthetic Construct

<400> 1747
ttgtgccatc tccaacttct 20

<210> 1748
<211> 20
<212> DNA
<213> Artificial Sequence

<220>
<223> Synthetic Construct

<400> 1748
gtacagcatc ctgctgcaaa 20

<210> 1749
<211> 23
<212> DNA
<213> Artificial Sequence

<220>
<223> Synthetic Construct

<400> 1749
catgagaagg atataaaagt ttg 23

<210> 1750
<211> 20
<212> DNA
<213> Artificial Sequence

<220>
<223> Synthetic Construct

<400> 1750
aaggccttca aaataagaat 20

<210> 1751
<211> 20
<212> DNA
<213> Artificial Sequence

<220>
<223> Synthetic Construct

<400> 1751
ttggccaaca tctcaacaga 20

<210> 1752
<211> 22
<212> DNA
<213> Artificial Sequence

<220>
<223> Synthetic Construct

<400> 1752
tcatttagca ttcccagact ca 22

<210> 1753
<211> 19
<212> DNA

<213> Artificial Sequence

<220>
<223> Synthetic Construct

<400> 1753
actctggggt gttctccga 19

<210> 1754
<211> 20
<212> DNA
<213> Artificial Sequence

<220>
<223> Synthetic Construct

<400> 1754
tcgattccag gaagtacaga 20

<210> 1755
<211> 22
<212> DNA
<213> Artificial Sequence

<220>
<223> Synthetic Construct

<400> 1755
ttcaatagct gttcagacac aa 22

<210> 1756
<211> 21
<212> DNA
<213> Artificial Sequence

<220>
<223> Synthetic Construct

<400> 1756
gcactgggat agtctaattc t 21

<210> 1757
<211> 18
<212> DNA
<213> Artificial Sequence

<220>
<223> Synthetic Construct

<400> 1757
cattctgggc actgggag 18

<210> 1758
<211> 20
<212> DNA
<213> Artificial Sequence

<220>

<223> Synthetic Construct

<400> 1758
cagctcccat gatgacacta 20

<210> 1759
<211> 23
<212> DNA
<213> Artificial Sequence

<220>
<223> Synthetic Construct

<400> 1759
cctgaccact actcttcaaa aca 23

<210> 1760
<211> 20
<212> DNA
<213> Artificial Sequence

<220>
<223> Synthetic Construct

<400> 1760
ccctagtttc caccaatctg 20

<210> 1761
<211> 21
<212> DNA
<213> Artificial Sequence

<220>
<223> Synthetic Construct

<400> 1761
ggtgccatgt ctccatgtat c 21

<210> 1762
<211> 19
<212> DNA
<213> Artificial Sequence

<220>
<223> Synthetic Construct

<400> 1762
cccatcacta cagcaagcc 19

<210> 1763
<211> 23
<212> DNA
<213> Artificial Sequence

<220>
<223> Synthetic Construct

<400> 1763

aaaatagaac acagagtgta gga 23

<210> 1764
<211> 20
<212> DNA
<213> Artificial Sequence

<220>
<223> Synthetic Construct

<400> 1764
attcaatttc cttgaccaat 20

<210> 1765
<211> 19
<212> DNA
<213> Artificial Sequence

<220>
<223> Synthetic Construct

<400> 1765
aatcaggctt tggacaggc 19

<210> 1766
<211> 24
<212> DNA
<213> Artificial Sequence

<220>
<223> Synthetic Construct

<400> 1766
gacttgagca acacaaatgt atga 24

<210> 1767
<211> 21
<212> DNA
<213> Artificial Sequence

<220>
<223> Synthetic Construct

<400> 1767
accattatgt tgttcctttt c 21

<210> 1768
<211> 21
<212> DNA
<213> Artificial Sequence

<220>
<223> Synthetic Construct

<400> 1768
gccctgtctc tctgtagctt t 21

<210> 1769
<211> 18
<212> DNA
<213> Artificial Sequence

<220>
<223> Synthetic Construct

<400> 1769
cggccctcct ttctgaag 18

<210> 1770
<211> 19
<212> DNA
<213> Artificial Sequence

<220>
<223> Synthetic Construct

<400> 1770
aggaaacttg gagggtggg 19

<210> 1771
<211> 20
<212> DNA
<213> Artificial Sequence

<220>
<223> Synthetic Construct

<400> 1771
taggatctga agggctccca 20

<210> 1772
<211> 19
<212> DNA
<213> Artificial Sequence

<220>
<223> Synthetic Construct

<400> 1772
acagcatagc tccactgcc 19

<210> 1773
<211> 20
<212> DNA
<213> Artificial Sequence

<220>
<223> Synthetic Construct

<400> 1773
taaggcgatc aagaacaccc 20

<210> 1774
<211> 20
<212> DNA

<213> Artificial Sequence

<220>
<223> Synthetic Construct

<400> 1774
ttttacgacg gatgggagat 20

<210> 1775
<211> 19
<212> DNA
<213> Artificial Sequence

<220>
<223> Synthetic Construct

<400> 1775
gtgctaaggc tggtgtccc 19

<210> 1776
<211> 22
<212> DNA
<213> Artificial Sequence

<220>
<223> Synthetic Construct

<400> 1776
ctatagtagg tggaggtgca gg 22

<210> 1777
<211> 21
<212> DNA
<213> Artificial Sequence

<220>
<223> Synthetic Construct

<400> 1777
tgaagaggaa gaagaacctc c 21

<210> 1778
<211> 20
<212> DNA
<213> Artificial Sequence

<220>
<223> Synthetic Construct

<400> 1778
ggtttgggtt cactgagttt 20

<210> 1779
<211> 18
<212> DNA
<213> Artificial Sequence

<220>

<223> Synthetic Construct

<400> 1779
agagcagccc cggtaact 18

<210> 1780
<211> 20
<212> DNA
<213> Artificial Sequence

<220>
<223> Synthetic Construct

<400> 1780
ctccttctct ccagcccatt 20

<210> 1781
<211> 22
<212> DNA
<213> Artificial Sequence

<220>
<223> Synthetic Construct

<400> 1781
ttgaatttca gctacaccta ga 22

<210> 1782
<211> 20
<212> DNA
<213> Artificial Sequence

<220>
<223> Synthetic Construct

<400> 1782
aatagtggct ggttgccaat 20

<210> 1783
<211> 20
<212> DNA
<213> Artificial Sequence

<220>
<223> Synthetic Construct

<400> 1783
atggacggga tgtacgtgtc 20

<210> 1784
<211> 20
<212> DNA
<213> Artificial Sequence

<220>
<223> Synthetic Construct

<400> 1784

tgagcagttg ctttgcattc 20

<210> 1785
<211> 21
<212> DNA
<213> Artificial Sequence

<220>
<223> Synthetic Construct

<400> 1785
ttggacttct tgtgtttgtg a 21

<210> 1786
<211> 21
<212> DNA
<213> Artificial Sequence

<220>
<223> Synthetic Construct

<400> 1786
tcagcaaggg aattcaggtt a 21

<210> 1787
<211> 20
<212> DNA
<213> Artificial Sequence

<220>
<223> Synthetic Construct

<400> 1787
ggggccactt agaagatgtg 20

<210> 1788
<211> 20
<212> DNA
<213> Artificial Sequence

<220>
<223> Synthetic Construct

<400> 1788
catcccatat gatggctgtg 20

<210> 1789
<211> 22
<212> DNA
<213> Artificial Sequence

<220>
<223> Synthetic Construct

<400> 1789
ggacactctg aaggaactca gg 22

<210> 1790
<211> 20
<212> DNA
<213> Artificial Sequence

<220>
<223> Synthetic Construct

<400> 1790
cagccaagga ggaggatgat 20

<210> 1791
<211> 20
<212> DNA
<213> Artificial Sequence

<220>
<223> Synthetic Construct

<400> 1791
tcggagggta ctcaggaaag 20

<210> 1792
<211> 19
<212> DNA
<213> Artificial Sequence

<220>
<223> Synthetic Construct

<400> 1792
gattctggct gatggggac 19

<210> 1793
<211> 20
<212> DNA
<213> Artificial Sequence

<220>
<223> Synthetic Construct

<400> 1793
atccaccatt gcagatgtca 20

<210> 1794
<211> 20
<212> DNA
<213> Artificial Sequence

<220>
<223> Synthetic Construct

<400> 1794
tttagggatt tgcaaggtca 20

<210> 1795
<211> 19
<212> DNA

<213> Artificial Sequence

<220>
<223> Synthetic Construct

<400> 1795
aactcgatga aagtggccc 19

<210> 1796
<211> 20
<212> DNA
<213> Artificial Sequence

<220>
<223> Synthetic Construct

<400> 1796
ccaaggggag agtctgagga 20

<210> 1797
<211> 18
<212> DNA
<213> Artificial Sequence

<220>
<223> Synthetic Construct

<400> 1797
ccaacacgca gagtgcca 18

<210> 1798
<211> 21
<212> DNA
<213> Artificial Sequence

<220>
<223> Synthetic Construct

<400> 1798
catgcagtgt ctgcagtttt c 21

<210> 1799
<211> 18
<212> DNA
<213> Artificial Sequence

<220>
<223> Synthetic Construct

<400> 1799
tcactcgcca tgtgcagg 18

<210> 1800
<211> 18
<212> DNA
<213> Artificial Sequence

<220>

<223>  Synthetic Construct

<400>  1800
gaagcagaga aatgcggg                                                18

## Description

### RELATED APPLICATIONS

The present application claims the benefit of U.S. Provisional Patent Application No. 62/342,839, filed May 27, 2016, the disclosure of which is hereby incorporated by reference in its entirety for all purposes.

### FIELD OF THE INVENTION

Technology provided herein relates in part to methods, processes, machines and apparatuses for detecting genetic variations. In some embodiments, the technology relates to non-invasive assessment of a chromosome abnormality, which include, without limitation, prenatal tests for detecting an aneuploidy (e.g., trisomy 21 (Down syndrome) and trisomy 18 (Edward syndrome)) and copy number variations.

### BACKGROUND

Genetic information of living organisms (e.g., animals, plants and microorganisms) and other forms of replicating genetic information (e.g., viruses) is encoded in deoxyribonucleic acid (DNA) or ribonucleic acid (RNA). Genetic information is a succession of nucleotides or modified nucleotides representing the primary structure of chemical or hypothetical nucleic acids. In humans, the complete genome contains about 30,000 genes located on 24 chromosomes (i.e., 22 autosomes, an X chromosome and a Y chromosome; see The Human Genome, T. Strachan, BIOS Scientific Publishers, 1992). Each gene encodes a specific protein, which after expression via transcription and translation fulfills a specific biochemical function within a living cell.

Many medical conditions are caused by one or more genetic variations. Certain genetic variations cause medical conditions that include, for example, hemophilia, thalassemia, Duchenne Muscular Dystrophy (DMD), Huntington's Disease (HD), Alzheimer's Disease and Cystic Fibrosis (CF) (Human Genome Mutations, D. N. Cooper and M. Krawczak, BIOS Publishers, 1993). Such genetic diseases can result from an addition, substitution, or deletion of a single nucleotide in DNA of a particular gene. Certain birth defects are caused by a chromosomal abnormality, also referred to as an aneuploidy, such as Trisomy 21 (Down's Syndrome), Trisomy 13 (Patau Syndrome), Trisomy 18 (Edward's Syndrome), Monosomy X (Turner's Syndrome) and certain sex chromosome aneuploidies such as Klinefelter's Syndrome (XXY), for example. Another genetic variation is fetal gender, which can often be determined based on sex chromosomes X and Y.

Identifying one or more genetic variations (e.g., copy number alterations, single nucleotide variations, chromosome alterations, translocations, deletions, insertions, and the like) or variances can lead to diagnosis of, or determining predisposition to, a particular medical condition. Identifying a genetic variance can result in facilitating a medical decision and/or employing a helpful medical procedure. In certain cases, identification of one or more genetic variations or variances involves the analysis of circulating cell-free nucleic acid. Circulating cell-free nucleic acid (CCF-NA), such as cell-free DNA (CCF-DNA) for example, is composed of DNA fragments that originate from cell death and circulate in peripheral blood. Additionally, cell-free fetal DNA (CFF-DNA) can be detected in the maternal bloodstream and used for various noninvasive prenatal diagnostics.

### SUMMARY

Provided herein are methods useful for determining the presence or absence of a genetic variation. In some cases the genetic variation is a chromosomal aneuploidy detected in a sample of circulating cell free nucleic acid obtained from a pregnant female. For example, the methods can specifically enrich for a selected set of paralog targets to enable noninvasive detection of fetal trisomy 21 (T21) and trisomy 18 (T18), or other aneuploidies, using cell-free DNA (cfDNA) from maternal plasma. A method often amplifies multiple paralog targets in a single reaction. A method often measures amplified products using massively parallel sequencing and determines deviations from expected (euploid) paralog ratios based on the read depth from each paralog.

The methods provided herein are also useful for the detection of copy number variants (CNVs). CNV's have been associated with a number of diseases and conditions, including but not limited to, autism, reproductive challenges and cancer.

Also provided are systems, machines and computer program products that carry out processes, or parts of processes, described herein. Certain embodiments are described further in the following description, examples, claims and drawings.

## BRIEF DESCRIPTION OF THE DRAWINGS

The drawings illustrate certain embodiments of the technology and are not limiting. For clarity and ease of illustration, the drawings are not made to scale and, in some instances, various aspects may be shown exaggerated or enlarged to facilitate an understanding of particular embodiments.

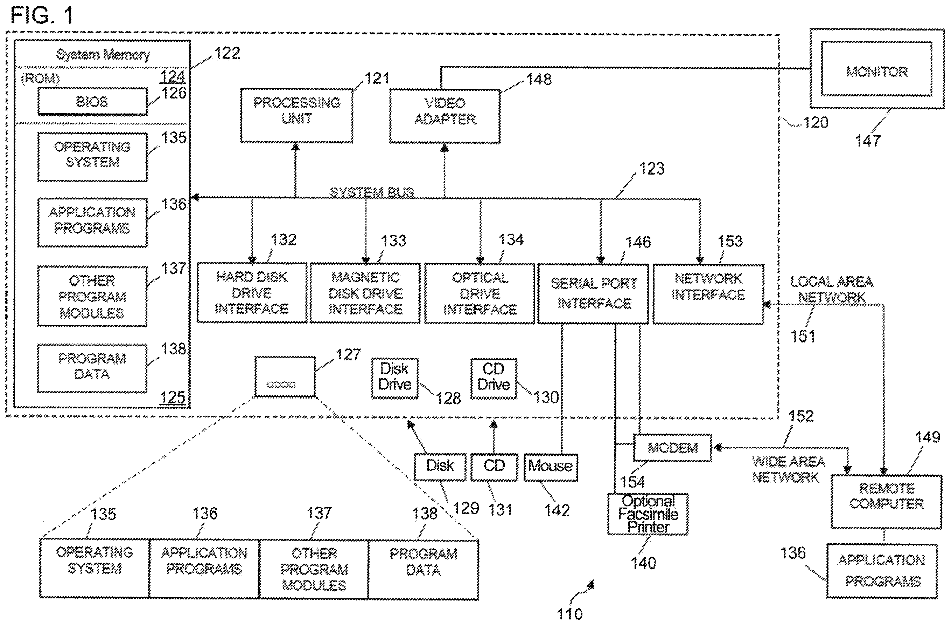

Fig. 1 shows an illustrative embodiment of a system in which certain embodiments of the technology may be implemented.

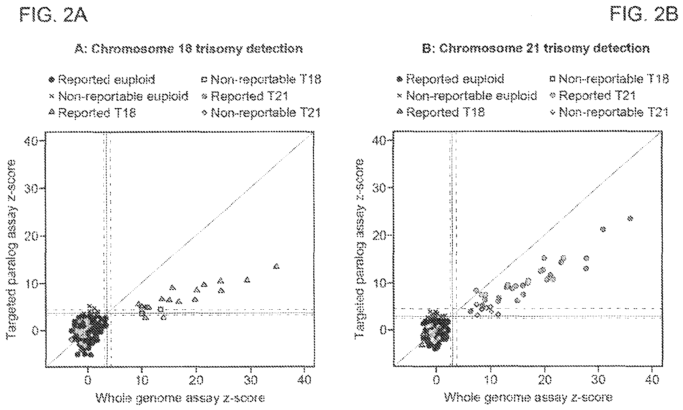

Fig. 2A and 2B shows fetal trisomy classification performance. Comparison of z-scores generated using MaterniT21® PLUS Assay ground truth data versus targeted paralog assay data for (FIG. 2A) fetal chromosome 18 trisomy and (FIG. 2B) fetal chromosome 21 trisomy. Dashed lines represent regional bounds for which no classification was achieved; solid diagonal represents z-score equivalency between the two datasets. The two independent fetal trisomy classifiers are significantly correlated in both cases (F-test, chromosome 21: $r^2$=0.85, p<$10^{-15}$, chromosome 18: $r^2$=0.79, p<$10^{-6}$). The observed classification gap between affected and unaffected samples was greater in whole genome data than in targeted paralog assay data.

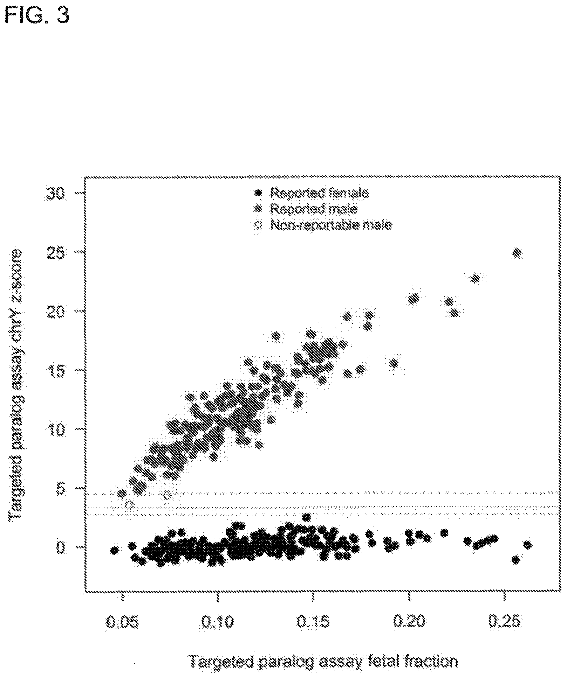

Fig. 3 shows fetal sex classification performance. Data are grouped by fetal sex as determined by the MaterniT21® PLUS Assay ground truth data. Horizontal broken line represents heuristic threshold for fetal sex classification, with points above this line classified as male fetuses. Non-reportable male fetal samples are visible with lower than expected percent of total sequencing reads mapped to chromosome Y assays.

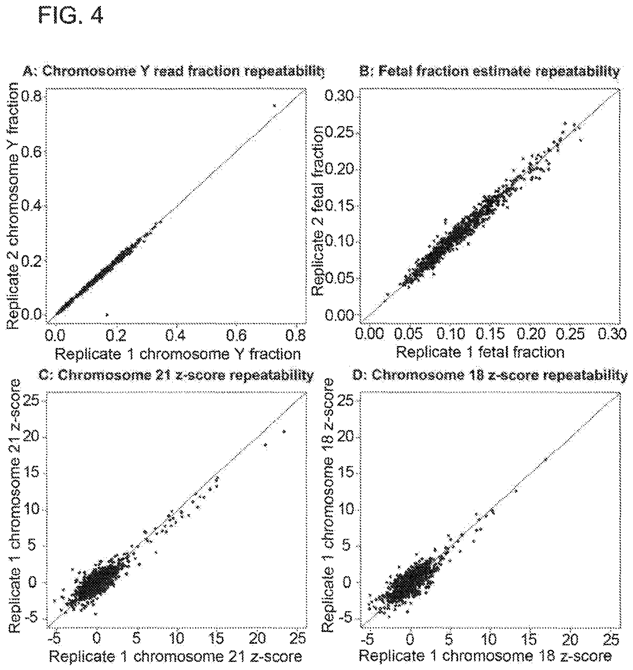

Fig. 4 shows reproducibility of targeted paralog assay data. (A) Comparison of percentage of reads mapped to chromosome Y assays in first versus second evaluation of data (fetal sex classification, $r^2$=0.99, p<$10^{-15}$, F-test). (B) Comparison of fraction fetal origin DNA estimates in first versus second evaluation of data ($r^2$=0.97, p<$10^{-15}$, F-test). (C) Comparison of z-scores for fetal chromosome 21 classification in first versus second evaluation of data ($r^2$=0.87, p<$10^{-15}$ , F-test). (D) Comparison of z-scores for fetal chromosome 18 classification in first versus second evaluation of data ($r^2$=0.75, p<$10^{-15}$, F-test). In all cases, data were found to be highly robust to run-to-run variation observed during sequencing.

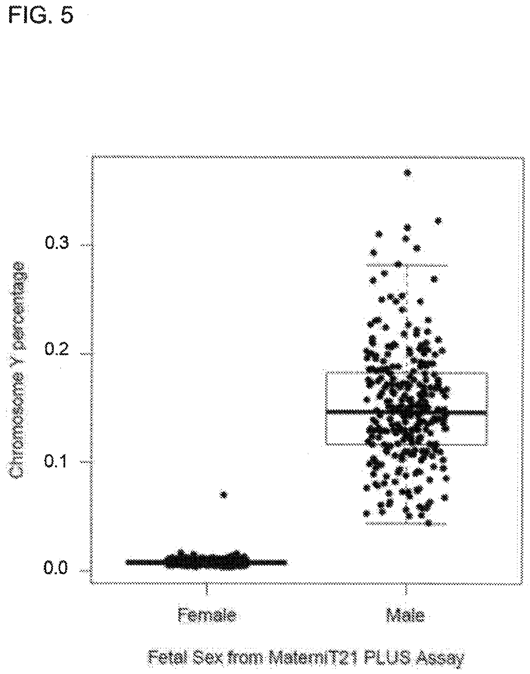

Fig. 5 shows fetal sex classification performance in classification model training dataset. Data are grouped by fetal sex as determined by the MaterniT21® PLUS Assay. The percent of total sequencing reads mapped to chromosome Y assays is significantly greater in male fetus samples than in female fetus samples (p<10-15, Wilcoxon test).

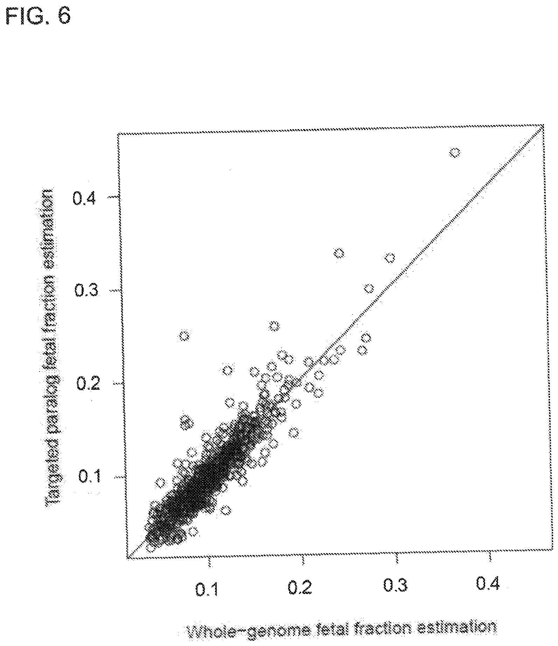

Fig. 6 shows an estimation of fetal fraction in classification model training dataset. Fetal fraction estimated by the MaterniT21® PLUS Assay versus the estimation using the SNP panel integrated as part of the targeted paralog assay. The two independent estimates of fetal fraction are significantly correlated.

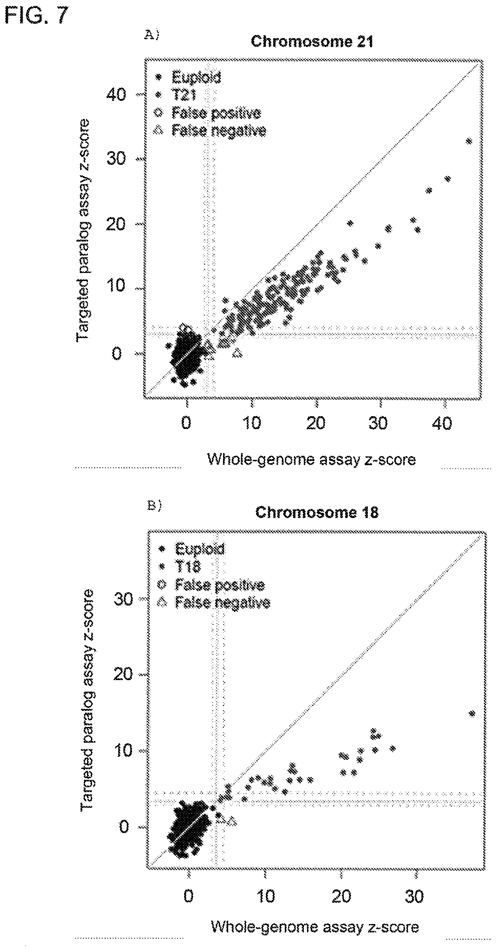

Fig. 7A and 7B shows fetal trisomy classification performance in classification model training dataset. Comparison of z-scores generated using MaterniT21® PLUS Assay data versus targeted paralog assay data for (FIG. 7A) fetal chromosome 21 trisomy and (FIG. 7B) fetal chromosome 18 trisomy. Broken lines represent regional bounds for which no classification can be achieved; solid diagonal represents z-score equivalency between the two datasets. The two independent fetal trisomy classifiers are significantly correlated in both cases (F-test, chromosome 21: $r^2$=0.86, p<$10^{-15}$. chromosome 18: r2=0.84, p<$10^{-15}$). In both cases, the magnitude of the z-score classifier is diminished in targeted paralog assay data versus MaterniT21® PLUS Assay data.

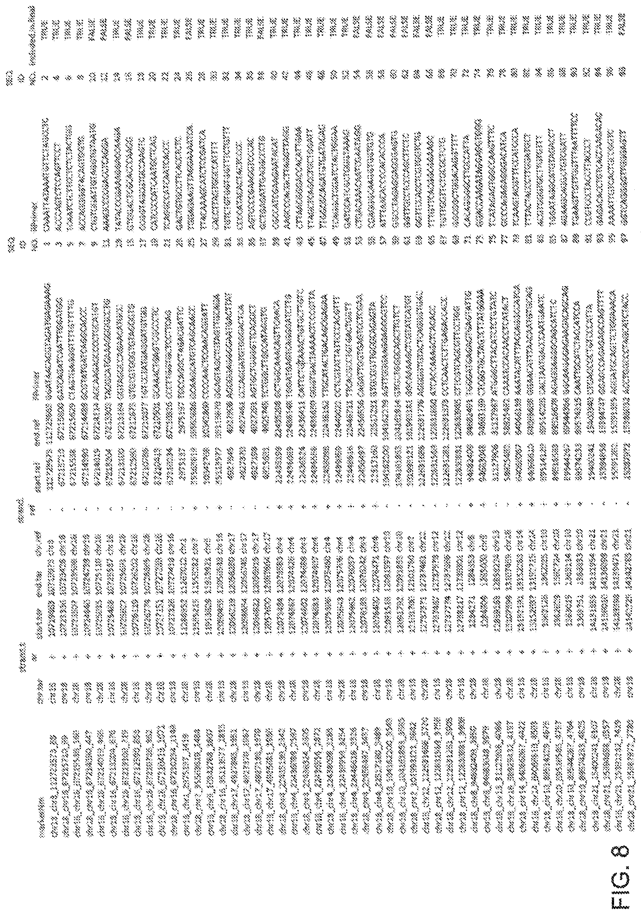

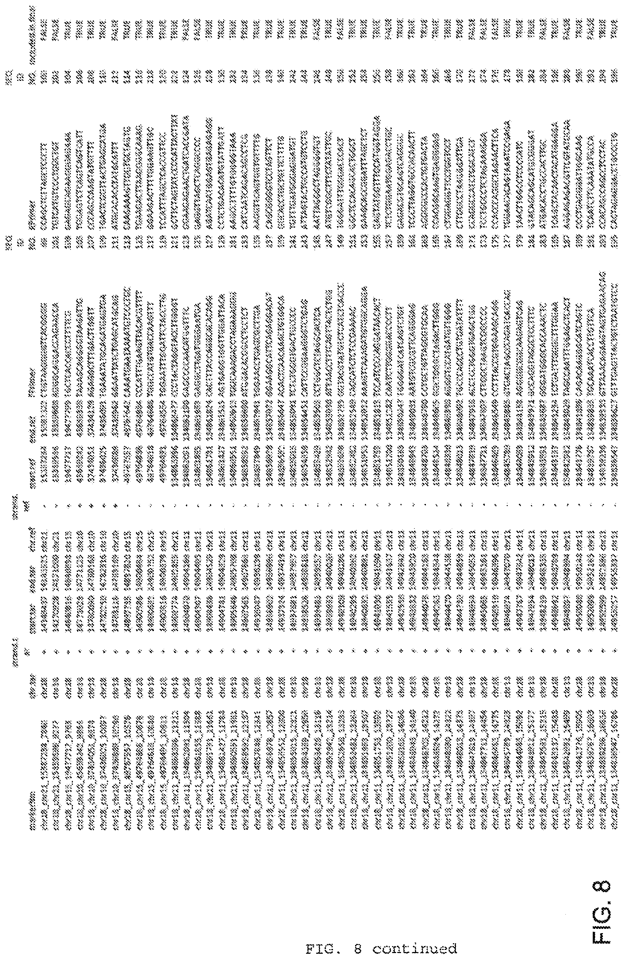

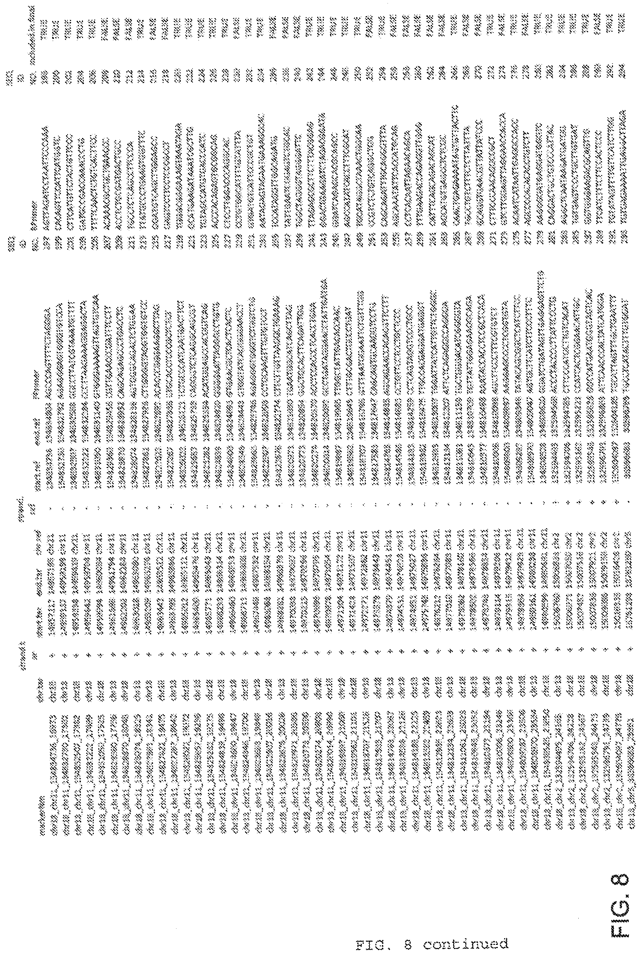

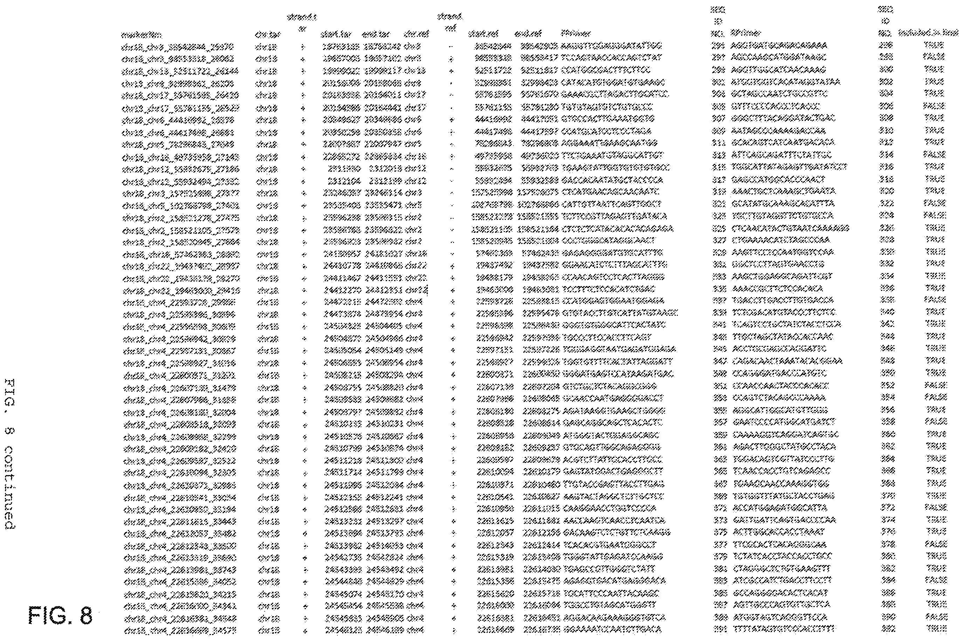

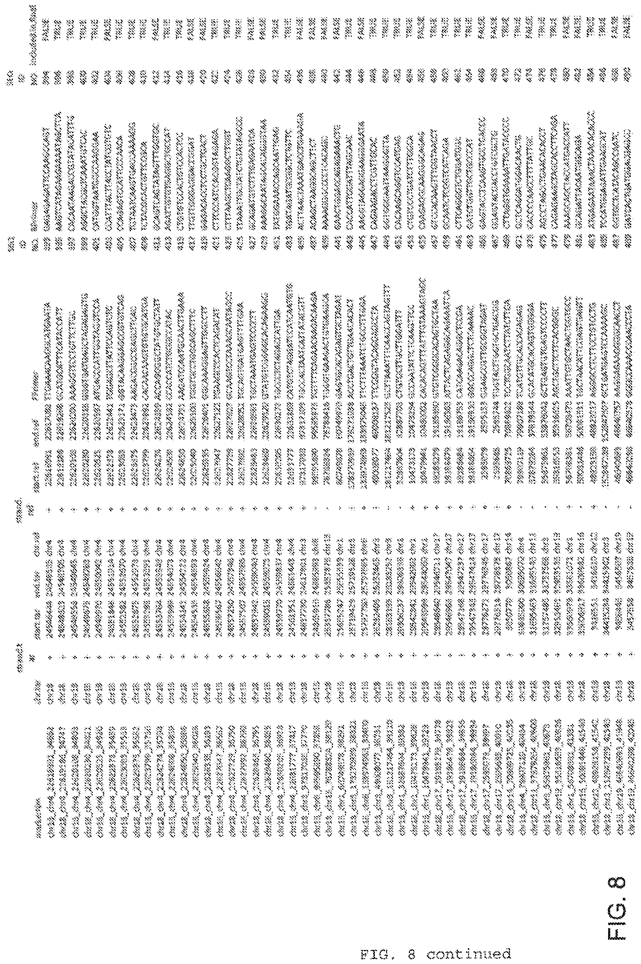

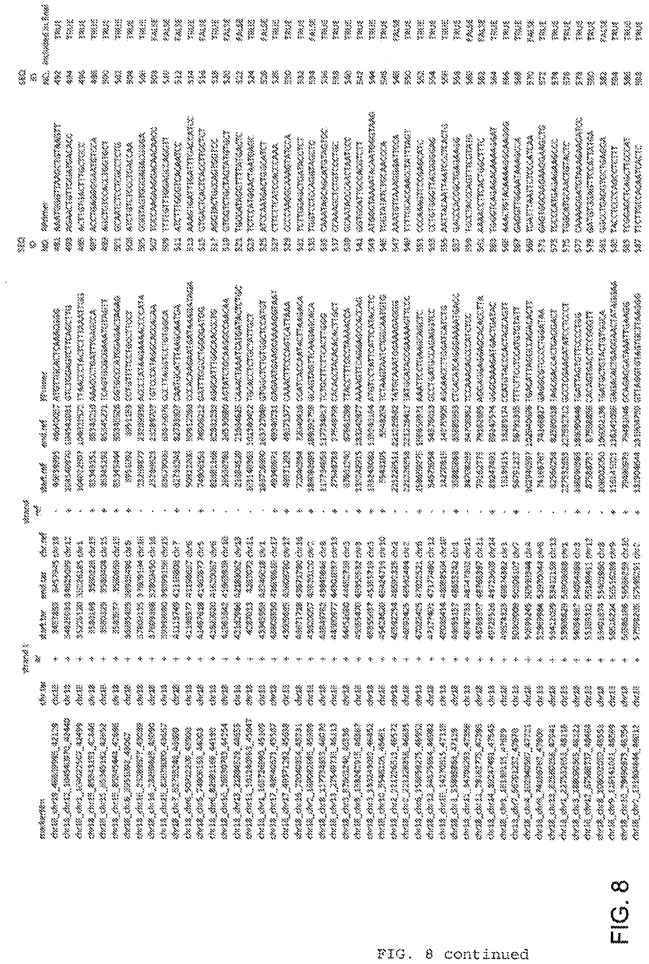

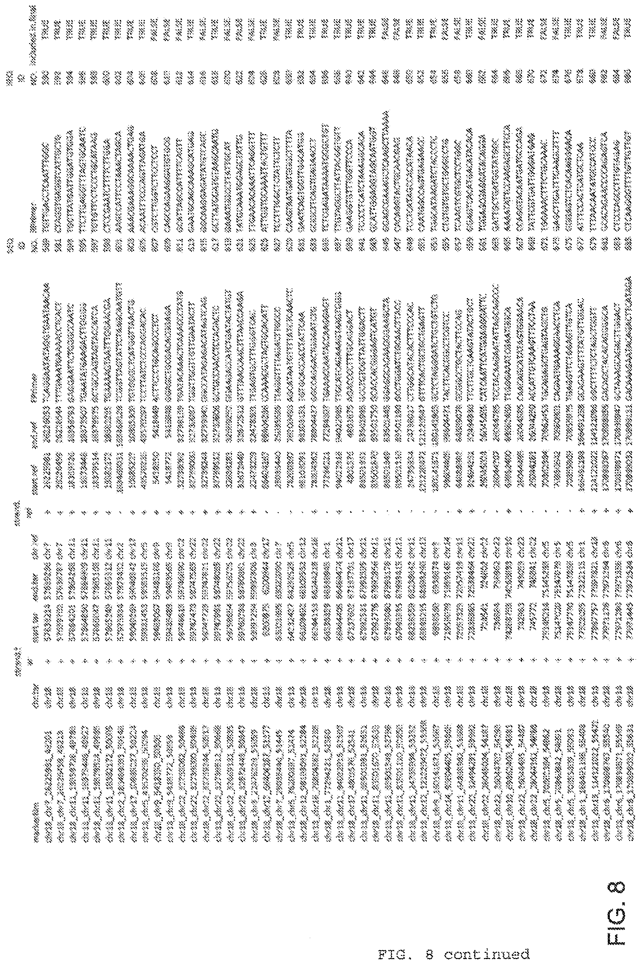

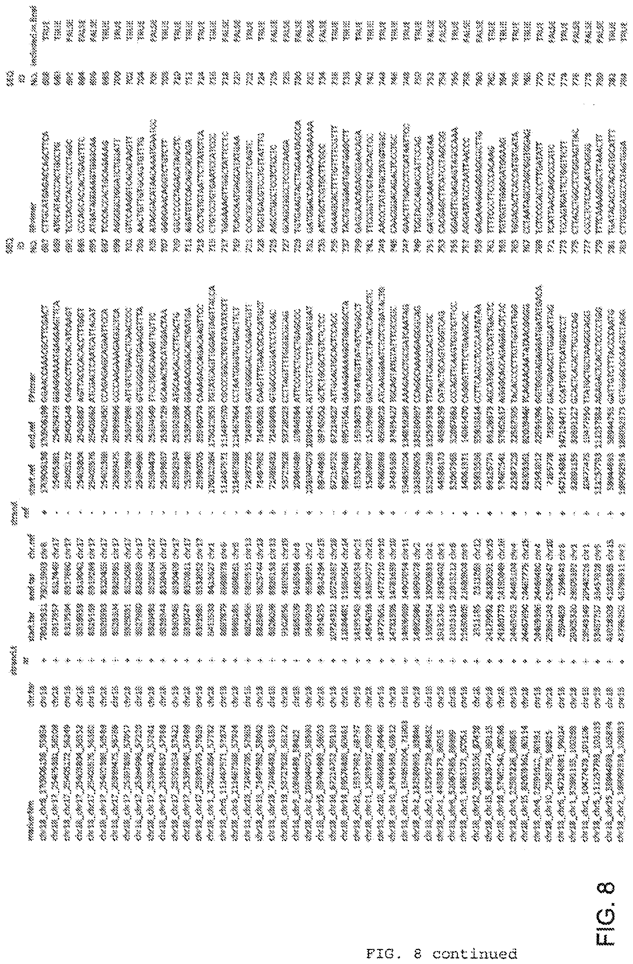

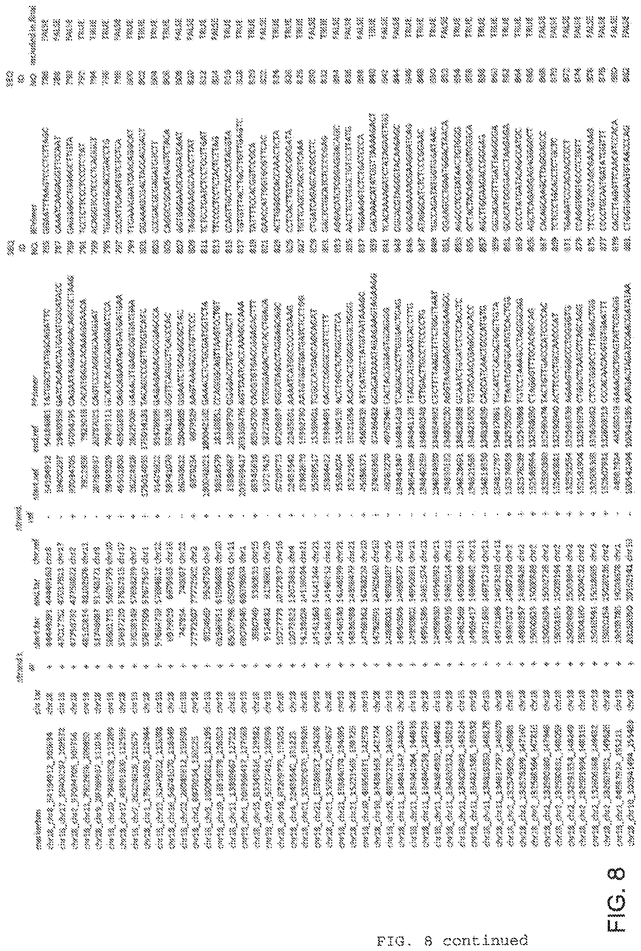

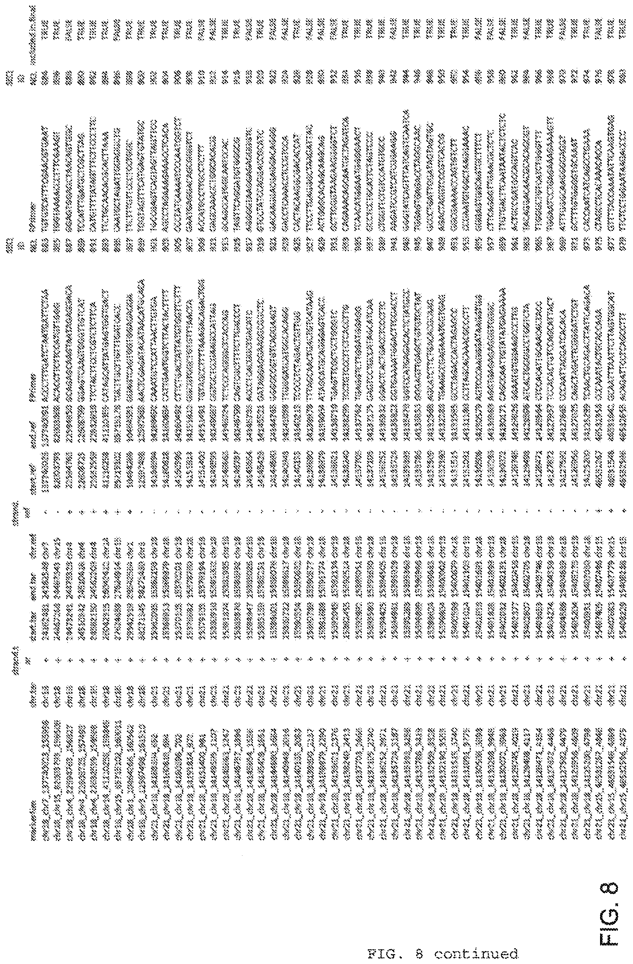

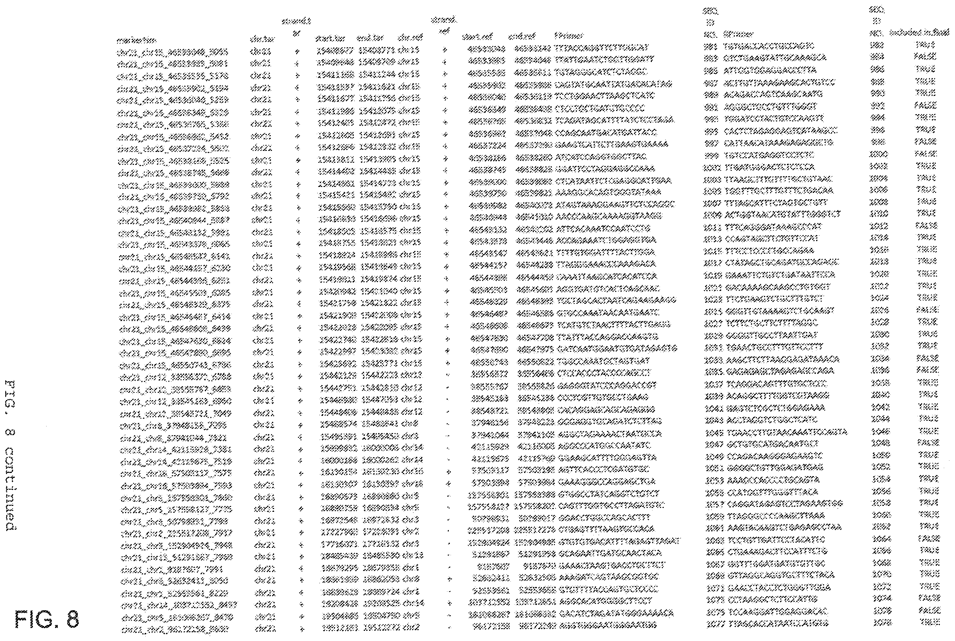

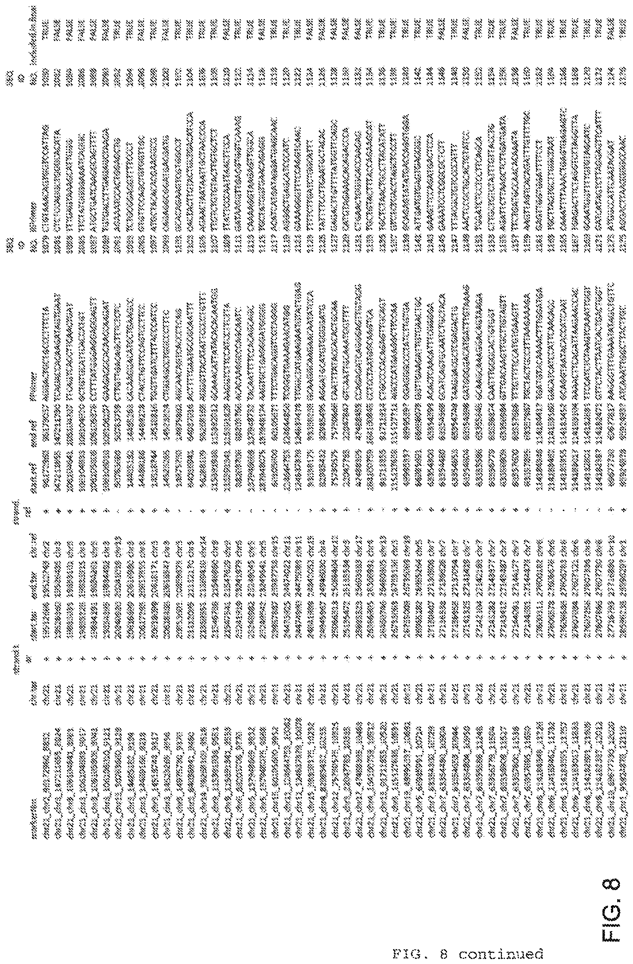

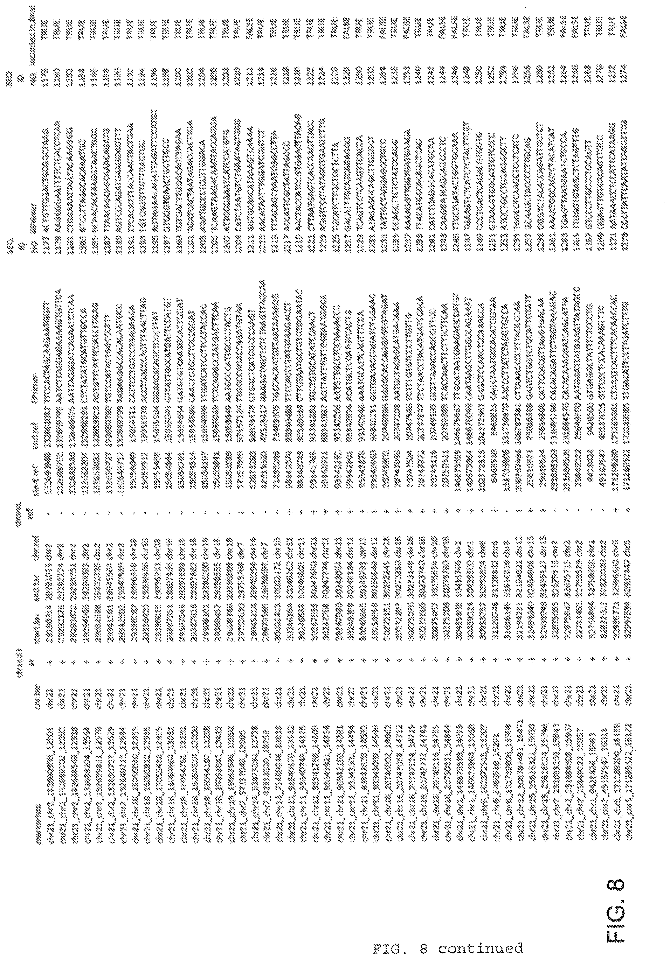

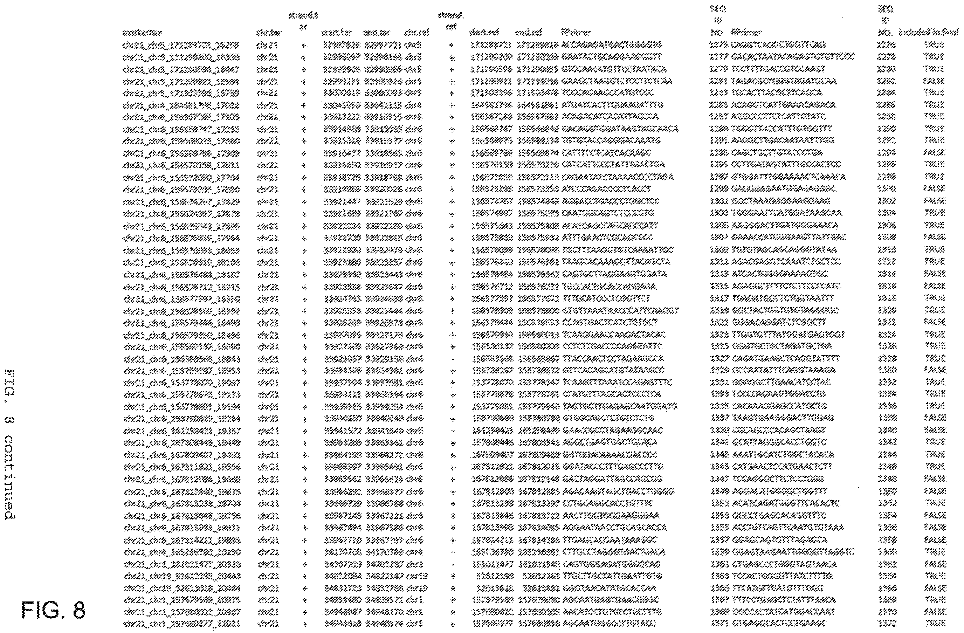

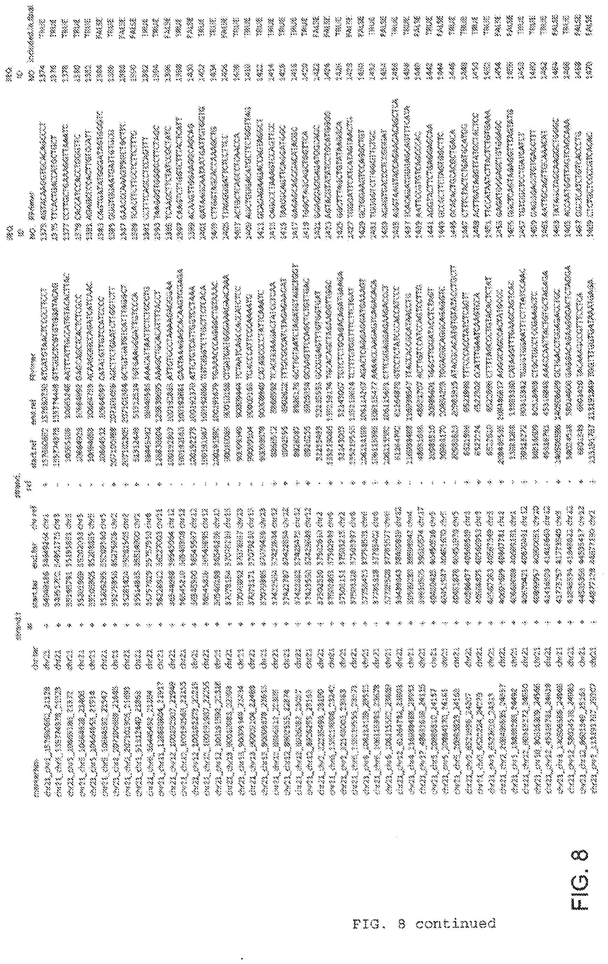

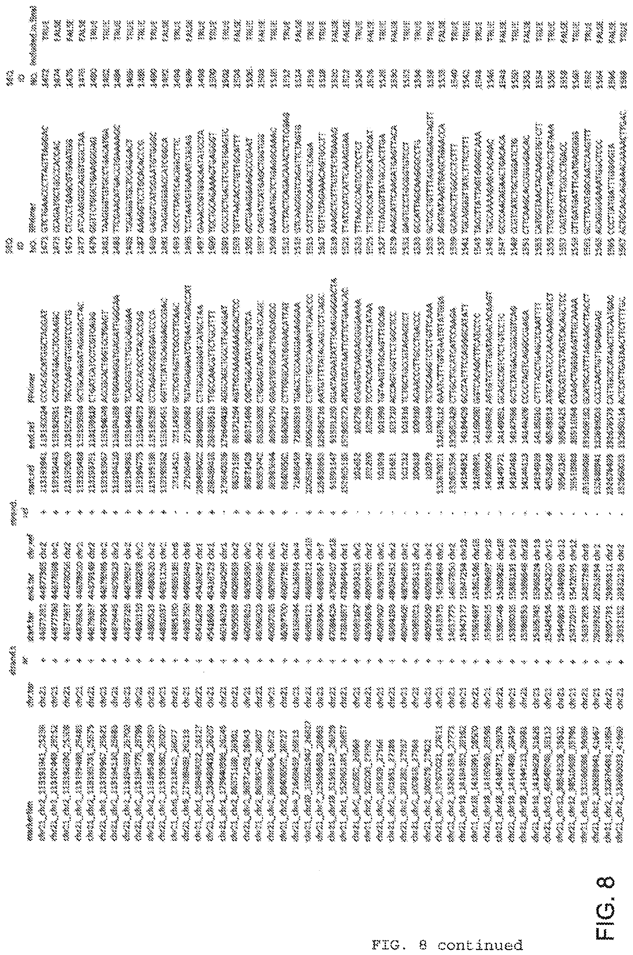

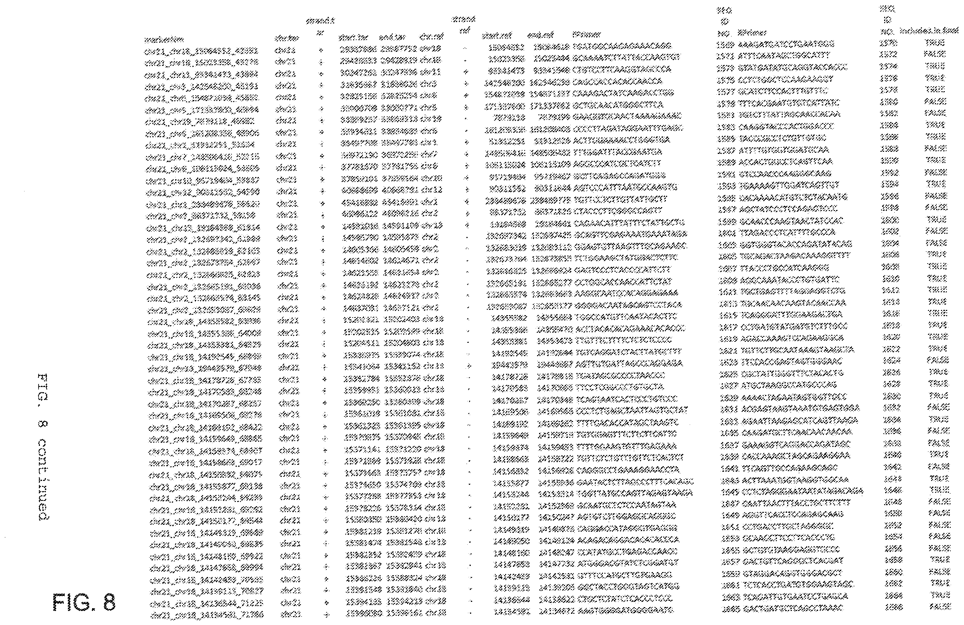

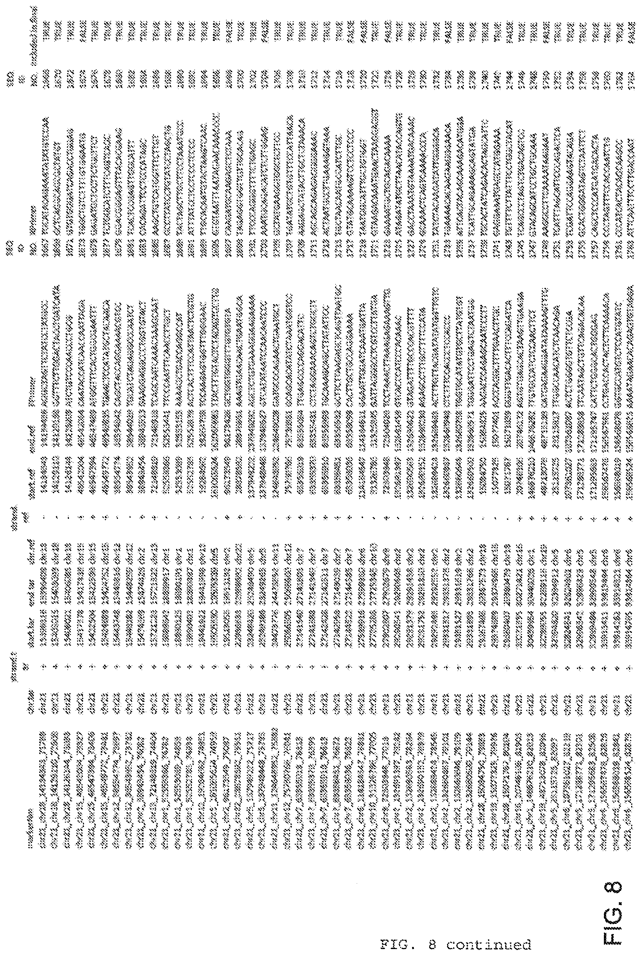

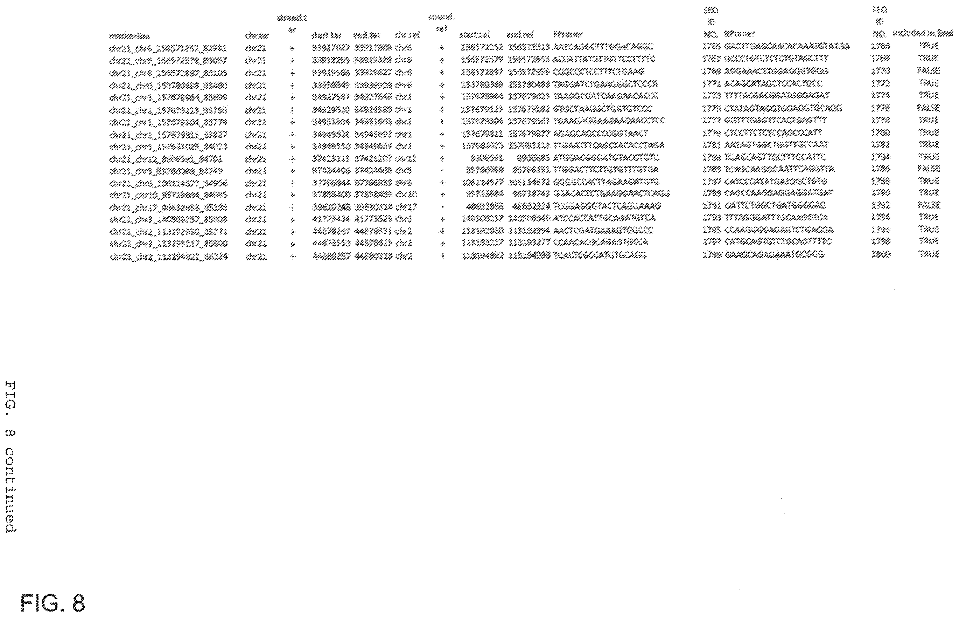

Fig. 8 lists paralogous polynucleotide species sets (assays) for chromosome 21 and chromosome 18. For each assay the target chromosome (21 or 18) position, reference chromosome position, forward primer, reverse primer and whether the particular assay was utilized in the testing of the method for aneuploidy detection is shown.

## DETAILED DESCRIPTION

Provided herein are methods useful for detecting genetic variations. In some embodiments, the technology relates to the non-invasive determination of the presence or absence of a chromosome abnormality.

For example, in some embodiments provided is a method for determining the presence or absence of a genetic variation, comprising:

a. amplifying in a single reaction, a plurality of paralogous polynucleotide species from nucleic acid in a sample, comprising contacting the nucleic acid with amplification primers under amplification conditions, wherein the paralogous polynucleotide species of each of the sets are amplified by a single pair of amplification primers and each primer of the pair of amplification primers is complementary to less than 20 positions in a human genome;

b. determining the amount of each amplified paralogous polynucleotide species in each of the sets;

c. determining a paralog ratio for each of the sets between the amount of each amplified paralogous polynucleotide species in each of the sets, thereby generating a plurality of paralog ratios; and

d. determining the presence or absence of the genetic variation based on the plurality of paralog ratios.

In some cases the genetic variant is a chromososmal abnormality. The chromosomal abnormality may be inherited. Or, the chromosomal abnomality may be a somatic mutation. In some cases the chromosmal abnormality is associated with cancer. In other cases, the chromosomal abnomality is associated with other genetic diseases. In some embodiments, the chromosomal abnormality is detected in fetal DNA. Or the chromosomal abnormality may be detected in the DNA of any subject (e.g., non-fetal male or female).

For example, methods described herein may be used to determine presence or absence of a fetal aneuploidy (e.g., full chromosome aneuploidy, partial chromosome aneuploidy or segmental chromosomal aberration (e.g., mosaicism, deletion and/or insertion)) for a test sample from a pregnant female bearing a fetus. Methods described herein sometimes detect trisomy for one or more chromosomes (e.g., chromosome 18, chromosome 21 or combination thereof) or segment thereof. Or, other aneuploidies may be detected. Provided herein are methods for non-invasive prenatal testting (NIPT) incorporating elements of targeted amplicon sequencing, but with targeting specifically designed to co-amplify exactly two paralogous targets using a single primer pair to facilitate self-normalization of each target through the determination of a locus-specific paralog ratio. One target paralog is mapped to a chromosome of interest (e.g., chromosomes 18 or 21) and a paired paralog sequence is mapped to an alternative autosome.

For example, provided in certain embodiments are methods for determining presence or absence of a chromosomal aneuploidy, that include: (a) amplifying in a single reaction at least 100 sets of paralogous polynucleotide species from nucleic acid in a sample of circulating cell free nucleic acid of a pregnant female comprising fetally derived and maternally derived nucleic acid, comprising contacting the circulating cell free nucleic acid with amplification primers under amplification conditions, wherein the paralogous polynucleotide species of each of the sets are amplified by a single pair of amplification primers and each primer of the pair of amplification primers is complementary to less than 20 positions in a human genome; (b) determining the amount of each amplified paralogous polynucleotide species in each of the sets; (c) determining a paralog ratio for each of the sets between the amount of each amplified paralogous polynucleotide species in each of the sets, thereby generating a plurality of paralog ratios; and (d) determining the presence or absence of a chromosomal aneuploidy based on the plurality of paralog ratios. Various embodiments are described in more detail herein.

*Sets of paralogous polynucleotide species*

The term "sets of paralogous polynucleotide species" refers to nucleotide sequence species located on different chromosomes at different loci that share a significant level of sequence identity. One paralogous polynucleotide species in a set is located in one chromosome and the other paralogous polynucleotide species of a set is located in another chromosome.

Paralogous polynucleotide species of a set share about 50%, 60%, 70%, 80%, 85%, 86%, 87%, 88%, 89%, 90%, 91%, 92%, 93% or 94%, and all intermediate values thereof, identity to one another in some embodiments. Paralogous polynucleotide species of a set are "substantially identical" to one another to one another in some embodiments, which refers to species that share 95%, 96%, 97%, 98% or 99% identity, or greater than 99% identity, with one another. In certain embodiments, paralogous polynucleotide species of a set may be identical to one another with the exception of one or two base pair mismatches. For example, paralogous polynucleotide species in a set may be identical to one another with the exception of one or two base pair mismatch for a nucleotide sequence species length of about 100 base pairs (e.g., about 60, 62, 64, 66, 68, 70, 72, 74, 76, 78, 80, 82, 84, 86, 88, 90, 92, 94, 96, 98, 100, 102, 104, 106, 108, 110, 112, 114, 116, 118 or 120 base pair sequence length). Paralogous polynucleotide species sometimes have a common evolutionary origin and sometimes are duplicated over time in a genome of interest. Paralogous polynucleotide species sometimes conserve sequence and gene structure (e.g., number and relative position of introns and exons and often transcript length).

A "set" includes paralogous polynucleotide species located on a target chromosome and one or more reference chromosomes. The term "reference chromosome" refers to a chromosome that includes a paralogous polynucleotide species as a subsequence, and is a chromosome not associated with a particular chromosome abnormality being screened. For example, in a prenatal screening method for Down syndrome (i.e., trisomy 21), chromosome 21 is the target chromosome and another chromosome (e.g., chromosome 5) is the reference chromosome. In certain embodiments, a reference chromosome can be associated with a chromosome abnormality. For example, chromosome 21 can be the target chromosome and chromosome 18 can be the reference chromosome when screening for Down syndrome, and chromosome 18 can the target chromosome and chromosome 21 can be the reference chromosome when screening for Edward syndrome. A set of paralogous polynucleotide species is sometimes referred to as an assay. Or, other sets of chromosomes may be assayed.

Base pair mismatches between paralogous polynucleotide species in a set are not significantly polymorphic in certain embodiments, and the nucleotides that give rise to the mismatches are present at a rate of over 95% of subjects and chromosomes in a given population (e.g., the same nucleotides that give rise to the mismatches are present in about 98%, 99% or over 99% of subjects and chromosomes in a population). Each paralogous polynucleotide species of a set, in its entirety, often is present in a significant portion of a population without modification (e.g., present without modification in about 97%, 98%, 99%, or over 99% of subjects and chromosomes in a population).

Paralogous polynucleotide species in a set may be of any convenient length. For example, paralogous polynucleotide species of a set can be about 50 to about 200 base pairs in length, or about 10, 15, 20, 25, 30, 35, 40, 45, 50, 55, 60, 65, 70, 75, 80, 85, 90, 95, 100, 200, base pairs in length. In some embodiments, a paralogous polynucleotide species in a set is about 100 base pairs in length (e.g., about 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118 or 120 base pairs in length). In certain embodiments, paralogous polynucleotide sequence species of a set are of identical length, and sometimes the paralogous polynucleotide sequence species of a set are of a different length (e.g., one species is longer by about 1 to about 20 nucleotides (e.g., about 2, 3, 4, 5, 6, 7, 8, 9, 10, 11, 12, 13, 14, 15, 16, 17, 18, 19, 20 nucleotides longer).

Paralogous polynucleotide species of a set are non-exonic in some embodiments, and sometimes the paralogous polynucleotide species of a set are intronic, partially intronic, partially exonic or partially non-exonic. In certain embodiments, the paralogous polynucleotide species of a set comprises an exonic nucleotide sequence.

Alignment techniques and sequence identity assessment methodology that are known can be used to locate potential paralogous polynucleotide species on either chromosome 18 or chromosome 21 (target chromosomes) and autosomes (reference chromosomes). Such analyses can be performed *in silico*. For example, a target chromosome (e.g., either chromosome 21 or chromosome 18) is sampled, for example, at 10 base pair intervals in 100 base pair segments and each segment aligned to a human reference genome. Segments which map to exactly two regions, one on the target chromosome and one on a reference chromosome, are extracted and merged to obtain paralogous polynucleotide species. Each set of paralogous polynucleotide species is aligned in order to locate nucleotide differences distinguishing target and reference species and to obtain the consensus sequence for primer design.

In some embodiments, the paralogous polynucleotide species sets (assays) comprise one or more of the assays in set forth in Fig. 8 for the analysis of chromosomes 21 and/or 18. Figure 8 provides the paralogous sequences and associated forward and reverse primer pairs for analyzing these particular sequences. Analogous sets of paralogous sequences and associated primer pairs may be derived for other chromosomes.

*Amplification Primers*

Because paralogous polynucleotide species of a set have a high degree of sequence similarity, single pairs of amplification primers can be designed to specifically amplify the two paralogous polynucleotide species of a set at a substantially reproducible level. The term "substantially reproducible level" as used herein refers to consistency of amplification levels for the paralogous polynucleotide species of a set. In some embodiments, a substantially reproducible level varies by 10%, 5%, 4%, 3%, 2%, 1.5%, 1%, 0.5%, 0.1%, 0.05%, 0.01%, 0.005% or 0.001%.

Amplification primers can be designed and synthesized using suitable processes. Amplification primer design software is available and primer design methodology is known. Amplification primer design generally consists of evaluating paralogous polynucleotide species on either side of the one or two base pair mismatch of the species and then choosing the suitable pair of sequences that best meet defined primer criteria. Certain relevant criteria for amplification primer design include: primer GC content, primer size and primer melting temperature. In some embodiments, the primer design parameters are: primer GC content = 30-70%, primer size = 18-24 base pairs and primer melting temperature = 52-64°C. In certain embodiments, the primer size can be 20 base pairs and the primer melting temperature can be 60°C. In certain embodiments, the amplification primers for a set of paralogous polynucleotide species are chosen to produce two equal-sized amplicons (target and reference). In some embodiments, the size of the amplicons is between 60-100 base pairs.

Designed amplification primers can be filtered to ensure desired sequence properties and to reduce possible sources of variation in the context of multiplexed PCR. In some embodiments, criteria for filtering amplification primers is based on one or more of the following properties: (1) each primer pair is predicted to produce exactly two equal-sized amplicons between 60-100bp, (2) each primer pair is predicted to produce zero off-target amplification events allowing maximal 3-base mis-priming, (3) each individual primer sequence is complementary to less than 20 positions in a reference human genome, (4) no primer overlaps with any annotated single nucleotide variant with >1% minor allele frequency in dbSNP build137 [39], (5) primers flank at least one position that distinguishes target from reference paralogous polynucleotide species, and (6) all independent amplicons (i.e., sets of paralogous polynucleotide species) are at least 100-bp apart. In certain embodiments, each individual primer of a pair of amplification primers for a set of paralogous polynucleotide species is complementary to less than 20 positions in a reference human genome. In certain embodiments, amplification primers satisfy all of the above criterion.

In some embodiments, to minimize undesired primer-primer interactions in the multiplexed PCR reaction, *in silico* multiplexing optimization is performed. The reverse complement of each primer sequence is locally aligned to all the other primers using known alignment programs such as the Biostrings function 'pairwiseAlignment' [35] in R version 3.0 [36] and a primer-specific alignment score can be calculated. Amplification primers with the largest total alignment score are identified and removed from the primer pool.

In some embodiments, the amplification primers comprise one or more of the amplification primer pairs shown in Fig. 8.

*Multiplex targeted amplification reactions*

Because of technical variance a single marker often is not sufficient for classification of a chromosomal abnormality, multiple markers may be used to reduce the variance and improve the accuracy. Thus, the methods herein provide multiplexed assays for the detection of genetic variants. The methods may be used, in some cases, for the analysis of maternal samples comprising fetal nucleic acid. In some embodiments, the sample is - procured through non-invasive means. For example, in some cases cell free DNA is used.

A typical maternal plasma sample from a pregnant female has between 4-32% (+-2%) cell-free fetal nucleic acid. In order to reliably and accurately detect a fetal chromosomal abnormality, with sufficient specificity and/or sensitivity suitable for a high degree of clinical utility, in a background of maternal nucleic acid, sensitive quantitative methods are needed that can take advantage of the increased power provided by using multiple markers (e.g., multiple sets of paralogous polynucleotide species). By increasing the number of sets of paralogous polynucleotide species, the specificity and sensitivity of the method can be high enough for robust clinical utility as a screening test for chromosome aneuploidy - even in a sample that comprises a mixture of fetal and maternal nucleic acid and a small fetal fraction.

In some embodiments, sets of paralogous polynucleotide species in the nucleic acid in a sample of circulating cell free nucleic acid of a pregnant female are enriched in multiplex PCR targeted enrichment. In some embodiments, a plurality of sets of paralogous polynucleotide species from the nucleic acid in a sample are amplified in a single reaction (multiplex reaction, multiplex amplification reaction). As used herein "multiplex amplification" refers to simultaneous amplification of many sets of paralogous polynucleotide species in one reaction by using multiple pairs of amplification primers (e.g., a different pair of amplification primers for each set of paralogous polynucleotide species). In some embodiments, multiplex amplification of a plurality paralogous polynucleotide sequences may be performed in a single reaction vessel or well.

In certain embodiments, the number of sets of paralogous polynucleotide species (assays) multiplexed include, without limitation, at least 100; at least 150, at least 200, at least 250, at least 300, at least 350, at least 400, at least 450, at least 500, at least 550, at least 600, at least 650, at least 700, at least 750, at least 800, at least 850, at least 900, at least 950 and at least 1000 sets of paralogous polynucleotide species or more per target region. The exact number is dictated by the number of independent measures (assays) required to estimate the predicted response using the multiplexed paralogs, e.g., the ability to obtain statistically significant results using the multiplexed paralogs. For example, a sample where the target abnormality is present in a minority fraction of DNA (discussed in detail herein) may require the use of more assays, i.e., higher number of sets of paralogous polynucleotide species, than a sample where the target abnormality is present in about 50% of the alleles (e.g., a haplotype analysis).

In certain embodiments, for example for the analysis of fetal DNA from a maternal sample, or cancer cells present at a low amount (<5%) there are at least 250 sets of paralogous polynucleotide species or at least 500 sets of paralogous polynucleotide species (chromosome 21 targets or chromosome 18 targets).

In certain embodiments, the number of sets of paralogous polynucleotide species (assays) multiplexed include, without limitation, about 50 to about 1,000 sets of paralogous polynucleotide species, and sometimes about 49-51, 51-53, 53-55, 55-57, 57-59, 59-61, 61-63, 63-65, 65-67, 67-69, 69-71, 71-73, 73-75, 75-77, 77-79, 79-81, 81-83, 83-85, 85-87, 87-89, 89-91, 91-93, 93-95, 95-97, 97-101, 101-103, 103-105, 105-107, 107-109, 109-111, 111-113, 113-115, 115-117, 117-119, 121-123, 123-125, 125-127, 127-129, 129-131, 131-133, 133-135, 135-137, 137-139, 139-141, 141-143, 143-145, 145-147, 147-149, 149-151, 151-153, 153-155, 155-157, 157-159, 159-161, 161-163, 163-165, 165-167, 167-169, 169-171, 171-173, 173-175, 175-177, 177-179, 179-181, 181-183, 183-185, 185-187, 187-189, 189-191, 191-193, 193-195, 195-197, 197-199, 199-201, 201-203, 203-205, 205-207, 207-209, 209-211, 211-213, 213-215, 215-217, 217-219, 219-221, 221-223, 223-225, 225-227, 227-229, 229-231, 231-233, 233-235, 235-237, 237-239, 239-241, 241-243, 243-245, 245-247, 247-249, 249-251, 251-253, 253-255, 255-257, 257-259, 259-261, 261-263, 263-265, 265-267, 267-269, 269-271, 271-273, 273-275, 275-277, 277-279, 279-281, 281-283, 283-285, 285-287, 287-289, 289-291, 291-293, 293-295, 295-297, 297-299, 299-301, 301- 303, 303- 305, 305- 307, 307- 309, 309- 311, 311- 313, 313- 315, 315- 317, 317- 319, 319-321, 321-323, 323-325, 325-327, 327-329, 329-331, 331-333, 333- 335, 335-337, 337-339, 339-341, 341-343, 343-345, 345-347, 347-349, 349-351, 351-353, 353-355, 355-357, 357-359, 359-361, 361-363, 363-365, 365-367, 367-369, 369-371, 371-373, 373-375, 375-377, 377-379, 379-381, 381-383, 383-385, 385-387, 387-389, 389-391, 391-393, 393-395, 395-397, 397-401, 401- 403, 403- 405, 405- 407, 407- 409, 409- 411, 411- 413, 413- 415, 415- 417, 417- 419, 419-421, 421-423, 423-425, 425-427, 427-429, 429-431, 431-433, 433- 435, 435-437, 437-439, 439-441, 441-443, 443-445, 445-447, 447-449, 449-451, 451-453, 453-455, 455-457, 457-459, 459-461, 461-463, 463-465, 465-467, 467-469, 469-471, 471-473, 473-475, 475-477, 477-479, 479-481, 481-483, 483-485, 485-487, 487-489, 489-491, 491-493, 493-495, 495-497, 497-501 sets of paralogous polynucleotide species or more.

Nucleic acids amplified in a single multiplex amplification reaction can include, but are not limited to the following illustrative examples. In some embodiments, the sets of paralogous polynucleotide species have the target chromosome as chromosome 21 and the reference chromosome as an autosome other than chromosome 21. In some embodiments, the sets of paralogous polynucleotide species have the target chromosome as chromosome 18 and the reference chromosome as an autosome other than chromosome 18. In some embodiments, the sets of paralogous polynucleotide species comprise sets with chromosome 21 as the target chromosome together with sets with chromosome 18 as the target chromosome. In certain embodiments, there are at least 250 sets of paralogous polynucleotide species for target chromosome 21 and there are at least 250 sets of paralogous polynucleotide species for target chromosome 18. In certain embodiments, there are at least 500 sets of paralogous polynucleotide species for target chromosome 21 and there are at least 500 sets of paralogous polynucleotide species for target chromosome 18. In certain embodiments, sets with chromosome 21 as the target chromosome together with sets with chromosome 18 as the target chromosome also include Y-chromosome polynucleotides and polynucleotides comprising single nucleotide polymorphic (SNP) loci. In certain embodiments, there are at least 10 Y-chromosome polynucleotides and at least 150 polynucleotides comprising single nucleotide polymorphic (SNP) loci.

Nucleic acid can be extracted from a biological sample obtained from a subject, as described herein. A subject sometimes is female or male (e.g., human female or human male), and a female can be a pregnant female at any suitable stage of pregnancy (e.g., first, second or third trimester). Extracted nucleic acid from the test subject sometimes is circulating cell free nucleic acid, and circulating cell free nucleic acid sometimes is from a blood plasma, blood serum or urine sample from a test subject. Extracted circulating cell free nucleic acid sometimes is not manipulated prior to performing amplification (e.g., the cell free nucleic acid often is not cleaved by an exonuclease or endonuclease). Any suitable method of amplification of nucleic acid may be utilized.

*Universal amplification reactions*

In some embodiments, each of the amplified sets of paralogous polynucleotide species is further amplified by universal PCR to prepare a library of enriched paralogous polynucleotide species for further analysis, such as by sequencing. In certain embodiments, universal PCR utilizes as primer sites, partial sequencing adaptor motifs that are introduced during the previous multiplex PCR. In some embodiments, universal PCR incorporates a sample specific DNA sequence that can be used to identify the sample. Sometimes identification of a sample specific DNA sequence is by sequencing.

*Detection and quantitation of amplified paralogous polynucleotide species*

Amplified paralogous polynucleotide species can be detected and quantified by any suitable detection process. Detection of amplified paralogous polynucleotide species is performed by any method that can detect single nucleotide differences (differences between the paralogous polynucleotide species on a target chromosome and the paired paralogous polynucleotide species on a reference chromosome). Methods include, but are not limited to, digital PCR, quantitative PCR, allele-specific hybridization-based assays, RFLP analysis, single nucleotide primer extension-based assays and sequencing-based assays. In some embodiments, detection and determining the amount of (quantitation) of amplified paralogous polynucleotide species is by a sequencing process. A sequencing process utilized often is a whole-genome sequencing process, and in some embodiments is a targeted sequencing process (e.g., a process that sequences a subset of all nucleic acid in a sample, the amplified paralogous polynucleotide species). A sequencing process utilized sometimes includes sequencing by synthesis. The sequence reads resulting from the sequencing process are mapped to a reference genome. Reads identified as representing paralogous polynucleotide species are quantified and typically expressed as counts.

In some embodiments, sequence reads are filtered prior to being quantified. In some embodiments, the filtering requires that sequence reads: (1) are of a minimum read length, (2) have a mapped position of the 5'-end of the read within 5 base pairs of the expected 5'-end of a designated target region, and (3) are not ambiguously aligned. In some embodiments, the minimum read length is greater than or equal to about 20 base pairs, greater than or equal to about 30 base pairs, greater than or equal to about 40 base pairs, greater than or equal to about 50 base pairs or greater than or equal to about 60 base pairs. In certain embodiments, the minimum read length is greater than or equal to about 45 base pairs.

*Paralog ratio*

In some embodiments, a paralog ratio is determined for the amount of each amplified paralogous polynucleotide species in a set in the sample. In some embodiments, a paralog ratio is calculated as the ratio of the counts of the paralogous polynucleotide species on a target chromosome to the counts of the paralogous polynucleotide species on a reference chromosome (i.e., target paralog read depth to reference paralog read depth).

*Fetal fraction and sex determination*

In some embodiments, the amount of fetal nucleic acid in a sample (e.g., fetal fraction) is quantified and used in conjunction with paralog ratios in the determination of the presence or absence of a chromosome aneuploidy. In certain embodiments, determination of the presence or absence of aneuploidy is made only for samples having a certain threshold amount of fetal nucleic acid. A suitable process for quantifying fetal fraction can be utilized, non-limiting examples of which include a mass spectrometric process, sequencing process or combination thereof. In certain embodiments, fetal fraction can be determined based on allelic ratios of polymorphic sequences (e.g., single nucleotide polymorphisms (SNPs)). In such a method for determining fetal fraction, for example, nucleotide sequence reads are obtained for a maternal sample and fetal fraction is determined by comparing the total number of nucleotide sequence reads that map to a first allele (inferred to be fetal) and the total number of nucleotide sequence reads that map to a second allele (inferred to be maternal) at an informative polymorphic site (e.g., SNP) in a reference genome (e.g., maternal homozygous SNP loci are distinguished from fetal heterozygous SNP loci). In some embodiments, multiple SNP loci are amplified and analyzed. In certain embodiments 50 to 200 SNP loci are amplified and analyzed. In some embodiments, 150 SNP loci are amplified and analyzed.

In some embodiments, fetal sex can be determined by amplifying selected Y chromosome regions and determining the proportion of sequence reads that align to chromosome Y. In some embodiments, multiple Y chromosome regions are amplified and analyzed. In certain embodiments, 5 to 50 Y chromosome regions are amplified and analyzed. In some embodiments, 10 Y chromosome regions are amplified and analyzed.

In certain embodiments, PCR primers designed to selectively amplify informative single nucleotide polymorphisms and PCR primers designed to selectively amplifying Y chromosome regions are included in the multiplex paralogous polynucleotide species amplification reaction.

*Data processing*

In some embodiments, data (e.g., counts, counts expressed as a ratio) is processed further (e.g., mathematically and/or statistically manipulated) and/or displayed to facilitate providing an outcome. In some embodiments, paralog ratios are transformed by a suitable method operation or mathematical operation known in the art. In certain embodiments, paralog ratios are logarithm-transformed to zero-center values.

In some embodiments, paralog ratios or logarithm-transformed paralog ratios are further processed. A processing step can comprise one or more mathematical and/or statistical manipulations. Any suitable mathematical and/or statistical manipulation (statistic), alone or in combination, may be used to analyze and/or manipulate paralog ratios or logarithm-transformed paralog ratios described herein. Any suitable number of mathematical and/or statistical manipulations can be used.

In certain embodiments, the statistic is a z-score. A z-score is a quotient of (a) subtraction product of (i) the logarithm-transformed paralog ratio, less (ii) a median of the logarithm-transformed paralog ratio of reference euploid samples, divided by (b) a MAD value derived from reference euploid samples. For each set of paralogous polynucleotide species (an assay), a median absolute deviation (MAD) is calculated. The median absolute deviation is the median absolute deviation of logarithm-transformed assay ratios in euploid samples.

In certain embodiments, the statistic is a weighted z-score. A weighted z-score is calculated by weighting individual non-outlier z-scores paralog ratios according to assay MAD or discriminatory power.

In some embodiments, a sample statistic is determined according to the statistic for each of the sets of paralogous polynucleotide species. A sample statistic can be any suitable mathematical and/or statistical manipulation. In certain embodiments, a sample statistic comprises summing statistics for each of sets of paralogous polynucleotide species. In some embodiments, a sample statistic comprises summing z-scores or weighted z-scores of logarithm-transformed paralog ratios.

A sample z-score can be calculated by z-scores of a plurality paralogous polynucleotide species sets, with the exclusion of outliers of z greater than 4 MAD or less than -4 MAD. A sample z-score can be determined according to

$$Z = \frac{\sum_{i=1}^{N} z_i}{\sqrt{N}},$$

where $z_i$ is the z-score of a non-outlier paralog ratio and N is the number of non-outlier paralogs.

A weighted sample z-score can be calculated by weighted z-scores of a plurality paralogous polynucleotide species sets according to

$$Z_w = \frac{\sum_{i=1}^{N} w_i z_i}{\sqrt{\sum_{i=1}^{N} w_i^2}},$$

where $w_i$ is the weight for each paralog ratio.

*Aneuploidy classification*

Methods described herein can provide a determination of the presence or absence of a chromosome aneuploidy based on paralog ratios, thereby providing an outcome. Methods herein sometimes provide a classification of a sample as fetal euploid, fetal trisomy 21 or fetal trisomy 18. Any classification method can be utilized, including but not limited to z-score, PCA, K-nearest neighbors, support vector machine and neural network.

In some embodiments, classification of the presence or absence of a chromosome aneuploidy is based on z-score. Thresholds for z-score based trisomy 21 and trisomy 18 classification can be determined based on training set samples (where the training set includes samples known to have trisomy 21 or trisomy 18 and samples known to not have trisomy 21 or trisomy 18).

In some embodiments, optimum performing paralogous polynucleotide species sets (assays or assay sets) are determined independently for fetal trisomy 21 and trisomy 18 classification. In certain embodiments, assays for aneuploidy classification can be assigned a relative weight based on a number of factors including, but not limited to: sufficient depth of sequencing coverage, small variance expressed as assay MAD, and measurable signal differentiating aneuploid and euploid populations as measured by the p-value from the Wilcoxon rank sum test on log-transformed assay ratios, for example.

Classification performance can be assessed in any suitable manner. In some embodiments, classification performance is assessed by determining a sensitivity and/or specificity for classification of multiple samples with known chromosome aneuploidy. In certain embodiments, a classification processes described herein is characterized by a sensitivity of about 90% or greater (e.g., sensitivity of 85, 86, 87, 88, 89, 90, 91, 92, 93, 94, 95, 96, 97, 98, 99, 99.5, 99.9% or greater) and/or independently by a specificity of about 90% or greater (e.g., specificity of 85, 86, 87, 88, 89, 90, 91, 92, 93, 94, 95, 96, 97, 98, 99, 99.5, 99.9% or greater). In certain embodiments, sensitivity is greater than 96% and specificity is greater than 99% for fetal aneuploidy detection.

In some embodiments, variations of z-score based classification methods can be utilized, including but not limited to weighting z-scores by assay variance or discriminatory power, merging assays based on physical location and assay correlations prior to z-score calculation.

Reporting of the classified results can ultimately provide useful information. Reporting of the results sometimes is based on an empirically or statistically derived threshold. In some embodiments, this threshold is used to produce to a result that is determined to be positive or negative for fetal aneuploidy. In some embodiments, a result is reported as a likelihood of fetal aneuploidy. In certain embodiments, this likelihood can be reported as a risk score. In some embodiments, the risk score is reported based solely on the results from the methods described herein. In some embodiments, the result is reported based upon the results from the method described herein and additional information about a patient. In certain embodiments, for noninvasive prenatal testing, this information can be, but is not limited to, maternal age and fetal fraction.

*Samples*

Provided herein are systems, methods and products for analyzing nucleic acids. In some embodiments, nucleic acid fragments in a mixture of nucleic acid fragments are analyzed. A mixture of nucleic acids can comprise two or more nucleic acid fragment species having different nucleotide sequences, different fragment lengths, different origins (e.g., genomic origins, fetal vs. maternal origins, cell or tissue origins, sample origins, subject origins, and the like), or combinations thereof.

Nucleic acid or a nucleic acid mixture utilized in systems, methods and products described herein often is isolated from a sample obtained from a subject. A subject can be any living or non-living organism, including but not limited to a human, a non-human animal, a plant, a bacterium, a fungus, a protest or a pathogen. Any human or non-human animal can be selected, and may include, for example, mammal, reptile, avian, amphibian, fish, ungulate, ruminant, bovine (e.g., cattle), equine (e.g., horse), caprine and ovine (e.g., sheep, goat), swine (e.g., pig), camelid (e.g., camel, llama, alpaca), monkey, ape (e.g., gorilla, chimpanzee), ursid (e.g., bear), poultry, dog, cat, mouse, rat, fish, dolphin, whale and shark. A subject may be a male or female (e.g., woman, a pregnant woman). A subject may be any age (e.g.,

an embryo, a fetus, an infant, a child, an adult).

Nucleic acid may be isolated from any type of suitable biological specimen or sample (e.g., a test sample). A sample or test sample can be any specimen that is isolated or obtained from a subject or part thereof (e.g., a human subject, a pregnant female, a fetus). A sample sometimes is from a pregnant female subject bearing a fetus at any stage of gestation (e.g., first, second or third trimester for a human subject), and sometimes is from a post-natal subject. A sample sometimes is from a pregnant subject bearing a fetus that is euploid for all chromosomes, and sometimes is from a pregnant subject bearing a fetus having a chromosome aneuploidy (e.g., one, three (i.e., trisomy (e.g., T21, T18, T13)), or four copies of a chromosome) or other genetic variation. Non-limiting examples of specimens include fluid or tissue from a subject, including, without limitation, blood or a blood product (e.g., serum, plasma, or the like), umbilical cord blood, chorionic villi, amniotic fluid, cerebrospinal fluid, spinal fluid, lavage fluid (e.g., bronchoalveolar, gastric, peritoneal, ductal, ear, arthroscopic), biopsy sample (e.g., from pre-implantation embryo; cancer biopsy), celocentesis sample, cells (blood cells, placental cells, embryo or fetal cells, fetal nucleated cells or fetal cellular remnants, normal cells, abnormal cells (e.g., cancer cells)) or parts thereof (e.g., mitochondrial, nucleus, extracts, or the like), washings of female reproductive tract, urine, feces, sputum, saliva, nasal mucous, prostate fluid, lavage, semen, lymphatic fluid, bile, tears, sweat, breast milk, breast fluid, the like or combinations thereof. In some embodiments, a biological sample is a cervical swab from a subject. A fluid or tissue sample from which nucleic acid is extracted may be acellular (e.g., cell-free). In some embodiments, a fluid or tissue sample may contain cellular elements or cellular remnants. In some embodiments, fetal cells may be included in the sample.

A sample can be a liquid sample. A liquid sample can comprise extracellular nucleic acid (e.g., circulating cell-free DNA). Non-limiting examples of liquid samples, include, blood or a blood product (e.g., serum, plasma, or the like), urine, a liquid sample described above, the like or combinations thereof. In certain embodiments, a sample is a liquid biopsy, which generally refers to an assessment of a liquid sample from a subject for the presence, absence, progression or remission of a disease. In certain instances, extracellular nucleic acid is analyzed in a liquid biopsy.

In some embodiments, a biological sample may be blood, plasma or serum. The term "blood" encompasses whole blood, blood product or any fraction of blood, such as serum, plasma, buffy coat, or the like as conventionally defined. Blood or fractions thereof often comprise nucleosomes. Nucleosomes comprise nucleic acids and are sometimes cell-free or intracellular. Blood also comprises buffy coats. Buffy coats are sometimes isolated by utilizing a ficoll gradient. Buffy coats can comprise white blood cells (e.g., leukocytes, T-cells, B-cells, platelets, and the like). Blood plasma refers to the fraction of whole blood resulting from centrifugation of blood treated with anticoagulants. Blood serum refers to the watery portion of fluid remaining after a blood sample has coagulated. Fluid or tissue samples often are collected in accordance with standard protocols hospitals or clinics generally follow. For blood, an appropriate amount of peripheral blood (e.g., between 3 to 40 milliliters, between 5 to 50 milliliters) often is collected and can be stored according to standard procedures prior to or after preparation.

An analysis of nucleic acid found in a subjects blood may be performed using, e.g., whole blood, serum, or plasma. An analysis of fetal DNA found in maternal blood, for example, may be performed using, e.g., whole blood, serum, or plasma. Methods for preparing serum or plasma from blood obtained from a subject (e.g., a maternal subject) are known. For example, a subject’s blood (e.g., a pregnant woman’s blood) can be placed in a tube containing EDTA or a specialized commercial product such as Vacutainer SST (Becton Dickinson, Franklin Lakes, N.J.) to prevent blood clotting, and plasma can then be obtained from whole blood through centrifugation. Serum may be obtained with or without centrifugation-following blood clotting. If centrifugation is used then it is typically, though not exclusively, conducted at an appropriate speed, e.g., 1,500-3,000 times g. Plasma or serum may be subjected to additional centrifugation steps before being transferred to a fresh tube for nucleic acid extraction. In addition to the acellular portion of the whole blood, nucleic acid may also be recovered from the cellular fraction, enriched in the buffy coat portion, which can be obtained following centrifugation of a whole blood sample from the subject and removal of the plasma.

A sample may be heterogeneous. For example, a sample may include more than one cell type and/or one or more nucleic acid species. In some instances, a sample may include fetal cells and maternal cells. In some instances, a sample may include (i) fetal derived and maternal derived nucleic acid, and/or more generally (ii) mutated and wild-type nucleic acid. In some instances, a sample may include a minority nucleic acid species and a majority nucleic acid species, as described in further detail below. In some instances, a sample may include cells and/or nucleic acid from a single subject or may include cells and/or nucleic acid from multiple subjects.

For prenatal applications of technology described herein, fluid or tissue sample may be collected from a female at a gestational age suitable for testing, or from a female who is being tested for possible pregnancy. Suitable gestational age may vary depending on the prenatal test being performed. In certain embodiments, a pregnant female subject sometimes is in the first trimester of pregnancy, at times in the second trimester of pregnancy, or sometimes in the third trimester of pregnancy. In certain embodiments, a fluid or tissue is collected from a pregnant female between about 1 to about 45 weeks of fetal gestation (e.g., at 1-4, 4-8, 8-12, 12-16, 16-20, 20-24, 24-28, 28-32, 32-36, 36-40 or 40-44 weeks of fetal gestation), and sometimes between about 5 to about 28 weeks of fetal gestation (e.g., at 6, 7, 8, 9,10, 11, 12, 13, 14, 15, 16, 17, 18, 19, 20, 21, 22, 23, 24, 25, 26 or 27 weeks of fetal gestation). In certain embodiments a fluid or tissue sample is collected from a pregnant female during or just after (e.g., 0 to 72 hours after) giving birth (e.g., vaginal or non-vaginal birth (e.g., surgical delivery)).

*Cell types*

As used herein, a "cell type" refers to a type of cell that can be distinguished from another type of cell. Extracellular nucleic acid can include nucleic acid from several different cell types. Non-limiting examples of cell types that can contribute nucleic acid to circulating cell-free nucleic acid include liver cells (e.g., hepatocytes), lung cells, spleen cells, pancreas cells, colon cells, skin cells, bladder cells, eye cells, brain cells, esophagus cells, cells of the head, cells of the neck, cells of the ovary, cells of the testes, prostate cells, placenta cells, epithelial cells, endothelial cells, adipocyte cells, kidney/renal cells, heart cells, muscle cells, blood cells (e.g., white blood cells), central nervous system (CNS) cells, the like and combinations of the foregoing. In some embodiments, cell types that contribute nucleic acid to circulating cell-free nucleic acid analyzed include white blood cells, endothelial cells and hepatocyte liver cells. Different cell types can be screened as part of identifying and selecting nucleic acid loci for which a marker state is the same or substantially the same for a cell type in subjects having a medical condition and for the cell type in subjects not having the medical condition, as described in further detail herein.

A particular cell type sometimes remains the same or substantially the same in subjects having a medical condition and in subjects not having a medical condition. In a non-limiting example, the number of living or viable cells of a particular cell type may be reduced in a cell degenerative condition, and the living, viable cells are not modified, or are not modified significantly, in subjects having the medical condition.

A particular cell type sometimes is modified as part of a medical condition and has one or more different properties than in its original state. In a non-limiting example, a particular cell type may proliferate at a higher than normal rate, may transform into a cell having a different morphology, may transform into a cell that expresses one or more different cell surface markers and/or may become part of a tumor, as part of a cancer condition. In embodiments for which a particular cell type (i.e., a progenitor cell) is modified as part of a medical condition, the marker state for each of the one or more markers assayed often is the same or substantially the same for the particular cell type in subjects having the medical condition and for the particular cell type in subjects not having the medical condition. Thus, the term "cell type" sometimes pertains to a type of cell in subjects not having a medical condition, and to a modified version of the cell in subjects having the medical condition. In some embodiments, a "cell type" is a progenitor cell only and not a modified version arising from the progenitor cell. A "cell type" sometimes pertains to a progenitor cell and a modified cell arising from the progenitor cell. In such embodiments, a marker state for a marker analyzed often is the same or substantially the same for a cell type in subjects having a medical condition and for the cell type in subjects not having the medical condition.

Different cell types can be distinguished by any suitable characteristic, including without limitation, one or more different cell surface markers, one or more different morphological features, one or more different functions, one or more different protein (e.g., histone) modifications and one or more different nucleic acid markers. Non-limiting examples of nucleic acid markers include single-nucleotide polymorphisms (SNPs), methylation state of a nucleic acid locus, short tandem repeats, insertions (e.g., microinsertions), deletions (microdeletions) the like and combinations thereof. Non-limiting examples of protein (e.g., histone) modifications include acetylation, methylation, ubiquitylation, phosphorylation, sumoylation, the like and combinations thereof.

As used herein, the term a "related cell type" refers to a cell type having multiple characteristics in common with another cell type. In related cell types, 75% or more cell surface markers sometimes are common to the cell types (e.g., about 80%, 85%, 90% or 95% or more of cell surface markers are common to the related cell types).

*Nucleic acid*

Provided herein are methods for analyzing nucleic acid. The terms "nucleic acid" and "nucleic acid molecule" may be used interchangeably throughout the disclosure. The terms refer to nucleic acids of any composition from, such as DNA (e.g., complementary DNA (cDNA), genomic DNA (gDNA) and the like), RNA (e.g., message RNA (mRNA), short inhibitory RNA (siRNA), ribosomal RNA (rRNA), tRNA, microRNA, RNA highly expressed by a fetus or placenta, and the like), and/or DNA or RNA analogs (e.g., containing base analogs, sugar analogs and/or a non-native backbone and the like), RNA/DNA hybrids and polyamide nucleic acids (PNAs), all of which can be in single- or double-stranded form, and unless otherwise limited, can encompass known analogs of natural nucleotides that can function in a similar manner as naturally occurring nucleotides. A nucleic acid may be, or may be from, a plasmid, phage, virus, bacterium, autonomously replicating sequence (ARS), mitochondria, centromere, artificial chromosome, chromosome, or other nucleic acid able to replicate or be replicated in vitro or in a host cell, a cell, a cell nucleus or cytoplasm of a cell in certain embodiments. A template nucleic acid in some embodiments can be from a single chromosome (e.g., a nucleic acid sample may be from one chromosome of a sample obtained from a diploid organism). Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, single nucleotide polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues. The term nucleic acid is used interchangeably with locus, gene, cDNA, and mRNA encoded by a gene. The term also may include, as equivalents, derivatives, variants and analogs of RNA or DNA synthesized from nucleotide analogs, single-stranded ("sense" or "antisense," "plus" strand or "minus" strand, "forward" reading frame or "reverse" reading frame) and double-stranded polynucleotides. The term "gene" refers to a section of DNA involved in producing a polypeptide chain; and generally includes regions preceding and following the coding region (leader and trailer) involved in the transcription/translation of the gene product and the regulation of the transcription/translation, as well as intervening sequences (introns) between individual coding regions (exons). A nucleotide or base generally refers to the purine and pyrimidine molecular units of nucleic acid (e.g., adenine (A), thymine (T), guanine (G), and cytosine (C)). For RNA, the base thymine is replaced with uracil. Nucleic acid length or size may be expressed as a number of bases.

Nucleic acid may be single or double stranded. Single stranded DNA, for example, can be generated by denaturing double stranded DNA by heating or by treatment with alkali, for example. In certain embodiments, nucleic acid is in a D-loop structure, formed by strand invasion of a duplex DNA molecule by an oligonucleotide or a DNA-like molecule such as peptide nucleic acid (PNA). D loop formation can be facilitated by addition of E. Coli RecA protein and/or by alteration of salt concentration, for example, using methods known in the art.

Nucleic acid provided for processes described herein may contain nucleic acid from one sample or from two or more samples (e.g., from 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more samples).

Nucleic acid may be derived from one or more sources (e.g., biological sample, blood, cells, serum, plasma, buffy coat, urine, lymphatic fluid, skin, soil, and the like) by methods known in the art. Any suitable method can be used for isolating, extracting and/or purifying DNA from a biological sample (e.g., from blood or a blood product), non-limiting examples of which include methods of DNA preparation (e.g., described by Sambrook and Russell, Molecular Cloning: A Laboratory Manual 3d ed., 2001), various commercially available reagents or kits, such as Qiagen's QIAamp Circulating Nucleic Acid Kit, QiaAmp DNA Mini Kit or QiaAmp DNA Blood Mini Kit (Qiagen, Hilden, Germany), GenomicPrep™ Blood DNA Isolation Kit (Promega, Madison, Wis.), and GFX™ Genomic Blood DNA Purification Kit (Amersham, Piscataway, N.J.), the like or combinations thereof.

In some embodiments, nucleic acid is extracted from cells using a cell lysis procedure. Cell lysis procedures and reagents are known in the art and may generally be performed by chemical (e.g., detergent, hypotonic solutions, enzymatic procedures, and the like, or combination thereof), physical (e.g., French press, sonication, and the like), or electrolytic lysis methods. Any suitable lysis procedure can be utilized. For example, chemical methods generally employ lysing agents to disrupt cells and extract the nucleic acids from the cells, followed by treatment with chaotropic salts. Physical methods such as freeze/thaw followed by grinding, the use of cell presses and the like also are useful. In some instances, a high salt and/or an alkaline lysis procedure may be utilized.

Nucleic acids can include extracellular nucleic acid in certain embodiments. The term "extracellular nucleic acid" as used herein can refer to nucleic acid isolated from a source having substantially no cells and also is referred to as "cell-free" nucleic acid, "circulating cell-free nucleic acid" (e.g., CCF fragments, ccf DNA) and/or "cell-free circulating nucleic acid." Extracellular nucleic acid can be present in and obtained from blood (e.g., from the blood of a human subject). Extracellular nucleic acid often includes no detectable cells and may contain cellular elements or cellular remnants. Non-limiting examples of acellular sources for extracellular nucleic acid are blood, blood plasma, blood serum and urine. As used herein, the term "obtain cell-free circulating sample nucleic acid" includes obtaining a sample directly (e.g., collecting a sample, e.g., a test sample) or obtaining a sample from another who has collected a sample. Without being limited by theory, extracellular nucleic acid may be a product of cell apoptosis and cell breakdown, which provides basis for extracellular nucleic acid often having a series of lengths across a spectrum (e.g., a "ladder").

Extracellular nucleic acid can include different nucleic acid species, and therefore is referred to herein as "heterogeneous" in certain embodiments. For example, blood serum or plasma from a pregnant female can include maternal nucleic acid and fetal nucleic acid. In some instances, fetal nucleic acid sometimes is about 5% to about 50% of the overall nucleic acid (e.g., about 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49% of the total nucleic acid is cancer or fetal nucleic acid).

At least two different nucleic acid species can exist in different amounts in extracellular nucleic acid and sometimes are referred to as minority species and majority species. For example, in some cases (e.g., fetal DNA or cancer DNA) the DNA of interest is a minority species. In certain embodiments, a genetic variation (e.g., copy number alteration, single nucleotide variation, chromosome alteration, translocation) is determined for a minority nucleic acid species. In certain embodiments, a genetic variation is determined for a majority nucleic acid species. Generally it is not intended that the terms "minority" or "majority" be rigidly defined in any respect. In one aspect, a nucleic acid that is considered "minority," for example, can have an abundance of at least about 0.1% of the total nucleic acid in a sample to less than 50% of the total nucleic acid in a sample. In some embodiments, a minority nucleic acid can have an abundance of at least about 1 % of the total nucleic acid in a sample to about 40% of the total nucleic acid in a sample. In some embodiments, a minority nucleic acid can have an abundance of at least about 2% of the total nucleic acid in a sample to about 30% of the total nucleic acid in a sample. In some embodiments, a minority nucleic acid can have an abundance of at least about 3% of the total nucleic acid in a sample to about 25% of the total nucleic acid in a sample. For example, a minority nucleic acid can have an abundance of about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29% or 30% of the total nucleic acid in a sample. In some instances, a minority species of extracellular nucleic acid sometimes is about 1% to about 40% of the overall nucleic acid (e.g., about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% or 40% of the nucleic acid is minority species nucleic acid). In some embodiments, the minority nucleic acid is extracellular DNA. In some embodiments, the minority nucleic acid is extracellular DNA from apoptotic tissue. In some embodiments, the minority nucleic acid is extracellular fetal DNA.

In another aspect, a nucleic acid that is considered "majority," for example, can have an abundance greater than 50% of the total nucleic acid in a sample to about 99.9% of the total nucleic acid in a sample. In some embodiments, a majority nucleic acid can have an abundance of at least about 60% of the total nucleic acid in a sample to about 99% of the total nucleic acid in a sample. In some embodiments, a majority nucleic acid can have an abundance of at least about 70% of the total nucleic acid in a sample to about 98% of the total nucleic acid in a sample. In some embodiments, a majority nucleic acid can have an abundance of at least about 75% of the total nucleic acid in a sample to about 97% of the total nucleic acid in a sample. For example, a majority nucleic acid can have an abundance of at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% of the total nucleic acid in a sample. In some embodiments, the majority nucleic acid is extracellular DNA. In some embodiments, the majority nucleic acid is extracellular maternal DNA.

In some embodiments, a minority species of extracellular nucleic acid is of a length of about 500 base pairs or less (e.g., about 80, 85, 90, 91, 92, 93, 94, 95, 96, 97, 98, 99 or 100% of minority species nucleic acid is of a length of about 500 base pairs or less). In some embodiments, a minority species of extracellular nucleic acid is of a length of about 300 base pairs or less (e.g., about 80, 85, 90, 91, 92, 93, 94, 95, 96, 97, 98, 99 or 100% of minority species nucleic acid is of a length of about 300 base pairs or less). In some embodiments, a minority species of extracellular nucleic acid is of a length of about 250 base pairs or less (e.g., about 80, 85, 90, 91, 92, 93, 94, 95, 96, 97, 98, 99 or 100% of minority species nucleic acid is of a length of about 250 base pairs or less). In some embodiments, a minority species of extracellular nucleic acid is of a length of about 200 base pairs or less (e.g., about 80, 85, 90, 91, 92, 93, 94, 95, 96, 97, 98, 99 or 100% of minority species nucleic acid is of a length of about 200 base pairs or less). In some embodiments, a minority species of extracellular nucleic acid is of a length of about 150 base pairs or less (e.g., about 80, 85, 90, 91, 92, 93, 94, 95, 96, 97, 98, 99 or 100% of minority species nucleic acid is of a length of about 150 base pairs or less). In some embodiments, a minority species of extracellular nucleic acid is of a length of about 100 base pairs or less (e.g., about 80, 85, 90, 91, 92, 93, 94, 95, 96, 97, 98, 99 or 100% of minority species nucleic acid is of a length of about 100 base pairs or less). In some embodiments, a minority species of extracellular nucleic acid is of a length of about 50 base pairs or less (e.g., about 80, 85, 90, 91, 92, 93, 94, 95, 96, 97, 98, 99 or 100% of minority species nucleic acid is of a length of about 50 base pairs or less).

Nucleic acid may be provided for conducting methods described herein with or without processing of the sample(s) containing the nucleic acid. In some embodiments, nucleic acid is provided for conducting methods described herein after processing of the sample(s) containing the nucleic acid. For example, a nucleic acid can be extracted, isolated, purified, partially purified or amplified from the sample(s). The term "isolated" as used herein refers to nucleic acid removed from its original environment (e.g., the natural environment if it is naturally occurring, or a host cell if expressed exogenously), and thus is altered by human intervention (e.g., "by the hand of man") from its original environment. The term "isolated nucleic acid" as used herein can refer to a nucleic acid removed from a subject (e.g., a human subject). An isolated nucleic acid can be provided with fewer non-nucleic acid components (e.g., protein, lipid) than the amount of components present in a source sample. A composition comprising isolated nucleic acid can be about 50% to greater than 99% free of non-nucleic acid components. A composition comprising isolated nucleic acid can be about 90%, 91%, 92%, 93%, 94%, 95%, 96%, 97%, 98%, 99% or greater than 99% free of non-nucleic acid components. The term "purified" as used herein can refer to a nucleic acid provided that contains fewer non-nucleic acid components (e.g., protein, lipid, carbohydrate) than the amount of non-nucleic acid components present prior to subjecting the nucleic acid to a purification procedure. A composition comprising purified nucleic acid may be about 80%, 81%, 82%, 83%, 84%, 85%, 86%, 87%, 88%, 89%, 90%, 91%, 92%, 93%, 94%, 95%, 96%, 97%, 98%, 99% or greater than 99% free of other non-nucleic acid components. The term "purified" as used herein can refer to a nucleic acid provided that contains fewer nucleic acid species than in the sample source from which the nucleic acid is derived. A composition comprising purified nucleic acid may be about 90%, 91%, 92%, 93%, 94%, 95%, 96%, 97%, 98%, 99% or greater than 99% free of other nucleic acid species. For example, fetal nucleic acid can be purified from a mixture comprising maternal and fetal nucleic acid. In certain examples, small fragments of fetal nucleic acid (e.g., 30 to 500 bp fragments) can be purified, or partially purified, from a mixture comprising both fetal and maternal nucleic acid fragments. In certain examples, nucleosomes comprising smaller fragments of fetal nucleic acid can be purified from a mixture of larger nucleosome complexes comprising larger fragments of maternal nucleic acid. In some embodiments, nucleic acid is provided for conducting methods described herein without prior processing of the sample(s) containing the nucleic acid. For example, nucleic acid may be analyzed directly from a sample without prior extraction, purification, partial purification, and/or amplification.

In some embodiments nucleic acids are sheared or cleaved prior to, during or after a method described herein. The term "shearing" or "cleavage" generally refers to a procedure or conditions in which a nucleic acid molecule, such as a nucleic acid template gene molecule or amplified product thereof, may be severed into two (or more) smaller nucleic acid molecules. Such shearing or cleavage can be sequence specific, base specific, or nonspecific, and can be accomplished by any of a variety of methods, reagents or conditions, including, for example, chemical, enzymatic, physical shearing (e.g., physical fragmentation). Sheared or cleaved nucleic acids may have a nominal, average or mean length of about 5 to about 10,000 base pairs, about 100 to about 1,000 base pairs, about 100 to about 500 base pairs, or about 10, 15, 20, 25, 30, 35, 40, 45, 50, 55, 60, 65, 70, 75, 80, 85, 90, 95, 100, 200, 300, 400, 500, 600, 700, 800, 900, 1000, 2000, 3000, 4000, 5000, 6000, 7000, 8000 or 9000 base pairs.

Sheared or cleaved nucleic acids can be generated by a suitable method, non-limiting examples of which include physical methods (e.g., shearing, e.g., sonication, French press, heat, UV irradiation, the like), enzymatic processes (e.g., enzymatic cleavage agents (e.g., a suitable nuclease, a suitable restriction enzyme, a suitable methylation sensitive restriction enzyme)), chemical methods (e.g., alkylation, DMS, piperidine, acid hydrolysis, base hydrolysis, heat, the like, or combinations thereof), processes described in U.S. Patent Application Publication No. 2005/0112590, the like or combinations thereof. The average, mean or nominal length of the resulting nucleic acid fragments can be controlled by selecting an appropriate fragment-generating method.

The term "amplified" as used herein refers to subjecting a target nucleic acid in a sample to a process that linearly or exponentially generates amplicon nucleic acids having the same or substantially the same nucleotide sequence as the target nucleic acid, or part thereof. In certain embodiments the term "amplified" refers to a method that comprises a polymerase chain reaction (PCR). In certain instances, an amplified product can contain one or more nucleotides more than the amplified nucleotide region of a nucleic acid template sequence (e.g., a primer can contain "extra" nucleotides such as a transcriptional initiation sequence, in addition to nucleotides complementary to a nucleic acid template gene molecule, resulting in an amplified product containing "extra" nucleotides or nucleotides not corresponding to the amplified nucleotide region of the nucleic acid template gene molecule).

Nucleic acid also may be exposed to a process that modifies certain nucleotides in the nucleic acid before providing nucleic acid for a method described herein. A process that selectively modifies nucleic acid based upon the methylation state of nucleotides therein can be applied to nucleic acid, for example. In addition, conditions such as high temperature, ultraviolet radiation, x-radiation, can induce changes in the sequence of a nucleic acid molecule. Nucleic acid may be provided in any suitable form useful for conducting a sequence analysis.

*Enriching nucleic acids*

In some embodiments, nucleic acid (e.g., extracellular nucleic acid) is enriched or relatively enriched for a subpopulation or species of nucleic acid. Nucleic acid subpopulations can include, for example, fetal nucleic acid, maternal nucleic acid, nucleic acid comprising fragments of a particular length or range of lengths, or nucleic acid from a particular genome region (e.g., single chromosome, set of chromosomes, and/or certain chromosome regions). Such enriched samples can be used in conjunction with a method provided herein. Thus, in certain embodiments, methods of the technology comprise an additional step of enriching for a subpopulation of nucleic acid in a sample, such as, for example, fetal nucleic acid. In certain embodiments, a method for determining fetal fraction also can be used to enrich for fetal nucleic acid. In certain embodiments, maternal nucleic acid is selectively removed (partially, substantially, almost completely or completely) from the sample. In certain embodiments, enriching for a particular low copy number species nucleic acid (e.g., fetal nucleic acid) may improve quantitative sensitivity. Methods for enriching a sample for a particular species of nucleic acid are described, for example, in U.S. Patent No. 6,927,028, International Patent Application Publication No. WO2007/140417, International Patent Application Publication No. WO2007/147063, International Patent Application Publication No. WO2009/032779, International Patent Application Publication No. WO2009/032781, International Patent Application Publication No. WO2010/033639, International Patent Application Publication No.

WO2011/034631, International Patent Application Publication No. WO2006/056480, and International Patent Application Publication No. WO2011/143659, the entire content of each is incorporated herein by reference, including all text, tables, equations and drawings.

In some embodiments, nucleic acid is enriched for certain target fragment species and/or reference fragment species. In certain embodiments, nucleic acid is enriched for a specific nucleic acid fragment length or range of fragment lengths using one or more length-based separation methods described below. In certain embodiments, nucleic acid is enriched for fragments from a select genomic region (e.g., chromosome) using one or more sequence-based separation methods described herein and/or known in the art. Non-limiting examples of methods for enriching for a nucleic acid subpopulation in a sample include methods that exploit epigenetic differences between nucleic acid species (e.g., methylation-based fetal nucleic acid enrichment methods described in U.S. Patent Application Publication No. 2010/0105049, which is incorporated by reference herein); restriction endonuclease enhanced polymorphic sequence approaches (e.g., such as a method described in U.S. Patent Application Publication No. 2009/0317818, which is incorporated by reference herein); selective enzymatic degradation approaches; massively parallel signature sequencing (MPSS) approaches; amplification (e.g., PCR)-based approaches (e.g., loci-specific amplification methods, multiplex SNP allele PCR approaches; universal amplification methods); pull-down approaches (e.g., biotinylated ultramer pull-down methods); extension and ligation-based methods (e.g., molecular inversion probe (MIP) extension and ligation); and combinations thereof.

In some embodiments, nucleic acid is enriched for fragments from a select genomic region (e.g., chromosome) using one or more sequence-based separation methods described herein. Sequence-based separation generally is based on nucleotide sequences present in the fragments of interest (e.g., target and/or reference fragments) and substantially not present in other fragments of the sample or present in an insubstantial amount of the other fragments (e.g., 5% or less). In some embodiments, sequence-based separation can generate separated target fragments and/or separated reference fragments. Separated target fragments and/or separated reference fragments often are isolated away from the remaining fragments in the nucleic acid sample. In certain embodiments, the separated target fragments and the separated reference fragments also are isolated away from each other (e.g., isolated in separate assay compartments). In certain embodiments, the separated target fragments and the separated reference fragments are isolated together (e.g., isolated in the same assay compartment). In some embodiments, unbound fragments can be differentially removed or degraded or digested.

In some embodiments, a selective nucleic acid capture process is used to separate target and/or reference fragments away from a nucleic acid sample. Commercially available nucleic acid capture systems include, for example, Nimblegen sequence capture system (Roche NimbleGen, Madison, WI); Illumina BEADARRAY platform (Illumina, San Diego, CA); Affymetrix GENECHIP platform (Affymetrix, Santa Clara, CA); Agilent SureSelect Target Enrichment System (Agilent Technologies, Santa Clara, CA); and related platforms. Such methods typically involve hybridization of a capture oligonucleotide to a part or all of the nucleotide sequence of a target or reference fragment and can include use of a solid phase (e.g., solid phase array) and/or a solution based platform. Capture oligonucleotides (sometimes referred to as "bait") can be selected or designed such that they preferentially hybridize to nucleic acid fragments from selected genomic regions or loci (e.g., one of chromosomes 21, 18, 13, X or Y, or a reference chromosome). In certain embodiments, a hybridization-based method (e.g., using oligonucleotide arrays) can be used to enrich for nucleic acid sequences from certain chromosomes (e.g., a potentially aneuploid chromosome, reference chromosome or other chromosome of interest) or regions of interest thereof.

In some embodiments, nucleic acid is enriched for a particular nucleic acid fragment length, range of lengths, or lengths under or over a particular threshold or cutoff using one or more length-based separation methods. Nucleic acid fragment length typically refers to the number of nucleotides in the fragment. Nucleic acid fragment length also is sometimes referred to as nucleic acid fragment size. In some embodiments, a length-based separation method is performed without measuring lengths of individual fragments. In some embodiments, a length based separation method is performed in conjunction with a method for determining length of individual fragments. In some embodiments, length-based separation refers to a size fractionation procedure where all or part of the fractionated pool can be isolated (e.g., retained) and/or analyzed. Size fractionation procedures are known in the art (e.g., separation on an array, separation by a molecular sieve, separation by gel electrophoresis, separation by column chromatography (e.g., size-exclusion columns), and microfluidics-based approaches). In certain instances, length-based separation approaches can include selective sequence tagging approaches, fragment circularization, chemical treatment (e.g., formaldehyde, polyethylene glycol (PEG) precipitation), mass spectrometry and/or size-specific nucleic acid amplification, for example.

*Nucleic acid quantification*

The amount of nucleic acid (e.g., concentration, relative amount, absolute amount, copy number, and the like) in a sample may be determined. The amount of a minority nucleic acid (e.g., concentration, relative amount, absolute amount, copy number, and the like) in nucleic acid is determined in some embodiments. In certain embodiments, the amount of a minority nucleic acid species in a sample is referred to as "minority species fraction." In some embodiments "minority species fraction" refers to the fraction of a minority nucleic acid species in circulating cell-free nucleic acid in a sample (e.g., a blood sample, a serum sample, a plasma sample, a urine sample) obtained from a subject.

The amount of a minority nucleic acid in extracellular nucleic acid can be quantified and used in conjunction with a method provided herein. Thus, in certain embodiments, methods described herein comprise an additional step of determining the amount of a minority nucleic acid. The amount of a minority nucleic acid can be determined in a sample from a subject before or after processing to prepare sample nucleic acid. In certain embodiments, the amount of a minority nucleic acid is determined in a sample after sample nucleic acid is processed and prepared, which amount is utilized for further assessment. In some embodiments, an outcome comprises factoring the minority species fraction in the sample nucleic acid (e.g., adjusting counts, removing samples, making a call or not making a call).

A determination of minority species fraction can be performed before, during, or at any one point in a method described herein, or after certain methods described herein (e.g., detection of a genetic variation). For example, to conduct a genetic variation determination method with a certain sensitivity or specificity, a minority nucleic acid quantification method may be implemented prior to, during or after genetic variation determination to identify those samples with greater than about 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25% or more minority nucleic acid. In some embodiments, samples determined as having a certain threshold amount of minority nucleic acid (e.g., about 15% or more minority nucleic acid; about 4% or more minority nucleic acid) are further analyzed for a genetic variation, or the presence or absence of a genetic variation, for example. In certain embodiments, determinations of, for example, a genetic variation are selected (e.g., selected and communicated to a patient) only for samples having a certain threshold amount of a minority nucleic acid (e.g., about 15% or more minority nucleic acid; about 4% or more minority nucleic acid).

The amount of fetal nucleic acid (e.g., concentration, relative amount, absolute amount, copy number, and the like) in nucleic acid is determined in some embodiments. In certain embodiments, the amount of fetal nucleic acid in a sample is referred to as "fetal fraction." In some embodiments "fetal fraction" refers to the fraction of fetal nucleic acid in circulating cell-free nucleic acid in a sample (e.g., a blood sample, a serum sample, a plasma sample, a urine sample) obtained from a pregnant female. Certain methods described herein or known in the art for determining fetal fraction can be used for determining a minority species fraction.

In certain instances, fetal fraction may be determined according to markers specific to a male fetus (e.g., Y-chromosome STR markers (e.g., DYS 19, DYS 385, DYS 392 markers); RhD marker in RhD-negative females), allelic ratios of polymorphic sequences, or according to one or more markers specific to fetal nucleic acid and not maternal nucleic acid (e.g., differential epigenetic biomarkers (e.g., methylation) between mother and fetus, or fetal RNA markers in maternal blood plasma (see e.g., Lo, 2005, Journal of Histochemistry and Cytochemistry 53 (3): 293-296)). Determination of fetal fraction sometimes is performed using a fetal quantifier assay (FQA) as described, for example, in U.S. Patent Application Publication No. 2010/0105049, which is hereby incorporated by reference. This type of assay allows for the detection and quantification of fetal nucleic acid in a maternal sample based on the methylation status of the nucleic acid in the sample.

In certain embodiments, a minority species fraction can be determined based on allelic ratios of polymorphic sequences (e.g., single nucleotide polymorphisms (SNPs)), such as, for example, using a method described in U.S. Patent Application Publication No. 2011/0224087, which is hereby incorporated by reference. In such a method for determining fetal fraction, for example, nucleotide sequence reads are obtained for a maternal sample and fetal fraction is determined by comparing the total number of nucleotide sequence reads that map to a first allele and the total number of nucleotide sequence reads that map to a second allele at an informative polymorphic site (e.g., SNP) in a reference genome.

A minority species fraction can be determined, in some embodiments, using methods that incorporate information derived from chromosomal aberrations as described, for example, in International Patent Application Publication No. WO2014/055774, which is incorporated by reference herein. A minority species fraction can be determined, in some embodiments, using methods that incorporate information derived from sex chromosomes as described, for example, in U.S. Patent Application Publication No. 2013/0288244 and U.S. Patent Application Publication No. 2013/0338933, each of which is incorporated by reference herein.

A minority species fraction can be determined in some embodiments using methods that incorporate fragment length information (e.g., fragment length ratio (FLR) analysis, fetal ratio statistic (FRS) analysis as described in International Patent Application Publication No. WO2013/177086, which is incorporated by reference herein). Cell-free fetal nucleic acid fragments generally are shorter than maternally-derived nucleic acid fragments (see e.g., Chan et al. (2004) Clin. Chem. 50:88-92; Lo et al. (2010) Sci. Transl. Med. 2:61ra91). Thus, fetal fraction can be determined, in some embodiments, by counting fragments under a particular length threshold and comparing the counts, for example, to counts from fragments over a particular length threshold and/or to the amount of total nucleic acid in the sample. Methods for counting nucleic acid fragments of a particular length are described in further detail in International Patent Application Publication No. WO2013/177086.

A minority species fraction can be determined, in some embodiments, according to portion-specific fraction estimates (e.g., as described in International Patent Application Publication No. WO 2014/205401, which is incorporated by reference herein). Without being limited to theory, the amount of reads from fetal CCF fragments (e.g., fragments of a particular length, or range of lengths) often map with ranging frequencies to portions (e.g., within the same sample, e.g., within the same sequencing run). Also, without being limited to theory, certain portions, when compared among multiple samples, tend to have a similar representation of reads from fetal CCF fragments (e.g., fragments of a particular length, or range of lengths), and that the representation correlates with portion-specific fetal fractions (e.g., the relative amount, percentage or ratio of CCF fragments originating from a fetus). Portion-specific fetal fraction estimates generally are determined according to portion-specific parameters and their relation to fetal fraction.

In some embodiments, the determination of minority species fraction (e.g, fetal fraction) is not required or necessary for identifying the presence or absence of a genetic variation. In some embodiments, identifying the presence or absence of a genetic variation does not require a sequence differentiation of a minority nucleic acid versus a majority nucleic acid. In certain embodiments, this is because the summed contribution of both minority and majority sequences in a particular chromosome, chromosome portion or part thereof is analyzed. In some embodiments, identifying the presence or absence of a genetic variation does not rely on a priori sequence information that would distinguish minority nucleic acid from majority nucleic acid.

*Nucleic acid library*

In some embodiments a nucleic acid library is a plurality of polynucleotide molecules (e.g., a sample of nucleic acids) that are prepared, assembled and/or modified for a specific process, non-limiting examples of which include immobilization on a solid phase (e.g., a solid support, a flow cell, a bead), enrichment, amplification, cloning, detection and/or for nucleic acid sequencing. In certain embodiments, a nucleic acid library is prepared prior to or during a sequencing process. A nucleic acid library (e.g., sequencing library) can be prepared by a suitable method as known in the art. A nucleic acid library can be prepared by a targeted or a non-targeted preparation process.

In some embodiments a library of nucleic acids is modified to comprise a chemical moiety (e.g., a functional group) configured for immobilization of nucleic acids to a solid support. In some embodiments a library of nucleic acids is modified to comprise a biomolecule (e.g., a functional group) and/or member of a binding pair configured for immobilization of the library to a solid support, non-limiting examples of which include thyroxin-binding globulin, steroid-binding proteins, antibodies, antigens, haptens, enzymes, lectins, nucleic acids, repressors, protein A, protein G, avidin, streptavidin, biotin, complement component C1q, nucleic acid-binding proteins, receptors, carbohydrates, oligonucleotides, polynucleotides, complementary nucleic acid sequences, the like and combinations thereof. Some examples of specific binding pairs include, without limitation: an avidin moiety and a biotin moiety; an antigenic epitope and an antibody or immunologically reactive fragment thereof; an antibody and a hapten; a digoxigen moiety and an anti-digoxigen antibody; a fluorescein moiety and an anti-fluorescein antibody; an operator and a repressor; a nuclease and a nucleotide; a lectin and a polysaccharide; a steroid and a steroid-binding protein; an active compound and an active compound receptor; a hormone and a hormone receptor; an enzyme and a substrate; an immunoglobulin and protein A; an oligonucleotide or polynucleotide and its corresponding complement; the like or combinations thereof.

In some embodiments, a library of nucleic acids is modified to comprise one or more polynucleotides of known composition, non-limiting examples of which include an identifier (e.g., a tag, an indexing tag), a capture sequence, a label, an adapter, a restriction enzyme site, a promoter, an enhancer, an origin of replication, a stem loop, a complimentary sequence (e.g., a primer binding site, an annealing site), a suitable integration site (e.g., a transposon, a viral integration site), a modified nucleotide, the like or combinations thereof. Polynucleotides of known sequence can be added at a suitable position, for example on the 5' end, 3' end or within a nucleic acid sequence. Polynucleotides of known sequence can be the same or different sequences. In some embodiments a polynucleotide of known sequence is configured to hybridize to one or more oligonucleotides immobilized on a surface (e.g., a surface in flow cell). For example, a nucleic acid molecule comprising a 5' known sequence may hybridize to a first plurality of oligonucleotides while the 3' known sequence may hybridize to a second plurality of oligonucleotides. In some embodiments a library of nucleic acid can comprise chromosome-specific tags, capture sequences, labels and/or adaptors. In some embodiments, a library of nucleic acids one or more detectable labels. In some embodiments one or more detectable labels may be incorporated into a nucleic acid library at a 5' end, at a 3' end, and/or at any nucleotide position within a nucleic acid in the library. In some embodiments a library of nucleic acids hybridized oligonucleotides. In certain embodiments hybridized oligonucleotides are labeled probes. In some embodiments a library of nucleic acids comprises hybridized oligonucleotide probes prior to immobilization on a solid phase.

In some embodiments, a polynucleotide of known sequence comprises a universal sequence. A universal sequence is a specific nucleotide sequence that is integrated into two or more nucleic acid molecules or two or more subsets of nucleic acid molecules where the universal sequence is the same for all molecules or subsets of molecules that it is integrated into. A universal sequence is often designed to hybridize to and/or amplify a plurality of different sequences using a single universal primer that is complementary to a universal sequence. In some embodiments two (e.g., a pair) or more universal sequences and/or universal primers are used. A universal primer often comprises a universal sequence. In some embodiments adapters (e.g., universal adapters) comprise universal sequences. In some embodiments one or more universal sequences are used to capture, identify and/or detect multiple species or subsets of nucleic acids.

In certain embodiments of preparing a nucleic acid library, (e.g., in certain sequencing by synthesis procedures), nucleic acids are size selected and/or fragmented into lengths of several hundred base pairs, or less (e.g., in preparation for library generation). In some embodiments, library preparation is performed without fragmentation (e.g., when using cell-free DNA).

In certain embodiments, a ligation-based library preparation method is used (e.g., ILLUMINA TRUSEQ, Illumina, San Diego CA). Ligation-based library preparation methods often make use of an adaptor (e.g., a methylated adaptor) design which can incorporate an index sequence at the initial ligation step and often can be used to prepare samples for single-read sequencing, paired-end sequencing and multiplexed sequencing. For example, nucleic acids (e.g., fragmented nucleic acids or cell-free DNA) may be end repaired by a fill-in reaction, an exonuclease reaction or a combination thereof. In some embodiments the resulting blunt-end repaired nucleic acid can then be extended by a single nucleotide, which is complementary to a single nucleotide overhang on the 3' end of an adapter/primer. Any nucleotide can be used for the extension/overhang nucleotides. In some embodiments nucleic acid library preparation comprises ligating an adapter oligonucleotide. Adapter oligonucleotides are often complementary to flow-cell anchors, and sometimes are utilized to immobilize a nucleic acid library to a solid support, such as the inside surface of a flow cell, for example. In some embodiments, an adapter oligonucleotide comprises an identifier, one or more sequencing primer hybridization sites (e.g., sequences complementary to universal sequencing primers, single end sequencing primers, paired end sequencing primers, multiplexed sequencing primers, and the like), or combinations thereof (e.g., adapter/sequencing, adapter/identifier, adapter/identifier/sequencing).

An identifier can be a suitable detectable label incorporated into or attached to a nucleic acid (e.g., a polynucleotide) that allows detection and/or identification of nucleic acids that comprise the identifier. In some embodiments an identifier is incorporated into or attached to a nucleic acid during a sequencing method (e.g., by a polymerase). Non-limiting examples of identifiers include nucleic acid tags, nucleic acid indexes or barcodes, a radiolabel (e.g., an isotope), metallic label, a fluorescent label, a chemiluminescent label, a phosphorescent label, a fluorophore quencher, a dye, a protein (e.g., an enzyme, an antibody or part thereof, a linker, a member of a binding pair), the like or combinations thereof. In some embodiments an identifier (e.g., a nucleic acid index or barcode) is a unique, known and/or identifiable sequence of nucleotides or nucleotide analogues. In some embodiments identifiers are six or more contiguous nucleotides. A multitude of fluorophores are available with a variety of different excitation and emission spectra. Any suitable type and/or number of fluorophores can be used as an identifier. In some embodiments 1 or more, 2 or more, 3 or more, 4 or more, 5 or more, 6 or more, 7 or more, 8 or more, 9 or more, 10 or more, 20 or more, 30 or more or 50 or more different identifiers are utilized in a method described herein (e.g., a nucleic acid detection and/or sequencing method). In some embodiments, one or two types of identifiers (e.g., fluorescent labels) are linked to each nucleic acid in a library. Detection and/or quantification of an identifier can be performed by a suitable method, apparatus or machine, non-limiting examples of which include flow cytometry, quantitative polymerase chain reaction (qPCR), gel electrophoresis, a luminometer, a fluorometer, a spectrophotometer, a suitable gene-chip or microarray analysis, Western blot, mass spectrometry, chromatography, cytofluorimetric analysis, fluorescence microscopy, a suitable fluorescence or digital imaging method, confocal laser scanning microscopy, laser scanning cytometry, affinity chromatography, manual batch mode separation, electric field suspension, a suitable nucleic acid sequencing method and/or nucleic acid sequencing apparatus, the like and combinations thereof.

In some embodiments, a transposon-based library preparation method is used (e.g., EPICENTRE NEXTERA, Epicentre, Madison, WI). Transposon-based methods typically use in vitro transposition to simultaneously fragment and tag DNA in a single-tube reaction (often allowing incorporation of platform-specific tags and optional barcodes), and prepare sequencer-ready libraries.

In some embodiments a nucleic acid library or parts thereof are amplified (e.g., amplified by a PCR-based method). In some embodiments a sequencing method comprises amplification of a nucleic acid library. A nucleic acid library can be amplified prior to or after immobilization on a solid support (e.g., a solid support in a flow cell). Nucleic acid amplification includes the process of amplifying or increasing the numbers of a nucleic acid template and/or of a complement thereof that are present (e.g., in a nucleic acid library), by producing one or more copies of the template and/or its complement. Amplification can be carried out by a suitable method. A nucleic acid library can be amplified by a thermocycling method or by an isothermal amplification method. In some embodiments a rolling circle amplification method is used. In some embodiments amplification takes place on a solid support (e.g., within a flow cell) where a nucleic acid library or portion thereof is immobilized. In certain sequencing methods, a nucleic acid library is added to a flow cell and immobilized by hybridization to anchors under suitable conditions. This type of nucleic acid amplification is often referred to as solid phase amplification. In some embodiments of solid phase amplification, all or a portion of the amplified products are synthesized by an extension initiating from an immobilized primer. Solid phase amplification reactions are analogous to standard solution phase amplifications except that at least one of the amplification oligonucleotides (e.g., primers) is immobilized on a solid support.

In some embodiments solid phase amplification comprises a nucleic acid amplification reaction comprising only one species of oligonucleotide primer immobilized to a surface. In certain embodiments solid phase amplification comprises a plurality of different immobilized oligonucleotide primer species. In some embodiments solid phase amplification may comprise a nucleic acid amplification reaction comprising one species of oligonucleotide primer immobilized on a solid surface and a second different oligonucleotide primer species in solution. Multiple different species of immobilized or solution based primers can be used. Non-limiting examples of solid phase nucleic acid amplification reactions include interfacial amplification, bridge amplification, emulsion PCR, WildFire amplification (e.g., U.S. Patent Application Publication No. 2013/0012399), the like or combinations thereof.

*Nucleic acid sequencing and processing*

Methods provided herein generally include nucleic acid sequencing and analysis. In some embodiments, nucleic acid is sequenced and the sequencing product (e.g., a collection of sequence reads) is processed prior to, or in conjunction with, an analysis of the sequenced nucleic acid. For example, sequence reads may be processed according to one or more of the following: aligning, mapping, filtering portions, selecting portions, counting, normalizing, weighting, generating a profile, and the like, and combinations thereof. Certain processing steps may be performed in any order and certain processing steps may be repeated. For example, portions may be filtered followed by sequence read count normalization, and, in certain embodiments, sequence read counts may be normalized followed by portion filtering. In some embodiments, a portion filtering step is followed by sequence read count normalization followed by a further portion filtering step. Certain sequencing methods and processing steps are described in further detail below.

*Sequencing*

In some embodiments, nucleic acid (e.g., nucleic acid fragments, sample nucleic acid, cell-free nucleic acid) is sequenced. In certain instances, a full or substantially full sequence is obtained and sometimes a partial sequence is obtained. Nucleic acid sequencing generally produces a collection of sequence reads. As used herein, "reads" (e.g., "a read," "a sequence read") are short nucleotide sequences produced by any sequencing process described herein or known in the art. Reads can be generated from one end of nucleic acid fragments ("single-end reads"), and sometimes are generated from both ends of nucleic acid fragments (e.g., paired-end reads, double-end reads).

The length of a sequence read is often associated with the particular sequencing technology. High-throughput methods, for example, provide sequence reads that can vary in size from tens to hundreds of base pairs (bp). Nanopore sequencing, for example, can provide sequence reads that can vary in size from tens to hundreds to thousands of base pairs. In some embodiments, sequence reads are of a mean, median, average or absolute length of about 15 bp to about 900 bp long. In certain embodiments sequence reads are of a mean, median, average or absolute length of about 1000 bp or more.

In some embodiments the nominal, average, mean or absolute length of single-end reads sometimes is about 15 contiguous nucleotides to about 50 or more contiguous nucleotides, about 15 contiguous nucleotides to about 40 or more contiguous nucleotides, and sometimes about 15 contiguous nucleotides or about 36 or more contiguous nucleotides. In certain embodiments the nominal, average, mean or absolute length of single-end reads is about 20 to about 30 bases, or about 24 to about 28 bases in length. In certain embodiments the nominal, average, mean or absolute length of single-end reads is about 1, 2, 3, 4, 5, 6, 7, 8, 9, 10, 11, 12, 13, 14, 15, 16, 17, 18, 19, 21, 22, 23, 24, 25, 26, 27, 28 or about 29 bases or more in length. In certain embodiments, the nominal, average, mean or absolute length of paired-end reads sometimes is about 10 contiguous nucleotides to about 25 contiguous nucleotides or more (e.g., about 10, 11, 12, 13, 14, 15, 16, 17, 18, 19, 20, 21, 22, 23, 24 or 25 nucleotides in length or more), about 15 contiguous nucleotides to about 20 contiguous nucleotides or more, and sometimes is about 17 contiguous nucleotides or about 18 contiguous nucleotides.

In some embodiments, nucleotide sequence reads obtained from a sample are partial nucleotide sequence reads. As used herein, "partial nucleotide sequence reads" refers to sequence reads of any length with incomplete sequence information, also referred to as sequence ambiguity. Partial nucleotide sequence reads may lack information regarding nucleobase identity and/or nucleobase position or order. Partial nucleotide sequence reads generally do not include sequence reads in which the only incomplete sequence information (or in which less than all of the bases are sequenced or determined) is from inadvertent or unintentional sequencing errors. Such sequencing errors can be inherent to certain sequencing processes and include, for example, incorrect calls for nucleobase identity, and missing or extra nucleobases. Thus, for partial nucleotide sequence reads herein, certain information about the sequence is often deliberately excluded. That is, one deliberately obtains sequence information with respect to less than all of the nucleobases or which might otherwise be characterized as or be a sequencing error. In some embodiments, a partial nucleotide sequence read can span a portion of a nucleic acid fragment. In some embodiments, a partial nucleotide sequence read can span the entire length of a nucleic acid fragment. Partial nucleotide sequence reads are described, for example, in International Patent Application Publication No. WO2013/052907, the entire content of which is incorporated herein by reference, including all text, tables, equations and drawings.

Reads generally are representations of nucleotide sequences in a physical nucleic acid. For example, in a read containing an ATGC depiction of a sequence, "A" represents an adenine nucleotide, "T" represents a thymine nucleotide, "G" represents a guanine nucleotide and "C" represents a cytosine nucleotide, in a physical nucleic acid. Sequence reads obtained from a sample from a subject can be reads from a mixture of a minority nucleic acid and a majority nucleic acid. For example, sequence reads obtained from the blood of a pregnant female can be reads from a mixture of fetal nucleic acid and maternal nucleic acid. A mixture of relatively short reads can be transformed by processes described herein into a representation of genomic nucleic acid present in the subject, and/or a representation of genomic nucleic acid present in a fetus. In certain instances, a mixture of relatively short reads can be transformed into a representation of a copy number alteration, a genetic variation or an aneuploidy, for example. In one example, reads of a mixture of maternal and fetal nucleic acid can be transformed into a representation of a composite chromosome or a part thereof comprising features of one or both maternal and fetal chromosomes.

In some instances, circulating cell free nucleic acid fragments (CCF fragments) obtained from a pregnant female comprise nucleic acid fragments originating from fetal cells (i.e., fetal fragments) and nucleic acid fragments originating from maternal cells (i.e., maternal fragments). Sequence reads derived from CCF fragments originating from a fetus are referred to herein as "fetal reads." Sequence reads derived from CCF fragments originating from the genome of a pregnant female (e.g., a mother) bearing a fetus are referred to herein as "maternal reads." CCF fragments from which fetal reads are obtained are referred to herein as fetal templates and CCF fragments from which maternal reads are obtained are referred herein to as maternal templates.

In certain embodiments, "obtaining" nucleic acid sequence reads of a sample from a subject and/or "obtaining" nucleic acid sequence reads of a biological specimen from one or more reference persons can involve directly sequencing nucleic acid to obtain the sequence information. In some embodiments, "obtaining" can involve receiving sequence information obtained directly from a nucleic acid by another.

In some embodiments, some or all nucleic acids in a sample are enriched and/or amplified (e.g., non-specifically, e.g., by a PCR based method) prior to or during sequencing. In certain embodiments specific nucleic acid species or subsets in a sample are enriched and/or amplified prior to or during sequencing. In some embodiments, a species or subset of a preselected pool of nucleic acids is sequenced randomly. In some embodiments, nucleic acids in a sample are not enriched and/or amplified prior to or during sequencing.

In some embodiments, a representative fraction of a genome is sequenced and is sometimes referred to as "coverage" or "fold coverage." For example, a 1-fold coverage indicates that roughly 100% of the nucleotide sequences of the genome are represented by reads. In some instances, fold coverage is referred to as (and is directly proportional to) "sequencing depth." In some embodiments, "fold coverage" is a relative term referring to a prior sequencing run as a reference. For example, a second sequencing run may have 2-fold less coverage than a first sequencing run. In some embodiments a genome is sequenced with redundancy, where a given region of the genome can be covered by two or more reads or overlapping reads (e.g., a "fold coverage" greater than 1, e.g., a 2-fold coverage). In some embodiments, a genome (e.g., a whole genome) is sequenced with about 0.1-fold to about 100-fold coverage, about 0.2-fold to 20-fold coverage, or about 0.2-fold to about 1-fold coverage (e.g., about 0.2-, 0.3-, 0.4-, 0.5-, 0.6-, 0.7-, 0.8-, 0.9-, 1-, 2-, 3-, 4-, 5-, 6-, 7-, 8-, 9-, 10-, 15-, 20-, 30-, 40-, 50-, 60-, 70-, 80-, 90-fold coverage). In some embodiments, specific parts of a genome (e.g., genomic parts from targeted and/or probe-based methods) are sequenced and fold coverage values generally refer to the fraction of the specific genomic parts sequenced (i.e., fold coverage values do not refer to the whole genome). In some instances, specific genomic parts are sequenced at 1000-fold coverage or more. For example, specific genomic parts may be sequenced at 2000-fold, 5,000-fold, 10,000-fold, 20,000-fold, 30,000-fold, 40,000-fold or 50,000-fold coverage.

In some embodiments, one nucleic acid sample from one individual is sequenced. In certain embodiments, nucleic acids from each of two or more samples are sequenced, where samples are from one individual or from different individuals. In certain embodiments, nucleic acid samples from two or more biological samples are pooled, where each biological sample is from one individual or two or more individuals, and the pool is sequenced. In the latter embodiments, a nucleic acid sample from each biological sample often is identified by one or more unique identifiers.

In some embodiments, a sequencing method utilizes identifiers that allow multiplexing of sequence reactions in a sequencing process. The greater the number of unique identifiers, the greater the number of samples and/or chromosomes for detection, for example, that can be multiplexed in a sequencing process. A sequencing process can be performed using any suitable number of unique identifiers (e.g., 4, 8, 12, 24, 48, 96, or more).

A sequencing process sometimes makes use of a solid phase, and sometimes the solid phase comprises a flow cell on which nucleic acid from a library can be attached and reagents can be flowed and contacted with the attached nucleic acid. A flow cell sometimes includes flow cell lanes, and use of identifiers can facilitate analyzing a number of samples in each lane. A flow cell often is a solid support that can be configured to retain and/or allow the orderly passage of reagent solutions over bound analytes. Flow cells frequently are planar in shape, optically transparent, generally in the millimeter or sub-millimeter scale, and often have channels or lanes in which the analyte/reagent interaction occurs. In some embodiments the number of samples analyzed in a given flow cell lane is dependent on the number of unique identifiers utilized during library preparation and/or probe design. Multiplexing using 12 identifiers, for example, allows simultaneous analysis of 96 samples (e.g., equal to the number of wells in a 96 well microwell plate) in an 8 lane flow cell. Similarly, multiplexing using 48 identifiers, for example, allows simultaneous analysis of 384 samples (e.g., equal to the number of wells in a 384 well microwell plate) in an 8 lane flow cell. Non-limiting examples of commercially available multiplex sequencing kits include Illumina's multiplexing sample preparation oligonucleotide kit and multiplexing sequencing primers and PhiX control kit (e.g., Illumina's catalog numbers PE-400-1001 and PE-400-1002, respectively).

Any suitable method of sequencing nucleic acids can be used, non-limiting examples of which include Maxim & Gilbert, chain-termination methods, sequencing by synthesis, sequencing by ligation, sequencing by mass spectrometry, microscopy-based techniques, the like or combinations thereof. In some embodiments, a first generation technology, such as, for example, Sanger sequencing methods including automated Sanger sequencing methods, including microfluidic Sanger sequencing, can be used in a method provided herein. In some embodiments, sequencing technologies that include the use of nucleic acid imaging technologies (e.g., transmission electron microscopy (TEM) and atomic force microscopy (AFM)), can be used. In some embodiments, a high-throughput sequencing method is used. High-throughput sequencing methods generally involve clonally amplified DNA templates or single DNA molecules that are sequenced in a massively parallel fashion, sometimes within a flow cell. Next generation (e.g., 2nd and 3rd generation) sequencing techniques capable of sequencing DNA in a massively parallel fashion can be used for methods described herein and are collectively referred to herein as "massively parallel sequencing" (MPS). In some embodiments, MPS sequencing methods utilize a targeted approach, where specific chromosomes, genes or regions of interest are sequenced. In certain embodiments, a non-targeted approach is used where most or all nucleic acids in a sample are sequenced, amplified and/or captured randomly.

In some embodiments a targeted enrichment, amplification and/or sequencing approach is used. A targeted approach often isolates, selects and/or enriches a subset of nucleic acids in a sample for further processing by use of sequence-specific oligonucleotides. In some embodiments a library of sequence-specific oligonucleotides are utilized to target (e.g., hybridize to) one or more sets of nucleic acids in a sample. Sequence-specific oligonucleotides and/or primers are often selective for particular sequences (e.g., unique nucleic acid sequences) present in one or more chromosomes, genes, exons, introns, and/or regulatory regions of interest. Any suitable method or combination of methods can be used for enrichment, amplification and/or sequencing of one or more subsets of targeted nucleic acids. In some embodiments targeted sequences are isolated and/or enriched by capture to a solid phase (e.g., a flow cell, a bead) using one or more sequence-specific anchors. In some embodiments targeted sequences are enriched and/or amplified by a polymerase-based method (e.g., a PCR-based method, by any suitable polymerase based extension) using sequence-specific primers and/or primer sets. Sequence specific anchors often can be used as sequence-specific primers.

MPS sequencing sometimes makes use of sequencing by synthesis and certain imaging processes. A nucleic acid sequencing technology that may be used in a method described herein is sequencing-by-synthesis and reversible terminator-based sequencing (e.g., Illumina's Genome Analyzer; Genome Analyzer II; HISEQ 2000; HISEQ 2500 (Illumina, San Diego CA)). With this technology, millions of nucleic acid (e.g., DNA) fragments can be sequenced in parallel. In one example of this type of sequencing technology, a flow cell is used which contains an optically transparent slide with 8 individual lanes on the surfaces of which are bound oligonucleotide anchors (e.g., adaptor primers).

Sequencing by synthesis generally is performed by iteratively adding (e.g., by covalent addition) a nucleotide to a primer or preexisting nucleic acid strand in a template directed manner. Each iterative addition of a nucleotide is detected and the process is repeated multiple times until a sequence of a nucleic acid strand is obtained. The length of a sequence obtained depends, in part, on the number of addition and detection steps that are performed. In some embodiments of sequencing by synthesis, one, two, three or more nucleotides of the same type (e.g., A, G, C or T) are added and detected in a round of nucleotide addition. Nucleotides can be added by any suitable method (e.g., enzymatically or chemically). For example, in some embodiments a polymerase or a ligase adds a nucleotide to a primer or to a preexisting nucleic acid strand in a template directed manner. In some embodiments of sequencing by synthesis, different types of nucleotides, nucleotide analogues and/or identifiers are used. In some embodiments reversible terminators and/or removable (e.g., cleavable) identifiers are used. In some embodiments fluorescent labeled nucleotides and/or nucleotide analogues are used. In certain embodiments sequencing by synthesis comprises a cleavage (e.g., cleavage and removal of an identifier) and/or a washing step. In some embodiments the addition of one or more nucleotides is detected by a suitable method described herein or known in the art, non-limiting examples of which include any suitable imaging apparatus, a suitable camera, a digital camera, a CCD (Charge Couple Device) based imaging apparatus (e.g., a CCD camera), a CMOS (Complementary Metal Oxide Silicon) based imaging apparatus (e.g., a CMOS camera), a photo diode (e.g., a photomultiplier tube), electron microscopy, a field-effect transistor (e.g., a DNA field-effect transistor), an ISFET ion sensor (e.g., a CHEMFET sensor), the like or combinations thereof.

Any suitable MPS method, system or technology platform for conducting methods described herein can be used to obtain nucleic acid sequence reads. Non-limiting examples of MPS platforms include Illumina/Solex/HiSeq (e.g., Illumina's Genome Analyzer; Genome Analyzer II; HISEQ 2000; HISEQ), SOLiD, Roche/454, PACBIO and/or SMRT, Helicos True Single Molecule Sequencing, Ion Torrent and Ion semiconductor-based sequencing (e.g., as developed by Life Technologies), WildFire, 5500, 5500xl W and/or 5500xl W Genetic Analyzer based technologies (e.g., as developed and sold by Life Technologies, U.S. Patent Application Publication No. 2013/0012399); Polony sequencing, Pyrosequencing, Massively Parallel Signature Sequencing (MPSS), RNA polymerase (RNAP) sequencing, LaserGen systems and methods, Nanopore-based platforms, chemical-sensitive field effect transistor (CHEMFET) array, electron microscopy-based sequencing (e.g., as developed by ZS Genetics, Halcyon Molecular), nanoball sequencing, the like or combinations thereof. Other sequencing methods that may be used to conduct methods herein include digital PCR, sequencing by hybridization, nanopore sequencing, chromosome-specific sequencing (e.g., using DANSR (digital analysis of selected regions) technology.

In some embodiments, sequence reads are generated, obtained, gathered, assembled, manipulated, transformed, processed, and/or provided by a sequence module. A machine comprising a sequence module can be a suitable machine and/or apparatus that determines the sequence of a nucleic acid utilizing a sequencing technology known in the art. In some embodiments a sequence module can align, assemble, fragment, complement, reverse complement, and/or error check (e.g., error correct sequence reads).

*Mapping reads*

Sequence reads can be mapped and the number of reads mapping to a specified nucleic acid region (e.g., a chromosome or portion thereof) are referred to as counts. Any suitable mapping method (e.g., process, algorithm, program, software, module, the like or combination thereof) can be used. Certain aspects of mapping processes are described hereafter.

Mapping nucleotide sequence reads (i.e., sequence information from a fragment whose physical genomic position is unknown) can be performed in a number of ways, and often comprises alignment of the obtained sequence reads with a matching sequence in a reference genome. In such alignments, sequence reads generally are aligned to a reference sequence and those that align are designated as being "mapped," as "a mapped sequence read" or as "a mapped read." In certain embodiments, a mapped sequence read is referred to as a "hit" or "count." In some embodiments, mapped sequence reads are grouped together according to various parameters and assigned to particular genomic portions, which are discussed in further detail below.

The terms "aligned," "alignment," or "aligning" generally refer to two or more nucleic acid sequences that can be identified as a match (e.g., 100% identity) or partial match. Alignments can be done manually or by a computer (e.g., a software, program, module, or algorithm), non-limiting examples of which include the Efficient Local Alignment of Nucleotide Data (ELAND) computer program distributed as part of the Illumina Genomics Analysis pipeline. Alignment of a sequence read can be a 100% sequence match. In some cases, an alignment is less than a 100% sequence match (i.e., non-perfect match, partial match, partial alignment). In some embodiments an alignment is about a 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76% or 75% match. In some embodiments, an alignment comprises a mismatch. In some embodiments, an alignment comprises 1, 2, 3, 4 or 5 mismatches. Two or more sequences can be aligned using either strand (e.g., sense or antisense strand). In certain embodiments a nucleic acid sequence is aligned with the reverse complement of another nucleic acid sequence.

Various computational methods can be used to map each sequence read to a portion. Non-limiting examples of computer algorithms that can be used to align sequences include, without limitation, BLAST, BLITZ, FASTA, BOWTIE 1, BOWTIE 2, ELAND, MAQ, PROBEMATCH, SOAP, BWA or SEQMAP, or variations thereof or combinations thereof. In some embodiments, sequence reads can be aligned with sequences in a reference genome. In some embodiments, sequence reads can be found and/or aligned with sequences in nucleic acid databases known in the art including, for example, GenBank, dbEST, dbSTS, EMBL (European Molecular Biology Laboratory) and DDBJ (DNA Databank of Japan). BLAST or similar tools can be used to search identified sequences against a sequence database. Search hits can then be used to sort the identified sequences into appropriate portions (described hereafter), for example.

In some embodiments, a read may uniquely or non-uniquely map to portions in a reference genome. A read is considered as "uniquely mapped" if it aligns with a single sequence in the reference genome. A read is considered as "non-uniquely mapped" if it aligns with two or more sequences in the reference genome. In some embodiments, non-uniquely mapped reads are eliminated from further analysis (e.g. quantification). A certain, small degree of mismatch (0-1) may be allowed to account for single nucleotide polymorphisms that may exist between the reference genome and the reads from individual samples being mapped, in certain embodiments. In some embodiments, no degree of mismatch is allowed for a read mapped to a reference sequence.

As used herein, the term "reference genome" can refer to any particular known, sequenced or characterized genome, whether partial or complete, of any organism or virus which may be used to reference identified sequences from a subject. For example, a reference genome used for human subjects as well as many other organisms can be found at the National Center for Biotechnology Information at World Wide Web URL ncbi.nlm.nih.gov. A "genome" refers to the complete genetic information of an organism or virus, expressed in nucleic acid sequences. As used herein, a reference sequence or reference genome often is an assembled or partially assembled genomic sequence from an individual or multiple individuals. In some embodiments, a reference genome is an assembled or partially assembled genomic sequence from one or more human individuals. In some embodiments, a reference genome comprises sequences assigned to chromosomes.

In certain embodiments, mappability is assessed for a genomic region (e.g., portion, genomic portion). Mappability is the ability to unambiguously align a nucleotide sequence read to a portion of a reference genome, typically up to a specified number of mismatches, including, for example, 0, 1, 2 or more mismatches. For a given genomic region, the expected mappability can be estimated using a sliding-window approach of a preset read length and averaging the resulting read-level mappability values. Genomic regions comprising stretches of unique nucleotide sequence sometimes have a high mappability value.

*Portions*

In some embodiments, mapped sequence reads are grouped together according to various parameters and assigned to particular genomic portions (e.g., portions of a reference genome). A "portion" also may be referred to herein as a "genomic section," "bin," "partition," "portion of a reference genome," "portion of a chromosome" or "genomic portion."

A portion often is defined by partitioning of a genome according to one or more features. Non-limiting examples of certain partitioning features include length (e.g., fixed length, non-fixed length) and other structural features. Genomic portions sometimes include one or more of the following features: fixed length, non-fixed length, random length, non-random length, equal length, unequal length (e.g., at least two of the genomic portions are of unequal length), do not overlap (e.g., the 3' ends of the genomic portions sometimes abut the 5' ends of adjacent genomic portions), overlap (e.g., at least two of the genomic portions overlap), contiguous, consecutive, not contiguous, and not consecutive. Genomic portions sometimes are about 1 to about 1,000 kilobases in length (e.g., about 2, 3, 4, 5, 6, 7, 8, 9, 10, 15, 20, 25, 30, 35, 40, 45, 50, 55, 60, 65, 70, 75, 80, 85, 90, 95, 100, 200, 300, 400, 500, 600, 700, 800, 900 kilobases in length), about 5 to about 500 kilobases in length, about 10 to about 100 kilobases in length, or about 40 to about 60 kilobases in length.

Partitioning sometimes is based on, or is based in part on certain informational features, such as, information content and information gain, for example. Non-limiting examples of certain informational features include speed and/or convenience of alignment, sequencing coverage variability, GC content (e.g., stratified GC content, particular GC contents, high or low GC content), uniformity of GC content, other measures of sequence content (e.g., fraction of individual nucleotides, fraction of pyrimidines or purines, fraction of natural vs. non-natural nucleic acids, fraction of methylated nucleotides, and CpG content), methylation state, duplex melting temperature, amenability to sequencing or PCR, uncertainty value assigned to individual portions of a reference genome, and/or a targeted search for particular features. In some embodiments, information content may be quantified using a p-value profile measuring the significance of particular genomic locations for distinguishing between groups of confirmed normal and abnormal subjects (e.g. euploid and trisomy subjects, respectively).

In some embodiments, partitioning a genome may eliminate similar regions (e.g., identical or homologous regions or sequences) across a genome and only keep unique regions. Regions removed during partitioning may be within a single chromosome, may be one or more chromosomes, or may span multiple chromosomes. In some embodiments, a partitioned genome is reduced and optimized for faster alignment, often focusing on uniquely identifiable sequences.

In some embodiments, genomic portions result from a partitioning based on non-overlapping fixed size, which results in consecutive, non-overlapping portions of fixed length. Such portions often are shorter than a chromosome and often are shorter than a copy number variation region (e.g., a region that is duplicated or is deleted), the latter of which can be referred to as a segment. A "segment" or "genomic segment" often includes two or more fixed-length genomic portions, and often includes two or more consecutive fixed-length portions (e.g., about 2 to about 100 such portions (e.g., 2, 3, 4, 5, 6, 7, 8, 9, 10, 11, 12, 13, 14, 15, 16, 17, 18, 19, 20, 25, 30, 35, 40, 45, 50, 60, 70, 80, 90 such portions)).

Multiple portions sometimes are analyzed in groups, and sometimes reads mapped to portions are quantified according to a particular group of genomic portions. Where portions are partitioned by structural features and correspond to regions in a genome, portions sometimes are grouped into one or more segments and/or one or more regions. Non-limiting examples of regions include sub-chromosome (i.e., shorter than a chromosome), chromosome, autosome, sex chromosome and combinations thereof. One or more sub-chromosome regions sometimes are genes, gene fragments, regulatory sequences, introns, exons, segments (e.g., a segment spanning a copy number alteration region), microduplications, microdeletions and the like. A region sometimes is smaller than a chromosome of interest or is the same size of a chromosome of interest, and sometimes is smaller than a reference chromosome or is the same size as a reference chromosome.

*Filtering and/or selecting portions*

In some embodiments, one or more processing steps can comprise one or more portion filtering steps and/or portion selection steps. The term "filtering" as used herein refers to removing portions or portions of a reference genome from consideration. In certain embodiments one or more portions are filtered (e.g., subjected to a filtering process) thereby providing filtered portions. In some embodiments a filtering process removes certain portions and retains portions (e.g., a subset of portions). Following a filtering process, retained portions are often referred to herein as filtered portions.

Portions of a reference genome can be selected for removal based on any suitable criteria, including but not limited to redundant data (e.g., redundant or overlapping mapped reads), non-informative data (e.g., portions of a reference genome with zero median counts), portions of a reference genome with over represented or under represented sequences, noisy data, the like, or combinations of the foregoing. A filtering process often involves removing one or more portions of a reference genome from consideration and subtracting the counts in the one or more portions of a reference genome selected for removal from the counted or summed counts for the portions of a reference genome, chromosome or chromosomes, or genome under consideration. In some embodiments, portions of a reference genome can be removed successively (e.g., one at a time to allow evaluation of the effect of removal of each individual portion), and in certain embodiments all portions of a reference genome marked for removal can be removed at the same time. In some embodiments, portions of a reference genome characterized by a variance above or below a certain level are removed, which sometimes is referred to herein as filtering "noisy" portions of a reference genome. In certain embodiments, a filtering process comprises obtaining data points from a data set that deviate from the mean profile level of a portion, a chromosome, or part of a chromosome by a predetermined multiple of the profile variance, and in certain embodiments, a filtering process comprises removing data points from a data set that do not deviate from the mean profile level of a portion, a chromosome or part of a chromosome by a predetermined multiple of the profile variance. In some embodiments, a filtering process is utilized to reduce the number of candidate portions of a reference genome analyzed for the presence or absence of a genetic variation and/or copy number alteration (e.g., aneuploidy, microdeletion, microduplication). Reducing the number of candidate portions of a reference genome analyzed for the presence or absence of a genetic variation and/or copy number alteration often reduces the complexity and/or dimensionality of a data set, and sometimes increases the speed of searching for and/or identifying genetic variations and/or copy number alterations by two or more orders of magnitude.

Portions may be processed (e.g., filtered and/or selected) by any suitable method and according to any suitable parameter. Non-limiting examples of features and/or parameters that can be used to filter and/or select portions include redundant data (e.g., redundant or overlapping mapped reads), non-informative data (e.g., portions of a reference genome with zero mapped counts), portions of a reference genome with over represented or under represented sequences, noisy data, counts, count variability, coverage, mappability, variability, a repeatability measure, read density, variability of read density, a level of uncertainty, guanine-cytosine (GC) content, CCF fragment length and/or read length (e.g., a fragment length ratio (FLR), a fetal ratio statistic (FRS)), DNaseI-sensitivity, methylation state, acetylation, histone distribution, chromatin structure, percent repeats, the like or combinations thereof. Portions can be filtered and/or selected according to any suitable feature or parameter that correlates with a feature or parameter listed or described herein. Portions can be filtered and/or selected according to features or parameters that are specific to a portion (e.g., as determined for a single portion according to multiple samples) and/or features or parameters that are specific to a sample (e.g., as determined for multiple portions within a sample). In some embodiments portions are filtered and/or removed according to relatively low mappability, relatively high variability, a high level of uncertainty, relatively long CCF fragment lengths (e.g., low FRS, low FLR), relatively large fraction of repetitive sequences, high GC content, low GC content, low counts, zero counts, high counts, the like, or combinations thereof. In some embodiments portions (e.g., a subset of portions) are selected according to suitable level of mappability, variability, level of uncertainty, fraction of repetitive sequences, count, GC content, the like, or combinations thereof. In some embodiments portions (e.g., a subset of portions) are selected according to relatively short CCF fragment lengths (e.g., high FRS, high FLR). Counts and/or reads mapped to portions are sometimes processed (e.g., normalized) prior to and/or after filtering or selecting portions (e.g., a subset of portions). In some embodiments counts and/or reads mapped to portions are not processed prior to and/or after filtering or selecting portions (e.g., a subset of portions).

In some embodiments, portions may be filtered according to a measure of error (e.g., standard deviation, standard error, calculated variance, p-value, mean absolute error (MAE), average absolute deviation and/or mean absolute deviation (MAD)). In certain instances, a measure of error may refer to count variability. In some embodiments portions are filtered according to count variability. In certain embodiments count variability is a measure of error determined for counts mapped to a portion (i.e., portion) of a reference genome for multiple samples (e.g., multiple sample obtained from multiple subjects, e.g., 50 or more, 100 or more, 500 or more 1000 or more, 5000 or more or 10,000 or more subjects). In some embodiments, portions with a count variability above a pre-determined upper range are filtered (e.g., excluded from consideration). In some embodiments portions with a count variability below a pre-determined lower range are filtered (e.g., excluded from consideration). In some embodiments, portions with a count variability outside a pre-determined range are filtered (e.g., excluded from consideration). In some embodiments portions with a count variability within a pre-determined range are selected (e.g., used for determining the presence or absence of a copy number alteration). In some embodiments, count variability of portions represents a distribution (e.g., a normal distribution). In some embodiments portions are selected within a quantile of the distribution. In some embodiments portions within a 99% quantile of the distribution of count variability are selected.

Sequence reads from any suitable number of samples can be utilized to identify a subset of portions that meet one or more criteria, parameters and/or features described herein. Sequence reads from a group of samples from multiple subjects sometimes are utilized. In some embodiments, the multiple subjects include pregnant females. In some embodiments, the multiple subjects include healthy subjects. One or more samples from each of the multiple subjects can be addressed (e.g., 1 to about 20 samples from each subject (e.g., about 2, 3, 4, 5, 6, 7, 8, 9, 10, 11, 12, 13, 14, 15, 16, 17, 18 or 19 samples)), and a suitable number of subjects may be addressed (e.g., about 2 to about 10,000 subjects (e.g., about 10, 20, 30, 40, 50, 60, 70, 80, 90, 100, 150, 200, 250, 300, 350, 400, 500, 600, 700, 800, 900, 1000, 2000, 3000, 4000, 5000, 6000, 7000, 8000, 9000 subjects)). In some embodiments, sequence reads from the same test sample(s) from the same subject are mapped to portions in the reference genome and are used to generate the subset of portions.

Portions can be selected and/or filtered by any suitable method. In some embodiments portions are selected according to visual inspection of data, graphs, plots and/or charts. In certain embodiments portions are selected and/or filtered (e.g., in part) by a system or a machine comprising one or more microprocessors and memory. In some embodiments portions are selected and/or filtered (e.g., in part) by a non-transitory computer-readable storage medium with an executable program stored thereon, where the program instructs a microprocessor to perform the selecting and/or filtering.

In some embodiments, sequence reads derived from a sample are mapped to all or most portions of a reference genome and a pre-selected subset of portions are thereafter selected. For example, a subset of portions to which reads from fragments under a particular length threshold preferentially map may be selected. Certain methods for pre-selecting a subset of portions are described in U.S. Patent Application Publication No. 2014/0180594, which is incorporated by reference herein. Reads from a selected subset of portions often are utilized in further steps of a determination of the presence or absence of a genetic variation, for example. Often, reads from portions not selected are not utilized in further steps of a determination of the presence or absence of a genetic variation (e.g., reads in the non-selected portions are removed or filtered).

In some embodiments portions associated with read densities (e.g., where a read density is for a portion) are removed by a filtering process and read densities associated with removed portions are not included in a determination of the presence or absence of a copy number alteration (e.g., a chromosome aneuploidy, microduplication, microdeletion). In some embodiments a read density profile comprises and/or consists of read densities of filtered portions. Portions are sometimes filtered according to a distribution of counts and/or a distribution of read densities. In some embodiments portions are filtered according to a distribution of counts and/or read densities where the counts and/or read densities are obtained from one or more reference samples. One or more reference samples may be referred to herein as a training set. In some embodiments portions are filtered according to a distribution of counts and/or read densities where the counts and/or read densities are obtained from one or more test samples. In some embodiments portions are filtered according to a measure of uncertainty for a read density distribution. In certain embodiments, portions that demonstrate a large deviation in read densities are removed by a filtering process. For example, a distribution of read densities (e.g., a distribution of average mean, or median read densities) can be determined, where each read density in the distribution maps to the same portion. A measure of uncertainty (e.g., a MAD) can be determined by comparing a distribution of read densities for multiple samples where each portion of a genome is associated with measure of uncertainty. According to the foregoing example, portions can be filtered according to a measure of uncertainty (e.g., a standard deviation (SD), a MAD) associated with each portion and a predetermined threshold. In certain instances, portions comprising MAD values within the acceptable range are retained and portions comprising MAD values outside of the acceptable range are removed from consideration by a filtering process. In some embodiments, according to the foregoing example, portions comprising read densities values (e.g., median, average or mean read densities) outside a pre-determined measure of uncertainty are often removed from consideration by a filtering process. In some embodiments portions comprising read densities values (e.g., median, average or mean read densities) outside an inter-quartile range of a distribution are removed from consideration by a filtering process. In some embodiments portions comprising read densities values outside more than 2 times, 3 times, 4 times or 5 times an inter-quartile range of a distribution are removed from consideration by a filtering process. In some embodiments portions comprising read densities values outside more than 2 sigma, 3 sigma, 4 sigma, 5 sigma, 6 sigma, 7 sigma or 8 sigma (e.g., where sigma is a range defined by a standard deviation) are removed from consideration by a filtering process.

*Sequence read quantification*

Sequence reads that are mapped or partitioned based on a selected feature or variable can be quantified to determine the amount or number of reads that are mapped to one or more portions (e.g., portion of a reference genome), in some embodiments. In certain embodiments the quantity of sequence reads that are mapped to a portion or segment is referred to as a count or read density.

A count often is associated with a genomic portion. In some embodiments a count is determined from some or all of the sequence reads mapped to (i.e., associated with) a portion. In certain embodiments, a count is determined from some or all of the sequence reads mapped to a group of portions (e.g., portions in a segment or region (described herein)).

A count can be determined by a suitable method, operation or mathematical process. A count sometimes is the direct sum of all sequence reads mapped to a genomic portion or a group of genomic portions corresponding to a segment, a group of portions corresponding to a subregion of a genome (e.g., copy number variation region, copy number duplication region, copy number deletion region, microduplication region, microdeletion region, chromosome region, autosome region, sex chromosome region) and/or sometimes is a group of portions corresponding to a genome. A read quantification sometimes is a ratio, and sometimes is a ratio of a quantification for portion(s) in region *a* to a quantification for portion(s) in region *b*. Region *a* sometimes is one portion, segment region, copy number variation region, copy number duplication region, copy number deletion region, microduplication region, microdeletion region, chromosome region, autosome region and/or sex chromosome region. Region b independently sometimes is one portion, segment region, copy number variation region, copy number duplication region, copy number deletion region, microduplication region, microdeletion region, chromosome region, autosome region, sex chromosome region, a region including all autosomes, a region including sex chromosomes and/or a region including all chromosomes.

In some embodiments, a count is derived from raw sequence reads and/or filtered sequence reads. In certain embodiments a count is an average, mean or sum of sequence reads mapped to a genomic portion or group of genomic portions (e.g., genomic portions in a region). In some embodiments, a count is associated with an uncertainty value. A count sometimes is adjusted. A count may be adjusted according to sequence reads associated with a genomic portion or group of portions that have been weighted, removed, filtered, normalized, adjusted, averaged, derived as a mean, derived as a median, added, or combination thereof.

A sequence read quantification sometimes is a read density. A read density may be determined and/or generated for one or more segments of a genome. In certain instances, a read density may be determined and/or generated for one or more chromosomes. In some embodiments a read density comprises a quantitative measure of counts of sequence reads mapped to a segment or portion of a reference genome. A read density can be determined by a suitable process. In some embodiments a read density is determined by a suitable distribution and/or a suitable distribution function. Non-limiting examples of a distribution function include a probability function, probability distribution function, probability density function (PDF), a kernel density function (kernel density estimation), a cumulative distribution function, probability mass function, discrete probability distribution, an absolutely continuous univariate distribution, the like, any suitable distribution, or combinations thereof. A read density may be a density estimation derived from a suitable probability density function. A density estimation is the construction of an estimate, based on observed data, of an underlying probability density function. In some embodiments a read density comprises a density estimation (e.g., a probability density estimation, a kernel density estimation). A read density may be generated according to a process comprising generating a density estimation for each of the one or more portions of a genome where each portion comprises counts of sequence reads. A read density may be generated for normalized and/or weighted counts mapped to a portion or segment. In some instances, each read mapped to a portion or segment may contribute to a read density, a value (e.g., a count) equal to its weight obtained from a normalization process described herein. In some embodiments read densities for one or more portions or segments are adjusted. Read densities can be adjusted by a suitable method. For example, read densities for one or more portions can be weighted and/or normalized.

Reads quantified for a given portion or segment can be from one source or different sources. In one example, reads may be obtained from a nucleic acid sample from a pregnant female bearing a fetus. In such circumstances, reads mapped to one or more portions often are reads representative of both the fetus and the mother of the fetus (e.g., a pregnant female subject). In certain embodiments some of the reads mapped to a portion are from a fetal genome and some of the reads mapped to the same portion are from a maternal genome.

*Levels*

In some embodiments, a value (e.g., a number, a quantitative value) is ascribed to a level. A level can be determined by a suitable method, operation or mathematical process (e.g., a processed level). A level often is, or is derived from, counts (e.g., normalized counts) for a set of portions. In some embodiments a level of a portion is substantially equal to the total number of counts mapped to a portion (e.g., counts, normalized counts). Often a level is determined from counts that are processed, transformed or manipulated by a suitable method, operation or mathematical process known in the art. In some embodiments a level is derived from counts that are processed and non-limiting examples of processed counts include weighted, removed, filtered, normalized, adjusted, averaged, derived as a mean (e.g., mean level), added, subtracted, transformed counts or combination thereof. In some embodiments a level comprises counts that are normalized (e.g., normalized counts of portions). A level can be for counts normalized by a suitable process, non-limiting examples of which are described herein. A level can comprise normalized counts or relative amounts of counts. In some embodiments a level is for counts or normalized counts of two or more portions that are averaged and the level is referred to as an average level. In some embodiments a level is for a set of portions having a mean count or mean of normalized counts which is referred to as a mean level. In some embodiments a level is derived for portions that comprise raw and/or filtered counts. In some embodiments, a level is based on counts that are raw. In some embodiments a level is associated with an uncertainty value (e.g., a standard deviation, a MAD). In some embodiments a level is represented by a Z-score or p-value.

A level for one or more portions is synonymous with a "genomic section level" herein. The term "level" as used herein is sometimes synonymous with the term "elevation." A determination of the meaning of the term "level" can be determined from the context in which it is used. For example, the term "level," when used in the context of portions, profiles, reads and/or counts often means an elevation. The term "level," when used in the context of a substance or composition (e.g., level of RNA, plexing level) often refers to an amount. The term "level," when used in the context of uncertainty (e.g., level of error, level of confidence, level of deviation, level of uncertainty) often refers to an amount.

Normalized or non-normalized counts for two or more levels (e.g., two or more levels in a profile) can sometimes be mathematically manipulated (e.g., added, multiplied, averaged, normalized, the like or combination thereof) according to levels. For example, normalized or non-normalized counts for two or more levels can be normalized according to one, some or all of the levels in a profile. In some embodiments normalized or non-normalized counts of all levels in a profile are normalized according to one level in the profile. In some embodiments normalized or non-normalized counts of a fist level in a profile are normalized according to normalized or non-normalized counts of a second level in the profile.

Non-limiting examples of a level (e.g., a first level, a second level) are a level for a set of portions comprising processed counts, a level for a set of portions comprising a mean, median or average of counts, a level for a set of portions comprising normalized counts, the like or any combination thereof. In some embodiments, a first level and a second level in a profile are derived from counts of portions mapped to the same chromosome. In some embodiments, a first level and a second level in a profile are derived from counts of portions mapped to different chromosomes.

In some embodiments a level is determined from normalized or non-normalized counts mapped to one or more portions. In some embodiments, a level is determined from normalized or non-normalized counts mapped to two or more portions, where the normalized counts for each portion often are about the same. There can be variation in counts (e.g., normalized counts) in a set of portions for a level. In a set of portions for a level there can be one or more portions having counts that are significantly different than in other portions of the set (e.g., peaks and/or dips). Any suitable number of normalized or non-normalized counts associated with any suitable number of portions can define a level.

In some embodiments one or more levels can be determined from normalized or non-normalized counts of all or some of the portions of a genome. Often a level can be determined from all or some of the normalized or non-normalized counts of a chromosome, or part thereof. In some embodiments, two or more counts derived from two or more portions (e.g., a set of portions) determine a level. In some embodiments two or more counts (e.g., counts from two or more portions) determine a level. In some embodiments, counts from 2 to about 100,000 portions determine a level. In some embodiments, counts from 2 to about 50,000, 2 to about 40,000, 2 to about 30,000, 2 to about 20,000, 2 to about 10,000, 2 to about 5000, 2 to about 2500, 2 to about 1250, 2 to about 1000, 2 to about 500, 2 to about 250, 2 to about 100 or 2 to about 60 portions determine a level. In some embodiments counts from about 10 to about 50 portions determine a level. In some embodiments counts from about 20 to about 40 or more portions determine a level. In some embodiments, a level comprises counts from about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60 or more portions. In some embodiments, a level corresponds to a set of portions (e.g., a set of portions of a reference genome, a set of portions of a chromosome or a set of portions of a part of a chromosome).

In some embodiments, a level is determined for normalized or non-normalized counts of portions that are contiguous. In some embodiments portions (e.g., a set of portions) that are contiguous represent neighboring regions of a genome or neighboring regions of a chromosome or gene. For example, two or more contiguous portions, when aligned by merging the portions end to end, can represent a sequence assembly of a DNA sequence longer than each portion. For example two or more contiguous portions can represent of an intact genome, chromosome, gene, intron, exon or part thereof. In some embodiments a level is determined from a collection (e.g., a set) of contiguous portions and/or non-contiguous portions.

*Data processing and normalization*

Mapped sequence reads that have been counted are referred to herein as raw data, since the data represents unmanipulated counts (e.g., raw counts). In some embodiments, sequence read data in a data set can be processed further (e.g., mathematically and/or statistically manipulated) and/or displayed to facilitate providing an outcome. In certain embodiments, data sets, including larger data sets, may benefit from pre-processing to facilitate further analysis. Pre-processing of data sets sometimes involves removal of redundant and/or uninformative portions or portions of a reference genome (e.g., portions of a reference genome with uninformative data, redundant mapped reads, portions with zero median counts, over represented or under represented sequences). Without being limited by theory, data processing and/or preprocessing may (i) remove noisy data, (ii) remove uninformative data, (iii) remove redundant data, (iv) reduce the complexity of larger data sets, and/or (v) facilitate transformation of the data from one form into one or more other forms. The terms "pre-processing" and "processing" when utilized with respect to data or data sets are collectively referred to herein as "processing." Processing can render data more amenable to further analysis, and can generate an outcome in some embodiments. In some embodiments one or more or all processing methods (e.g., normalization methods, portion filtering, mapping, validation, the like or combinations thereof) are performed by a processor, a micro-processor, a computer, in conjunction with memory and/or by a microprocessor controlled apparatus.

The term "noisy data" as used herein refers to (a) data that has a significant variance between data points when analyzed or plotted, (b) data that has a significant standard deviation (e.g., greater than 3 standard deviations), (c) data that has a significant standard error of the mean, the like, and combinations of the foregoing. Noisy data sometimes occurs due to the quantity and/or quality of starting material (e.g., nucleic acid sample), and sometimes occurs as part of processes for preparing or replicating DNA used to generate sequence reads. In certain embodiments, noise results from certain sequences being overrepresented when prepared using PCR-based methods. Methods described herein can reduce or eliminate the contribution of noisy data, and therefore reduce the effect of noisy data on the provided outcome.

The terms "uninformative data," "uninformative portions of a reference genome," and "uninformative portions" as used herein refer to portions, or data derived therefrom, having a numerical value that is significantly different from a predetermined threshold value or falls outside a predetermined cutoff range of values. The terms "threshold" and "threshold value" herein refer to any number that is calculated using a qualifying data set and serves as a limit of diagnosis of a genetic variation (e.g., a copy number alteration, an aneuploidy, a microduplication, a microdeletion, a chromosomal aberration, and the like). In certain embodiments, a threshold is exceeded by results obtained by methods described herein and a subject is diagnosed with a copy number alteration. A threshold value or range of values often is calculated by mathematically and/or statistically manipulating sequence read data (e.g., from a reference and/or subject), in some embodiments, and in certain embodiments, sequence read data manipulated to generate a threshold value or range of values is sequence read data (e.g., from a reference and/or subject). In some embodiments, an uncertainty value is determined. An uncertainty value generally is a measure of variance or error and can be any suitable measure of variance or error. In some embodiments an uncertainty value is a standard deviation, standard error, calculated variance, p-value, or mean absolute deviation (MAD). In some embodiments an uncertainty value can be calculated according to a formula described herein.

Any suitable procedure can be utilized for processing data sets described herein. Non-limiting examples of procedures suitable for use for processing data sets include filtering, normalizing, weighting, monitoring peak heights, monitoring peak areas, monitoring peak edges, peak level analysis, peak width analysis, peak edge location analysis, peak lateral tolerances, determining area ratios, mathematical processing of data, statistical processing of data, application of statistical algorithms, analysis with fixed variables, analysis with optimized variables, plotting data to identify patterns or trends for additional processing, the like and combinations of the foregoing. In some embodiments, data sets are processed based on various features (e.g., GC content, redundant mapped reads, centromere regions, telomere regions, the like and combinations thereof) and/or variables (e.g., subject gender, subject age, subject ploidy, fetal gender, maternal age, maternal ploidy, percent contribution of fetal nucleic acid, the like or combinations thereof). In certain embodiments, processing data sets as described herein can reduce the complexity and/or dimensionality of large and/or complex data sets. A non-limiting example of a complex data set includes sequence read data generated from one or more test subjects and a plurality of reference subjects of different ages and ethnic backgrounds. In some embodiments, data sets can include from thousands to millions of sequence reads for each test and/or reference subject.

Data processing can be performed in any number of steps, in certain embodiments. For example, data may be processed using only a single processing procedure in some embodiments, and in certain embodiments data may be processed using 1 or more, 5 or more, 10 or more or 20 or more processing steps (e.g., 1 or more processing steps, 2 or more processing steps, 3 or more processing steps, 4 or more processing steps, 5 or more processing steps, 6 or more processing steps, 7 or more processing steps, 8 or more processing steps, 9 or more processing steps, 10 or more processing steps, 11 or more processing steps, 12 or more processing steps, 13 or more processing steps, 14 or more processing steps, 15 or more processing steps, 16 or more processing steps, 17 or more processing steps, 18 or more processing steps, 19 or more processing steps, or 20 or more processing steps). In some embodiments, processing steps may be the same step repeated two or more times (e.g., filtering two or more times, normalizing two or more times), and in certain embodiments, processing steps may be two or more different processing steps (e.g., filtering, normalizing; normalizing, monitoring peak heights and edges; filtering, normalizing, normalizing to a reference, statistical manipulation to determine p-values, and the like), carried out simultaneously or sequentially. In some embodiments, any suitable number and/or combination of the same or different processing steps can be utilized to process sequence read data to facilitate providing an outcome. In certain embodiments, processing data sets by the criteria described herein may reduce the complexity and/or dimensionality of a data set.

In some embodiments one or more processing steps can comprise one or more normalization steps. Normalization can be performed by a suitable method described herein or known in the art. In certain embodiments, normalization comprises adjusting values measured on different scales to a notionally common scale. In certain embodiments, normalization comprises a sophisticated mathematical adjustment to bring probability distributions of adjusted values into alignment. In some embodiments normalization comprises aligning distributions to a normal distribution. In certain embodiments normalization comprises mathematical adjustments that allow comparison of corresponding normalized values for different datasets in a way that eliminates the effects of certain gross influences (e.g., error and anomalies). In certain embodiments normalization comprises scaling. Normalization sometimes comprises division of one or more data sets by a predetermined variable or formula. Normalization sometimes comprises subtraction of one or more data sets by a predetermined variable or formula. Non-limiting examples of normalization methods include portion-wise normalization, normalization by GC content, median count (median bin count, median portion count) normalization, linear and nonlinear least squares regression, LOESS, GC LOESS, LOWESS (locally weighted scatterplot smoothing), principal component normalization, repeat masking (RM), GC-normalization and repeat masking (GCRM), cQn and/or combinations thereof. In some embodiments, the determination of a presence or absence of a copy number alteration (e.g., an aneuploidy, a microduplication, a microdeletion) utilizes a normalization method (e.g., portion-wise normalization, normalization by GC content, median count (median bin count, median portion count) normalization, linear and nonlinear least squares regression, LOESS, GC LOESS, LOWESS (locally weighted scatterplot smoothing), principal component normalization, repeat masking (RM), GC-normalization and repeat masking (GCRM), cQn, a normalization method known in the art and/or a combination thereof). Described in greater detail hereafter are certain examples of normalization processes that can be utilized, such as LOESS normalization, principal component normalization, and hybrid normalization methods, for example. Aspects of certain normalization processes also are described, for example, in International Patent Application Publication No. WO2013/052913 and International Patent Application Publication No. WO2015/05 1 163, each of which is incorporated by reference herein.

Any suitable number of normalizations can be used. In some embodiments, data sets can be normalized 1 or more, 5 or more, 10 or more or even 20 or more times. Data sets can be normalized to values (e.g., normalizing value) representative of any suitable feature or variable (e.g., sample data, reference data, or both). Non-limiting examples of types of data normalizations that can be used include normalizing raw count data for one or more selected test or reference portions to the total number of counts mapped to the chromosome or the entire genome on which the selected portion or sections are mapped; normalizing raw count data for one or more selected portions to a median reference count for one or more portions or the chromosome on which a selected portion is mapped; normalizing raw count data to previously normalized data or derivatives thereof; and normalizing previously normalized data to one or more other predetermined normalization variables. Normalizing a data set sometimes has the effect of isolating statistical error, depending on the feature or property selected as the predetermined normalization variable. Normalizing a data set sometimes also allows comparison of data characteristics of data having different scales, by bringing the data to a common scale (e.g., predetermined normalization variable). In some embodiments, one or more normalizations to a statistically derived value can be utilized to minimize data differences and diminish the importance of outlying data. Normalizing portions, or portions of a reference genome, with respect to a normalizing value sometimes is referred to as "portion-wise normalization."

In certain embodiments, a processing step can comprise one or more mathematical and/or statistical manipulations. Any suitable mathematical and/or statistical manipulation, alone or in combination, may be used to analyze and/or manipulate a data set described herein. Any suitable number of mathematical and/or statistical manipulations can be used. In some embodiments, a data set can be mathematically and/or statistically manipulated 1 or more, 5 or more, 10 or more or 20 or more times. Non-limiting examples of mathematical and statistical manipulations that can be used include addition, subtraction, multiplication, division, algebraic functions, least squares estimators, curve fitting, differential equations, rational polynomials, double polynomials, orthogonal polynomials, z-scores, p-values, chi values, phi values, analysis of peak levels, determination of peak edge locations, calculation of peak area ratios, analysis of median chromosomal level, calculation of mean absolute deviation, sum of squared residuals, mean, standard deviation, standard error, the like or combinations thereof. A mathematical and/or statistical manipulation can be performed on all or a portion of sequence read data, or processed products thereof. Non-limiting examples of data set variables or features that can be statistically manipulated include raw counts, filtered counts, normalized counts, peak heights, peak widths, peak areas, peak edges, lateral tolerances, P-values, median levels, mean levels, count distribution within a genomic region, relative representation of nucleic acid species, the like or combinations thereof.

In some embodiments, a processing step can comprise the use of one or more statistical algorithms. Any suitable statistical algorithm, alone or in combination, may be used to analyze and/or manipulate a data set described herein. Any suitable number of statistical algorithms can be used. In some embodiments, a data set can be analyzed using 1 or more, 5 or more, 10 or more or 20 or more statistical algorithms. Non-limiting examples of statistical algorithms suitable for use with methods described herein include principal component analysis, decision trees, counternulls, multiple comparisons, omnibus test, Behrens-Fisher problem, bootstrapping, Fisher's method for combining independent tests of significance, null hypothesis, type I error, type II error, exact test, one-sample Z test, two-sample Z test, one-sample t-test, paired t-test, two-sample pooled t-test having equal variances, two-sample unpooled t-test having unequal variances, one-proportion z-test, two-proportion z-test pooled, two-proportion z-test unpooled, one-sample chi-square test, two-sample F test for equality of variances, confidence interval, credible interval, significance, meta analysis, simple linear regression, robust linear regression, the like or combinations of the foregoing. Non-limiting examples of data set variables or features that can be analyzed using statistical algorithms include raw counts, filtered counts, normalized counts, peak heights, peak widths, peak edges, lateral tolerances, P-values, median levels, mean levels, count distribution within a genomic region, relative representation of nucleic acid species, the like or combinations thereof.

In certain embodiments, a data set can be analyzed by utilizing multiple (e.g., 2 or more) statistical algorithms (e.g., least squares regression, principal component analysis, linear discriminant analysis, quadratic discriminant analysis, bagging, neural networks, support vector machine models, random forests, classification tree models, K-nearest neighbors, logistic regression and/or smoothing) and/or mathematical and/or statistical manipulations (e.g., referred to herein as manipulations). The use of multiple manipulations can generate an N-dimensional space that can be used to provide an outcome, in some embodiments. In certain embodiments, analysis of a data set by utilizing multiple manipulations can reduce the complexity and/or dimensionality of the data set. For example, the use of multiple manipulations on a reference data set can generate an N-dimensional space (e.g., probability plot) that can be used to represent the presence or absence of a genetic variation and/or copy number alteration, depending on the status of the reference samples (e.g., positive or negative for a selected copy number alteration). Analysis of test samples using a substantially similar set of manipulations can be used to generate an N-dimensional point for each of the test samples. The complexity and/or dimensionality of a test subject data set sometimes is reduced to a single value or N-dimensional point that can be readily compared to the N-dimensional space generated from the reference data. Test sample data that fall within the N-dimensional space populated by the reference subject data are indicative of a genetic status substantially similar to that of the reference subjects. Test sample data that fall outside of the N-dimensional space populated by the reference subject data are indicative of a genetic status substantially dissimilar to that of the reference subjects. In some embodiments, references are euploid or do not otherwise have a genetic variation and/or copy number alteration and/or medical condition.

After data sets have been counted, optionally filtered, normalized, and optionally weighted the processed data sets can be further manipulated by one or more filtering and/or normalizing and/or weighting procedures, in some embodiments. A data set that has been further manipulated by one or more filtering and/or normalizing and/or weighting procedures can be used to generate a profile, in certain embodiments. The one or more filtering and/or normalizing and/or weighting procedures sometimes can reduce data set complexity and/or dimensionality, in some embodiments. An outcome can be provided based on a data set of reduced complexity and/or dimensionality. In some embodiments, a profile plot of processed data further manipulated by weighting, for example, is generated to facilitate classification and/or providing an outcome. An outcome can be provided based on a profile plot of weighted data, for example.

Filtering or weighting of portions can be performed at one or more suitable points in an analysis. For example, portions may be filtered or weighted before or after sequence reads are mapped to portions of a reference genome. Portions may be filtered or weighted before or after an experimental bias for individual genome portions is determined in some embodiments. In certain embodiments, portions may be filtered or weighted before or after levels are calculated.

After data sets have been counted, optionally filtered, normalized, and optionally weighted, the processed data sets can be manipulated by one or more mathematical and/or statistical (e.g., statistical functions or statistical algorithm) manipulations, in some embodiments. In certain embodiments, processed data sets can be further manipulated by calculating Z-scores for one or more selected portions, chromosomes, or portions of chromosomes. In some embodiments, processed data sets can be further manipulated by calculating P-values. In certain embodiments, mathematical and/or statistical manipulations include one or more assumptions pertaining to ploidy and/or fraction of a minority species (e.g., .fetal fraction). In some embodiments, a profile plot of processed data further manipulated by one or more statistical and/or mathematical manipulations is generated to facilitate classification and/or providing an outcome. An outcome can be provided based on a profile plot of statistically and/or mathematically manipulated data. An outcome provided based on a profile plot of statistically and/or mathematically manipulated data often includes one or more assumptions pertaining to ploidy and/or fraction of a minority species (e.g.,; fetal fraction).

In some embodiments, analysis and processing of data can include the use of one or more assumptions. A suitable number or type of assumptions can be utilized to analyze or process a data set. Non-limiting examples of assumptions that can be used for data processing and/or analysis include subject ploidy, cancer cell contribution, maternal ploidy, fetal contribution, prevalence of certain sequences in a reference population, ethnic background, prevalence of a selected medical condition in related family members, parallelism between raw count profiles from different patients and/or runs after GC-normalization and repeat masking (e.g., GCRM), identical matches represent PCR artifacts (e.g., identical base position), assumptions inherent in a nucleic acid quantification assay (e.g., fetal quantifier assay (FQA)), assumptions regarding twins (e.g., if 2 twins and only 1 is affected the effective fetal fraction is only 50% of the total measured fetal fraction (similarly for triplets, quadruplets and the like)), cell free DNA (e.g., cfDNA) uniformly covers the entire genome, the like and combinations thereof.

In those instances where the quality and/or depth of mapped sequence reads does not permit an outcome prediction of the presence or absence of a genetic variation and/or copy number alteration at a desired confidence level (e.g., 95% or higher confidence level), based on the normalized count profiles, one or more additional mathematical manipulation algorithms and/or statistical prediction algorithms, can be utilized to generate additional numerical values useful for data analysis and/or providing an outcome. The term "normalized count profile" as used herein refers to a profile generated using normalized counts. Examples of methods that can be used to generate normalized counts and normalized count profiles are described herein. As noted, mapped sequence reads that have been counted can be normalized with respect to test sample counts or reference sample counts. In some embodiments, a normalized count profile can be presented as a plot.

Described in greater detail hereafter are non-limiting examples of processing steps and normalization methods that can be utilized, such as normalizing to a window (static or sliding), weighting, determining bias relationship, LOESS normalization, principal component normalization, hybrid normalization, generating a profile and performing a comparison.

*Normalizing to a window (static or sliding)*

In certain embodiments, a processing step comprises normalizing to a static window, and in some embodiments, a processing step comprises normalizing to a moving or sliding window.

The term "window" as used herein refers to one or more portions chosen for analysis, and sometimes is used as a reference for comparison (e.g., used for normalization and/or other mathematical or statistical manipulation). The term "normalizing to a static window" as used herein refers to a normalization process using one or more portions selected for comparison between a test subject and reference subject data set. In some embodiments the selected portions are utilized to generate a profile. A static window generally includes a predetermined set of portions that do not change during manipulations and/or analysis. The terms "normalizing to a moving window" and "normalizing to a sliding window" as used herein refer to normalizations performed to portions localized to the genomic region (e.g., immediate surrounding portions, adjacent portion or sections, and the like) of a selected test portion, where one or more selected test portions are normalized to portions immediately surrounding the selected test portion. In certain embodiments, the selected portions are utilized to generate a profile. A sliding or moving window normalization often includes repeatedly moving or sliding to an adjacent test portion, and normalizing the newly selected test portion to portions immediately surrounding or adjacent to the newly selected test portion, where adjacent windows have one or more portions in common. In certain embodiments, a plurality of selected test portions and/or chromosomes can be analyzed by a sliding window process.

In some embodiments, normalizing to a sliding or moving window can generate one or more values, where each value represents normalization to a different set of reference portions selected from different regions of a genome (e.g., chromosome). In certain embodiments, the one or more values generated are cumulative sums (e.g., a numerical estimate of the integral of the normalized count profile over the selected portion, domain (e.g., part of chromosome), or chromosome). The values generated by the sliding or moving window process can be used to generate a profile and facilitate arriving at an outcome. In some embodiments, cumulative sums of one or more portions can be displayed as a function of genomic position. Moving or sliding window analysis sometimes is used to analyze a genome for the presence or absence of microdeletions and/or microduplications. In certain embodiments, displaying cumulative sums of one or more portions is used to identify the presence or absence of regions of copy number alteration (e.g., microdeletion, microduplication).

*Weighting*

In some embodiments, a processing step comprises a weighting. The terms "weighted," "weighting" or "weight function" or grammatical derivatives or equivalents thereof, as used herein, refer to a mathematical manipulation of a portion or all of a data set sometimes utilized to alter the influence of certain data set features or variables with respect to other data set features or variables (e.g., increase or decrease the significance and/or contribution of data contained in one or more portions or portions of a reference genome, based on the quality or usefulness of the data in the selected portion or portions of a reference genome). A weighting function can be used to increase the influence of data with a relatively small measurement variance, and/or to decrease the influence of data with a relatively large measurement variance, in some embodiments. For example, portions of a reference genome with under represented or low quality sequence data can be "down weighted" to minimize the influence on a data set, whereas selected portions of a reference genome can be "up weighted" to increase the influence on a data set. A non-limiting example of a weighting function is [1 / (standard deviation)$^2$]. Weighting portions sometimes removes portion dependencies. In some embodiments one or more portions are weighted by an eigen function (e.g., an eigenfunction). In some embodiments an eigen function comprises replacing portions with orthogonal eigen-portions. A weighting step sometimes is performed in a manner substantially similar to a normalizing step. In some embodiments, a data set is adjusted (e.g., divided, multiplied, added, subtracted) by a predetermined variable (e.g., weighting variable). In some embodiments, a data set is divided by a predetermined variable (e.g., weighting variable). A predetermined variable (e.g., minimized target function, Phi) often is selected to weigh different parts of a data set differently (e.g., increase the influence of certain data types while decreasing the influence of other data types).

*Bias relationships*

In some embodiments, a processing step comprises determining a bias relationship. For example, one or more relationships may be generated between local genome bias estimates and bias frequencies. The term "relationship" as use herein refers to a mathematical and/or a graphical relationship between two or more variables or values. A relationship can be generated by a suitable mathematical and/or graphical process. Non-limiting examples of a relationship include a mathematical and/or graphical representation of a function, a correlation, a distribution, a linear or non-linear equation, a line, a regression, a fitted regression, the like or a combination thereof. Sometimes a relationship comprises a fitted relationship. In some embodiments a fitted relationship comprises a fitted regression. Sometimes a relationship comprises two or more variables or values that are weighted. In some embodiments a relationship comprise a fitted regression where one or more variables or values of the relationship a weighted. Sometimes a regression is fitted in a weighted fashion. Sometimes a regression is fitted without weighting. In certain embodiments, generating a relationship comprises plotting or graphing.

In certain embodiments, a relationship is generated between GC densities and GC density frequencies. In some embodiments generating a relationship between (i) GC densities and (ii) GC density frequencies for a sample provides a sample GC density relationship. In some embodiments generating a relationship between (i) GC densities and (ii) GC density frequencies for a reference provides a reference GC density relationship. In some embodiments, where local genome bias estimates are GC densities, a sample bias relationship is a sample GC density relationship and a reference bias relationship is a reference GC density relationship. GC densities of a reference GC density relationship and/or a sample GC density relationship are often representations (e.g., mathematical or quantitative representation) of local GC content.

In some embodiments a relationship between local genome bias estimates and bias frequencies comprises a distribution. In some embodiments a relationship between local genome bias estimates and bias frequencies comprises a fitted relationship (e.g., a fitted regression). In some embodiments a relationship between local genome bias estimates and bias frequencies comprises a fitted linear or non-linear regression (e.g., a polynomial regression). In certain embodiments a relationship between local genome bias estimates and bias frequencies comprises a weighted relationship where local genome bias estimates and/or bias frequencies are weighted by a suitable process. In some embodiments a weighted fitted relationship (e.g., a weighted fitting) can be obtained by a process comprising a quantile regression, parameterized distributions or an empirical distribution with interpolation. In certain embodiments a relationship between local genome bias estimates and bias frequencies for a test sample, a reference or part thereof, comprises a polynomial regression where local genome bias estimates are weighted. In some embodiments a weighed fitted model comprises weighting values of a distribution. Values of a distribution can be weighted by a suitable process. In some embodiments, values located near tails of a distribution are provided less weight than values closer to the median of the distribution. For example, for a distribution between local genome bias estimates (e.g., GC densities) and bias frequencies (e.g., GC density frequencies), a weight is determined according to the bias frequency for a given local genome bias estimate, where local genome bias estimates comprising bias frequencies closer to the mean of a distribution are provided greater weight than local genome bias estimates comprising bias frequencies further from the mean.

In some embodiments, a processing step comprises normalizing sequence read counts by comparing local genome bias estimates of sequence reads of a test sample to local genome bias estimates of a reference (e.g., a reference genome, or part thereof). In some embodiments, counts of sequence reads are normalized by comparing bias frequencies of local genome bias estimates of a test sample to bias frequencies of local genome bias estimates of a reference. In some embodiments counts of sequence reads are normalized by comparing a sample bias relationship and a reference bias relationship, thereby generating a comparison.

Counts of sequence reads may be normalized according to a comparison of two or more relationships. In certain embodiments two or more relationships are compared thereby providing a comparison that is used for reducing local bias in sequence reads (e.g., normalizing counts). Two or more relationships can be compared by a suitable method. In some embodiments a comparison comprises adding, subtracting, multiplying and/or dividing a first relationship from a second relationship. In certain embodiments comparing two or more relationships comprises a use of a suitable linear regression and/or a non-linear regression. In certain embodiments comparing two or more relationships comprises a suitable polynomial regression (e.g., a $3^{rd}$ order polynomial regression). In some embodiments a comparison comprises adding, subtracting, multiplying and/or dividing a first regression from a second regression. In some embodiments two or more relationships are compared by a process comprising an inferential framework of multiple regressions. In some embodiments two or more relationships are compared by a process comprising a suitable multivariate analysis. In some embodiments two or more relationships are compared by a process comprising a basis function (e.g., a blending function, e.g., polynomial bases, Fourier bases, or the like), splines, a radial basis function and/or wavelets.

In certain embodiments a distribution of local genome bias estimates comprising bias frequencies for a test sample and a reference is compared by a process comprising a polynomial regression where local genome bias estimates are weighted. In some embodiments a polynomial regression is generated between (i) ratios, each of which ratios comprises bias frequencies of local genome bias estimates of a reference and bias frequencies of local genome bias estimates of a sample and (ii) local genome bias estimates. In some embodiments a polynomial regression is generated between (i) a ratio of bias frequencies of local genome bias estimates of a reference to bias frequencies of local genome bias estimates of a sample and (ii) local genome bias estimates. In some embodiments a comparison of a distribution of local genome bias estimates for reads of a test sample and a reference comprises determining a log ratio (e.g., a log2 ratio) of bias frequencies of local genome bias estimates for the reference and the sample. In some embodiments a comparison of a distribution of local genome bias estimates comprises dividing a log ratio (e.g., a log2 ratio) of bias frequencies of local genome bias estimates for the reference by a log ratio (e.g., a log2 ratio) of bias frequencies of local genome bias estimates for the sample.

Normalizing counts according to a comparison typically adjusts some counts and not others. Normalizing counts sometimes adjusts all counts and sometimes does not adjust any counts of sequence reads. A count for a sequence read sometimes is normalized by a process that comprises determining a weighting factor and sometimes the process does not include directly generating and utilizing a weighting factor. Normalizing counts according to a comparison sometimes comprises determining a weighting factor for each count of a sequence read. A weighting factor is often specific to a sequence read and is applied to a count of a specific sequence read. A weighting factor is often determined according to a comparison of two or more bias relationships (e.g., a sample bias relationship compared to a reference bias relationship). A normalized count is often determined by adjusting a count value according to a weighting factor. Adjusting a count according to a weighting factor sometimes includes adding, subtracting, multiplying and/or dividing a count for a sequence read by a weighting factor. A weighting factor and/or a normalized count sometimes are determined from a regression (e.g., a regression line). A normalized count is sometimes obtained directly from a regression line (e.g., a fitted regression line) resulting from a comparison between bias frequencies of local genome bias estimates of a reference (e.g., a reference genome) and a test sample. In some embodiments each count of a read of a sample is provided a normalized count value according to a comparison of (i) bias frequencies of a local genome bias estimates of reads compared to (ii) bias frequencies of a local genome bias estimates of a reference. In certain embodiments, counts of sequence reads obtained for a sample are normalized and bias in the sequence reads is reduced.

*LOESS normalization*

In some embodiments, a processing step comprises a LOESS normalization. LOESS is a regression modeling method known in the art that combines multiple regression models in a k-nearest-neighbor-based meta-model. LOESS is sometimes referred to as a locally weighted polynomial regression. GC LOESS, in some embodiments, applies an LOESS model to the relationship between fragment count (e.g., sequence reads, counts) and GC composition for portions of a reference genome. Plotting a smooth curve through a set of data points using LOESS is sometimes called an LOESS curve, particularly when each smoothed value is given by a weighted quadratic least squares regression over the span of values of the y-axis scattergram criterion variable. For each point in a data set, the LOESS method fits a low-degree polynomial to a subset of the data, with explanatory variable values near the point whose response is being estimated. The polynomial is fitted using weighted least squares, giving more weight to points near the point whose response is being estimated and less weight to points further away. The value of the regression function for a point is then obtained by evaluating the local polynomial using the explanatory variable values for that data point. The LOESS fit is sometimes considered complete after regression function values have been computed for each of the data points. Many of the details of this method, such as the degree of the polynomial model and the weights, are flexible.

*Principal component analysis*

In some embodiments, a processing step comprises a principal component analysis (PCA). In some embodiments, sequence read counts (e.g., sequence read counts of a test sample) is adjusted according to a principal component analysis (PCA). In some embodiments a read density profile (e.g., a read density profile of a test sample) is adjusted according to a principal component analysis (PCA). A read density profile of one or more reference samples and/or a read density profile of a test subject can be adjusted according to a PCA. Removing bias from a read density profile by a PCA related process is sometimes referred to herein as adjusting a profile. A PCA can be performed by a suitable PCA method, or a variation thereof. Non-limiting examples of a PCA method include a canonical correlation analysis (CCA), a Karhunen-Loeve transform (KLT), a Hotelling transform, a proper orthogonal decomposition (POD), a singular value decomposition (SVD) of X, an eigenvalue decomposition (EVD) of XTX, a factor analysis, an Eckart-Young theorem, a Schmidt-Mirsky theorem, empirical orthogonal functions (EOF), an empirical eigenfunction decomposition, an empirical component analysis, quasiharmonic modes, a spectral decomposition, an empirical modal analysis, the like, variations or combinations thereof. A PCA often identifies and/or adjusts for one or more biases in a read density profile. A bias identified and/or adjusted for by a PCA is sometimes referred to herein as a principal component. In some embodiments one or more biases can be removed by adjusting a read density profile according to one or more principal component using a suitable method. A read density profile can be adjusted by adding, subtracting, multiplying and/or dividing one or more principal components from a read density profile. In some embodiments, one or more biases can be removed from a read density profile by subtracting one or more principal components from a read density profile. Although bias in a read density profile is often identified and/or quantitated by a PCA of a profile, principal components are often subtracted from a profile at the level of read densities. A PCA often identifies one or more principal components. In some embodiments a PCA identifies a 1$^{st}$, 2$^{nd}$, 3$^{rd}$, 4$^{th}$, 5$^{th}$, 6$^{th}$, 7$^{th}$, 8$^{th}$, 9$^{th}$, and a 10$^{th}$ or more principal components. In certain embodiments, 1, 2, 3, 4, 5, 6, 7, 8, 9, 10 or more principal components are used to adjust a profile. In certain embodiments, 5 principal components are used to adjust a profile. Often, principal components are used to adjust a profile in the order of appearance in a PCA. For example, where three principal components are subtracted from a read density profile, a 1$^{st}$, 2$^{nd}$ and 3$^{rd}$ principal component are used. Sometimes a bias identified by a principal component comprises a feature of a profile that is not used to adjust a profile. For example, a PCA may identify a copy number alteration (e.g., an aneuploidy, microduplication, microdeletion, deletion, translocation, insertion) and/or a gender difference as a principal component. Thus, in some embodiments, one or more principal components are not used to adjust a profile. For example, sometimes a 1$^{st}$, 2$^{nd}$ and 4$^{th}$ principal component are used to adjust a profile where a 3$^{rd}$ principal component is not used to adjust a profile.

A principal component can be obtained from a PCA using any suitable sample or reference. In some embodiments principal components are obtained from a test sample (e.g., a test subject). In some embodiments principal components are obtained from one or more references (e.g., reference samples, reference sequences, a reference set). In certain instances, a PCA is performed on a median read density profile obtained from a training set comprising multiple samples resulting in the identification of a 1$^{st}$ principal component and a 2$^{nd}$ principal component. In some embodiments, principal components are obtained from a set of subjects devoid of a copy number alteration in question. In some embodiments, principal components are obtained from a set of known euploids. Principal component are often identified according to a PCA performed using one or more read density profiles of a reference (e.g., a training set). One or more principal components obtained from a reference are often subtracted from a read density profile of a test subject thereby providing an adjusted profile.

*Hybrid normalization*

In some embodiments, a processing step comprises a hybrid normalization method. A hybrid normalization method may reduce bias (e.g., GC bias), in certain instances. A hybrid normalization, in some embodiments, comprises (i) an analysis of a relationship of two variables (e.g., counts and GC content) and (ii) selection and application of a normalization method according to the analysis. A hybrid normalization, in certain embodiments, comprises (i) a regression (e.g., a regression analysis) and (ii) selection and application of a normalization method according to the regression. In some embodiments counts obtained for a first sample (e.g., a first set of samples) are normalized by a different method than counts obtained from another sample (e.g., a second set of samples). In some embodiments counts obtained for a first sample (e.g., a first set of samples) are normalized by a first normalization method and counts obtained from a second sample (e.g., a second set of samples) are normalized by a second normalization method. For example, in certain embodiments a first normalization method comprises use of a linear regression and a second normalization method comprises use of a non-linear regression (e.g., a LOESS, GC-LOESS, LOWESS regression, LOESS smoothing).

In some embodiments a hybrid normalization method is used to normalize sequence reads mapped to portions of a genome or chromosome (e.g., counts, mapped counts, mapped reads). In certain embodiments raw counts are normalized and in some embodiments adjusted, weighted, filtered or previously normalized counts are normalized by a hybrid normalization method. In certain embodiments, levels or Z-scores are normalized. In some embodiments counts mapped to selected portions of a genome or chromosome are normalized by a hybrid normalization approach. Counts can refer to a suitable measure of sequence reads mapped to portions of a genome, non-limiting examples of which include raw counts (e.g., unprocessed counts), normalized counts (e.g., normalized by LOESS, principal component, or a suitable method), portion levels (e.g., average levels, mean levels, median levels, or the like), Z-scores, the like, or combinations thereof. The counts can be raw counts or processed counts from one or more samples (e.g., a test sample, a sample from a pregnant female). In some embodiments counts are obtained from one or more samples obtained from one or more subjects.

In some embodiments a normalization method (e.g., the type of normalization method) is selected according to a regression (e.g., a regression analysis) and/or a correlation coefficient. A regression analysis refers to a statistical technique for estimating a relationship among variables (e.g., counts and GC content). In some embodiments a regression is generated according to counts and a measure of GC content for each portion of multiple portions of a reference genome. A suitable measure of GC content can be used, non-limiting examples of which include a measure of guanine, cytosine, adenine, thymine, purine (GC), or pyrimidine (AT or ATU) content, melting temperature ($T_m$) (e.g., denaturation temperature, annealing temperature, hybridization temperature), a measure of free energy, the like or combinations thereof. A measure of guanine (G), cytosine (C), adenine (A), thymine (T), purine (GC), or pyrimidine (AT or ATU) content can be expressed as a ratio or a percentage. In some embodiments any suitable ratio or percentage is used, non-limiting examples of which include GC/AT, GC/total nucleotide, GC/A, GC/T, AT/total nucleotide, AT/GC, AT/G, AT/C, G/A, C/A, G/T, G/A, G/AT, C/T, the like or combinations thereof. In some embodiments a measure of GC content is a ratio or percentage of GC to total nucleotide content. In some embodiments a measure of GC content is a ratio or percentage of GC to total nucleotide content for sequence reads mapped to a portion of reference genome. In certain embodiments the GC content is determined according to and/or from sequence reads mapped to each portion of a reference genome and the sequence reads are obtained from a sample. In some embodiments a measure of GC content is not determined according to and/or from sequence reads. In certain embodiments, a measure of GC content is determined for one or more samples obtained from one or more subjects.

In some embodiments generating a regression comprises generating a regression analysis or a correlation analysis. A suitable regression can be used, non-limiting examples of which include a regression analysis, (e.g., a linear regression analysis), a goodness of fit analysis, a Pearson's correlation analysis, a rank correlation, a fraction of variance unexplained, Nash-Sutcliffe model efficiency analysis, regression model validation, proportional reduction in loss, root mean square deviation, the like or a combination thereof. In some embodiments a regression line is generated. In certain embodiments generating a regression comprises generating a linear regression. In certain embodiments generating a regression comprises generating a non-linear regression (e.g., an LOESS regression, an LOWESS regression).

In some embodiments a regression determines the presence or absence of a correlation (e.g., a linear correlation), for example between counts and a measure of GC content. In some embodiments a regression (e.g., a linear regression) is generated and a correlation coefficient is determined. In some embodiments a suitable correlation coefficient is determined, non-limiting examples of which include a coefficient of determination, an $R^2$ value, a Pearson's correlation coefficient, or the like.

In some embodiments goodness of fit is determined for a regression (e.g., a regression analysis, a linear regression). Goodness of fit sometimes is determined by visual or mathematical analysis. An assessment sometimes includes determining whether the goodness of fit is greater for a non-linear regression or for a linear regression. In some embodiments a correlation coefficient is a measure of a goodness of fit. In some embodiments an assessment of a goodness of fit for a regression is determined according to a correlation coefficient and/or a correlation coefficient cutoff value. In some embodiments an assessment of a goodness of fit comprises comparing a correlation coefficient to a correlation coefficient cutoff value. In some embodiments an assessment of a goodness of fit for a regression is indicative of a linear regression. For example, in certain embodiments, a goodness of fit is greater for a linear regression than for a non-linear regression and the assessment of the goodness of fit is indicative of a linear regression. In some embodiments an assessment is indicative of a linear regression and a linear regression is used to normalized the counts. In some embodiments an assessment of a goodness of fit for a regression is indicative of a non-linear regression. For example, in certain embodiments, a goodness of fit is greater for a non-linear regression than for a linear regression and the assessment of the goodness of fit is indicative of a non-linear regression. In some embodiments an assessment is indicative of a non-linear regression and a non-linear regression is used to normalized the counts.

In some embodiments an assessment of a goodness of fit is indicative of a linear regression when a correlation coefficient is equal to or greater than a correlation coefficient cutoff. In some embodiments an assessment of a goodness of fit is indicative of a non-linear regression when a correlation coefficient is less than a correlation coefficient cutoff. In some embodiments a correlation coefficient cutoff is pre-determined. In some embodiments a correlation coefficient cutoff is about 0.5 or greater, about 0.55 or greater, about 0.6 or greater, about 0.65 or greater, about 0.7 or greater, about 0.75 or greater, about 0.8 or greater or about 0.85 or greater.

In some embodiments a specific type of regression is selected (e.g., a linear or non-linear regression) and, after the regression is generated, counts are normalized by subtracting the regression from the counts. In some embodiments subtracting a regression from the counts provides normalized counts with reduced bias (e.g., GC bias). In some embodiments a linear regression is subtracted from the counts. In some embodiments a non-linear regression (e.g., a LOESS, GC-LOESS, LOWESS regression) is subtracted from the counts. Any suitable method can be used to subtract a regression line from the counts. For example, if counts x are derived from portion *i* (e.g., a portion *i*) comprising a GC content of 0.5 and a regression line determines counts y at a GC content of 0.5, then x-y = normalized counts for portion *i*. In some embodiments counts are normalized prior to and/or after subtracting a regression. In some embodiments, counts normalized by a hybrid normalization approach are used to generate levels, Z-scores, levels and/or profiles of a genome or a part thereof. In certain embodiments, counts normalized by a hybrid normalization approach are analyzed by methods described herein to determine the presence or absence of a genetic variation (e.g., copy number alteration).

In some embodiments a hybrid normalization method comprises filtering or weighting one or more portions before or after normalization. A suitable method of filtering portions, including methods of filtering portions (e.g., portions of a reference genome) described herein can be used. In some embodiments, portions (e.g., portions of a reference genome) are filtered prior to applying a hybrid normalization method. In some embodiments, only counts of sequencing reads mapped to selected portions (e.g., portions selected according to count variability) are normalized by a hybrid normalization. In some embodiments counts of sequencing reads mapped to filtered portions of a reference genome (e.g., portions filtered according to count variability) are removed prior to utilizing a hybrid normalization method. In some embodiments a hybrid normalization method comprises selecting or filtering portions (e.g., portions of a reference genome) according to a suitable method (e.g., a method described herein). In some embodiments a hybrid normalization method comprises selecting or filtering portions (e.g., portions of a reference genome) according to an uncertainty value for counts mapped to each of the portions for multiple test samples. In some embodiments a hybrid normalization method comprises selecting or filtering portions (e.g., portions of a reference genome) according to count variability. In some embodiments a hybrid normalization method comprises selecting or filtering portions (e.g., portions of a reference genome) according to GC content, repetitive elements, repetitive sequences, introns, exons, the like or a combination thereof.

*Profiles*

In some embodiments, a processing step comprises generating one or more profiles (e.g., profile plot) from various aspects of a data set or derivation thereof (e.g., product of one or more mathematical and/or statistical data processing steps known in the art and/or described herein). The term "profile" as used herein refers to a product of a mathematical and/or statistical manipulation of data that can facilitate identification of patterns and/or correlations in large quantities of data. A "profile" often includes values resulting from one or more manipulations of data or data sets, based on one or more criteria. A profile often includes multiple data points. Any suitable number of data points may be included in a profile depending on the nature and/or complexity of a data set. In certain embodiments, profiles may include 2 or more data points, 3 or more data points, 5 or more data points, 10 or more data points, 24 or more data points, 25 or more data points, 50 or more data points, 100 or more data points, 500 or more data points, 1000 or more data points, 5000 or more data points, 10,000 or more data points, or 100,000 or more data points.

In some embodiments, a profile is representative of the entirety of a data set, and in certain embodiments, a profile is representative of a part or subset of a data set. That is, a profile sometimes includes or is generated from data points representative of data that has not been filtered to remove any data, and sometimes a profile includes or is generated from data points representative of data that has been filtered to remove unwanted data. In some embodiments, a data point in a profile represents the results of data manipulation for a portion. In certain embodiments, a data point in a profile includes results of data manipulation for groups of portions. In some embodiments, groups of portions may be adjacent to one another, and in certain embodiments, groups of portions may be from different parts of a chromosome or genome.

Data points in a profile derived from a data set can be representative of any suitable data categorization. Non-limiting examples of categories into which data can be grouped to generate profile data points include: portions based on size, portions based on sequence features (e.g., GC content, AT content, position on a chromosome (e.g., short arm, long arm, centromere, telomere), and the like), levels of expression, chromosome, the like or combinations thereof. In some embodiments, a profile may be generated from data points obtained from another profile (e.g., normalized data profile renormalized to a different normalizing value to generate a renormalized data profile). In certain embodiments, a profile generated from data points obtained from another profile reduces the number of data points and/or complexity of the data set. Reducing the number of data points and/or complexity of a data set often facilitates interpretation of data and/or facilitates providing an outcome.

A profile (e.g., a genomic profile, a chromosome profile, a profile of a part of a chromosome) often is a collection of normalized or non-normalized counts for two or more portions. A profile often includes at least one level, and often comprises two or more levels (e.g., a profile often has multiple levels). A level generally is for a set of portions having about the same counts or normalized counts. Levels are described in greater detail herein. In certain embodiments, a profile comprises one or more portions, which portions can be weighted, removed, filtered, normalized, adjusted, averaged, derived as a mean, added, subtracted, processed or transformed by any combination thereof. A profile often comprises normalized counts mapped to portions defining two or more levels, where the counts are further normalized according to one of the levels by a suitable method. Often counts of a profile (e.g., a profile level) are associated with an uncertainty value.

A profile comprising one or more levels is sometimes padded (e.g., hole padding). Padding (e.g., hole padding) refers to a process of identifying and adjusting levels in a profile that are due to copy number alterations (e.g., maternal microduplications or microdeletions). In some embodiments, levels are padded that are due to microduplications or microdeletions in a fetus. Microduplications or microdeletions in a profile can, in some embodiments, artificially raise or lower the overall level of a profile (e.g., a profile of a chromosome) leading to false positive or false negative determinations of a chromosome aneuploidy (e.g., a trisomy). In some embodiments, levels in a profile that are due to microduplications and/or deletions are identified and adjusted (e.g., padded and/or removed) by a process sometimes referred to as padding or hole padding.

A profile comprising one or more levels can include a first level and a second level. In some embodiments a first level is different (e.g., significantly different) than a second level. In some embodiments a first level comprises a first set of portions, a second level comprises a second set of portions and the first set of portions is not a subset of the second set of portions. In certain embodiments, a first set of portions is different than a second set of portions from which a first and second level are determined. In some embodiments a profile can have multiple first levels that are different (e.g., significantly different, e.g., have a significantly different value) than a second level within the profile. In some embodiments a profile comprises one or more first levels that are significantly different than a second level within the profile and one or more of the first levels are adjusted. In some embodiments a first level within a profile is removed from the profile or adjusted (e.g., padded). A profile can comprise multiple levels that include one or more first levels significantly different than one or more second levels and often the majority of levels in a profile are second levels, which second levels are about equal to one another. In some embodiments greater than 50%, greater than 60%, greater than 70%, greater than 80%, greater than 90% or greater than 95% of the levels in a profile are second levels.

A profile sometimes is displayed as a plot. For example, one or more levels representing counts (e.g., normalized counts) of portions can be plotted and visualized. Non-limiting examples of profile plots that can be generated include raw count (e.g., raw count profile or raw profile), normalized count, portion-weighted, z-score, p-value, area ratio versus fitted ploidy, median level versus ratio between fitted and measured minority species fraction, principal components, the like, or combinations thereof. Profile plots allow visualization of the manipulated data, in some embodiments. In certain embodiments, a profile plot can be utilized to provide an outcome (e.g., area ratio versus fitted ploidy, median level versus ratio between fitted and measured minority species fraction, principal components). The terms "raw count profile plot" or "raw profile plot" as used herein refer to a plot of counts in each portion in a region normalized to total counts in a region (e.g., genome, portion, chromosome, chromosome portions of a reference genome or a part of a chromosome). In some embodiments, a profile can be generated using a static window process, and in certain embodiments, a profile can be generated using a sliding window process.

A profile generated for a test subject sometimes is compared to a profile generated for one or more reference subjects, to facilitate interpretation of mathematical and/or statistical manipulations of a data set and/or to provide an outcome. In some embodiments, a profile is generated based on one or more starting assumptions, e.g., assumptions described herein. In certain embodiments, a test profile often centers around a predetermined value representative of the absence of a copy number alteration, and often deviates from a predetermined value in areas corresponding to the genomic location in which the copy number alteration is located in the test subject, if the test subject possessed the copy number alteration. In test subjects at risk for, or suffering from a medical condition associated with a copy number alteration, the numerical value for a selected portion is expected to vary significantly from the predetermined value for non-affected genomic locations. Depending on starting assumptions (e.g., fixed ploidy or optimized ploidy, fixed fetal fraction or optimized fetal fraction, or combinations thereof) the predetermined threshold or cutoff value or threshold range of values indicative of the presence or absence of a copy number alteration can vary while still providing an outcome useful for determining the presence or absence of a copy number alteration. In some embodiments, a profile is indicative of and/or representative of a phenotype.

In some embodiments, the use of one or more reference samples that are substantially free of a copy number alteration in question can be used to generate a reference count profile (e.g., a reference median count profile), which may result in a predetermined value representative of the absence of the copy number alteration, and often deviates from a predetermined value in areas corresponding to the genomic location in which the copy number alteration is located in the test subject, if the test subject possessed the copy number alteration. In test subjects at risk for, or suffering from a medical condition associated with a copy number alteration, the numerical value for the selected portion or sections is expected to vary significantly from the predetermined value for non-affected genomic locations. In certain embodiments, the use of one or more reference samples known to carry the copy number alteration in question can be used to generate a reference count profile (a reference median count profile), which may result in a predetermined value representative of the presence of the copy number alteration, and often deviates from a predetermined value in areas corresponding to the genomic location in which a test subject does not carry the copy number alteration. In test subjects not at risk for, or suffering from a medical condition associated with a copy number alteration, the numerical value for the selected portion or sections is expected to vary significantly from the predetermined value for affected genomic locations.

By way of a non-limiting example, normalized sample and/or reference count profiles can be obtained from raw sequence read data by (a) calculating reference median counts for selected chromosomes, portions or parts thereof from a set of references known not to carry a copy number alteration, (b) removal of uninformative portions from the reference sample raw counts (e.g., filtering); (c) normalizing the reference counts for all remaining portions of a reference genome to the total residual number of counts (e.g., sum of remaining counts after removal of uninformative portions of a reference genome) for the reference sample selected chromosome or selected genomic location, thereby generating a normalized reference subject profile; (d) removing the corresponding portions from the test subject sample; and (e) normalizing the remaining test subject counts for one or more selected genomic locations to the sum of the residual reference median counts for the chromosome or chromosomes containing the selected genomic locations, thereby generating a normalized test subject profile. In certain embodiments, an additional normalizing step with respect to the entire genome, reduced by the filtered portions in (b), can be included between (c) and (d).

In some embodiments a read density profile is determined. In some embodiments a read density profile comprises at least one read density, and often comprises two or more read densities (e.g., a read density profile often comprises multiple read densities). In some embodiments, a read density profile comprises a suitable quantitative value (e.g., a mean, a median, a Z-score, or the like). A read density profile often comprises values resulting from one or more read densities. A read density profile sometimes comprises values resulting from one or more manipulations of read densities based on one or more adjustments (e.g., normalizations). In some embodiments a read density profile comprises unmanipulated read densities. In some embodiments, one or more read density profiles are generated from various aspects of a data set comprising read densities, or a derivation thereof (e.g., product of one or more mathematical and/or statistical data processing steps known in the art and/or described herein). In certain embodiments, a read density profile comprises normalized read densities. In some embodiments a read density profile comprises adjusted read densities. In certain embodiments a read density profile comprises raw read densities (e.g., unmanipulated, not adjusted or normalized), normalized read densities, weighted read densities, read densities of filtered portions, z-scores of read densities, p-values of read densities, integral values of read densities (e.g., area under the curve), average, mean or median read densities, principal components, the like, or combinations thereof. Often read densities of a read density profile and/or a read density profile is associated with a measure of uncertainty (e.g., a MAD). In certain embodiments, a read density profile comprises a distribution of median read densities. In some embodiments a read density profile comprises a relationship (e.g., a fitted relationship, a regression, or the like) of a plurality of read densities. For example, sometimes a read density profile comprises a relationship between read densities (e.g., read densities value) and genomic locations (e.g., portions, portion locations). In some embodiments, a read density profile is generated using a static window process, and in certain embodiments, a read density profile is generated using a sliding window process. In some embodiments a read density profile is sometimes printed and/or displayed (e.g., displayed as a visual representation, e.g., a plot or a graph).

In some embodiments, a read density profile corresponds to a set of portions (e.g., a set of portions of a reference genome, a set of portions of a chromosome or a subset of portions of a part of a chromosome). In some embodiments a read density profile comprises read densities and/or counts associated with a collection (e.g., a set, a subset) of portions. In some embodiments, a read density profile is determined for read densities of portions that are contiguous. In some embodiments, contiguous portions comprise gaps comprising regions of a reference sequence and/or sequence reads that are not included in a density profile (e.g., portions removed by a filtering). Sometimes portions (e.g., a set of portions) that are contiguous represent neighboring regions of a genome or neighboring regions of a chromosome or gene. For example, two or more contiguous portions, when aligned by merging the portions end to end, can represent a sequence assembly of a DNA sequence longer than each portion. For example two or more contiguous portions can represent an intact genome, chromosome, gene, intron, exon or part thereof. Sometimes a read density profile is determined from a collection (e.g., a set, a subset) of contiguous portions and/or non-contiguous portions. In some cases, a read density profile comprises one or more portions, which portions can be weighted, removed, filtered, normalized, adjusted, averaged, derived as a mean, added, subtracted, processed or transformed by any combination thereof.

A read density profile is often determined for a sample and/or a reference (e.g., a reference sample). A read density profile is sometimes generated for an entire genome, one or more chromosomes, or for a part of a genome or a chromosome. In some embodiments, one or more read density profiles are determined for a genome or part thereof. In some embodiments, a read density profile is representative of the entirety of a set of read densities of a sample, and in certain embodiments, a read density profile is representative of a part or subset of read densities of a sample. That is, sometimes a read density profile comprises or is generated from read densities representative of data that has not been filtered to remove any data, and sometimes a read density profile includes or is generated from data points representative of data that has been filtered to remove unwanted data.

In some embodiments a read density profile is determined for a reference (e.g., a reference sample, a training set). A read density profile for a reference is sometimes referred to herein as a reference profile. In some embodiments a reference profile comprises a read densities obtained from one or more references (e.g., reference sequences, reference samples). In some embodiments a reference profile comprises read densities determined for one or more (e.g., a set of) known euploid samples. In some embodiments a reference profile comprises read densities of filtered portions. In some embodiments a reference profile comprises read densities adjusted according to the one or more principal components.

*Performing a comparison*

In some embodiments, a processing step comprises preforming a comparison (e.g., comparing a test profile to a reference profile). Two or more data sets, two or more relationships and/or two or more profiles can be compared by a suitable method. Non-limiting examples of statistical methods suitable for comparing data sets, relationships and/or profiles include Behrens-Fisher approach, bootstrapping, Fisher's method for combining independent tests of significance, Neyman-Pearson testing, confirmatory data analysis, exploratory data analysis, exact test, F-test, Z-test, T-test, calculating and/or comparing a measure of uncertainty, a null hypothesis, counternulls and the like, a chi-square test, omnibus test, calculating and/or comparing level of significance (e.g., statistical significance), a meta analysis, a multivariate analysis, a regression, simple linear regression, robust linear regression, the like or combinations of the foregoing. In certain embodiments comparing two or more data sets, relationships and/or profiles comprises determining and/or comparing a measure of uncertainty. A "measure of uncertainty" as used herein refers to a measure of significance (e.g., statistical significance), a measure of error, a measure of variance, a measure of confidence, the like or a combination thereof. A measure of uncertainty can be a value (e.g., a threshold) or a range of values (e.g., an interval, a confidence interval, a Bayesian confidence interval, a threshold range). Non-limiting examples of a measure of uncertainty include p-values, a suitable measure of deviation (e.g., standard deviation, sigma, absolute deviation, mean absolute deviation, the like), a suitable measure of error (e.g., standard error, mean squared error, root mean squared error, the like), a suitable measure of variance, a suitable standard score (e.g., standard deviations, cumulative percentages, percentile equivalents, Z-scores, T-scores, R-scores, standard nine (stanine), percent in stanine, the like), the like or combinations thereof. In some embodiments determining the level of significance comprises determining a measure of uncertainty (e.g., a p-value). In certain embodiments, two or more data sets, relationships and/or profiles can be analyzed and/or compared by utilizing multiple (e.g., 2 or more) statistical methods (e.g., least squares regression, principal component analysis, linear discriminant analysis, quadratic discriminant analysis, bagging, neural networks, support vector machine models, random forests, classification tree models, K-nearest neighbors, logistic regression and/or loss smoothing) and/or any suitable mathematical and/or statistical manipulations (e.g., referred to herein as manipulations).

In some embodiments, a processing step comprises a comparison of two or more profiles (e.g., two or more read density profiles). Comparing profiles may comprise comparing profiles generated for a selected region of a genome. For example, a test profile may be compared to a reference profile where the test and reference profiles were determined for a region of a genome (e.g., a reference genome) that is substantially the same region. Comparing profiles sometimes comprises comparing two or more subsets of portions of a profile (e.g., a read density profile). A subset of portions of a profile may represent a region of a genome (e.g., a chromosome, or region thereof). A profile (e.g., a read density profile) can comprise any amount of subsets of portions. Sometimes a profile (e.g., a read density profile) comprises two or more, three or more, four or more, or five or more subsets. In certain embodiments, a profile (e.g., a read density profile) comprises two subsets of portions where each portion represents regions of a reference genome that are adjacent. In some embodiments, a test profile can be compared to a reference profile where the test profile and reference profile both comprise a first subset of portions and a second subset of portions where the first and second subsets represent different regions of a genome. Some subsets of portions of a profile may comprise copy number alterations and other subsets of portions are sometimes substantially free of copy number alterations. Sometimes all subsets of portions of a profile (e.g., a test profile) are substantially free of a copy number alteration. Sometimes all subsets of portions of a profile (e.g., a test profile) comprise a copy number alteration. In some embodiments a test profile can comprise a first subset of portions that comprise a copy number alteration and a second subset of portions that are substantially free of a copy number alteration.

In certain embodiments, comparing two or more profiles comprises determining and/or comparing a measure of uncertainty for two or more profiles. Profiles (e.g., read density profiles) and/or associated measures of uncertainty are sometimes compared to facilitate interpretation of mathematical and/or statistical manipulations of a data set and/or to provide an outcome. A profile (e.g., a read density profile) generated for a test subject sometimes is compared to a profile (e.g., a read density profile) generated for one or more references (e.g., reference samples, reference subjects, and the like). In some embodiments, an outcome is provided by comparing a profile (e.g., a read density profile) from a test subject to a profile (e.g., a read density profile) from a reference for a chromosome, portions or parts thereof, where a reference profile is obtained from a set of reference subjects known not to possess a copy number alteration (e.g., a reference). In some embodiments an outcome is provided by comparing a profile (e.g., a read density profile) from a test subject to a profile (e.g., a read density profile) from a reference for a chromosome, portions or parts thereof, where a reference profile is obtained from a set of reference subjects known to possess a specific copy number alteration (e.g., a chromosome aneuploidy, a microduplication, a microdeletion).

In certain embodiments, a profile (e.g., a read density profile) of a test subject is compared to a predetermined value representative of the absence of a copy number alteration, and sometimes deviates from a predetermined value at one or more genomic locations (e.g., portions) corresponding to a genomic location in which a copy number alteration is located. For example, in test subjects (e.g., subjects at risk for, or suffering from a medical condition associated with a copy number alteration), profiles are expected to differ significantly from profiles of a reference (e.g., a reference sequence, reference subject, reference set) for selected portions when a test subject comprises a copy number alteration in question. Profiles (e.g., read density profiles) of a test subject are often substantially the same as profiles (e.g., read density profiles) of a reference (e.g., a reference sequence, reference subject, reference set) for selected portions when a test subject does not comprise a copy number alteration in question. Profiles (e.g., read density profiles) may be compared to a predetermined threshold and/or threshold range. The term "threshold" as used herein refers to any number that is calculated using a qualifying data set and serves as a limit of diagnosis of a copy number alteration (e.g., an aneuploidy, a microduplication, a microdeletion, and the like). In certain embodiments a threshold is exceeded by results obtained by methods described herein and a subject is diagnosed with a copy number alteration. In some embodiments, a threshold value or range of values may be calculated by mathematically and/or statistically manipulating sequence read data (e.g., from a reference and/or subject). A predetermined threshold or threshold range of values indicative of the presence or absence of a copy number alteration can vary while still providing an outcome useful for determining the presence or absence of a copy number alteration. In certain embodiments, a profile (e.g., a read density profile) comprising normalized read densities and/or normalized counts is generated to facilitate classification and/or providing an outcome. An outcome can be provided based on a plot of a profile (e.g., a read density profile) comprising normalized counts (e.g., using a plot of such a read density profile).

*Decision Analysis*

In some embodiments, a determination of an outcome (e.g., making a call) or a determination of the presence or absence of a copy number alteration (e.g., chromosome aneuploidy, microduplication, microdeletion) is made according to a decision analysis. Certain decision analysis features are described in International Patent Application Publication No. WO2014/190286, which is incorporated by reference herein. For example, a decision analysis sometimes comprises applying one or more methods that produce one or more results, an evaluation of the results, and a series of decisions based on the results, evaluations and/or the possible consequences of the decisions and terminating at some juncture of the process where a final decision is made. In some embodiments a decision analysis is a decision tree. A decision analysis, in some embodiments, comprises coordinated use of one or more processes (e.g., process steps, e.g., algorithms). A decision analysis can be performed by a person, a system, an apparatus, software (e.g., a module), a computer, a processor (e.g., a microprocessor), the like or a combination thereof. In some embodiments a decision analysis comprises a method of determining the presence or absence of a copy number alteration (e.g., chromosome aneuploidy, microduplication or microdeletion) with reduced false negative and reduced false positive determinations, compared to an instance in which no decision analysis is utilized (e.g., a determination is made directly from normalized counts). In some embodiments a decision analysis comprises determining the presence or absence of a condition associated with one or more copy number alterations.

In some embodiments a decision analysis comprises generating a profile for a genome or a region of a genome (e.g., a chromosome or part thereof). A profile can be generated by any suitable method, known or described herein. In some embodiments, a decision analysis comprises a segmenting process. Segmenting can modify and/or transform a profile thereby providing one or more decomposition renderings of a profile. A profile subjected to a segmenting process often is a profile of normalized counts mapped to portions in a reference genome or part thereof. As addressed herein, raw counts mapped to the portions can be normalized by one or more suitable normalization processes (e.g., LOESS, GC-LOESS, principal component normalization, or combination thereof) to generate a profile that is segmented as part of a decision analysis. A decomposition rendering of a profile is often a transformation of a profile. A decomposition rendering of a profile is sometimes a transformation of a profile into a representation of a genome, chromosome or part thereof.

In certain embodiments, a segmenting process utilized for the segmenting locates and identifies one or more levels within a profile that are different (e.g., substantially or significantly different) than one or more other levels within a profile. A level identified in a profile according to a segmenting process that is different than another level in the profile, and has edges that are different than another level in the profile, is referred to herein as a level for a discrete segment.

A segmenting process can generate, from a profile of normalized counts or levels, a decomposition rendering in which one or more discrete segments can be identified. A discrete segment generally covers fewer portions than what is segmented (e.g., chromosome, chromosomes, autosomes).

In some embodiments, segmenting locates and identifies edges of discrete segments within a profile. In certain embodiments, one or both edges of one or more discrete segments are identified. For example, a segmentation process can identify the location (e.g., genomic coordinates, e.g., portion location) of the right and/or the left edges of a discrete segment in a profile. A discrete segment often comprises two edges. For example, a discrete segment can include a left edge and a right edge. In some embodiments, depending upon the representation or view, a left edge can be a 5'-edge and a right edge can be a 3'-edge of a nucleic acid segment in a profile. In some embodiments, a left edge can be a 3'-edge and a right edge can be a 5'-edge of a nucleic acid segment in a profile. Often the edges of a profile are known prior to segmentation and therefore, in some embodiments, the edges of a profile determine which edge of a level is a 5'-edge and which edge is 3'-edge. In some embodiments one or both edges of a profile and/or discrete segment is an edge of a chromosome.

In some embodiments, the edges of a discrete segment are determined according to a decomposition rendering generated for a reference sample (e.g., a reference profile). In some embodiments a null edge height distribution is determined according to a decomposition rendering of a reference profile (e.g., a profile of a chromosome or part thereof). In certain embodiments, the edges of a discrete segment in a profile are identified when the level of the discrete segment is outside a null edge height distribution. In some embodiments, the edges of a discrete segment in a profile are identified according a Z-score calculated according to a decomposition rendering for a reference profile.

In some instances, segmenting generates two or more discrete segments (e.g., two or more fragmented levels, two or more fragmented segments) in a profile. In some embodiments, a decomposition rendering derived from a segmenting process is over-segmented or fragmented and comprises multiple discrete segments. Sometimes discrete segments generated by segmenting are substantially different and sometimes discrete segments generated by segmenting are substantially similar. Substantially similar discrete segments (e.g., substantially similar levels) often refers to two or more adjacent discrete segments in a segmented profile each having a level that differs by less than a predetermined level of uncertainty. In some embodiments, substantially similar discrete segments are adjacent to each other and are not separated by an intervening segment. In some embodiments, substantially similar discrete segments are separated by one or more smaller segments. In some embodiments substantially similar discrete segments are separated by about 1 to about 20, about 1 to about 15, about 1 to about 10 or about 1 to about 5 portions where one or more of the intervening portions have a level significantly different than the level of each of the substantially similar discrete segments. In some embodiments, the level of substantially similar discrete segments differs by less than about 3 times, less than about 2 times, less than about 1 time or less than about 0.5 times a level of uncertainty. Substantially similar discrete segments, in some embodiments, comprise a median level that differs by less than 3 MAD (e.g., less than 3 sigma), less than 2 MAD, less than 1 MAD or less than about 0.5 MAD, where a MAD is calculated from a median level of each of the segments. Substantially different discrete segments, in some embodiments, are not adjacent or are separated by 10 or more, 15 or more or 20 or more portions. Substantially different discrete segments generally have substantially different levels. In certain embodiments, substantially different discrete segments comprises levels that differ by more than about 2.5 times, more than about 3 times, more than about 4 times, more than about 5 times, more than about 6 times a level of uncertainty. Substantially different discrete segments, in some embodiments, comprise a median level that differs by more than 2.5 MAD (e.g., more than 2.5 sigma), more than 3 MAD, more than 4 MAD, more than about 5 MAD or more than about 6 MAD, where a MAD is calculated from a median level of each of the discrete segments.

In some embodiments, a segmentation process comprises determining (e.g., calculating) a level (e.g., a quantitative value, e.g., a mean or median level), a level of uncertainty (e.g., an uncertainty value), Z-score, Z-value, p-value, the like or combinations thereof for one or more discrete segments in a profile or part thereof. In some embodiments a level (e.g., a quantitative value, e.g., a mean or median level), a level of uncertainty (e.g., an uncertainty value), Z-score, Z-value, p-value, the like or combinations thereof are determined (e.g., calculated) for a discrete segment.

Segmenting can be performed, in full or in part, by one or more decomposition generating processes. A decomposition generating process may provide, for example, a decomposition rendering of a profile. Any decomposition generating process described herein or known in the art may be used. Non-limiting examples of a decomposition generating process include circular binary segmentation (CBS) (see e.g., Olshen et al. (2004) Biostatistics 5(4):557-72; Venkatraman, ES, Olshen, AB (2007) Bioinformatics 23(6):657-63); Haar wavelet segmentation (see e.g., Haar, Alfred (1910) Mathematische Annalen 69(3):331-371); maximal overlap discrete wavelet transform (MODWT) (see e.g., Hsu et al. (2005) Biostatistics 6 (2):211-226); stationary wavelet (SWT) (see e.g., Y. Wang and S. Wang (2007) International Journal of Bioinformatics Research and Applications 3(2):206-222); dual-tree complex wavelet transform (DTCWT) (see e.g., Nguyen et al. (2007) Proceedings of the 7th IEEE International Conference, Boston MA, on October 14-17, 2007, pages 137-144); maximum entropy segmentation, convolution with edge detection kernel, Jensen Shannon Divergence, Kullback-Leibler divergence, Binary Recursive Segmentation, a Fourier transform, the like or combinations thereof.

In some embodiments, segmenting is accomplished by a process that comprises one process or multiple sub-processes, non-limiting examples of which include a decomposition generating process, thresholding, leveling, smoothing, polishing, the like or combination thereof. Thresholding, leveling, smoothing, polishing and the like can be performed in conjunction with a decomposition generating process, for example.

In some embodiments, a decision analysis comprises identifying a candidate segment in a decomposition rendering. A candidate segment is determined as being the most significant discrete segment in a decomposition rendering. A candidate segment may be the most significant in terms of the number of portions covered by the segment and/or in terms of the absolute value of the level of normalized counts for the segment. A candidate segment sometimes is larger and sometimes substantially larger than other discrete segments in a decomposition rendering. A candidate segment can be identified by a suitable method. In some embodiments, a candidate segment is identified by an area under the curve (AUC) analysis. In certain embodiments, where a first discrete segment has a level and/or covers a number of portions substantially larger than for another discrete segment in a decomposition rendering, the first segment comprises a larger AUC. Where a level is analyzed for AUC, an absolute value of a level often is utilized (e.g., a level corresponding to normalized counts can have a negative value for a deletion and a positive value for a duplication). In certain embodiments, an AUC is determined as an absolute value of a calculated AUC (e.g., a resulting positive value). In certain embodiments, a candidate segment, once identified (e.g., by an AUC analysis or by a suitable method) and optionally after it is validated, is selected for a z-score calculation, or the like, to determine if the candidate segment represents a genetic variation (e.g., an aneuploidy, microdeletion or microduplication).

In some embodiments, a decision analysis comprises a comparison. In some embodiments, a comparison comprises comparing at least two decomposition renderings. In some embodiments, a comparison comprises comparing at least two candidate segments. In certain embodiments, each of the at least two candidate segments is from a different decomposition rendering. For example, a first candidate segment can be from a first decomposition rendering and a second candidate segment can be from a second decomposition rendering. In some embodiments, a comparison comprises determining if two decomposition renderings are substantially the same or different. In some embodiments, a comparison comprises determining if two candidate segments are substantially the same or different. Two candidate segments can be determined as substantially the same or different by a suitable comparison method, non-limiting examples of which include by visual inspection, by comparing levels or Z-scores of the two candidate segments, by comparing the edges of the two candidate segments, by overlaying either the two candidate segments or their corresponding decomposition renderings, the like or combinations thereof.

*Outcome*

Methods described herein can provide a determination of the presence or absence of a genetic variation for a sample (e.g., fetal aneuploidy), thereby providing an outcome (e.g., thereby providing an outcome determinative of the presence or absence of a genetic variation). Methods herein sometimes provide a classification as to the presence or absence of a genetic variation for a sample (i.e., a classification outcome). A genetic variation often includes a gain, a loss and/or alteration (e.g., duplication, deletion, fusion, insertion, mutation, reorganization, substitution or aberrant methylation) of genetic information (e.g., chromosomes, parts of chromosomes, polymorphic regions, translocated regions, altered nucleotide sequence, the like or combinations of the foregoing) that results in a detectable change in the genome or genetic information of a test subject with respect to a reference. Presence or absence of a genetic variation can be determined by transforming, analyzing and/or manipulating sequence reads that have been mapped to portions (e.g., counts, counts of genomic portions of a reference genome). In certain embodiments, an outcome is determined according to normalized counts, read densities, read density profiles, and the like.

In some embodiments, reference samples are euploid for a selected part of a genome (e.g., region), and a measure of uncertainty between a test profile and a reference profile is assessed for the selected region. In some embodiments, a determination of the presence or absence of a genetic variation is according to the number of deviations (e.g., measures of deviations, MAD) between a test profile and a reference profile for a selected region of a genome (e.g., a chromosome, or part thereof). In some embodiments, the presence of a genetic variation is determined when the number of deviations between a test profile and a reference profile is greater than about 1, greater than about 1.5, greater than about 2, greater than about 2.5, greater than about 2.6,greater than about 2.7, greater than about 2.8, greater than about 2.9,greater than about 3,greater than about 3.1, greater than about 3.2, greater than about 3.3, greater than about 3.4, greater than about 3.5, greater than about 4, greater than about 5, or greater than about 6. For example, sometimes a test profile and a reference profile differ by more than 3 measures of deviation (e.g., 3 sigma, 3 MAD) and the presence of a genetic variation is determined. A deviation of greater than three between a test profile and a reference profile often is indicative of a non-euploid test subject (e.g., presence of a genetic variation) for a selected region. A test profile significantly greater than a reference profile for a selected region, which reference is euploid for the selected region, sometimes is determinative of a trisomy. Test profiles significantly below a reference profile, which reference profile is indicative of euploidy, sometimes are determinative of a monosomy.

In some embodiments, the absence of a genetic variation is determined when the number of deviations between a test profile and reference profile for a selected region of a genome is less than about 3.5, less than about 3.4, less than about 3.3, less than about 3.2, less than about 3.1,less than about 3.0,less than about 2.9,less than about 2.8,less than about 2.7,less than about 2.6, less than about 2.5, less than about 2.0, less than about 1.5, or less than about 1.0. For example, sometimes a test profile differs from a reference profile by less than 3 measures of deviation (e.g., 3 sigma, 3 MAD) and the absence of a genetic variation is determined. In some embodiments, deviation of less than three between test profiles and reference profiles (e.g., 3-sigma for standard deviation) often is indicative of a region that is euploid (e.g., absence of a genetic variation). A measure of deviation between test profiles for a test sample and reference profiles for one or more reference subjects can be plotted and visualized (e.g., z-score plot).

In some embodiments, a determination of the presence or absence of a genetic variation is determined according to a call zone. In certain embodiments, a call is made (e.g., a call determining the presence or absence of a genetic variation) when a value (e.g., a profile, a read density profile and/or a measure of uncertainty) or collection of values falls within a pre-defined range (e.g., a zone, a call zone). In some embodiments, a call zone is defined according to a collection of values (e.g., profiles, read density profiles and/or measures of uncertainty) that are obtained from the same patient sample. In certain embodiments, a call zone is defined according to a collection of values that are derived from the same chromosome or part thereof. In some embodiments, a call zone based on a genetic variation determination is defined according a measure of uncertainty (e.g., high level of confidence, e.g., low measure of uncertainty) and/or a quantification of a minority nucleic acid species (e.g.,fetal fraction; e.g., 2%, 3%, 4% or higher minority species fraction).

In some embodiments, a call zone is defined by a confidence level of about 99% or greater, about 99.1% or greater, about 99.2% or greater, about 99.3% or greater, about 99.4% or greater, about 99.5% or greater, about 99.6% or greater, about 99.7% or greater, about 99.8% or greater or about 99.9% or greater. In some embodiments, a call is made without using a call zone. In some embodiments, a call is made using a call zone and additional data or information. In some embodiments, a call is made based on a comparison without the use of a call zone. In some embodiments, a call is made based on visual inspection of a profile (e.g., visual inspection of read densities).

In some embodiments, a no-call zone is where a call is not made. In some embodiments, a no-call zone is defined by a value or collection of values that indicate low accuracy, high risk, high error, low level of confidence, high measure of uncertainty, the like or a combination thereof. In some embodiments, a no-call zone is defined, in part, by a minority species fraction of about 5% or less, about 4% or less, about 3% or less, about 2.5% or less, about 2.0% or less, about 1.5% or less or about 1.0% or less.

A genetic variation sometimes is associated with medical condition. An outcome determinative of a genetic variation is sometimes an outcome determinative of the presence or absence of a condition (e.g., a medical condition), disease, syndrome or abnormality, or includes, detection of a condition, disease, syndrome or abnormality. In certain embodiments, a diagnosis comprises assessment of an outcome. An outcome determinative of the presence or absence of a condition (e.g., a medical condition), disease, syndrome or abnormality by methods described herein can sometimes be independently verified by further testing (e.g., by scan (e.g., CT scan, MRI), karyotyping and/or amniocentesis). Analysis and processing of data can provide one or more outcomes. The term "outcome" as used herein can refer to a result of data processing that facilitates determining the presence or absence of a genetic variation (e.g., an aneuploidy, a copy number variation). In certain embodiments, the term "outcome" as used herein refers to a conclusion that predicts and/or determines the presence or absence of a genetic variation. In certain embodiments, the term "outcome" as used herein refers to a conclusion that predicts and/or determines a risk or probability of the presence or absence of a genetic variation in a subject.

A diagnosis sometimes comprises use of an outcome. For example, a health practitioner may analyze an outcome and provide a diagnosis based on, or based in part on, the outcome. In some embodiments, determination, detection or diagnosis of a condition, syndrome or abnormality comprises use of an outcome determinative of the presence or absence of a genetic variation. In some embodiments, an outcome based on counted mapped sequence reads or transformations thereof is determinative of the presence or absence of a genetic variation. In certain embodiments, an outcome generated utilizing one or more methods (e.g., data processing methods) described herein is determinative of the presence or absence of one or more conditions, syndromes or abnormalities. In certain embodiments, a diagnosis comprises a determination of a presence or absence of a condition, syndrome or abnormality. In certain instances, a diagnosis comprises a determination of a genetic variation as the nature and/or cause of a condition, syndrome or abnormality. In certain embodiments, an outcome is not a diagnosis. An outcome often comprises one or more numerical values generated using a processing method described herein in the context of one or more considerations of probability. A consideration of risk or probability can include, but is not limited to: a measure of uncertainty, a confidence level, sensitivity, specificity, standard deviation, coefficient of variation (CV) and/or confidence level, Z-scores, Chi values, Phi values, ploidy values, fitted minority species fraction, area ratios, median level, the like or combinations thereof. A consideration of probability can facilitate determining whether a subject is at risk of having, or has, a genetic variation, and an outcome determinative of a presence or absence of a genetic disorder often includes such a consideration.

An outcome sometimes is a phenotype. An outcome sometimes is a phenotype with an associated level of confidence (e.g., a measure of uncertainty, e.g., a fetus is positive for trisomy 21 with a confidence level of 99%, a test subject is negative for a cancer associated with a genetic variation at a confidence level of 95%). Different methods of generating outcome values sometimes can produce different types of results. Generally, there are four types of possible scores or calls that can be made based on outcome values generated using methods described herein: true positive, false positive, true negative and false negative. The terms "score," "scores," "call" and "calls" as used herein refer to calculating the probability that a particular genetic variation is present or absent in a subject/sample. The value of a score may be used to determine, for example, a variation, difference, or ratio of mapped sequence reads that may correspond to a genetic variation. For example, calculating a positive score for a selected genetic variation or portion from a data set, with respect to a reference genome can lead to an identification of the presence or absence of a genetic variation, which genetic variation sometimes is associated with a medical condition (e.g., preeclampsia, trisomy, monosomy, and the like). In some embodiments, an outcome comprises a read density, a read density profile and/or a plot (e.g., a profile plot). In those embodiments in which an outcome comprises a profile, a suitable profile or combination of profiles can be used for an outcome. Non-limiting examples of profiles that can be used for an outcome include z-score profiles, p-value profiles, chi value profiles, phi value profiles, the like, and combinations thereof.

An outcome generated for determining the presence or absence of a genetic variation sometimes includes a null result (e.g., a data point between two clusters, a numerical value with a standard deviation that encompasses values for both the presence and absence of a genetic variation, a data set with a profile plot that is not similar to profile plots for subjects having or free from the genetic variation being investigated). In some embodiments, an outcome indicative of a null result still is a determinative result, and the determination can include the need for additional information and/or a repeat of the data generation and/or analysis for determining the presence or absence of a genetic variation.

An outcome can be generated after performing one or more processing steps described herein, in some embodiments. In certain embodiments, an outcome is generated as a result of one of the processing steps described herein, and in some embodiments, an outcome can be generated after each statistical and/or mathematical manipulation of a data set is performed. An outcome pertaining to the determination of the presence or absence of a genetic variation can be expressed in a suitable form, which form comprises without limitation, a probability (e.g., odds ratio, p-value), likelihood, value in or out of a cluster, value over or under a threshold value, value within a range (e.g., a threshold range), value with a measure of variance or confidence, or risk factor, associated with the presence or absence of a genetic variation for a subject or sample. In certain embodiments, comparison between samples allows confirmation of sample identity (e.g., allows identification of repeated samples and/or samples that have been mixed up (e.g., mislabeled, combined, and the like)).

In some embodiments, an outcome comprises a value above or below a predetermined threshold or cutoff value and/or a measure of uncertainty or a confidence level associated with the value. In certain embodiments, a predetermined threshold or cutoff value is an expected level or an expected level range. An outcome also can describe an assumption used in data processing. In certain embodiments, an outcome comprises a value that falls within or outside a predetermined range of values (e.g., a threshold range) and the associated uncertainty or confidence level for that value being inside or outside the range. In some embodiments, an outcome comprises a value that is equal to a predetermined value (e.g., equal to 1, equal to zero), or is equal to a value within a predetermined value range, and its associated uncertainty or confidence level for that value being equal or within or outside a range. An outcome sometimes is graphically represented as a plot (e.g., profile plot).

As noted above, an outcome can be characterized as a true positive, true negative, false positive or false negative. The term "true positive" as used herein refers to a subject correctly diagnosed as having a genetic variation. The term "false positive" as used herein refers to a subject wrongly identified as having a genetic variation. The term "true negative" as used herein refers to a subject correctly identified as not having a genetic variation. The term "false negative" as used herein refers to a subject wrongly identified as not having a genetic variation. Two measures of performance for any given method can be calculated based on the ratios of these occurrences: (i) a sensitivity value, which generally is the fraction of predicted positives that are correctly identified as being positives; and (ii) a specificity value, which generally is the fraction of predicted negatives correctly identified as being negative.

In certain embodiments, one or more of sensitivity, specificity and/or confidence level are expressed as a percentage. In some embodiments, the percentage, independently for each variable, is greater than about 90% (e.g., about 90, 91, 92, 93, 94, 95, 96, 97, 98 or 99%, or greater than 99% (e.g., about 99.5%, or greater, about 99.9% or greater, about 99.95% or greater, about 99.99% or greater)). Coefficient of variation (CV) in some embodiments is expressed as a percentage, and sometimes the percentage is about 10% or less (e.g., about 10, 9, 8, 7, 6, 5, 4, 3, 2 or 1%, or less than 1% (e.g., about 0.5% or less, about 0.1% or less, about 0.05% or less, about 0.01% or less)). A probability (e.g., that a particular outcome is not due to chance) in certain embodiments is expressed as a Z-score, a p-value, or the results of a t-test. In some embodiments, a measured variance, confidence interval, sensitivity, specificity and the like (e.g., referred to collectively as confidence parameters) for an outcome can be generated using one or more data processing manipulations described herein. Specific examples of generating outcomes and associated confidence levels are described, for example, in International Patent Application Publication No. WO2013/052913, the entire content of which is incorporated herein by reference, including all text, tables, equations and drawings.

The term "sensitivity" as used herein refers to the number of true positives divided by the number of true positives plus the number of false negatives, where sensitivity (sens) may be within the range of $0 \leq sens \leq 1$. The term "specificity" as used herein refers to the number of true negatives divided by the number of true negatives plus the number of false positives, where sensitivity (spec) may be within the range of $0 \leq spec \leq 1$. In some embodiments, a method that has sensitivity and specificity equal to one, or 100%, or near one (e.g., between about 90% to about 99%) sometimes is selected. In some embodiments, a method having a sensitivity equaling 1, or 100% is selected, and in certain embodiments, a method having a sensitivity near 1 is selected (e.g., a sensitivity of about 90%, a sensitivity of about 91%, a sensitivity of about 92%, a sensitivity of about 93%, a sensitivity of about 94%, a sensitivity of about 95%, a sensitivity of about 96%, a sensitivity of about 97%, a sensitivity of about 98%, or a sensitivity of about 99%). In some embodiments, a method having a specificity equaling 1, or 100% is selected, and in certain embodiments, a method having a specificity near 1 is selected (e.g., a specificity of about 90%, a specificity of about 91%, a specificity of about 92%, a specificity of about 93%, a specificity of about 94%, a specificity of about 95%, a specificity of about 96%, a specificity of about 97%, a specificity of about 98%, or a specificity of about 99%).

A genetic variation or an outcome determinative of a genetic variation identified by methods described herein can be independently verified by further testing (e.g., by targeted sequencing of nucleic acid). An outcome typically is provided to a health care professional (e.g., laboratory technician or manager; physician or assistant). In certain embodiments, an outcome is provided on a suitable visual medium (e.g., a peripheral or component of a machine, e.g., a printer or display). In some embodiments, an outcome determinative of the presence or absence of a genetic variation is provided to a healthcare professional in the form of a report, and in certain embodiments the report comprises a display of an outcome value and an associated confidence parameter. Generally, an outcome can be displayed in a suitable format that facilitates determination of the presence or absence of a genetic variation and/or medical condition. Non-limiting examples of formats suitable for use for reporting and/or displaying data sets or reporting an outcome include digital data, a graph, a 2D graph, a 3D graph, and 4D graph, a picture (e.g., a jpg, bitmap (e.g., bmp), pdf, tiff, gif, raw, png, the like or suitable format), a pictograph, a chart, a table, a bar graph, a pie graph, a diagram, a flow chart, a scatter plot, a map, a histogram, a density chart, a function graph, a circuit diagram, a block diagram, a bubble map, a constellation diagram, a contour diagram, a cartogram, spider chart, Venn diagram, nomogram, and the like, and combination of the foregoing.

Generating an outcome often can be viewed as a transformation of nucleic acid sequence reads, or the like, into a representation of a subject's cellular nucleic acid. For example, transmuting sequence reads of nucleic acid from a subject by a method described herein, and generating an outcome can be viewed as a transformation of relatively small sequence read fragments to a representation of relatively large and complex structure of nucleic acid in the subject. In some embodiments, an outcome results from a transformation of sequence reads from a subject into a representation of an existing nucleic acid structure present in the subject (e.g., a genome, a chromosome, chromosome segment, mixture of CF nucleic acid fragments in the subject).

*Use of outcomes*

A health care professional, or other qualified individual, receiving a report comprising one or more outcomes determinative of the presence or absence of a genetic variation can use the displayed data in the report to make a call regarding the status of the test subject or patient. The healthcare professional can make a recommendation based on the provided outcome, in some embodiments. A health care professional or qualified individual can provide a test subject or patient with a call or score with regards to the presence or absence of the genetic variation based on the outcome value or values and associated confidence parameters provided in a report, in some embodiments. In certain embodiments, a score or call is made manually by a healthcare professional or qualified individual, using visual observation of the provided report. In certain embodiments, a score or call is made by an automated routine, sometimes embedded in software, and reviewed by a healthcare professional or qualified individual for accuracy prior to providing information to a test subject or patient. The term "receiving a report" as used herein refers to obtaining, by a communication means, a written and/or graphical representation comprising an outcome, which upon review allows a healthcare professional or other qualified individual to make a determination as to the presence or absence of a genetic variation in a test subject or patient. The report may be generated by a computer or by human data entry, and can be communicated using electronic means (e.g., over the internet, via computer, via fax, from one network location to another location at the same or different physical sites), or by a other method of sending or receiving data (e.g., mail service, courier service and the like). In some embodiments, the outcome is transmitted to a health care professional in a suitable medium, including, without limitation, in verbal, document, or file form. The file may be, for example, but not limited to, an auditory file, a computer readable file, a paper file, a laboratory file or a medical record file.

The term "providing an outcome" and grammatical equivalents thereof, as used herein also can refer to a method for obtaining such information, including, without limitation, obtaining the information from a laboratory (e.g., a laboratory file). A laboratory file can be generated by a laboratory that carried out one or more assays or one or more data processing steps to determine the presence or absence of the medical condition. The laboratory may be in the same location or different location (e.g., in another country) as the personnel identifying the presence or absence of the medical condition from the laboratory file. For example, the laboratory file can be generated in one location and transmitted to another location in which the information therein will be transmitted to the subject. The laboratory file may be in tangible form or electronic form (e.g., computer readable form), in certain embodiments.

In some embodiments, an outcome can be provided to a health care professional, physician or qualified individual from a laboratory and the health care professional, physician or qualified individual can make a diagnosis based on the outcome. In some embodiments, an outcome can be provided to a health care professional, physician or qualified individual from a laboratory and the health care professional, physician or qualified individual can make a diagnosis based, in part, on the outcome along with additional data and/or information and other outcomes.

A healthcare professional or qualified individual, can provide a suitable recommendation based on the outcome or outcomes provided in the report. Non-limiting examples of recommendations that can be provided based on the provided outcome report includes, surgery, radiation therapy, chemotherapy, genetic counseling, after birth treatment solutions (e.g., life planning, long term assisted care, medicaments, symptomatic treatments), pregnancy termination, organ transplant, blood transfusion, the like or combinations of the foregoing. In some embodiments, the recommendation is dependent on the outcome based classification provided (e.g., cancer, stage and/or type of cancer, Down's syndrome, Turner syndrome, medical conditions associated with genetic variations in T13, medical conditions associated with genetic variations in T18).

Laboratory personnel (e.g., a laboratory manager) can analyze values (e.g., test profiles, reference profiles, level of deviation) underlying a determination of the presence or absence of a genetic variation. For calls pertaining to presence or absence of a genetic variation that are close or questionable, laboratory personnel can re-order the same test, and/or order a different test, that makes use of the same or different sample nucleic acid from a test subject.

*Machines, software and interfaces*

Certain processes and methods described herein (e.g., mapping, counting, normalizing, range setting, adjusting, categorizing and/or determining sequence reads, counts, levels and/or profiles) often cannot be performed without a computer, microprocessor, software, module or other machine. Methods described herein typically are computer-implemented methods, and one or more portions of a method sometimes are performed by one or more processors (e.g., microprocessors), computers, systems, apparatuses, or machines (e.g., microprocessor-controlled machine).

Computers, systems, apparatuses, machines and computer program products suitable for use often include, or are utilized in conjunction with, computer readable storage media. Non-limiting examples of computer readable storage media include memory, hard disk, CD-ROM, flash memory device and the like. Computer readable storage media generally are computer hardware, and often are non-transitory computer-readable storage media. Computer readable storage media are not computer readable transmission media, the latter of which are transmission signals per se.

Provided herein are computer readable storage media with an executable program stored thereon, where the program instructs a microprocessor to perform a method described herein. Provided also are computer readable storage media with an executable program module stored thereon, where the program module instructs a microprocessor to perform part of a method described herein. Also provided herein are systems, machines, apparatuses and computer program products that include computer readable storage media with an executable program stored thereon, where the program instructs a microprocessor to perform a method described herein. Provided also are systems, machines and apparatuses that include computer readable storage media with an executable program module stored thereon, where the program module instructs a microprocessor to perform part of a method described herein.

Also provided are computer program products. A computer program product often includes a computer usable medium that includes a computer readable program code embodied therein, the computer readable program code adapted for being executed to implement a method or part of a method described herein. Computer usable media and readable program code are not transmission media (i.e., transmission signals per se). Computer readable program code often is adapted for being executed by a processor, computer, system, apparatus, or machine.

In some embodiments, methods described herein (e.g., quantifying, counting, filtering, normalizing, transforming, clustering and/or determining sequence reads, counts, levels, profiles and/or outcomes) are performed by automated methods. In some embodiments, one or more steps of a method described herein are carried out by a microprocessor and/or computer, and/or carried out in conjunction with memory. In some embodiments, an automated method is embodied in software, modules, microprocessors, peripherals and/or a machine comprising the like, that perform methods described herein. As used herein, software refers to computer readable program instructions that, when executed by a microprocessor, perform computer operations, as described herein.

Sequence reads, counts, levels and/or profiles sometimes are referred to as "data" or "data sets." In some embodiments, data or data sets can be characterized by one or more features or variables (e.g., sequence based (e.g., GC content, specific nucleotide sequence, the like), function specific (e.g., expressed genes, cancer genes, the like), location based (genome specific, chromosome specific, portion or portion-specific), the like and combinations thereof). In certain embodiments, data or data sets can be organized into a matrix having two or more dimensions based on one or more features or variables. Data organized into matrices can be organized using any suitable features or variables. In certain embodiments, data sets characterized by one or more features or variables sometimes are processed after counting.

Machines, software and interfaces may be used to conduct methods described herein. Using machines, software and interfaces, a user may enter, request, query or determine options for using particular information, programs or processes (e.g., mapping sequence reads, processing mapped data and/or providing an outcome), which can involve implementing statistical analysis algorithms, statistical significance algorithms, statistical algorithms, iterative steps, validation algorithms, and graphical representations, for example. In some embodiments, a data set may be entered by a user as input information, a user may download one or more data sets by suitable hardware media (e.g., flash drive), and/or a user may send a data set from one system to another for subsequent processing and/or providing an outcome (e.g., send sequence read data from a sequencer to a computer system for sequence read mapping; send mapped sequence data to a computer system for processing and yielding an outcome and/or report).

A system typically comprises one or more machines. Each machine comprises one or more of memory, one or more microprocessors, and instructions. Where a system includes two or more machines, some or all of the machines may be located at the same location, some or all of the machines may be located at different locations, all of the machines may be located at one location and/or all of the machines may be located at different locations. Where a system includes two or more machines, some or all of the machines may be located at the same location as a user, some or all of the machines may be located at a location different than a user, all of the machines may be located at the same location as the user, and/or all of the machine may be located at one or more locations different than the user.

A system sometimes comprises a computing machine and a sequencing apparatus or machine, where the sequencing apparatus or machine is configured to receive physical nucleic acid and generate sequence reads, and the computing apparatus is configured to process the reads from the sequencing apparatus or machine. The computing machine sometimes is configured to determine a classification outcome from the sequence reads.

A user may, for example, place a query to software which then may acquire a data set via internet access, and in certain embodiments, a programmable microprocessor may be prompted to acquire a suitable data set based on given parameters. A programmable microprocessor also may prompt a user to select one or more data set options selected by the microprocessor based on given parameters. A programmable microprocessor may prompt a user to select one or more data set options selected by the microprocessor based on information found via the internet, other internal or external information, or the like. Options may be chosen for selecting one or more data feature selections, one or more statistical algorithms, one or more statistical analysis algorithms, one or more statistical significance algorithms, iterative steps, one or more validation algorithms, and one or more graphical representations of methods, machines, apparatuses, computer programs or a non-transitory computer-readable storage medium with an executable program stored thereon.

Systems addressed herein may comprise general components of computer systems, such as, for example, network servers, laptop systems, desktop systems, handheld systems, personal digital assistants, computing kiosks, and the like. A computer system may comprise one or more input means such as a keyboard, touch screen, mouse, voice recognition or other means to allow the user to enter data into the system. A system may further comprise one or more outputs, including, but not limited to, a display screen (e.g., CRT or LCD), speaker, FAX machine, printer (e.g., laser, ink jet, impact, black and white or color printer), or other output useful for providing visual, auditory and/or hardcopy output of information (e.g., outcome and/or report).

In a system, input and output components may be connected to a central processing unit which may comprise among other components, a microprocessor for executing program instructions and memory for storing program code and data. In some embodiments, processes may be implemented as a single user system located in a single geographical site. In certain embodiments, processes may be implemented as a multi-user system. In the case of a multi-user implementation, multiple central processing units may be connected by means of a network. The network may be local, encompassing a single department in one portion of a building, an entire building, span multiple buildings, span a region, span an entire country or be worldwide. The network may be private, being owned and controlled by a provider, or it may be implemented as an internet based service where the user accesses a web page to enter and retrieve information. Accordingly, in certain embodiments, a system includes one or more machines, which may be local or remote with respect to a user. More than one machine in one location or multiple locations may be accessed by a user, and data may be mapped and/or processed in series and/or in parallel. Thus, a suitable configuration and control may be utilized for mapping and/or processing data using multiple machines, such as in local network, remote network and/or "cloud" computing platforms.

A system can include a communications interface in some embodiments. A communications interface allows for transfer of software and data between a computer system and one or more external devices. Non-limiting examples of communications interfaces include a modem, a network interface (such as an Ethernet card), a communications port, a PCMCIA slot and card, and the like. Software and data transferred via a communications interface generally are in the form of signals, which can be electronic, electromagnetic, optical and/or other signals capable of being received by a communications interface. Signals often are provided to a communications interface via a channel. A channel often carries signals and can be implemented using wire or cable, fiber optics, a phone line, a cellular phone link, an RF link and/or other communications channels. Thus, in an example, a communications interface may be used to receive signal information that can be detected by a signal detection module.

Data may be input by a suitable device and/or method, including, but not limited to, manual input devices or direct data entry devices (DDEs). Non-limiting examples of manual devices include keyboards, concept keyboards, touch sensitive screens, light pens, mouse, tracker balls, joysticks, graphic tablets, scanners, digital cameras, video digitizers and voice recognition devices. Non-limiting examples of DDEs include bar code readers, magnetic strip codes, smart cards, magnetic ink character recognition, optical character recognition, optical mark recognition, and turnaround documents.

In some embodiments, output from a sequencing apparatus or machine may serve as data that can be input via an input device. In certain embodiments, mapped sequence reads may serve as data that can be input via an input device. In certain embodiments, nucleic acid fragment size (e.g., length) may serve as data that can be input via an input device. In certain embodiments, output from a nucleic acid capture process (e.g., genomic region origin data) may serve as data that can be input via an input device. In certain embodiments, a combination of nucleic acid fragment size (e.g., length) and output from a nucleic acid capture process (e.g., genomic region origin data) may serve as data that can be input via an input device. In certain embodiments, simulated data is generated by an in silico process and the simulated data serves as data that can be input via an input device. The term "in silico" refers to research and experiments performed using a computer. In silico processes include, but are not limited to, mapping sequence reads and processing mapped sequence reads according to processes described herein.

A system may include software useful for performing a process or part of a process described herein, and software can include one or more modules for performing such processes (e.g., sequencing module, logic processing module, data display organization module). The term "software" refers to computer readable program instructions that, when executed by a computer, perform computer operations. Instructions executable by the one or more microprocessors sometimes are provided as executable code, that when executed, can cause one or more microprocessors to implement a method described herein. A module described herein can exist as software, and instructions (e.g., processes, routines, subroutines) embodied in the software can be implemented or performed by a microprocessor. For example, a module (e.g., a software module) can be a part of a program that performs a particular process or task. The term "module" refers to a self-contained functional unit that can be used in a larger machine or software system. A module can comprise a set of instructions for carrying out a function of the module. A module can transform data and/or information. Data and/or information can be in a suitable form. For example, data and/or information can be digital or analogue. In certain embodiments, data and/or information sometimes can be packets, bytes, characters, or bits. In some embodiments, data and/or information can be any gathered, assembled or usable data or information. Non-limiting examples of data and/or information include a suitable media, pictures, video, sound (e.g. frequencies, audible or non-audible), numbers, constants, a value, objects, time, functions, instructions, maps, references, sequences, reads, mapped reads, levels, ranges, thresholds, signals, displays, representations, or transformations thereof. A module can accept or receive data and/or information, transform the data and/or information into a second form, and provide or transfer the second form to a machine, peripheral, component or another module. A module can perform one or more of the following non-limiting functions: mapping sequence reads, providing counts, assembling portions, providing or determining a level, providing a count profile, normalizing (e.g., normalizing reads, normalizing counts, and the like), providing a normalized count profile or levels of normalized counts, comparing two or more levels, providing uncertainty values, providing or determining expected levels and expected ranges(e.g., expected level ranges, threshold ranges and threshold levels), providing adjustments to levels (e.g., adjusting a first level, adjusting a second level, adjusting a profile of a chromosome or a part thereof, and/or padding), providing identification (e.g., identifying a copy number alteration, genetic variation or aneuploidy), categorizing, plotting, and/or determining an outcome, for example. A microprocessor can, in certain embodiments, carry out the instructions in a module. In some embodiments, one or more microprocessors are required to carry out instructions in a module or group of modules. A module can provide data and/or information to another module, machine or source and can receive data and/or information from another module, machine or source.

A computer program product sometimes is embodied on a tangible computer-readable medium, and sometimes is tangibly embodied on a non-transitory computer-readable medium. A module sometimes is stored on a computer readable medium (e.g., disk, drive) or in memory (e.g., random access memory). A module and microprocessor capable of implementing instructions from a module can be located in a machine or in a different machine. A module and/or microprocessor capable of implementing an instruction for a module can be located in the same location as a user (e.g., local network) or in a different location from a user (e.g., remote network, cloud system). In embodiments in which a method is carried out in conjunction with two or more modules, the modules can be located in the same machine, one or more modules can be located in different machine in the same physical location, and one or more modules may be located in different machines in different physical locations.

A machine, in some embodiments, comprises at least one microprocessor for carrying out the instructions in a module. Sequence read quantifications (e.g., counts) sometimes are accessed by a microprocessor that executes instructions configured to carry out a method described herein. Sequence read quantifications that are accessed by a microprocessor can be within memory of a system, and the counts can be accessed and placed into the memory of the system after they are obtained. In some embodiments, a machine includes a microprocessor (e.g., one or more microprocessors) which microprocessor can perform and/or implement one or more instructions (e.g., processes, routines and/or subroutines) from a module. In some embodiments, a machine includes multiple microprocessors, such as microprocessors coordinated and working in parallel. In some embodiments, a machine operates with one or more external microprocessors (e.g., an internal or external network, server, storage device and/or storage network (e.g., a cloud)). In some embodiments, a machine comprises a module (e.g., one or more modules). A machine comprising a module often is capable of receiving and transferring one or more of data and/or information to and from other modules.

In certain embodiments, a machine comprises peripherals and/or components. In certain embodiments, a machine can comprise one or more peripherals or components that can transfer data and/or information to and from other modules, peripherals and/or components. In certain embodiments, a machine interacts with a peripheral and/or component that provides data and/or information. In certain embodiments, peripherals and components assist a machine in carrying out a function or interact directly with a module. Non-limiting examples of peripherals and/or components include a suitable computer peripheral, I/O or storage method or device including but not limited to scanners, printers, displays (e.g., monitors, LED, LCT or CRTs), cameras, microphones, pads (e.g., ipads, tablets), touch screens, smart phones, mobile phones, USB I/O devices, USB mass storage devices, keyboards, a computer mouse, digital pens, modems, hard drives, jump drives, flash drives, a microprocessor, a server, CDs, DVDs, graphic cards, specialized I/O devices (e.g., sequencers, photo cells, photo multiplier tubes, optical readers, sensors, etc.), one or more flow cells, fluid handling components, network interface controllers, ROM, RAM, wireless transfer methods and devices (Bluetooth, WiFi, and the like,), the world wide web (www), the internet, a computer and/or another module.

Software often is provided on a program product containing program instructions recorded on a computer readable medium, including, but not limited to, magnetic media including floppy disks, hard disks, and magnetic tape; and optical media including CD-ROM discs, DVD discs, magneto-optical discs, flash memory devices (e.g., flash drives), RAM, floppy discs, the like, and other such media on which the program instructions can be recorded. In online implementation, a server and web site maintained by an organization can be configured to provide software downloads to remote users, or remote users may access a remote system maintained by an organization to remotely access software. Software may obtain or receive input information. Software may include a module that specifically obtains or receives data (e.g., a data receiving module that receives sequence read data and/or mapped read data) and may include a module that specifically processes the data (e.g., a processing module that processes received data (e.g., filters, normalizes, provides an outcome and/or report). The terms "obtaining" and "receiving" input information refers to receiving data (e.g., sequence reads, mapped reads) by computer communication means from a local, or remote site, human data entry, or any other method of receiving data. The input information may be generated in the same location at which it is received, or it may be generated in a different location and transmitted to the receiving location. In some embodiments, input information is modified before it is processed (e.g., placed into a format amenable to processing (e.g., tabulated)).

Software can include one or more algorithms in certain embodiments. An algorithm may be used for processing data and/or providing an outcome or report according to a finite sequence of instructions. An algorithm often is a list of defined instructions for completing a task. Starting from an initial state, the instructions may describe a computation that proceeds through a defined series of successive states, eventually terminating in a final ending state. The transition from one state to the next is not necessarily deterministic (e.g., some algorithms incorporate randomness). By way of example, and without limitation, an algorithm can be a search algorithm, sorting algorithm, merge algorithm, numerical algorithm, graph algorithm, string algorithm, modeling algorithm, computational genometric algorithm, combinatorial algorithm, machine learning algorithm, cryptography algorithm, data compression algorithm, parsing algorithm and the like. An algorithm can include one algorithm or two or more algorithms working in combination. An algorithm can be of any suitable complexity class and/or parameterized complexity. An algorithm can be used for calculation and/or data processing, and in some embodiments, can be used in a deterministic or probabilistic/predictive approach. An algorithm can be implemented in a computing environment by use of a suitable programming language, non-limiting examples of which are C, C++, Java, Perl, Python, Fortran, and the like. In some embodiments, an algorithm can be configured or modified to include margin of errors, statistical analysis, statistical significance, and/or comparison to other information or data sets (e.g., applicable when using a neural net or clustering algorithm).

In certain embodiments, several algorithms may be implemented for use in software. These algorithms can be trained with raw data in some embodiments. For each new raw data sample, the trained algorithms may produce a representative processed data set or outcome. A processed data set sometimes is of reduced complexity compared to the parent data set that was processed. Based on a processed set, the performance of a trained algorithm may be assessed based on sensitivity and specificity, in some embodiments. An algorithm with the highest sensitivity and/or specificity may be identified and utilized, in certain embodiments.

In certain embodiments, simulated (or simulation) data can aid data processing, for example, by training an algorithm or testing an algorithm. In some embodiments, simulated data includes hypothetical various samplings of different groupings of sequence reads. Simulated data may be based on what might be expected from a real population or may be skewed to test an algorithm and/or to assign a correct classification. Simulated data also is referred to herein as "virtual" data. Simulations can be performed by a computer program in certain embodiments. One possible step in using a simulated data set is to evaluate the confidence of identified results, e.g., how well a random sampling matches or best represents the original data. One approach is to calculate a probability value (p-value), which estimates the probability of a random sample having better score than the selected samples. In some embodiments, an empirical model may be assessed, in which it is assumed that at least one sample matches a reference sample (with or without resolved variations). In some embodiments, another distribution, such as a Poisson distribution for example, can be used to define the probability distribution.

A system may include one or more microprocessors in certain embodiments. A microprocessor can be connected to a communication bus. A computer system may include a main memory, often random access memory (RAM), and can also include a secondary memory. Memory in some embodiments comprises a non-transitory computer-readable storage medium. Secondary memory can include, for example, a hard disk drive and/or a removable storage drive, representing a floppy disk drive, a magnetic tape drive, an optical disk drive, memory card and the like. A removable storage drive often reads from and/or writes to a removable storage unit. Non-limiting examples of removable storage units include a floppy disk, magnetic tape, optical disk, and the like, which can be read by and written to by, for example, a removable storage drive. A removable storage unit can include a computer-usable storage medium having stored therein computer software and/or data.

A microprocessor may implement software in a system. In some embodiments, a microprocessor may be programmed to automatically perform a task described herein that a user could perform. Accordingly, a microprocessor, or algorithm conducted by such a microprocessor, can require little to no supervision or input from a user (e.g., software may be programmed to implement a function automatically). In some embodiments, the complexity of a process is so large that a single person or group of persons could not perform the process in a timeframe short enough for determining the presence or absence of a genetic variation.

In some embodiments, secondary memory may include other similar means for allowing computer programs or other instructions to be loaded into a computer system. For example, a system can include a removable storage unit and an interface device. Non-limiting examples of such systems include a program cartridge and cartridge interface (such as that found in video game devices), a removable memory chip (such as an EPROM, or PROM) and associated socket, and other removable storage units and interfaces that allow software and data to be transferred from the removable storage unit to a computer system.

Fig. 1 illustrates a non-limiting example of a computing environment 110 in which various systems, methods, algorithms, and data structures described herein may be implemented. The computing environment 110 is only one example of a suitable computing environment and is not intended to suggest any limitation as to the scope of use or functionality of the systems, methods, and data structures described herein. Neither should computing environment 110 be interpreted as having any dependency or requirement relating to any one or combination of components illustrated in computing environment 110. A subset of systems, methods, and data structures shown in Fig. 1 can be utilized in certain embodiments. Systems, methods, and data structures described herein are operational with numerous other general purpose or special purpose computing system environments or configurations. Examples of known computing systems, environments, and/or configurations that may be suitable include, but are not limited to, personal computers, server computers, thin clients, thick clients, hand-held or laptop devices, multiprocessor systems, microprocessor-based systems, set top boxes, programmable consumer electronics, network PCs, minicomputers, mainframe computers, distributed computing environments that include any of the above systems or devices, and the like.

The operating environment 110 of Fig. 1 includes a general purpose computing device in the form of a computer 120, including a processing unit 121, a system memory 122, and a system bus 123 that operatively couples various system components including the system memory 122 to the processing unit 121. There may be only one or there may be more than one processing unit 121, such that the processor of computer 120 includes a single central-processing unit (CPU), or a plurality of processing units, commonly referred to as a parallel processing environment. The computer 120 may be a conventional computer, a distributed computer, or any other type of computer.

The system bus 123 may be any of several types of bus structures including a memory bus or memory controller, a peripheral bus, and a local bus using any of a variety of bus architectures. The system memory may also be referred to as simply the memory, and includes read only memory (ROM) 124 and random access memory (RAM). A basic input/output system (BIOS) 126, containing the basic routines that help to transfer information between elements within the computer 120, such as during start-up, is stored in ROM 124. The computer 120 may further include a hard disk drive interface 127 for reading from and writing to a hard disk, not shown, a magnetic disk drive 128 for reading from or writing to a removable magnetic disk 129, and an optical disk drive 130 for reading from or writing to a removable optical disk 131 such as a CD ROM or other optical media.

The hard disk drive 127, magnetic disk drive 128, and optical disk drive 130 are connected to the system bus 123 by a hard disk drive interface 132, a magnetic disk drive interface 133, and an optical disk drive interface 134, respectively. The drives and their associated computer-readable media provide nonvolatile storage of computer-readable instructions, data structures, program modules and other data for the computer 120. Any type of computer-readable media that can store data that is accessible by a computer, such as magnetic cassettes, flash memory cards, digital video disks, Bernoulli cartridges, random access memories (RAMs), read only memories (ROMs), and the like, may be used in the operating environment.

A number of program modules may be stored on the hard disk, magnetic disk 129, optical disk 131, ROM 124, or RAM, including an operating system 135, one or more application programs 136, other program modules 137, and program data 138. A user may enter commands and information into the personal computer 120 through input devices such as a keyboard 140 and pointing device 142. Other input devices (not shown) may include a microphone, joystick, game pad, satellite dish, scanner, or the like. These and other input devices are often connected to the processing unit 121 through a serial port interface 146 that is coupled to the system bus, but may be connected by other interfaces, such as a parallel port, game port, or a universal serial bus (USB). A monitor 147 or other type of display device is also connected to the system bus 123 via an interface, such as a video adapter 148. In addition to the monitor, computers typically include other peripheral output devices (not shown), such as speakers and printers.

The computer 120 may operate in a networked environment using logical connections to one or more remote computers, such as remote computer 149. These logical connections may be achieved by a communication device coupled to or a part of the computer 120, or in other manners. The remote computer 149 may be another computer, a server, a router, a network PC, a client, a peer device or other common network node, and typically includes many or all of the elements described above relative to the computer 120, although only a memory storage device 150 has been illustrated in Fig. 1. The logical connections depicted in Fig. 1 include a local-area network (LAN) 151 and a wide-area network (WAN) 152. Such networking environments are commonplace in office networks, enterprise-wide computer networks, intranets and the Internet, which all are types of networks.

When used in a LAN-networking environment, the computer 120 is connected to the local network 151 through a network interface or adapter 153, which is one type of communications device. When used in a WAN-networking environment, the computer 120 often includes a modem 154, a type of communications device, or any other type of communications device for establishing communications over the wide area network 152. The modem 154, which may be internal or external, is connected to the system bus 123 via the serial port interface 146. In a networked environment, program modules depicted relative to the personal computer 120, or portions thereof, may be stored in the remote memory storage device. It is appreciated that the network connections shown are non-limiting examples and other communications devices for establishing a communications link between computers may be used.

Thus, also provided in this disclosure is a system for determining the presence or absence of a genetic variation. The system comprises a component for amplifying in a single reaction a plurality of paralogous polynucleotide species from nucleic acid in a sample, comprising contacting the nucleic acid with amplification primers under amplification conditions, wherein the paralogous polynucleotide species of each of the sets are amplified by a single pair of amplification primers and each primer of the pair of amplification primers is complementary to less than 20 positions in a human genome, a component for determining the amount of each amplified paralogous polynucleotide species in each of the sets, a component for determining a paralog ratio for each of the sets between the amount of each amplified paralogous polynucleotide species in each of the sets, thereby generating a plurality of paralog ratios; and a component for determining the presence or absence of the genetic variation based on the plurality of paralog ratios. In some cases, one or more components described above comprise a computer processor. For example, the system may comprises a computer processor that is configured to determine the amount of each amplified paralogous polynucleotide species in each of the sets; a computer processor that is configured to determine a paralog ratio for each of the sets between the amount of each amplified paralogous polynucleotide species in each of the sets, thereby generating a plurality of paralog ratios; and/or a computer processor that is configured to determine the presence or absence of the genetic variation based on the plurality of paralog ratios.

*Transformations*

As noted above, data sometimes is transformed from one form into another form. The terms "transformed," "transformation," and grammatical derivations or equivalents thereof, as used herein refer to an alteration of data from a physical starting material (e.g., test subject and/or reference subject sample nucleic acid) into a digital representation of the physical starting material (e.g., sequence read data), and in some embodiments includes a further transformation into one or more numerical values or graphical representations of the digital representation that can be utilized to provide an outcome. In certain embodiments, the one or more numerical values and/or graphical representations of digitally represented data can be utilized to represent the appearance of a test subject's physical genome (e.g., virtually represent or visually represent the presence or absence of a genomic insertion, duplication or deletion; represent the presence or absence of a variation in the physical amount of a sequence associated with medical conditions). A virtual representation sometimes is further transformed into one or more numerical values or graphical representations of the digital representation of the starting material. These methods can transform physical starting material into a numerical value or graphical representation, or a representation of the physical appearance of a test subject's nucleic acid.

In some embodiments, transformation of a data set facilitates providing an outcome by reducing data complexity and/or data dimensionality. Data set complexity sometimes is reduced during the process of transforming a physical starting material into a virtual representation of the starting material (e.g., sequence reads representative of physical starting material). A suitable feature or variable can be utilized to reduce data set complexity and/or dimensionality. Non-limiting examples of features that can be chosen for use as a target feature for data processing include GC content, fetal gender prediction, fragment size (e.g., length of CCF fragments, reads or a suitable representation thereof (e.g., FRS)), fragment sequence, identification of a copy number alteration, identification of chromosomal aneuploidy, identification of particular genes or proteins, identification of cancer, diseases, inherited genes/traits, chromosomal abnormalities, a biological category, a chemical category, a biochemical category, a category of genes or proteins, a gene ontology, a protein ontology, co-regulated genes, cell signaling genes, cell cycle genes, proteins pertaining to the foregoing genes, gene variants, protein variants, co-regulated genes, co-regulated proteins, amino acid sequence, nucleotide sequence, protein structure data and the like, and combinations of the foregoing. Non-limiting examples of data set complexity and/or dimensionality reduction include; reduction of a plurality of sequence reads to profile plots, reduction of a plurality of sequence reads to numerical values (e.g., normalized values, Z-scores, p-values); reduction of multiple analysis methods to probability plots or single points; principal component analysis of derived quantities; and the like or combinations thereof.

*Genetic variations and medical conditions*

The presence or absence of a genetic variation can be determined using a method or apparatus described herein. In certain embodiments, the presence or absence of one or more genetic variations is determined according to an outcome provided by methods and apparatuses described herein. A genetic variation generally is a particular genetic phenotype present in certain individuals, and often a genetic variation is present in a statistically significant subpopulation of individuals. In some embodiments, a genetic variation is a chromosome abnormality or copy number alteration (e.g., aneuploidy, duplication of one or more chromosomes, loss of one or more chromosomes, partial chromosome abnormality or mosaicism (e.g., loss or gain of one or more regions of a chromosome), translocation, inversion, each of which is described in greater detail herein). Non-limiting examples of genetic variations include one or more copy number alterations, deletions (e.g., microdeletions), duplications (e.g., microduplications), insertions, mutations (e.g., single nucleotide variations), polymorphisms (e.g., single-nucleotide polymorphisms), fusions, repeats (e.g., short tandem repeats), distinct methylation sites, distinct methylation patterns, the like and combinations thereof. An insertion, repeat, deletion, duplication, mutation or polymorphism can be of any length, and in some embodiments, is about 1 base or base pair (bp) to about 250 megabases (Mb) in length. In some embodiments, an insertion, repeat, deletion, duplication, mutation or polymorphism is about 1 base or base pair (bp) to about 50,000 kilobases (kb) in length (e.g., about 10 bp, 50 bp, 100 bp, 500 bp, 1 kb, 5 kb, 10kb, 50 kb, 100 kb, 500 kb, 1000 kb, 5000 kb or 10,000 kb in length).

A genetic variation is sometime a deletion. In certain instances, a deletion is a mutation (e.g., a genetic aberration) in which a part of a chromosome or a sequence of DNA is missing. A deletion is often the loss of genetic material. Any number of nucleotides can be deleted. A deletion can comprise the deletion of one or more entire chromosomes, a region of a chromosome, an allele, a gene, an intron, an exon, any non-coding region, any coding region, a part thereof or combination thereof. A deletion can comprise a microdeletion. A deletion can comprise the deletion of a single base.

A genetic variation is sometimes a duplication. In certain instances, a duplication is a mutation (e.g., a genetic aberration) in which a part of a chromosome or a sequence of DNA is copied and inserted back into the genome. In certain embodiments, a genetic duplication (e.g., duplication) is any duplication of a region of DNA. In some embodiments, a duplication is a nucleic acid sequence that is repeated, often in tandem, within a genome or chromosome. In some embodiments, a duplication can comprise a copy of one or more entire chromosomes, a region of a chromosome, an allele, a gene, an intron, an exon, any non-coding region, any coding region, part thereof or combination thereof. A duplication can comprise a microduplication. A duplication sometimes comprises one or more copies of a duplicated nucleic acid. A duplication sometimes is characterized as a genetic region repeated one or more times (e.g., repeated 1, 2, 3, 4, 5, 6, 7, 8, 9 or 10 times). Duplications can range from small regions (thousands of base pairs) to whole chromosomes in some instances. Duplications frequently occur as the result of an error in homologous recombination or due to a retrotransposon event. Duplications have been associated with certain types of proliferative diseases. Duplications can be characterized using genomic microarrays or comparative genetic hybridization (CGH).

A genetic variation is sometimes an insertion. An insertion is sometimes the addition of one or more nucleotide base pairs into a nucleic acid sequence. An insertion is sometimes a microinsertion. In certain embodiments, an insertion comprises the addition of a region of a chromosome into a genome, chromosome, or part thereof. In certain embodiments, an insertion comprises the addition of an allele, a gene, an intron, an exon, any non-coding region, any coding region, part thereof or combination thereof into a genome or part thereof. In certain embodiments, an insertion comprises the addition (e.g., insertion) of nucleic acid of unknown origin into a genome, chromosome, or part thereof. In certain embodiments, an insertion comprises the addition (e.g., insertion) of a single base.

As used herein a "copy number alteration" generally is a class or type of genetic variation or chromosomal aberration. A copy number alteration also may be referred to as a copy number variation, and can be a deletion (e.g., microdeletion), duplication (e.g., a microduplication) or insertion (e.g., a microinsertion). Often, the prefix "micro" as used herein sometimes is a region of nucleic acid less than 5 Mb in length. A copy number alteration can include one or more deletions (e.g., microdeletion), duplications and/or insertions(e.g., a microduplication, microinsertion) of a part of a chromosome. In certain embodiments, a duplication comprises an insertion. In certain embodiments, an insertion is a duplication. In certain embodiments, an insertion is not a duplication.

In some embodiments, a copy number alteration is a fetal copy number alteration. Often, a fetal copy number alteration is a copy number alteration in the genome of a fetus. In some embodiments, a copy number alteration is a maternal and/or fetal copy number alteration. In certain embodiments, a maternal and/or fetal copy number alteration is a copy number alteration within the genome of a pregnant female (e.g., a female subject bearing a fetus), a female subject that gave birth or a female capable of bearing a fetus. A copy number alteration can be a heterozygous copy number alteration where the alteration (e.g., a duplication or deletion) is present on one allele of a genome. A copy number alteration can be a homozygous copy number alteration where the alteration is present on both alleles of a genome. In some embodiments, a copy number alteration is a heterozygous or homozygous fetal copy number alteration. In some embodiments, a copy number alteration is a heterozygous or homozygous maternal and/or fetal copy number alteration. A copy number alteration sometimes is present in a maternal genome and a fetal genome, a maternal genome and not a fetal genome, or a fetal genome and not a maternal genome.

"Ploidy" is a reference to the number of chromosomes present in a subject. In certain embodiments, "ploidy" is the same as "chromosome ploidy." In humans, for example, autosomal chromosomes are often present in pairs. For example, in the absence of a genetic variation, most humans have two of each autosomal chromosome (e.g., chromosomes 1-22). The presence of the normal complement of 2 autosomal chromosomes in a human is often referred to as euploid or diploid. "Microploidy" is similar in meaning to ploidy. "Microploidy" often refers to the ploidy of a part of a chromosome. The term "microploidy" sometimes is a reference to the presence or absence of a copy number alteration (e.g., a deletion, duplication and/or an insertion) within a chromosome (e.g., a homozygous or heterozygous deletion, duplication, or insertion, the like or absence thereof).

A genetic variation for which the presence or absence is identified for a subject is associated with a medical condition in certain embodiments. Thus, technology described herein can be used to identify the presence or absence of one or more genetic variations that are associated with a medical condition or medical state. Non-limiting examples of medical conditions include those associated with intellectual disability (e.g., Down Syndrome), aberrant cell-proliferation (e.g., cancer), presence of a micro-organism nucleic acid (e.g., virus, bacterium, fungus, yeast), and preeclampsia.

Non-limiting examples of genetic variations, medical conditions and states are described hereafter.

*Chromosome abnormalities*

In some embodiments, the presence or absence of a chromosome abnormality can be determined by using a method and/or apparatus described herein. Chromosome abnormalities include, without limitation, copy number alterations, and a gain or loss of an entire chromosome or a region of a chromosome comprising one or more genes. Chromosome abnormalities include monosomies, trisomies, polysomies, loss of heterozygosity, translocations, deletions and/or duplications of one or more nucleotide sequences (e.g., one or more genes), including deletions and duplications caused by unbalanced translocations. The term "chromosomal abnormality" or "aneuploidy" as used herein refer to a deviation between the structure of the subject chromosome and a normal homologous chromosome. The term "normal" refers to the predominate karyotype or banding pattern found in healthy individuals of a particular species, for example, a euploid genome (e.g., diploid in humans, e.g., 46,XX or 46,XY). As different organisms have widely varying chromosome complements, the term "aneuploidy" does not refer to a particular number of chromosomes, but rather to the situation in which the chromosome content within a given cell or cells of an organism is abnormal. In some embodiments, the term "aneuploidy" herein refers to an imbalance of genetic material caused by a loss or gain of a whole chromosome, or part of a chromosome. An "aneuploidy" can refer to one or more deletions and/or insertions of a region of a chromosome. The term "euploid," in some embodiments, refers a normal complement of chromosomes.

The term "monosomy" as used herein refers to lack of one chromosome of the normal complement. Partial monosomy can occur in unbalanced translocations or deletions, in which only a part of the chromosome is present in a single copy. Monosomy of sex chromosomes (45, X) causes Turner syndrome, for example. The term "disomy" refers to the presence of two copies of a chromosome. For organisms such as humans that have two copies of each chromosome (those that are diploid or "euploid"), disomy is the normal condition. For organisms that normally have three or more copies of each chromosome (those that are triploid or above), disomy is an aneuploid chromosome state. In uniparental disomy, both copies of a chromosome come from the same parent (with no contribution from the other parent).

The term "trisomy" as used herein refers to the presence of three copies, instead of two copies, of a particular chromosome. The presence of an extra chromosome 21, which is found in human Down syndrome, is referred to as "Trisomy 21." Trisomy 18 and Trisomy 13 are two other human autosomal trisomies. Trisomy of sex chromosomes can be seen in females (e.g., 47, XXX in Triple X Syndrome) or males (e.g., 47, XXY in Klinefelter's Syndrome; or 47,XYY in Jacobs Syndrome). In some embodiments, a trisomy is a duplication of most or all of an autosome. In certain embodiments, a trisomy is a whole chromosome aneuploidy resulting in three instances (e.g., three copies) of a particular type of chromosome (e.g., instead of two instances (e.g., a pair) of a particular type of chromosome for a euploid).

The terms "tetrasomy" and "pentasomy" as used herein refer to the presence of four or five copies of a chromosome, respectively. Although rarely seen with autosomes, sex chromosome tetrasomy and pentasomy have been reported in humans, including XXXX, XXXY, XXYY, XYYY, XXXXX, XXXXY, XXXYY, XXYYY and XYYYY.

The term "mosaicism" as used herein refers to aneuploidy in some cells, but not all cells, of an organism. Certain chromosome abnormalities can exist as mosaic and non-mosaic chromosome abnormalities. For example, certain trisomy 21 individuals have mosaic Down syndrome and some have non-mosaic Down syndrome. Different mechanisms can lead to mosaicism. For example, (i) an initial zygote may have three 21st chromosomes, which normally would result in simple trisomy 21, but during the course of cell division one or more cell lines lost one of the 21st chromosomes; and (ii) an initial zygote may have two 21st chromosomes, but during the course of cell division one of the 21 st chromosomes were duplicated. Somatic mosaicism likely occurs through mechanisms distinct from those typically associated with genetic syndromes involving complete or mosaic aneuploidy. Methods and protocols described herein can identify presence or absence of non-mosaic and mosaic chromosome abnormalities.

Following is a non-limiting list of chromosome abnormalities that can be potentially identified by methods, processes, machines and apparatuses described herein.

| Chromosome | Abnormality | Disease Association |
|---|---|---|
| X | XO | Turner's Syndrome |
| Y | XXY | Klinefelter syndrome |
| Y | XYY | Double Y syndrome |
| Y | XXX | Trisomy X syndrome |
| Y | XXXX | Four X syndrome |
| Y | Xp21 deletion | Duchenne's/Becker syndrome, congenital adrenal hypoplasia, chronic granulomatus disease |
| Y | Xp22 deletion | steroid sulfatase deficiency |
| Y | Xq26 deletion | X-linked lymphproliferative disease |
| 1 | 1p (somatic) monosomy trisomy | neuroblastoma |
| 2 | monosomy trisomy 2q | growth retardation, developmental and mental delay, and minor physical abnormalities |
| 3 | monosomy trisomy (somatic) | Non-Hodgkin's lymphoma |
| 4 | monosomy trsiomy (somatic) | Acute non lymphocytic leukaemia (ANLL) |
| 5 | 5p | Cri du chat; Lejeune syndrome |
| 5 | 5q (somatic) monosomy trisomy | myelodysplastic syndrome |
| 6 | monosmy trisomy (somatic) | clear-cell sarcoma |

(continued)

| Chromosome | Abnormality | Disease Association |
|---|---|---|
| 7 | 7q11.23 deletion | William's syndrome |
| 7 | monosomy trisomy | monosomy 7 syndrome of childhood; somatic: renal cortical adenomas; myelodysplastic syndrome |
| 8 | 8q24.1 deletion | Langer-Giedon syndrome |
| 8 | monosomy trisomy | myelodysplastic syndrome; Warkany syndrome; somatic: chronic myelogenous leukemia |
| 9 | monosomy 9p | Alfi's syndrome |
| 9 | monosomy 9p partial trisomy | Rethore syndrome |
| 9 | trisomy | complete trisomy 9 syndrome; mosaic trisomy 9 syndrome |
| 10 | Monosomy trisomy (somatic) | ALL or ANLL |
| 11 | 11p- | Aniridia; Wilms tumor |
| 11 | 11q- | Jacobson Syndrome |
| 11 | monosomy (somatic) trisomy | myeloid lineages affected (ANLL, MDS) |
| 12 | monosomy trisomy (somatic) | CLL, Juvenile granulosa cell tumor (JGCT) |
| 13 | 13q- | 13q-syndrome; Orbeli syndrome |
| 13 | 13q 14 deletion | retinoblastoma |
| 13 | monosomy trisomy | Patau's syndrome |
| 14 | monsomy trisomy (somatic) | myeloid disorders (MDS, ANLL, atypical CML) |
| 15 | 15q11-q13 deletion monosomy | Prader-Willi, Angelman's syndrome |
| 15 | trisomy (somatic) | myeloid and lymphoid lineages affected, e.g., MDS, ANLL, ALL, CLL) |
| 16 | 16q 13.3 deletion monosomy trisomy (somatic) | Rubenstein- Taybi papillary renal cell carcinomas (malignant) |
| 17 | 17p-(somatic) | 17p syndrome in myeloid malignancies |
| 17 | 17 q 11.2 deletion | Smith- Magenis |
| 17 | 17q13.3 | Miller-Dieker |
| 17 | monosomy trisomy (somatic) | renal cortical adenomas |
| 17 | 17p11.2-12 trisomy | Charcot-Marie Tooth Syndrome type 1; HNPP |
| 18 | 18p- | 18p partial monosomy syndrome or Grouchy Lamy Thieffry syndrome |
| 18 | 18q- | Grouchy Lamy Salmon Landry Syndrome |
| 18 | monosomy trisomy | Edwards Syndrome |
| 19 | monosomy trisomy | |
| 20 | 20p- | trisomy 20p syndrome |
| 20 | 20p11.2-12 deletion | Alagille |

(continued)

| Chromosome | Abnormality | Disease Association |
|---|---|---|
| 20 | 20q- | somatic: MDS, ANLL, polycythemia vera, chronic neutrophilic leukemia |
| 20 | monosomy trisomy (somatic) | papillary renal cell carcinomas (malignant) |
| 21 | monosomy trisomy | Down's syndrome |
| 22 | 22q11.2 deletion | DiGeorge's syndrome, velocardiofacial syndrome, conotruncal anomaly face syndrome, autosomal dominant Opitz G/BBB syndrome, Caylor cardiofacial syndrome |
| 22 | monosomy trisomy | complete trisomy 22 syndrome |

In certain embodiments, presence or absence of a fetal chromosome abnormality is identified (e.g., trisomy 21, trisomy 18 and/or trisomy 13). Presence or absence of one or more of the chromosome abnormalities described in the table above may be identified in some embodiments.

*Medical disorders and medical conditions*

Methods described herein can be applicable to any suitable medical disorder or medical condition. Non-limiting examples of medical disorders and medical conditions include cell proliferative disorders and conditions, wasting disorders and conditions, degenerative disorders and conditions, autoimmune disorders and conditions, pre-eclampsia, chemical or environmental toxicity, liver damage or disease, kidney damage or disease, vascular disease, high blood pressure, and myocardial infarction.

*Preeclampsia*

In some embodiments, the presence or absence of preeclampsia is determined by using a method or apparatus described herein. Preeclampsia is a condition in which hypertension arises in pregnancy (e.g., pregnancy-induced hypertension) and is associated with significant amounts of protein in the urine. In certain instances, preeclampsia may be associated with elevated levels of extracellular nucleic acid and/or alterations in methylation patterns. For example, a positive correlation between extracellular fetal-derived hypermethylated RASSF1A levels and the severity of pre-eclampsia has been observed. In certain instances, increased DNA methylation is observed for the H19 gene in preeclamptic placentas compared to normal controls.

*Markers*

In certain instances, a polynucleotide in abnormal or diseased cells is modified with respect to nucleic acid in normal or non-diseased cells (e.g., single nucleotide variation, copy number variation). In some instances, a polynucleotide is present in abnormal or diseased cells and not present in normal or non-diseased cells, and sometimes a polynucleotide is not present in abnormal or diseased cells and is present in normal or non-diseased cells. Thus, a marker sometimes is a single nucleotide variation and/or a copy number variation (e.g., a differentially expressed DNA or RNA (e.g., mRNA)). For example, patients with metastatic diseases may be identified by cancer-specific markers and/or certain single nucleotide polymorphisms or short tandem repeats, for example. Non-limiting examples of cancer types that may be positively correlated with elevated levels of circulating DNA include breast cancer, colorectal cancer, gastrointestinal cancer, hepatocellular cancer, lung cancer, melanoma, non-Hodgkin lymphoma, leukemia, multiple myeloma, bladder cancer, hepatoma, cervical cancer, esophageal cancer, pancreatic cancer, and prostate cancer. Various cancers can possess, and can sometimes release into the bloodstream, nucleic acids with characteristics that are distinguishable from nucleic acids from non-cancerous healthy cells, such as, for example, epigenetic state and/or sequence variations, duplications and/or deletions. Such characteristics can, for example, be specific to a particular type of cancer. Accordingly, methods described herein sometimes provide an outcome based on determining the presence or absence of a particular marker, and sometimes an outcome is presence or absence of a particular type of condition (e.g., a particular type of cancer).

*Compositions*

Also provided herein are compositions for detecting the presence or absence of a genetic variation, comprising a mixture of a plurality of amplification primer pairs that can amplify the plurality of sets of paralogous polynucleotide species disclosed herein. In some embodiments, each primer of the plurality of amplification primer pairs is complementary to less than 20 positions in a human genome. In some embodiments, the amplification primers for each of the paralogous polynucleotide species sets produces two equal-sized amplicons with a size between 60 to 100 bp. In some embodiments, the amplification primers for each of the sets produces zero off-target amplification events allowing maximal 3-base pair mis-priming. In some embodiments, the amplification primers for each of the sets do not overlap with any annotated single nucleotide variant with greater than 1% minor allele frequency in dbSNP build 137.

In certain embodiments, certain molecules (e.g., nucleic acid primers, amplified DNA products, etc.) used in accordance with and/or provided by the invention comprise one or more detectable entities or moieties, i.e., such molecules are "labeled" with such entities or moieties.

Any of a wide variety of detectable agents can be used in the practice of the disclosure. Suitable detectable agents include, but are not limited to: various ligands, radionuclides; fluorescent dyes; chemiluminescent agents (such as, for example, acridinum esters, stabilized dioxetanes, and the like); bioluminescent agents; spectrally resolvable inorganic fluorescent semiconductors nanocrystals (i.e., quantum dots); microparticles; metal nanoparticles (e.g., gold, silver, copper, platinum, etc.); nanoclusters; paramagnetic metal ions; enzymes; colorimetric labels (such as, for example, dyes, colloidal gold, and the like); biotin; dioxigenin; haptens; and proteins for which antisera or monoclonal antibodies are available.

In some embodiments, the detectable moiety is biotin. Biotin can be bound to avidins (such as streptavidin), which are typically conjugated (directly or indirectly) to other moieties (e.g., fluorescent moieties) that are detectable themselves.

Below are described some non-limiting examples of some detectable moieties that may be used.

*Fluorescent dyes*

In certain embodiments, a detectable moiety is a fluorescent dye. Numerous known fluorescent dyes of a wide variety of chemical structures and physical characteristics are suitable for use in the practice of the disclosure. A fluorescent detectable moiety can be stimulated by a laser with the emitted light captured by a detector. The detector can be a charge-coupled device (CCD) or a confocal microscope, which records its intensity.

Suitable fluorescent dyes include, but are not limited to, fluorescein and fluorescein dyes (e.g., fluorescein isothiocyanine or FITC, naphthofluorescein, 4',5'-dichloro-2',7'-dimethoxyfluorescein, 6-carboxyfluorescein or FAM, etc.), carbocyanine, merocyanine, styryl dyes, oxonol dyes, phycoerythrin, erythrosin, eosin, rhodamine dyes (e.g., carboxytetramethyl-rhodamine or TAMRA, carboxyrhodamine 6G, carboxy-X-rhodamine (ROX), lissamine rhodamine B, rhodamine 6G, rhodamine Green, rhodamine Red, tetramethylrhodamine (TMR), etc.), coumarin and coumarin dyes (e.g., methoxycoumarin, dialkylaminocoumarin, hydroxycoumarin, aminomethylcoumarin (AMCA), etc.), Q-DOTS. Oregon Green Dyes (e.g., Oregon Green 488, Oregon Green 500, Oregon Green 514., etc.), Texas Red, Texas Red-X, SPECTRUM REDTM, SPECTRUM GREENTM, cyanine dyes (e.g., CY-3TM, CY-5TM, CY-3.5TM, CY5.5TM, etc.), ALEXA FLUORTM dyes (e.g., ALEXA FLUORTM 350, ALEXA FLUORTM 488, ALEXA FLUORTM 532, ALEXA FLUORTM 546, ALEXA FLUORTM 568, ALEXA FLUORTM 594, ALEXA FLUORTM 633, ALEXA FLUORTM 660, ALEXA FLUORTM 680, etc.), BODIPYTM dyes (e.g., BODIPYTM FL, BODIPYTM R6G, BODIPYTM TMR, BODIPYTM TR, BODIPYTM 530/550, BODIPYTM 558/568, BODIPYTM 564/570, BODIPYTM 576/589, BODIPYTM 581/591, BODIPYTM 630/650, BODIPYTM 650/665, etc.), IRDyes (e.g., IRD40, IRD 700, IRD 800, etc.), and the like. For more examples of suitable fluorescent dyes and methods for coupling fluorescent dyes to other chemical entities such as proteins and peptides, see, for example, "The Handbook of Fluorescent Probes and Research Products", 9th Ed., Molecular Probes, Inc., Eugene, OR. Favorable properties of fluorescent labeling agents include high molar absorption coefficient, high fluorescence quantum yield, and photostability. In some embodiments, labeling fluorophores exhibit absorption and emission wavelengths in the visible (i.e., between 400 and 750 nm) rather than in the ultraviolet range of the spectrum (i.e., lower than 400 nm).

A detectable moiety may include more than one chemical entity such as in fluorescent resonance energy transfer (FRET). Resonance transfer results an overall enhancement of the emission intensity. For instance, see Ju et. al. (1995) Proc. Nat'l Acad. Sci. (USA) 92:4347, the entire contents of which are herein incorporated by reference. To achieve resonance energy transfer, the first fluorescent molecule (the "donor" fluor) absorbs light and transfers it through the resonance of excited electrons to the second fluorescent molecule (the "acceptor" fluor). In one approach, both the donor and acceptor dyes can be linked together and attached to the oligo primer. Methods to link donor and acceptor dyes to a nucleic acid have been described, for example, in U.S. Pat. No. 5,945,526 to Lee et al., the entire contents of which are herein incorporated by reference. Donor/acceptor pairs of dyes that can be used include, for example, fluorescein/tetramethylrohdamine, IAEDANS/fluroescein, EDANS/DABCYL, fluorescein/fluorescein, BODIPY FL/BODIPY FL, and Fluorescein/ QSY 7 dye. See, e.g., U.S. Pat. No. 5,945,526 to Lee et al. Many of these dyes also are commercially available, for instance, from Molecular Probes Inc. (Eugene, Oreg.). Suitable donor fluorophores include 6-carboxyfluorescein (FAM), tetrachloro-6-carboxyfluorescein (TET), 2'-chloro-7'-phenyl-1,4-dichloro-6-carboxyfluorescein (VIC), and the like.

*Radioactive isotopes*

In certain embodiments, a detectable moiety is a radioactive isotope. For example, a molecule may be isotopically-labeled (i.e., may contain one or more atoms that have been replaced by an atom having an atomic mass or mass number different from the atomic mass or mass number usually found in nature) or an isotope may be attached to the molecule. Non-limiting examples of isotopes that can be incorporated into molecules include isotopes of hydrogen, carbon, fluorine, phosphorous, copper, gallium, yttrium, technetium, indium, iodine, rhenium, thallium, bismuth, astatine, samarium, and lutetium (i.e., 3H, 13C, 14C, 18F, 19F, 32P, 35S, 64Cu, 67Cu, 67Ga, 90Y, 99mTc, 111In, 1251, 1231, 1291, 1311, 1351, 186Re, 187Re, 201T1, 212Bi, 213Bi, 211At, 153Sm, 177Lu).

In some embodiments, signal amplification is achieved using labeled dendrimers as the detectable moiety (see, e.g., Physiol Genomics 3:93-99, 2000), the entire contents of which are herein incorporated by reference in their entirety. Fluorescently labeled dendrimers are available from Genisphere (Montvale, N.J.). These may be chemically conjugated to the oligonucleotide primers by methods

*Kits*

Also provided herein are kits for determining the presence of absence of a genetic variation. The kit comprises a mixture of a plurality of pairs of amplification primer pairs, wherein each pair amplifies a set of paralogous sequences disclosed herein. Each primer of the amplification primer pairs is complementary to less than 20 positions in a human genome. In certain embodiments, the plurality of amplification primer pairs are selected from the amplification primer pairs disclosed in Figure 8. In some embodiments, the kit comprises a DNA polymerase and optionally reagents for amplifying a plurality set of paralogous sequences.

In some embodiments, kits comprise paralog sequences and associated primers of interest. In some embodiments, kits are provided in a form of an array such as a microarray or a mutation panel. In some embodiments, provided kits further comprise reagents for carring out various detection methods described herein (e.g., sequencing, hybridization, etc.). For example, kits may optionally contain buffers, enzymes, containers and/or reagents for use in methods described herein.

In some embodiments, provided kits further comprise a control indicative of a healthy individual, e.g., a nucleic acid and/or protein sample from an individual who does not have the genetic variant, and/or genetic disease and/or syndrome of interest. Kits may also contain instructions on how to determine if an individual has the disease and/or syndrome of interest, or is at risk of developing the disease and/or syndrome of interest.

In some embodiments, provided is a computer readable medium encoding information corresponding to the biomarker of interest. Such computer readable medium may be included in a kit of the invention.

*Methods of Making Paralog Contig Assay Systems*

Also provided are methods of generating paralog assay systems. The method may comprise: identifying paralogous contigs on a first reference chromosome and a second target chromosome; extracting those sequences which are almost identical in sequence and that map to exactly two regions, one region on the first reference chromosome and one region on the second target chromosome; and merging the sequences to form paralog contigs.

In an embodiment, all potential paralogous contigs located on the second target chromosome (e.g., chromosome 21) and alternative autosomes (reference chromosomes) are identified *in silico* by sampling each target chromosome and reference chromosome at 10 base pair intervals in 100 base pair segments. Or, other similar intervals (e.g., 10, 15, 20 base pairs, etc.) and segments (e.g., 75, 100, 150, 200 base pairs) may be used. In an embodiment, each segment may then be aligned to the human reference genome (e.g., hg19, GRCh37). In an embodiment, the alignment may comprise using Bowtie version 2.1.0 [34]. Or, other alignment methods may be used. Segments which map to exactly two regions, one on the target chromosome and one on a reference chromosome, may then be extracted and the corresponding regions merged to obtain paralogous contigs. In certain embodiments, each paralogous pair may be globally aligned to locate nucleotide differences distinguishing target and reference paralogs and to obtain the consensus sequence. For example, in certain embodiments the sequences may be aligned using the Biostrings function 'pairwise Alignment' [32] in R version 3.0 [36]. Or, other alignment methods may be used.

The method may further comprise designing primers that amplify, but differentiate, the contigs from both the reference and the target chromosome. Primer design may include consideration of GC content, melting temperature, and multiplexing optimization. In an embodiment, to ensure that all primer pairs are specific to target and reference paralogs and to accommodate the short length of cfDNA, only the primer pairs that produce two equal-sized amplicons with a size between 60-100 bp are selected. In an embodiment, the designed primers may be filtered to ensure desired sequence properties and to reduce possible sources of variation in the context of multiplexed PCR. In certain embodiments, the final filtered set of paralog targeting primers satisfy at least some, if not all of the following selection criteria: (1) each primer pair is predicted to produce exactly two equal-sized amplicons between 60-100 bp *in silico*, (2) each primer pair is predicted to produce zero off-target amplification events allowing maximal 3-base mis-priming , (3) each individual primer sequence is complementary to less than 20 positions in the genome, (4) no primer overlaps with any annotated single nucleotide variant with >1% minor allele frequency (e.g., using dbSNP build 137 [39] or other similar analysis), (5) primers flank at least one position that distinguished target from reference paralogs, and (6) all independent amplicons are at least 100-bp apart.

Certain methods described herein may be performed in conjunction with methods described, for example in International Patent Application Publication No. WO2013/052913, International Patent Application Publication No. WO2013/052907, International Patent Application Publication No. WO2013/055817, International Patent Application Publication No. WO2013/109981, International Patent Application Publication No. WO2013/177086, International Patent Application Publication No. WO2013/192562, International Patent Application Publication No. WO2014/116598, International Patent Application Publication No. WO2014/055774, International Patent Application Publication No. WO2014/190286, International Patent Application Publication No. WO2014/205401, International Patent Application Publication No. WO2015/051163, International Patent Application Publication No. WO2015/138774, International Patent Application Publication No. WO2015/054080, International Patent Application Publication No. WO2015/183872, International Patent Application Publication No. WO2016/019042, and International Patent Application Publication No. WO 2016/057901, the entire content of each is incorporated herein by reference, including all text, tables, equations and drawings.

## EXAMPLES

The examples set forth below illustrate certain embodiments and do not limit the technology.

*Example 1: Targeted paralog sequencing*

The following example describes a method for NIPT based on a targeted sequencing strategy specifically enriching for paralogous motifs on chromosomes of interest for fetal aneuploidy detection, chromosome 18 and 21. In this study, targeted paralog assay libraries were prepared for 1334 total samples as part of both classification training and a blinded evaluation dataset. Of these, 480 samples were fully blinded with an unknown composition of fetal euploid and aneuploid samples. Using a conservative classification strategy, all fetal aneuploidy cases were correctly classified, resulting in 100% sensitivity and specificity for fetal chromosome 21 trisomies and 87.5% sensitivity and 100% specificity for fetal chromosome 18 trisomies. Notably, these results were achieved at less than 25% of the standard depth of sequencing for a single sample using genome-wide sequencing strategies for aneuploidy detection.

MATERIALS AND METHODS

*Identification and filtering of paralogous contigs*

All potential paralogous contigs located on either chromosome 18 or chromosome 21 (target chromosomes) and alternative autosomes (reference chromosomes) were identified *in silico* by sampling each target chromosome at 10 base pair intervals in 100 base pair segments. Each segment was aligned to the human reference genome (hg19, GRCh37) using Bowtie version 2.1.0 [34]. Segments which mapped to exactly two regions, one on the target chromosome and one on a reference chromosome, were extracted and the corresponding regions were merged to obtain paralogous contigs. Each paralogous pair was then globally aligned using the Biostrings function 'pairwiseAlignment' [32] in R version 3.0 [36] to locate nucleotide differences distinguishing target and reference paralogs and to obtain the consensus sequence.

*Design of multiplexed PCR primer sequences*

Primer 3 version 2.2.3 [37:38] was used to design PCR primer pairs surrounding each nucleotide that differentiates the target and reference paralogs. The primer design parameters were: primer GC content = 30-70%, primer size = 18-24 bp, optimal size = 20 bp, primer melting temperature = 52-64°C, optimal Tm = 60°C. All resulting primer pairs were aligned as paired-end reads to the reference genome (hg19, GRCh37) using Bowtie version 2.1.0 [34] and required perfect primer matching to the reference. To ensure that all primer pairs were specific to target and reference paralogs and to accommodate the short length of cfDNA, only the primer pairs that produced two equal-sized amplicons with a size between 60-100bp were selected.

The designed primers were then filtered to ensure desired sequence properties and to reduce possible sources of variation in the context of multiplexed PCR. The final filtered set of paralog targeting primers satisfied the following selection criteria: (1) each primer pair was predicted to produce exactly two equal-sized amplicons between 60-100bp *in silico*, (2) each primer pair was predicted to produce zero off-target amplification events allowing maximal 3-base mis-priming , (3) each individual primer sequence was complementary to less than 20 positions in the genome, (4) no primer overlapped with any annotated single nucleotide variant with >1% minor allele frequency in dbSNP build137 [39], (5) primers flanked at least one position that distinguished target from reference paralogs, and (6) all independent amplicons were at least 100-bp apart.

Additional PCR primers were designed to amplify selected chrY regions and single nucleotide polymorphisms (SNPs) for the determination of fetal sex, the amount of input DNA, and estimation of the fraction of fetal origin DNA, respectively. All primer specifications were similar to those described above with the exception of filter criteria specific to paralog state of amplicons.

To minimize undesired primer-primer interactions in the multiplexed PCR reaction, *in silico* multiplexing optimization was performed. The reverse complement of each primer sequence was locally aligned to all the other primers using the Biostrings function 'pairwiseAlignment' [35] in R version 3.0 [36] and a primer-specific alignment score was calculated. A "hub" assay, defined as the assay with the largest total alignment score, was identified and removed from the primer pool. Hub assays with the maximum predicted primer-primer alignment score were iteratively eliminated from the primer pool until the number of remaining assays reached a minimum remaining assay threshold of 450, 450, 10, and 150 assays for chromosome 21 paralogs, chromosome 18 paralogs, chromosome Y fetal sex assays, and SNPs, respectively.

*Sample acquisition and blood processing*

Whole blood (10 mL per aliquot) was collected and plasma separated as previously described [15]. Two aliquots of plasma were obtained from each individual and were processed independently.

*Nucleic acid extraction*

After blood processing, cfDNA was extracted using an automated system capable of extracting 96 samples in parallel using an optimized version of the manufacturer's protocol (AMPure XP; Beckman Coulter).

*Library preparation*

Library preparation was accomplished through a two-phase PCR-based targeted enrichment process. Extracted cfDNA was used as template for single-well, 1060-plex PCR reactions. Following amplification, samples were purified using AMPure XP magnetic beads (Beckman Coulter) in a semi-automated process implemented on Zephyr liquid handling platforms (Caliper LifeSciences). Purified samples were quantified as described previously [8]. Purified and normalized products were then used as template for a subsequent PCR reaction using partial sequencing adapter motifs introduced during multiplexed PCR as priming sites. Final library samples were then purified, quantified, and normalized as described above.

*Massively parallel sequencing*

Isomolar paralog-targeted libraries (n=96) were sequenced together on two lanes of a HiSeq Rapid V1 flowcell on a HiSeq2500 instrument (Illumina). Sequencing by synthesis was performed for 80 cycles followed by 8 additional cycles to read each sample index. Data were merged across both lanes of each flowcell prior to further processing.

*Sequencing metrics*

All BCL output files from the sequencing instrument were converted to FASTQ format and aligned to the human reference genome (hg19, GRCh37) using Bowtie version 2.1.0 [34]. Subsequent processing to evaluate paralog targeting library quality and chromosome 18 or chromosome 21 dosage was performed using custom scripts to calculate the following metrics:

*On-target rate*

On-target rate was calculated as the fraction of reads mapped to expected genomic coordinates out of all sequencing reads. On-target reads were additionally filtered to require that (1) read length ≥45 bp, (2) mapped position of the 5'-end of the read was within 5 bp of the 5'-end of a designated target region, and (3) that reads were not ambiguously aligned.

*Ambiguous reads*

Ambiguously aligned reads were defined as reads that could be aligned to more than one position with equivalent confidence (i.e. the best alignment and the second best alignment had the same alignment score). Ambiguously aligned reads may have arisen due to reference genome uncertainty in paralogous regions, unpredicted amplification targets for paralog primers, or process errors. All ambiguously aligned reads were removed from further analysis.

*Assay paralog ratio*

The paralog ratio of an assay was calculated as the ratio of target paralog read depth to reference paralog read depth. Ratios were logarithm-transformed to zero-center values for additional analyses.

*Assay MAD*

Assay median absolute deviation (MAD) was calculated in the base R package [36] as the median absolute deviation of logarithm-transformed assay ratios in euploid samples.

<u>Aneuploidy classification approaches</u>

*Z-score calculation*

To calculate the z-score of a selected assay, the logarithm-transformed paralog assay ratio was first z-scaled by offsetting by the median and scaling by the MAD values derived from reference euploid samples. The sample level z-score was then calculated by combining individual assay z-scores with the exclusion of outliers of z > 4*MAD or < -4MAD from the distribution center:

$$Z = \frac{\sum_{i=1}^{N} z_i}{\sqrt{N}},$$

where $z_i$ is the z-score of a non-outlier assay.

A variation to the above calculation is the weighted z-score, which was calculated by weighting individual non-outlier z-scores according to assay MAD or discriminatory power.

$$Z_W = \frac{\sum_{i=1}^{N} w_i z_i}{\sqrt{\sum_{i=1}^{N} w_i^2}},$$

where $w_i$ is the weight for each individual assay.

*Machine learning based aneuploidy classification*

An artificial neural network approach was additionally implemented for aneuploidy classification using R package neuralnet [40] in R version 3.0 [36]. Prior to training of classification models, all assays were first filtered to require a minimum of 150 reads and no observed copy number variant (CNV) indication. Potential batch-level process bias was removed by offsetting assay ratios by plate-specific medians. To reduce model complexity and avoid over fitting, principal component analysis (PCA) was applied to transform the data into principal coordinate space. Two to fifteen high dimension principal components were then used to train alternative classification models and three-fold cross validation was used to select the best classification model.

Fetal sex determination

Fetal sex was determined based on the proportion of sequencing reads aligning to chromosome Y, calculated as the total sequencing read depth of chromosome Y assays normalized by total depth of all assays. The chromosome Y percentage is expected to be elevated in male, relative to female, samples.

Estimation of the fraction of fetal fraction

The fraction of fetal (placental) DNA for each sample was calculated based on the sequencing read counts of the 150 SNP assays. All SNP assays with fewer than 100 sequencing reads were removed from analysis. Maternal homozygous and fetal heterozygous SNP loci were identified by k-means clustering implemented in the kmeans base function in R version 3.0 [36]. The fetal fraction was then estimated using the median observed frequency of all the identified SNPs with the above genotype. Estimates of fetal fraction for each sample were compared against estimates from whole-genome MaterniT21® PLUS laboratory developed test sequence data using published methods [41].

RESULTS

Blinded paralog assay performance evaluation

The performance of targeted sequencing of paralogs for fetal aneuploidy detection was evaluated in 768 maternal plasma DNA samples with results available from genome-wide sequencing using the MaterniT21® PLUS laboratory developed test. Of these samples, 288 were fetal euploid samples as determined by MaterniT21® PLUS results and provided a null distribution of assay performance parameters. An additional 480 samples were blinded, research consented samples containing unknown numbers of fetal chromosome 21 and chromosome 18 trisomies to determine assay performance.

Global sequencing metrics for performance evaluation samples are summarized in Table 1. 576 independent samples were processed in addition to the 768 samples described above for the purpose of assay selection and training of classification models. Data include a set of euploid samples to provide a null distribution of assay performance and a set of blinded fully blinded samples of varied fetal genotype. MatemiT21® PLUS Assay ground truth data were obtained for all included samples. Sequencing read depth of performance evaluation samples was slightly reduced relative to the assay selection and classification model training dataset. Similarly, assay variance was slightly increased; however, the paralog assay ratio remained constant between the two datasets.

TABLE 1.

| | Total Reads (millions) | Aligned reads (%) | On-target reads (%) |
|---|---|---|---|
| Presumed euploids (N=240) | 2.121 | 95.3 | 83.3 |
| Blinded samples (N=480) | 2.265 | 95.2 | 83.4 |

*Trisomy classification results*

Based on previous experimental and empirical observations, the following acceptance criteria were used for sample quality control: sequencing read counts >1.2 million, on-target rate >75%, and fetal DNA fraction >4%. Of the 480 blinded samples, two were excluded due to insufficient sequencing read counts and five samples were excluded due to insufficient on-target rate. Notably, on-target rates for the five on-target rate excluded samples ranged from 72-75% and could potentially have been classified; however, due to the lack of empirical data to determine a statistically significant threshold for on-target rate, a heuristic threshold was utilized. The exclusion of these seven samples left 473 total blinded samples for classification.

The union of z-scores and artificial neural network results were utilized to classify samples as fetal euploid, fetal trisomy 21, or fetal trisomy 18. All classifications were determined independently by two laboratory directors who evaluated the z-scores and artificial neural network results independently to provide curated classification results prior to sample unblinding. After laboratory director classification, z-scores and fetal fraction values of all putative fetal trisomy samples were used to create linear models for chromosomes 18 and 21. All putative non-trisomy samples with differences in expected z-scores and observed z-score that are within three times the MAD of the residual model distributions were reported without classification. In total, five samples classified by lab directors were reassigned as unclassified using this method. The final classification was thus based on a conservative classification strategy such that only concordant calls were reported. Classification results were considered with respect to MaterniT21® PLUS Assay results as ground truth.

The conservative classification scheme pursued here resulted in 41 of 473 blinded samples without unambiguous fetal aneuploidy classification results, or a "no-call" rate of 8.7%. Of these ambiguously classified samples, 8, 3, and 30 were identified as fetal trisomy 21, fetal trisomy 18, and fetal euploid cases, respectively; consequently, the set of samples without unambiguous fetal aneuploidy classification results was enriched for 26.8% fetal trisomy cases versus 12.3% fetal trisomy cases in the study population. For samples with fetal aneuploidy classification results, the union of all statistical metrics provided perfectly concordant classification results for fetal trisomy 21 samples with zero false positive or false negative samples (Table 2 - Fetal aneuploidy classification results). Of the eight samples defined by the MaterniT21® PLUS LDT as fetal trisomy 21 cases that could not be unambiguously classified using the targeted paralog strategy described here, six had a relatively low estimated fetal fraction (<8%), which would be expected to reduce signal, but were otherwise undistinguished; the remaining two samples without classification results were flagged due to ambiguity in the fetal trisomy 18 classification and might have otherwise been correctly classified. Similarly, the classification of fetal trisomy 18 samples yielded zero false positives, but two false negatives which both had marginally low fetal fraction (<8%) and average z-score classification results (2.45-2.47). Classification results are summarized in Figure 2.

TABLE 2.

| Targeted Paralog Result | | MaterniT21® PLUS Result | | |
|---|---|---|---|---|
| SAMPLE ACCEPTANCE | Total | Euploid | T18 | T21 |
| Failed fetal DNA fraction | 0 | 0 | 0 | 0 |
| Failed read count | 2 | 2 | 0 | 0 |
| Failed on-target rate | 5 | 3 | 1 | 1 |
| Total Samples | 480 | 420 | 20 | 40 |
| Accepted samples | 473 | 415 | 19 | 39 |
| SAMPLE CLASSIFICATION | Total | Euploid | T18 | T21 |
| Ambiguous Chr 21 and 18 | 2 | 1 | 0 | 1 |
| Ambiguous Chr 21 | 25 | 19 | 0 | 6 |
| Ambiguous Chr 18 | 14 | 10 | 3 | 1 |
| Fetal Chr 21 trisomy | 31 | 0 | 0 | 31 |
| Fetal Chr 18 trisomy | 14 | 0 | 14 | 0 |
| Fetal euploid | 387 | 385 | 2 | 0 |

The reduced sensitivity in classification of fetal trisomy 18 relative to fetal trisomy 21 may be due to increased noise observed in chromosome 18 assays and inadequately trained classification models for fetal trisomy 18 detection due to the small training set sample size. In comparison, MaterniT21® PLUS Assay results yielded z-scores of 10.66 and 13.99 for the two abovementioned unclassified samples and the overall z-scores were higher from the MaterniT21 ® PLUS LDT than those generated from targeted paralog assays, representative of a larger classification metric gap between affected and unaffected samples when using a genome-wide approach (Figure 2). These observations illustrate the challenges with sub-genomic targeted approaches compared to a genome-wide sequencing strategy for noninvasive fetal aneuploidy classification.

*Fetal sex determination*

Chromosome Y representation was transformed into a standard normal distribution by z-scaling. The offset and scaling factors were set by the flowcell specific median and MAD of chromosome Y sequencing read representation of all samples <0.07%, respectively. Of the 473 total blinded samples passing acceptance criteria, one discordant fetal sex classification and four ambiguous fetal sex classifications based on z-scores were observed (Table 3- Fetal sex classification results). The sample with discordant fetal sex classification was classified as unreportable based on discordant z-score and risk-score trisomy classification results. Summary of classification results with respect to MaterniT21 ® PLUS. Reported results are derived from a heuristic threshold for percentage of sequencing reads mapped to chromosome Y assays. All four unclassified fetal sex samples had fetal fraction <7.5% (Figure 3).

TABLE 3.

| Targeted Paralog Result | | MaterniT21® PLUS Result | |
|---|---|---|---|
| SAMPLE ACCEPTANCE | Total | Male fetus | Female fetus |
| Failed fetal DNA fraction | 0 | 0 | 0 |
| Failed read count | 2 | 2 | 0 |
| Failed on-target rate | 5 | 1 | 4 |
| Total Samples | 480 | 240 | 240 |
| Accepted samples | 473 | 237 | 236 |
| SAMPLE CLASSIFICATION | Total | Male fetus | Female fetus |
| Ambiguous fetal sex | 4 | 4 | 0 |
| Male fetus | 234 | 233 | 1 |
| Female fetus | 235 | 0 | 235 |

*Reproducibility of targeted paralog based fetal aneuploidy detection*

The blinded sample targeted paralog libraries were re-sequenced to test the reproducibility of observed results and to evaluate the impact of variable sequencing depth on assay performance. The original experiment yielded a mean of 2.2 million sequencing reads per sample while the re-sequenced experiment yielded a mean of 1.27 million sequencing reads with similar on-target rates of 83%. Chromosome Y fraction was highly concordant between the two sequencing experiments ($r^2 = 0.99$, $p<10^{-15}$, Figure 4A). A similarly high concordance of estimated fraction of fetal origin DNA was observed ($r^2 = 0.97$, $p<10^{-15}$, Figure 4B). In both of the above cases, discordant data points can be attributed to QC failures due to insufficient read count and/or on-target rate. Finally, the calculated chromosome 21 and chromosome 18 z-scores show high concordance from experiment to experiment (Chromosome 21: $r^2 = 0.87$, $p<10^{-15}$, Figure 4C. Chromosome 18: $r^2 = 0.75$, $p<10^{-15}$, Figure 4D). Jointly, these results suggest that assay performance is robust to variation in sequencing performance.

DISCUSSION

This study applied a targeted sequencing strategy specifically enriching for paralogous motifs anchored on chromosomes of interest (chromosomes 18 and 21) for fetal aneuploidy detection.

In this study, targeted paralog assay libraries for 1334 total samples were prepared as part of both classification training and a blinded evaluation dataset. Of these, 480 samples were fully blinded with an unknown composition of fetal euploid and aneuploid samples. Using a conservative classification strategy, all fetal aneuploidy cases were correctly classified, resulting in 100% sensitivity and specificity for fetal chromosome 21 trisomies and 87.5% sensitivity and 100% specificity for fetal chromosome 18 trisomies. Notably, these results were achieved at less than 25% of the standard depth of sequencing for a single sample using genome-wide sequencing strategies for aneuploidy detection. However, an unambiguous classification result was returned for only 432 of 480 fully blinded samples, reflecting a combined 10% QC failure and ambiguous classification rate. While this result is similar to some other published methods for targeted sequencing based non-invasive fetal aneuploidy detection [14, 42], it remains an order of magnitude higher than published genome-wide sequencing fetal aneuploidy detection strategies [12].

The targeted paralog sequencing strategy described here offers several distinct advantages for non-invasive fetal aneuploidy detection. First and foremost, the sequencing requirements and cost per sample are reduced relative to a genome-wide sequencing assay. Further, the workflow for preparation and sequencing of such libraries is amenable to full automation, potentially facilitating extremely high-throughput screening of cfDNA samples in a clinical laboratory environment. Finally, paralog ratio testing provides additional content relative to simple counting metrics, is a more direct strategy than SNP-based testing, and offers the capacity to effectively self-normalize each targeted paralog through the calculation of locus-specific paralog ratios to reduce some of the noise inherent in sequencing datasets.

The assay described herein specifically enriches for paralog targets used for fetal aneuploidy detection in maternal cfDNA. This assay was found to have high sensitivity and specificity for fetal chromosome 18 and 21 trisomies, but to suffer from a relatively high rate of ambiguous classification. These data demonstrate that a target enrichment strategy selecting for paralogous motifs in cfDNA samples is technically feasible for high-throughput classification of fetal aneuploidies from maternal blood samples, correctly classifying >95% of all fetal aneuploidies tested with no false positive results observed.

*Example 2: Assay selection and classification model training*

The performance of all available paralog assays was first assessed in a training data set consisting of NGS libraries previously prepared from 341 fetal euploid, 200 fetal chromosome 21 triploid, and 35 fetal chromosome 18 triploid cfDNA samples. The objectives for these data were four-fold: (1) to evaluate the ability to determine fetal sex, (2) to evaluate the capacity to estimate fetal fraction, (3) to select the best performing assays, and (4) to train fetal aneuploidy classification models. Global sequencing metrics for samples included in the assay selection and classification model training set are included in Table 4. Data include model training set with MaterniT21® PLUS Assay ground truth data known through the course of data analysis.

Table 4

| | Total Reads (millions) | Aligned reads (%) | On-target reads (%) |
|---|---|---|---|
| Model Training (N=576) | 2.892 | 93.7 | 72.4 |

*Fetal sex determination*

A small fraction of total assays designed were targeted to chromosome Y; for each sample, the percentage of total sequencing reads mapping to chromosome Y was calculated using assays uniquely targeting chromosome Y regions. Good separation between male and female samples were observed (Figure 5). The percentage of chromosome Y reads from male fetus samples was >0.0435% and the percentage of chromosome Y sequencing reads from female fetus samples was <0.0159% with one discordant case.

*Relative quantitation of fetal DNA*

Fetal fraction in each sample were estimated using allele frequencies measured from a panel of 150 segregating SNP loci and compared to an estimation method based on whole genome sequence data [41] from independent aliquots of each sample processed using the MaterniT21® PLUS Assay (Sequenom). Two samples exhibited outlier behavior and displayed high variance in the SNP loci fetal fraction estimation. Nevertheless, estimation of relative abundance of fetal DNA in all samples was highly correlated with the orthogonal whole-genome sequence based estimate ($r^2 = 0.82$, $p<10^{-15}$, F-test, Figure 6).

*Z-score classification*

At minimum, optimal assays for aneuploidy classification require sufficient depth of sequencing coverage, small variance expressed as assay MAD, and measurable signal differentiating aneuploid and euploid populations as measured by the p-value from the Wilcoxon rank sum test on log-transformed assay ratios. Using a grid search approach, the optimal assay sets were determined independently for z-score based fetal trisomy 21 and trisomy 18 classification. Specifically, 305 chromosome 21 assays with coverage > 700, MAD < 0.28, p-value < 0.1, and no evidence for CNV were identified as the optimal feature set for z-score based fetal trisomy 21 classification. The optimal thresholds for chromosome 18 assays were relaxed due to the small number of training chromosome 18 fetal trisomy samples. 296 chromosome 18 assays with coverage>200, MAD<0.3 and p-value<0.3 were selected as the optimal feature set for z-score based fetal trisomy 18 classification. The specific chromosome 21 and chromosome 18 assays utilized for classification are designated in Fig. 8 by a "+" in the last column of the table (included in final).

Optimal features were used to calculate z-scores for all training set samples. A trisomy sample was identified when the z-score exceeded 4 for chromosome 21 or 4.5 for chromosome 18; no positive or negative classification of fetal trisomy could be made when the z-score was between 2.5 and 4.0 for chromosome 21 or between 3.0 and 4.5 for chromosome 18. The resulting filtered training data were validated against independent aliquots of each sample processed using the MaterniT21® PLUS Assay (Sequenom) and suggested sensitivity >96% and specificity >99% for fetal aneuploidy detection with indeterminate results in <3% of samples could be achieved (Figure 7). Several variations of the z-score based classification methods were also evaluated. These included weighting z-scores by assay variance or discriminatory power and merging assays based on physical location and assay correlations prior to z-score calculation. These alternative z-score based classification methods yield, at best similar performance to the standard z-score method described and were not pursued further.

*Artificial neural network based classification*

Implementation of artificial neural networks with seven principal components for aneuploidy classification yielded the maximum sensitivity and specificity among all of the models tested, at 97% and 99% predicted, respectively. Using artificial neural network classification, samples containing 5-10% fetal DNA were ambiguously assigned; consequently, no positive or negative classification of fetal trisomy could be made when the artificial neural network regression model was between 0.05 and 0.95.

*Classification model performance*

The final optimal classification of fetal aneuploidies employed the union of z-score and artificial neural network results to identify fetal trisomies only when both approaches were concordant. Fetal aneuploidy classification results for classification model training are shown in Table 5. Classification model training data were used to establish heuristic thresholds for subsequent blinded classification. Classifications herein therefore represent best case classification scenarios with respect to MaterniT21® PLUS Assay ground truth data. Seventeen samples with estimated fetal (placental) origin DNA fraction <4% and ten samples for which identity could not be unambiguously assigned were excluded from the final analysis. No classification could be made in 5% and 3.6% of fetal trisomy 21 and fetal trisomy 18 cases, respectively. All other samples could be unambiguously and correctly classified in the model training dataset (Table 5).

Table 5

| Targeted Paralog Result | | MaterniT21 ® PLUS Result | | |
|---|---|---|---|---|
| SAMPLE ACCEPTANCE | Total | Euploid | T18 | T21 |
| Failed fetal DNA fraction | 17 | 10 | 4 | 3 |
| Total Samples | 566 | 341 | 31 | 194 |
| Accepted samples | 549 | 331 | 27 | 191 |
| T21 SAMPLE CLASSIFICATION | Total | Euploid | T18 | T21 |
| Ambiguous classification | 26 | 14 | 0 | 12 |
| Discordant classification | 2 | 1 | 0 | 1 |
| Concordant classification | 521 | 316 | 27 | 178 |
| T18 SAMPLE CLASSIFICATION | Total | Euploid | T18 | T21 |
| Ambiguous classification | 19 | 11 | 5 | 3 |
| Discordant classification | 1 | 1 | 0 | 0 |
| Concordant classification | 529 | 319 | 22 | 188 |

REFERENCES

1. Lo YM, Corbetta N, Chamberlain PF, Rai V, Sargent IL, et al. (1997) Presence of fetal DNA in maternal plasma and serum. Lancet 350: 485-487. doi: 10.1016/s0140-6736(97)02174-0
2. Lo YM, Tein MS, Lau TK, Haines CJ, Leung TN, et al. (1998) Quantitative analysis of fetal DNA in maternal plasma and serum: implications for noninvasive prenatal diagnosis. Am J Hum Genet 62: 768-775. doi: 10.1086/301800
3. Nygren AO, Dean J, Jensen TJ, Kruse S, Kwong W, et al. (2010) Quantification of fetal DNA by use of methylation-based DNA discrimination. Clin Chem 56: 1627-1635. doi: 10.1373/clinchem.2010.146290
4. Lo YM, Fun FM, Chan KC, Tsui NB, Chong KC, et al. (2007) Digital PCR for the molecular detection of fetal chromosomal aneuploidy. Proc Natl Acad Sci USA 104:13116-13121. doi: 10.1073/pnas.0705765104
5. Fan HC, Quake SR (2007) Detection of aneuploidy with digital polymerase chain reaction. Anal Chem 79:7576-7579. doi: 10.1021/ac0709394
6. Chiu RW, Chan KC, Gao Y, Lau VY, Zheng W, et al. (2008) Noninvasive prenatal diagnosis of fetal chromosomal aneuploidy by massively parallel genomic sequencing of DNA in maternal plasma. Proc Natl Acad Sci U S A 105: 20458-20463. doi: 10.1073/pnas.0810641105
7. Fan HC, Blumenfeld YJ, Chitkara U, Hudgins L, Quake SR (2008) Noninvasive diagnosis of fetal aneuploidy by shotgun sequencing DNA from maternal blood. Proc Natl Acad Sci U S A 105: 16266-16271. doi: 10.1073/pnas.0808319105

8. Ehrich M, Deciu C, Zwiefelhofer T, Tynan JA, Cagasan L, et al. (2011) Noninvasive detection of fetal trisomy 21 by sequencing of DNA in maternal blood: a study in a clinical setting. Am J Obstet Gynecol 204: 205 e1-11. doi: 10.1016/j.ajog.2010.12.060

9. Palomaki GE, Kloza EM, Lambert-Messerlian GM, Haddow JE, Neveux LM, et al. (2011) DNA sequencing of maternal plasma to detect Down syndrome: An international clinical validation study. Genet Med 13: 913-920. doi: 10.1097/gim.0b013e3182368a0e

10. Chiu RW, Akolekar R, Zheng YW, Leung TY, Sun H, et al. (2011) Non-invasive prenatal assessment of trisomy 21 by multiplexed maternal plasma DNA sequencing: large scale validity study. BMJ 342: c7401. doi: http://dx.doi.org/10.1136/bmj.c7401

11. Sehnert AJ, Rhees B, Comstock D, de Feo E, Heilek G, et al. (2011) Optimal detection of fetal chromosomal abnormalities by massively parallel DNA sequencing of cell-free fetal DNA from maternal blood. Clin Chem doi:10.1373/clinchem.2011.165910.

12. Palomaki GE, Deciu C, Kloza EM, Lambert-Messerlian GM, Haddow JE, et al. (2012) DNA sequencing of maternal plasma reliably identifies trisomy 18 and trisomy 13 as well as Down syndrome: an international collaborative study. Genet Med 14: 296-305. doi: 10.1038/gim.2011.73

13. Dan S, Wang W, Ren J, Li Y, Hu H, Xu Z, et al. (2012) Clinical application of massively parallel sequencing-based prenatal noninvasive fetal trisomy test for trisomies 21 and 18 in 11,105 pregnancies with mixed risk factors. Prenat Diagn 232: 1225-32.

14. Bianchi DW, Platt LD, Goldberg JD, Abuhamad AZ, Sehnert AJ, et al. (2012) Genome-wide fetal aneuploidy detection by maternal plasma DNA sequencing. Obstet Gynecol 119: 890-901. doi: 10.1002/pd.4002

15. Jensen TJ, Zwiefelhofer T, Tim RC, Džakula Z, Kim SK, et al. (2013) High-throughput massively parallel sequencing for fetal aneuploidy detection from maternal plasma. PLoS ONE 8: e57381. doi: 10.1371/journal.pone.0057381

16. Gil MM, Akolekar R, Quezada MS, Bregant B, Nicolaides KH (2014) Analysis of cell-free DNA in maternal blood in screening for aneuploidies: meta-analysis. Fetal Diagn Ther 35:156-173. doi: 10.1002/uog.14791

17. Dondorp W, deWert G, Bombard Y, Bianchi DW, Bergmann C, et al. (2015) Non-invasive prenatal testing for aneuploidy and beyond: challenges of responsible innovation in prenatal testing. European J Human Genetics 23(11):1438-1450. DOI: 10.1038/ejhg.2015.57

18. Fan HC, Quake SR (2010) Sensitivity of noninvasive prenatal detection of fetal aneuploidy from maternal plasma using shotgun sequencing is limited only by counting statistics. PLoS ONE 5: e10439. doi: 10.1371/journal.pone.0010439

19. Zhao C, Tynan J, Ehrich M, Hannum G, McCullough R, et al. (2015) Detection of fetal subchromosomal abnormalities by sequencing circulating cell-free DNA from maternal plasma. Clin Chem 61(4):608-616. doi: 10.1373/clinchem.2014.233312

20. Bianchi DW, Wilkins-Haug L (2014) Integration of noninvasive DNA testing for aneuploidy into prenatal care: What has happened since the rubber met the road? Clin Chem 60:78-87. doi: 10.1373/clinchem.2013.202663

21. Sparks AB, Wang ET, Struble CA, Barrett W, Stokowski R, et al. (2012) Selective analysis of cell-free DNA in maternal blood for evaluation of fetal trisomy. Prenat Diagn 32: 3-9. doi: 10.1002/pd.2922

22. Sparks AB, Struble CA, Wang ET, Song K, Oliphant A. (2012) Noninvasive prenatal detection and selective analysis of cell-free DNA obtained from maternal blood: evaluation for trisomy 21 and trisomy 18. Am J Obstet Gynecol 206: 319.e1-9. doi: 10.1016/j.ajog.2012.01.030

23. Ashoor G, Syngelaki A, Wagner M, Birdir C, Nicolaides KH. (2012) Chromosome-selective sequencing of maternal plasma cell-free DNA for first-trimester detection of trisomy 21 and trisomy 18. Am J Obstet Gynecol 206:322.e1-5. doi: 10.1016/j.ajog.2012.01.029

24. Zimmerman B, Hill M, Gemelos G, Demko Z, Banjevic M, et al. (2012) Noninvasive prenatal aneuploidy testing of chromosomes 13, 18, 21, X, and Y using targeted sequencing of polymorphic loci. Prenat Diagn 32:1233-1231. doi: 10.1002/pd.3993

25. Norton ME, Brar H, Weiss J, Karimi A, Laurent LC, et al. (2012) Non-Invasive Chromosomal Evaluation (NICE) Study: results of a multicenter prospective cohort study for detection of fetal trisomy 21 and trisomy 18. Am J Obstet Gynecol 207: 137 e1-8. doi: 10.1016/j.ajog.2012.05.021

26. Nicolaides KH, Syngelaki A, Gil M, Atanasova V, Markova D (2013) Validation of targeted sequencing of single-nucleotide polymorphisms for non-invasive prenatal detection of aneuploidy of chromosomes 13, 18, 21, X, and Y. Prenat Diagn 33:575-579. doi: 10.1002/pd.4103

27. Norton ME, Jacobsson B, Swarmy GK, Laurent LC, Ranzini AC, et al. (2015) Cell-free DNA analysis for noninvasive examination of trisomy. New Eng J Med doi: 10.1056/NEJMoa 1407349

28. Redon R, Ishikawa S, Fitch KR, Feuk L, Perry GH, et al. (2006) Global variation in copy number in the human genome. Nature 444:444-454. doi:10.1038/nature05329
29. McCarroll SA, Altshuler DM (2007) Copy-number variation and association studies of human disease. Nat Genet 39: S37-S42. doi:10.1038/ng2080
30. Walker S, Janyakhantikul S, Armour JAL (2008) Multiplex paralogue ratio tests for accurate measurement of multiallelic CNVs. Genomics 93: 98-103 doi:10.1016/j.ygeno.2008.09.004
31. Ohno S (1970) Evolution by gene duplication. Springer, New York. ISBN 3642866611
32. Deutsch, S, Choudhury U, Merla G, Howald C, Sylvan A, et al. (2004) Detection of aneuploidies by paralogous sequence quantification. J Med Genet 41:908-915. doi:10.1 136/jmg.2004.023184
33. Howald C, Merla G, Digilio MC, Amenta S, Lyle R, et al. (2006) Two high throughput technologies to detect aneuploidies identify new Williams-Beuren syndrome patients with atypical deletions. J Med Genet 43: 266-273. doi: 10.1136/jmg.2005.034009
34. Langmead B, Salzberg S (2012) Fast gapped-read alignment with Bowtie 2. Nature Methods 9:357-359. doi: 10.1038/nmeth.1923
35. Pages H, Aboyoun P, Gentleman R, DebRoy S (). Biostrings: String objects representing biological sequences, and matching algorithms. R package version 2.29.0.
36. R Core Team (2013). R: A language and environment for statistical computing. R Foundation for Statistical Computing, Vienna, Austria. ISBN 3-900051-07-0, URL http://www.R-project.org/.
37. Untergrasser A, Cutcutache I, Koressaar T, Ye J, Faircloth BC, Remm M, Rozen SG (2012) Primer3 - new capabilities and interfaces. Nucleic Acids Research 40(15):e115. doi: 10.1093/nar/gks596
38. Koressaar T, Remm M (2007) Enhancements and modifications of primer design program Primer3. Bioinformatics 23(10):1289-91
39. Database of Single Nucleotide Polymorphisms (dbSNP). Bethesda (MD): National Center for Biotechnology Information, National Library of Medicine. (dbSNP Build ID:137)
40. Guenther F (2012) Package 'neuralnet'. R package version 1.32.
41. Kim SK, Hannum G, Geis J, Tynan J, Hogg G, et al. (2015) Determination of fetal DNA fraction from the plasma of pregnanat women using sequence read counts. Prenat Diagn 35(8):810-805. doi: 10.1002/pd.4615
42. Nicolaides KH, Syngelaki A, Ashoor G, Birdir C, Touzet G (2012) Noninvasive prenatal testing for fetal trisomies in a routinely screened first-trimester population. Am J Obstet Gynecol 207:374.e1-6. doi: 10.1016/j.ajog.2012.08.033
43. Chandrananda D, Thorne NP, Bahlo M. (2015) High-resolution characterization of sequence signatures due to non-random cleavage of cell-free DNA. BMC Med Genomics 8:29. doi: 10.1186/s12920-015-0107-z
44. Snyder MW, Kircher M, Hill AJ, Daza RM, Shendure J. (2016) Cell-free DNA comprises an in vivo nucleosome footprint that informs its tissues-of-origin. Cell 164:57-68. doi: 10.1016/j.cell/2015.11.050
45. Yu SCY, Chan KCA, Zheng YWL, Jiang P, Liao GJW, Sun H, Akolekar R, et al. (2014) Size-based molecular diagnostics using plasma DNA for noninvasive prenatal testing. Proc Natl Acad Sci 111(23): 8583-8588. doi/10.1073/pnas.
46. Lo YMD, Chan KCA, Sun H, Chen EZ, Jiang P, Lun FMF, et al (2010) Maternal Plasma DNA Sequencing Reveals the Genome-Wide Genetic and Mutational Profile of the Fetus. Sci Trans Med 2(61):61ra91. DOI: 10.1126/scitranslmed.3001720
47. Lanman RB, Mortimr SA, Zill OA, Sebisanovic D, Lopez R, Blau S, et al. (2015) Analytical and clinical validation of a digital sequencing panel for quantitative, highly accurate evaluation of cell-free circulating tumor DNA. PLoS ONE 10(10):e0140712. doi: 10.1371/journal.pone.0140712

*Example 3: Examples of certain embodiments*

Listed hereafter are non-limiting examples of certain embodiments of the technology.

1. A method for determining the presence or absence of a genetic variation, comprising:

a. amplifying in a single reaction a plurality of paralogous polynucleotide species from nucleic acid in a sample, comprising contacting the nucleic acid with amplification primers under amplification conditions, wherein the paralogous polynucleotide species of each of the sets are amplified by a single pair of amplification primers and each primer of the pair of amplification primers is complementary to less than 20 positions in a human genome;
b. determining the amount of each amplified paralogous polynucleotide species in each of the sets;
c. determining a paralog ratio for each of the sets between the amount of each amplified paralogous polynucleotide species in each of the sets, thereby generating a plurality of paralog ratios; and
d. determining the presence or absence of the genetic variation based on the plurality of paralog ratios.

2. The method of embodiment 1, wherein the nucleic acid is circulating cell free nucleic acid.

3. The method of embodiment 1 or 2, wherein the nucleic acid is from a pregnant female comprising fetally derived and maternally derived nucleic acid.

4. The method of any one of embodiments 1-3, wherein the paralogous polynucleotide species in each of the sets are present on two or more different chromosomes at different loci, comprising a target chromosome and one or more reference chromosomes not associated with the chromosomal aneuploidy.

5. The method of any one of embodiments 1-4, wherein the genetic variation is a copy number alteration.

6. The method of embodiment 5, wherein the genetic variation is a single nucleotide variation.

7. The method of any one of embodiments 1-5, wherein the genetic variation is aneuploidy.

8. The method of any one of embodiments 1-7, wherein the plurality of sets of paralogous polynucleotide species comprises at least 100 sets.

9. The method of embodiment 4, wherein determining a paralog ratio in (c) is between (i) the .amount of amplified paralogous polynucleotide species from a target chromosome, and (ii) the amount of amplified paralogous polynucleotide species from a reference chromosome.

10. The method of any one of embodiments 1 to 9, wherein the paralogous polynucleotide species in each of the sets have primer hybridization sequences with a degree of sequence similarity that a single pair of amplification primers hybridizes to the paralogous polynucleotide species of each of the sets.

11. The method of any one of embodiments 1 to 10, wherein the paralogous polynucleotide species in each of the sets differ by one or more mismatch nucleotides.

12. The method of embodiment 11, wherein the determining the amount of each amplified paralogous polynucleotide species in each of the sets is by detecting the one or more mismatch nucleotides.

13. The method of any one of embodiments 1 to 12, wherein amplifying in a single reaction in (a) is at least 100, 250, or 500 sets of paralogous polynucleotide species.

14. The method of any one of embodiments 1 to 13, comprising determining fetal sex by a process comprising contacting the circulating cell free nucleic acid in the single reaction in (a) with primers specific for Y-chromosome polynucleotides under amplification conditions and amplifying Y-chromosome polynucleotides.

15. The method of embodiment 14, wherein there are at least 10 Y-chromosome polynucleotides are amplified.

16. The method of any one of embodiments 1 to 15, comprising determining fetal fraction by a process comprising contacting the circulating cell free nucleic acid in the single reaction in (a) with primers specific for polynucleotides flanking or comprising single nucleotide polymorphic (SNP) loci under amplification conditions, and amplifying polynucleotides containing the SNP loci.

17. The method of embodiment 16, wherein there are at least 150 polynucleotides comprising single nucleotide polymorphic (SNP) loci that are amplified.

18. The method of any one of embodiments 4 to 17, wherein the target chromosome is chromosome 21 and the reference chromosome is an autosome other than chromosome 21.

19. The method of any one of embodiments 4 to 17, wherein the target chromosome is chromosome 18 and the reference chromosome is an autosome other than chromosome

20. The method of any one of embodiments 4 to 17, wherein the sets of paralogous polynucleotide species comprise sets wherein the target chromosome is chromosome 21 and sets wherein the target chromosome is chromosome 18.

21. The method of embodiment 20, wherein there are at least 250 sets of paralogous polynucleotide species for target chromosome 21 and there are at least 250 sets of paralogous polynucleotide species for target chromosome 18.

22. The method of embodiment 21, wherein there are at least 500 sets of paralogous polynucleotide species for target chromosome 21 and there are at least 500 sets of paralogous polynucleotide species for target chromosome 18.

23. The method of any one of embodiments 20 to 22, comprising amplifying Y-chromosome polynucleotides and amplifying polynucleotides comprising single nucleotide polymorphic (SNP) loci.

24. The method of embodiment 23, wherein there are at least 10 Y-chromosome polynucleotides that are amplified and at least 150 polynucleotides comprising single nucleotide polymorphic (SNP) loci that are amplified.

25. The method of any one of embodiments 1 to 24, wherein the amplification primers for each of the paralogous polynucleotide species sets produces two equal-sized amplicons with a size between 60 to 100 bp.

26. The method of any one of embodiments 1 to 25, wherein the amplification primers for each of the sets produces zero off-target amplification events allowing maximal 3-base pair mis-priming.

27. The method of any one of embodiments 1 to 26, wherein the amplification primers for each of the sets do not overlap with any annotated single nucleotide variant with greater than 1% minor allele frequency in dbSNP build137.

28. The method of any one of embodiments 1 to 27, wherein the amplification primers for each of the sets flank at least one position where the nucleotide sequence species in a set differ by one or more mismatch nucleotides.

29. The method of any one of embodiments 1 to 28, wherein the amplification primers for each of the sets amplify a paralogous polynucleotide species set that is at least 100-bp apart from each of the other sets.

30. The method of any one of embodiments 1 to 29, wherein after (a) each of the amplified sets of paralogous polynucleotide species are further amplified by universal PCR.
31. The method of any one of embodiments 1 to 30, comprising generating sequence reads from each of the amplified paralogous polynucleotide species sets by a sequencing process.
32. The method of embodiment 31, wherein the sequence reads are filtered for a minimum number of reads and no copy number variants.
33. The method of embodiment 32, wherein the sequence reads are quantified to obtain counts.
34. The method of embodiment 33, wherein determining a paralog ratio in (c) is calculated as the ratio of the counts of the paralogous polynucleotide species on a target chromosome to the counts of the paralogous polynucleotide species on a reference chromosome.
35. The method of embodiment 33, wherein the paralog ratio is logarithm-transformed to zero-center values.
36. The method of any one of embodiments 1 to 35, comprising determining a statistic for each of the paralog ratios or logarithm-transformed paralog ratios.
37. The method of embodiment 36, wherein the statistic is a z-score.
38. The method of embodiment 37, wherein the z-score is a quotient of (a) subtraction product of (i) the logarithm-transformed paralog ratio, less (ii) a median of the logarithm-transformed paralog ratio of reference euploid samples, divided by (b) a MAD value derived from reference euploid samples.
39. The method of embodiment 36, wherein the statistic is a weighted z-score.
40. The method of embodiment 39, wherein the weighted z-score is calculated by weighting individual non-outlier z-scores paralog ratios according to assay MAD or discriminatory power.
41. The method of embodiment 36, comprising determining a sample statistic according to the statistic for each of the paralog ratios or logarithm-transformed paralog ratios.
42. The method of embodiment 41, comprising summing statistics for each of the sets.
43. The method of embodiment 41 or 42, wherein a sample z-score is calculated by z-scores of a plurality paralogous polynucleotide species sets, with the exclusion of outliers of z greater than 4 MAD or less than -4 MAD.
44. The method of embodiment 41 or 42, comprising determining a sample z-score according to

$$Z = \frac{\sum_{i=1}^{N} z_i}{\sqrt{N}},$$

where $z_i$ is the z-score of a non-outlier paralog ratio and N is the number of non-outlier paralogs.
45. The method of embodiment 41 or 42, wherein a sample z-score is calculated by weighted z-scores of a plurality paralogous polynucleotide species sets.
46. The method of embodiment 41 or 42, comprising determining a sample weighted z-score according to

$$Z_w = \frac{\sum_{i=1}^{N} w_i z_i}{\sqrt{\sum_{i=1}^{N} w_i^2}},$$

where $w_i$ is the weight for each paralog ratio.
47. The method of any one of embodiments 36 to 46, wherein a classification is generated for the presence or absence of a chromosomal aneuploidy according to the statistics.
48. The method of embodiment 47, wherein the statistic is z-score.
49. The method of any one of embodiments 1 to 47, wherein the paralogous polynucleotide species sets comprise one or more of the sets in Figure 8.
50. The method of any one of embodiments 1 to 49, wherein the amplification primers comprise one or more pairs of the amplification primers in Figure 8.
51. The method of any one of embodiments 1-2, 5-6, 8-13, and 25-50, wherein the genetic variation is cancer.
52. The method of any one of the embodiments 1-2, 5-6, 8-13, and 25-50, wherein the genetic variation is an inherited mutation.
53. The method of any one of embodiments 1-2, 5-6, 8-13, and 25-50, wherein the genetic variation is a somatic mutation.
54. A composition comprising a mixture of a plurality of amplification primer pairs that can amplify the plurality of sets of paralogous polynucleotide species of any of embodiments 1-54, wherein the each primer of the plurality of amplification primer pairs is complementary to less than 20 positions in a human genome,

wherein the amplification primers for each of the paralogous polynucleotide species sets produces two equal-sized amplicons with a size between 60 to 100 bp;
wherein the amplification primers for each of the sets produces zero off-target amplification events allowing maximal 3-base pair mis-priming; and/or
wherein the amplification primers for each of the sets do not overlap with any annotated single nucleotide variant with greater than 1% minor allele frequency in dbSNP build137.

55. The composition of embodiment 54, wherein the composition comprises a plurality of amplification primer pairs in Figure 8.
56. A kit comprising the composition of any of embodiments 54- 55 and a DNA polymerase.
57. A system for determining the presence or absence of a genetic variation, comprising

a) a component for amplifying in a single reaction a plurality of paralogous polynucleotide species from nucleic acid in a sample, comprising contacting the nucleic acid with amplification primers under amplification conditions, wherein the paralogous polynucleotide species of each of the sets are amplified by a single pair of amplification primers and each primer of the pair of amplification primers is complementary to less than 20 positions in a human genome;
b) a component for determining the amount of each amplified paralogous polynucleotide species in each of the sets;
c) a component for determining a paralog ratio for each of the sets between the amount of each amplified paralogous polynucleotide species in each of the sets, thereby generating a plurality of paralog ratios; and
d) a component for determining the presence or absence of the genetic variation based on the plurality of paralog ratios.

58. The system of embodiment 57, wherein one or more of components b)-d) comprise a computer processor.
59. A method of generating paralog assay systems comprising: identifying paraolgous contigs on a first reference chromosome and a second target chromosome; extracting those sequences which are substantially identical in sequence and that map to exactly two regions, one region on the first reference chromosome and one region on the second target chromosome; and merging the sequences in each region to form paralog contigs.
60. The method of embodiment 59, further comprising designing primers that amplify, but differentiate, the contigs from both the reference and the target chromosome.

The entirety of each patent, patent application, publication and document referenced herein hereby is incorporated by reference. Citation of the above patents, patent applications, publications and documents is not an admission that any of the foregoing is pertinent prior art, nor does it constitute any admission as to the contents or date of these publications or documents. Their citation is not an indication of a search for relevant disclosures. All statements regarding the date(s) or contents of the documents is based on available information and is not an admission as to their accuracy or correctness.

Modifications may be made to the foregoing without departing from the basic aspects of the technology. Although the technology has been described in substantial detail with reference to one or more specific embodiments, those of ordinary skill in the art will recognize that changes may be made to the embodiments specifically disclosed in this application, yet these modifications and improvements are within the scope and spirit of the technology.

The technology illustratively described herein suitably may be practiced in the absence of any element(s) not specifically disclosed herein. Thus, for example, in each instance herein any of the terms "comprising," "consisting essentially of," and "consisting of" may be replaced with either of the other two terms. The terms and expressions which have been employed are used as terms of description and not of limitation, and use of such terms and expressions do not exclude any equivalents of the features shown and described or portions thereof, and various modifications are possible within the scope of the technology claimed. The terms "method" and "process" are used interchangeably herein. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%). Thus, it should be understood that although the present technology has been specifically disclosed by representative embodiments and optional features, modification and variation of the concepts herein disclosed may be resorted to by those skilled in the art, and such modifications and variations are considered within the scope of this technology.

Certain embodiments of the technology are set forth in the item(s) that follow(s).

The present invention furthermore relates to the following items:

1. A method for determining the presence or absence of a genetic variation , comprising:

a. amplifying in a single reaction a plurality of paralogous polynucleotide species from nucleic acid in a sample, comprising contacting the nucleic acid with amplification primers under amplification conditions, wherein the paralogous polynucleotide species of each of the sets are amplified by a single pair of amplification primers and each primer of the pair of amplification primers is complementary to less than 20 positions in a human genome;
b. determining the amount of each amplified paralogous polynucleotide species in each of the sets;
c. determining a paralog ratio for each of the sets between the amount of each amplified paralogous polynucleotide species in each of the sets, thereby generating a plurality of paralog ratios; and
d. determining the presence or absence of the genetic variation based on the plurality of paralog ratios.

2. The method of item 1, wherein the nucleic acid is circulating cell free nucleic acid.
3. The method of item 1 or 2, wherein the nucleic acid is from a pregnant female comprising fetally derived and maternally derived nucleic acid.
4. The method of any one of items 1-3, wherein the paralogous polynucleotide species in each of the sets are present on two or more different chromosomes at different loci, comprising a target chromosome and one or more reference chromosomes not associated with the chromosomal aneuploidy.
5. The method of any one of items 1-4, wherein the genetic variation is a copy number alteration.
6. The method of item 5, wherein the genetic variation is a single nucleotide variation.
7. The method of any one of items 1-5, wherein the genetic variation is aneuploidy.
8. The method of any one of items 1-7, wherein the plurality of sets of paralogous polynucleotide species comprises a number of sets so as to provide statistical accuracy for samples comprising minority DNA as the target, optionally at least 100, 250, or 500 sets.
9. The method of item 4, wherein determining a paralog ratio in (c) is between (i) the. amount of amplified paralogous polynucleotide species from a target chromosome and (ii) the amount of amplified paralogous polynucleotide species from a reference chromosome.
10. The method of any one of items 1 to 9, wherein the paralogous polynucleotide .
species in each of the sets have primer hybridization sequences with a degree of sequence similarity that a single pair of amplification primers hybridizes to the paralogous polynucleotide species of each of the sets.
11. The method of any one of items 1 to 10, wherein the paralogous polynucleotide species in each of the sets differ by one or more mismatch nucleotides.
12. The method of item 11, wherein the determining the amount of each amplified .
paralogous polynucleotide species in each of the sets is by detecting the one or more mismatch nucleotides.
13. The method of any one of items 1 to 12, wherein amplifying in a single reaction in. (a) is at least 500 sets of paralogous polynucleotide species.
14. The method of any one of items 1 to 13, comprising determining fetal sex by a process comprising contacting the circulating cell free nucleic acid in the single reaction in (a) with primers specific for Y-chromosome polynucleotides under amplification conditions and amplifying Y-chromosome polynucleotides.
15. The method of item 14, wherein there are at least 10 Y-chromosome polynucleotides are amplified.
16. The method of any one of items 1 to 15, comprising determining fetal fraction by a process comprising contacting the circulating cell free nucleic acid in the single reaction in (a) with primers specific for polynucleotides flanking or comprising single nucleotide polymorphic (SNP) loci under amplification conditions, and amplifying polynucleotides containing the SNP loci.
17. The method of item 16, wherein there are at least 150 polynucleotides comprising

- single nucleotide polymorphic (SNP) loci that are amplified.

18. The method of any one of items 4 to 17, wherein the target chromosome is .
chromosome 21 and the reference chromosome is an autosome other than chromosome 21.
19. The method of any one of items 4 to 17, wherein the target chromosome is .
chromosome 18 and the reference chromosome is an autosome other than chromosome
20. The method of any one of items 4 to 17, wherein the sets of paralogous polynucleotide species comprise sets wherein the target chromosome is chromosome 21 and sets wherein the target chromosome is chromosome 18.
21. The method of item 20, wherein there are at least 250 sets of paralogous polynucleotide species for target chromosome 21 and there are at least 250 sets of paralogous polynucleotide species for target chromosome 18.

22. The method of item 21, wherein there are at least 500 sets of paralogous polynucleotide species for target chromosome 21 and there are at least 500 sets of paralogous polynucleotide species for target chromosome 18.
23. The method of any one of items 20 to 22, comprising amplifying Y-chromosome polynucleotides and amplifying polynucleotides comprising single nucleotide polymorphic (SNP) loci.
24. The method of item 23, wherein there are at least 10 Y-chromosome polynucleotides that are amplified and at least 150 polynucleotides comprising single nucleotide polymorphic (SNP) loci that are amplified.
25. The method of any one of items 1 to 24, wherein the amplification primers for each of the paralogous polynucleotide species sets produces two equal-sized amplicons with a size between 60 to 100 bp.
26. The method of any one of items 1 to 25, wherein the amplification primers for each of the sets produces zero off-target amplification events allowing maximal 3-base pair mis-priming.
27. The method of any one of items 1 to 26, wherein the amplification primers for each of the sets do not overlap with any annotated single nucleotide variant with greater than 1% minor allele frequency.
28. The method of any one of items 1 to 27, wherein the amplification primers for each of the sets flank at least one position where the nucleotide sequence species in a set differ by one or more mismatch nucleotides.
29. The method of any one of items 1 to 28, wherein the amplification primers for each of the sets amplify a paralogous polynucleotide species set that is at least 100-bp apart from each of the other sets.
30. The method of any one of items 1 to 29, wherein after (a) each of the amplified sets of paralogous polynucleotide species are further amplified by universal PCR.
31. The method of any one of items 1 to 30, comprising generating sequence reads from each of the amplified paralogous polynucleotide species sets by a sequencing process.
32. The method of item 31, wherein the sequence reads are filtered for a minimum number of reads and no copy number variants.
33. The method of item 32, wherein the sequence reads are quantified to obtain counts.
34. The method of item 33, wherein determining a paralog ratio in (c) is calculated as the ratio of the counts of the paralogous polynucleotide species on a target chromosome to the counts of the paralogous polynucleotide species on a reference chromosome.
35. The method of item 33, wherein the paralog ratio is logarithm-transformed to zero-center values.
36. The method of any one of items 1 to 35, comprising determining a statistic for each of the paralog ratios or logarithm-transformed paralog ratios.
37. The method of item 36, wherein the statistic is a z-score.
38. The method of item 37, wherein the z-score is a quotient of (a) subtraction product of (i) the logarithm-transformed paralog ratio, less (ii) a median of the logarithm-transformed paralog ratio of reference euploid samples, divided by (b) a MAD value derived from reference euploid samples.
39. The method of item 36, wherein the statistic is a weighted z-score.
40. The method of item 39, wherein the weighted z-score is calculated by weighting individual non-outlier z-scores paralog ratios according to assay MAD or discriminatory power.
41. The method of item 36, comprising determining a sample statistic according to the statistic for each of the paralog ratios or logarithm-transformed paralog ratios.
42. The method of item 41, comprising summing statistics for each of the sets.
43. The method of item 41 or 42, wherein a sample z-score is calculated by z-scores of a plurality paralogous polynucleotide species sets, with the exclusion of outliers of z greater than 4 MAD or less than -4 MAD.
44. The method of item 41 or 42, comprising determining a sample z-score according to

$$Z = \frac{\sum_{i=1}^{N} z_i}{\sqrt{N}},$$

where $z_i$ is the z-score of a non-outlier paralog ratio and N is the number of non-outlier paralogs.
45. The method of item 41 or 42, wherein a sample z-score is calculated by weighted z-scores of a plurality paralogous polynucleotide species sets.
46. The method of item 41 or 42, comprising determining a sample weighted z-score according to

$$Z_w = \frac{\sum_{i=1}^{N} w_i z_i}{\sqrt{\sum_{i=1}^{N} w_i^2}},$$

where $w_i$ is the weight for each paralog ratio.
47. The method of any one of items 36 to 46, wherein a classification is generated for the presence or absence of a chromosomal aneuploidy according to the statistics.
48. The method of item 47, wherein the statistic is z-score.
49. The method of any one of items 1 to 47, wherein the paralogous polynucleotide species sets comprise one or more of the sets in Fig. 8.
50. The method of any one of items 1 to 49, wherein the amplification primers comprise one or more pairs of the amplification primers in Figure 8.
51. The method of any one of items 1-2, 5-6, 8-13, and 25-50, wherein the genetic variation is cancer.
52. The method of any one of the items 1-2, 5-6, 8-13, and 25-50, wherein the genetic variation is an inherited mutation.
53. The method of any one of items 1-2, 5-6, 8-13, and 25-50, wherein the genetic variation is a somatic mutation.
54. A composition comprising a mixture of a plurality of amplification primer pairs that can amplify the plurality of sets of paralogous polynucleotide species of any of items 1-54, wherein the each primer of the plurality of amplification primer pairs is complementary to less than 20 positions in a human genome,

wherein the amplification primers for each of the paralogous polynucleotide species sets produces two equal-sized amplicons with a size between 60 to 100 bp;
wherein the amplification primers for each of the sets produces zero off-target amplification events allowing maximal 3-base pair mis-priming; and/or
wherein the amplification primers for each of the sets do not overlap with any annotated single nucleotide variant with greater than 1% minor allele frequency in dbSNP build137.

55. The composition of item 54, wherein the composition comprises a plurality of amplification primer pairs in Figure 8.
56. A kit comprising the composition of any of items 54- 55 and a DNA polymerase.
57. A system for determining the presence or absence of a genetic variation, comprising

a) a component for amplifying in a single reaction a plurality of paralogous polynucleotide species from nucleic acid in a sample, comprising contacting the nucleic acid with amplification primers under amplification conditions, wherein the paralogous polynucleotide species of each of the sets are amplified by a single pair of amplification primers and each primer of the pair of amplification primers is complementary to less than 20 positions in a human genome;
b) a component for determining the amount of each amplified paralogous polynucleotide species in each of the sets;
c) a component for determining a paralog ratio for each of the sets between the amount of each amplified paralogous polynucleotide species in each of the sets, thereby generating a plurality of paralog ratios; and
d) a component for determining the presence or absence of the genetic variation based on the plurality of paralog ratios.

58. The system of item 57, wherein one or more of components b)-d) comprise a computer processor.
59. A method of generating paralog assay systems comprising: identifying paralogous contigs on a first reference chromosome and a second target chromosome; extracting those sequences which are substantially identical in sequence and that map to exactly two regions, one region on the first reference chromosome and one region on the second target chromosome; and merging the sequences in each region to form paralog contigs.
60. The method of item 59, further comprising designing primers that amplify, but differentiate, the contigs from both the reference and the target chromosome.

## Claims

1. A method of generating a paralog assay system, comprising:

   identifying, by a computing system, potential paralogous contigs located on a reference chromosome and a target chromosome by sampling the reference chromosome and the target chromosome;
   aligning, by the computing system, each of the paralogous contigs to a human reference genome;
   extracting, by the computing system, pairs of sequences which are substantially identical in sequence and that map to exactly two regions, one region on the reference chromosome and one region on the target chromosome;
   merging, by the computing system, each extracted pair of sequences to form pairs of target and reference paralogous contigs;
   aligning, by the computing system, each of the pairs of target and reference paralogous contigs to locate nucleotide differences distinguishing the target and reference paralogous contigs;
   designing, by the computing system, pairs of primers for each of the pairs of target and reference paralogous contigs, wherein each of the pairs of primers are designed to surround the nucleotide differences distinguishing respectively each of the target and reference paralogous contigs; and
   selecting, by the computing system, a pair of primers from the pairs of primers designed for each of the pairs of target and reference paralogous contigs to be included as a part of the paralog assay system.

2. The method of claim 1, wherein (i) each selected pair of primers is predicted to produce exactly two equal-sized amplicons between 60-100 bp, (ii) each selected pair of primers is predicted to produce zero off-target amplification events allowing maximal 3-base mis-priming, (iii) each selected pair of primers is complementary to less than 20 positions in the human reference genome, (iv) each selected pair of primers flank at least one position that distinguishes target from reference paralogous contigs, and (v) all pairs of target and reference paralogous contigs are at least 100 bp apart.

3. The method of claim 1, wherein the reference chromosome and the target chromosome are sampled at 10 base pair intervals in 100 base pair segments.

4. The method of claim 1, wherein the pair of primers selected from the pairs of primers for each of the pairs of target and reference paralogous contigs produce two equal-sized amplicons with a size between 60-100bp.

5. The method of claim 1, wherein the pair of primers selected from the pairs of primers for each of the pairs of target and reference paralogous contigs produce zero off-target amplification events allowing maximal 3-base pair mis-priming.

6. The method of claim 1, wherein the pair of primers selected from the pairs of primers for each of the pairs of target and reference paralogous contigs do not overlap with any annotated single nucleotide variant with greater than 1% minor allele frequency.

7. The method of claim 1, wherein the pair of primers selected from the pairs of primers for each of the pairs of target and reference paralogous contigs flank at least one position where the nucleotide sequence species in a set differ by one or more mismatch nucleotides.

8. The method of claim 1, wherein the pair of primers selected from the pairs of primers for each of the pairs of target and reference paralogous contigs amplify a paralogous polynucleotide species set that is at least 100-bp apart from each of the other sets of paralogous polynucleotide species.

9. A system comprising:

   one or more processors;

a memory coupled to the one or more processors, the memory storing a plurality of instructions executable by the one or more processors, the plurality of instructions comprising instructions that when executed by the one or more processors cause the one or more processors to perform processing comprising:

identifying potential paralogous contigs located on a reference chromosome and a target chromosome by sampling the reference chromosome and the target chromosome;
aligning each of the paralogous contigs to a human reference genome;
extracting pairs of sequences which are substantially identical in sequence and that map to exactly two regions, one region on the reference chromosome and one region on the target chromosome;
merging each extracted pair of sequences form pairs of target and reference paralogous contigs;
aligning each of the pairs of target and reference paralogous contigs to locate nucleotide differences distinguishing the target and reference paralogous contigs;
designing pairs of primers for each of the pairs of target and reference paralogous contigs, wherein each of the pairs of primers are designed to surround the nucleotide differences distinguishing respectively each of the target and reference paralogous contigs; and
selecting a pair of primers from the pairs of primers designed for each of the pairs of target and reference paralogous contigs to be included as a part of the paralog assay system.

**10.** The system of claim 9, wherein (i) each selected pair of primers is predicted to produce exactly two equal-sized amplicons between 60-100 bp, (ii) each selected pair of primers is predicted to produce zero off-target amplification events allowing maximal 3-base mis-priming, (iii) each selected pair of primers is complementary to less than 20 positions in the human reference genome, (iv) each selected pair of primers flank at least one position that distinguishes target from reference paralogous contigs, and (v) all pairs of target and reference paralogous contigs are at least 100-bp apart.

**11.** The system of claim 9, wherein the reference chromosome and the target chromosome are sampled at 10 base pair intervals in 100 base pair segments.

**12.** A computer-program product tangibly embodied in a non-transitory machine-readable storage medium, including instructions configured to cause one or more data processors to perform actions including:

identifying potential paralogous contigs located on a reference chromosome and a target chromosome by sampling the reference chromosome and the target chromosome;
aligning each of the paralogous contigs to a human reference genome; extracting pairs of sequences which are substantially identical in sequence and that map to exactly two regions, one region on the reference chromosome and one region on the target chromosome;
merging each extracted pair of sequences form pairs of target and reference paralogous contigs;
aligning each of the pairs of target and reference paralogous contigs to locate nucleotide differences distinguishing the target and reference paralogous contigs;
designing pairs of primers for each of the pairs of target and reference paralogous contigs, wherein each of the pairs of primers are designed to surround the nucleotide differences distinguishing respectively each of the target and reference paralogous contigs; and
selecting a pair of primers from the pairs of primers designed for each of the pairs of target and reference paralogous contigs to be included as a part of the paralog assay system.

**13.** The computer-program product of claim 12, wherein (i) each selected pair of primers is predicted to produce exactly two equal-sized amplicons between 60-100 bp, (ii) each selected pair of primers is predicted to produce zero off-target amplification events allowing maximal 3-base mis-priming, (iii) each selected pair of primers is complementary to less than 20 positions in the human reference genome, (iv) each selected pair of primers flank at least one position that distinguishes target from reference paralogous contigs, and (v) all pairs of target and reference paralogous contigs are at least 100-bp apart.

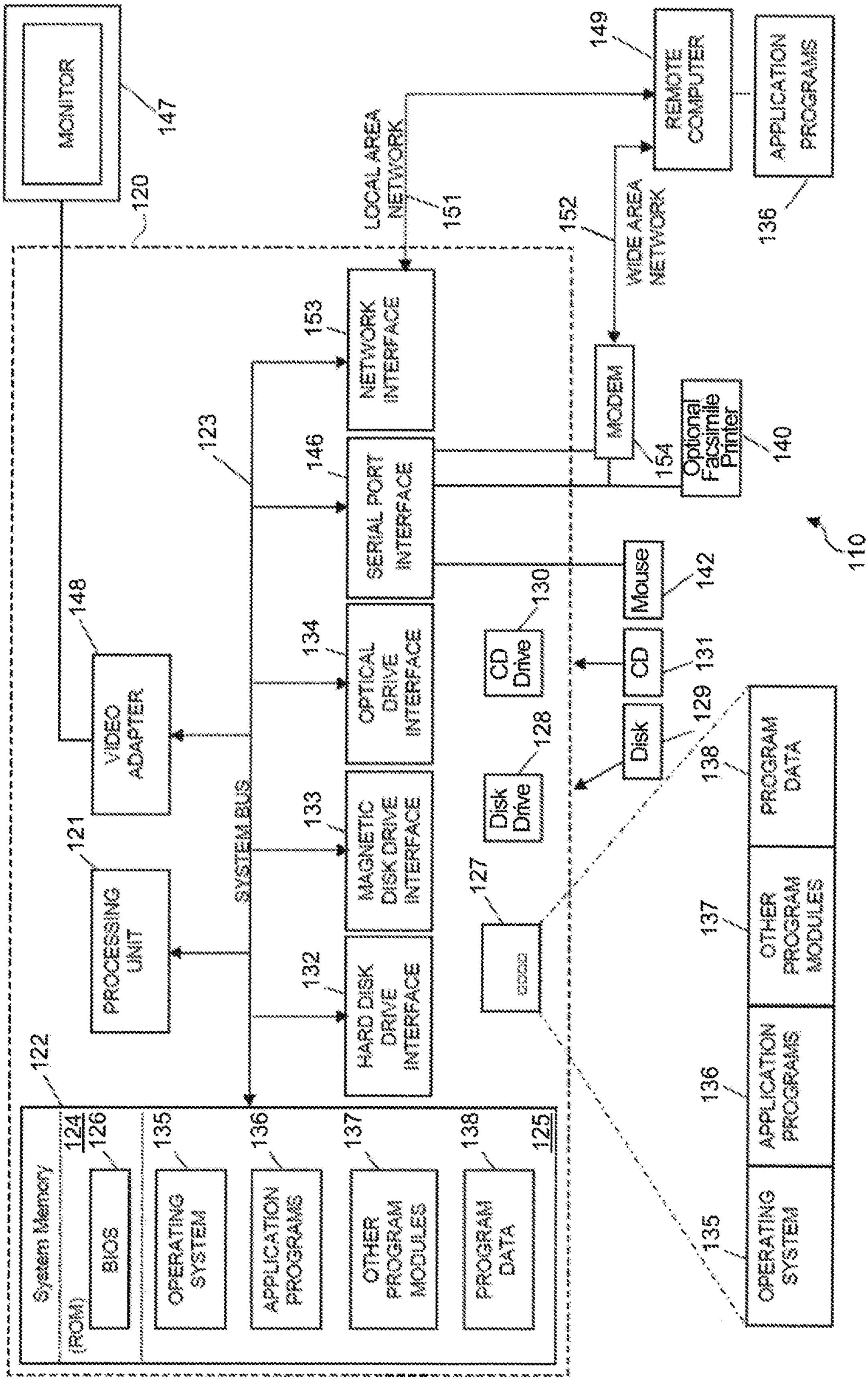

MONITOR
147
120
VIDEO ADAPTER
148
PROCESSING UNIT
121
SYSTEM BUS
123
System Memory
122
(ROM)
124
126
BIOS
135
OPERATING SYSTEM
136
APPLICATION PROGRAMS
137
OTHER PROGRAM MODULES
138
PROGRAM DATA
125
HARD DISK DRIVE INTERFACE
132
MAGNETIC DISK DRIVE INTERFACE
133
OPTICAL DRIVE INTERFACE
134
SERIAL PORT INTERFACE
146
NETWORK INTERFACE
153
127
Disk Drive
128
CD Drive
130
Disk
129
CD
131
Mouse
142
MODEM
154
Optional Facsimile Printer
140
LOCAL AREA NETWORK
151
WIDE AREA NETWORK
152
REMOTE COMPUTER
149
APPLICATION PROGRAMS
136
OPERATING SYSTEM
135
APPLICATION PROGRAMS
136
OTHER PROGRAM MODULES
137
PROGRAM DATA
138
110


FIG. 1

FIG. 2A

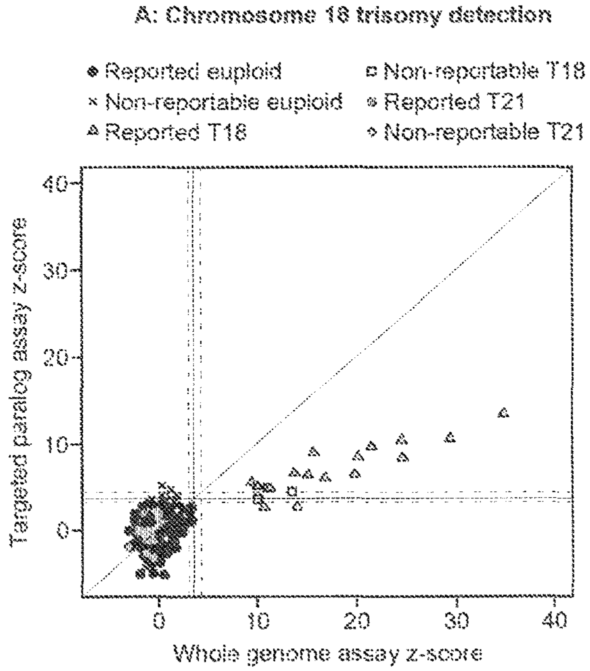
A: Chromosome 18 trisomy detection
Reported euploid
Non-reportable euploid
Reported T18
Non-reportable T18
Reported T21
Non-reportable T21
Targeted paralog assay z-score
Whole genome assay z-score
0
10
20
30
40

FIG. 2B

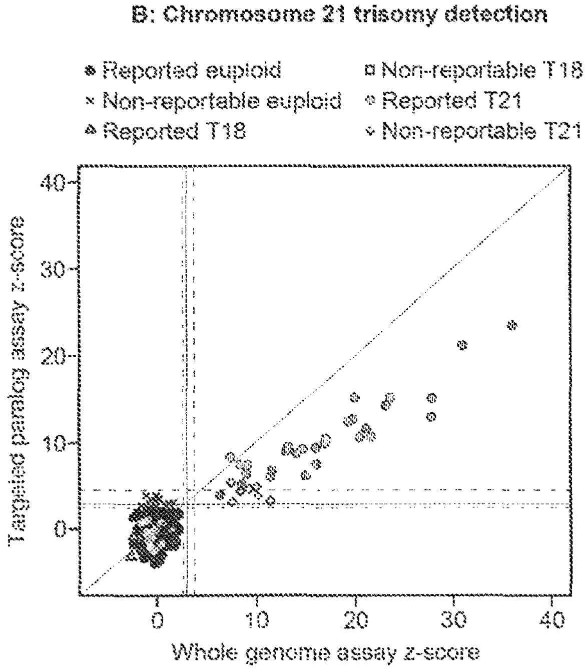
B: Chromosome 21 trisomy detection
Reported euploid
Non-reportable euploid
Reported T18
Non-reportable T18
Reported T21
Non-reportable T21
Targeted paralog assay z-score
Whole genome assay z-score
0
10
20
30
40

FIG. 3

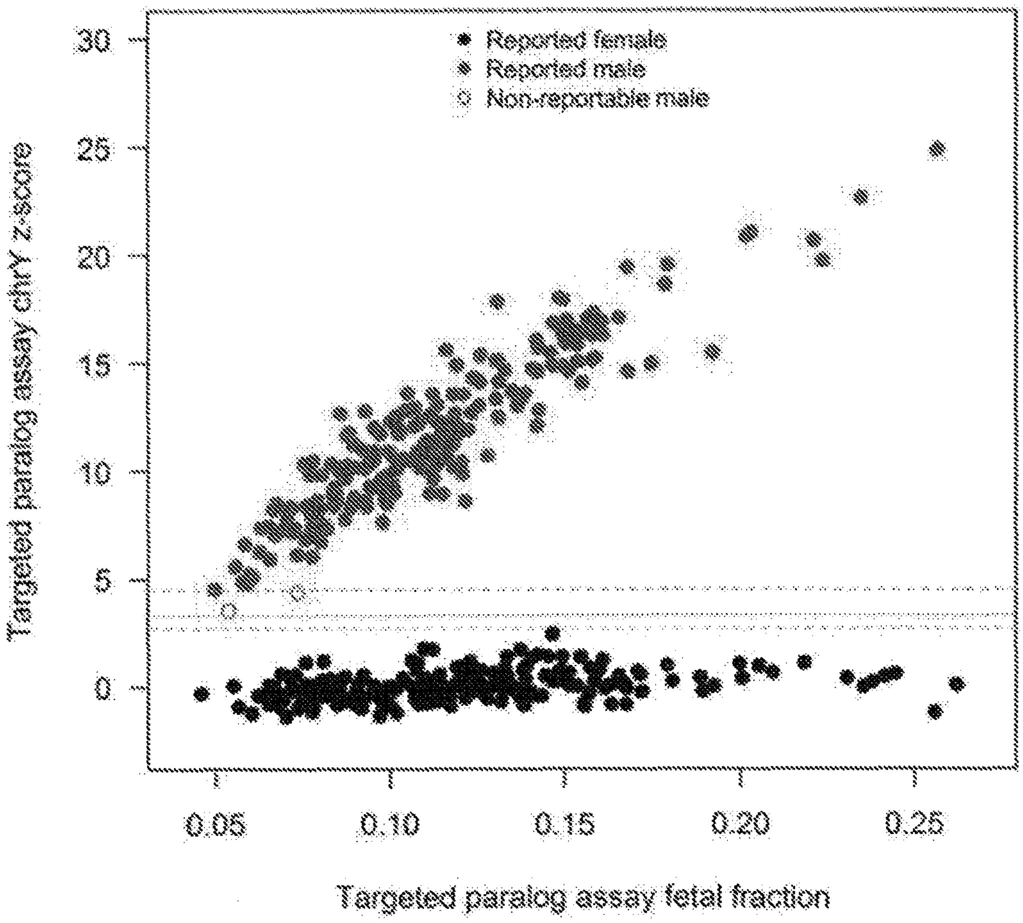
Reported female
Reported male
Non-reportable male
Targeted paralog assay chrY z-score
30
25
20
15
10
5
0
0.05
0.10
0.15
0.20
0.25
Targeted paralog assay fetal fraction

FIG. 4

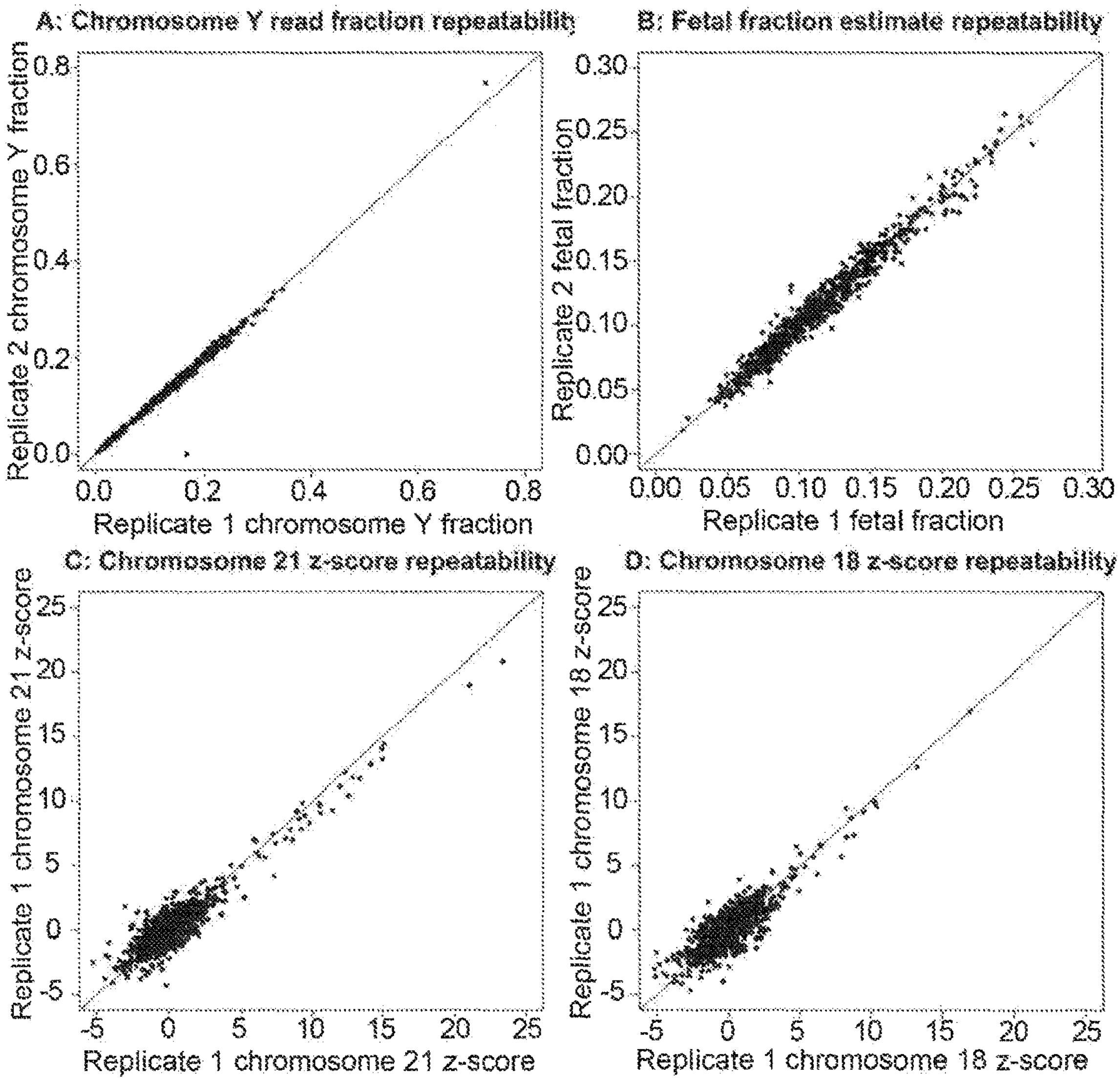
A: Chromosome Y read fraction repeatability
Replicate 2 chromosome Y fraction
Replicate 1 chromosome Y fraction
0.0
0.2
0.4
0.6
0.8
B: Fetal fraction estimate repeatability
Replicate 2 fetal fraction
Replicate 1 fetal fraction
0.00
0.05
0.10
0.15
0.20
0.25
0.30
C: Chromosome 21 z-score repeatability
Replicate 1 chromosome 21 z-score
Replicate 1 chromosome 21 z-score
D: Chromosome 18 z-score repeatability
Replicate 1 chromosome 18 z-score
Replicate 1 chromosome 18 z-score
-5
0
5
10
15
20
25

FIG. 5

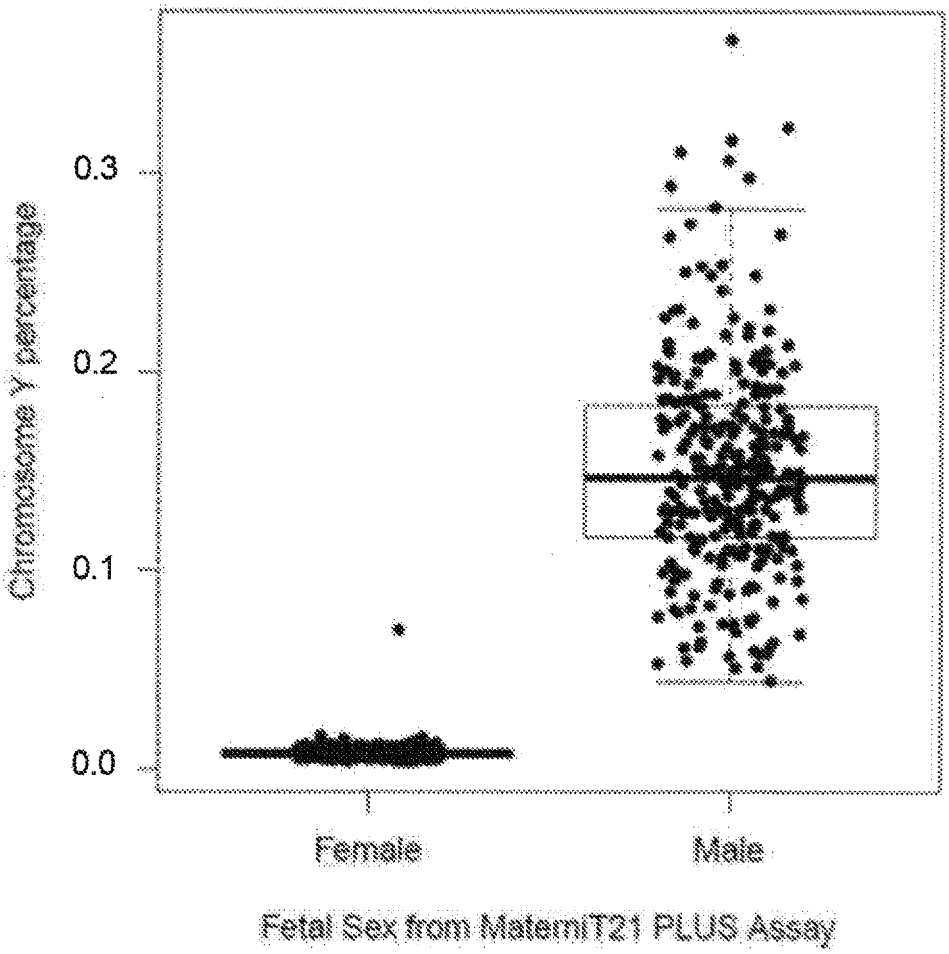
0.3
0.2
0.1
0.0
Chromosome Y percentage
Female
Male
Fetal Sex from MaterniT21 PLUS Assay

FIG. 6

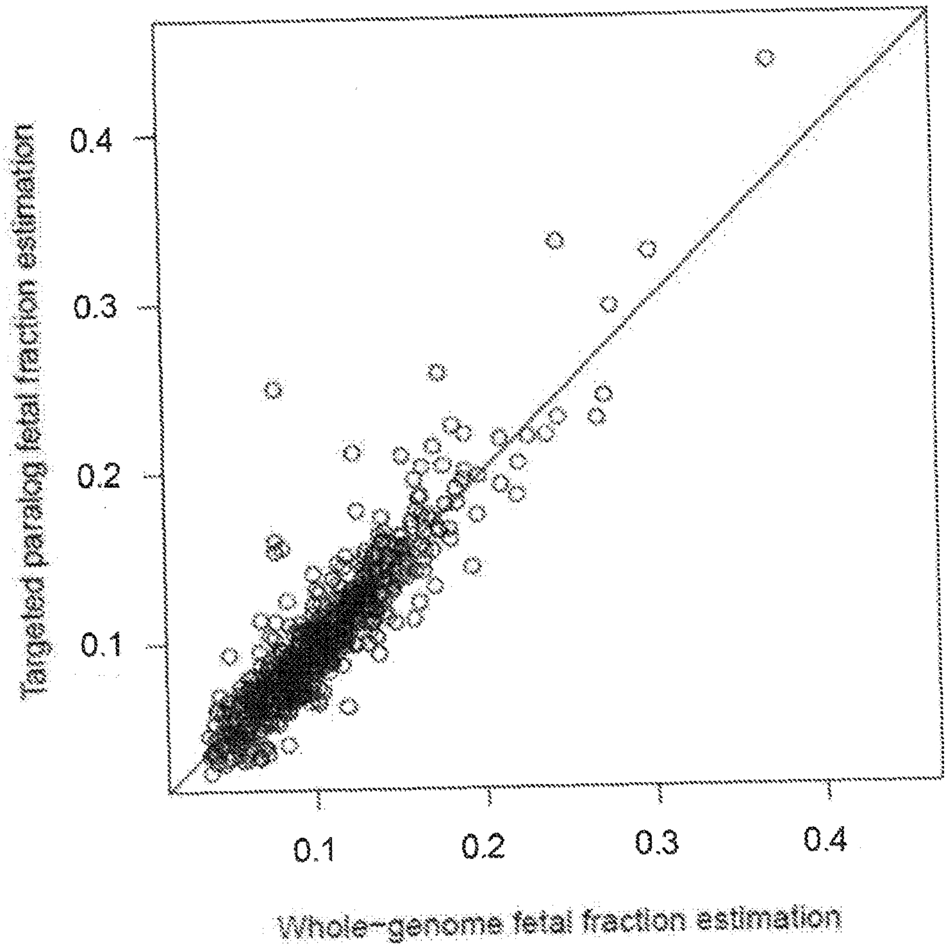
0.4
0.3
0.2
0.1
Targeted paralog fetal fraction estimation
0.1
0.2
0.3
0.4
Whole-genome fetal fraction estimation

FIG. 7

A)

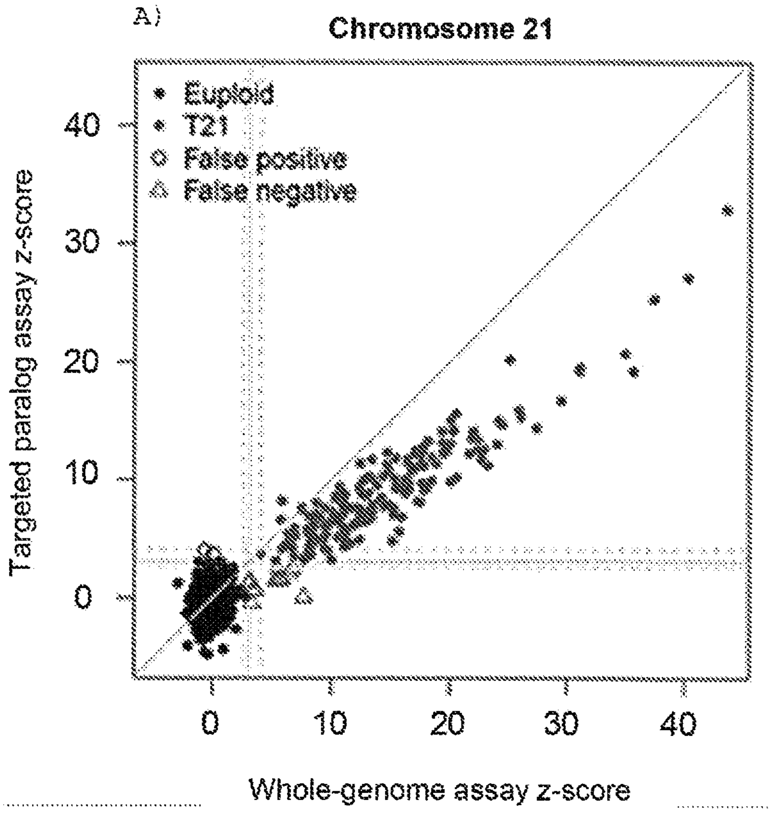
Chromosome 21
Euploid
T21
False positive
False negative
Targeted paralog assay z-score
Whole-genome assay z-score
40
30
20
10
0
0
10
20
30
40

B)

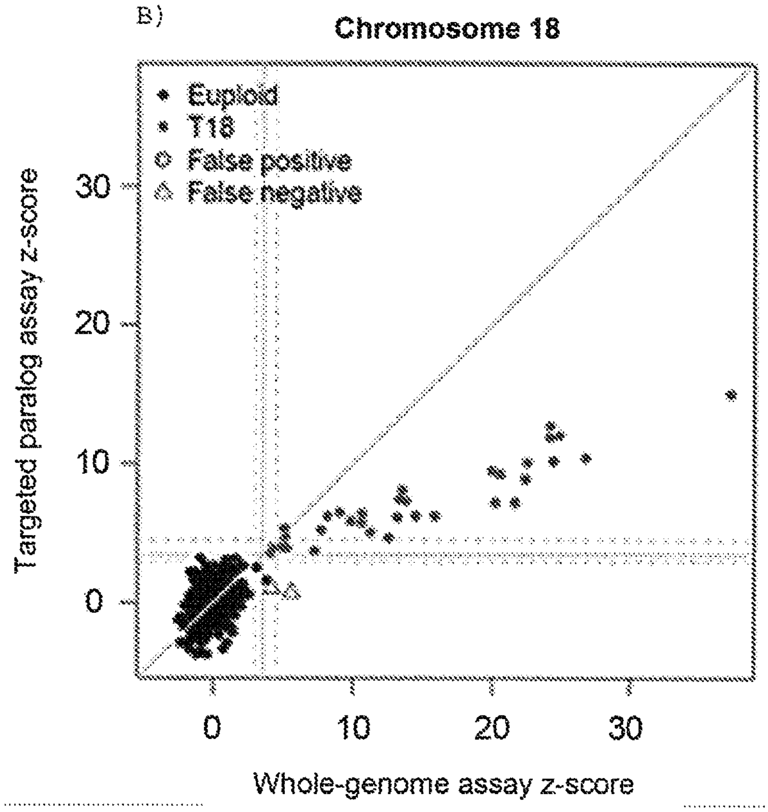
Chromosome 18
Euploid
T18
False positive
False negative
Targeted paralog assay z-score
Whole-genome assay z-score
30
20
10
0
0
10
20
30

| markerID | chr.tar | strand.tar | start.tar | end.tar | chr.ref | strand.ref | start.ref | end.ref | Fprimer | SEQ ID NO. | Rprimer | SEQ ID NO. | included_in_final |
|---|---|---|---|---|---|---|---|---|---|---|---|---|---|
| chr18_chr3_132723573_89 | chr18 | + | 10719883 | 10719973 | chr3 | - | 132722973 | 132723063 | GGATAACAGGTAGATGAGAAAG | 1 | CAAATTATAAATGTTCTAGGCTG | 2 | TRUE |
| chr18_chr16_67215710_99 | chr18 | + | 10723336 | 10723426 | chr16 | - | 67215710 | 67215800 | GATCAGATCAATTGGGATGG | 3 | ACCCAGTCTCCAGTTCCT | 4 | TRUE |
| chr18_chr16_67215538_160 | chr18 | + | 10723507 | 10723598 | chr16 | - | 67215538 | 67215629 | CTAGTGAAAGGTTTTGTTTTG | 5 | TCAATCTCTTCCTCTCTACTGG | 6 | TRUE |
| chr18_chr16_67214390_447 | chr18 | + | 10724640 | 10724739 | chr16 | - | 67214390 | 67214489 | GCGTATGATGAGGCACCC | 7 | ACCAGGGGTACAGTGGTG | 8 | TRUE |
| chr18_chr16_67214019_466 | chr18 | + | 10725035 | 10725130 | chr16 | - | 67214019 | 67214114 | ACCAAGACCCCCTGCATGT | 9 | CTGTGGATGTAGGTGTAATG | 10 | FALSE |
| chr18_chr16_67213204_676 | chr18 | + | 10725468 | 10725567 | chr16 | - | 67213204 | 67213303 | TAGGCATGAAAGGGGCCTG | 11 | AAAGCCCGACCTCAGGA | 12 | FALSE |
| chr18_chr16_67213100_729 | chr18 | + | 10725607 | 10725691 | chr16 | - | 67213100 | 67213184 | GGTAGGCCAGAACATGGC | 13 | TATACCCGAAAGGACCAAGA | 14 | TRUE |
| chr18_chr16_67212590_859 | chr18 | + | 10726119 | 10726202 | chr16 | - | 67212590 | 67212675 | GTGCGTGGGTGTAGGGTG | 15 | GTGGACTGGCACCCAAGG | 16 | FALSE |
| chr18_chr16_67210786_982 | chr18 | + | 10726774 | 10726869 | chr16 | - | 67210786 | 67210877 | TGTCCTATGAGGATGTGG | 17 | GCAGTAGGGTACCAAGTC | 18 | TRUE |
| chr18_chr16_67210413_1071 | chr18 | + | 10727151 | 10727238 | chr16 | - | 67210413 | 67210501 | GCAAACTGAGTCGCCCTC | 19 | CATCCCCATCAATGCTCAG | 20 | TRUE |
| chr18_chr16_67210034_1348 | chr18 | + | 10727326 | 10727418 | chr16 | - | 67210034 | 67210126 | CCCTTGGTGCTCCTTCAG | 21 | TCAGGCCATCAATCACCC | 22 | TRUE |
| chr18_chr1_2075187_1419 | chr18 | + | 11286932 | 11287012 | chr1 | - | 2075187 | 2075267 | TGCCAGGCTAGAGGATTC | 23 | GACTGTGCCTTCACCCTC | 24 | TRUE |
| chr18_chr7_35563619_1484 | chr18 | + | 11959215 | 11959302 | chr7 | + | 35563619 | 35563686 | GCAAGCATGTCAGCAAGC | 25 | TGGAGAAGTTAGGAAAATCA | 26 | FALSE |
| chr18_chr8_10342768_1607 | chr18 | + | 11913809 | 11913921 | chr8 | - | 10342768 | 10342860 | CCCCAACTGCAACAGGATT | 27 | TTACAAAGCATCTCCGATCA | 28 | TRUE |
| chr18_chr16_35113577_1815 | chr18 | + | 12050455 | 12050548 | chr16 | + | 35113577 | 35113670 | GGAGTAGCTGTAGTTGGAGA | 29 | CACCTTACTGGCCATTTT | 30 | TRUE |
| chr18_chr17_4927845_1951 | chr18 | + | 12056139 | 12056230 | chr17 | - | 4927845 | 4927936 | ACGGGAAGGGAATGACTTAT | 31 | TGTCTGTGGTGGTTTCTGTTT | 32 | TRUE |
| chr18_chr17_4927370_1967 | chr18 | + | 12058654 | 12058745 | chr17 | - | 4927370 | 4927461 | CCCAGGATGTGGGACTCA | 33 | CCCCATAGACTACTCCC | 34 | TRUE |
| chr18_chr17_4927199_1970 | chr18 | + | 12058832 | 12058919 | chr17 | - | 4927199 | 4927293 | AGTGCTGAAGTTCAGGCT | 35 | AGTCCCCGAAGGTCCCAC | 36 | TRUE |
| chr18_chr17_4925681_2056 | chr18 | + | 12057650 | 12057664 | chr17 | - | 4925681 | 4925746 | TCTGGCTTGCCATAAGTG | 37 | GCTGAGATTGAGGACCTG | 38 | FALSE |
| chr18_chr4_22435193_2342 | chr18 | + | 12073404 | 12073583 | chr4 | + | 22435199 | 22435298 | GCTGGCAAACAGTTCAACA | 39 | CGCCATCGAAGAATACAT | 40 | TRUE |
| chr18_chr4_22436089_2567 | chr18 | + | 12074082 | 12074328 | chr4 | + | 22436089 | 22436148 | TGGATGAGTCAGGGATCTTG | 41 | AAGCCCAGTCTTAGGTTAGG | 42 | TRUE |
| chr18_chr4_22436324_2595 | chr18 | + | 12074602 | 12074689 | chr4 | + | 22436324 | 22436411 | CATTCTGAAACTGTGCTTGTC | 43 | CTTAACGGGACCACATTGAA | 44 | TRUE |
| chr18_chr4_22436556_2672 | chr18 | + | 12074883 | 12074977 | chr4 | + | 22436556 | 22436650 | GGATGACTAAAACTCCGTTA | 45 | TTAGCTCAGCCTGCTGGATT | 46 | TRUE |
| chr18_chr4_22438098_3166 | chr18 | + | 12075306 | 12075400 | chr4 | + | 22438098 | 22438192 | TTGCATACTGACAGCAGAGA | 47 | TTGGCCCAGATGTGATAGAG | 48 | TRUE |
| chr18_chr4_22439959_3254 | chr18 | + | 12075643 | 12075736 | chr4 | + | 22439959 | 22440022 | CCTCCTATTATTCCCACGTTT | 49 | TCAAGCTGGATCTACTGGAA | 50 | TRUE |
| chr18_chr4_22448616_3336 | chr18 | + | 12075962 | 12076057 | chr4 | + | 22448616 | 22448711 | TTCACGCCTGTGACTGGTT | 51 | GATGCATCGCTGGGTAAAG | 52 | TRUE |
| chr18_chr4_22456497_3437 | chr18 | + | 12076188 | 12076242 | chr4 | + | 22456497 | 22456556 | CAGATTGGTGAGTCCTCGAA | 53 | CTGACAAACAATCGAATAGG | 54 | FALSE |
| chr18_chr4_22517160_3469 | chr18 | + | 12076400 | 12076471 | chr4 | + | 22517160 | 22517231 | GTGCCGTTGGGCAGAGTA | 55 | CGAGGGCAAGGTGGTGTG | 56 | FALSE |
| chr18_chr10_104162200_3540 | chr18 | + | 12081518 | 12081597 | chr10 | - | 104162200 | 104162279 | AGTTGGGAAGGGAGGGGTCC | 57 | ATTTAACACCCCACACCCA | 58 | FALSE |
| chr18_chr10_104161863_3565 | chr18 | + | 12081782 | 12081853 | chr10 | - | 104161863 | 104161934 | GTCCTGGGCCAGCTTGTCT | 59 | GGCCTAGAGGAGGGAGTG | 60 | FALSE |
| chr18_chr2_101993121_3682 | chr18 | + | 12101700 | 12101790 | chr2 | - | 101993121 | 101993181 | GGCAAAAGCTCGTATCATGG | 61 | GGTTGCCTCCCAGCTTCTC | 62 | FALSE |
| chr18_chr12_122631686_3720 | chr18 | + | 12737371 | 12737461 | chr12 | - | 122631686 | 122631776 | AGGGGTGTACTCAGGGTGAC | 63 | GGTCCACCTCGTGGTCTG | 64 | FALSE |
| chr18_chr12_122631560_3758 | chr18 | + | 12737467 | 12737578 | chr12 | - | 122631560 | 122631660 | ATCACCAAAGCTCACACC | 65 | TTTGTTCAGGGCAGAAGC | 66 | FALSE |
| chr18_chr12_122631281_3905 | chr18 | + | 12737774 | 12737866 | chr12 | - | 122631281 | 122631373 | CCTCAACTCTTGAGACCACC | 67 | TGTTGGTTCTGCCCTCTG | 68 | TRUE |
| chr18_chr12_122630881_3988 | chr18 | + | 12738217 | 12738301 | chr12 | - | 122630881 | 122630965 | CTTCGTCAGGTTCCTGG | 69 | GGCGGCTGGACAGGTTTT | 70 | TRUE |
| chr18_chr8_94682409_3850 | chr18 | + | 1284271 | 1284353 | chr8 | + | 94682409 | 94682491 | TGGGATGAGACTGAGTATTG | 71 | CACAGGGGCTTGCCATTA | 72 | TRUE |
| chr18_chr8_94683048_3879 | chr18 | + | 1284906 | 1285000 | chr8 | + | 94683048 | 94683139 | CTCGGTGCTAGTCTTATGGAA | 73 | GGACCAAGATAGGAGGTGGG | 74 | TRUE |
| chr18_chr13_31127906_4066 | chr18 | + | 12859163 | 12859254 | chr13 | - | 31127906 | 31127987 | ATGGGCTTACATCTCTGTATC | 75 | TCATAGAGTGGCCAAGTTTC | 76 | TRUE |
| chr18_chr18_58825432_4157 | chr18 | + | 13107396 | 13107469 | chr18 | - | 58825403 | 58825482 | CAAATCCACAACCCTCATACT | 77 | GCCCAGAGAGGGACATCA | 78 | TRUE |
| chr18_chr14_64066067_4422 | chr18 | + | 13151181 | 13152068 | chr14 | + | 64066067 | 64066158 | AAATACATTTACCAAGGCATCA | 79 | TCAAGTACGTTTCCATCCCA | 80 | TRUE |
| chr18_chr14_64068610_4503 | chr18 | + | 13152667 | 13152725 | chr14 | + | 64068610 | 64068668 | GGAACATTTAACAATGTGCAG | 81 | TTTACTACCCTTGGATGCT | 82 | TRUE |
| chr18_chr10_89514129_4676 | chr18 | + | 1962126 | 1962225 | chr10 | + | 89514129 | 89514228 | CACTAATGACCCAATGAATC | 83 | ACGTGGGTGCTTGTGTTT | 84 | TRUE |
| chr18_chr10_89516583_4757 | chr18 | + | 1962659 | 1962734 | chr10 | + | 89516583 | 89516678 | AGAGGAGGGGCAGCATCTC | 85 | TGGAATAGGCATGTAGACCT | 86 | TRUE |
| chr18_chr10_89544267_4764 | chr18 | + | 1963019 | 1963134 | chr10 | + | 89544267 | 89544364 | GGCAAGAGAGAAGACAGCAG | 87 | AGAAGCAGGACTGTGGATT | 88 | TRUE |
| chr18_chr10_89574233_4825 | chr18 | + | 1368751 | 1368833 | chr10 | + | 89574233 | 89574315 | CAATTGCATCTACCATCCA | 89 | TGAAGTTGTTGGTTTAATTTTCC | 90 | TRUE |
| chr18_chr21_15400241_5107 | chr18 | + | 14131859 | 14131954 | chr21 | - | 15400241 | 15400340 | CTGACCCCTGTCCCATTA | 91 | CCGTCCCTACCTTACCCT | 92 | TRUE |
| chr18_chr21_15394658_5357 | chr18 | + | 14136030 | 14136098 | chr21 | - | 15394658 | 15394727 | GCAGCCCAGAGAGCCCAGTTTT | 93 | GAGACACCTGTCACCAAGACAG | 94 | TRUE |
| chr18_chr21_15391332_7419 | chr18 | + | 14139398 | 14139471 | chr21 | - | 15391262 | 15391335 | AGGATGCAGTTCTGGAAACA | 95 | AAAATTGTCACTGTCGGTC | 96 | TRUE |
| chr18_chr21_15387972_7780 | chr18 | + | 14140725 | 14140789 | chr21 | - | 15387972 | 15388033 | ACCTGGCCCTAGCATCTACC | 97 | GGTCAGGGGTTGGGAGTT | 98 | FALSE |

FIG. 8

| markerName | chr.tar | strand.tar | start.tar | end.tar | chr.ref | strand.ref | start.ref | end.ref | FPrimer | SEQ ID NO. | RPrimer | SEQ ID NO. | includedInFinal |
|---|---|---|---|---|---|---|---|---|---|---|---|---|---|
| chr18_chr21_15387284_7988 | chr18 | + | 14348437 | 14348525 | chr21 | - | 15387284 | 15387372 | CTGTAAGGGGGTCAGGGGG | 99 | CCAGCTCTTAGCTCCCTT | 100 | FALSE |
| chr18_chr21_15359596_8277 | chr18 | + | 14170938 | 14171030 | chr21 | - | 15359596 | 15359688 | AGGGCAGGACCAGAACCA | 101 | TGTCATGTCCCTGGCTGT | 102 | FALSE |
| chr18_chr15_19477217_9768 | chr18 | + | 14848016 | 14848098 | chr15 | + | 19477217 | 19477299 | TGCTCACCACCCTTTTCC | 103 | GAGAGCAGAACAGGAGAAA | 104 | TRUE |
| chr18_chr10_45658342_9854 | chr18 | + | 14773029 | 14773125 | chr10 | + | 45658342 | 45658438 | TAAAGCAGGGGTAAGATTG | 105 | TGGAGTCTCAGTCAGTCATT | 106 | TRUE |
| chr18_chr10_37434051_9874 | chr18 | + | 14780080 | 14780168 | chr10 | + | 37434051 | 37434139 | AGAGGCTTTGACTTGGTT | 107 | CCCAGCCAAGTAGTTTTT | 108 | TRUE |
| chr18_chr10_37436025_10097 | chr18 | + | 14782050 | 14782136 | chr10 | + | 37436025 | 37436091 | TGAAATATGCAGATGAGTGA | 109 | TGACTCCGTTACTGAGCATGA | 110 | TRUE |
| chr18_chr10_37438088_10190 | chr18 | + | 14783118 | 14783189 | chr10 | + | 37438088 | 37438162 | GGAATTATCTGAGCATGCAG | 111 | ATGCACACCTATGCATTT | 112 | FALSE |
| chr18_chr15_49767557_10376 | chr18 | + | 14897758 | 14897830 | chr15 | - | 49767557 | 49767642 | CAAATGAATAAAATGTCCCTGC | 113 | CAAGAAAGTTGGTGCTAGTG | 114 | TRUE |
| chr18_chr15_49764888_10678 | chr18 | + | 14900396 | 14900484 | chr15 | - | 49764888 | 49764984 | CCCATTTGAAGTTACAGTTTT | 115 | TGAAGACTTAAGGGGCAAAG | 116 | TRUE |
| chr18_chr15_49764618_10848 | chr18 | + | 14900681 | 14900752 | chr15 | - | 49764618 | 49764686 | TGGCCATGTGACCAAGTTT | 117 | GGAAGACTTTTGGAAGTTGC | 118 | TRUE |
| chr18_chr15_49764491_10911 | chr18 | + | 14900816 | 14900879 | chr15 | - | 49764491 | 49764554 | TGGAATTTGGATTCTACCTTG | 119 | TCCATTTAGCTCACCGTCC | 120 | TRUE |
| chr18_chr11_134865396_11212 | chr18 | + | 14902774 | 14902853 | chr11 | - | 134865396 | 134865477 | CCCTACTAGGTACCTGGGT | 121 | GCTTCTAGTATCCCCATTACTTAT | 122 | TRUE |
| chr18_chr11_134862091_11504 | chr18 | + | 14904079 | 14904168 | chr11 | - | 134862091 | 134862190 | GAGCCCCAACATGGTTTC | 123 | GGAAGAGAACTGATCACGATA | 124 | FALSE |
| chr18_chr11_134861855_11588 | chr18 | + | 14904307 | 14904405 | chr11 | - | 134861855 | 134861933 | GAGGCTGGATGGGAATGA | 125 | GAGGTTAGCTCAGGCCCC | 126 | FALSE |
| chr18_chr11_134861731_11642 | chr18 | + | 14904426 | 14904529 | chr11 | - | 134861731 | 134861824 | CACTTTACCAGGCACACAGG | 127 | AGATCAATGGAGTGAGAGAGG | 128 | TRUE |
| chr18_chr11_134861427_11784 | chr18 | + | 14904741 | 14904829 | chr11 | - | 134861427 | 134861515 | AGTGAGGTGGTTGGATTACA | 129 | CCTCTGACACATGTATTGATT | 130 | TRUE |
| chr18_chr11_134860951_11981 | chr18 | + | 14905646 | 14905708 | chr11 | - | 134860951 | 134861013 | TGGCAAAGACCTAGAAAGGG | 131 | AAGCCTTTGTGGGGTAAA | 132 | TRUE |
| chr18_chr11_134859592_12187 | chr18 | + | 14907561 | 14907668 | chr11 | - | 134859592 | 134859699 | ATGGACACGGCTCCTCT | 133 | CATCAATCAGACAGCCTCG | 134 | TRUE |
| chr18_chr11_134857849_12341 | chr18 | + | 14908047 | 14908139 | chr11 | - | 134857849 | 134857941 | TGGAACCTGAGCGCTTGA | 135 | AAGGTCAGTGGTGTTTG | 136 | TRUE |
| chr18_chr11_134856878_12657 | chr18 | + | 14908803 | 14908904 | chr11 | - | 134856878 | 134856972 | GGAAGGCATTCAGAGGACAT | 137 | CAGCGGGGTCCTAGTTCT | 138 | TRUE |
| chr18_chr11_134856552_12800 | chr18 | + | 14937324 | 14937419 | chr11 | - | 134856552 | 134856647 | ACACTCAACAAAGTGTGGCA | 139 | GGCACCTGCTGCTCTTTTG | 140 | TRUE |
| chr18_chr11_134855015_12921 | chr18 | + | 14937881 | 14937957 | chr11 | - | 134855015 | 134855091 | TCTCTGGGTGACTGGGCC | 141 | TGTTTGAGGGCAGAGATGT | 142 | TRUE |
| chr18_chr11_134854359_12950 | chr18 | + | 14938526 | 14938618 | chr11 | - | 134854359 | 134854451 | CATTCCGGAAGGGTCTGAG | 143 | ATTAATACTGCCATGTCCTG | 144 | TRUE |
| chr18_chr11_134853439_13116 | chr18 | + | 14939483 | 14939557 | chr11 | - | 134853439 | 134853503 | CCTGGCTCTAGGCACTCA | 145 | AATTAGGGCTAGGGGTGT | 146 | FALSE |
| chr18_chr11_134852942_13214 | chr18 | + | 14939983 | 14940028 | chr11 | - | 134852942 | 134853030 | ATTAACCTTTCAGTTACTCTGG | 147 | ATGTCGGCTTTCTATATTGC | 148 | TRUE |
| chr18_chr11_134852658_13295 | chr18 | + | 14940108 | 14940186 | chr11 | - | 134852658 | 134852739 | GGTAGGTATGTGCTCATCCACCC | 149 | TGGGAGTTGGGAGCCCACT | 150 | FALSE |
| chr18_chr11_134852482_13384 | chr18 | + | 14940295 | 14940382 | chr11 | - | 134852482 | 134852549 | CAGCATCTCTCCGAAAAC | 151 | GGCTCCACAGCACTGGCT | 152 | TRUE |
| chr18_chr11_134851953_13507 | chr18 | + | 14940832 | 14940892 | chr11 | - | 134851953 | 134852012 | AAAGTCAAAGATGTGGCAGGA | 153 | GAAGGCACCGGATTTAGCTCT | 154 | TRUE |
| chr18_chr11_134851733_13593 | chr18 | + | 14941030 | 14941090 | chr11 | - | 134851733 | 134851813 | TCCATCCCCAGGATAACACT | 155 | GAGTATGGTTTCCATGGTAGGA | 156 | TRUE |
| chr18_chr11_134851250_13727 | chr18 | + | 14941535 | 14941637 | chr11 | - | 134851250 | 134851352 | CAATCTTGGGGACCCCTT | 157 | TCTCTGGAAATGGAGACCTGC | 158 | FALSE |
| chr18_chr11_134850169_14096 | chr18 | + | 14942598 | 14942682 | chr11 | - | 134850169 | 134850247 | TGGGGATCATCAGTCTGT | 159 | GAGAGGTGCAGTCAGGGC | 160 | TRUE |
| chr18_chr11_134848949_14140 | chr18 | + | 14943832 | 14943920 | chr11 | - | 134848949 | 134849031 | AATGTCGCGTTCAGGGAG | 161 | TCCCTTAGGTGCACAACTT | 162 | TRUE |
| chr18_chr11_134848705_14212 | chr18 | + | 14944078 | 14944168 | chr11 | - | 134848705 | 134848790 | CCTGCTGTTAGGGTGCAA | 163 | AGGGGCCCACTGTGACTA | 164 | TRUE |
| chr18_chr11_134848524_14277 | chr18 | + | 14944263 | 14944344 | chr11 | - | 134848524 | 134848605 | GGCTGGGCTAGACTTGGG | 165 | CCACGGCAAGTGAGGGAG | 166 | TRUE |
| chr18_chr11_134848390_14322 | chr18 | + | 14944470 | 14944538 | chr11 | - | 134848390 | 134848398 | AGGGTCCATGATGTGGG | 167 | CTGGAGTTCCTGGTCCT | 168 | TRUE |
| chr18_chr11_134848013_14373 | chr18 | + | 14944780 | 14944859 | chr11 | - | 134848013 | 134848092 | TGCCCAGCCTGTGATATTT | 169 | CTTGGCCTAAGGAGGATTGA | 170 | TRUE |
| chr18_chr11_134847819_14397 | chr18 | + | 14944954 | 14945033 | chr11 | - | 134847819 | 134847918 | ACCTCCTGGGTGAGCTGG | 171 | CCAGGCCATCTGGCATCT | 172 | FALSE |
| chr18_chr11_134847731_14454 | chr18 | + | 14945068 | 14945161 | chr11 | - | 134847731 | 134847807 | CTTGGCTAAGTCGGCCCC | 173 | TCCTGCCCTCTAGAAAGGA | 174 | FALSE |
| chr18_chr11_134846465_14775 | chr18 | + | 14946310 | 14946396 | chr11 | - | 134846465 | 134846549 | CCTTTACCGTGGAAGCAGG | 175 | CCACCCAGGCTAGAACTTCA | 176 | FALSE |
| chr18_chr11_134845789_14923 | chr18 | + | 14946974 | 14947070 | chr11 | - | 134845789 | 134845885 | GTGACTAGCCCAGATCACCAG | 177 | TGGAACACAGTAAATCCGAGA | 178 | TRUE |
| chr18_chr11_134844085_15092 | chr18 | + | 14947767 | 14947842 | chr11 | - | 134844085 | 134844156 | AGGGGCCGGCAAGAGTCAG | 179 | TAACTTGGCCTCCCCATC | 180 | TRUE |
| chr18_chr11_134843812_15377 | chr18 | + | 14947894 | 14948003 | chr11 | - | 134843812 | 134843971 | GCCAGGTAGGCTCCTTTC | 181 | GTACAGCAGCATGGGGGAT | 182 | TRUE |
| chr18_chr11_134843591_15315 | chr18 | + | 14948239 | 14948335 | chr11 | - | 134843591 | 134843687 | GGGATGGGCACCAAACTC | 183 | ATGACACCTGCCACTTGC | 184 | FALSE |
| chr18_chr11_134843137_15433 | chr18 | + | 14948642 | 14948769 | chr11 | - | 134843137 | 134843234 | TCTGATTTGGCCTTTGGAA | 185 | TCAACTACAACTACATGAGGA | 186 | TRUE |
| chr18_chr11_134842932_15499 | chr18 | + | 14948897 | 14948994 | chr11 | - | 134842932 | 134843029 | TAGCCAATTTGGAGCTCACT | 187 | AGGAGAGACGTTCGTATCCAA | 188 | FALSE |
| chr18_chr11_134841776_15906 | chr18 | + | 14950088 | 14950148 | chr11 | - | 134841776 | 134841836 | CAGACAAGGGCATCAGTC | 189 | CCCTGAGGGATTGGCAAG | 190 | TRUE |
| chr18_chr11_134839767_16003 | chr18 | + | 14952089 | 14952165 | chr11 | - | 134839767 | 134839838 | TGCAAATCACCTTGTTCA | 191 | TCAATCTTCAAATATGCCA | 192 | FALSE |
| chr18_chr11_134839236_16656 | chr18 | + | 14952599 | 14952686 | chr11 | - | 134839236 | 134839323 | GTTAAACAGAAGTAGTGAGAACAG | 193 | CCACAGCCAGCCTCCTAC | 194 | TRUE |
| chr18_chr11_134838547_16786 | chr18 | + | 14953057 | 14953137 | chr11 | - | 134838547 | 134838627 | GTTTGAGTTACTGTCTAATGTCC | 195 | CACTAGAAGGACCTGCCCTG | 196 | TRUE |

FIG. 8

FIG. 8 continued

| markerName | chr.tar | strand.tar | start.tar | end.tar | chr.ref | strand.ref | start.ref | end.ref | Fprimer | SEQ ID NO. | Rprimer | SEQ ID NO. | included.in.final |
|---|---|---|---|---|---|---|---|---|---|---|---|---|---|
| chr18_chr11_134834736_16873 | chr18 | + | 14857117 | 14857185 | chr11 | - | 134834736 | 134834804 | AGCCCAGTTTTCTAGGGA | 197 | AGTTAGATCCTAATTCCCAAA | 198 | TRUE |
| chr18_chr11_134832730_17302 | chr18 | + | 14859137 | 14859198 | chr11 | - | 134832730 | 134832792 | AAAAGGAGTGGGTGTCCA | 199 | CACAGTTCCATTCATGGTC | 200 | TRUE |
| chr18_chr11_134832507_17382 | chr18 | + | 14859358 | 14859419 | chr11 | - | 134832507 | 134832568 | GGCCTTATCGTAAATGTTTT | 201 | CTTCATGTTCTACTGTTCCC | 202 | TRUE |
| chr18_chr11_134832222_17469 | chr18 | + | 14859642 | 14859704 | chr11 | - | 134832222 | 134832284 | CCCTAAGAAAGGAGGACTA | 203 | GATGCCGACCAAACCCTG | 204 | TRUE |
| chr18_chr11_134831050_17925 | chr18 | + | 14860794 | 14860884 | chr11 | - | 134831050 | 134831140 | GTGGGAAAAGTTAGTGTCAA | 205 | TTTTCAACTCTGTCACTTCC | 206 | TRUE |
| chr18_chr11_134829960_17756 | chr18 | + | 14861698 | 14861794 | chr11 | - | 134829960 | 134830056 | CGTTGAACCCGATTTCCT | 207 | ACAAACGCTGCTGAAGCC | 208 | FALSE |
| chr18_chr11_134829870_18046 | chr18 | + | 14862202 | 14862284 | chr11 | - | 134829870 | 134829952 | CAGCAGAGCCCTGACCTC | 209 | ACCTCTGCGATGACTGCC | 210 | FALSE |
| chr18_chr11_134828074_18225 | chr18 | + | 14863018 | 14863080 | chr11 | - | 134828074 | 134828136 | AGTGGGCAGACTCTGGAA | 211 | TGCCTCTCAGCCTTCCCA | 212 | FALSE |
| chr18_chr11_134827861_18342 | chr18 | + | 14863209 | 14863276 | chr11 | - | 134827861 | 134827926 | CTTGGGGTAGGTGGTGTCC | 213 | TTATGCCCTGAAGTGGTTTC | 214 | TRUE |
| chr18_chr11_134827622_18475 | chr18 | + | 14863442 | 14863512 | chr11 | - | 134827622 | 134827690 | ACACCCGGGAAAGCTTAG | 215 | GGATGTCAGCTTGGAGCC | 216 | FALSE |
| chr18_chr11_134827287_18643 | chr18 | + | 14863795 | 14863866 | chr11 | - | 134827287 | 134827356 | CTGCACCTCCGGCTCGT | 217 | GAGTCCTGCTCCCAGCT | 218 | FALSE |
| chr18_chr11_134826021_18872 | chr18 | + | 14865012 | 14865111 | chr11 | - | 134826021 | 134826121 | TCTGGCCATCAATGACTTCT | 219 | TGGACGAGGAAAGTAAGTAGA | 220 | TRUE |
| chr18_chr11_134825657_19296 | chr18 | + | 14865400 | 14865476 | chr11 | - | 134825657 | 134825732 | CAGGGTCTCAGGCAGGGT | 221 | GCATGAAGATAAATGGCTTG | 222 | TRUE |
| chr18_chr11_134825282_19275 | chr18 | + | 14865771 | 14865843 | chr11 | - | 134825282 | 134825354 | ACATGGAGCCACGGTCAG | 223 | TGTAGCCATGTGACCCATC | 224 | TRUE |
| chr18_chr11_134824838_19496 | chr18 | + | 14866233 | 14866314 | chr11 | - | 134824838 | 134824919 | GGGGAATTAGGGCCTGTG | 225 | ACCCACAGAGTGCGGCAG | 226 | TRUE |
| chr18_chr11_134824600_19647 | chr18 | + | 14866460 | 14866553 | chr11 | - | 134824600 | 134824693 | GTGAACGCTCACTCACTC | 227 | CTCCTTGGACCCAGGCAC | 228 | TRUE |
| chr18_chr11_134824346_19700 | chr18 | + | 14866713 | 14866808 | chr11 | - | 134824346 | 134824443 | GTGGTATCAGGGGAACCT | 229 | CCTGTGGCATTTGGATTTA | 230 | FALSE |
| chr18_chr11_134823663_19848 | chr18 | + | 14867466 | 14867532 | chr11 | - | 134823663 | 134823735 | AAGAGGGTGGGTGTGGTCTG | 231 | GTGGATGTCATTCCCGCTGT | 232 | TRUE |
| chr18_chr11_134822907_20016 | chr18 | + | 14868068 | 14868154 | chr11 | - | 134822907 | 134822969 | CCTCCAAGTTTGTGTCCT | 233 | AATAGAGTAGAATGAAAGCCAC | 234 | TRUE |
| chr18_chr11_134822676_20026 | chr18 | + | 14868282 | 14868378 | chr11 | - | 134822676 | 134822774 | CTCTTGTTAAGGCTGAAAG | 235 | TCCATAGGTTGGCAGATG | 236 | FALSE |
| chr18_chr11_134820971_20086 | chr18 | + | 14870038 | 14870097 | chr11 | - | 134820971 | 134821030 | TGAATGGCATCAGCTTAG | 237 | TATTGAATCGAATCCGCAC | 238 | FALSE |
| chr18_chr11_134820773_20590 | chr18 | + | 14870213 | 14870294 | chr11 | - | 134820773 | 134820854 | GACTCCACTTCAGATTGG | 239 | TGGCTAGGGTAGGGATTC | 240 | FALSE |
| chr18_chr11_134820274_20808 | chr18 | + | 14870696 | 14870795 | chr11 | - | 134820274 | 134820370 | ACCTCACCCTCACCTGAA | 241 | TTAGAGGCCTTCTTTAGGGAG | 242 | TRUE |
| chr18_chr11_134820014_20990 | chr18 | + | 14870976 | 14871064 | chr11 | - | 134820014 | 134820093 | GCCTAATAGGAACCTATTGATGA | 243 | GGACTGAAGATCTAGACGGATA | 244 | TRUE |
| chr18_chr11_134819887_21082 | chr18 | + | 14871104 | 14871172 | chr11 | - | 134819887 | 134819955 | TTGGCTATTGACACCAAC | 245 | GGATCAGAGCACCGCAGCC | 246 | TRUE |
| chr18_chr11_134819562_21104 | chr18 | + | 14871418 | 14871507 | chr11 | - | 134819562 | 134819649 | CTTTGCGACTGCCCTGAT | 247 | AGCCATATGCCTTTCGCAT | 248 | TRUE |
| chr18_chr11_134818707_21528 | chr18 | + | 14872271 | 14872362 | chr11 | - | 134818707 | 134818795 | GTTTAATGGAATTCTGTTGG | 249 | TGCATAGGCTAAACTGGCAA | 250 | TRUE |
| chr18_chr11_134817583_21707 | chr18 | + | 14873379 | 14873443 | chr11 | - | 134817583 | 134817647 | CAGCAGTGCAAGGTCCTG | 251 | CCGTCTGTGTCAGGCTGG | 252 | FALSE |
| chr18_chr11_134814783_22067 | chr18 | + | 14874379 | 14874451 | chr11 | - | 134814783 | 134814855 | AGCAGAGCCAGAGTTCTTT | 253 | CAGCAGATTTGCAGGCTTA | 254 | TRUE |
| chr18_chr11_134814586_22126 | chr18 | + | 14874551 | 14874628 | chr11 | - | 134814586 | 134814663 | CCTGTTCCTCCTGCTCCC | 255 | AGCAAATATTCACCATGCAG | 256 | FALSE |
| chr18_chr11_134814183_22225 | chr18 | + | 14874951 | 14875027 | chr11 | - | 134814183 | 134814259 | CCTCAGTAGGTCCCTGCC | 257 | CCTCACAATTAGAACAGCA | 258 | FALSE |
| chr18_chr11_134813382_22469 | chr18 | + | 14875748 | 14875836 | chr11 | - | 134813382 | 134813475 | TTGCCACGAGGATGAGGT | 259 | TTTGAAGACCAGAGGTTGGG | 260 | FALSE |
| chr18_chr11_134812805_22603 | chr18 | + | 14876212 | 14876284 | chr11 | - | 134812805 | 134812897 | TGGATTATCATTGTTCTGGGC | 261 | CATTTCAGCAGACAGCAT | 262 | FALSE |
| chr18_chr11_134812234_22803 | chr18 | + | 14877010 | 14877083 | chr11 | - | 134812134 | 134812207 | ATTCTCAGAGGCCCAGGGA | 263 | AGCATGTGAGCCTCTCGC | 264 | FALSE |
| chr18_chr11_134811061_23003 | chr18 | + | 14878082 | 14878160 | chr11 | - | 134811081 | 134811159 | TGCTGGGACATCGGGGTA | 265 | CAACTGAGAAAATAGTGTTACTTG | 266 | TRUE |
| chr18_chr11_134810645_23052 | chr18 | + | 14878502 | 14878586 | chr11 | - | 134810645 | 134810709 | TGTTATTGGAGAAGGCAGA | 267 | TACCTGTCTTTCTCTTATTTA | 268 | FALSE |
| chr18_chr11_134810377_23184 | chr18 | + | 14878798 | 14878834 | chr11 | - | 134810377 | 134810468 | AAATACCACCTCCGCTCACA | 269 | GCAGGTGAACGTTATTATCCC | 270 | FALSE |
| chr18_chr11_134810006_23246 | chr18 | + | 14879114 | 14879206 | chr11 | - | 134810006 | 134810098 | AGCTTCCCTTTGGTGTCT | 271 | CTTTCCCAACAGGCCGCT | 272 | TRUE |
| chr18_chr11_134809860_23368 | chr18 | + | 14879318 | 14879412 | chr11 | - | 134809860 | 134809957 | AAGGAGCCCTCGGTGTA | 273 | GTCTTGGAGTTAGGAACCACCA | 274 | FALSE |
| chr18_chr11_134809287_23506 | chr18 | + | 14879864 | 14879929 | chr11 | - | 134809287 | 134809346 | TATACAGCGGCTCATCTTCC | 275 | ACAATCATAATTGAGGCCACC | 276 | TRUE |
| chr18_chr11_134808970_23524 | chr18 | + | 14880141 | 14880238 | chr11 | - | 134808970 | 134809047 | AGTGCTTCATCTTCCCTTTC | 277 | AGCCCCACACACCTGTCTT | 278 | FALSE |
| chr18_chr11_134808528_23650 | chr18 | + | 14880596 | 14880681 | chr11 | - | 134808528 | 134808620 | GGATCTGATAGTTGAGGAGTTCTG | 279 | AAGGGCATGAAGATGGGTC | 280 | TRUE |
| chr18_chr2_132594495_24185 | chr18 | + | 15006780 | 15006858 | chr2 | + | 132594495 | 132594568 | ACCCTACCCCTTATTCCCTG | 281 | CAGGACTGCTGTCCCATTAC | 282 | TRUE |
| chr18_chr2_132594706_24228 | chr18 | + | 15006971 | 15007050 | chr2 | + | 132594706 | 132594785 | CTTCCCATGCTTGTCACAT | 283 | AGGCTCAATAAGATGATGG | 284 | TRUE |
| chr18_chr2_132595162_24367 | chr18 | + | 15007457 | 15007516 | chr2 | + | 132595162 | 132595221 | CCATCACTCGGAACATTGC | 285 | TGTGAGTCCTGCTTGTGAT | 286 | TRUE |
| chr18_chr2_132595546_24473 | chr18 | + | 15007838 | 15007921 | chr2 | + | 132595546 | 132595629 | GGCCATGAATGGGTAGTCAC | 287 | GGTGGGAAGAGCGCAGTTG | 288 | TRUE |
| chr18_chr2_132596791_24739 | chr18 | + | 15009086 | 15009156 | chr2 | + | 132596791 | 132596869 | ATTCAACCCACTATCATGGA | 289 | TTCATCTTTCTTTCCACTCCC | 290 | FALSE |
| chr18_chr2_132604087_24779 | chr18 | + | 15016338 | 15016408 | chr2 | + | 132604087 | 132604128 | TGCATTAGTTTGCTGAATTT | 291 | TGTATAGTTTTGTTCATCTTGG | 292 | TRUE |
| chr18_chr5_38399683_25851 | chr18 | + | 16761298 | 16761380 | chr5 | - | 38399683 | 38399765 | TGCCTCATACTTTGTGGAT | 293 | TGTGAGAAAATTGAAACTTAGA | 294 | TRUE |

FIG. 8 continued

FIG. 8

| markerName | chr.tar | strand.tar | start.tar | end.tar | chr.ref | strand.ref | start.ref | end.ref | Primer | SEQ ID NO. | Primer | SEQ ID NO. | included.in.final |
|---|---|---|---|---|---|---|---|---|---|---|---|---|---|
| [illegible] | [illegible] | + | [illegible] | [illegible] | [illegible] | - | [illegible] | [illegible] | [illegible] | 295 | [illegible] | 296 | TRUE |
| [illegible] | [illegible] | + | [illegible] | [illegible] | [illegible] | + | [illegible] | [illegible] | [illegible] | 297 | [illegible] | 298 | FALSE |
| [illegible] | [illegible] | + | [illegible] | [illegible] | [illegible] | + | [illegible] | [illegible] | [illegible] | 299 | [illegible] | 300 | TRUE |
| [illegible] | [illegible] | + | [illegible] | [illegible] | [illegible] | - | [illegible] | [illegible] | [illegible] | 301 | [illegible] | 302 | TRUE |
| [illegible] | [illegible] | + | [illegible] | [illegible] | [illegible] | - | [illegible] | [illegible] | [illegible] | 303 | [illegible] | 304 | TRUE |
| [illegible] | [illegible] | + | [illegible] | [illegible] | [illegible] | - | [illegible] | [illegible] | [illegible] | 305 | [illegible] | 306 | FALSE |
| [illegible] | [illegible] | + | [illegible] | [illegible] | [illegible] | + | [illegible] | [illegible] | [illegible] | 307 | [illegible] | 308 | TRUE |
| [illegible] | [illegible] | + | [illegible] | [illegible] | [illegible] | + | [illegible] | [illegible] | [illegible] | 309 | [illegible] | 310 | TRUE |
| [illegible] | [illegible] | + | [illegible] | [illegible] | [illegible] | - | [illegible] | [illegible] | [illegible] | 311 | [illegible] | 312 | TRUE |
| [illegible] | [illegible] | + | [illegible] | [illegible] | [illegible] | + | [illegible] | [illegible] | [illegible] | 313 | [illegible] | 314 | FALSE |
| [illegible] | [illegible] | + | [illegible] | [illegible] | [illegible] | - | [illegible] | [illegible] | [illegible] | 315 | [illegible] | 316 | TRUE |
| [illegible] | [illegible] | + | [illegible] | [illegible] | [illegible] | - | [illegible] | [illegible] | [illegible] | 317 | [illegible] | 318 | TRUE |
| [illegible] | [illegible] | + | [illegible] | [illegible] | [illegible] | - | [illegible] | [illegible] | [illegible] | 319 | [illegible] | 320 | TRUE |
| [illegible] | [illegible] | + | [illegible] | [illegible] | [illegible] | - | [illegible] | [illegible] | [illegible] | 321 | [illegible] | 322 | FALSE |
| [illegible] | [illegible] | + | [illegible] | [illegible] | [illegible] | - | [illegible] | [illegible] | [illegible] | 323 | [illegible] | 324 | FALSE |
| [illegible] | [illegible] | + | [illegible] | [illegible] | [illegible] | - | [illegible] | [illegible] | [illegible] | 325 | [illegible] | 326 | TRUE |
| [illegible] | [illegible] | + | [illegible] | [illegible] | [illegible] | - | [illegible] | [illegible] | [illegible] | 327 | [illegible] | 328 | TRUE |
| [illegible] | [illegible] | + | [illegible] | [illegible] | [illegible] | - | [illegible] | [illegible] | [illegible] | 329 | [illegible] | 330 | TRUE |
| [illegible] | [illegible] | + | [illegible] | [illegible] | [illegible] | + | [illegible] | [illegible] | [illegible] | 331 | [illegible] | 332 | TRUE |
| [illegible] | [illegible] | + | [illegible] | [illegible] | [illegible] | + | [illegible] | [illegible] | [illegible] | 333 | [illegible] | 334 | TRUE |
| [illegible] | [illegible] | + | [illegible] | [illegible] | [illegible] | + | [illegible] | [illegible] | [illegible] | 335 | [illegible] | 336 | TRUE |
| [illegible] | [illegible] | + | [illegible] | [illegible] | [illegible] | + | [illegible] | [illegible] | [illegible] | 337 | [illegible] | 338 | FALSE |
| [illegible] | [illegible] | + | [illegible] | [illegible] | [illegible] | + | [illegible] | [illegible] | [illegible] | 339 | [illegible] | 340 | TRUE |
| [illegible] | [illegible] | + | [illegible] | [illegible] | [illegible] | + | [illegible] | [illegible] | [illegible] | 341 | [illegible] | 342 | TRUE |
| [illegible] | [illegible] | + | [illegible] | [illegible] | [illegible] | + | [illegible] | [illegible] | [illegible] | 343 | [illegible] | 344 | TRUE |
| [illegible] | [illegible] | + | [illegible] | [illegible] | [illegible] | + | [illegible] | [illegible] | [illegible] | 345 | [illegible] | 346 | TRUE |
| [illegible] | [illegible] | + | [illegible] | [illegible] | [illegible] | + | [illegible] | [illegible] | [illegible] | 347 | [illegible] | 348 | TRUE |
| [illegible] | [illegible] | + | [illegible] | [illegible] | [illegible] | + | [illegible] | [illegible] | [illegible] | 349 | [illegible] | 350 | TRUE |
| [illegible] | [illegible] | + | [illegible] | [illegible] | [illegible] | + | [illegible] | [illegible] | [illegible] | 351 | [illegible] | 352 | FALSE |
| [illegible] | [illegible] | + | [illegible] | [illegible] | [illegible] | + | [illegible] | [illegible] | [illegible] | 353 | [illegible] | 354 | FALSE |
| [illegible] | [illegible] | + | [illegible] | [illegible] | [illegible] | + | [illegible] | [illegible] | [illegible] | 355 | [illegible] | 356 | TRUE |
| [illegible] | [illegible] | + | [illegible] | [illegible] | [illegible] | + | [illegible] | [illegible] | [illegible] | 357 | [illegible] | 358 | FALSE |
| [illegible] | [illegible] | + | [illegible] | [illegible] | [illegible] | + | [illegible] | [illegible] | [illegible] | 359 | [illegible] | 360 | TRUE |
| [illegible] | [illegible] | + | [illegible] | [illegible] | [illegible] | + | [illegible] | [illegible] | [illegible] | 361 | [illegible] | 362 | TRUE |
| [illegible] | [illegible] | + | [illegible] | [illegible] | [illegible] | + | [illegible] | [illegible] | [illegible] | 363 | [illegible] | 364 | TRUE |
| [illegible] | [illegible] | + | [illegible] | [illegible] | [illegible] | + | [illegible] | [illegible] | [illegible] | 365 | [illegible] | 366 | TRUE |
| [illegible] | [illegible] | + | [illegible] | [illegible] | [illegible] | + | [illegible] | [illegible] | [illegible] | 367 | [illegible] | 368 | TRUE |
| [illegible] | [illegible] | + | [illegible] | [illegible] | [illegible] | + | [illegible] | [illegible] | [illegible] | 369 | [illegible] | 370 | TRUE |
| [illegible] | [illegible] | + | [illegible] | [illegible] | [illegible] | + | [illegible] | [illegible] | [illegible] | 371 | [illegible] | 372 | FALSE |
| [illegible] | [illegible] | + | [illegible] | [illegible] | [illegible] | + | [illegible] | [illegible] | [illegible] | 373 | [illegible] | 374 | TRUE |
| [illegible] | [illegible] | + | [illegible] | [illegible] | [illegible] | + | [illegible] | [illegible] | [illegible] | 375 | [illegible] | 376 | TRUE |
| [illegible] | [illegible] | + | [illegible] | [illegible] | [illegible] | + | [illegible] | [illegible] | [illegible] | 377 | [illegible] | 378 | FALSE |
| [illegible] | [illegible] | + | [illegible] | [illegible] | [illegible] | + | [illegible] | [illegible] | [illegible] | 379 | [illegible] | 380 | TRUE |
| [illegible] | [illegible] | + | [illegible] | [illegible] | [illegible] | + | [illegible] | [illegible] | [illegible] | 381 | [illegible] | 382 | TRUE |
| [illegible] | [illegible] | + | [illegible] | [illegible] | [illegible] | + | [illegible] | [illegible] | [illegible] | 383 | [illegible] | 384 | FALSE |
| [illegible] | [illegible] | + | [illegible] | [illegible] | [illegible] | + | [illegible] | [illegible] | [illegible] | 385 | [illegible] | 386 | TRUE |
| [illegible] | [illegible] | + | [illegible] | [illegible] | [illegible] | + | [illegible] | [illegible] | [illegible] | 387 | [illegible] | 388 | TRUE |
| [illegible] | [illegible] | + | [illegible] | [illegible] | [illegible] | + | [illegible] | [illegible] | [illegible] | 389 | [illegible] | 390 | FALSE |
| [illegible] | [illegible] | + | [illegible] | [illegible] | [illegible] | + | [illegible] | [illegible] | [illegible] | 391 | [illegible] | 392 | TRUE |

FIG. 8

FIG. 8 continued

| marker.tgt | chr.tar | strand.tar | start.tar | end.tar | chr.ref | strand.ref | start.ref | end.ref | Primer | SEQ ID NO | Primer | SEQ ID NO | include.in.final |
|---|---|---|---|---|---|---|---|---|---|---|---|---|---|
| chr19_chr4_22616991_34682 | chr19 | + | 24546444 | 24546535 | chr4 | + | 22616991 | 22617082 | TTGAAACAAGGCATGAATA | 393 | GAGAAGAGATTCCAAGCCAGT | 394 | FALSE |
| chr18_chr4_22619184_34737 | chr18 | + | 24548623 | 24548705 | chr4 | + | 22619184 | 22619266 | GCATGCATTTCATACCATT | 395 | AAGTCATAGAAGAAAATAGCTCA | 396 | TRUE |
| chr19_chr4_22620108_34893 | chr19 | + | 24549556 | 24549645 | chr4 | + | 22620108 | 22620200 | AAAGGTCCTGTTCTTGC | 397 | CACAATAAGACCGTATACATTTG | 398 | TRUE |
| chr18_chr4_22620230_34821 | chr18 | + | 24549675 | 24549763 | chr4 | + | 22620230 | 22620318 | GGGTGATGAGCTAGAGAGTG | 399 | AGGCTAGGCTCAAATGTCAC | 400 | TRUE |
| chr19_chr4_22620523_34920 | chr19 | + | 24549970 | 24550042 | chr4 | + | 22620523 | 22620597 | ATGACCCATTGGTAGGTCCA | 401 | CATGGTAATGGCCAAGGAA | 402 | TRUE |
| chr18_chr4_22622378_35489 | chr18 | + | 24551846 | 24551914 | chr4 | + | 22622378 | 22622441 | TGGAGGTTATTCCAGTCTC | 403 | CCATTTACTTACCTATGTGTC | 404 | FALSE |
| chr19_chr4_22623083_35533 | chr19 | + | 24552502 | 24552570 | chr4 | + | 22623083 | 22623171 | GGTACAAGGAGGCGTGTCAG | 405 | CCAGAGTCCATTCCCAACA | 406 | TRUE |
| chr18_chr4_22623375_35583 | chr18 | + | 24552875 | 24552979 | chr4 | + | 22623375 | 22623473 | AAGACGGCCGAGCTTCAC | 407 | TGTAATCAAGTGACCAAAAGG | 408 | TRUE |
| chr19_chr4_22623799_35730 | chr19 | + | 24553268 | 24553351 | chr4 | + | 22623799 | 22623882 | CACAACAAGTGTGTGCATGA | 409 | TCTACGCACTGTTCCGCA | 410 | TRUE |
| chr18_chr4_22624274_35794 | chr18 | + | 24553764 | 24553849 | chr4 | + | 22624274 | 22624359 | ACCAGGGGCTATGTGCTATT | 411 | GCAGTCAGTATAGTTTGGTGC | 412 | FALSE |
| chr19_chr4_22624508_35859 | chr19 | + | 24553998 | 24554073 | chr4 | + | 22624508 | 22624582 | CCACCATCTGCGCCATAC | 413 | AGATTGTGTGAGGCTGCCAT | 414 | TRUE |
| chr18_chr4_22624650_35886 | chr18 | + | 24554141 | 24554222 | chr4 | + | 22624650 | 22624731 | AAGATCCAATGCAACTTGAAA | 415 | CTGTGTCCACTGTCCACTCC | 416 | TRUE |
| chr19_chr4_22625040_36028 | chr19 | + | 24554532 | 24554593 | chr4 | + | 22625040 | 22625100 | TGGTGCCTGGCAGCTTTC | 417 | TTGTTGGGAGGAGCGGAT | 418 | FALSE |
| chr18_chr4_22626338_36183 | chr18 | + | 24555808 | 24555924 | chr4 | + | 22626335 | 22626451 | GGCAAAGGAAGTTGGCCTT | 419 | GAGACACCTCCTGCTGACT | 420 | FALSE |
| chr19_chr4_22627047_36567 | chr19 | + | 24556567 | 24556642 | chr4 | + | 22627047 | 22627122 | TGAAGGTCACTCAGACAT | 421 | CTTCCCATCCACAGTAGAGA | 422 | TRUE |
| chr18_chr4_22627729_36750 | chr18 | + | 24557250 | 24557346 | chr4 | + | 22627729 | 22627827 | GCAAGGCCTAAAGCAATAGCC | 423 | CTTTAAGCTCAAGGCTTGGT | 424 | TRUE |
| chr19_chr4_22627992_36788 | chr19 | + | 24557507 | 24557586 | chr4 | + | 22627992 | 22628051 | TGCAGTGATGAGTTTGAA | 425 | TTAAATTGCTATCTGTATAGCCC | 426 | TRUE |
| chr18_chr4_22628463_36795 | chr18 | + | 24557942 | 24558040 | chr4 | + | 22628463 | 22628560 | TGCTAAATGAGTCCCCTT | 427 | GCAGATGTCTTTGAGAATCA | 428 | FALSE |
| chr19_chr4_22629460_36893 | chr19 | + | 24559012 | 24559073 | chr4 | + | 22629460 | 22629520 | GTATGTGGAGGCACAGAAGG | 429 | AAAGACAATAGCACAGGGTAA | 430 | FALSE |
| chr18_chr4_22630205_36973 | chr18 | + | 24559770 | 24559837 | chr4 | + | 22630205 | 22630270 | TGCCCTCTAGACCATTGA | 431 | TATGGAAACCAGCAATTGAG | 432 | TRUE |
| chr19_chr4_22631777_37417 | chr19 | + | 24561361 | 24561448 | chr4 | + | 22631777 | 22631868 | CATGTCTAGGATCCATCAATGTG | 433 | TGGTAGATGCGGCTCTGTTC | 434 | TRUE |
| chr18_chr3_97317038_37776 | chr18 | + | 24617730 | 24617801 | chr3 | + | 97317038 | 97317109 | TGCCACTAATCATTATAGCTT | 435 | ACTTAACTAAATGACCTGAAAGA | 436 | FALSE |
| chr19_chr6_96595890_37858 | chr19 | + | 24665910 | 24665988 | chr6 | - | 96595890 | 96595978 | TGTTTTCAGAACAAGACAAGA | 437 | ACAGCTAAGGCAGCTTCT | 438 | FALSE |
| chr18_chr15_78788324_38120 | chr18 | + | 25357786 | 25357878 | chr15 | - | 78788324 | 78788416 | TGGGTGAAGACTGTGAAGCA | 439 | AAAAGGGGCCCCTCACAGC | 440 | FALSE |
| chr19_chr1_60749878_38281 | chr19 | + | 25655067 | 25655159 | chr1 | - | 60749878 | 60749970 | GAGTGCACAGAGTGCTAGAT | 441 | GAACTGGACCAGAGAAACTG | 442 | FALSE |
| chr18_chr5_178279999_38821 | chr18 | + | 25719429 | 25719528 | chr5 | + | 178279999 | 178280098 | ACCTGACTGTTGAACACACT | 443 | CACCATTGGATTTAGCAAC | 444 | TRUE |
| chr19_chr6_133874963_38400 | chr19 | + | 25797789 | 25797865 | chr6 | + | 133874963 | 133875046 | TTCTTTAAATCTGCCTTGGA | 445 | AAGGTAGGAGGAAGGGAATA | 446 | FALSE |
| chr18_chr3_49008077_38734 | chr18 | + | 26283409 | 26283468 | chr3 | + | 49008077 | 49008137 | TTCGGGTACAGGAGGCTA | 447 | CAGAAGACCTGGTTGCAC | 448 | TRUE |
| chr19_chr3_181217464_39110 | chr19 | + | 28083183 | 28083253 | chr3 | - | 181217464 | 181217525 | GCTTAAATTTCAAGCAGTAGTTT | 449 | GGTGGGAATTAAGGGTTA | 450 | TRUE |
| chr18_chr1_32687604_39362 | chr18 | + | 29906037 | 29906938 | chr1 | - | 32687604 | 32687703 | CTGTGCTTGCTTGGATTT | 451 | CACAAGCAGGTCCATGAG | 452 | TRUE |
| chr19_chr1_10473173_39628 | chr19 | + | 29542942 | 29542983 | chr1 | + | 10473173 | 10473254 | GCCAATATTCTCAAGTTCC | 453 | CTGTCCCTGATCTTTGGCA | 454 | TRUE |
| chr18_chr1_10479941_39729 | chr18 | + | 29545998 | 29546062 | chr1 | + | 10479941 | 10480002 | CACACAGTTTATTTGTAAAGTAGC | 455 | CAAGAGGCAAGGGCAGAG | 456 | FALSE |
| chr19_chr17_19188279_39778 | chr19 | + | 29546640 | 29546711 | chr17 | + | 19188279 | 19188350 | GTGTGGGCACAGTGCTAA | 457 | GTCCACAAGTTTGGTAACCT | 458 | TRUE |
| chr18_chr17_19188479_39823 | chr18 | + | 29546968 | 29547047 | chr17 | + | 19188479 | 19188583 | ATTACTCAGAGGCAGAAATCA | 459 | GCAATCTCGGTCATCAGA | 460 | TRUE |
| chr19_chr17_19188684_39835 | chr19 | + | 29547168 | 29547237 | chr17 | + | 19188684 | 19188753 | CATCAAGACAGGCTCCGA | 461 | CTTCAGGGTCTGGATGGC | 462 | TRUE |
| chr18_chr17_19188864_39834 | chr18 | + | 29547345 | 29547414 | chr17 | + | 19188864 | 19188930 | GGCCCAGGACTCTCAAAAC | 463 | GATCTGGTTGCTGCCCAT | 464 | TRUE |
| chr19_chr17_2595079_39897 | chr19 | + | 29776271 | 29776345 | chr17 | + | 2595079 | 2595153 | GGAGCCGTTGCGGTAGAT | 465 | GAGTACCTGAAGTGCCTGACCC | 466 | FALSE |
| chr18_chr17_2595688_40010 | chr18 | + | 29776314 | 29776379 | chr17 | + | 2595688 | 2595744 | TGGTACTTGGTGCTGACGG | 467 | GGAGTACCACCTGTGCTG | 468 | FALSE |
| chr19_chr14_70869725_40235 | chr19 | + | 9350770 | 9350867 | chr14 | + | 70869725 | 70869822 | TCCTGGCAATCTTATCTTGA | 469 | CTTAGGTGAAAATTGATACCCC | 470 | TRUE |
| chr18_chr4_79907119_40404 | chr18 | + | 30685000 | 30685072 | chr4 | - | 79907119 | 79907188 | TCATGTTGCACCAGAGAG | 471 | GCTCAGGATGGACAACTG | 472 | TRUE |
| chr19_chr11_37679264_40600 | chr19 | + | 31695483 | 31695545 | chr11 | - | 37679264 | 37679344 | GCACATGCACAGTGGGGA | 473 | CACCCCAACCTTTTATTGC | 474 | FALSE |
| chr18_chr3_55879961_40673 | chr18 | + | 31757485 | 31757568 | chr3 | - | 55879961 | 55880043 | GCTGAGTGTGAGTCCCCTT | 475 | AGCCTAGGCTGAACACACCT | 476 | TRUE |
| chr19_chr19_35016553_40826 | chr19 | + | 32953461 | 32953535 | chr19 | - | 35016553 | 35016625 | AGCTGCTTCTCACGGGC | 477 | CAGAGAAGCTAGCACCTTCAGA | 478 | TRUE |
| chr18_chr1_56708381_41351 | chr18 | + | 33560979 | 33561071 | chr1 | - | 56708381 | 56708473 | AAATTGTGCTAACTGGTGCC | 479 | AAAGCAGCTACCATGACCATT | 480 | FALSE |
| chr19_chr16_50081446_41540 | chr19 | + | 33906917 | 33906982 | chr16 | - | 50081446 | 50081511 | TGCTAACATTCCAGTGAGTT | 481 | GCAGATTACAATGGCAGA | 482 | FALSE |
| chr18_chr12_48823158_41540 | chr18 | + | 3416551 | 3416610 | chr12 | + | 48823158 | 48823217 | AGGGCCTCTTCCTGTCCTG | 483 | ATGAGAATAAACTAAACACACCC | 484 | TRUE |
| chr19_chr3_152847238_41546 | chr19 | + | 34415234 | 34415302 | chr3 | - | 152847238 | 152847307 | GCTGATGAGTCCAAAAGC | 485 | TCCATGGAGATTGAAGCAT | 486 | TRUE |
| chr18_chr19_46640693_41946 | chr18 | + | 3456446 | 3456507 | chr19 | - | 46640693 | 46640757 | AAGGAGAAGGGGCAAGCT | 487 | GGTGCTCATACAGCCAATC | 488 | FALSE |
| chr19_chr19_46640298_42045 | chr19 | + | 3457858 | 3457938 | chr19 | - | 46640298 | 46640373 | GGGCCAAACCGAACCCTA | 489 | GATCACTGATGGAGGAGCC | 490 | FALSE |

FIG. 8

FIG. 8 continued

| marker.name | chr.tar | strand.tar | start.tar | end.tar | chr.ref | strand.ref | start.ref | end.ref | Fprimer | SEQ ID NO. | Rprimer | SEQ ID NO. | included.in.final |
|---|---|---|---|---|---|---|---|---|---|---|---|---|---|
| chr18_chr19_46639995_42129 | chr18 | + | 3457885 | 3457945 | chr19 | - | 46639995 | 46640057 | ATGTTGCACTCAAAGGGG | 491 | AAATGGGTTAAGCTGTAAGTT | 492 | TRUE |
| chr18_chr12_104940970_42440 | chr18 | + | 3482593 | 3482659 | chr12 | + | 104940970 | 104941031 | GTCTGGAGTCTTCAGCTTG | 493 | AGAACTGTTGGATGACACC | 494 | TRUE |
| chr18_chr1_104022567_42499 | chr18 | + | 35226120 | 35226185 | chr1 | - | 104022507 | 104022570 | TTAACCCTACTCTTAAATTGG | 495 | ACTTGTAACTTTGCTGCC | 496 | TRUE |
| chr18_chr15_85343151_42646 | chr18 | + | 3580160 | 3580228 | chr15 | + | 85343151 | 85343210 | AAAGCCTGATTTGAGGCA | 497 | ACCTGAGGGGAATGTCCA | 498 | TRUE |
| chr18_chr15_85345192_42692 | chr18 | + | 3580329 | 3580404 | chr15 | + | 85345192 | 85345271 | TCAGTGGGGAAATGTAGTT | 499 | AGCTGTCCACCGGGTGCT | 500 | TRUE |
| chr18_chr15_85345444_42686 | chr18 | + | 3580577 | 3580658 | chr15 | + | 85345444 | 85345526 | GGTGCCCATGGAGACTAGAG | 501 | GCAATCTCCTCGCCTCTG | 502 | TRUE |
| chr18_chr6_3951582_43067 | chr18 | + | 36095409 | 36095496 | chr6 | + | 3951092 | 3951189 | CCTGTTTTCCTGCCTTCCT | 503 | ATCTGTCTCCTGACCAA | 504 | TRUE |
| chr18_chr11_23289877_43088 | chr18 | + | 37809135 | 37809194 | chr11 | - | 23289877 | 23289936 | ACCCTATAAGAAAACCCCATA | 505 | CCGTAGTGCCAGGACCGGA | 506 | TRUE |
| chr18_chr16_23289625_43090 | chr18 | + | 37809388 | 37809450 | chr16 | - | 23289625 | 23289707 | TGTCCCATAGGCAGCACAA | 507 | TGCCAAGAAGGACAACAACC | 508 | FALSE |
| chr18_chr19_83679080_43457 | chr18 | + | 39999080 | 39999138 | chr19 | - | 83679080 | 83679076 | CCCTTAAGTCCTGTGGCA | 509 | TTTTGTTTGGAGCCAGGTT | 510 | FALSE |
| chr18_chr7_82738248_43880 | chr18 | + | 41119749 | 41119808 | chr7 | + | 82738248 | 82738307 | CAATGCATTTAAGCAATGA | 511 | ATCTTTGGGTCACAATCC | 512 | FALSE |
| chr18_chr6_90812280_43903 | chr18 | + | 41308577 | 41308637 | chr6 | - | 90812280 | 90812360 | CCCACAAGAATGATAAAGATAGA | 513 | AAAGTGATTTGATTTGACCATCC | 514 | TRUE |
| chr18_chr5_74906153_44063 | chr18 | + | 41467618 | 41467677 | chr5 | + | 74906153 | 74906212 | GATTTGGCTGGGGATGG | 515 | GTGACTCACTCACCTGCTCT | 516 | FALSE |
| chr18_chr6_82081208_44190 | chr18 | + | 41620830 | 41620887 | chr6 | - | 82081208 | 82081233 | AGGCATTTGGCAACCCTG | 517 | AGGTACTGGCAGTGGTCC | 518 | TRUE |
| chr18_chr10_26530783_44254 | chr18 | + | 41960842 | 41960909 | chr10 | - | 26530783 | 26530850 | AGTATCTGCAAGGCCAACAA | 519 | GTGGTCTGCTAGTATGTGCT | 520 | FALSE |
| chr18_chr13_21884528_44559 | chr18 | + | 42182964 | 42183032 | chr13 | - | 21884528 | 21884605 | CAAACTAAATCATGATACTCTGC | 521 | TGCCATAGGTTTTATGACTC | 522 | FALSE |
| chr18_chr11_101340863_45047 | chr18 | + | 4287013 | 4287072 | chr11 | - | 101340863 | 101340922 | TGCACCTCTGCTATTGCT | 523 | TCTCCATGGACTAATGAGC | 524 | TRUE |
| chr18_chr1_165726990_45109 | chr18 | + | 43945959 | 43946018 | chr1 | - | 165726990 | 165727049 | GTGGCTCTGTGGCTCCATGT | 525 | ATCCAATGACTGGCATCT | 526 | FALSE |
| chr18_chr17_48940571_45587 | chr18 | + | 43606950 | 43606610 | chr17 | + | 48940571 | 48940731 | GAGAATGAAGCAAAAGGTAAT | 527 | CTTCTTCATCCACCCAAA | 528 | TRUE |
| chr18_chr17_48971282_45608 | chr18 | + | 43609695 | 43609780 | chr17 | + | 48971282 | 48971377 | CAAACTTCCCAGTCATTAAA | 529 | CCCCAAGGCAAAGTATCCA | 530 | TRUE |
| chr18_chr16_72040354_45731 | chr18 | + | 43671728 | 43671790 | chr16 | + | 72040354 | 72040416 | CCATCACCAATACTTAAGACA | 531 | TCTTGGAGCTGATACCTCT | 532 | TRUE |
| chr18_chr1_189082595_45889 | chr18 | + | 43822057 | 43822120 | chr1 | + | 189082595 | 189082758 | GCAGTAGTTCAAGAGCACA | 533 | TGGTCCTCCAGGTAGCTC | 534 | FALSE |
| chr18_chr8_117734893_46070 | chr18 | + | 43849782 | 43849846 | chr8 | - | 117734893 | 117734957 | TCTGCTTCTTCACTTGGG | 535 | CAAATAGCAGCATGTACTGC | 536 | TRUE |
| chr18_chr13_27549735_46113 | chr18 | + | 44300677 | 44300887 | chr13 | + | 27549735 | 27549798 | CACACCTACACACACTTGCT | 537 | CCAAAGCTCTGTCCCTGC | 538 | TRUE |
| chr18_chr3_87661240_46330 | chr18 | + | 44452680 | 44452739 | chr3 | - | 87661240 | 87661298 | TTACCTTTGCCTAAAACCA | 539 | GCAATACCCAACTAATCCC | 540 | TRUE |
| chr18_chr8_133240915_46367 | chr18 | + | 45055470 | 45055532 | chr8 | + | 133240915 | 133240977 | AAAAGTTCAGGAGCCACCAG | 541 | GGTGCATTGCCAGGTCTT | 542 | TRUE |
| chr18_chr3_135243082_46452 | chr18 | + | 45355657 | 45355719 | chr3 | + | 135243082 | 135243144 | ATGTCCTATTCATTCATACCTC | 543 | ATGGCTAAAATACAATGAGTAAG | 544 | TRUE |
| chr18_chr10_5548205_46481 | chr18 | + | 45424620 | 45424719 | chr10 | + | 5548205 | 5548304 | TCTAAGTAATCTGGCAATGTG | 545 | TGGTATATCTGCAACCCA | 546 | TRUE |
| chr18_chr2_221226511_46572 | chr18 | + | 46582264 | 46582329 | chr2 | - | 221226511 | 221226582 | TATGCAAATGGAAAGAGGG | 547 | AAATGTTAAAGGGATTCCA | 548 | FALSE |
| chr18_chr2_221226358_46635 | chr18 | + | 46993425 | 46993494 | chr2 | - | 221226358 | 221226417 | TATGCATACCGAAAAGTTCCC | 549 | TTTTGCACCAACCTATTTAGT | 550 | TRUE |
| chr18_chr6_159858275_46952 | chr18 | + | 47022405 | 47022521 | chr6 | + | 159858275 | 159858371 | AAATTCCTGAAGCAGGTC | 551 | CCCAAGGTCCGGAGCATC | 552 | TRUE |
| chr18_chr12_54575954_46982 | chr18 | + | 47177401 | 47177460 | chr12 | - | 54575954 | 54576013 | GCCTGATGCCAGAGGTCC | 553 | CCTGTGGGTTACGGGGAG | 554 | TRUE |
| chr18_chr10_14270815_47118 | chr18 | + | 48088414 | 48088504 | chr10 | - | 14270815 | 14270905 | AGCAACCTTGGATCATCTG | 555 | AATTACAATTAATCCTCACTG | 556 | TRUE |
| chr18_chr1_35885893_47129 | chr18 | + | 48659157 | 48659242 | chr1 | + | 35885893 | 35885983 | CTCACATCAGGGAAAATGACC | 557 | GACCCACCGCTGAGAAAG | 558 | TRUE |
| chr18_chr11_34708299_47358 | chr18 | + | 48747793 | 48747853 | chr11 | - | 34708299 | 34708362 | TCCAAACACCCCATCTCC | 559 | TGCCTACCCAGTTTGCTATG | 560 | FALSE |
| chr18_chr11_79162775_47399 | chr18 | + | 48768307 | 48768387 | chr11 | - | 79162775 | 79162855 | ACCAGGAGGAGCACACCTTA | 561 | AAAACCTTCACTGGCTTTC | 562 | FALSE |
| chr18_chr14_89247481_47545 | chr18 | + | 49725516 | 49725609 | chr14 | + | 89247481 | 89247574 | GGGCAAAGATGACTGATAC | 563 | TGAGTCAGAGACAAAAGAAT | 564 | TRUE |
| chr18_chr1_18189115_47659 | chr18 | + | 49874318 | 49874382 | chr1 | - | 18189115 | 18189174 | CCATGTACCCACTAGCAGTT | 565 | AAACTGTACAAAAGGCAAGGG | 566 | TRUE |
| chr18_chr7_96791237_47670 | chr18 | + | 50303909 | 50303187 | chr7 | + | 96791237 | 96791316 | TTTCTTCCTCCATGTGTATT | 567 | GAGGTTGGAATAAAGCCA | 568 | TRUE |
| chr18_chr4_102940967_47723 | chr18 | + | 50959249 | 50959346 | chr4 | - | 102940967 | 102940606 | TGACATTAGGCTAGACACTT | 569 | TGATTTAATTCTCCCATCAA | 570 | FALSE |
| chr18_chr6_74169787_47808 | chr18 | + | 52979984 | 52979044 | chr6 | - | 74169787 | 74169847 | GAGGCGTCCCCTGGATAA | 571 | GAGTGGCAAGAAGGAAGCTG | 572 | TRUE |
| chr18_chr13_82566258_47941 | chr18 | + | 53412029 | 53412138 | chr13 | - | 82566258 | 82566318 | TAGCGACCACTGAGGACT | 573 | TCCCCATGAGAGAAGCCC | 574 | TRUE |
| chr18_chr1_227532653_48118 | chr18 | + | 53908629 | 53908688 | chr1 | - | 227532653 | 227532712 | GCCTGGAAGGATATCCTCCT | 575 | TGGCATGCAACTGTACTC | 576 | TRUE |
| chr18_chr3_188090368_48122 | chr18 | + | 54094387 | 54094466 | chr3 | + | 188090368 | 188090446 | TGATTAGTGTTCCCCTGG | 577 | CAAAAGGATGTAAAGAAGATCC | 578 | TRUE |
| chr18_chr12_87586737_48469 | chr18 | + | 55189312 | 55189411 | chr12 | - | 87586737 | 87586836 | CACAGTGACCTCCGGGTT | 579 | GATGTGGAGTTCCATTATAA | 580 | TRUE |
| chr18_chr8_106002050_48593 | chr18 | + | 55401874 | 55401960 | chr8 | - | 106002050 | 106002136 | TGATGCTGTACTGTGGCA | 581 | GGCTCAGGTGCTGAGGA | 582 | FALSE |
| chr18_chr9_116141021_48589 | chr18 | + | 56516224 | 56516288 | chr9 | + | 116141021 | 116141085 | GAGAGCACTGAGGAACTATAGAA | 583 | TACCTCCCCAGCCTCTTT | 584 | TRUE |
| chr18_chr10_79490873_48754 | chr18 | + | 56586186 | 56586259 | chr10 | + | 79490873 | 79491046 | GCAGACGATAAATTGAAGG | 585 | TCGGACCTCAGCTTCCCAT | 586 | TRUE |
| chr18_chr1_131904644_48813 | chr18 | + | 57598208 | 57598291 | chr1 | + | 131904644 | 131904730 | GTTAGGTGTAGTGCTTAAGGG | 587 | TTCTTGCCACAATCACTC | 588 | TRUE |

FIG. 8 continued

FIG. 8

| markerName | chrTar | strand.tar | start.tar | end.tar | chr.ref | strand.ref | start.ref | end.ref | FPrimer | SEQ ID NO. | RPrimer | SEQ ID NO. | included.in.final |
|---|---|---|---|---|---|---|---|---|---|---|---|---|---|
| chr18_chr7_26225961_48201 | chr18 | + | 57639214 | 57639296 | chr7 | + | 26225961 | 26226033 | TCAGGAATATAGGTGAATAACAA | 589 | TGTTGACCTCAATTTGGC | 590 | TRUE |
| chr18_chr7_26226450_48213 | chr18 | + | 57639702 | 57639787 | chr7 | + | 26226450 | 26226544 | TTTGAAATGAAAACCTCACT | 591 | CTAGGTGAGGTCATTGCTG | 592 | TRUE |
| chr18_chr11_18359726_49768 | chr18 | + | 57664201 | 57664268 | chr11 | + | 18359726 | 18359793 | GAAGAATTCTGGGCCAATC | 593 | TGCTTATGATTGGATGTGGA | 594 | TRUE |
| chr18_chr11_18373440_49827 | chr18 | + | 57684690 | 57684769 | chr11 | + | 18373440 | 18373507 | TGAATATGAAGACTTGGGG | 595 | TTCTTGAGGTTTAGTGGAATC | 596 | TRUE |
| chr18_chr11_18379514_49896 | chr18 | + | 57685047 | 57685108 | chr11 | + | 18379514 | 18379575 | GCTGCCAGTAGTACCATCA | 597 | TGTGTTCCTCCCTGCATAAG | 598 | TRUE |
| chr18_chr11_18382172_50006 | chr18 | + | 57685249 | 57685312 | chr11 | + | 18382172 | 18382235 | TGAAAAGTAATTTGGAACGA | 599 | CTCCGAATCTTTTCTTGGA | 600 | TRUE |
| chr18_chr2_180468033_50146 | chr18 | + | 57873934 | 57873932 | chr2 | - | 180468031 | 180468128 | TCGGTTAGTATTCTAAGCAATGTT | 601 | AAGCCATTCCTAAACTAGCA | 602 | TRUE |
| chr18_chr17_10685227_50224 | chr18 | + | 59040269 | 59040343 | chr17 | + | 10685227 | 10685300 | TGTGGGCTCATGGTTAACTG | 603 | AAACGAAAGGCAAAACTGAG | 604 | TRUE |
| chr18_chr5_43570235_50294 | chr18 | + | 59081453 | 59081519 | chr5 | + | 43570235 | 43570297 | TCCTTATCTCCCAGGACAC | 605 | ACAATTTCCCAGTTAGATGA | 606 | TRUE |
| chr18_chr9_5418350_50306 | chr18 | + | 59483067 | 59483146 | chr9 | + | 5418350 | 5418449 | ACTTCCCTGCAGCCCTCT | 607 | CGTCTTGGTCTTCCTCCT | 608 | FALSE |
| chr18_chr9_5418772_50658 | chr18 | + | 59483489 | 59483568 | chr9 | + | 5418772 | 5418848 | GGAGCTGCGACACGGAGA | 609 | CAGCCAGAAAGGATGTGCG | 610 | FALSE |
| chr18_chr22_32738362_50480 | chr18 | + | 59748618 | 59748690 | chr22 | + | 32738362 | 32738139 | TGATACAAACTGAAAGCCTATG | 611 | GCATTAGCCATTTCAGTT | 612 | FALSE |
| chr18_chr22_32738080_50485 | chr18 | + | 59747473 | 59747569 | chr22 | + | 32738080 | 32738087 | TGGTTGGTTGTTGAATACTT | 613 | GAATGCAGCAAAGCATGAG | 614 | TRUE |
| chr18_chr22_32739244_50812 | chr18 | + | 59747729 | 59747831 | chr22 | + | 32739244 | 32739340 | GGCCATAGAGACATAGTCAG | 615 | GGCAAGGAGATATGTCAGC | 616 | FALSE |
| chr18_chr22_32739512_50868 | chr18 | + | 59747981 | 59748055 | chr22 | + | 32739512 | 32739608 | GCTGCCAACCTCCAGACTC | 617 | GCTTATGGATGTAAGCAATG | 618 | TRUE |
| chr18_chr22_32869131_50895 | chr18 | + | 59758654 | 59758725 | chr22 | + | 32869131 | 32869202 | GGAAGACCATCTGATACTATGT | 619 | GAAATGGCCTTATTGCAT | 620 | FALSE |
| chr18_chr22_33872440_50947 | chr18 | + | 59762793 | 59780863 | chr22 | + | 33872440 | 33872512 | GTTTAACCAACTTTAACCAAGA | 621 | TATGCAAATGGAGCTATTG | 622 | FALSE |
| chr18_chr3_22478233_51059 | chr18 | + | 59897254 | 59897306 | chr3 | + | 22478233 | 22478283 | TTGAGTGCTTTGGGTCAC | 623 | TTGAACACAAATTCAGGTTT | 624 | FALSE |
| chr18_chr17_66404287_51377 | chr18 | + | 6202875 | 6202944 | chr17 | - | 66404287 | 66404356 | CCAAAGCTACTGCACATT | 625 | ATTGGTGCAAATTACTGTTT | 626 | TRUE |
| chr18_chr7_29035440_51445 | chr18 | + | 63021885 | 63022030 | chr7 | - | 29035440 | 29035509 | TTAGGTTTTAGACTTGCCC | 627 | TCCTTTGGCTCTATTCTCTT | 628 | FALSE |
| chr18_chr5_78280387_51474 | chr18 | + | 64032427 | 64280528 | chr5 | - | 78280387 | 78280488 | AGCATAATGTTTTATCTCAACTC | 629 | CAAGTAATGATGGGCTTTTA | 630 | TRUE |
| chr18_chr12_98108081_52184 | chr18 | + | 64109402 | 64109552 | chr12 | - | 98108081 | 98108131 | TGTGCACCACCTATTCAA | 631 | GAATCAGTGGTTGGCATGG | 632 | TRUE |
| chr18_chr18_78804362_52288 | chr18 | + | 66344153 | 66344218 | chr18 | + | 78804362 | 78804427 | GGCCAGGACTGGGATCTG | 633 | GCGCTTCAGTGAGAAGCT | 634 | TRUE |
| chr18_chr1_77294221_52380 | chr18 | + | 66536939 | 66536945 | chr1 | - | 77294221 | 77294307 | TGAAACCAATACCAAGGAGT | 635 | TCTCGAGATAAAATGGGCTGT | 636 | TRUE |
| chr18_chr11_94022918_52507 | chr18 | + | 66646408 | 66646474 | chr11 | + | 94022918 | 94022985 | TTGCATCAGAAAGTAAGTGGG | 637 | TTGTAGGCTTATTACAGTGTT | 638 | TRUE |
| chr18_chr17_4801576_52541 | chr18 | + | 67137602 | 67137701 | chr17 | - | 4801576 | 4801675 | AGACCCCTTTGCAGGACT | 639 | GAAGGTGTTTGGTTCCCA | 640 | TRUE |
| chr18_chr11_89501931_52651 | chr18 | + | 67892531 | 67892599 | chr11 | - | 89501931 | 89501998 | GCCTGTGGTTATTGGACTT | 641 | TCCCCTCATCTAAAGGCACA | 642 | TRUE |
| chr18_chr11_89501670_52653 | chr18 | + | 67892776 | 67892856 | chr11 | - | 89501670 | 89501750 | GCACCACGAGAAGTCATGT | 643 | GCATTGGAGGTAGCAATGGT | 644 | TRUE |
| chr18_chr11_89501349_52790 | chr18 | + | 67893080 | 67893178 | chr11 | - | 89501349 | 89501448 | GGAGGCAGAAGGGAAGCTA | 645 | GGAGCGAAAGCTCAAGCTTAAAA | 646 | FALSE |
| chr18_chr11_89501130_52850 | chr18 | + | 67895335 | 67895415 | chr11 | - | 89501130 | 89501190 | GCCTGGATCTGCAACTTACC | 647 | CACAGGTACTGCAACGAG | 648 | FALSE |
| chr18_chr11_24738934_53252 | chr18 | + | 68236559 | 68236642 | chr11 | - | 24738934 | 24739017 | CTTGGCATACACTTTCCCAC | 649 | TCCTCATAGCCACATAACA | 650 | TRUE |
| chr18_chr12_121228972_53386 | chr18 | + | 68865235 | 68865298 | chr12 | - | 121228972 | 121229047 | GTTTCACTGCTGCTGAGTT | 651 | CAATGGCCAGTGAGAAGC | 652 | TRUE |
| chr18_chr9_180141871_53667 | chr18 | + | 6898590 | 6898679 | chr9 | - | 180141871 | 180141965 | GCTCTAGGGATCTGCTGTCTG | 653 | TGACATAAGATCTACCCTC | 654 | TRUE |
| chr18_chr14_99604405_53908 | chr18 | + | 71959079 | 71959148 | chr14 | - | 99604405 | 99604471 | TACTTCACGGCTGTCC | 655 | CTGTGTGTGCTGGGCCTG | 656 | FALSE |
| chr18_chr11_64886992_53988 | chr18 | + | 72057329 | 72057419 | chr11 | + | 64886992 | 64887078 | GCGGCTTGCTACTTCCAG | 657 | TCAAGTCGTGCTCCTGGC | 658 | FALSE |
| chr18_chr22_32494281_53992 | chr18 | + | 72536806 | 72536464 | chr22 | - | 32494281 | 32494350 | TTCTTGCTCAAGTATACTGCT | 659 | GGAGTCACATGACATACAGA | 660 | TRUE |
| chr18_chr22_26045024_54187 | chr18 | + | 723541 | 724052 | chr22 | - | 26045024 | 26045086 | CATCAATTCATGGAAGGATTC | 661 | TGGAAGGAAGGATACAGGA | 662 | FALSE |
| chr18_chr22_26044707_54290 | chr18 | + | 736864 | 736962 | chr22 | - | 26044707 | 26044785 | TCCTACAAGAATATTAGCAGCCC | 663 | GATTGCTGATGGTATGGC | 664 | TRUE |
| chr18_chr10_69862400_54481 | chr18 | + | 74265758 | 74265833 | chr10 | + | 69862400 | 69862480 | TTGGGAAATCGAATGGCA | 665 | AAAATATTCCAAGAGCTTCCA | 666 | TRUE |
| chr18_chr22_26044485_54487 | chr18 | + | 742963 | 743059 | chr22 | - | 26044485 | 26044585 | CAACAGCATATAGTGGAACA | 667 | CCAGGTACAATGATGCCAGA | 668 | TRUE |
| chr18_chr22_26044161_54866 | chr18 | + | 745772 | 745841 | chr22 | - | 26044161 | 26044250 | ACCATTGTCAAGTTCCTAA | 669 | TATTCGTGATTGGGATGAG | 670 | TRUE |
| chr18_chr5_70862384_54862 | chr18 | + | 75145214 | 75145288 | chr5 | - | 70862384 | 70862453 | TGCAGAGCTGAGTAGCTG | 671 | TGGAAACTTTCTGCAAAC | 672 | FALSE |
| chr18_chr5_70860842_54891 | chr18 | + | 75147020 | 75147078 | chr5 | - | 70860842 | 70860903 | CAGAATGAAAGGAACCTCA | 673 | GAGCTTGATTTGAAGGTTTT | 674 | FALSE |
| chr18_chr5_70855809_55083 | chr18 | + | 75147770 | 75147838 | chr5 | - | 70855809 | 70855875 | TGAGGTTCTGGAGTTGTCA | 675 | GGGAGTCTCACAAGGGACA | 676 | TRUE |
| chr18_chr1_160491198_55408 | chr18 | + | 77322039 | 77322115 | chr1 | + | 160491198 | 160491258 | GCAGAAGTTTTATGTTGGAC | 677 | ATTTCCACTCATGCTCAA | 678 | TRUE |
| chr18_chr13_114121022_55421 | chr18 | + | 77867757 | 77867821 | chr13 | + | 114121022 | 114121086 | GGCTTTTCTCTGGCTGGTT | 679 | TTTAACAATATGCCATCCC | 680 | TRUE |
| chr18_chr6_170898787_55540 | chr18 | + | 77871176 | 77871264 | chr6 | + | 170898787 | 170898859 | GACAGCTACACAGGGGCA | 681 | GCACAGAACCCCAGAGTCA | 682 | FALSE |
| chr18_chr6_170898871_55549 | chr18 | + | 77871280 | 77871358 | chr6 | + | 170898871 | 170898947 | GCTAAAGCAGACTTGGACT | 683 | CTCCCAGCCTTTGTAGAG | 684 | FALSE |
| chr18_chr6_170899032_55633 | chr18 | + | 77871445 | 77871524 | chr6 | + | 170899032 | 170899111 | GACAAAGAATACAGACTCATAGA | 685 | CTCAAGGGTTTTGTTGTTGT | 686 | TRUE |

FIG. 8 continued

FIG. 8

| markerName | chr.tar | strand.tar | start.tar | end.tar | chr.ref | strand.ref | start.ref | end.ref | 5'Primer | SEQ ID NO. | 3'Primer | SEQ ID NO. | included.in.final |
|---|---|---|---|---|---|---|---|---|---|---|---|---|---|
| chr18_chr6_170906136_55854 | chr18 | + | 78013931 | 78013993 | chr6 | + | 170906136 | 170906198 | GGAACCAAACGCTTCGACT | 687 | CTTGCATGAGACCAGCTTCA | 688 | TRUE |
| chr18_chr17_25405381_56208 | chr18 | + | 8317357 | 8317449 | chr17 | - | 25405381 | 25405473 | GGAGGAAATGAAGAGTTCA | 689 | ATGCATACCCACTGCTG | 690 | TRUE |
| chr18_chr17_25405172_56249 | chr18 | + | 8317584 | 8317660 | chr17 | - | 25405172 | 25405248 | CAGGCCTTCCACATCAAGT | 691 | TCCTACCTACCTCCCTGGC | 692 | FALSE |
| chr18_chr17_25403804_56352 | chr18 | + | 8318959 | 8319042 | chr17 | - | 25403804 | 25403887 | AGTTACCCACACCTTGGT | 693 | CCCAGCACCACTGAGTTTC | 694 | FALSE |
| chr18_chr17_25402576_56582 | chr18 | + | 8319159 | 8319286 | chr17 | - | 25402576 | 25402662 | ATCGACCCAACATTACAT | 695 | ATGATAGGAAAGTGGGCAA | 696 | FALSE |
| chr18_chr17_25402398_56649 | chr18 | + | 8320393 | 8320459 | chr17 | - | 25402398 | 25402460 | CCAGAGAGCAGAATTCCA | 697 | TCCCACCACTGCAGAAAG | 698 | TRUE |
| chr18_chr17_25398475_56780 | chr18 | + | 8322524 | 8322595 | chr17 | - | 25398475 | 25398546 | CCCCAAGAAAGAGGCTCA | 699 | AAGGGCTGGATCTGGATT | 700 | TRUE |
| chr18_chr17_25397869_57057 | chr18 | + | 8325031 | 8325086 | chr17 | - | 25397869 | 25397886 | ATTGTCTGAACTCAACCCC | 701 | GTCCAAGATCAGACAAGTT | 702 | TRUE |
| chr18_chr17_25394996_57220 | chr18 | + | 8327980 | 8328058 | chr17 | - | 25394996 | 25395058 | GTGGGGAGTGAAGGTTTA | 703 | AACTGTTGTGCAGTGTTG | 704 | TRUE |
| chr18_chr17_25394478_57341 | chr18 | + | 8328498 | 8328584 | chr17 | - | 25394478 | 25394549 | TTCCTGGCAAAGTTGTTC | 705 | ATAGGACATAAGAAATGAAGCC | 706 | FALSE |
| chr18_chr17_25393657_57398 | chr18 | + | 8329344 | 8329436 | chr17 | - | 25393657 | 25393729 | GCAAACTGCATGGACTAA | 707 | GGGGAACAGGGCTGTCTT | 708 | TRUE |
| chr18_chr17_25392314_57422 | chr18 | + | 8330348 | 8330409 | chr17 | - | 25392314 | 25392398 | ATGCAACACCCTTCACTG | 709 | GGCTCCCTAGACATAGCTC | 710 | TRUE |
| chr18_chr17_25391948_57460 | chr18 | + | 8330747 | 8330811 | chr17 | - | 25391948 | 25392004 | GGAGACCGACACTGATGA | 711 | AGATGTCCACAGCACAGA | 712 | TRUE |
| chr18_chr17_25390705_57639 | chr18 | + | 8331982 | 8332052 | chr17 | - | 25390705 | 25390774 | CAAAGACCAGACAAGTTCC | 713 | CCCTGTGTAATTCTTATCTCA | 714 | TRUE |
| chr18_chr1_170021864_57762 | chr18 | + | 8413533 | 8413627 | chr1 | - | 170021864 | 170021958 | TGTATCAGTTGGAGTAGTTACCA | 715 | CTGTCCTGTGAATCCATCCC | 716 | TRUE |
| chr18_chr6_111467571_57874 | chr18 | + | 8607679 | 8608078 | chr6 | - | 111467571 | 111467670 | CGCCCGGCCTGTATATATCTT | 717 | TGGAAAGTTGGCTATTCCTC | 718 | FALSE |
| chr18_chr6_113467388_57934 | chr18 | + | 8608148 | 8608261 | chr6 | - | 113467388 | 113467464 | CCTAATGGGTGTGACTTCT | 719 | TCACCAATGAGCATATGAA | 720 | FALSE |
| chr18_chr13_71497735_57983 | chr18 | + | 8825456 | 8825515 | chr13 | + | 71497735 | 71497859 | GATGGGGACCCAGACTGTT | 721 | CCAGGCAGGGCTCAGTC | 722 | TRUE |
| chr18_chr13_71497983_58042 | chr18 | + | 8825645 | 8825744 | chr13 | + | 71497983 | 71498081 | CAAGTTTGAACGCACATGGT | 723 | TGGTGACGTCCTGTTATTTG | 724 | TRUE |
| chr18_chr13_71498482_58159 | chr18 | + | 8826038 | 8826158 | chr13 | + | 71498482 | 71498494 | GTGGCCGGAATCCTCAAC | 725 | AGCCTGCCTCCTCTCCTC | 726 | FALSE |
| chr18_chr19_59727928_58172 | chr18 | + | 9102856 | 9100951 | chr19 | - | 59727928 | 59728023 | CCTTAGTTTTGGGGGGAG | 727 | GCAGCGGCCTCCCTAAGA | 728 | FALSE |
| chr18_chr8_10846488_58423 | chr18 | + | 9165809 | 9165904 | chr8 | - | 10846488 | 10846584 | ATTCCCTCTCCTGAGCCC | 729 | TGTGAAGTACTTAGAATAGCCA | 730 | FALSE |
| chr18_chr2_203044079_58980 | chr18 | + | 9580938 | 9580991 | chr2 | + | 203044079 | 203044341 | ATTCCTTCCTTGAATGAT | 731 | GATGGACCAGAAACAAGAAAA | 732 | FALSE |
| chr18_chr15_89744898_59003 | chr18 | + | 9814235 | 9814294 | chr15 | - | 89744898 | 89745062 | GCTCTGCGCTGTCTCTCC | 733 | ATCTGCTCGTGCTTCGCC | 734 | FALSE |
| chr18_chr16_67214752_59138 | chr18 | + | 10724312 | 10724387 | chr16 | - | 67214752 | 67214827 | ATTGCAGTGCAGTCCACC | 735 | GAAAGGACTTTTGTTTTCGTTT | 736 | TRUE |
| chr18_chr14_89576488_63461 | chr18 | + | 11884481 | 11884554 | chr14 | + | 89576488 | 89576561 | GAAAGAAAAGGTGAGGCTA | 737 | TACTGTGGAGTGGTGGGCTT | 738 | TRUE |
| chr18_chr21_15337962_66797 | chr18 | + | 14159549 | 14159634 | chr21 | - | 15337962 | 15338073 | TGTATGGTTATATCTGGCT | 739 | GAGCAACAGACGGAACAGA | 740 | TRUE |
| chr18_chr21_15209987_68998 | chr18 | + | 14854194 | 14854077 | chr21 | - | 15209887 | 15210968 | GACTAGGACCTATACCAGACTC | 741 | TTCCGGTCTGTAGCTACTCC | 742 | TRUE |
| chr18_chr10_45860888_69446 | chr18 | + | 14772651 | 14772710 | chr10 | + | 45860888 | 45860903 | ATCAAGGAATCTCTCTGATACTG | 743 | AACCTATATGCTATGTGGC | 744 | TRUE |
| chr18_chr10_37495363_69812 | chr18 | + | 14781336 | 14781389 | chr10 | + | 37495363 | 37495427 | TGCAGTATGTATTTCCCGC | 745 | CAAGGGACAGAGTCCCTGC | 746 | TRUE |
| chr18_chr11_134859004_71903 | chr18 | + | 14869089 | 14907054 | chr11 | - | 134859004 | 134859089 | AAAGACCTGCGATCAATAG | 747 | GAACTTTGACCTCCATAGTTCC | 748 | TRUE |
| chr18_chr2_132580865_83846 | chr18 | + | 14992984 | 14993078 | chr2 | + | 132580865 | 132580957 | CCAGGCAAAAGAGGGCAG | 749 | TGGTACCAGACCATTCCAG | 750 | TRUE |
| chr18_chr2_132597238_84683 | chr18 | + | 15909254 | 15909338 | chr2 | + | 132597238 | 132597334 | TTAGTTCAGCCACTCTGC | 751 | GATGGACAAATCCCAGTAA | 752 | FALSE |
| chr18_chr1_44598173_86015 | chr18 | + | 19193316 | 19193402 | chr1 | - | 44598173 | 44598259 | CATACTCCAGTCGGTCAG | 753 | CACGAGCTTCATCCTAGCGG | 754 | FALSE |
| chr18_chr6_32067588_86849 | chr18 | + | 21015115 | 21015212 | chr6 | - | 32067588 | 32067682 | CCCAGTTCAAGTGGTGTTCC | 755 | GGAGTTCGAGAGTAGCCAAA | 756 | TRUE |
| chr18_chr9_14061371_87051 | chr18 | + | 21680885 | 21680934 | chr9 | + | 14061371 | 14061470 | CAGGGTTTTCTGAAGCAC | 757 | AGGATATCCCAATTAACCC | 758 | FALSE |
| chr18_chr12_59933536_87430 | chr18 | + | 2311185 | 2311268 | chr12 | - | 59933536 | 59933614 | CCTTCAGCCTCCCAATATAA | 759 | GACAAAGGAGGAGGGCTTG | 760 | FALSE |
| chr18_chr15_86126774_89115 | chr18 | + | 24179982 | 24180091 | chr15 | + | 86126774 | 86126873 | AGCATGGATCTCATTGACTC | 761 | TTTTCCCTTCCCCACAAG | 762 | TRUE |
| chr18_chr16_57402541_88586 | chr18 | + | 24180773 | 24180848 | chr16 | - | 57402541 | 57402617 | AGGGCACCAGAGGAACTCAC | 763 | TGTGGTTGGGGATGGAAG | 764 | TRUE |
| chr18_chr4_22587226_89865 | chr18 | + | 24465023 | 24465108 | chr4 | + | 22587226 | 22587305 | TACACCCCTTGTTGTATTGG | 765 | TGGACACTCACCATGTGATA | 766 | TRUE |
| chr18_chr15_82003361_90114 | chr18 | + | 24467690 | 24467775 | chr15 | - | 82003361 | 82003446 | TCAGAACAATATAACGGGG | 767 | CCTAATAGCCAGCGGTGGAG | 768 | TRUE |
| chr18_chr4_22591612_90191 | chr18 | + | 24469396 | 24469480 | chr4 | + | 22591612 | 22591696 | GGTGGGAGAGAAATGATATGACA | 769 | TCTCCCACCCTTGATATT | 770 | TRUE |
| chr18_chr10_7165778_98025 | chr18 | + | 25596148 | 25596247 | chr10 | - | 7165778 | 7165877 | GACTGAAGCCTTGGAGATTAG | 771 | TCATTAACCAGGCCCCATC | 772 | FALSE |
| chr18_chr6_147124081_99014 | chr18 | + | 2594453 | 2594543 | chr6 | + | 147124081 | 147124471 | CCATTGTTTCATGGTCCT | 773 | TCCAGTGATTCTGGTTCTT | 774 | TRUE |
| chr18_chr1_32891139_100298 | chr18 | + | 29805330 | 29805393 | chr1 | - | 32891139 | 32891227 | GCACTCACACTTGCCCAG | 775 | CTTACCTGGCTCTGGAGTTAC | 776 | FALSE |
| chr18_chr1_10477473_101136 | chr18 | + | 29549149 | 29549226 | chr1 | + | 10477473 | 10477550 | TTATGCTGCTAAGGCAGG | 777 | CGCCTGTCCACATCAGGG | 778 | FALSE |
| chr18_chr5_111257793_103133 | chr18 | + | 33457737 | 33457828 | chr5 | - | 111257793 | 111257884 | AGAGACTCACCTCCCCTGG | 779 | TTTCAAAAGGGCTTAAACTT | 780 | FALSE |
| chr18_chr15_58944693_105274 | chr18 | + | 42018303 | 42018368 | chr15 | + | 58944693 | 58944758 | GATTGTCTTTACCCCAATG | 781 | TGATACACCTACAGTGCATTT | 782 | TRUE |
| chr18_chr2_188092814_108883 | chr18 | + | 43796252 | 43796311 | chr2 | + | 188092814 | 188092873 | GTTGGGCGCAAGTCTAGG | 783 | CTTGGCAGCCATAGTGGA | 784 | TRUE |

FIG. 8 continued

FIG. 8

| marker.hm | chr.tar | strand.tar | start.tar | end.tar | chr.ref | strand.ref | start.ref | end.ref | Primer | SEQ ID NO. | Primer | SEQ ID NO | included.in.final |
|---|---|---|---|---|---|---|---|---|---|---|---|---|---|
| chr18_chr8_54184912_105894 | chr18 | + | 44449091 | 44449183 | chr8 | - | 54184912 | 54184881 | TATGGCTTATGGCAGATTC | 785 | GGAATTTAAGTCCCTCTTAGC | 786 | FALSE |
| chr18_chr17_19400297_108872 | chr18 | + | 47017752 | 47017813 | chr17 | - | 19400297 | 19400358 | GATCACAACTATGAATCGCATACC | 787 | CAAATCAAGAGGTTCCAAT | 788 | FALSE |
| chr18_chr2_97094705_109756 | chr18 | + | 47756790 | 47756822 | chr2 | + | 97094705 | 97094795 | CAGAATAAGGAAGGACAGGGCTAAG | 789 | CAAGAGGCTGAAGCTTGTA | 790 | FALSE |
| chr18_chr11_7902856_109850 | chr18 | + | 48102814 | 48102876 | chr11 | - | 7902856 | 7902918 | CACATGGACACAAAGGGACA | 791 | TCCTCTCCCTCCCTCTAT | 792 | TRUE |
| chr18_chr9_20758937_111076 | chr18 | + | 51748687 | 51748771 | chr9 | - | 20758937 | 20757021 | CAGTCCCAGGAGAAGGAT | 793 | ACAGGTCCTCCCTCAGGGT | 794 | TRUE |
| chr18_chr10_79489029_112289 | chr18 | + | 56601715 | 56601795 | chr10 | + | 79489029 | 79489111 | GCATCACACCAGAGAATCCA | 795 | TGAAAGTGCACCGACCTG | 796 | TRUE |
| chr18_chr17_45901800_112599 | chr18 | + | 57687230 | 57687318 | chr17 | + | 45901803 | 45901895 | CAGCAGAATAATGATGATGAA | 797 | CCCATTCAGATGTCTCTCA | 798 | FALSE |
| chr18_chr7_26229828_112879 | chr18 | + | 57838149 | 57838239 | chr7 | + | 26229828 | 26229088 | GAAAGTTGAGCCGTGATGAA | 799 | TTGAAAGAATGAACAGGCAT | 800 | TRUE |
| chr18_chr1_179014059_112984 | chr18 | + | 57877509 | 57877597 | chr1 | + | 179014059 | 179014131 | TACAGCCCGTTTGGTCATC | 801 | GGAAAGGGAACTACCACAAGT | 802 | TRUE |
| chr18_chr12_31479822_113383 | chr18 | + | 57884730 | 57884813 | chr12 | + | 31479822 | 31479889 | GAGAGAGACGCGGAGCCA | 803 | CCCGACGCGAGATGTCTT | 804 | TRUE |
| chr18_chr16_58741070_116849 | chr18 | + | 6879859 | 6879968 | chr16 | - | 58741070 | 58741135 | GGTTCAGGCTTCACCCAC | 805 | CAGGCCGCAATAAGTCTACA | 806 | TRUE |
| chr18_chr22_26049812_118505 | chr18 | + | 747984 | 748087 | chr22 | - | 26049812 | 26049888 | GGAATCTGCAGGCGCTAC | 807 | GGTGGGAAGCAAGATCAAT | 808 | FALSE |
| chr18_chr2_6879234_120025 | chr18 | + | 77772807 | 77772902 | chr2 | + | 6879234 | 6879329 | AAGTAAAGCCCTGTTCCC | 809 | TAGGGAAGGCAACCTTAT | 810 | FALSE |
| chr18_chr3_180042021_123195 | chr18 | + | 9534649 | 9534750 | chr3 | + | 180042021 | 180042102 | GAAACCTCTGCGATGGTCTA | 811 | TCTCCTGATCTCCTCCTTGAT | 812 | TRUE |
| chr18_chr10_18318579_126503 | chr18 | + | 61598851 | 61598653 | chr10 | - | 18318579 | 18318651 | CTAGGGCAGTTAAGTCCTGT | 813 | TTCCCTTCTCTACTCTTAG | 814 | FALSE |
| chr18_chr11_13889667_127522 | chr18 | + | 65037786 | 65037861 | chr11 | + | 13889667 | 13889730 | CGGGACCTTGTTCAACTT | 815 | CCAGTTGCTCACCATAGGTA | 816 | TRUE |
| chr18_chr1_203309417_127683 | chr18 | + | 66079946 | 66079858 | chr1 | - | 203309417 | 203309476 | AGTTAATCACCAAAGCCAAA | 817 | TGTGTTTACTTGCTTGTTGAGTC | 818 | TRUE |
| chr18_chr15_85345616_128382 | chr18 | + | 3580740 | 3580833 | chr15 | + | 85345616 | 85345700 | CAGGTGTGACGGAGACTTT | 819 | TATTTTCCACGGTCCGCA | 820 | TRUE |
| chr18_chr19_58727415_130598 | chr18 | + | 9124882 | 9124983 | chr19 | - | 58727415 | 58727504 | TCCAGGAGACTACACCTGACA | 821 | GAACCATTGGTGCGTTCAC | 822 | FALSE |
| chr18_chr16_67209773_131052 | chr18 | + | 10727773 | 10727837 | chr16 | - | 67209773 | 67209837 | GGCATAGCCTAGGAGCAGC | 823 | ACTTGGGCCACCAAACTCTA | 824 | TRUE |
| chr18_chr4_22485640_131223 | chr18 | + | 13073823 | 13073881 | chr4 | + | 22485640 | 22485601 | AAAATCATGGCCCCTGAAG | 825 | CCTCACTTGTCAGCGGGATA | 826 | TRUE |
| chr18_chr11_15592670_133926 | chr18 | + | 14138024 | 14138094 | chr11 | - | 15592670 | 15592780 | AATATGGTGATGATCTCCTGG | 827 | TGTTCAGCCAGCGTCAAA | 828 | TRUE |
| chr18_chr21_15389517_134206 | chr18 | + | 14141160 | 14141244 | chr21 | - | 15389517 | 15389601 | TGGCCATGAGCAGCAGAT | 829 | CTGATCAGAGCACGCCTC | 830 | FALSE |
| chr18_chr21_15384422_134857 | chr18 | + | 14146183 | 14146252 | chr21 | - | 15384422 | 15384491 | CACTCCGGGCCATTCTTT | 831 | GACTCTGCATCTCCCCGAG | 832 | TRUE |
| chr18_chr21_15384074_134895 | chr18 | + | 14146540 | 14146599 | chr21 | - | 15384074 | 15384131 | ACCTGGCTCTGGCCTTCA | 833 | AGGACATCTCGGAGGGACAGC | 834 | FALSE |
| chr18_chr21_15221565_136725 | chr18 | + | 14836288 | 14836379 | chr21 | - | 15221565 | 15221656 | ACACTACTGGGACGCTGGG | 835 | AACTTGTGGCTGTCCTATG | 836 | FALSE |
| chr18_chr10_45856371_142578 | chr18 | + | 14768162 | 14768223 | chr10 | + | 45856371 | 45856436 | ATTCATGCCTATGTAATAAAGC | 837 | TGGAAGGTCTCTGATCCCA | 838 | FALSE |
| chr18_chr10_57436363_143724 | chr18 | + | 14782591 | 14782640 | chr10 | + | 57436363 | 57436452 | GCAGATAAATAGAGAAGTAGAAAG | 839 | GACAAACATATGGTTAAAAGACT | 840 | TRUE |
| chr18_chr15_40767270_143930 | chr18 | + | 14988031 | 14988107 | chr15 | - | 40767270 | 40767348 | CACTACGGGAGTGCAGGG | 841 | TCACAAAAGATCTATAGAATTGG | 842 | FALSE |
| chr18_chr11_134841347_144624 | chr18 | + | 14960309 | 14960377 | chr11 | - | 134841347 | 134841419 | TCAGACACCTTGGGACTGAG | 843 | CGCACGTAGGGTACAAGAGC | 844 | FALSE |
| chr18_chr11_134841064_144636 | chr18 | + | 14950621 | 14950283 | chr11 | - | 134841064 | 134841128 | TTACCATGGAATCACCTTG | 845 | GGGAGAAAACGAAGGATCAG | 846 | TRUE |
| chr18_chr11_134840259_144734 | chr18 | + | 14961586 | 14961674 | chr11 | - | 134840259 | 134840348 | CTTGACTTGCCTTCCCTG | 847 | ATAGGCATCTCTCCGGAAC | 848 | TRUE |
| chr18_chr11_134834930_144882 | chr18 | + | 14958893 | 14958992 | chr11 | - | 134834930 | 134834992 | CGCTTTAAGCTTTCTGGTTAAT | 849 | TGCCAGATATGGTGGATAAC | 850 | TRUE |
| chr18_chr11_134830132_145019 | chr18 | + | 14960916 | 14961014 | chr11 | - | 134830132 | 134830230 | AGGTAAGGAGGGAGGAAGGC | 851 | GGAAGCCTGAATGGACTAACA | 852 | FALSE |
| chr18_chr11_134828491_145124 | chr18 | + | 14962569 | 14962686 | chr11 | - | 134828491 | 134828598 | GCAAATCTGCATCTCTCAGTC | 853 | AAGCCTCGTATAACTGTGGG | 854 | TRUE |
| chr18_chr11_134821585_145932 | chr18 | + | 14969417 | 14969482 | chr11 | - | 134821585 | 134821650 | TGTACAACCGAGCCACACC | 855 | GCTACTACAGGGAGTGGGCA | 856 | TRUE |
| chr18_chr11_134819350_148178 | chr18 | + | 14971680 | 14971719 | chr11 | - | 134819350 | 134819439 | CAGCATCAAGTGCCATGTG | 857 | AGCTTGGAAGACCGGGAG | 858 | TRUE |
| chr18_chr11_134817797_148870 | chr18 | + | 14973186 | 14973230 | chr11 | - | 134817797 | 134817891 | TGCATCTCACACTGCTTGTA | 859 | GGCACAGTTTGATTAGGGGA | 860 | TRUE |
| chr18_chr2_132574958_146985 | chr18 | + | 14987017 | 14987108 | chr2 | + | 132574958 | 132575050 | TTAATTGGTGCATGTCACTGG | 861 | GCACATGGGGACCTAAGAGA | 862 | TRUE |
| chr18_chr2_132578289_147140 | chr18 | + | 14988357 | 14988428 | chr2 | + | 132578289 | 132578388 | TGTCCTAAAGCCTAGGGCAG | 863 | GCCCTATGATACAGCCATGC | 864 | TRUE |
| chr18_chr2_132588564_147218 | chr18 | + | 15000823 | 15000888 | chr2 | + | 132588564 | 132588630 | CTAGGGTTTGCACGGCAG | 865 | AGCTCAGAGGAGAGGGGCT | 866 | TRUE |
| chr18_chr2_132590380_147946 | chr18 | + | 15002634 | 15002728 | chr2 | + | 132590380 | 132590474 | TACTGTTGACCCATCCCCAC | 867 | CACAGCAAGCTTAGGAGCC | 868 | FALSE |
| chr18_chr2_132590681_148059 | chr18 | + | 15003185 | 15003194 | chr2 | + | 132590681 | 132590940 | ACTTCCTGCCAACCCAT | 869 | TCTCCCTACAGCCTCTGTC | 870 | FALSE |
| chr18_chr2_132591554_148249 | chr18 | + | 15003609 | 15003694 | chr2 | + | 132591554 | 132591639 | AGAAGTGGCCCTGGGGTG | 871 | TGAGATCCCACAACCCCT | 872 | FALSE |
| chr18_chr2_132591904_148315 | chr18 | + | 15004180 | 15004032 | chr2 | + | 132591904 | 132591978 | CTGGCTCAATCTCAGCAGG | 873 | CCAGGTGGTGCCTCTGTT | 874 | FALSE |
| chr18_chr2_132606368_149432 | chr18 | + | 15018561 | 15018685 | chr2 | + | 132606368 | 132606462 | CTCATGGGCCTTTAGACTGG | 875 | TTCCTGTACCAGCGAACAAG | 876 | FALSE |
| chr18_chr2_132607931_149628 | chr18 | + | 15020154 | 15020236 | chr2 | + | 132607931 | 132608013 | CCCACAACAGGTGTGAAGTTT | 877 | CCGCTGCAATGATATGGTTT | 878 | FALSE |
| chr18_chr1_44557924_155211 | chr18 | + | 19038783 | 19038878 | chr1 | - | 44557924 | 44558019 | GAAACGCTGAGTATTCGAGG | 879 | CACCTTAGGTTCAACATCCACA | 880 | FALSE |
| chr18_chr10_100941494_159469 | chr18 | + | 20192050 | 20192141 | chr10 | - | 100941494 | 100941585 | AATGACTAGATCCAACGATATAA | 881 | CTGGTGGGAATGTAACCCAG | 882 | FALSE |

FIG. 8

FIG. 8 continued

| marker.id | chr.tar | strand.tar | start.tar | end.tar | chr.ref | strand.ref | start.ref | end.ref | 5'Primer | SEQ ID NO. | 3'Primer | SEQ ID NO. | included.in.final |
|---|---|---|---|---|---|---|---|---|---|---|---|---|---|
| chr18_chr7_137749023_155996 | chr18 | + | 24162481 | 24162548 | chr7 | - | 137749023 | 137749091 | ACCCTTTGATCTAATGATTCTAA | 883 | TGTGTCATTTGGAACGTGAAT | 884 | TRUE |
| chr18_chr15_82003793_156608 | chr18 | + | 24467264 | 24467343 | chr15 | - | 82003793 | 82003868 | ACACCTTCTTCCATGTTGGG | 885 | TGGTAAAACCCTTTCGAAGG | 886 | TRUE |
| chr18_chr4_22594763_156627 | chr18 | + | 24473241 | 24473320 | chr4 | + | 22594763 | 22594850 | GCAGAGCAAGTAATAGAGGACA | 887 | GGAGTGGAGCTAACAGTGGC | 888 | FALSE |
| chr18_chr4_22608725_157493 | chr18 | + | 24510342 | 24510416 | chr4 | + | 22608725 | 22608799 | GGAGTCAAGTGGGTTGTCAT | 889 | TCCATTTGATGCTCGCTTAG | 890 | TRUE |
| chr18_chr4_22632559_158888 | chr18 | + | 24582150 | 24582209 | chr4 | + | 22632559 | 22632628 | TTCTACTTCCTCGTCTCTTCA | 891 | CATGTTTATAGTTTCTTCCCTTC | 892 | TRUE |
| chr18_chr14_41120258_159846 | chr18 | + | 26042315 | 26042410 | chr14 | + | 41120258 | 41120359 | CATAGCATTATGAGTGGTGACT | 893 | TTCTGCAACAGGCACTTAAA | 894 | TRUE |
| chr18_chr15_89713302_160011 | chr18 | + | 27624859 | 27624914 | chr15 | + | 89713302 | 89713178 | TGCTTGCTTGTTTGATCACC | 895 | CAATGCTAGATTGGAGGCTG | 896 | FALSE |
| chr18_chr1_10464266_160562 | chr18 | + | 29942919 | 29942984 | chr1 | + | 10464266 | 10464331 | GGAGTCAGTGGTGGAGAGGA | 897 | TTCTTTGTTCCCTCCTGGC | 898 | TRUE |
| chr18_chr3_12597498_161510 | chr18 | + | 98271345 | 98271480 | chr3 | - | 12597498 | 12597565 | AACTGGAGATACATAACATGCACA | 899 | TGGTAGTTTCATGAGTGTATGC | 900 | TRUE |
| chr21_chr18_14189894_692 | chr21 | + | 15962095 | 15962159 | chr18 | - | 14189894 | 14189950 | CAAATGTCCATTAACTTGTGA | 901 | TAGGAAGTCAGTAGTTAGTTCC | 902 | TRUE |
| chr21_chr18_14160618_884 | chr21 | + | 15968813 | 15968879 | chr18 | - | 14160618 | 14160684 | CATTGAATGGTCTTACTACTTTT | 903 | AGCCTAGAAAGAAAACTCACA | 904 | TRUE |
| chr21_chr18_14160396_792 | chr21 | + | 15970138 | 15970201 | chr18 | - | 14160396 | 14160492 | CTTCTGACTATTATGTGGTTCTTT | 905 | CCCTATCAAAATCCAATGGTCT | 906 | TRUE |
| chr21_chr18_14151824_872 | chr21 | + | 15978082 | 15978780 | chr18 | - | 14151823 | 14151610 | GGCTGTGGCTGTGTTTAACTA | 907 | GAATGAGGACAGCGGGTCT | 908 | TRUE |
| chr21_chr18_14151400_981 | chr21 | + | 15979109 | 15979194 | chr18 | - | 14151400 | 14151491 | TGTAGCCTTTTAAAGGACAGACTGG | 909 | ACCATGCCTTGCCTCTTT | 910 | FALSE |
| chr21_chr18_14149595_1107 | chr21 | + | 15982938 | 15983020 | chr18 | - | 14149595 | 14149667 | GGTGCTCCGAAGCCATTAG | 911 | CAGCAAACCTGGCACAGG | 912 | FALSE |
| chr21_chr18_14146863_1247 | chr21 | + | 15981874 | 15981935 | chr18 | - | 14146863 | 14146924 | TCTCCCAGGGCTCACCAG | 913 | GCAGCCATGGCAATCCAC | 914 | TRUE |
| chr21_chr18_14146797_1296 | chr21 | + | 15988870 | 15988932 | chr18 | - | 14146797 | 14146798 | CAGTCACTTTTCTTGAGCCT | 915 | TAGTTCAGGATGTGGGCG | 916 | TRUE |
| chr21_chr18_14145654_1336 | chr21 | + | 15984947 | 15985026 | chr18 | - | 14145654 | 14145735 | ACCTCACTGCTGACATC | 917 | AGGGCGAAGGAGACAGGGTC | 918 | FALSE |
| chr21_chr18_14145439_1851 | chr21 | + | 15985159 | 15985231 | chr18 | - | 14145439 | 14145521 | GATAGGAGGAAGGCGGCTC | 919 | GTCCTATCCAGGACCGCATC | 920 | TRUE |
| chr21_chr18_14144680_1484 | chr21 | + | 15986021 | 15986078 | chr18 | - | 14144680 | 14144765 | GGGCCCGTGTCAGGAAGT | 921 | GACAAGGACGAGGACAGGG | 922 | FALSE |
| chr21_chr18_14140948_2016 | chr21 | + | 15989732 | 15989817 | chr18 | - | 14140948 | 14141039 | TTGGGATCATGGCACAGG | 923 | GACCTCAAACCTCCTGCA | 924 | FALSE |
| chr21_chr18_14140133_2083 | chr21 | + | 15990554 | 15990633 | chr18 | - | 14140133 | 14140213 | TCCCCTCTAGACTGTTGG | 925 | CACTACAAGGCGACACCAT | 926 | TRUE |
| chr21_chr18_14139890_2157 | chr21 | + | 15990790 | 15990877 | chr18 | - | 14139890 | 14139979 | TTTAGCAACTGACTGTCATAAG | 927 | TTCCTTGAGGGCTAAGATTAC | 928 | FALSE |
| chr21_chr18_14138979_2290 | chr21 | + | 15991688 | 15991774 | chr18 | - | 14138979 | 14139069 | ATAGCATGCAGGGAGTCACC | 929 | ACTGGGCACAAGAAGCAG | 930 | FALSE |
| chr21_chr18_14138631_2376 | chr21 | + | 15992048 | 15992134 | chr18 | - | 14138631 | 14138719 | TGAGTTCGCTCCTGGGTC | 931 | GCTTGGGTAAGAAGGGGTCT | 932 | FALSE |
| chr21_chr18_14138240_2413 | chr21 | + | 15992453 | 15992514 | chr18 | - | 14138240 | 14138299 | TCCTGTCCCTCTCACCTTG | 933 | CAGAAACAGCAATGCTAGATCA | 934 | TRUE |
| chr21_chr18_14137703_2666 | chr21 | + | 15992980 | 15993051 | chr18 | - | 14137705 | 14137762 | TGAGGTCTTGGATGGAGG | 935 | TCAACATGGAATGGGGAACT | 936 | TRUE |
| chr21_chr18_14137129_2740 | chr21 | + | 15993580 | 15993659 | chr18 | - | 14137129 | 14137175 | GAGTCCTGCCATAGCATCAA | 937 | GCCTCTGCATTCTAGTCCC | 938 | TRUE |
| chr21_chr18_14136252_2871 | chr21 | + | 15994425 | 15994505 | chr18 | - | 14136252 | 14136332 | GGACTCACTGACCTCCCTC | 939 | CTGGTTCTGTCCATGTGCC | 940 | TRUE |
| chr21_chr18_14135724_3187 | chr21 | + | 15994981 | 15995029 | chr18 | - | 14135724 | 14135821 | GGTGAAAATGGACTTGGACCT | 941 | AAGATCCGTCATCGGAAGG | 942 | FALSE |
| chr21_chr18_14135383_3258 | chr21 | + | 15995283 | 15995368 | chr18 | - | 14135383 | 14135470 | GGTCCATGAGACACTCTATGCC | 943 | GGGTATCAGTTTATCAGTCAATCA | 944 | TRUE |
| chr21_chr18_14133786_3419 | chr21 | + | 15996899 | 15996966 | chr18 | - | 14133786 | 14133853 | GGGAGTTGAGCTGTGTGCTAT | 945 | TGGAGTGAGACCTAGGCAAC | 946 | TRUE |
| chr21_chr18_14132548_3852 | chr21 | + | 15998424 | 15998483 | chr18 | - | 14132549 | 14132548 | AGCATCTTCTGACACCGAAG | 947 | GCCTGATTGGATAGTAGTGC | 948 | TRUE |
| chr21_chr18_14132190_3523 | chr21 | + | 15998684 | 15400062 | chr18 | - | 14132190 | 14132283 | TGAAGCCGAGAAATGGTGAG | 949 | AGACTAGGTCCGTCACCG | 950 | TRUE |
| chr21_chr18_14131515_3740 | chr21 | + | 15400598 | 15400679 | chr18 | - | 14131515 | 14131595 | GCCTAGACCACTAGAGCC | 951 | GGCGAAAACCAGTGTCTT | 952 | TRUE |
| chr21_chr18_14131024_3775 | chr21 | + | 15401024 | 15401103 | chr18 | - | 14131024 | 14131100 | CCTTAGCACAAACGCCCTT | 953 | CCGAATGTGGCTAAGGAAAC | 954 | TRUE |
| chr21_chr18_14130506_3893 | chr21 | + | 15401813 | 15401893 | chr18 | - | 14130506 | 14130579 | AGTCCCAAGGGATAAAGTGG | 955 | GGGAGTGGCAGTGCTTTCT | 956 | FALSE |
| chr21_chr18_14130298_3946 | chr21 | + | 15401828 | 15401899 | chr18 | - | 14130298 | 14130369 | AAGTGAGGAGAGGGTGGGC | 957 | CTTCAAGGTTGAACGCTCTC | 958 | FALSE |
| chr21_chr18_14130071_3969 | chr21 | + | 15402032 | 15402131 | chr18 | - | 14130072 | 14130171 | CAGCCAATTGTATATGAGAAAA | 959 | TTGTGACTTCAATAATACTCTCTC | 960 | FALSE |
| chr21_chr18_14129745_4039 | chr21 | + | 15402377 | 15402458 | chr18 | - | 14129745 | 14129826 | GGAATGTGGAAGCCCTGG | 961 | ACTGCCGATGCCAGTCAC | 962 | TRUE |
| chr21_chr18_14129498_4117 | chr21 | + | 15402607 | 15402705 | chr18 | - | 14129498 | 14129598 | ATCACTGGGGCCTGGTCTA | 963 | TACAGGACAACGCACAGCGT | 964 | FALSE |
| chr21_chr18_14128471_4354 | chr21 | + | 15403659 | 15403746 | chr18 | - | 14128471 | 14128564 | CTCCACATTGCAACACTACC | 965 | TTGGGCTGTCATCTGGGTTT | 966 | TRUE |
| chr21_chr18_14127872_4463 | chr21 | + | 15404274 | 15404359 | chr18 | - | 14127872 | 14127957 | TCCACACTGTCCAGCATTACT | 967 | TGGAATCCTGAGAAAGAAAGTT | 968 | TRUE |
| chr21_chr18_14127582_4479 | chr21 | + | 15404586 | 15404659 | chr18 | - | 14127582 | 14127665 | CCCAATTAGGATCACACA | 969 | ATTTGGGCAAGGGCAGGT | 970 | FALSE |
| chr21_chr18_14126956_4609 | chr21 | + | 15405204 | 15405279 | chr18 | - | 14126956 | 14127035 | CAGCTAGATTCGGGATCTGT | 971 | GCTTTGTGGGGTCCAAAT | 972 | TRUE |
| chr21_chr18_14125200_4798 | chr21 | + | 15406951 | 15407030 | chr18 | - | 14125200 | 14125299 | TCAACTGCAGACTTATCAGACA | 973 | CACCAATCATCAGCCTGAAA | 974 | TRUE |
| chr21_chr15_46531267_4846 | chr21 | + | 15407409 | 15407496 | chr15 | + | 46531267 | 46531358 | CCCAAATACTGCACCAGA | 975 | CTAGCCCTCACAAAGACCA | 976 | FALSE |
| chr21_chr15_46531548_4869 | chr21 | + | 15407683 | 15407779 | chr15 | + | 46531548 | 46531642 | GCAATTTAATTCTTAGTGGCAT | 977 | GTTTTACCAAAATATTCAAGTGAG | 978 | TRUE |
| chr21_chr15_46532986_4879 | chr21 | + | 15408229 | 15408318 | chr15 | + | 46532986 | 46533065 | ACAGATTCCTCAGCCTTT | 979 | TTCTCCTGGAATAAGAGCCC | 980 | TRUE |

FIG. 8 continued

FIG. 8

| marker.bin | chr.tar | strand.tar | start.tar | end.tar | chr.ref | strand.ref | start.ref | end.ref | Primer | SEQ ID NO. | RPrimer | SEQ ID NO. | included.in.final |
|---|---|---|---|---|---|---|---|---|---|---|---|---|---|
| chr21_chr15_46593040_5095 | chr21 | + | 15408677 | 15408771 | chr15 | + | 46593048 | 46593142 | TTTACCAGGTTCTTGGCAT | 981 | TGTGACCACCTGCCAGTC | 982 | TRUE |
| chr21_chr15_46593883_5081 | chr21 | + | 15409646 | 15409709 | chr15 | + | 46593985 | 46594048 | TTATTGAATCTGCTTGGATT | 983 | GTCTGAAGTATTGCAAAGCA | 984 | FALSE |
| chr21_chr15_46595536_5170 | chr21 | + | 15411188 | 15411244 | chr15 | + | 46595588 | 46595611 | TGTAGGGCATCTCTACGC | 985 | ATTGGTGGAGGACCTTA | 986 | TRUE |
| chr21_chr15_46595902_5194 | chr21 | + | 15411537 | 15411621 | chr15 | + | 46595902 | 46595986 | CAGTATGCAATTATGAGACATAG | 987 | ACTTGTTAAAGAAGCACTGTCC | 988 | TRUE |
| chr21_chr15_46596040_5259 | chr21 | + | 15411677 | 15411756 | chr15 | + | 46596040 | 46596119 | TCCTGGAACTTAAGCTCATC | 989 | ACAGACCAGTCAAGCAATG | 990 | TRUE |
| chr21_chr15_46596348_5329 | chr21 | + | 15411986 | 15412075 | chr15 | + | 46596349 | 46596438 | CTCCTGCTGATGTGCCCC | 991 | AGGGCTGCCTGTTTGGGT | 992 | FALSE |
| chr21_chr15_46596765_5368 | chr21 | + | 15412403 | 15412472 | chr15 | + | 46596765 | 46596832 | TCAGATAGCATTTTATCTCCTAAA | 993 | TGGATCCTACTGCCCAGTT | 994 | TRUE |
| chr21_chr15_46596860_5432 | chr21 | + | 15412508 | 15412591 | chr15 | + | 46596862 | 46596948 | CCAGCAATGACATGATTACC | 995 | CACTCTAGAGGAGTCATAAGCC | 996 | TRUE |
| chr21_chr15_46597224_5521 | chr21 | + | 15412846 | 15412932 | chr15 | + | 46597224 | 46597310 | GAAGTCATTCTTGAAGTGAAAA | 997 | CATTAACATAAAGAGAGGCTG | 998 | FALSE |
| chr21_chr15_46598186_5525 | chr21 | + | 15413811 | 15413885 | chr15 | + | 46598186 | 46598260 | ATCATCCAGGTGGCTTAC | 999 | TGTCCATGAGGTCCTCTC | 1000 | FALSE |
| chr21_chr15_46598748_5686 | chr21 | + | 15414402 | 15414485 | chr15 | + | 46598745 | 46598828 | GGATTCCTAGGAGGCCAAA | 1001 | TTGATGGGACTCTCTCCA | 1002 | TRUE |
| chr21_chr15_46599300_5688 | chr21 | + | 15414951 | 15414733 | chr15 | + | 46599300 | 46599382 | CTCATAATTCTGGAGGCATTGAA | 1003 | TTAAGCTTTGTTTTGCTGTAAC | 1004 | TRUE |
| chr21_chr15_46599750_5792 | chr21 | + | 15415421 | 15415482 | chr15 | + | 46599750 | 46599821 | AAAGGCACAGTGGGTATAAA | 1005 | TGGTTTGCTTTGTTTCTGACAA | 1006 | TRUE |
| chr21_chr15_46599982_5858 | chr21 | + | 15415660 | 15415790 | chr15 | + | 46599982 | 46600072 | ATAGTAAACGAAGTTCTCCAGGC | 1007 | TTTAGCATTTCTAGTGCTGTT | 1008 | TRUE |
| chr21_chr15_46600944_5887 | chr21 | + | 15416630 | 15416698 | chr15 | + | 46600948 | 46601010 | AACCCAAGCAAAAGGTAAGG | 1009 | ACTGGTAACATGTATTGGGTCT | 1010 | TRUE |
| chr21_chr15_46602132_5981 | chr21 | + | 15418005 | 15418575 | chr15 | + | 46602132 | 46602202 | ATTCACAAATCCAATCCTG | 1011 | TTTCAGGGATAAAGCCCAT | 1012 | FALSE |
| chr21_chr15_46603378_6066 | chr21 | + | 15418753 | 15418823 | chr15 | + | 46603378 | 46603446 | ACCAGAAATCTGGAGGTGA | 1013 | CCAGTAGCTCTGTTCCAT | 1014 | TRUE |
| chr21_chr15_46603537_6141 | chr21 | + | 15419224 | 15419296 | chr15 | + | 46603547 | 46603621 | TTTTGTGGATTTTACTTGGA | 1015 | TTTCCTCCCTGCCAGAA | 1016 | TRUE |
| chr21_chr15_46604157_6230 | chr21 | + | 15419568 | 15419649 | chr15 | + | 46604157 | 46604238 | TTAGGGAAAACCAAAGACA | 1017 | CTATAGCTGCAGATGCCAGAGC | 1018 | TRUE |
| chr21_chr15_46604396_6251 | chr21 | + | 15419813 | 15419874 | chr15 | + | 46604396 | 46604458 | CAAATTAAGCATCACATCCA | 1019 | GAAATTCTGTCTGATAATTCCA | 1020 | TRUE |
| chr21_chr15_46605503_6285 | chr21 | + | 15420942 | 15421040 | chr15 | + | 46605503 | 46605601 | AGGTGATGTCACTGAGCAAC | 1021 | GACAAAAGCAAGCCTGTGGT | 1022 | TRUE |
| chr21_chr15_46606329_6379 | chr21 | + | 15421798 | 15421822 | chr15 | + | 46606329 | 46606395 | TGCTAGCACTAATCAGAAGAAGG | 1023 | TTCTGAAGTCTGCTTTGTCT | 1024 | TRUE |
| chr21_chr15_46606487_6414 | chr21 | + | 15421908 | 15422008 | chr15 | + | 46606487 | 46606586 | GTGCCAAATAACAATGAATC | 1025 | GGGTTGTAAAAGTCTGCAAGT | 1026 | FALSE |
| chr21_chr15_46606606_6499 | chr21 | + | 15422018 | 15422088 | chr15 | + | 46606606 | 46606673 | TCATGTCTAACTTTTACTTGAGG | 1027 | TCTTCTGCTTCTTTTAGGC | 1028 | TRUE |
| chr21_chr15_46607630_6634 | chr21 | + | 15422740 | 15422819 | chr15 | + | 46607630 | 46607708 | TTATTTACCAGGACCAAGTG | 1029 | GGGGTTGCCTTAATTGAT | 1030 | TRUE |
| chr21_chr15_46607890_6695 | chr21 | + | 15422987 | 15423080 | chr15 | + | 46607890 | 46607975 | GATCAATGGAATGTGATAGAGTG | 1031 | TGAACTGCCTTTGTTCCTT | 1032 | TRUE |
| chr21_chr15_46650743_6796 | chr21 | + | 15423692 | 15423771 | chr15 | + | 46650743 | 46650822 | TGGCCAAATGCTAGTGAT | 1033 | AAGCTTCTTAAGGAGATAAACA | 1034 | FALSE |
| chr21_chr12_38556372_6788 | chr21 | + | 15442128 | 15442233 | chr12 | - | 38556372 | 38556466 | CTCCACCCTACCCCAGCCT | 1035 | GACAGAGCTAGAGAGCCAGA | 1036 | FALSE |
| chr21_chr12_38555767_6859 | chr21 | + | 15442751 | 15442810 | chr12 | - | 38555767 | 38555826 | GAAGGTATCCCAGGACCGT | 1037 | TCAGGACAGTTTGTGCTCC | 1038 | TRUE |
| chr21_chr12_38546183_6950 | chr21 | + | 15446980 | 15447059 | chr12 | - | 38546183 | 38546236 | CCCTGGTTGTGCCTGAAG | 1039 | ACAAGCTTTTGGTCGTAAGG | 1040 | TRUE |
| chr21_chr12_38548721_7049 | chr21 | + | 15448409 | 15448488 | chr12 | - | 38548721 | 38548806 | CACAGGAGCACAGAGGGG | 1041 | GAGTCTGGCTCTGAGAAA | 1042 | TRUE |
| chr21_chr8_37948158_7299 | chr21 | + | 15488574 | 15488641 | chr8 | - | 37948156 | 37948223 | GGGAGGTGCAGATCTCTTAG | 1043 | ACCTAGGTCTGGCTCATC | 1044 | TRUE |
| chr21_chr8_37941044_7321 | chr21 | + | 15495391 | 15495458 | chr8 | - | 37941044 | 37941108 | AGGCTAGAAAACTAATGCCA | 1045 | TGAACCTTGTAACAAATTGCAGTA | 1046 | TRUE |
| chr21_chr14_42115929_7381 | chr21 | + | 15999981 | 16000006 | chr14 | - | 42115929 | 42116006 | AGGCCCATGGCCAATATC | 1047 | GCTGTGCATGACAATGCT | 1048 | FALSE |
| chr21_chr14_42115679_7519 | chr21 | + | 16000168 | 16000262 | chr14 | - | 42115679 | 42115760 | GGAAGCATTTTGGGAGTTA | 1049 | CCAGACAAGGGAGAAGTC | 1050 | TRUE |
| chr21_chr16_57500117_7575 | chr21 | + | 16130154 | 16130230 | chr16 | + | 57500117 | 57500196 | AGTTCACCCTGGATGTGC | 1051 | GGGGCTGTTGGAGATGAG | 1052 | TRUE |
| chr21_chr16_57503884_7593 | chr21 | + | 16130307 | 16130397 | chr16 | + | 57503884 | 57503984 | GAAAGGGCCAGGAGCTGA | 1053 | AAACCCACCCCTGCAGTA | 1054 | TRUE |
| chr21_chr5_157958301_7660 | chr21 | + | 16890579 | 16890680 | chr5 | - | 157958301 | 157958380 | GTGGCCTATCAGGTCTGTCT | 1055 | CCATGGTTTGGGTTTACA | 1056 | TRUE |
| chr21_chr5_157958127_7735 | chr21 | + | 16890758 | 16890838 | chr5 | - | 157958127 | 157958200 | CAGTTTGGTGCCTTAGATGTC | 1057 | CAGGATAGAGTCCTAGAAGTGG | 1058 | TRUE |
| chr21_chr3_50798031_7789 | chr21 | + | 16872548 | 16872632 | chr3 | - | 50798031 | 50798117 | GGACCTGGCCAGCACTTT | 1059 | TTAGGGCCCCAAGCTTAAA | 1060 | TRUE |
| chr21_chr2_225817208_7917 | chr21 | + | 17227960 | 17228091 | chr2 | - | 225817208 | 225817279 | CTGAGTTTTAAGTGCCAGA | 1061 | AAGTACAAGTCTGAGAGCCTAA | 1062 | TRUE |
| chr21_chr3_152904924_7948 | chr21 | + | 17716871 | 17716152 | chr3 | - | 152904924 | 152904985 | GTGTGTGACATTTTAGAGTTAGAT | 1063 | TCCTGTTGATTCCCACATC | 1064 | FALSE |
| chr21_chr13_94291887_7992 | chr21 | + | 18485450 | 18485530 | chr13 | - | 94291887 | 94291958 | GCAGAATTGATGCAACTACA | 1065 | CTGAAAGACTTCCATTTCTG | 1066 | TRUE |
| chr21_chr1_9187607_7994 | chr21 | + | 18679295 | 18679358 | chr1 | - | 9187607 | 9187670 | GAAACTAAGTGAGGTGCTTCT | 1067 | GGTTTGGATGATGTGTTGC | 1068 | TRUE |
| chr21_chr8_52632411_8090 | chr21 | + | 18861959 | 18862058 | chr8 | + | 52632411 | 52632495 | AAAGATCAGTAAGCCGTGC | 1069 | GTTAGGCAGGTGCTTTCTACA | 1070 | TRUE |
| chr21_chr1_92559561_8229 | chr21 | + | 18889623 | 18889724 | chr1 | - | 92559561 | 92559658 | GTGTTTTACCAGTGCTCCC | 1071 | GAACCTACCCTCTGGGTTGGA | 1072 | TRUE |
| chr21_chr14_103710952_8497 | chr21 | + | 19308438 | 19308528 | chr14 | + | 103710952 | 103711041 | AGGCACATGGGCTTCCT | 1073 | CCTAAGGCTCTGCCATTG | 1074 | FALSE |
| chr21_chr5_181086367_8470 | chr21 | + | 19504685 | 19504750 | chr5 | - | 181086367 | 181086432 | GACATCTAGATATGGGAAAACA | 1075 | TCCAAGGATTGGAGGACAC | 1076 | FALSE |
| chr21_chr2_96172158_8660 | chr21 | + | 19512183 | 19512272 | chr2 | - | 96172158 | 96172249 | AAGTGAGAATGGAGAATGG | 1077 | TTAGCACCATAAGCCAGAG | 1078 | TRUE |

FIG. 8 continued

FIG. 8

| markerNm | chr.tm | strand.tm | start.tm | end.tm | chr.ref | strand.ref | start.ref | end.ref | FPrimer | SEQ ID NO. | RPrimer | SEQ ID NO. | included.in.final |
|---|---|---|---|---|---|---|---|---|---|---|---|---|---|
| chr21_chr2_96172960_9851 | chr21 | + | 19512666 | 19512749 | chr2 | + | 96172960 | 96173057 | AGGACTGCTACCCTTTCTA | 1079 | CTGTAAACAGTGGTCCATTAG | 1080 | TRUE |
| chr21_chr3_147211895_9924 | chr21 | + | 19526960 | 19526945 | chr3 | + | 147211895 | 147211790 | TCAGACCACAAGATAGTGAAT | 1081 | CTCTCCAGGCTGGGCACATA | 1082 | FALSE |
| chr21_chr5_109594641_9983 | chr21 | + | 19853395 | 19859410 | chr5 | + | 109594641 | 109594787 | TTCAGTCACCTCAACTGAT | 1083 | TTTGAGTAAAGCATTGGG | 1084 | FALSE |
| chr21_chr3_108104893_9917 | chr21 | + | 19883728 | 19883815 | chr3 | + | 108104893 | 108104970 | GCTGTGCATTCACCATAT | 1085 | TTCTATGGGAAAATCAGGC | 1086 | FALSE |
| chr21_chr5_109626926_9941 | chr21 | + | 19894191 | 19894283 | chr5 | + | 109626926 | 109626978 | CCTTTATGGAGGAGGAGTT | 1087 | ATGCTGATCAAGGCAGTTTT | 1088 | FALSE |
| chr21_chr3_108106030_9121 | chr21 | + | 19934346 | 19934492 | chr3 | + | 108106030 | 108106127 | GAAGACCACAACAAGAT | 1089 | TGTGACTTGAGAGCTAAGA | 1090 | FALSE |
| chr21_chr13_50763660_9139 | chr21 | + | 20240630 | 20241039 | chr13 | - | 50763660 | 50763739 | CTTGTTGACAGCTTTCTGTC | 1091 | AGAAATCCACTGGAGCTG | 1092 | TRUE |
| chr21_chr3_14485182_9194 | chr21 | + | 20616899 | 20619980 | chr3 | + | 14485182 | 14485263 | CACAAGGACATGCTGAAGCC | 1093 | TCTGGGAGGTTTTCCCT | 1094 | TRUE |
| chr21_chr3_14488186_9233 | chr21 | + | 20617765 | 20617855 | chr3 | + | 14488186 | 14488228 | CTACCTGTTCCAGTCCTTCC | 1095 | GTGTTCCAGCTGTGGTTGC | 1096 | FALSE |
| chr21_chr3_14518744_9317 | chr21 | + | 20618099 | 20618171 | chr3 | + | 14518744 | 14518816 | TGGTTGATCTTTACCCATCC | 1097 | ATGCTACACACGAAGGCG | 1098 | TRUE |
| chr21_chr3_14525285_9356 | chr21 | + | 20618485 | 20618547 | chr3 | + | 14525285 | 14525324 | CTGGAGCCTGGCCCTTTC | 1099 | CAGAGGCGGATGACGATG | 1100 | FALSE |
| chr21_chr5_14975730_9376 | chr21 | + | 20853691 | 20859878 | chr5 | - | 14975730 | 14975802 | AGCAACAGTCACCCTCAG | 1101 | GCACAGAAGTCGTGGCCT | 1102 | TRUE |
| chr21_chr5_64026941_9460 | chr21 | + | 21012095 | 21012170 | chr5 | - | 64026941 | 64027016 | ACTTTTGAATGCCGCAATTT | 1103 | CACTACTTCTACTGCTGACATCCA | 1104 | TRUE |
| chr21_chr14_96288109_9518 | chr21 | + | 21352995 | 21359430 | chr14 | + | 96288109 | 96288198 | AGGGTTACATATTCCCCTGTTT | 1105 | AGAACTAATAATTGCTAACCCA | 1106 | TRUE |
| chr21_chr9_115981936_9551 | chr21 | + | 21546736 | 21548860 | chr9 | - | 115981936 | 115982012 | GCAAACATTATACACAAATGG | 1107 | TTCTCTGTGTACTTGTGCTCT | 1108 | TRUE |
| chr21_chr9_115991941_9553 | chr21 | + | 21547541 | 21547629 | chr9 | + | 115991941 | 115992029 | AAGGTCTTCCATCCTCTTA | 1109 | TTATTCCAATTAACTTCCA | 1110 | FALSE |
| chr21_chr6_88205706_9778 | chr21 | + | 23241919 | 23241979 | chr6 | - | 88205706 | 88205766 | ACACAAGGCCCAGCAATC | 1111 | GATAAGGTTGAGTGGTCAAAG | 1112 | TRUE |
| chr21_chr5_137948669_9852 | chr21 | + | 23249062 | 23249045 | chr5 | - | 137948669 | 137948733 | TACAATTTTCCACAGCAGC | 1113 | CAAAAGGTAAGAGTTGGCA | 1114 | TRUE |
| chr21_chr5_137948075_9888 | chr21 | + | 23249542 | 23249641 | chr5 | - | 137948075 | 137948174 | AAGTGCTGAGGGATGGGG | 1115 | TGCTGTGGTGAACAGAGG | 1116 | FALSE |
| chr21_chr15_60105600_9952 | chr21 | + | 23857867 | 23857758 | chr15 | - | 60105600 | 60105671 | TTTCTGACAGGTCGTAGGG | 1117 | ACATCATGATAGGATGAGCAAC | 1118 | TRUE |
| chr21_chr11_123644755_10062 | chr21 | + | 24475925 | 24474022 | chr11 | - | 123644755 | 123644850 | TCGGGTGAAAGAACATGG | 1119 | AGGGCTGAGCATCCCATC | 1120 | TRUE |
| chr21_chr11_124637378_10078 | chr21 | + | 24474990 | 24475088 | chr11 | - | 124637378 | 124637478 | TTGACTATGAAGAATGTATTGAG | 1121 | GAAAGGGTTTCCAAGTCAAC | 1122 | TRUE |
| chr21_chr15_99308175_10292 | chr21 | + | 24801986 | 24802052 | chr15 | - | 99308175 | 99308238 | GCAAGGCAAGAACAATATCCA | 1123 | TTTTCTTGATCTGGCATTT | 1124 | FALSE |
| chr21_chr4_82085432_10333 | chr21 | + | 24949967 | 24950030 | chr4 | + | 82085432 | 82085496 | AGGATGCATGTGGGGATG | 1125 | TATTTTCCTTGAATGCTACAC | 1126 | FALSE |
| chr21_chr12_75790575_10335 | chr21 | + | 25066313 | 25066404 | chr12 | + | 75790575 | 75790668 | CAATTTATACCACACTGCAA | 1127 | GAGACTTGTTTTATGGTTCAGC | 1128 | FALSE |
| chr21_chr5_22047785_10385 | chr21 | + | 25135472 | 25135534 | chr5 | + | 22047785 | 22047847 | GTCAATTGCAAATCGTTTT | 1129 | CATGTAGAAACACAGACCCA | 1130 | FALSE |
| chr21_chr17_47488385_10469 | chr21 | + | 25605323 | 25605383 | chr17 | - | 47488385 | 47488485 | CCAGAGATCAGGGAGTTGTAGG | 1131 | CTGAACTGGGCACCAAGAG | 1132 | FALSE |
| chr21_chr4_164100759_10512 | chr21 | + | 26266365 | 26266381 | chr4 | + | 164100759 | 164100845 | CCTCCTAATGGCAAGTCA | 1133 | TGCTGTACTTTACCAGAAGCAT | 1134 | TRUE |
| chr21_chr13_91711855_10520 | chr21 | + | 26460786 | 26460866 | chr13 | - | 91711855 | 91711914 | CTGCCCCACAGAGTTGCAGT | 1135 | TGCTCTAACTGCCTTACATATT | 1136 | TRUE |
| chr21_chr3_115127638_10591 | chr21 | + | 26713050 | 26713138 | chr3 | + | 115127638 | 115127711 | AGCCCATGAAGGCTTCCAA | 1137 | GTCCCTGACCTAGCTCCCTT | 1138 | TRUE |
| chr21_chr19_49995337_10681 | chr21 | + | 26725098 | 26725180 | chr19 | + | 49995337 | 49995397 | GGAGGGCCCTATCTTGTGA | 1139 | GCAGAGTATATGACCAGGTGGA | 1140 | TRUE |
| chr21_chr5_66985991_10714 | chr21 | + | 26985182 | 26985264 | chr5 | + | 66985991 | 66986079 | GATTGAAGATTGTGAACTGC | 1141 | ATTGATGTGAGTGACGGC | 1142 | TRUE |
| chr21_chr7_63354830_10729 | chr21 | + | 27130467 | 27130808 | chr7 | + | 63354830 | 63354894 | ACACTCAACATTTCGGGGA | 1143 | GAAGTTTCAGATGACTCCA | 1144 | TRUE |
| chr21_chr7_63354498_10804 | chr21 | + | 27136638 | 27136626 | chr7 | + | 63354498 | 63354598 | GCATCAGTGCAATCTGCTACA | 1145 | GAAATCCTCGGCGCTCTT | 1146 | FALSE |
| chr21_chr7_63354653_10944 | chr21 | + | 27136958 | 27137054 | chr7 | + | 63354653 | 63354748 | TAAGGACGCTCGAGACTG | 1147 | TTTACGCTGTCCCATTT | 1148 | TRUE |
| chr21_chr7_63354804_10950 | chr21 | + | 27141325 | 27141428 | chr7 | + | 63354804 | 63354896 | GATGGGGACATGATTTGTAAAG | 1149 | AACTCCGCTGCACTGTATCC | 1150 | FALSE |
| chr21_chr7_63355586_11248 | chr21 | + | 27142104 | 27142186 | chr7 | + | 63355586 | 63355648 | GCAAGCAAAGGACAGTAAGA | 1151 | TGGATCTCCTCCTTCAGCA | 1152 | TRUE |
| chr21_chr7_63356779_11584 | chr21 | + | 27143282 | 27143377 | chr7 | + | 63356779 | 63356874 | CAATTTGCCCACTGTGGT | 1153 | CTTGCTGTCTATTGTTACCTG | 1154 | TRUE |
| chr21_chr7_63356808_11527 | chr21 | + | 27143412 | 27143487 | chr7 | + | 63356808 | 63356884 | GTTGACAAGTTGCTTGTAGTT | 1155 | AGATTCTTAAGCTCTTGATGATA | 1156 | TRUE |
| chr21_chr7_63357600_11536 | chr21 | + | 27144061 | 27144177 | chr7 | + | 63357600 | 63357686 | TTTGTTTTCCATGTGAAGTT | 1157 | TTCTGATGCCAACACAAATA | 1158 | FALSE |
| chr21_chr7_63357895_11680 | chr21 | + | 27144381 | 27144478 | chr7 | + | 63357895 | 63357987 | TGCTACTGCCTTAAGAAAGA | 1159 | AAGTTAGTCACAGATTTGTTTTGC | 1160 | TRUE |
| chr21_chr6_114184946_11726 | chr21 | + | 27606111 | 27606182 | chr6 | - | 114184946 | 114184417 | TGATGTACAAAACTTTGGATGA | 1161 | GAGTTGGTGGATTTTCCT | 1162 | TRUE |
| chr21_chr6_114189462_11782 | chr21 | + | 27606578 | 27606676 | chr6 | - | 114189462 | 114189560 | GACATCATCCATTCAACAGC | 1163 | TGCTTAGTGCTTGGCAAT | 1164 | TRUE |
| chr21_chr6_114183355_11787 | chr21 | + | 27606686 | 27606763 | chr6 | - | 114183355 | 114183452 | GCAGGTAATACACCATCAAT | 1165 | CAAATTTTAAACTGAGTGAGAAGTC | 1166 | TRUE |
| chr21_chr6_114189017_11833 | chr21 | + | 27607084 | 27607121 | chr6 | - | 114189017 | 114189104 | AAAACTTCAGATTAAGAACCAC | 1167 | TGAGACTTCTAGGTCTTAGGTTA | 1168 | TRUE |
| chr21_chr6_114182801_11869 | chr21 | + | 27607256 | 27607334 | chr6 | - | 114182801 | 114182881 | TTCCATCTCTAAGTCAAATTGGT | 1169 | GCAATGATTTTGTGTAGCATC | 1170 | TRUE |
| chr21_chr6_114182387_12013 | chr21 | + | 27607696 | 27607780 | chr6 | - | 114182387 | 114182471 | GTTCTACTTAATCACTGACTGGT | 1171 | GATCATAGTCTTAGGAGTTCATTT | 1172 | TRUE |
| chr21_chr10_69677730_12020 | chr21 | + | 27716799 | 27716880 | chr10 | + | 69677730 | 69677817 | AAGGGTTTGAAATATAGCTGTTC | 1173 | ATGGCATTCAATAGGAT | 1174 | FALSE |
| chr21_chr1_95924878_12110 | chr21 | + | 28396218 | 28396297 | chr1 | + | 95924878 | 95924937 | ATCAAATTGGCTTACTGC | 1175 | AGGACTAAGGGGCAAC | 1176 | TRUE |

FIG. 8 continued

FIG. 8

| amplicon | chr.tar | strand.tar | start.tar | end.tar | chr.ref | strand.ref | start.ref | end.ref | FPrimer | SEQ ID NO. | RPrimer | SEQ ID NO. | included.in.final |
|---|---|---|---|---|---|---|---|---|---|---|---|---|---|
| chr21_chr2_132806088_12201 | chr21 | + | 29280813 | 29281015 | chr2 | - | 132806088 | 132806287 | TTCCACTAGCAAGAATGGTT | 1177 | ACTGTTGGACTGCAGCTAAG | 1178 | TRUE |
| chr21_chr2_132809702_12202 | chr21 | + | 29292176 | 29292279 | chr2 | - | 132809702 | 132809798 | AATCTTAGGGGAAAAGTGTTCA | 1179 | AAGGGGAATTTTCTCACCTCAA | 1180 | TRUE |
| chr21_chr2_132808548_12218 | chr21 | + | 29293672 | 29293751 | chr2 | - | 132808548 | 132808625 | AATTAGGGATCAGAATCTCAA | 1181 | CTGGAAATTAATACAAGGGG | 1182 | TRUE |
| chr21_chr2_132808204_12264 | chr21 | + | 29294006 | 29294095 | chr2 | - | 132808204 | 132808291 | CTCTATATGCATGTTGCCA | 1183 | GTCCTTAGGCACAAATGG | 1184 | TRUE |
| chr21_chr2_132859831_12270 | chr21 | + | 29832538 | 29832635 | chr2 | - | 132859831 | 132859928 | AGTGTTCATTCCATCTTGAG | 1185 | GCAACACTAAGGTAACTGGC | 1186 | TRUE |
| chr21_chr2_132860727_12629 | chr21 | + | 29341501 | 29341569 | chr2 | - | 132860727 | 132860795 | TGTCCATACTGGCCCTTT | 1187 | TTAACAGCAGCAAACAGATG | 1188 | TRUE |
| chr21_chr2_132649712_12884 | chr21 | + | 29942582 | [illegible] | chr2 | - | 132649712 | 132649796 | TAGAAGGCCCACACAATGCC | 1189 | AGTCCCAGATGAAGGGGTTT | 1190 | TRUE |
| chr21_chr18_15058040_12889 | chr21 | + | 29896037 | 29896108 | chr18 | - | 15058040 | 15058111 | CATTCCTGGCCTGAGAACA | 1191 | TTCACATTTACCAACTACTGAA | 1192 | TRUE |
| chr21_chr18_15059822_12935 | chr21 | + | 29896420 | 29896496 | chr18 | - | 15059822 | 15059878 | ACCTGACCCACTTTAAGTTAG | 1193 | TGTCAGGTTGTTGAGTGC | 1194 | TRUE |
| chr21_chr18_15055488_12959 | chr21 | + | 29896813 | 29896903 | chr18 | - | 15055488 | 15055594 | GGAACACAGCTGCCTTAT | 1195 | AGTAGAAGAGATTAGATTCCATGT | 1196 | TRUE |
| chr21_chr18_15054964_13081 | chr21 | + | 29897351 | 29897438 | chr18 | - | 15054964 | 15055044 | GTTATTGCCATGATTCCATGT | 1197 | GTGGGTGAACTGCTTGCC | 1198 | TRUE |
| chr21_chr18_15054781_13121 | chr21 | + | 29897546 | 29897629 | chr18 | - | 15054781 | 15054864 | GATGTGTCAAGGCATTGGAT | 1199 | TGTGACTTGGGCACCTAGAA | 1200 | TRUE |
| chr21_chr18_15054514_13208 | chr21 | + | 29897816 | 29897902 | chr18 | - | 15054514 | 15054590 | CAACTGTGCTTGCCGGAT | 1201 | TGATGACTAATAGACCACTTGA | 1202 | TRUE |
| chr21_chr18_15054197_13258 | chr21 | + | 29898101 | 29898200 | chr18 | - | 15054197 | 15054296 | TTGATCATCCTTCCTACCAC | 1203 | AGATGCCTCTCTTGGACA | 1204 | TRUE |
| chr21_chr18_15053941_13415 | chr21 | + | 29898457 | 29898555 | chr18 | - | 15053941 | 15054039 | TCTCAGGCCTATGACTTCAA | 1205 | TCAAGTAAGACAAGTACCAGGA | 1206 | TRUE |
| chr21_chr18_15053586_13502 | chr21 | + | 29898746 | 29898809 | chr18 | - | 15053586 | 15053649 | AATGCCCATTGCCCTACTG | 1207 | ATTGGGAAATCATCCATGTG | 1208 | TRUE |
| chr21_chr7_57157048_13660 | chr21 | + | 29752630 | 29752706 | chr7 | + | 57157048 | 57157124 | TTGGCCAGACCAGATGTAA | 1209 | CATCTAATGTCTGAATAGTGGG | 1210 | TRUE |
| chr21_chr14_52873294_13738 | chr21 | + | 29945214 | 29945294 | chr14 | - | 52873298 | 52873378 | GTGACTCATGGCCCAAGT | 1211 | GGTGCAACATAAAGTCAAAA | 1212 | FALSE |
| chr21_chr7_42313320_13759 | chr21 | + | 29978595 | 29978690 | chr7 | - | 42313320 | 42313417 | AAAGGTAGTTCTCTAAGTTACCAA | 1213 | AACATAATTTGGATGGGTCT | 1214 | TRUE |
| chr21_chr13_71488346_13813 | chr21 | + | 30002413 | 30002472 | chr13 | - | 71488346 | 71488405 | TGCCACAATGTTAATAAAAGG | 1215 | TTTACAGCAAATCGGCCTTA | 1216 | TRUE |
| chr21_chr11_98340970_13982 | chr21 | + | 30246364 | 30246462 | chr11 | + | 98340370 | 98340468 | TTCCACCCTATGTAAGAACT | 1217 | ACCATTGGCTATTAGCCC | 1218 | TRUE |
| chr21_chr11_98340748_14135 | chr21 | + | 30246588 | 30246603 | chr11 | + | 98340748 | 98340814 | CTTGTAATGCTGTGTGGAATAC | 1219 | AACTACCATCCGTGGAGTTACAG | 1220 | TRUE |
| chr21_chr11_98341788_14409 | chr21 | + | 30247555 | 30247650 | chr11 | + | 98341788 | 98341863 | TGCTGTGCATATCCAACT | 1221 | CTTAATGAGTCACCAAGTTACC | 1222 | TRUE |
| chr21_chr11_98341921_14424 | chr21 | + | 30247708 | 30247774 | chr11 | + | 98341921 | 98341987 | AGTTATTTGTTGGTAATGGCA | 1223 | AGGTCCCTATAGGTGAATCTTG | 1224 | TRUE |
| chr21_chr11_98342192_14581 | chr21 | + | 30247980 | 30248054 | chr11 | + | 98342192 | 98342266 | AATGTTGCCTCCAAAACCC | 1225 | TGCATCTTGCTGCTCTTA | 1226 | TRUE |
| chr21_chr11_98342301_14494 | chr21 | + | 30248089 | 30248184 | chr11 | + | 98342301 | 98342396 | ACATGCCCCAGGTCACTG | 1227 | GACATTTGCATCAGAGGG | 1228 | FALSE |
| chr21_chr11_98342878_14530 | chr21 | + | 30248665 | 30248733 | chr11 | + | 98342878 | 98342946 | AAATGCATTCAGTTTCCA | 1229 | TCAGTCCTCAAGTTCACCA | 1230 | TRUE |
| chr21_chr11_98349069_14598 | chr21 | + | 30254858 | 30254948 | chr11 | + | 98349069 | 98349151 | GCTGAAAGCGAGATCTGGAAC | 1231 | ATAGAGGCAGCTTGGGCT | 1232 | TRUE |
| chr21_chr16_20746902_14602 | chr21 | + | 30272151 | 30272245 | chr16 | + | 20746902 | 20746996 | GAGGCACCAGGAGTGTAGAT | 1233 | TATTGCTAGGAGGCTGCC | 1234 | FALSE |
| chr21_chr16_20747058_14712 | chr21 | + | 30272287 | 30272352 | chr16 | + | 20747058 | 20747123 | AATGCTACAGCATGACAAA | 1235 | GCAGCTTCTCTATCCAGG | 1236 | TRUE |
| chr21_chr16_20747524_14725 | chr21 | + | 30273076 | 30273148 | chr16 | + | 20747524 | 20747596 | TCTTTGTGTCCCTTGTTG | 1237 | AAAAGGTTGGTGATGAAGA | 1238 | FALSE |
| chr21_chr16_20747772_14741 | chr21 | + | 30273668 | 30273742 | chr16 | + | 20747772 | 20747847 | TTCTAACCTACATGATCCACA | 1239 | TTACATGCCACAGCTCAG | 1240 | TRUE |
| chr21_chr16_20749116_14789 | chr21 | + | 30275457 | 30275534 | chr16 | + | 20749116 | 20749198 | GCTTTAAACCAGGGTTCC | 1241 | GATCTCAGGCACATGCAA | 1242 | TRUE |
| chr21_chr16_20750311_14844 | chr21 | + | 30276706 | 30276782 | chr16 | + | 20750311 | 20750385 | TCACCAACTTCTTTCTTCAA | 1243 | CAAGGATCAGCAGCCCTC | 1244 | FALSE |
| chr21_chr1_146875599_14929 | chr21 | + | 30435698 | 30435766 | chr1 | + | 146875599 | 146875667 | TTGCATAATGAAGAGCCATGT | 1245 | TTGCTGATACTGGTGCAAA | 1246 | FALSE |
| chr21_chr1_146879964_15068 | chr21 | + | 30439124 | 30439200 | chr1 | + | 146879964 | 146879040 | CAATAAGCTTGGCCAGAAAT | 1247 | TGAAGTCTCATCTCTACTTGGT | 1248 | TRUE |
| chr21_chr6_102372515_15207 | chr21 | + | 30983757 | 30983834 | chr6 | - | 102372515 | 102372592 | GAGCTCCAACTCCAAACCA | 1249 | CCCTGACTCAGACGTGGTG | 1250 | TRUE |
| chr21_chr6_6468532_15261 | chr21 | + | 31128746 | 31128832 | chr6 | + | 6468532 | 6468625 | CAGCTAAGGCACATGGTAA | 1251 | GTAAGGTGGCATTGTCCC | 1252 | TRUE |
| chr21_chr6_131739806_15398 | chr21 | + | 31636146 | 31636210 | chr6 | - | 131739806 | 131739870 | AACCCTTATCTAGGTGCCA | 1253 | ATGCTGCCTGGAGGGCTT | 1254 | TRUE |
| chr21_chr12_108787481_15471 | chr21 | + | 32194231 | 32194321 | chr12 | - | 108787481 | 108787581 | CTTAAACCCCTTTACCCCAA | 1255 | TGGCCTCAAGCTCCTCATC | 1256 | TRUE |
| chr21_chr15_25610821_15610 | chr21 | + | 32459840 | 32459908 | chr15 | + | 25610821 | 25610889 | GAATCTGGTCTGCATTGTATT | 1257 | GCAAGCTACCCCTTGCAG | 1258 | FALSE |
| chr21_chr15_25616524_15746 | chr21 | + | 32465049 | 32465127 | chr15 | + | 25616524 | 25616608 | CATTCCACGTTAGGTGACAA | 1259 | GGGTCTACACCAGATTGCTCT | 1260 | TRUE |
| chr21_chr2_231685109_15843 | chr21 | + | 32675085 | 32675155 | chr2 | - | 231685109 | 231685180 | CACACAGATTCTGGTAAAGAC | 1261 | AAAATGGCAGTCTACATGAT | 1262 | TRUE |
| chr21_chr2_231684508_15857 | chr21 | + | 32675647 | 32675713 | chr2 | - | 231684508 | 231684576 | CACACAAAGAAACAGCATTA | 1263 | TGAGTTAAAGAATCTGCCA | 1264 | FALSE |
| chr21_chr2_25648022_15897 | chr21 | + | 32783481 | 32783529 | chr2 | - | 25648022 | 25648090 | AATGGATTATGAAGTTATAGCC | 1265 | TTGGGTGTAGCTCTAGTTTG | 1266 | FALSE |
| chr21_chr1_9428428_15963 | chr21 | + | 32798884 | 32798958 | chr1 | - | 9428428 | 9428500 | GTGAGGCTATTTCTCCCTG | 1267 | GTCCCTTGCCCTGCAGTT | 1268 | TRUE |
| chr21_chr2_49187547_16013 | chr21 | + | 32822031 | 32822083 | chr2 | - | 49187547 | 49187603 | GTTCCTCTCACAAAGTTC | 1269 | GGAGTTGTGACAGTTGCC | 1270 | TRUE |
| chr21_chr5_171289209_16158 | chr21 | + | 32986771 | 32986830 | chr5 | + | 171289209 | 171289261 | CTAAATCACTTTCACAACCAC | 1271 | AATAAACCTCCATTCATAAGG | 1272 | TRUE |
| chr21_chr5_171289522_16172 | chr21 | + | 32987594 | 32987667 | chr5 | + | 171289522 | 171289595 | TTGACATTCCTGATCTTTG | 1273 | CGCTTATTCAATATTAGGTTTG | 1274 | FALSE |

FIG. 8 continued

FIG. 8

| marker.tar | chr.tar | strand.tar | start.tar | end.tar | chr.ref | strand.ref | start.ref | end.ref | FPrimer | SEQ ID NO | RPrimer | SEQ ID NO | included.in.final |
|---|---|---|---|---|---|---|---|---|---|---|---|---|---|
| [illegible] | chr21 | + | [illegible] | [illegible] | chr5 | + | [illegible] | [illegible] | [illegible] | 1275 | [illegible] | 1276 | TRUE |
| [illegible] | chr21 | + | [illegible] | [illegible] | chr5 | + | [illegible] | [illegible] | [illegible] | 1277 | [illegible] | 1278 | TRUE |
| [illegible] | chr21 | + | [illegible] | [illegible] | chr5 | + | [illegible] | [illegible] | [illegible] | 1279 | [illegible] | 1280 | TRUE |
| [illegible] | chr21 | + | [illegible] | [illegible] | chr5 | + | [illegible] | [illegible] | [illegible] | 1281 | [illegible] | 1282 | FALSE |
| [illegible] | chr21 | + | [illegible] | [illegible] | chr5 | + | [illegible] | [illegible] | [illegible] | 1283 | [illegible] | 1284 | TRUE |
| [illegible] | chr21 | + | [illegible] | [illegible] | chr4 | + | [illegible] | [illegible] | [illegible] | 1285 | [illegible] | 1286 | TRUE |
| [illegible] | chr21 | + | [illegible] | [illegible] | chr6 | + | [illegible] | [illegible] | [illegible] | 1287 | [illegible] | 1288 | TRUE |
| [illegible] | chr21 | + | [illegible] | [illegible] | chr6 | + | [illegible] | [illegible] | [illegible] | 1289 | [illegible] | 1290 | TRUE |
| [illegible] | chr21 | + | [illegible] | [illegible] | chr6 | + | [illegible] | [illegible] | [illegible] | 1291 | [illegible] | 1292 | TRUE |
| [illegible] | chr21 | + | [illegible] | [illegible] | chr6 | + | [illegible] | [illegible] | [illegible] | 1293 | [illegible] | 1294 | FALSE |
| [illegible] | chr21 | + | [illegible] | [illegible] | chr6 | + | [illegible] | [illegible] | [illegible] | 1295 | [illegible] | 1296 | TRUE |
| [illegible] | chr21 | + | [illegible] | [illegible] | chr6 | + | [illegible] | [illegible] | [illegible] | 1297 | [illegible] | 1298 | TRUE |
| [illegible] | chr21 | + | [illegible] | [illegible] | chr6 | + | [illegible] | [illegible] | [illegible] | 1299 | [illegible] | 1300 | FALSE |
| [illegible] | chr21 | + | [illegible] | [illegible] | chr6 | + | [illegible] | [illegible] | [illegible] | 1301 | [illegible] | 1302 | FALSE |
| [illegible] | chr21 | + | [illegible] | [illegible] | chr6 | + | [illegible] | [illegible] | [illegible] | 1303 | [illegible] | 1304 | TRUE |
| [illegible] | chr21 | + | [illegible] | [illegible] | chr6 | + | [illegible] | [illegible] | [illegible] | 1305 | [illegible] | 1306 | TRUE |
| [illegible] | chr21 | + | [illegible] | [illegible] | chr6 | + | [illegible] | [illegible] | [illegible] | 1307 | [illegible] | 1308 | FALSE |
| [illegible] | chr21 | + | [illegible] | [illegible] | chr6 | + | [illegible] | [illegible] | [illegible] | 1309 | [illegible] | 1310 | TRUE |
| [illegible] | chr21 | + | [illegible] | [illegible] | chr6 | + | [illegible] | [illegible] | [illegible] | 1311 | [illegible] | 1312 | TRUE |
| [illegible] | chr21 | + | [illegible] | [illegible] | chr6 | + | [illegible] | [illegible] | [illegible] | 1313 | [illegible] | 1314 | FALSE |
| [illegible] | chr21 | + | [illegible] | [illegible] | chr6 | + | [illegible] | [illegible] | [illegible] | 1315 | [illegible] | 1316 | FALSE |
| [illegible] | chr21 | + | [illegible] | [illegible] | chr6 | + | [illegible] | [illegible] | [illegible] | 1317 | [illegible] | 1318 | TRUE |
| [illegible] | chr21 | + | [illegible] | [illegible] | chr6 | + | [illegible] | [illegible] | [illegible] | 1319 | [illegible] | 1320 | TRUE |
| [illegible] | chr21 | + | [illegible] | [illegible] | chr6 | + | [illegible] | [illegible] | [illegible] | 1321 | [illegible] | 1322 | FALSE |
| [illegible] | chr21 | + | [illegible] | [illegible] | chr6 | + | [illegible] | [illegible] | [illegible] | 1323 | [illegible] | 1324 | TRUE |
| [illegible] | chr21 | + | [illegible] | [illegible] | chr6 | + | [illegible] | [illegible] | [illegible] | 1325 | [illegible] | 1326 | TRUE |
| [illegible] | chr21 | + | [illegible] | [illegible] | chr6 | - | [illegible] | [illegible] | [illegible] | 1327 | [illegible] | 1328 | TRUE |
| [illegible] | chr21 | + | [illegible] | [illegible] | chr6 | + | [illegible] | [illegible] | [illegible] | 1329 | [illegible] | 1330 | FALSE |
| [illegible] | chr21 | + | [illegible] | [illegible] | chr6 | + | [illegible] | [illegible] | [illegible] | 1331 | [illegible] | 1332 | TRUE |
| [illegible] | chr21 | + | [illegible] | [illegible] | chr6 | + | [illegible] | [illegible] | [illegible] | 1333 | [illegible] | 1334 | TRUE |
| [illegible] | chr21 | + | [illegible] | [illegible] | chr6 | + | [illegible] | [illegible] | [illegible] | 1335 | [illegible] | 1336 | TRUE |
| [illegible] | chr21 | + | [illegible] | [illegible] | chr6 | + | [illegible] | [illegible] | [illegible] | 1337 | [illegible] | 1338 | FALSE |
| [illegible] | chr21 | + | [illegible] | [illegible] | chr6 | - | [illegible] | [illegible] | [illegible] | 1339 | [illegible] | 1340 | FALSE |
| [illegible] | chr21 | + | [illegible] | [illegible] | chr6 | + | [illegible] | [illegible] | [illegible] | 1341 | [illegible] | 1342 | TRUE |
| [illegible] | chr21 | + | [illegible] | [illegible] | chr6 | + | [illegible] | [illegible] | [illegible] | 1343 | [illegible] | 1344 | TRUE |
| [illegible] | chr21 | + | [illegible] | [illegible] | chr6 | + | [illegible] | [illegible] | [illegible] | 1345 | [illegible] | 1346 | TRUE |
| [illegible] | chr21 | + | [illegible] | [illegible] | chr6 | + | [illegible] | [illegible] | [illegible] | 1347 | [illegible] | 1348 | FALSE |
| [illegible] | chr21 | + | [illegible] | [illegible] | chr6 | + | [illegible] | [illegible] | [illegible] | 1349 | [illegible] | 1350 | FALSE |
| [illegible] | chr21 | + | [illegible] | [illegible] | chr6 | + | [illegible] | [illegible] | [illegible] | 1351 | [illegible] | 1352 | TRUE |
| [illegible] | chr21 | + | [illegible] | [illegible] | chr6 | + | [illegible] | [illegible] | [illegible] | 1353 | [illegible] | 1354 | FALSE |
| [illegible] | chr21 | + | [illegible] | [illegible] | chr6 | + | [illegible] | [illegible] | [illegible] | 1355 | [illegible] | 1356 | FALSE |
| [illegible] | chr21 | + | [illegible] | [illegible] | chr6 | + | [illegible] | [illegible] | [illegible] | 1357 | [illegible] | 1358 | FALSE |
| [illegible] | chr21 | + | [illegible] | [illegible] | chr4 | - | [illegible] | [illegible] | [illegible] | 1359 | [illegible] | 1360 | TRUE |
| [illegible] | chr21 | + | [illegible] | [illegible] | chr1 | - | [illegible] | [illegible] | [illegible] | 1361 | [illegible] | 1362 | FALSE |
| [illegible] | chr21 | + | [illegible] | [illegible] | chr19 | + | [illegible] | [illegible] | [illegible] | 1363 | [illegible] | 1364 | TRUE |
| [illegible] | chr21 | + | [illegible] | [illegible] | chr19 | + | [illegible] | [illegible] | [illegible] | 1365 | [illegible] | 1366 | FALSE |
| [illegible] | chr21 | + | [illegible] | [illegible] | chr1 | + | [illegible] | [illegible] | [illegible] | 1367 | [illegible] | 1368 | TRUE |
| [illegible] | chr21 | + | [illegible] | [illegible] | chr1 | + | [illegible] | [illegible] | [illegible] | 1369 | [illegible] | 1370 | FALSE |
| [illegible] | chr21 | + | [illegible] | [illegible] | chr1 | + | [illegible] | [illegible] | [illegible] | 1371 | [illegible] | 1372 | TRUE |

FIG. 8

FIG. 8 continued

| connection | chr.tar | strand.tar | start.tar | end.tar | chr.ref | strand.ref | start.ref | end.ref | Primer | SEQ ID NO. | Primer | SEQ ID NO. | included.in.final |
|---|---|---|---|---|---|---|---|---|---|---|---|---|---|
| [illegible] | chr21 | + | [illegible] | [illegible] | [illegible] | + | [illegible] | [illegible] | [illegible] | 1373 | [illegible] | 1374 | TRUE |
| [illegible] | chr21 | + | [illegible] | [illegible] | [illegible] | - | [illegible] | [illegible] | [illegible] | 1375 | [illegible] | 1376 | TRUE |
| [illegible] | chr21 | + | [illegible] | [illegible] | [illegible] | - | [illegible] | [illegible] | [illegible] | 1377 | [illegible] | 1378 | TRUE |
| [illegible] | chr21 | + | [illegible] | [illegible] | [illegible] | - | [illegible] | [illegible] | [illegible] | 1379 | [illegible] | 1380 | TRUE |
| [illegible] | chr21 | + | [illegible] | [illegible] | [illegible] | - | [illegible] | [illegible] | [illegible] | 1381 | [illegible] | 1382 | TRUE |
| [illegible] | chr21 | + | [illegible] | [illegible] | [illegible] | - | [illegible] | [illegible] | [illegible] | 1383 | [illegible] | 1384 | FALSE |
| [illegible] | chr21 | + | [illegible] | [illegible] | [illegible] | + | [illegible] | [illegible] | [illegible] | 1385 | [illegible] | 1386 | TRUE |
| [illegible] | chr21 | + | [illegible] | [illegible] | [illegible] | + | [illegible] | [illegible] | [illegible] | 1387 | [illegible] | 1388 | FALSE |
| [illegible] | chr21 | + | [illegible] | [illegible] | [illegible] | + | [illegible] | [illegible] | [illegible] | 1389 | [illegible] | 1390 | FALSE |
| [illegible] | chr21 | + | [illegible] | [illegible] | [illegible] | - | [illegible] | [illegible] | [illegible] | 1391 | [illegible] | 1392 | TRUE |
| [illegible] | chr21 | + | [illegible] | [illegible] | [illegible] | + | [illegible] | [illegible] | [illegible] | 1393 | [illegible] | 1394 | TRUE |
| [illegible] | chr21 | + | [illegible] | [illegible] | [illegible] | - | [illegible] | [illegible] | [illegible] | 1395 | [illegible] | 1396 | FALSE |
| [illegible] | chr21 | + | [illegible] | [illegible] | [illegible] | - | [illegible] | [illegible] | [illegible] | 1397 | [illegible] | 1398 | TRUE |
| [illegible] | chr21 | + | [illegible] | [illegible] | [illegible] | - | [illegible] | [illegible] | [illegible] | 1399 | [illegible] | 1400 | FALSE |
| [illegible] | chr21 | + | [illegible] | [illegible] | [illegible] | - | [illegible] | [illegible] | [illegible] | 1401 | [illegible] | 1402 | TRUE |
| [illegible] | chr21 | + | [illegible] | [illegible] | [illegible] | - | [illegible] | [illegible] | [illegible] | 1403 | [illegible] | 1404 | TRUE |
| [illegible] | chr21 | + | [illegible] | [illegible] | [illegible] | - | [illegible] | [illegible] | [illegible] | 1405 | [illegible] | 1406 | FALSE |
| [illegible] | chr21 | + | [illegible] | [illegible] | [illegible] | - | [illegible] | [illegible] | [illegible] | 1407 | [illegible] | 1408 | TRUE |
| [illegible] | chr21 | + | [illegible] | [illegible] | [illegible] | - | [illegible] | [illegible] | [illegible] | 1409 | [illegible] | 1410 | TRUE |
| [illegible] | chr21 | + | [illegible] | [illegible] | [illegible] | - | [illegible] | [illegible] | [illegible] | 1411 | [illegible] | 1412 | TRUE |
| [illegible] | chr21 | + | [illegible] | [illegible] | [illegible] | + | [illegible] | [illegible] | [illegible] | 1413 | [illegible] | 1414 | TRUE |
| [illegible] | chr21 | + | [illegible] | [illegible] | [illegible] | + | [illegible] | [illegible] | [illegible] | 1415 | [illegible] | 1416 | TRUE |
| [illegible] | chr21 | + | [illegible] | [illegible] | [illegible] | + | [illegible] | [illegible] | [illegible] | 1417 | [illegible] | 1418 | TRUE |
| [illegible] | chr21 | + | [illegible] | [illegible] | [illegible] | + | [illegible] | [illegible] | [illegible] | 1419 | [illegible] | 1420 | TRUE |
| [illegible] | chr21 | + | [illegible] | [illegible] | [illegible] | - | [illegible] | [illegible] | [illegible] | 1421 | [illegible] | 1422 | FALSE |
| [illegible] | chr21 | + | [illegible] | [illegible] | [illegible] | + | [illegible] | [illegible] | [illegible] | 1423 | [illegible] | 1424 | FALSE |
| [illegible] | chr21 | + | [illegible] | [illegible] | [illegible] | - | [illegible] | [illegible] | [illegible] | 1425 | [illegible] | 1426 | TRUE |
| [illegible] | chr21 | + | [illegible] | [illegible] | [illegible] | + | [illegible] | [illegible] | [illegible] | 1427 | [illegible] | 1428 | FALSE |
| [illegible] | chr21 | + | [illegible] | [illegible] | [illegible] | + | [illegible] | [illegible] | [illegible] | 1429 | [illegible] | 1430 | FALSE |
| [illegible] | chr21 | + | [illegible] | [illegible] | [illegible] | + | [illegible] | [illegible] | [illegible] | 1431 | [illegible] | 1432 | TRUE |
| [illegible] | chr21 | + | [illegible] | [illegible] | [illegible] | + | [illegible] | [illegible] | [illegible] | 1433 | [illegible] | 1434 | FALSE |
| [illegible] | chr21 | + | [illegible] | [illegible] | [illegible] | + | [illegible] | [illegible] | [illegible] | 1435 | [illegible] | 1436 | TRUE |
| [illegible] | chr21 | + | [illegible] | [illegible] | [illegible] | - | [illegible] | [illegible] | [illegible] | 1437 | [illegible] | 1438 | TRUE |
| [illegible] | chr21 | + | [illegible] | [illegible] | [illegible] | - | [illegible] | [illegible] | [illegible] | 1439 | [illegible] | 1440 | FALSE |
| [illegible] | chr21 | + | [illegible] | [illegible] | [illegible] | - | [illegible] | [illegible] | [illegible] | 1441 | [illegible] | 1442 | TRUE |
| [illegible] | chr21 | + | [illegible] | [illegible] | [illegible] | - | [illegible] | [illegible] | [illegible] | 1443 | [illegible] | 1444 | FALSE |
| [illegible] | chr21 | + | [illegible] | [illegible] | [illegible] | - | [illegible] | [illegible] | [illegible] | 1445 | [illegible] | 1446 | TRUE |
| [illegible] | chr21 | + | [illegible] | [illegible] | [illegible] | + | [illegible] | [illegible] | [illegible] | 1447 | [illegible] | 1448 | TRUE |
| [illegible] | chr21 | + | [illegible] | [illegible] | [illegible] | + | [illegible] | [illegible] | [illegible] | 1449 | [illegible] | 1450 | TRUE |
| [illegible] | chr21 | + | [illegible] | [illegible] | [illegible] | + | [illegible] | [illegible] | [illegible] | 1451 | [illegible] | 1452 | TRUE |
| [illegible] | chr21 | + | [illegible] | [illegible] | [illegible] | + | [illegible] | [illegible] | [illegible] | 1453 | [illegible] | 1454 | FALSE |
| [illegible] | chr21 | + | [illegible] | [illegible] | [illegible] | - | [illegible] | [illegible] | [illegible] | 1455 | [illegible] | 1456 | FALSE |
| [illegible] | chr21 | + | [illegible] | [illegible] | [illegible] | + | [illegible] | [illegible] | [illegible] | 1457 | [illegible] | 1458 | TRUE |
| [illegible] | chr21 | + | [illegible] | [illegible] | [illegible] | - | [illegible] | [illegible] | [illegible] | 1459 | [illegible] | 1460 | TRUE |
| [illegible] | chr21 | + | [illegible] | [illegible] | [illegible] | - | [illegible] | [illegible] | [illegible] | 1461 | [illegible] | 1462 | TRUE |
| [illegible] | chr21 | + | [illegible] | [illegible] | [illegible] | + | [illegible] | [illegible] | [illegible] | 1463 | [illegible] | 1464 | FALSE |
| [illegible] | chr21 | + | [illegible] | [illegible] | [illegible] | - | [illegible] | [illegible] | [illegible] | 1465 | [illegible] | 1466 | TRUE |
| [illegible] | chr21 | + | [illegible] | [illegible] | [illegible] | + | [illegible] | [illegible] | [illegible] | 1467 | [illegible] | 1468 | FALSE |
| [illegible] | chr21 | + | [illegible] | [illegible] | [illegible] | + | [illegible] | [illegible] | [illegible] | 1469 | [illegible] | 1470 | FALSE |

FIG. 8

FIG. 8 continued

| markerName | chr.tar | strand.tar | start.tar | end.tar | chr.ref | strand.ref | start.ref | end.ref | 5Primer | SEQ ID NO. | 3Primer | SEQ ID NO. | included.in.final |
|---|---|---|---|---|---|---|---|---|---|---|---|---|---|
| chr21_chr2_113191941_25238 | chr21 | + | 44877282 | 44877365 | chr2 | + | 113191941 | 113192024 | CCCACGCATGGCTAGGAT | 1471 | GTCTGAACCCTTAGTTAGGAC | 1472 | TRUE |
| chr21_chr2_113192443_25352 | chr21 | + | 44877780 | 44877858 | chr2 | + | 113192443 | 113192521 | CCTCCATGAGCCTCCAAGC | 1473 | CCAGATGCTGCCCACCAC | 1474 | FALSE |
| chr21_chr2_113192630_25388 | chr21 | + | 44877967 | 44878056 | chr2 | + | 113192630 | 113192719 | TGCCAAGTGTGGTCCCTG | 1475 | CTCCTGAAGCGTGGATGG | 1476 | FALSE |
| chr21_chr2_113193488_25480 | chr21 | + | 44878824 | 44878920 | chr2 | + | 113193488 | 113193584 | GCTGCAGGATAGGAGCTAC | 1477 | ATCAAGGGCAGGTGGCTAA | 1478 | FALSE |
| chr21_chr2_113193781_25575 | chr21 | + | 44879067 | 44879149 | chr2 | + | 113193731 | 113193813 | CTGATCATCCTCGTCAGG | 1479 | GGTTCTGGCTGAAGGGAG | 1480 | TRUE |
| chr21_chr2_113193967_25621 | chr21 | + | 44879304 | 44879386 | chr2 | + | 113193967 | 113194048 | ACCGCACTGGTCCTGAGT | 1481 | TAAGGGTGTGCCTGACATGA | 1482 | TRUE |
| chr21_chr2_113194130_25680 | chr21 | + | 44879445 | 44879523 | chr2 | + | 113194110 | 113194188 | GTGGAAGGTGAGATTGGGAA | 1483 | TTCGAACATGACCTGAAAAGC | 1484 | TRUE |
| chr21_chr2_113194393_25703 | chr21 | + | 44879728 | 44879827 | chr2 | + | 113194393 | 113194492 | TCAGGGTCTTGGCAGGAA | 1485 | TGGAGGTGCTCCAGGACT | 1486 | TRUE |
| chr21_chr2_113194775_25796 | chr21 | + | 44880130 | 44880208 | chr2 | + | 113194775 | 113194873 | ACCCCACGCCTACACCAG | 1487 | AAAGGTCCTGCACACCCG | 1488 | TRUE |
| chr21_chr2_113195188_25959 | chr21 | + | 44880523 | 44880630 | chr2 | + | 113195198 | 113195286 | CCTAGAGCGGTGATCCCA | 1489 | GAGGTCATGGAATGTGGGC | 1490 | TRUE |
| chr21_chr2_113195362_26027 | chr21 | + | 44881037 | 44881126 | chr2 | + | 113195362 | 113195451 | GGTTCTATGCAGGAGCCGAG | 1491 | TAAGAGGGACCATCGGCA | 1492 | FALSE |
| chr21_chr6_27114510_26077 | chr21 | + | 44885180 | 44885286 | chr6 | - | 27114510 | 27114597 | GCTCGTAGTTCGCCTTCAAC | 1493 | CGCCTTAGTCACAGCTTTC | 1494 | TRUE |
| chr21_chr6_27108488_26119 | chr21 | + | 44885792 | 44885848 | chr6 | - | 27108488 | 27108582 | TGTAGAGAATCTGAATAGACCAT | 1495 | TCCTAATGTGAAATCCCAG | 1496 | FALSE |
| chr21_chr1_238489022_26127 | chr21 | + | 45416208 | 45416297 | chr1 | + | 238489022 | 238489081 | CTTGCAGGGGTCATGCTAA | 1497 | GAAACCGTGGCAATATCCTA | 1498 | TRUE |
| chr21_chr1_238489418_26207 | chr21 | + | 45416694 | 45416733 | chr1 | + | 238489418 | 238489513 | TTGCCAACGTTCTGCTTT | 1499 | TGCTGCAGAAACTGAGGGT | 1500 | TRUE |
| chr21_chr1_179640336_26246 | chr21 | + | 46014019 | 46014099 | chr1 | - | 179640336 | 179640416 | AGGCAGCTGGCTTGCAGAT | 1501 | CGCCTCTGACTTCTGTGAGTC | 1502 | TRUE |
| chr21_chr2_86871198_26301 | chr21 | + | 46095598 | 46095659 | chr2 | + | 86871198 | 86871254 | AGTTGCAAAGAAAGCACTCC | 1503 | TGTTAACACCTGTTGCATTT | 1504 | FALSE |
| chr21_chr2_86871438_26343 | chr21 | + | 46095833 | 46095890 | chr2 | + | 86871438 | 86871496 | CGCTGGCATATGCTGTCA | 1505 | GCTGAAGGAAGCCCGAAT | 1506 | TRUE |
| chr21_chr2_86885740_26607 | chr21 | + | 46096833 | 46096898 | chr2 | + | 86885740 | 86885808 | CTGGAGTAATACTGTCCAGC | 1507 | CAGTATCATGAGCTGGTGG | 1508 | FALSE |
| chr21_chr2_86898864_26632 | chr21 | + | 46097085 | 46097160 | chr2 | + | 86898664 | 86898750 | GGAGTGTGCATTGACAGCC | 1509 | GAAGATGCTCTGAAGCAAAC | 1510 | TRUE |
| chr21_chr2_86408560_26727 | chr21 | + | 46097740 | 46097765 | chr2 | + | 86408560 | 86408627 | CTTTGGCAATGGAACATTAT | 1511 | CCTTACTCAGACAAACTCTTGGAG | 1512 | TRUE |
| chr21_chr4_71668498_26815 | chr21 | + | 46136494 | 46136594 | chr4 | - | 71668498 | 71668519 | TGAGCTCCAAGGAGAGGAA | 1513 | GTCAAGGGTCAGATTCTAGTG | 1514 | FALSE |
| chr21_chr10_100581947_26827 | chr21 | + | 46880121 | 46880211 | chr10 | + | 100581947 | 100582038 | CTGTTTGTCCAAAATTCAACCC | 1515 | CATTTGCCAAAGCTCAGA | 1516 | TRUE |
| chr21_chr7_129858633_26893 | chr21 | + | 46883904 | 46883987 | chr7 | + | 129858653 | 129858736 | AATGTTTTATACAGCTCTCAGC | 1517 | TGTTCTAGAAACAGTGCCTT | 1518 | TRUE |
| chr21_chr18_91591147_26929 | chr21 | + | 47084439 | 47084507 | chr18 | + | 91591147 | 91591230 | GGATAGAATATTTCAAGAGGGACTA | 1519 | AAAGCTCTTTGTCTCTGAAAG | 1520 | FALSE |
| chr21_chr1_152965185_26957 | chr21 | + | 47084887 | 47084944 | chr1 | + | 152965185 | 152965272 | ATGATGATAATTGTTCTGAACAC | 1521 | TTATCCATCATTCAAAGGAA | 1522 | FALSE |
| chr21_chr2_102652_26968 | chr21 | + | 48093167 | 48093251 | chr2 | - | 102652 | 102736 | GGAGGTCAAGAGGGGAAAA | 1523 | TTTAACCCAGTCCTCCTCT | 1524 | TRUE |
| chr21_chr2_102202_27092 | chr21 | + | 48093616 | 48093700 | chr2 | - | 102200 | 102289 | TCCTACCAATGAGCCTATAA | 1525 | TTCTGCCATTTGGCATTACAT | 1526 | FALSE |
| chr21_chr2_101929_27166 | chr21 | + | 48093907 | 48093975 | chr2 | - | 101929 | 101998 | TGTAAGTGGCAGTTGCAG | 1527 | TCTACCGTTATGCCACTTGA | 1528 | TRUE |
| chr21_chr2_101651_27186 | chr21 | + | 48094186 | 48094252 | chr2 | - | 101651 | 101720 | ACCAGTGGTTCTGGCTCC | 1529 | AAGCATTCAAGATGAGTTACA | 1530 | FALSE |
| chr21_chr2_101282_27287 | chr21 | + | 48094608 | 48094685 | chr2 | - | 101232 | 101316 | TCTGCTGCCGTAGAGCCT | 1531 | GAGTCCCTGCGGTGTCCT | 1532 | TRUE |
| chr21_chr2_100918_27364 | chr21 | + | 48095021 | 48095113 | chr2 | - | 100918 | 100980 | AGAACCCTTGCCTGACCC | 1533 | GCCATTAGCAAGGCCCTG | 1534 | TRUE |
| chr21_chr2_100379_27422 | chr21 | + | 48095509 | 48095573 | chr2 | - | 100379 | 100448 | TCTGCAGGTCTCTGTCAAA | 1535 | GCTGCTGTTTAGGATAGAGTAGTT | 1536 | TRUE |
| chr21_chr2_132670021_27611 | chr21 | + | 14618375 | 14618468 | chr2 | - | 132670021 | 132670112 | GAATCTTTGTGAATGTATGGA | 1537 | AGGTATAAGTGAGCTAAACCA | 1538 | FALSE |
| chr21_chr2_132652354_27771 | chr21 | + | 14637779 | 14637850 | chr2 | - | 132652354 | 132652429 | CTTGCTGCATCATCCAAGA | 1539 | GCAAGCTTGGCCTCTTT | 1540 | TRUE |
| chr21_chr18_14184352_28162 | chr21 | + | 15347177 | 15347258 | chr18 | - | 14184352 | 14184439 | GCCTATTTCAGGGCATATT | 1541 | TGCAAGGTTATCTTTCCTT | 1542 | TRUE |
| chr21_chr18_14180891_28520 | chr21 | + | 15351487 | 15351566 | chr18 | - | 14180891 | 14180980 | GGTCACAGCTTCATCCCC | 1543 | TAGCTTATTAGTGGGGCAAA | 1544 | TRUE |
| chr21_chr18_14160800_28566 | chr21 | + | 15368615 | 15368697 | chr18 | - | 14160800 | 14160882 | AGTGTCTTGATAGACACAAGT | 1545 | TGCAAAAGGAGCACAGAC | 1546 | TRUE |
| chr21_chr18_14149771_28874 | chr21 | + | 15380746 | 15380828 | chr18 | - | 14149771 | 14149851 | GCAGCCGTCTCTGTCCTC | 1547 | GCCCAACAGAACTGACACA | 1548 | FALSE |
| chr21_chr18_14147468_28932 | chr21 | + | 15383035 | 15383131 | chr18 | - | 14147468 | 14147566 | GCTCTATGACCGGCGTCAG | 1549 | CCGTCATCTGCTGGATCTG | 1550 | TRUE |
| chr21_chr18_14144113_28981 | chr21 | + | 15386893 | 15386948 | chr18 | - | 14144113 | 14144200 | CCCCTAGTCAGGCCGAGA | 1551 | CTTCAAGCACCGGGGACAC | 1552 | FALSE |
| chr21_chr18_14134929_31628 | chr21 | + | 15395748 | 15395834 | chr18 | - | 14134929 | 14135030 | CTTTTACCTGAGCTCAATTTT | 1553 | CATGGTAACTACAAGGTGTCTT | 1554 | TRUE |
| chr21_chr15_46548248_38112 | chr21 | + | 15424134 | 15424220 | chr15 | + | 46548248 | 46548318 | ATGTATATCCAAACAAGGATCT | 1555 | TTGTGTTCTTATGAGCTGTAAA | 1556 | TRUE |
| chr21_chr12_98542328_38832 | chr21 | + | 15440824 | 15440903 | chr12 | - | 98542328 | 98542423 | ATGCGTCTGTAGTCACAGCTCC | 1557 | CAGTGCATTGGCCTGACC | 1558 | FALSE |
| chr21_chr12_98510888_38796 | chr21 | + | 15472019 | 15472095 | chr12 | - | 98510888 | 98510964 | CGAAGTGGGAAAGTTGCAAAA | 1559 | CTTGATGATTTCATGAGGGG | 1560 | TRUE |
| chr21_chr8_131088088_39088 | chr21 | + | 24837269 | 24837349 | chr8 | - | 131088088 | 131088162 | GCATGCATTTAGAAGCTTACCT | 1561 | GCTTTAATGGCAATCAAGTTT | 1562 | TRUE |
| chr21_chr2_132689841_41467 | chr21 | + | 29828282 | 29828394 | chr2 | - | 132689841 | 132689903 | CCCCAACTGTTGAGAGAG | 1563 | ACAGGAGAAATGGACTCCC | 1564 | FALSE |
| chr21_chr2_132876489_41884 | chr21 | + | 29856731 | 29856811 | chr2 | - | 132876489 | 132876579 | CATTGGTCATAACTTCAATGAC | 1565 | CCCTTATGATTTGGGGTA | 1566 | FALSE |
| chr21_chr2_132860033_41980 | chr21 | + | 29932152 | 29932238 | chr2 | - | 132860033 | 132860114 | ACTCATTGATAACTTCTTTTGC | 1567 | ACTGCAACAGAAACAAAACTTGAC | 1568 | TRUE |

FIG. 8 continued

FIG. 8

| marker.name | chr.tar | strand.tar | start.tar | end.tar | chr.ref | strand.ref | start.ref | end.ref | Primer | SEQ ID NO. | Primer | SEQ ID NO. | included.in.final |
|---|---|---|---|---|---|---|---|---|---|---|---|---|---|
| chr21_chr18_15064952_43551 | chr21 | + | 29887696 | 29887752 | chr18 | - | 15064952 | 15064618 | TGATGGCCAGAGAAACAGG | 1569 | AAAGATGATCCTGAATGGG | 1570 | TRUE |
| chr21_chr18_15023356_43576 | chr21 | + | 29428893 | 29428919 | chr18 | - | 15023356 | 15023444 | GCAAAATCTTATTACCAAGTGT | 1571 | ATTTCAATAGCTGGCATTT | 1572 | FALSE |
| chr21_chr11_93341473_43884 | chr21 | + | 30247261 | 30247336 | chr11 | + | 93341473 | 93341548 | CTGTCCTTCAAGGTAGCCCA | 1573 | GTATGATATGCAGGTACCACC | 1574 | TRUE |
| chr21_chr3_142548200_45191 | chr21 | + | 31830967 | 31830026 | chr3 | + | 142548200 | 142548259 | CAGCCACCACACCAACCA | 1575 | CCTCTGGCTCAAGAAGGT | 1576 | TRUE |
| chr21_chr6_154871098_45681 | chr21 | + | 32825158 | 32825254 | chr6 | + | 154871098 | 154871187 | CAAAGACTATCAAGACCTGG | 1577 | GCATCTTCCACTTTGTTTC | 1578 | TRUE |
| chr21_chr5_171337800_46904 | chr21 | + | 33900708 | 33900771 | chr5 | + | 171337800 | 171337862 | GCTGCAACATGGGCTTCA | 1579 | TTTCACGAATGTGTCATTATC | 1580 | FALSE |
| chr21_chr19_7879118_46881 | chr21 | + | 33889257 | 33889318 | chr19 | - | 7879118 | 7879199 | GAAGGTGCAACTAAAAGAAAC | 1581 | TGCCTTATTAGCAAGCACAA | 1582 | FALSE |
| chr21_chr6_161208338_48906 | chr21 | + | 33994821 | 33994880 | chr6 | - | 161208338 | 161208408 | CCCCTTAGATAGGAATTTGAGC | 1583 | CAAGGTACCCACTGGACCC | 1584 | TRUE |
| chr21_chr1_51912251_51604 | chr21 | + | 35497708 | 35497783 | chr1 | + | 51912251 | 51912329 | ACTTGGAAAACCTGGGTGA | 1585 | TACCGGCCTCTGTTGTGC | 1586 | TRUE |
| chr21_chr7_148806418_52015 | chr21 | + | 36972190 | 36972258 | chr7 | + | 148806418 | 148806482 | TTTGGATTTACCGAATGA | 1587 | ATTTTGTGGTGGATCCAA | 1588 | FALSE |
| chr21_chr6_106115024_54509 | chr21 | + | 37781570 | 37781798 | chr6 | + | 106115024 | 106115099 | AGGCCCATCGCTCATCTT | 1589 | ACCACTGGCTCAGTTCAA | 1590 | TRUE |
| chr21_chr10_95719404_55487 | chr21 | + | 37859105 | 37859164 | chr10 | + | 95719404 | 95719467 | GCTTCAGAGCCAGATGGG | 1591 | GTCCCACCCAAGGGCAAG | 1592 | FALSE |
| chr21_chr12_96311552_54590 | chr21 | + | 40668690 | 40668791 | chr12 | + | 96311552 | 96311644 | AGTCCATTTAATGCCAAGTG | 1593 | TGAAAAGTTGGATCAGTTGT | 1594 | TRUE |
| chr21_chr1_228489676_58520 | chr21 | + | 43418882 | 43418961 | chr1 | + | 228489676 | 228489776 | TGTTCCTCTTGTTATTGCTT | 1595 | GACAAAACATGTCTCTACAATG | 1596 | FALSE |
| chr21_chr2_98371752_58156 | chr21 | + | 46096122 | 46096216 | chr2 | + | 98371752 | 98371826 | CTACCCTTCGGGCCAGTT | 1597 | AGCTATCCTCCAAGACTGCC | 1598 | FALSE |
| chr21_chr13_19184866_61514 | chr21 | + | 14593016 | 14593109 | chr13 | + | 19184868 | 19184961 | CAGAACATTTATTTCTATTGCTG | 1599 | GCAACCAAGTAACTATGCAC | 1600 | TRUE |
| chr21_chr2_132697342_61888 | chr21 | + | 14595790 | 14595875 | chr2 | - | 132697342 | 132697425 | GCAGTTCGAGAAATGAAATAGA | 1601 | TTAGACCCTCATTTGCCCA | 1602 | FALSE |
| chr21_chr2_132883019_63103 | chr21 | + | 14605366 | 14605456 | chr2 | - | 132883019 | 132883112 | GGAGTGTTAAGTTTGCAGAAGC | 1603 | GGTGGGTACACCAGATATACAG | 1604 | FALSE |
| chr21_chr2_132673794_63667 | chr21 | + | 14614602 | 14614671 | chr2 | - | 132673794 | 132673852 | TCTGGAAGCTGTGAGCTCTTC | 1605 | TGCAGACTAAGACAAAGGTTTT | 1606 | TRUE |
| chr21_chr2_132886825_63823 | chr21 | + | 14623355 | 14623454 | chr2 | - | 132886825 | 132886924 | GAGTCCCTCACCCCATTCTT | 1607 | TTACCCTGCCATCAAGGG | 1608 | TRUE |
| chr21_chr2_132665191_66036 | chr21 | + | 14633191 | 14633278 | chr2 | - | 132665191 | 132665277 | CCTGGCAGCAACCATTCTAT | 1609 | AGGCAAATACCCTGTGATTC | 1610 | TRUE |
| chr21_chr2_132883874_83145 | chr21 | + | 14624828 | 14624917 | chr2 | - | 132883874 | 132883963 | AAGGCAATGCACAGGAGAAA | 1611 | TGCTGAGTTTAGGAGGTGTG | 1612 | TRUE |
| chr21_chr2_132659087_69809 | chr21 | + | 14637081 | 14637121 | chr2 | - | 132659087 | 132659127 | GGGGACAATAAGAGTCCTACA | 1613 | TGCAACAACAAGTACAACCAA | 1614 | TRUE |
| chr21_chr18_14355882_83838 | chr21 | + | 15201321 | 15201403 | chr18 | - | 14355882 | 14355964 | TGGCCATGTTCAATACACTTC | 1615 | TCAGGGATTTGGAAGAGTGA | 1616 | TRUE |
| chr21_chr18_14355386_64008 | chr21 | + | 15002835 | 15002924 | chr18 | - | 14355386 | 14355470 | ACCTACAGACAGAAACACACC | 1617 | CCTGATGTATGATGTCTTTGCC | 1618 | TRUE |
| chr21_chr18_14353381_64829 | chr21 | + | 15204511 | 15204603 | chr18 | - | 14353381 | 14353473 | TTGTTTCTTTTCTCTCTCCC | 1619 | AGACCAAAGTCCAGAAGGCA | 1620 | TRUE |
| chr21_chr18_14192545_66809 | chr21 | + | 15333975 | 15334074 | chr18 | - | 14192545 | 14192644 | TGTCAGGATCTACTTATGCTTT | 1621 | TGTTCTTGCCAATAAAGTAAGCA | 1622 | TRUE |
| chr21_chr13_19443978_67549 | chr21 | + | 15341064 | 15341152 | chr13 | + | 19443978 | 19444067 | AGTTGTGATTAGCCCAGGAGA | 1623 | TTCCACCGAGTAGTGGGAAC | 1624 | FALSE |
| chr21_chr18_14178728_67798 | chr21 | + | 15361784 | 15361876 | chr18 | - | 14178728 | 14178818 | TGATAGCGCCCCTAACCC | 1625 | CGCTATTGGGTTCTACACTG | 1626 | TRUE |
| chr21_chr18_14176568_68248 | chr21 | + | 15359491 | 15359583 | chr18 | - | 14176568 | 14176660 | TTCCTCGGACCTGTGCTA | 1627 | ATGCTAAGGGCATGCCCAG | 1628 | TRUE |
| chr21_chr18_14170287_68257 | chr21 | + | 15362090 | 15362139 | chr18 | - | 14170287 | 14170348 | TCAGTAATCACTCCCTGTCCC | 1629 | AAAACTAGAATAGTGAGTTGCC | 1630 | TRUE |
| chr21_chr18_14169506_68278 | chr21 | + | 15361019 | 15361081 | chr18 | - | 14169506 | 14169568 | CCCTCTGAGCTAATTAGTGCTAT | 1631 | ACGAGTAAGTAAATGTGAGTGGA | 1632 | FALSE |
| chr21_chr18_14169193_68422 | chr21 | + | 15361323 | 15361395 | chr18 | - | 14169193 | 14169265 | TTTTGACACCATAGCTAAGTC | 1633 | AGAATTAAGAGCATCAGTTAAGA | 1634 | TRUE |
| chr21_chr18_14159649_68865 | chr21 | + | 15370875 | 15370946 | chr18 | - | 14159649 | 14159719 | TGTGGAGTTTCTTCTTCATTC | 1635 | CAAGATGCTTCAACAACACAA | 1636 | FALSE |
| chr21_chr18_14158874_68987 | chr21 | + | 15371141 | 15371229 | chr18 | - | 14158874 | 14158953 | TTTTGGAAGTGTTTGAGAAA | 1637 | GAAAGGTCAGGACCAGATAGC | 1638 | FALSE |
| chr21_chr18_14158648_69017 | chr21 | + | 15371868 | 15371938 | chr18 | - | 14158648 | 14158722 | TGTTGTCTGTTTGTTCTCACTCT | 1639 | CACCAAAGCTAGCAGAAGAAA | 1640 | TRUE |
| chr21_chr18_14156882_69079 | chr21 | + | 15379463 | 15379752 | chr18 | - | 14156882 | 14156908 | CAGGGCCTGAAAAGGAACCTA | 1641 | TTCAGTTGCCAGAAGCAGC | 1642 | FALSE |
| chr21_chr18_14155877_69138 | chr21 | + | 15374650 | 15374768 | chr18 | - | 14155877 | 14155936 | GAATACTCTTAGCCCTTTCACAGC | 1643 | ACTTAAATGGTAAGGTGGCAA | 1644 | TRUE |
| chr21_chr18_14155244_69259 | chr21 | + | 15377288 | 15377353 | chr18 | - | 14155244 | 14155314 | TGGTTATGCCAGTTAGAGTAAGA | 1645 | CCTCTAGGGAATAATATAGACAGA | 1646 | TRUE |
| chr21_chr18_14152281_69562 | chr21 | + | 15378226 | 15378314 | chr18 | - | 14152281 | 14152368 | GCAATGCTCTCCAATAGTAA | 1647 | CAATTAACTTTACCTGCTTCTT | 1648 | FALSE |
| chr21_chr18_14150177_69548 | chr21 | + | 15380350 | 15380420 | chr18 | - | 14150177 | 14150247 | AGTGTCTGGAGGCAGGC | 1649 | AGGTTCACCTCCAGAGCAAG | 1650 | FALSE |
| chr21_chr18_14149319_69669 | chr21 | + | 15381239 | 15381278 | chr18 | - | 14149319 | 14149378 | CAGGACCATAGGGTGAGGG | 1651 | CCTGAGCTTGCTAGAGGC | 1652 | FALSE |
| chr21_chr18_14149090_69839 | chr21 | + | 15381474 | 15381548 | chr18 | - | 14149090 | 14149129 | ACAGACAGAGACACACACCA | 1653 | GCAACCTTCCTTCACCCTG | 1654 | FALSE |
| chr21_chr18_14148150_69922 | chr21 | + | 15382352 | 15382429 | chr18 | - | 14148150 | 14148247 | CCATATGCCTGAGACCAACC | 1655 | GCTGTGTAAGGAGGTGCCC | 1656 | FALSE |
| chr21_chr18_14147658_69994 | chr21 | + | 15382867 | 15382941 | chr18 | - | 14147658 | 14147732 | ATGAGACGTATCTCAGATGT | 1657 | GACTGTTCAGGGCTCAGAT | 1658 | TRUE |
| chr21_chr18_14142489_70915 | chr21 | + | 15388236 | 15388324 | chr18 | - | 14142489 | 14142581 | GTTTCCAAGCTGTGAAGG | 1659 | GTAGGACAGGTGGGACGCT | 1660 | FALSE |
| chr21_chr18_14138113_70827 | chr21 | + | 15391938 | 15391940 | chr18 | - | 14138113 | 14138205 | GGCTACCTGGGTAGTCATGG | 1661 | TCTCACCTGATGTGGAAGTAGC | 1662 | TRUE |
| chr21_chr18_14136544_71225 | chr21 | + | 15394133 | 15394213 | chr18 | - | 14136544 | 14136622 | CTGCTCTATCTCACCCTCCC | 1663 | TCAGATTGTGAATCCTGAGCA | 1664 | TRUE |
| chr21_chr18_14134581_71366 | chr21 | + | 15396080 | 15396161 | chr18 | - | 14134581 | 14134672 | AAGTGGGGATGGGGAATG | 1665 | GACTGATGCTCAGCCTAAAC | 1666 | FALSE |

FIG. 8 continued

FIG. 8

| markerline | chr.tar | strand.tar | start.tar | end.tar | chr.ref | strand.ref | start.ref | end.ref | FPrimer | SEQ ID NO. | RPrimer | SEQ ID NO. | included in final |
|---|---|---|---|---|---|---|---|---|---|---|---|---|---|
| chr21_chr18_14134543_71788 | chr21 | + | 15390318 | 15390408 | chr18 | - | 14134543 | 14134458 | AGGCCAGTTCTATCCTGGCC | 1667 | TGCATACAGAAATATGTGTCCAA | 1668 | TRUE |
| chr21_chr18_14129110_71808 | chr21 | + | 15403015 | 15403089 | chr18 | - | 14129110 | 14129198 | GGTTTCTTGGACTACCTGATCATA | 1669 | GCTCAGGCAGGCTATGT | 1670 | TRUE |
| chr21_chr18_14126144_71880 | chr21 | + | 15406021 | 15406085 | chr18 | - | 14126144 | 14126209 | ATCTGTCCAAGCCTGGG | 1671 | GTGTGGGATCAGACCTGGAG | 1672 | TRUE |
| chr21_chr15_46542004_73327 | chr21 | + | 15417378 | 15417438 | chr15 | + | 46542004 | 46542064 | CAATACCATCAACCAATTAGA | 1673 | TGGCTGTCTTTGTGGAATG | 1674 | FALSE |
| chr21_chr15_46547394_73406 | chr21 | + | 15422904 | 15422998 | chr15 | + | 46547394 | 46547489 | ATGGTTTCACTGGGAATTT | 1675 | GAGATGCCCCTCTGGTTCT | 1676 | TRUE |
| chr21_chr15_46549772_73481 | chr21 | + | 15424689 | 15424752 | chr15 | + | 46549772 | 46549835 | TGAACTCCATATGCTACAACA | 1677 | TCTGGCATCTTTCAGTCAGC | 1678 | TRUE |
| chr21_chr12_38594774_73697 | chr21 | + | 15443748 | 15443816 | chr12 | - | 38594774 | 38594842 | CAGCTACCAGGAAAACGTCC | 1679 | GGACGGGAGTTTACACGAAG | 1680 | TRUE |
| chr21_chr12_38543962_73782 | chr21 | + | 15448182 | 15448259 | chr12 | - | 38543962 | 38544039 | TGCATCTAGAGGCCCAATCT | 1681 | TCACTCCAAGTTGGCATTT | 1682 | TRUE |
| chr21_chr2_38843454_74083 | chr21 | + | 15474389 | 15474459 | chr2 | + | 38843454 | 38843513 | GAAGGAGGCCCTGGTGTACT | 1683 | CACAGATTCCTCCCATAGC | 1684 | TRUE |
| chr21_chr13_72148819_74404 | chr21 | + | 15721123 | 15721323 | chr13 | + | 72148819 | 72148918 | TCAGCAATCAAACAAAGCAAT | 1685 | AAGCTGTCATGGTTTCTTGT | 1686 | TRUE |
| chr21_chr1_52558866_74783 | chr21 | + | 15888543 | 15888617 | chr1 | - | 52558866 | 52558941 | TTCCCAATTTCAACCTTGCT | 1687 | GCCTTAGCTGTATCCTAGCTG | 1688 | TRUE |
| chr21_chr1_52553089_74809 | chr21 | + | 15890125 | 15890191 | chr1 | - | 52553089 | 52553155 | AAAAGCTGACCAGGCCAT | 1689 | TACTAGCTTGCTTCCTAAATGCC | 1690 | TRUE |
| chr21_chr1_52552785_74893 | chr21 | + | 15890403 | 15890487 | chr1 | - | 52552785 | 52552879 | ACTCACTTTCCATTAATTCTGTG | 1691 | ATTTATCCTCCTCCCTCCC | 1692 | TRUE |
| chr21_chr12_19264682_74951 | chr21 | + | 15941622 | 15941698 | chr12 | - | 19264682 | 19264758 | TCCAAGAGTGGTTTGGGAAC | 1693 | TTGCACAATGTACTAAGTCAAC | 1694 | TRUE |
| chr21_chr5_182069624_74958 | chr21 | + | 15905830 | 15905939 | chr5 | - | 182069624 | 182069885 | TTACTTTGTAGTCTAGGGTCCTTG | 1695 | GTGTAATTTAATAGAACAAACCCC | 1696 | TRUE |
| chr21_chr2_96173549_75087 | chr21 | + | 15912058 | 15912132 | chr2 | + | 96173549 | 96173628 | GCTGGTGGGTTTGTGTGTA | 1697 | CAAGATGCAAGAGCTCTAAA | 1698 | FALSE |
| chr21_chr22_22878980_75951 | chr21 | + | 22989563 | 22989681 | chr22 | + | 22878980 | 22879084 | AAAGTGAAGCAACTGAATGGCA | 1699 | TAGAGGTGGTTGTTGCACAG | 1700 | TRUE |
| chr21_chr5_137949232_75717 | chr21 | + | 23248420 | 23248490 | chr5 | - | 137949232 | 137949280 | AGCTCTGTGAAAGGAAGAAAA | 1701 | TTCCCAGGGTTTTCTGAGC | 1702 | TRUE |
| chr21_chr5_137948448_75795 | chr21 | + | 23249186 | 23249265 | chr5 | - | 137948448 | 137948527 | GTCATATAATCCAACAATCCC | 1703 | AAATGCAGACATCTCTTGGAG | 1704 | FALSE |
| chr21_chr11_124648952_75882 | chr21 | + | 24479776 | 24479852 | chr11 | - | 124648952 | 124649028 | GATGCCCAGAACTGAATGCT | 1705 | GCTATGAAGGTGGCTCTTCC | 1706 | TRUE |
| chr21_chr12_75780766_76041 | chr21 | + | 25086505 | 25086601 | chr12 | + | 75780766 | 75780861 | GCAGCAGATATCTAAATGGTCC | 1707 | TGATATGCTGTGTTTCCATTAACA | 1708 | TRUE |
| chr21_chr7_68358019_76133 | chr21 | + | 27141940 | 27141993 | chr7 | + | 68358019 | 68358084 | TTGAGCCCCAGGACATTC | 1709 | AAGAGCTATACTTGCTCTAAACA | 1710 | TRUE |
| chr21_chr7_68355370_76299 | chr21 | + | 27141988 | 27143949 | chr7 | + | 68355370 | 68355431 | CTCTAGGAAACAGTCTGGCTT | 1711 | AGCAGCAGAGACGGGAAAC | 1712 | TRUE |
| chr21_chr7_68358910_76613 | chr21 | + | 27142426 | 27142511 | chr7 | + | 68358910 | 68358998 | TGCAAAGCAGCTTATATTCC | 1713 | ACTAATGCCTGAAAGGTAAA | 1714 | TRUE |
| chr21_chr7_68358451_76672 | chr21 | + | 27143958 | 27144039 | chr7 | + | 68358451 | 68358532 | GCTTCTTAAGAGCTCAAGTTAATGC | 1715 | TGCTAACAATGCCATCTTGC | 1716 | TRUE |
| chr21_chr7_68358036_76823 | chr21 | + | 27144522 | 27144585 | chr7 | + | 68358036 | 68358098 | CACTTGCTGCCAGGAAAA | 1717 | GTATGCAAAAGTCCTCCTCCC | 1718 | FALSE |
| chr21_chr6_114184647_76881 | chr21 | + | 27599916 | 27599980 | chr6 | - | 114184647 | 114184611 | GGAATTGGATCAAATGATTA | 1719 | TAATGGCATTTGCTGTGGT | 1720 | FALSE |
| chr21_chr10_31326780_77005 | chr21 | + | 27735286 | 27735345 | chr10 | - | 31326780 | 31326845 | GATTAGGGCCTCTGTCCTTATGA | 1721 | GTAAGACAAATGAACTAAGGAGGT | 1722 | TRUE |
| chr21_chr8_72803948_77013 | chr21 | + | 27902807 | 27902879 | chr8 | + | 72803948 | 72804020 | TCCTAAACTTAAAGAGAAAGTTG | 1723 | GAAAATGCTGCAGACAAAA | 1724 | FALSE |
| chr21_chr2_132681307_78082 | chr21 | + | 28280541 | 28280648 | chr2 | - | 132681307 | 132681458 | GTCACCCATCCCACAAAC | 1725 | ATAGATATGCTTAACATACCAGTG | 1726 | TRUE |
| chr21_chr2_132690563_78284 | chr21 | + | 28281379 | 28281458 | chr2 | - | 132690563 | 132690622 | GTAGATTTTGCCCACGTTTT | 1727 | GACCTAAATGTAAAATGACAAAC | 1728 | TRUE |
| chr21_chr2_132690152_78378 | chr21 | + | 28281752 | 28281830 | chr2 | - | 132690152 | 132690230 | AGAGCCTTGCTTTCCATA | 1729 | GCAAACTCAGTCAAAACCTA | 1730 | TRUE |
| chr21_chr2_132689424_78546 | chr21 | + | 28291461 | 28291552 | chr2 | - | 132689424 | 132689483 | ACTTGCTTAGATATATGATTGTG | 1731 | TATCTACAAGATATTGGGGCA | 1732 | TRUE |
| chr21_chr2_132680887_79101 | chr21 | + | 28931317 | 28931378 | chr2 | - | 132680887 | 132680948 | CTCTTTCCAGCCTCCACCC | 1733 | TGAAAACACAACAAGGGAACA | 1734 | FALSE |
| chr21_chr2_132680646_79139 | chr21 | + | 28931527 | 28931619 | chr2 | - | 132680646 | 132680758 | TGGTGCATATGTGCTTATGTGT | 1735 | ATTCACTACAGCAAAGACATGGA | 1736 | TRUE |
| chr21_chr2_132680500_79144 | chr21 | + | 28931896 | 28931766 | chr2 | - | 132680500 | 132680571 | TGGGATTCCTGAGTCTAATGG | 1737 | TCATTGCAGAAAGCAGTATGA | 1738 | TRUE |
| chr21_chr18_15084790_79883 | chr21 | + | 29367488 | 29367573 | chr18 | - | 15084790 | 15084829 | AAGACCCAGAGCAATCCCTT | 1739 | TGCACTATCAGACACTAGCATTC | 1740 | TRUE |
| chr21_chr18_15077325_79976 | chr21 | + | 29374889 | 29374968 | chr18 | - | 15077325 | 15077401 | ACCCAGGCTTTTGAACTTGC | 1741 | GAGGAAATGAGCCATGGAAA | 1742 | TRUE |
| chr21_chr18_15071767_80104 | chr21 | + | 29380407 | 29380479 | chr18 | - | 15071767 | 15071839 | GGGTTGACACTTTCCAGATCA | 1743 | TGTTTCTTATTTCCTGGCTACAT | 1744 | FALSE |
| chr21_chr16_20746100_81903 | chr21 | + | 30271395 | 30271421 | chr16 | + | 20746100 | 20746172 | AGTGTGAGCACTAAGTTGAAGA | 1745 | TCAGGCCTAGTCTGACAGTCC | 1746 | TRUE |
| chr21_chr1_146876210_82013 | chr21 | + | 30489964 | 30489229 | chr1 | - | 146876210 | 146876282 | TTGTGCCATCTCCAACTTCT | 1747 | GTACAGCATCCTGCTGCAAA | 1748 | TRUE |
| chr21_chr19_48713078_82096 | chr21 | + | 32289095 | 32289118 | chr19 | + | 48713078 | 48713138 | GATGAGAAGGATATAAAAGTTTG | 1749 | AAGGCCTTCAAAATAAGAAT | 1750 | FALSE |
| chr21_chr5_28115725_82097 | chr21 | + | 32894820 | 32894912 | chr5 | + | 28115725 | 28115817 | TTGGCCAACATCTCAACAGA | 1751 | TCATTTAGCATTCCCAGACTCA | 1752 | TRUE |
| chr21_chr6_107361027_82218 | chr21 | + | 32824841 | 32824901 | chr6 | + | 107361027 | 107361087 | ACTCTGGGGTGTTCTCCGA | 1753 | TCGATTCCAGGAAGTACAGA | 1754 | TRUE |
| chr21_chr5_171288771_82301 | chr21 | + | 32898542 | 32898623 | chr5 | + | 171288771 | 171288858 | TTCAATAGCTGTCCAGACACAA | 1755 | GCACTGGGATAGTCTAATTCT | 1756 | TRUE |
| chr21_chr5_171295683_82508 | chr21 | + | 32989484 | 32989548 | chr5 | + | 171295683 | 171295747 | CATTCTGGGCACTGGGAG | 1757 | CAGCTCCCATGATGACACTA | 1758 | TRUE |
| chr21_chr6_156567478_82879 | chr21 | + | 33913411 | 33913484 | chr6 | + | 156567478 | 156567561 | CCTGACCACTACTCTTCAAAACA | 1759 | CCCTAGTTTCCACCAATCTG | 1760 | TRUE |
| chr21_chr6_156568019_82942 | chr21 | + | 33914262 | 33914321 | chr6 | + | 156568019 | 156568078 | GGTGCCATGTCTCCATGTATC | 1761 | CTCATCACTACAGCAAGCC | 1762 | TRUE |
| chr21_chr6_156568524_82879 | chr21 | + | 33914785 | 33914864 | chr6 | + | 156568524 | 156568605 | AAAATAGAACACAGAGTGTAGGA | 1763 | ATTCAATTTCCTTGACCAAT | 1764 | FALSE |

FIG. 8 continued

FIG. 8

| markername | chr.tar | strand.tar | start.tar | end.tar | chr.ref | strand.ref | start.ref | end.ref | Fprimer | SEQ ID NO. | Rprimer | SEQ ID NO. | included.in.final |
|---|---|---|---|---|---|---|---|---|---|---|---|---|---|
| chr21_chr6_156571252_82981 | chr21 | + | 33917927 | 33917988 | chr6 | + | 156571252 | 156571313 | AATCAGGCTTTGGACAGGC | 1765 | GACTTGAGCAACACAAATGTATGA | 1766 | TRUE |
| chr21_chr6_156572579_83057 | chr21 | + | 33938259 | 33938328 | chr6 | + | 156572579 | 156572648 | ACCATTATGTTGTTCCTTTC | 1767 | GCCCTGTCTCTCTGTAGCTTT | 1768 | TRUE |
| chr21_chr6_156572887_83105 | chr21 | + | 33919568 | 33919627 | chr6 | + | 156572887 | 156572946 | CGGCCCTCCTTTCTGAAG | 1769 | AAGAAACTTGGAGGGTGGG | 1770 | FALSE |
| chr21_chr6_155780389_83480 | chr21 | + | 33939949 | 33940038 | chr6 | + | 155780389 | 155780468 | TAGGATCTGAAGGGCTCCCA | 1771 | ACAGCATAGCTCCACTGCC | 1772 | TRUE |
| chr21_chr1_157678984_83699 | chr21 | + | 34027587 | 34027648 | chr1 | + | 157678984 | 157679023 | TAAGGCGATCAAGAACACC | 1773 | TTTTACAAGGGATGGGAGAT | 1774 | TRUE |
| chr21_chr1_157679123_83765 | chr21 | + | 34029510 | 34029589 | chr1 | + | 157679123 | 157679182 | GTGCTAAGGCTGGTGTCC | 1775 | CTATAGTAGGTGGAGGTGCAGG | 1776 | FALSE |
| chr21_chr1_157679304_83779 | chr21 | + | 34031604 | 34031663 | chr1 | + | 157679304 | 157679363 | TGAAGAGGAAGAAGAACCTCC | 1777 | GGTTTGGGTTCACTGAGTTT | 1778 | TRUE |
| chr21_chr1_157679811_83827 | chr21 | + | 34045628 | 34045697 | chr1 | + | 157679811 | 157679877 | AGAGCAGCCCGGGTAACT | 1779 | CTCCTTCTCTCCAGCCCATT | 1780 | TRUE |
| chr21_chr1_157681023_84023 | chr21 | + | 34049553 | 34049639 | chr1 | + | 157681023 | 157681113 | TTGAATTTCAGCTACACCTAGA | 1781 | AATAGTGACTGGTTGCCAAT | 1782 | TRUE |
| chr21_chr12_8806581_84701 | chr21 | + | 37423113 | 37423207 | chr12 | + | 8806581 | 8806685 | ATGGACGGGATGTACGTGTC | 1783 | TGAGCAGTTGCTTTGCATTC | 1784 | TRUE |
| chr21_chr5_85786088_84749 | chr21 | + | 37424406 | 37424468 | chr5 | - | 85786088 | 85786183 | TTGGACTTCTTGTGTTTGTGA | 1785 | TCAGCAAGGGAATTCAGGTTA | 1786 | FALSE |
| chr21_chr6_106114577_84956 | chr21 | + | 37786044 | 37786099 | chr6 | + | 106114577 | 106114672 | GGGGCCACTTAGAAGATGTG | 1787 | CATCCCATATGATGGCTGTG | 1788 | TRUE |
| chr21_chr10_95718684_84985 | chr21 | + | 37858403 | 37858459 | chr10 | + | 95718684 | 95718743 | GGACACTCTGAAGGAACTCAGG | 1789 | CAGCCAAGGAGGAGGATGAT | 1790 | TRUE |
| chr21_chr17_48631958_85198 | chr21 | + | 39610348 | 39610424 | chr17 | - | 48631958 | 48632024 | TCGGAGGCTACTCAGGAAAG | 1791 | GATTCTGGCTGATGGGGAC | 1792 | FALSE |
| chr21_chr3_140506037_85808 | chr21 | + | 41773434 | 41773523 | chr3 | + | 140506037 | 140506048 | ATCCACCATTGCAGATGTCA | 1793 | TTTAGGGATTTGCAAGGTCA | 1794 | TRUE |
| chr21_chr2_111192980_85771 | chr21 | + | 44078367 | 44078431 | chr2 | + | 111192980 | 111192994 | AACTCGATGAAAGTGGCC | 1795 | CCAAGGGGAGAAGTCTGAGGA | 1796 | TRUE |
| chr21_chr2_111193217_85800 | chr21 | + | 44078553 | 44078613 | chr2 | + | 111193217 | 111193277 | CCAACACGCAGAGTGGCA | 1797 | CATGCAGTGTCTGCAGTTTC | 1798 | TRUE |
| chr21_chr2_111194922_86134 | chr21 | + | 44080257 | 44080319 | chr2 | + | 111194922 | 111194989 | TCAGTGGCATGTGCAGG | 1799 | GAAGCAGAGAAATGCGGG | 1800 | TRUE |

FIG. 8 continued

FIG. 8

Europäisches Patentamt
European Patent Office
Office européen des brevets

# EUROPEAN SEARCH REPORT

Application Number
EP 21 21 5237

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ARMOUR JOHN A L ET AL: "Accurate, high-throughput typing of copy number variation using paralogue ratios from dispersed repeats",<br>NUCLEIC ACIDS RESEARCH, INFORMATION RETRIEVAL LTD,<br>vol. 35, no. 3,<br>14 December 2006 (2006-12-14), page E19, XP002473374,<br>ISSN: 0305-1048, DOI: 10.1093/NAR/GKL1089<br>* pages 2-3; figures 1, 2 *<br>----- | 1-13 | INV.<br>C12Q1/68<br>G16B25/00<br>G16B30/00<br>G16B35/00<br>G16C20/60<br>G16H15/00<br>C12Q1/6844<br>G16B30/10<br>G16H50/30 |
| A | WALKER S ET AL: "Multiplex Paralogue Ratio Tests for accurate measurement of multiallelic CNVs",<br>GENOMICS, ACADEMIC PRESS, SAN DIEGO, US,<br>vol. 93, no. 1,<br>1 January 2009 (2009-01-01), pages 98-103, XP025745539,<br>ISSN: 0888-7543, DOI: 10.1016/J.YGENO.2008.09.004<br>[retrieved on 2008-10-22]<br>* page 102, "Materials and methods" *<br>----- | 1-13 | TECHNICAL FIELDS SEARCHED (IPC)<br>C12Q<br>G06F |
| X | C. D. VEAL ET AL: "Automated design of paralogue ratio test assays for the accurate and rapid typing of copy number variation",<br>BIOINFORMATICS.,<br>vol. 29, no. 16, 6 June 2013 (2013-06-06), pages 1997-2003, XP055335230,<br>GB<br>ISSN: 1367-4803, DOI: 10.1093/bioinformatics/btt330<br>* item 2; figures *<br>-----<br>-/-- | 1-13 | |

The present search report has been drawn up for all claims

| Place of search | Date of completion of the search | Examiner |
|---|---|---|
| Munich | 8 July 2022 | Hennard, Christophe |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 21 5237

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2005/037388 A1 (ANTONARAKIS STYLIANOS [CH] ET AL) 17 February 2005 (2005-02-17) * claims; [0011] - [0015]; [0099]; [0106] - [0115]; examples * ----- | 1-13 | |
| A | WO 2008/157264 A2 (SEQUENOM INC [US]; STYLLI CHARI GEORGIOU [US]) 24 December 2008 (2008-12-24) * page 3, lines 4-9; page 6, lines 8-17 * ----- | 1-13 | |
| X,P | C. K. ELLISON, ET AL: "Using Targeted Sequencing of Paralogous Sequences for Noninvasive Detection of Selected Fetal Aneuploidies.", CLINICAL CHEMISTRY, vol. 62, no. 12, 30 September 2016 (2016-09-30), pages 1621-1629, XP009195289, DOI: 10.1373/clinchem.2016.260034 * pages 1622-1624 * ----- | 1-13 | |
| | | | TECHNICAL FIELDS SEARCHED (IPC) |

The present search report has been drawn up for all claims

2

| Place of search | Date of completion of the search | Examiner |
|---|---|---|
| Munich | 8 July 2022 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 21 21 5237

This annex lists the patent family members relating to the patent documents cited in the above-mentioned European search report. The members are as contained in the European Patent Office EDP file on
The European Patent Office is in no way liable for these particulars which are merely given for the purpose of information.

08-07-2022

| Patent document cited in search report | Publication date | Patent family member(s) | Publication date |
|---|---|---|---|
| US 2005037388 A1 | 17-02-2005 | NONE | |
| WO 2008157264 A2 | 24-12-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62342839
- US 20050112590
- US 6927028 B
- WO 2007140417 A
- WO 2007147063 A
- WO 2009032779 A
- WO 2009032781 A
- WO 2010033639 A
- WO 2011034631 A
- WO 2006056480 A
- WO 2011143659 A
- US 20100105049
- US 20090317818
- US 20110224087
- WO 2014055774 A
- US 20130288244
- US 20130338933
- WO 2013177086 A
- WO 2014205401 A
- US 20130012399
- WO 2013052907 A
- US 20140180594
- WO 2013052913 A
- WO 2015051163 A
- WO 2014190286 A
- US 5945526 A, Lee
- WO 2013055817 A
- WO 2013109981 A
- WO 2013192562 A
- WO 2014116598 A
- WO 2015138774 A
- WO 2015054080 A
- WO 2015183872 A
- WO 2016019042 A
- WO 2016057901 A


### Non-patent literature cited in the description


- **T. STRACHAN.** The Human Genome. BIOS Scientific Publishers, 1992
- **D. N. COOPER ; M. KRAWCZAK.** Human Genome Mutations. BIOS Publishers, 1993
- **SAMBROOK ; RUSSELL.** Molecular Cloning: A Laboratory Manual. 2001
- **LO.** *Journal of Histochemistry and Cytochemistry,* 2005, vol. 53 (3), 293-296
- **CHAN et al.** *Clin. Chem.,* 2004, vol. 50, 88-92
- **LO et al.** *Sci. Transl. Med.,* 2010, vol. 2, 61-91
- **OLSHEN et al.** *Biostatistics,* 2004, vol. 5 (4), 557-72
- **VENKATRAMAN, ES ; OLSHEN, AB.** *Bioinformatics,* 2007, vol. 23 (6), 657-63
- **HAAR, ALFRED.** *Mathematische Annalen,* 1910, vol. 69 (3), 331-371
- **HSU et al.** *Biostatistics,* 2005, vol. 6 (2), 211-226
- **Y. WANG ; S. WANG.** *International Journal of Bioinformatics Research and Applications,* 2007, vol. 3 (2), 206-222
- **NGUYEN et al.** *Proceedings of the 7th IEEE International Conference, Boston MA,* 2007, 137-144
- **EUGENE, OR.** The Handbook of Fluorescent Probes and Research Products. Molecular Probes, Inc,
- **JU.** *Proc. Nat'l Acad. Sci. (USA),* 1995, vol. 92, 4347
- *Physiol Genomics,* 2000, vol. 3, 93-99
- **LO YM ; CORBETTA N ; CHAMBERLAIN PF ; RAI V ; SARGENT IL et al.** Presence of fetal DNA in maternal plasma and serum. *Lancet,* 1997, vol. 350, 485-487
- **LO YM ; TEIN MS ; LAU TK ; HAINES CJ ; LEUNG TN et al.** Quantitative analysis of fetal DNA in maternal plasma and serum: implications for noninvasive prenatal diagnosis. *Am J Hum Genet,* 1998, vol. 62, 768-775
- **NYGREN AO ; DEAN J ; JENSEN TJ ; KRUSE S ; KWONG W et al.** Quantification of fetal DNA by use of methylation-based DNA discrimination. *Clin Chem,* 2010, vol. 56, 1627-1635
- **LO YM ; FUN FM ; CHAN KC ; TSUI NB ; CHONG KC et al.** Digital PCR for the molecular detection of fetal chromosomal aneuploidy. *Proc Natl Acad Sci USA,* 2007, vol. 104, 13116-13121
- **FAN HC ; QUAKE SR.** Detection of aneuploidy with digital polymerase chain reaction. *Anal Chem,* 2007, vol. 79, 7576-7579

- **CHIU RW ; CHAN KC ; GAO Y ; LAU VY ; ZHENG W et al.** Noninvasive prenatal diagnosis of fetal chromosomal aneuploidy by massively parallel genomic sequencing of DNA in maternal plasma. *Proc Natl Acad Sci U S A,* 2008, vol. 105, 20458-20463
- **FAN HC ; BLUMENFELD YJ ; CHITKARA U ; HUDGINS L ; QUAKE SR.** Noninvasive diagnosis of fetal aneuploidy by shotgun sequencing DNA from maternal blood. *Proc Natl Acad Sci U S A,* 2008, vol. 105, 16266-16271
- **EHRICH M ; DECIU C ; ZWIEFELHOFER T ; TYNAN JA ; CAGASAN L et al.** Noninvasive detection of fetal trisomy 21 by sequencing of DNA in maternal blood: a study in a clinical setting. *Am J Obstet Gynecol,* 2011, vol. 204 (205), e1-11
- **PALOMAKI GE ; KLOZA EM ; LAMBERT-MESSERLIAN GM ; HADDOW JE ; NEVEUX LM et al.** DNA sequencing of maternal plasma to detect Down syndrome: An international clinical validation study. *Genet Med,* 2011, vol. 13, 913-920
- **CHIU RW ; AKOLEKAR R ; ZHENG YW ; LEUNG TY ; SUN H et al.** Non-invasive prenatal assessment of trisomy 21 by multiplexed maternal plasma DNA sequencing: large scale validity study. *BMJ,* 2011, vol. 342, c7401, http://dx.doi.org/10.1136/bmj.c7401
- **SEHNERT AJ ; RHEES B ; COMSTOCK D ; DE FEO E ; HEILEK G et al.** Optimal detection of fetal chromosomal abnormalities by massively parallel DNA sequencing of cell-free fetal DNA from maternal blood. *Clin Chem,* 2011
- **PALOMAKI GE ; DECIU C ; KLOZA EM ; LAMBERT-MESSERLIAN GM ; HADDOW JE et al.** DNA sequencing of maternal plasma reliably identifies trisomy 18 and trisomy 13 as well as Down syndrome: an international collaborative study. *Genet Med,* 2012, vol. 14, 296-305
- **DAN S ; WANG W ; REN J ; LI Y ; HU H ; XU Z et al.** Clinical application of massively parallel sequencing-based prenatal noninvasive fetal trisomy test for trisomies 21 and 18 in 11,105 pregnancies with mixed risk factors. *Prenat Diagn,* 2012, vol. 232, 1225-32
- **BIANCHI DW ; PLATT LD ; GOLDBERG JD ; ABUHAMAD AZ ; SEHNERT AJ et al.** Genome-wide fetal aneuploidy detection by maternal plasma DNA sequencing. *Obstet Gynecol,* 2012, vol. 119, 890-901
- **JENSEN TJ ; ZWIEFELHOFER T ; TIM RC ; DŽAKULA Z ; KIM SK et al.** High-throughput massively parallel sequencing for fetal aneuploidy detection from maternal plasma. *PLoS ONE,* 2013, vol. 8, e57381
- **GIL MM ; AKOLEKAR R ; QUEZADA MS ; BREGANT B ; NICOLAIDES KH.** Analysis of cell-free DNA in maternal blood in screening for aneuploidies: meta-analysis. *Fetal Diagn Ther,* 2014, vol. 35, 156-173
- **DONDORP W ; DEWERT G ; BOMBARD Y ; BIANCHI DW ; BERGMANN C et al.** Non-invasive prenatal testing for aneuploidy and beyond: challenges of responsible innovation in prenatal testing. *European J Human Genetics,* 2015, vol. 23 (11), 1438-1450
- **FAN HC ; QUAKE SR.** Sensitivity of noninvasive prenatal detection of fetal aneuploidy from maternal plasma using shotgun sequencing is limited only by counting statistics. *PLoS ONE,* 2010, vol. 5, e10439
- **ZHAO C ; TYNAN J ; EHRICH M ; HANNUM G ; MCCULLOUGH R et al.** Detection of fetal subchromosomal abnormalities by sequencing circulating cell-free DNA from maternal plasma. *Clin Chem,* 2015, vol. 61 (4), 608-616
- **BIANCHI DW ; WILKINS-HAUG L.** Integration of noninvasive DNA testing for aneuploidy into prenatal care: What has happened since the rubber met the road?. *Clin Chem,* 2014, vol. 60, 78-87
- **SPARKS AB ; WANG ET ; STRUBLE CA ; BARRETT W ; STOKOWSKI R et al.** Selective analysis of cell-free DNA in maternal blood for evaluation of fetal trisomy. *Prenat Diagn,* 2012, vol. 32, 3-9
- **SPARKS AB ; STRUBLE CA ; WANG ET ; SONG K ; OLIPHANT A.** Noninvasive prenatal detection and selective analysis of cell-free DNA obtained from maternal blood: evaluation for trisomy 21 and trisomy 18. *Am J Obstet Gynecol,* 2012, vol. 206, 319
- **ASHOOR G ; SYNGELAKI A ; WAGNER M ; BIRDIR C ; NICOLAIDES KH.** Chromosome-selective sequencing of maternal plasma cell-free DNA for first-trimester detection of trisomy 21 and trisomy 18. *Am J Obstet Gynecol,* 2012, vol. 206, 322
- **ZIMMERMAN B ; HILL M ; GEMELOS G ; DEMKO Z ; BANJEVIC M et al.** Noninvasive prenatal aneuploidy testing of chromosomes 13, 18, 21, X, and Y using targeted sequencing of polymorphic loci. *Prenat Diagn,* 2012, vol. 32, 1233-1231
- **NORTON ME ; BRAR H ; WEISS J ; KARIMI A ; LAURENT LC et al.** Non-Invasive Chromosomal Evaluation (NICE) Study: results of a multicenter prospective cohort study for detection of fetal trisomy 21 and trisomy 18. *Am J Obstet Gynecol,* 2012, vol. 207, 137
- **NICOLAIDES KH ; SYNGELAKI A ; GIL M ; ATANASOVA V ; MARKOVA D.** Validation of targeted sequencing of single-nucleotide polymorphisms for non-invasive prenatal detection of aneuploidy of chromosomes 13, 18, 21, X, and Y. *Prenat Diagn,* 2013, vol. 33, 575-579
- **NORTON ME ; JACOBSSON B ; SWARMY GK ; LAURENT LC ; RANZINI AC et al.** Cell-free DNA analysis for noninvasive examination of trisomy. *New Eng J Med,* 2015
- **REDON R ; ISHIKAWA S ; FITCH KR ; FEUK L ; PERRY GH et al.** Global variation in copy number in the human genome. *Nature,* 2006, vol. 444, 444-454

- **MCCARROLL SA ; ALTSHULER DM.** Copy-number variation and association studies of human disease. *Nat Genet,* 2007, vol. 39, S37-S42
- **WALKER S ; JANYAKHANTIKUL S ; ARMOUR JAL.** Multiplex paralogue ratio tests for accurate measurement of multiallelic CNVs. *Genomics,* 2008, vol. 93, 98-103
- **OHNO S.** Evolution by gene duplication. Springer, 1970
- **DEUTSCH, S ; CHOUDHURY U ; MERLA G ; HOWALD C ; SYLVAN A et al.** Detection of aneuploidies by paralogous sequence quantification. *J Med Genet,* 2004, vol. 41, 908-915
- **HOWALD C ; MERLA G ; DIGILIO MC ; AMENTA S ; LYLE R et al.** Two high throughput technologies to detect aneuploidies identify new Williams-Beuren syndrome patients with atypical deletions. *J Med Genet,* 2006, vol. 43, 266-273
- **LANGMEAD B ; SALZBERG S.** Fast gapped-read alignment with Bowtie 2. *Nature Methods,* 2012, vol. 9, 357-359
- **PAGES H ; ABOYOUN P ; GENTLEMAN R ; DEBROY S.** Biostrings: String objects representing biological sequences, and matching algorithms. *R package version 2.29.0*
- R: A language and environment for statistical computing. R Foundation for Statistical Computing, 2013
- **UNTERGRASSER A ; CUTCUTACHE I ; KORESSAAR T ; YE J ; FAIRCLOTH BC ; REMM M ; ROZEN SG.** Primer3 - new capabilities and interfaces. *Nucleic Acids Research,* 2012, vol. 40 (15), e115
- **KORESSAAR T ; REMM M.** Enhancements and modifications of primer design program Primer3. *Bioinformatics,* 2007, vol. 23 (10), 1289-91
- Database of Single Nucleotide Polymorphisms (dbSNP). National Center for Biotechnology Information, National Library of Medicine
- **GUENTHER F.** Package 'neuralnet. *R package version 1.32,* 2012
- **KIM SK ; HANNUM G ; GEIS J ; TYNAN J ; HOGG G et al.** Determination of fetal DNA fraction from the plasma of pregnanat women using sequence read counts. *Prenat Diagn,* 2015, vol. 35 (8), 810-805
- **NICOLAIDES KH ; SYNGELAKI A ; ASHOOR G ; BIRDIR C ; TOUZET G.** Noninvasive prenatal testing for fetal trisomies in a routinely screened first-trimester population. *Am J Obstet Gynecol,* 2012, vol. 207, 374
- **CHANDRANANDA D ; THORNE NP ; BAHLO M.** High-resolution characterization of sequence signatures due to non-random cleavage of cell-free DNA. *BMC Med Genomics,* 2015, vol. 8, 29
- **SNYDER MW ; KIRCHER M ; HILL AJ ; DAZA RM ; SHENDURE J.** Cell-free DNA comprises an in vivo nucleosome footprint that informs its tissues-of-origin. *Cell,* 2016, vol. 164, 57-68
- **YU SCY ; CHAN KCA ; ZHENG YWL ; JIANG P ; LIAO GJW ; SUN H ; AKOLEKAR R et al.** Size-based molecular diagnostics using plasma DNA for noninvasive prenatal testing. *Proc Natl Acad Sci,* 2014, vol. 111 (23), 8583-8588
- **LO YMD ; CHAN KCA ; SUN H ; CHEN EZ ; JIANG P ; LUN FMF et al.** Maternal Plasma DNA Sequencing Reveals the Genome-Wide Genetic and Mutational Profile of the Fetus. *Sci Trans Med,* 2010, vol. 2 (61), 61-91
- **LANMAN RB ; MORTIMR SA ; ZILL OA ; SEBISANOVIC D ; LOPEZ R ; BLAU S et al.** Analytical and clinical validation of a digital sequencing panel for quantitative, highly accurate evaluation of cell-free circulating tumor DNA. *PLoS ONE,* 2015, vol. 10 (10), e0140712